# EUROPEAN PATENT APPLICATION

(11) **EP 2 481 813 A1**
(43) Date of publication of application: **01.08.2012**
(21) Application number: 11152864.2
(22) Date of filing: 01.02.2011
(51) Int. Cl.: C12Q 1/68

(54) **Markers of cutaneous melanoma and uses thereof**

(71) Applicant: Centro di Riferimento Oncologico - Istituto Nazionale Tumori - Aviano, 33081 Aviano (PN) (IT); Azienda Ospedaliera Universitaria Senese, 53100 Siena (IT)
(72) Inventor: Sigalotti, Luca, 33085 Maniago (PN) (IT); Maio, Michele, 33080 Roveredo in Piano (PN) (IT)
(74) Representative: Capasso, Olga

(57) **Abstract**

The invention relates to a method for determining prognosis, predicting or monitoring response to therapy of patients affected by cutaneous melanoma through the evaluation of the methylation of different genomic DNA sequences, including the Long Interspersed Nucleotide Element-1 (*LINE-1*) repetitive DNA sequences and/or the concomitant methylation profile of specific groups of genes defined as methylation signature.

SEQUENCE LISTING

<110>  Azienda Ospedaliera Universitaria Senese
       CRO-Centro Riferimento Oncologico di Aviano
       Sigalotti, Luca
       Maio, Michele

<120>  MARKERS OF CUTANEOUS MELANOMA AND USES THEREOF

<130>  BE 109204

<160>  229

<170>  PatentIn version 3.5

<210>  1
<211>  2001
<212>  DNA
<213>  Homo sapiens

<400>  1
aaattaaaaa aaaaattttt ttttttgagt tggggtcttg ctttgtcacc cagcctggag      60

tgcagtggca cgaacatggc tcactgcagc ctcgacttcc caggctaagg cgatccttcc     120

tacctcagcc tcccaagtag ctgggactgt ggacgcacac cactacaccc ggctattttt     180

ttttaaaatt ttttattttt ttggtatttt tgtagcaatg gggtttcacc atgttgccca     240

ggctggtcac aaacttctgg gcttggatga tcctcctgtc ttggcctccc agtgttagga     300

ttacaggtgc gagccaccgc gtctggctga tgtcttctca tgatgagatt ccggttgtgc     360

cttcctggct ggaatgccac atagatgatg ctgtgacctg cgaagacctt cacatctgga     420

ggcacacgac accctcctgc ctctcacagg tgatttattt taaccccttg gtcaagatgt     480

ctgattcctc tactgtatag ttactctttt tccttttgtt actaataagc aatctatcaa     540

gagatgcttc gtgaccgtgc tgacaccctc ccccctcact gaagtcttcc ccccagattt     600

agcccccact gatgagtttt gcacacaggc tgtcttcttg taggcagccc cctctgctac     660

ctttgttttt ccgggaagga ccttagatcc tgggtaggtg ctcatcctgc ctggcagtcg     720

agtgcaatgc tgctgtcctg gccaatgccc agccttcctc atttgccccg gcttcaaggc     780

attggttcac agtcccgtac gggcagctag accatctcac agcaagcagc actctgggag     840

gctcatctca tttcctgccc tggaagcttc ccttcctcac tgcagccact gaagctgcag     900

gctagatcaa ggggaccaca gagttgtcca gtccccaggg tgggggtgcc aggagccctt     960

gtcctccgta gcaggtctca gaggctgacg atggggaaat cgcactggtg aagctgccgt    1020

ggggtcagga ggaacctgcg ataagacgcc caacagttag ggcaggtgct ttgaggtttg    1080

gagcctggga ccccacagga agtgacttcg tgctgagcta atgttgcttc atcctggcgc    1140

cagtctcagg ccaagagctg caggctggca tggctggggg gatgtcagcg gagtgccctg    1200

agcctgggcc aggaggtctg cagggccagt ccccagggcc aggcaggcag tgtccccctc    1260

ccatcacgcc cacctcctgg agcctgcccc cgtggagggc ctacgtggct gccgccgtcc    1320

tctgctacat caacctcctg aattacatga actggttcat cattgcaggt gaggagggga    1380

tggctaccct gggcagtacc tgctgctgtg cccaccagcc aggggctgca gatccatggt    1440

ggccttccag ccatcaccca tctttctgct cctgggccca tctctggtgt tctcacctct 1500

agaggtggtt ttcagctgtg ctcgaatcgg ggaggaccaa ctgatgttcc caatccagcc 1560

tcaactaaga agggacacag gctgggcatg gtggctcccg cttgtaattc cagcactttg 1620

ggaggctgag gcaggaagat tgcttgagcc caggagtttg agaccaacct ggacaacata 1680

gtgagactct gtctctattt atttattttt ttttttgaga cagagtctcg ctctgtcgcc 1740

caggctggag tgtagtggcg caatctcagc tcactgcaac ctctgtctcc caggttcaag 1800

tgattctcct gcctcagcct cccgagtagc tgggattaca ggcgtgcacc accacatccg 1860

gctaattttt gtattttgag tagaggagga gtagggacgg ggttttacca tgctagtcag 1920

gctggtctcg aactcctgac ctcaggtgat ccacccgcct cggcctccca aagtgctggg 1980

attacaggca tgagccaccg t 2001

<210> 2
<211> 2001
<212> DNA
<213> Homo sapiens

<400> 2
attgtggcct gtgtgtaaga gtatgctcat gtgaagctac attgtgtttg cttgtggaca 60

tgaggtgttt gtggctcctg gatgctttgt ctgttgaatc ctgaccccaa gtgacctgct 120

gatacagggc accttggaag tcaattaggc caaaagaccg tgtctgtgca ctaatgagaa 180

gctttcccca ggtgcccctg cccaccttct ccacctctcc tgtggttcct gcctggcttg 240

ggtttggcct ttttaaccca aagacaacat gcatccaaat gagattttct ctagtgcata 300

cgtctctctc tccctccctt ctcctctgcc ttccctgtat ctcagaaaga aaaggcctag 360

gaataaggaa aaggaggagg aagaaacatt tttttgagga cataccatgc ggcaggcctg 420

tgctgggcag tatacacgca ttataggcaa ttgtagacag ggcttccctt gtgtcaggca 480

ctgttctaag cacttcaccc ctaaggctgg gttaatccac gtatccaccc tatgaggtag 540

gtgttgttct tttcccactt tcacagagaa ggcaagaaat cacacagtga gaaagtggca 600

gagctgggat tcaggcctca gcttctccca cacaagccca cagatgggag gatgaacaag 660

gagtcagagt ggacctgtgg ctctcccctg agaggatacc aggattcgag gctcagcgcc 720

ctgggaggag tgaacccctc agtttcctga gcctcatcag aagcagtctg gggaggctgg 780

ggcccagcca atggcagtgc aggtcccagc tcctcctcct cccaggctgg gccagcacac 840

ccggcaggct ctgtcctgga aacaggcttc aacgggcttc cccgaaaacc ttccccgctt 900

ctggatatga aattcaagct gcttgctgag tcctattgcc ggctgctggg agccaggaga 960

gccctgagga gtagtcactc aggtaagggc atctgtgtcc gcatgtgccc tgccccttgg 1020

catacgtggc aggaggacac ttctctgctg gggagtggtg ggatgtggcc tggacacatc 1080

tgccaccctg ggtcctgatg gtggaggaat aaggggctct gggttctttc aggtctcccc 1140

agccgggcaa aggtaggagc aggggccagg ccggaagctc agcaggcagg gtagctctgc 1200

ctgggcctgc aaagccctgc cccactcctc cccaagtagg gatgggctgg gtgagagccc 1260

ctggggctaa gggcggggcc ttcctgggtg ttccctgaga agctgcagga gtcctacctt 1320

agacaggact gagcatgcaa gatggtgatt caaaacagag ctccccccaa ccccggcccc 1380

cacctccacc catcccccgt ccaagtggcg caggtggtgt ggagacaggg tggtgggcaa 1440

aggttccccc agccaccctc atccaggatc cacacacaag gggaaggaca gaatccgtct 1500

gcacacaaac aggcaggcag ctcattgaaa cttgaggaaa gggtctgagg cctaaggctg 1560

ggcagttccc ggagggtggg ctctagagcc agcctgcctg ggttcaaatc ctagctctgc 1620

cacttattag ctgtgtgacg ttgagcaagt taattaatcc ctctgtgttc ctctttcctc 1680

atctgtaaat tgggaataaa aaatatgccc tttcctaagg ttgctgtgaa ggaataatgt 1740

actgattcat acaaagaact tagagcggtg cctggcacgg catgaatgct tcatggatat 1800

tggctgttat cactactctg ctttgcagtt gatcatgcag aggctccaga gagcctggtg 1860

agcttcagcc tggaccagca tgggaaggcc atcatgagcc ctttagtggc taggcagaca 1920

ggttccttgg ggtcccaggg tgcacagttg gttgaaacca cagtgcatgg gtcttaatgg 1980

acacaccccc atctccagga a 2001

<210> 3
<211> 2001
<212> DNA
<213> Homo sapiens

<400> 3
tgggtccaga gaggttaagt gacctgttca aggtcacaca tcaatccagt tgtagacctg 60

gggccagaat ccaggattag atctcaccag gctctctgtt cccaccacac tcttcttcca 120

aatggggcat cagctcctgg gtgaggactg tggatctgcc acaggaaccc cattccacca 180

accttcccaa gcctctccat cccaatccca catcgctccc cttcacctgc acaaactgct 240

ccatggccca ctggtccagg caggtgtagt tctccaggaa gcgcagcttc tccaactgaa 300

actggtccct ggactgagcc tccgcctgcc tctctagcag ctggaagata gtggcaccga 360

gcagcaggta gcagacatag gccagcagca ggggcagcac ccggccaccc cagcagctgc 420

agagcccagc actgggcatg ctgtggccag gaccctgcca gggccgtggg gctggctatg 480

ggggagaggt gggaaacagg tgaggagtaa gcacctaggg gcctgtgccc aggctgccgc 540

ctgccctgcc ctcctcctcg gcacaggtgt ctggaggctg gggagactgt ttgaacagtg 600

cggctcagct tggctgcact aagtgctatg ctgtgagtgc aaacaggccg gccaccccca 660

cccggccttt gtttccatgg acagcagcaa gtcagtgtca gggacccttc aaagagacct 720

accaggttct cctgagtgct tcatgtctct ctctctgtct ccaagttgga tagatgtttc 780

tgggctaaat cggataggtg tgctcactct gtccaggccc cagtgggctc tctcggccgc 840

ccccagcgtc ttcacccttg aaggctgtaa gaccccagct taggagctgg gaagaggtca 900

gaatgggtat gaataaacct tctactcagt tgccttggct acaaaatggg gcaggataat 960

tgaactgcct attgggggaa cctcaggatg agtaaactgc gtgacaaatc agggcaaagt 1020

aaatagacag gtgattgtct cactcagcct gatggtaaga gtatcagggt tgattctgct 1080

gataagttgg attcattggt tttataactc ttgctaattt gtcttgcatg aagccctcta 1140

gtgggttacc ttcagaggat atgtcggtga aatatatgtg tgaattaaaa agaaactata 1200

agtatatcct gatcctggga tggtaagagg ggctgtctct gagtggatgg aattgagagt 1260

gctttccttt ctctttgcct gtgtgtgtgt gtgtacacat gcttgtttat gtacatgtat 1320

aacagcttta tacagatata attcacatgc tgtatcgtcc acacatttaa agtgtgcaat 1380

tcagtgggtt ttagtatatt cacagggtct tcaagatcca gccctgttgc aacatgtaac 1440

agtacttctt ccttctttct ggctgagtaa tatttcattg tgtggctata acacatttgt 1500

ttatccattt gatttctagt tgatggacat ttgggttgtc cctgtttcct tctattaaga 1560

acaaagcagc tatgaacctc tgtgtatgag ttttttttgt ggacatgtgt ttgcattact 1620

cacaggtaca tacctaggag tagaatttct ggactatctg gccactctgt attgaacttt 1680

ttgaggaact gccaggctat tttccaaagt ggctgaacca ttttatattc tcactagccc 1740

agggcgagga ttccaatatc tctgctgtgt tttctgctgt ttcctttaat gagctgtggc 1800

tgcagacact gtgagttgcc tgctgagtat cctttccctc ctccccagct agcagaaccc 1860

cggctttgta agtgttaacc ccaggtaatt gagcataatt ggtctaagtc atagtgctaa 1920

tcttgctccc ctctgtctca gcccctgaga gcagtgaagt cactgaagac ccataagaca 1980

gtgggcaaga gggccaggct g 2001

<210> 4
<211> 2001
<212> DNA
<213> Homo sapiens

<400> 4
tgggcgcagc aggccatggg ttaccacggc cagacccagc agcagcaggg gccagcgctg 60

cctgaggttg gggcctggct caccaggggc ctcaaggccc ccctggcctg tccctggcct 120

catcggccct cagggtcaga ggtccttgtg gcctgacttg cagtgtgggc tccctagaga 180

gtggtgccct ggtccctctc gctggcagca gctgtcccat tgcaccccag tccatgtggc 240

cccatcctca tcctctggtc cctgccccca gccaggcccc ttccagagtc catgaccgtt 300

acccctgggg ttctgtccca gaggcacatc tgccaactgc cctgctgtgg tcaggatcca 360

gggctcaact gcctcccaca cctgtcagct tggccttgcc cctggggccc tgggtgcttt 420

gtttggggac ctcccaccac aggcagaggg tcagcaagag ctgaggtcac acagggaggt 480

gggatgcgcc atggcccccc aagaccctcc cttggggctt tcctttggag gcacctgcgc 540

cctggatggg cttgagtccc ctcggggctg gtcccaagag gtgatgcacc tgacttccct 600

ccttccccac ttccccgagg gcgccctggg tctgtggagc atctcactgg ggtccagaga 660

gctgcccagc aggagtcccc cagggcttga gctcaggggc ctgggctgag gctcctccaa 720

ccggtcctag agggaggtgt ggttagagag caggaaacag gtttgggttt agccagaagc 780

cactcaacgg ttcaagtcca ggaatcatga gataagaatg aaaatgctgt ttaatgcacc 840

atgttcagca cctcactgac ctcacatcac acagacacac acacacacac caaccacccc 900

agcttcctga aggtataatt gacaaaactg tatatatatt taccacctac agtgcgatgc 960

tttgccacac tgcaaaatga ttaaatcaag ctgacacaca cgtccgtcat ctcacatact 1020

tatgcttttg tagtgagaac atggaagatc taatcttagc aattttccag tatacaatac 1080

agtttttaac tgtagactcc atgttgtacg atacatctcc agagttcatt cttatagatt 1140

tcactcaaag gccagtagct tcctcctgag cggtgagtgc atcatgtcat ggcttctgac 1200

aggcccccgg tcctctccca gcccagcatc cccgaccctc tcccaacatc tcgcgaccct 1260

gtgctttggt gttgagggag cagtggcagt caggagggac tgagcacctg tgctgggcgg 1320

ccagggccag gctcttttgc tggtgctgcc agagacgcct caggtcaccc aagagtctca 1380

ggttcctctc cttcctccag aggcggggct ggaaatgctg ctggctctgc tcacactcca 1440

gggctgggtt gaggttggtg ttgggagata atgcatgtca gtgttgtttt cttcatctgt 1500

gaaaaagcag taacatacac acaacctaaa atttatcatc ttgacctttt tttttttttt 1560

gagagtcttg ctgtgtcgcc aggctggagt gcagtgtggc gtgatctcag ctcactgaaa 1620

cctccgaatc cctggttcaa gcgattctcc tgcctcagcc tcctgagtag ctgggattac 1680

aggcatgcac caccacgaca agctaatttt tgtattttta gtagagatgg ggttcaccat 1740

gttggccagg ctggtctcga tctcctaacc tcatgatcca cccgcctggg cctcccaaag 1800

tgctgggatc acaggtgtga gccaccgcgc ctggcccatc ttgaccattt ttaagtacac 1860

agttcagtgg cattaactac attcacaatg tcgtgcaacc atcaccacca tccatttcta 1920

gatttttctt ttttcagagt cttgctctgt tgcccaggct ggagtgcagt ggcgcaatct 1980

tagctcatgg caatctcttt c 2001

<210> 5
<211> 2001
<212> DNA
<213> Homo sapiens

<400> 5
attttttgta tttttagtag agatggggtt tcaccatgtt agccaagatg gtctcgatct 60

cctgacctca tgatccacct gccttggcct cccaaagtgc tgggattaca ggcgtgagcc 120

accgcacctg gcccagaata tttatttaga gagtgatgtc aggcctaagt gttgatagtg 180

ggtagtagtg ggcaagatgc ctggccctct ctctttcttc tcatgagaga aggtggaatg 240

ccttccacca aagaaacgag acctccccat gaccagctgc tccactaacc acacatcctc 300

cagtgatgcc tctgaatggt cccgaggggc tgtggtggcc gggcagagcc aggcaggagc 360

cagagtcagc ctggggggtg atggagctga ggccatcacc ggtctgacag tggaccagta 420

tggcatgctg tataaggtgg ctgtgccgcc tgccaccttc tcaccaactg gcctcccatc 480

tgtggtgaat atgagcccct tgcccccgga aaaaaaataa tattgcatat gcagttgatt 540

aaatggcata ctccatggct attaggaatg atgaaaatgg gctgggtgcg gtggctcaca 600

cctgtaatcc cagcactttg gtaggccaag gtgggcagat cacctgaggt cgggagtttg 660

acaccagcct aatcaacatg gagctgtgtt tactgagtgg ggctttcgca ggctggagct 720

aagaatttcc agtatgcgta acagccacag cccaaatatc tgccagtgag ttgtgtaatt 780

ccccagagca ggcctgggca gtgtctgggt ggggcctggg agccacagga gacgcccaaa 840

gccaggcaga gcccgggggc gagggggcgg caggcaggtg tagcgctgcc ctgggcgggc 900

ttgcaccccc acacccaagt gagcggcctg ctcactcctc agctgcagga gccagacgtg 960

tggagtccca gcagaggcca acctgtgtct cttcatctcc gtgagaaagg tgcccccgaa 1020

gtgaaagaga tggcctggtg gaaagcctgg gtaagtggga gctggcagga gatttgggtt 1080

cctgcatggt gttgggtgct ggtcaccagg gagtcctgag aaatgtaaag tcgcccttgg 1140

ctgggagtgg ctcagtctac atttggctgc ctcctgggcc aagcatggag ggtgggaagg 1200

ggccgagaag aaacagtccc aggcctagag tgcccacggc agggatggga cctgggccgg 1260

gagacaggct gatgaagaaa tctagaaatg caagggggaa gctgaggttt gagaagagtc 1320

caggggaggg acccttgcag gggtggggcc actcaaagga gggggtatca tggttgagcc 1380

ttgtgtgtaa atgtgtatgg ggtgcacaca cgtgtgcatt tggggagaat caggcatact 1440

gctcctggca gagggaactg tgtgcagagg gtcagagggc ggggagcttg gctttgaggg 1500

aagctgctca gcacagctca agggtcctgg taccggagga gatggacctg gggaagcagg 1560

cagggccagg tcctgaaagg ccttgcctgg caggctgagg agttaggctt taccttaaga 1620

gaagtgggaa ggcactgaag ggctttgagc atgagagtaa cctgaacctg ctgttgggga 1680

cccatcaatg ccttcacctc agggatcaat gatgttcatt tgtcaagact ggagagggaa 1740

gaggggaagc aggaggacct agcctgctag agcctggagg aacccggggc tcagggcagc 1800

ccatgcccag cagaactgat gggttctcgg gagctttctg tctatggttg agcctgaggc 1860

catcgctgtg ctaaggagtt gaatatccag aaggggacag aggtgacatc tcccggctgc 1920

cggctctgca cacccttttc ctttcctccc aggcagcctc aggatgtcag ggctgcactt 1980

tctgttttgc agatgatgaa a 2001

<210> 6
<211> 2001
<212> DNA
<213> Homo sapiens

<400> 6
aaacacaaaa ggaaaaaaaa taggcaaaaa taaagattat ctcatatgga atttcagtaa 60

gatacttcta ttacttggaa aattccaaag gtttttgtcc taggagaaat gccacattct 120

tttttacaca gcagattcct actctaaccc tccccatact tcctacctcc ttagttctcc 180

ttgccaccga attatcctga catatgtact tacgcgtttg gttatttggg gcctccatca 240

cattaaactt tatgtttgat gacattatgg aatttgtttt gcttgctcct gtgtttcttg 300

aacctactac actgcctaat ataatatgtg ctcaaaattt ttgaaagaaa aatataaacc 360

aggtaaacaa atcatttctt acagtgaagt gatggaaatc agtcttaata gaaatcagat 420

atccctcatt agtcctagtg gtgttgctgc cagtctttaa acaacttcct accaaagatt 480

ttcttcagag ccatcatcac ttctttgttc ctaagggtgt agattagtgg attcagtaca 540

ggggtgacgg cgctatacat gatggccatt atccggtcct gaatcatgga ggtggctgaa 600

gcaggacgaa tatatgtgaa gcccacaggt ccatagaaaa gacataccac cataaaatgg 660

gaggcacaag tggacagagc cttgtggagt attctgcagg acctgttctt aaacagaagg 720

aagccaatta catagaagca ggagagaaga gtcagaaaga aagctcccat ggatatgctg 780

cctgtgacaa tggaaagaag ccattgattg agtaatgtgt cactacaggc caattctaag 840

agcggcttga catcgtagaa gaagtgattg agtttctgag agccacaaaa actcaggtgt 900

gcagtcatga cagaatgcat cagagcgtaa aagaagctga tgagccaggc cgcagctgcc 960

aacagaatac acacctgggg gttcatgatg acagtgtagc gaagaggatt gcagatggca 1020

acaaaacggt caaaggccat gatagccagt aaaatggcct ctgtgcttcc caaaaagtgg 1080

aagaagtgta gctgggtgat acagcctaga aaagatatag ccctgcgact gcacacgagg 1140

tttacgagca gcttgggcag tgtcactgaa gaataagaaa tatccagaca agaaaggttt 1200

cccagaaaaa aatacatagg ggagtggagt tgtggttcca aaacaaccat caccaatata 1260

gatccatttc caatcaagtt tatcaggtaa atgattaaga aaatcccaaa gaagaaaggc 1320

tgcagctcct gaacaccagt caggccaagt agaagaaact cattcattgt agtgacattc 1380

tccattgctc tgggaagcaa atttaacaat aacagaatta atttttctga atttttaatt 1440

ttacactgtg aggattaaat aaagcaagtt tactatttgg aggaacctag gggtgagcaa 1500

cagtgtgaag aatagttacg aacagtaaaa ctaaatttat gtacaacaaa attgaggaag 1560

actaatatac ctgataaatt ctgagctgtt aaaatggcac tagactaata aaagcattat 1620

attatgaaca aatcagatat agataaaggg catttgtttt caaaactagg aagtgcaaga 1680

tttgatggac ataatcataa cctcatgtat ggcattagaa ttatgtatta caaaaatttt 1740

aaacatacaa acagggaata gtttttcaac ttatgattct aggttctggc aaaaattcag 1800

atgtagctca gtggggattt catcacttct tctaagatac ataaaagcca caagagagta 1860

acacatgtct tctgacaacc caaactttca gtgaaagtaa gaaacataaa cacccacaaa 1920

ccaaatgttg tatgagtaga ggagaaaaaa acagcaaaat tagcatcatt agtcacaaga 1980

ctgtgtcata gcaatgaggc a 2001

<210> 7
<211> 2001
<212> DNA
<213> Homo sapiens

<400> 7
ccagaggaag tgggggtctt ctgaagggtt taagtaggct gggaggacat gatcttagga 60

agctcactct ggtgtcagtt gcaggatgga tttgagagga gcaagttagg tgtagatgcc 120

catgatgatg ccaagatctg ggcaacagac aggaaggccc tagctcagaa gtggctctag 180

ggaaggtgag gtgcatagaa ttgagagatg ctcagtagat ggcatgagca gtgcttgatg 240

attgtctggg ttggtggagg aaggtggaca gggaaaagca gaaagctacg atggtgcctc 300

aaggggctga gtgatgtcac tcacagagac agaatgtata gagtgaatgt tcagactcac 360

aggaagtcca aaactacata ccccaacgtg aggtgctgtg ggacatccgg ggtgcagggt 420

ccagagagca ggtaggtaga gtttagaaga gggctgggtc cacaatgcag ccttggatgt 480

tctcaatgta agagttgtgg gagatgaagc cttgtgagtg gatgggaaca cccaggtgca 540

tttcaggtga agcaagggga caagaggctg aggacacaga caagcaaatc ctagatcttc 600

catcagtccc tagaaggcac gatgtgtgcc cctcccagca cacagcctga gccctagcac 660

agagctggcc gcagagaggg cagcagtgaa tgtgtcctcg gtggttcctc cagatggggc 720

ctttgtccgc agtgcacttg tctctgcctg ggttgctata gtaacccaca gatgcagaga 780

gacttggcct ccgtgttgcc atggaaacca gcaattgggt gtccctgtgt ggcatggcca 840

ctgagacctt gaggattcag tctggtcctg aagggtttgg ggggagactg cgaccagaag 900

atgtttccat gtcctaatta atgggtgatg gtggttgtta gtctgactgt tgccacggtg 960

atgaagggag acatccaagt gctggtttca gtactgaggc gaatacaggg aatttcaaca 1020

ggctccaggt cttactatgc agcctgaagt gggaccatcc cttaaaccca ctccatcctg 1080

tggccacgat gggggccagg acacctttgc cttctttctg ggtttctttc tttgccgaga 1140

cagggatttt gttcccagga ggcactccct ggcccatggg atctcagcat tcaaagcagc 1200

acaggaaacc tgggcccctg aaacggggcc accgaagaga tcggtaagct ttcaacatcc 1260

tgcctttagc ccatgggccc aaccattgcg tcagctccac ccacccaccc tcactgacct 1320

caacccttta cagtcccaag gcctcccctt cctgatggtg ctgctcccct atcaccacgt 1380

ggagccccac aggtatatcc ccgcaaaccc ctcttgctcc agacaggtga tcagggaggc 1440

ttcattactc caggagctgc ccaggacctc agggaggtcc cccaccatgg gagtaagggc 1500

ctctccccca gcctccaggg ctccggcagc tccacactcc ggccctctgc ctcttctgga 1560

gggctcctcc ttccagcttc tgcctgtctc cccacccccc agtgcttcat tcccatggct 1620

caggtcatca ctgccctcat ctggactgcc tgcctgcctg cctgccatcc ggggcccaca 1680

gagtgagcac tctatggaag tccctctacc ctgatccatc acgtaactca cctactgagg 1740

cagctgagac cagggcactg aaggaggcct tgagtgacca ggtgtggcca ggcagcaggc 1800

agggatgatg aggtgaggct gcaaggatgt ggagagtcac cccttctttt cctcctcagg 1860

agaacaacca ggaggaagta ctggaaggaa ggaagggaga tcgctcggtg agctggaggg 1920

gaggagggcg tgccggccct gggaaagggc caaccacagg agactgtgag ggaggtgggc 1980

ggaagacgtg ggtggctcag g 2001

<210> 8
<211> 2001
<212> DNA
<213> Homo sapiens

<400> 8
agcttgagta aattccaatt aattcttccc aactcattct aaagccacag aaaaacctca 60

ctgaaaagga cttctgtcta cgttaaagtg cttttaccca ataccttgtg aagctccctt 120

tcctggggga cacagttcgt ggtctctggc ttttggcagg tggtccactg catttccaca 180

ttcaggtgat actccaggtg gtcagtgacc tgtgggcgcc aggcgtgggg gaggcacagc 240
ttgtcccctt ctcccctgtc tgcggtttct gtgcctgggg gctccgctac cccacgtcca 300
agggcaagga gctggtccta ggcggattag cgggctcctc tctcctctat ttgactcatg 360
attgtttttg tcattccaag agaattaacc agaaaccatc agaaacatga actttccccc 420
tgggcaaatc tatcccttgg gtaatgcaag tgacgctagt gattctggat aggggtctgt 480
aaactttttc cgtaaagggc tacatagtaa acatcttacg ttttattggc catgtggtct 540
ctgtaactac tcagttcggc atgagtgtgg cagcagcaag cagtggcaga caaagcacag 600
gcatgctcat ggctaggtca caagaacacg acttacaaaa gtgggcggca ggcagttttg 660
gcccttggga tgtaatgtgc tgaggatccc tggtcctggg ttttgtttaa gtgccttaat 720
ttcatcaccc tagtgaattt atgcttctct taaagtgctc acatcaagct aagcaaggtt 780
gaatttccct tgagtgggac tattcctgac accagtggcc acccgatgtg ggcgtatcag 840
gcctggggag aggcgggaag tcatacactc acctgcctct ggactttaag gactcggaag 900
atcctgaagt ggtacttgtc atcactttcc ttgttatact ggtcggtgat ccactgcaag 960
ctgtccttcg catagttttc tactgccatc acttcatgga cgcttagaaa ggttttcttc 1020
cttgcttggt aggggagggc catcagagtc aatagaatgg ccaacaggag ctgtagggcc 1080
tgccagggct cagccatcat ccttcagctg cagaggaaca ggaagagtgt tctctgtact 1140
cctcagggac aagtcgaatc acactgaccc tacctgccct ggcaggattt aagagcagct 1200
ggtggtggac acccacagct ctcctctcct cctcctcccc cttcatcagc agctgaactc 1260
gaggggagag cagcaagaga ctgtgcaatg gtttttgcat aagttcatgc cacacacaca 1320
caggataaca cacacatttt ctcttttgag gggctgctcc ctcagcaaca cacaggcttc 1380
tgtggaccca gcattgtctt ggtgggagcc aggcgagggc ccctgggagt cctggaggat 1440
ggtagctgct ggagacacgg ggagattgga tgccaggact ggcgcattca taggtgagca 1500
gtggggggtg ggcactgtgg ggaggggccg cctgcaccgg aagccactct cccccagcct 1560
ggttcctgga gacgagttct ggaaaatccc ctcccttaga ggacaaagga gcagaggcac 1620
cctacagaaa ggtgtgaagg gagggagagt caactctgac accgacgatt ccccagtacc 1680
gacaagtcct agagtggcct ccgctggact tgcatgtttt ggaggaggag gcagtggcga 1740
ggaagcttca ggtgtgagac acagggagct ttctggttgg acattctggt ctcctaaagg 1800
tcccatcctg gggacctgat gggagtcccc agacttggct gccagttaga gaaggaagaa 1860
gccactggtg ggtggaggct ggagcttgtg tcccacggca cacagaatgc aggcttagtg 1920
cggttatgtt cgattcctag aaggcctgtt atggtgtctt ttgggaagat gcgatataaa 1980
aatgtgcagt gatggctcct g 2001

<210> 9
<211> 2001
<212> DNA
<213> Homo sapiens

<400> 9

agggtctcct cctattctaa agctaatatt cagttgtata tatacatata aaccacagtc 60
cctttatcca ttcatccact gatggacacc taggctgttt ccataattct ttagtaaatc 120
acagaaccct cttgttttct cttctaactt agcaaacttc atttgattga gtcacaacaa 180
aatatttatc acaagtaaac aagaaagatg gaaaatttct gatcaatgat gaccatccat 240
gttgctgatg atggaacaaa aagccagatt aaacaagtaa gacagataaa atcgttgatt 300
agatccattg cttcaactag aaagggaaca taggtgaaac atctactctg tactcttgta 360
tgtggggttt taaaaacaaa ccttaatacc aaaatgaaag acaagatcag caaatattag 420
gtgagaagtt taaaaaatta ttggtatttt gcatatagga aaataattta aaataaagtc 480
cctttacttc attgtacatt taattgaatt tggaggtttt ttatagtaag aaatgaaagt 540
tcgattgaga agagatgcca gctttcactc accatttctg acagtctccc aggaaccttc 600
ttcgtacact gcctttggta agctcctttc tcactggctt tattggcttg tgtctttcat 660
ttgtaggaac agttaactga caaagctgca gaggcaggct gagaggtggc tccaagagaa 720
ccctggccaa gccatttatg gtcaggggag tagccaagcc ctcagtccat ccctcgggaa 780
agatgggctg ctgctgcatg gccgacgtgt atggcctggt tcacccaccc tctttgcctt 840
gattttctgt ccttactttt caggctgctt tcttggctat acctccatct cagcaaagaa 900
gccacagctg gcctgagagc ttgggggctt ctgcagcctg cagccctttg gaaacaatgg 960
gttacatgag gctgaggctg gccactccat ccccagctga cgggagtcca cattaaccat 1020
gttactgata atgatggagt cattgtgtta catggacaac agcatggaca ttcgggatgt 1080
ctggaggagg ggtgggggcc cattctgttg caggagtcac actgctgggg gctccagctg 1140
ctgcaaggtg ctgcactcag tggccaatga gctgcctggt gtgagttcag cctcaccccc 1200
tctctgtttt tgtattttgg gtgggaagct ctctagacct ggaggaagcc atggacttgg 1260
gctgctttat aacttacaat ttccctgtta gggatttgcc tctttaaaat ggtgaatgag 1320
ttgctaataa aacaacagaa ttaaaccaat aatttggcca ttgaaatgaa aaccactgtg 1380
agaggagcag ataggagtgt aaggagagag cgactcaagt gacagaggag gccgttgttt 1440
agatgactct gggagatggc actgaagctg tgcgtgaaag atgagaagag ggcagacatg 1500
tgaagggttg agggaggaga gtcccaggca gaagccacag caaatgctaa gccctgtaag 1560
cttggtgcac tcccagagca ggcaggaggc cggttggctg gaacaacata aaccagggag 1620
tcacccggga aggggctaag gacggagtga gtttgagccc ttttgatgtg acatgggagg 1680
gcatggtaag attttcatcc ttagatgaaa ccttttccaa gggtttttca gtgggagaga 1740
tgcatgatgt aattgctggt gacgaggatt gctagggctt ctctgtggag ccaagattac 1800
aggggaagag agtggaagca ggtggctggc tagtggagtc caggtgagcg atgaaggtgg 1860
ctgctgtagg aagacagcag tgaatgattg tgctctctac tgtggaggtg gagccactgg 1920
gattaaatga tgaaaaaaat atggggcggg gaagggcgag gtgagggaaa gagaagaata 1980
aagccaactc ctgggtttga g 2001

<210> 10
<211> 2001
<212> DNA
<213> Homo sapiens

<400> 10
tatttaaaga tcatgtcaag gggaggataa tggggctcag agaggcaaaa tttgatggtg 60

tttattaagc agagagaaac acagaagctg gagccgtcta gtccttagag agtgtgctcc 120

aaggtgggac cagggaggga gcaaggcctt ggctgggcct gggcaaagct accagaggac 180

caaccggctt cctctcctct gattccacag cctactgctg ctggtgaatt tctgatcaag 240

aattctgctc cccacaaaga actgcctaga gggtgggacc aagccacccc tcacacttga 300

gcatccagtc cactccatcg tgatgatgat gatgatcata gtattggtga ctacagctaa 360

ccctcattaa gctctcacta tgtgccaggc tccgttccaa gctctttaca aataccaact 420

ccttcaaact ccccaaccac cctcagaagt agcgtttcta ccccgatttt atagatgaga 480

aaattgaggc attgttaaat aactctccaa gtttatacag cttgtaagtg gaaaagccaa 540

gattcaaact caggccatct gactccagaa cctgcacatc agactgcccc gcacttaact 600

gttgttggta acagttagtt gtccatctgc ttagcatgtc catcccttct ggggttggct 660

ggggcaagtc agggcccaac tcggcacagc cagctccgga gcactaacct aatgggggtt 720

ttgcaagact cgggactgtg tgcttcacct ggaaagtgcc aggtgtgtgg ataagccatg 780

tgagcagctg gcttcctggg caatgcccct ccccacttca gtgtcccagg cccctttagg 840

gcccagccct ccaggcactg tgagctggga atgaggtgaa ggaggactag gcttaccctc 900

tgtgtgcctc cattttctca ccagtaaact gtgtgtgttg gatcttacga tcttctaaaa 960

taactccagc tccaaatgaa ggccagtgga cccatgaaca cgctggctct tgcccagtgt 1020

gctgagaaga gggcaggagg gaggagtagg gtgggcacgg cagggcacag ggtgcagggc 1080

aggtgggttc agtttgattt caaagccgac tgcccgccgc cccaggtgct ctggtgtacc 1140

caataggggc tgcattggcc aggcggccta ggctctgcgg gtggagtgaa gatttctcta 1200

cctcccagtt tctccctttt cagctctggt aggagagagg aagagtgaag aggaagtcaa 1260

aagttctgtt catctggaag ggctgggagt tgcttcctgg gatggggagc tccgaagcca 1320

attcagaaga cggccccggg gtctggcggg gaggcccacc gctgcccaga gggctcggcc 1380

ggccgggagg gggcgctgtg tcctccccac gccctgcccg cgggctccta gcagcgttcg 1440

ccgcgctgca acgcaatgcg cgcccggctt tcccctgggc agcgttcagt gcccgcttgt 1500

cctgcagggc accgccgtct ccctttcatc ctggggctcc caggtccaaa gggttcccag 1560

atccagcctc ttgcctgaac caaagtggcc aggcactcag tttgtaagat gaacaaatga 1620

aggaaagaag gaacgaatga ataagggctc atcttgcaga gcgttcgcgt ctcagcgcgc 1680

accaggcaca tcagtgccaa aggtgctggg ggtcggcccc agcgcccggc gcggctggaa 1740

tgggggcggc gaggagctcc tcctagccag ccgcagggcg ggaactgccc aaacgcaggt 1800

cgcagcctca ctgggttccg cggcggctcc gctcagtagg gaccgccctg ttccctagcc 1860

cccaatagcc tcgccctcat aaattcacct gagcaccctg gcccaagtca ccccagatcc 1920

ctgggacttc ccaggccccg acctcggccg ggcgggaggc tgatccttta ccgcggcccc 1980

cttcccccat tcccggcttc t 2001

<210> 11
<211> 2001
<212> DNA
<213> Homo sapiens

<400> 11
aatgaggttt tcaacaagcg tcttggaccc ttagaagttt caagtgacag cccttattag 60

tggcacccct taggggctcc ctcaaactca atgccaagac tagcctgact ggagggaact 120

tagacaatgg agtgggcatt tgatgttcca gcatcttgag tgggtgccat tattctgtga 180

cacctggatc ctggggttcc ataagctggg gttctaggat gtccattctt agttgagtct 240

catttcctgt catatcgacg tcaaaggccc aagaccttcc tcctcccctt gagcaaacaa 300

gccacgccct gcaccaaagt ccatctcacc ttccagctac cctaggtaat tttcctggta 360

attcagtgtt tctaggaaag catgagtctt tccacccccc gccctgagat ttactgacat 420

atgaacacat atgattggca tgatataaga accactcatc tgctggccat ctctccaggt 480

ctggagccag agaagatgat ctgaaattgt agcaggagaa attgaggtag gatactaaga 540

aagcttttca ggagtggggc taggcaagag gtgcagcatg agggaataag agtgaatcct 600

cagtatctaa gggaggtggc aggtggcggg ggacttcttt ctttgggtca tctttggtgg 660

tgatttgaca ggaaggaaca aagtggcttc aaacattata caagtctcta gtctgttctg 720

tgtcctgttt tctttctcat tctttcagtg tggaatctat atgaccctgg gagggatgtt 780

ggttggaaga atgaccagct gatggagatg ctgctgtaat tattggtggt aataatgggc 840

agcagtgagc cacccggtgt gacagtgtag gagaaaacag tccaaactcc tgccaaactc 900

tctctactga tggcaaatca gaggagactc aaattgtaag tttatagtgg tctggctttt 960

ggccatgaca atgacacctt gcccttttaa tttggggccc gtgcaaatat tcactgaaag 1020

ctgtcaagag gaaaacagaa ttggttattg aatcacttgc ttcctctagg tgtatgaaaa 1080

ataatttcaa gtttaacaaa cacaaggaaa ccgcagggtc catgtcaaag ctgatgagct 1140

atttctgaaa ctcgtgcaga attgtggttt gtgtggtcta tgtcacggca cccttgaggg 1200

agagtgggca attgcctgaa cttggaggct gtgtcctgtc cccaggctgc tccagggctg 1260

cctccttccg actgggcctt cttatctggg actgttgagg gcaacaggcc ttccgaagac 1320

cagtgaagaa ggaggccctg caaacaggag gctgacaggg taggaacgag gccatgatcc 1380

ctttgcagaa ggacaaccag gaggagggtg tctgccccat ctgccaggag agcctgaagg 1440

aggccgtgag caccaactgc ggacatctct tctgtcgagt gtgcctgaca cagcatgtgg 1500

agaaggcctc agcctctggg gtcttctgct gccccctctg ccggaagccc tgttctgagg 1560

aggtgctagg gacaggctat atctgcccca accaccagaa gagggtgtgc aggttctgtg 1620

aggagagcag acttcttcta tgtgtggaat gcctggtgtc ccctgaacac atgtctcatc 1680

atgaactgac cattgaaaat gccctcagcc actacaaggt aagcctgggt caccgcagcc 1740

aggccctgcc tccacctcgc tgaggtgctg catcctacat gttcatcatg cctggcacct 1800

cagagtagct caacaatgga catctctctt tgtttcttct gcttcatcct gttttggacc 1860

cttgtcttgc ttttctgtgt atattttgag gctgatgttt ccatgcatta atgtgagtct 1920

gtctaaaaga ggatattgtc agtgtgatgt tagagtccca gtctgctcat ctgtagaata 1980

gagtaattgg actaactaat g 2001

<210> 12
<211> 2001
<212> DNA
<213> Homo sapiens

<400> 12
gacagatcta gactcagttt caaaaaaaaa aaaagactca ggccaaatct ccttccttta 60

tgatgcaaac tttctcggcc ccattccacc taatcaggct gctatttcct cccctggctc 120

ccagcattac acctctaggt caagcctttg ccacagtagg ctataattta tgtatctatc 180

cctctacctc tctctctctc tctttttttt tttttttttt ttttttttga gacagactct 240

cactctgtca cccaggttgg agtgcagtgg cgccatctca gctcactgca acctctgcct 300

cctgggttca ggcaattctc ctatctcagc ctgccactat gcccagctaa tttttgtatt 360

tttagtagag acagggtttt gctatgttag gccgggctgg tcttgaactc ctgacctcag 420

gtgatccacc ggcctcagcc tcccaaagtg ttgggattac aggcatgagc caccacaccc 480

agccttctgc ctctcttgct agaccatgaa tttgttgggc gctgggtctc tttcctctct 540

ttccttggtg cccagcaaag tgctcagcac agaagaggtg cttgatatat gcttattgaa 600

gtaatcatca acaaacattt atcaatcatc aatatattta tttattgagg atttactatg 660

aggtggggaa actgaggcat gaaaaaagag ctggttcaag gcaaatctag aatgaggatc 720

caggctcctg ccccccagag ctagcctgtg catttgagaa aggaccaata ccccccaaga 780

taagcatctg gaatggggac gagggtgcag cgtggttacc ttggatgccc atctcctttc 840

gacaccactg gatgaagtta gagacattgt ccctggcctg gaaggtacct ggctgggcgg 900

ccccattgca ggagaccccg acccggggca tgggaatctt ctgggcttgg gcaggtgcct 960

cagccaggaa ggccagggca gcgtcagtga caacgttggc gtgttggcac agcaccaggc 1020

ccgtttccag cacctgcagg aagttggctg cgtcgatgtc cagcccatag aggtcgcgaa 1080

gccactcagc caggtcttcc ttcatggcct ccaggtactg ctcactcgac ttgaaaggcc 1140

ggatactgca cacaggcggc cctagggtcc tgggcttcct cctgcctccc gcaggctggg 1200

acatggctgg accccagcag ggcaggaggt gggcacctcc cctctcccac tgccgcctct 1260

ttcctcccgc tgctgctggg tctcggctgg gttcttcctg attctgaggt tcccgtgtac 1320

tcgctgagag cccgggacct ccagtgctgc tacttgccac tggcttcagc tgccaggcct 1380

ggcccagccc ctgccccccc acattcctca gtccttgcca gagctttccc tggtggccct 1440

gcagcaacct tgctctcagg ctgcccagtc cacttggagt tcacccctct gaggccagat 1500

ggaagtgagg acactcctct ctttccccag agctggagag atcaagctgg ctgcctccta 1560

ccctctgctg gctgcggcca cagagcctgg ggtggctgcc ggcctcccca ctctgctttc 1620

ctcccctctc cttggagaca gcggctgcta caaacactcc ttgttaaccc ttcccagcag 1680

gcgaggactg ccctgcagaa ccaagggaga gggaggagag gttggggctg gggctggctt 1740

ctctcacggg gctggccaaa tgggaacttt gggggagtgg agggaaagca ggggtagggg 1800

ttgccatacc ccttggtgtt tagtgagtac aggagaatgg gaatgaaaca gacatagaaa 1860

tagggcccaa aactcataag tgtagcctta aatcctgtgt gaccatcata tactacctag 1920

cctctctgtg ccttagtttc tcaactgtaa aatgggggta ataatgttta cctttttggt 1980

gtgttgtgag ggataaagaa a 2001

<210> 13
<211> 2001
<212> DNA
<213> Homo sapiens

<400> 13
taagaggaat tgctcttgca ggtggtaaag gaaggcaaaa agctgaggat agtaagagtg 60

agaggccggg cacagtggct aggctgtaat cccagcactt tgggaggcca aagcaggcag 120

atcatctgag gtcaggagtt ggagaccagc ctggccaaaa tagtgaaatc ccttctctac 180

taaaaataca aaaattagcc aggcatggtg gcatgtgcct gtagtcccag ctactcttga 240

ggctgaggca ggaaaatcac ttgaactcag gaggtggagg ttgaagtgag ccgagagcat 300

gccaccgcac tccagcctag gcaacagagg gagactccgt ctccaaaaaa acaaataaat 360

aaaaataaaa acaaaaatac aaaaaatagc caggtgtggt ggtacaagcc tgtagtccca 420

gctactcttg aggctgaggc aggagagttg cttgaacccg ggaggcagag gttgcagtaa 480

gccaagatga tgccactgca ctccagcctg ggtgacagag agagactcca tctcaaaaaa 540

aaaaaaaaag tgagaaccac actcacaata atattcataa tgagaactgg catttatggg 600

gcacggactg tgcaccaggc actgtgctga atgctttctg cgcatgattt tgtttgacac 660

tcacaacatc tgtgtgaggt ggggcctgtc actctcccag ttagatgatg acgctgaagt 720

tcagagagct ttgctggcac tgcttgagag gaagggccaa ccccggcttt gaggcctgag 780

tcctcttaac caccccactg tgttagggac agggttcccc tatgattgca gaaggtctga 840

gcagaaaaag ccctccgata tcatgtaccc aaggctctag gtcttcatgg ggaaactgaa 900

gatcagaaag gggcagtggc ctgcacaaag tcacaggtta gagtttggag gacacaggtg 960

gaacagcact tgccaggcgc tgcagtgtgg gtggtgacat cgtcagggcc cgctgggctc 1020

acagggcctc caggcaaccg gggggccttg ggtccccagt tacacactgt ggtcacatca 1080

gccaggctga cctgagggtc actgcaggac cagcctctgc tgagggctgg ctcacctgca 1140

tggagggggt tgctcccacc ccaagcatga gtctgaccca cgcaccagct cactctcccc 1200

tcaggaccac cgacttgccc agtcactgaa tggagaccac ctgctgtggt caccttgcca 1260

ggcccttctc tgtagacaca ggagaccgat cttgaggagc cagaggcaga gcccagctgg 1320

gagccggggt ttcactggtc agtcggtcag tctctgcagc tgcagcctga gatcagccaa 1380

gcccctcctg aggctcctac ctggctcact ttgccctttc tccacagggg tctgatctct 1440

ctgctctgct tttccaaact atttctcttt ctctctctcg ctctctggtt ctctctctct 1500

ctctctctct ctctctctct ctctctctca tcagatggct gactggaggc agggtcccag 1560

cccaaggatg gggttggggt ggaggtggcg aacctgggtt ggtccccact ggatgctggt 1620

cctcactctc atggcagacg gctttctttg aggccaggac tgggtgatgg tgtcgctacc 1680

cccgccgcag tctgacgtca cgctgccggg ccccaccaga ctggagggcg agcgccaagg 1740

ggacctcatg caggcaccgg gcctcccagg ctcccctgcc ccacagagtg taagtacccg 1800

gcatctgggc ctgggtttag gccaaggcct gcggctgccc aggcaacaag aagaccagtc 1860

accttaccgc ccccagggac ctcccagggc ctgtcttgtc ccctccacca gcaaacaagt 1920

cctcagggac cccgagatcc agaggggtca ctcctcatcc cttcagtctg agcctgtccc 1980

agggctgtga gccccatcag a 2001

<210> 14
<211> 2001
<212> DNA
<213> Homo sapiens

<400> 14
aggggtaaca gcagactagg gaggcctggg gtcctccaca cgcctgtgtg gggtgacttt 60

ggcccccagc aacaaggcag gcctgaggtc tgcagaggga ggcagcccac tcctcagccg 120

ggggcgtcta tcctcctcct ggccccatcc ctcacctgtg ggggaggcta agtgggtgtc 180

agggccctgt cagagggtga gccacccccg ggggtgccca tcggccacca gcctcccttc 240

agtagacacc cagcccaagt gacagcagtg gcatggcact gctgagcaca gggagcctca 300

aggtcagtgc ctggctcctg gtatcctccg tggtccccag gacaccccca cacagaggtc 360

ccagaacccc caaagctggc acaagagccc aggaacaccc tgcccagcac cctccaggtc 420

cctcttttag gatctgggtg agctaaagaa gccaaaagaa aggctgggcg cggtggctca 480

cgcttgtaat cctagcactt tgggaggccg aggtgggcgg attgcctgag ctcaggagtt 540

ggagaccagc ctgggcaaca cattgaaacc ccatctctac taaaatacaa aaaaaattag 600

ccaggtgtgg tggcgtgcac ctgtagtccc agctactcgg gagcctgagg caggggaatt 660

gcttgaaccc aggaggcaga ggttgcagtg agctgagatt gcatcactgc actccagccc 720

gggtgacaga gtgagactcc atctcaaaaa aagaaaaaaa aaaaaaaaaa aaaaaaaaaa 780

agaagccaaa agagggtctt cggcccagcc cgcccctccc caacagccct gggtgtggca 840

gggagcctga ccagttccct ccagcctgcg tcatggagcc ctgggcgtgg ctgcagggtt 900

taaagagccg acccacgtgc ccagcagcct cctcagatcc gttctctgcg ctgccagctc 960

aggtgagccc tcgccaaggt gacctcgcag gtgagcaggc gtccactttg gggctgctgg 1020

gtgaaggggg tgggtgccca agaattctga gtaatgagga gcctgttgag gggtctgagg 1080

acagatatgg ctgcagcagc cgcttgaagg cctcctgctt tgcacccact atcatatgga 1140

tggtgggttg ggggttggag gaaacagaag ccagcccttg gctaaagaat atatgagctg 1200

gtggggtata cctctggtcc caactgcttg agatactcag gtgaggagat cacctgagcc 1260

aagagagttc aaggctgcag tgagccgaga tcacgccact gctctccatc ctgggtaaca 1320

gagcaagacc ctgtctcaaa aaaaaaaaaa atagtaataa taataataat aaaataaaaa 1380

atgtgaccta gagttgagac cctcgtattc caggaactcc aggttggtga aggtagagag 1440

accagtgaag gctgggggaa gccagctttg gagactgctc ctagcccaag aaccgatgtt 1500

cagaagcagt gcctaacagt ctctccctcc agtgcctccc tcccactgca gaaggaccat 1560

gtgtttattt atggatgtat ttatttattt attttgagat ggagtctccc tctgttgccc 1620

aggctggagt gcagtgccag gatctcggct cactgcaacc tccaccttcc aggtttaagt 1680

gattctcctg cctcagcttc ccaagtagct gggaatacag gcgcctgtca ccacactcag 1740

ctaatttttg tatttttagt atagatgggg ttttgccatg ttggccaggc tgatcttgaa 1800

ctcctgacct caggtgatcc acccgcctca gcctcccaaa gtgctgggat tataggtgtg 1860

agccactgcg cccggcctat ggatgtattt attgtaacag gtttttagtg cttactgtgt 1920

gctaggcata gttcttaggc ttgacaaata gtagttcacg taattccatc gctataacct 1980

tatgaggttg ctactattct g 2001

<210> 15
<211> 2001
<212> DNA
<213> Homo sapiens

<400> 15
cagagaaaga tgatatattt taagaaagac aaggaagcag aaatgtttag tgcagaatct 60

gatctaactg gctttcaaag gctggtggtc gtggaatgtg agggaggcca ctggataaac 120

tgaggcctcc tcaatgtcag tctcctcctg gttcccttgg accagaacaa gaccagaccc 180

cagatctgca gtgggtcctc tcacggccct gggccactgc ccccttacct tgttgatgaa 240

agatgcgaaa cggttgttga ggcacttgat ctgctccttc tcatccctct ttaccctctg 300

cacagtcggg tctatctcca gtgccagtgg gaccagcagg ctctcattga tggtgacagg 360

ggtgatgcag gcagaaggcc cacaggtggc tcccagtcgg tacccgaagc caggcagggt 420

acctccacac ctggaggcta cccggggcct cccaaagccc acgttgcaca ggctccgtga 480

gccaaggcag cccagagctc ggaggcccct accacccccg ggccggcatg gccccttgct 540

cactgcatag tgggtgacca tccggggcat gacagccgag tatgagctga aactctgact 600

gccacacctg gagcctggct ggaaagagtg gtacgacatg gcaggagaga gaggcagaga 660

aatgcggccc tggaggaaag aaccggggca ggatgatcaa gtcaggctca gcttcccccc 720

ttttatctgg tagggggatg gaggactgag atgtgtcaaa ccccaaatca ccattaaact 780

aggtttatga gcttgggata ttggcagagc taagtagcaa ggtggataat tagggtagcg 840

aagagcaaag agaaggggcc aatggtcagt gctggtgggc cctataaaaa tcctatttgt 900

cctccttcct tgattgtggg ggtgatgaaa ggggccaacc agacagatat gttccatgta 960

tccagtggct cagcctcaag gaaagggaga gttgggacta cgtcatttca taggagggga 1020

ggcgtgccat gaattataca ccctggcttc tctgggatta gagggcaagt cccatatgct 1080

taaagatgct ctggctcttt ccccaaggct gctcctaggc ttttttgcag atcctcaacc 1140

caagtgccct ctgtgggctc ctaggtgagt caggaagttc ccaggaggaa ggcttttggg 1200

gtgggagaat cctccctaca acaggccgga ccttaaccct cctgagctct ggctcagtga 1260

cccactgtcc catgagggtc ttgaaggtag acagactcca ggcatagaca cttttctaca 1320

gggcccatca gtccacaaag gctgcaggga actcaagagg agaggcctgg tgcttgagaa 1380

gggcagagga agggacgctc tcctcaacac ttctggggcc cccagaaggc atgatttcag 1440

catcaggtct cggcttccat gtggctgttc ctttcccctt ttgcccaacc cagcctgagt 1500

gtccctgagg tcaacagagc cagctttggc ctgaggatgt ctctggggag agttgttgcc 1560

cttgggaatt ttcaggtgga ggtgggattc aggttttctt tccagctcag gtcgcagaca 1620

cagctctttg gagatgtctg aagcccccgc ctcactctct ttcctcctct tttccatcaa 1680

tttcatcagt gaaacttttg ccatggccga atggcctttc ccttccacac tgagacttat 1740

caccaggtca aaatcaaacc aataataata ataataacaa taataaacat ttattgggca 1800

tttattgtgt gttgggacca ctccgtactc tttacatcca tggtctcatt taattctctg 1860

accacacaat gagatagtta ctatgccaca gacaggaaaa tgggaagcac agcagttaag 1920

agcaacatcc ccaagtcaca cagcttgtgg ctgatcagct gggagtccat cctgttcctt 1980

ttgacttcac cctgccatgc t 2001

<210> 16
<211> 2001
<212> DNA
<213> Homo sapiens

<400> 16
acgtgaatac acacaaacac acaaacagct tggctgctct gggtgaagca tgtgtgtgtt 60

tgggggaaat ttccttcccc acccgtggtc gtccatttgc ccatgagcag cttccgaggc 120

aggtccgtgg accccaacgc tgtgtaacac cgtgtgaaaa acacaaaggc ttgcagccct 180

ctcactgcag acgtgggaac acgactctgt ttaaaaccct tcaaagcccg ccttcttgcc 240

gtgtgatgct gcctgggcca gcagggcagg tcaccacgct gtctcttcaa agcagctcgc 300

tcatgcccac agcgctgggc acaagggcag ccacgagctg tttgatttct gcgtccccag 360

cagatggctt ggtgcacagg ggcagggctc gtctgtccct ctcactgctg cgtcccaggc 420

tatgtctcca ccatgtaact tcagacaagt tcctcagcgt ctctgtgtcc tggttccctc 480

atttgtaaca tcaggatatt gataaggatc atagtaatag ggatgtcctt atggggcctc 540

atgaaaatga agagttctct gtgatgtgcc tggaaccaca gagggcagca agtgggagcc 600

acagaaagtc atctctttat catagttgtc attggtaact atgacggtta tcaagggcag 660

gggtctccca ccttctgcat ccaggccttg tcattcctta taaaattttc tcctttcaag 720

gactttcaat gccctgaaaa cggaatgctc ctccctgctc cccatcagct gctgcagagc 780

cctgaacccc ttgaacttgc cttgcttagt tctttcaggg gctgccttgg atgccacctc 840

acttccctct tcttggctct gtggctgtca gaggggctgg gcactgggtt acttcatcag 900

ttcctgggtg ttgatcggca ggaaacagtg gtttcctgtt catatcctgc ctgagcctgg 960

gccgggtgga ggtgtcctgc atggagtctt gtgaaatcgc gtggactgca acacccagca 1020

gcgcggtgag tccaccctga aatccacagt gacccacgcg gcatgtgctg ggaattgaac 1080

gcttggggaa agagctgggc tccagcggat cagccccgac caagtcgttc ccacagctca 1140

ggaccggacg ctacaagccc actttgtagc agtttggcgg acggcaaact tttgcaacac 1200

agtgcaaaaa ccatttaaca cctttaaggg aattggaaat gggtttttca tgtgtggtgg 1260

tgctgaaaat tgagaataat gacagaatta ttaaaaacag ccttctcggc cttagactct 1320

taattgcctc tggtctgtaa aactgacaaa taagcactgt agaagccaca gatgaaatat 1380

gtcgtcttgg gagccctgga gaattgtctc tgaattcttt tgtttatgga ttgtagaaac 1440

tgctttgggg gtagttggat gtagaagcag aaggacagag tggggagctg ggagcaggag 1500

gcctgggttt gtctcctgag tctgtttcca ctggggcctg aggctcccgt gtgagggaac 1560

atgaatgggc caggcacatg ggccctgtgg ggtgacagag ctgtgggctc catggtggct 1620

ctgtgggcag ctagcttggg gatggctgag ataaagacaa gtagttttcc ttcctgcagg 1680

acttctcaga gcctttagtg tggttataat gatagaaaac agtattaagt ccataccctg 1740

aaccagaaac aatgctaaaa gctttgcact tggccttttt ttttcttttt cttttatcaa 1800

catgtgtgag gacggaagct taggcttaca gatatggact cattgagagt gagtggacgt 1860

gtctgatctt taacccagtt ctgtctgacc aaaaagcctg tgctctaacc actgtgcacc 1920

cctgcctgtc tgtcctgttg agagaactgt agggaaagcc ttgagattat gtatccagtg 1980

ctcccctcct cctgattgga a 2001

<210> 17
<211> 2001
<212> DNA
<213> Homo sapiens

<400> 17
tgaccttgct caggtccatc tccgggtgtc agtttcttca tctacaatgc aagagggttg 60

cccacctctg agaacccttc taaccccaaa tctcacccta tgaatctaag aacaccaacc 120

cctcgccatc ctaagtatca cagagccagg caagcatggg tgagagctca gaccatcctt 180

gttggactaa aaggaagggg cagactgccc atggggggca gccgagaggg tcaggccccc 240

ataggtcctc agcctgcttc aacctcaaag gggatggggg gctgagtggt gccagaggag 300

cagcaggctc gctcggggag agtagggcct taggatagaa gggaaatgaa ctaaacaacc 360

agcttcctcc caaaccagtt tcaggccagg gctgggaatt tcacaaaaaa gcagaaggcg 420

ctctgtgaac atttcctgcc ccgccccagc ccccttcctg gcagcattac cacactgctc 480

acctgtgaag caatcttccg gagacagggc caaagggcca agtgccccag tcaggagctg 540

cctataaatg ccgagcctgc acagctctgg caaacactct gtgtggctcc tcggctttgg 600

taagtgagct gccagcttcc ccaggcagaa gcctgcctgc cgattccttc tttccttccc 660

tgacccaact tccttccaaa tcctcctcct agaagccctc cttggttggc cctgcctact 720

ttaaagcttc tttcacattt tcttaggtca tgttcccctg gggcctcctg ccctcaaatg 780

ctttgctttt tggcactctg tagatattct aaaaaatcat tttgtacatg tgtgtgacag 840

gccatctccc agttaagttg cagcctgtgc tttcttttta ttttgcactt cccccactat 900

ttctgtgagt gcttagtagg aagtgtcaaa gaagcttgac agcattttct tctaagtgtc 960

ccaactcttg gttttccatt acacagacag agtgcaagac gatgacttgc aaaatgtcgc 1020

agctggaacg caacatagag accatcatca acaccttcca ccaatactct gtgaagctgg 1080

ggcacccaga caccctgaac cagggggaat tcaaagagct ggtgcgaaaa gatctgcaaa 1140

attttctcaa ggtagggctg gactctggca ggtctgaccc agcctcaccg cagtttgggt 1200

tgacaaggga ggatgggagt atgggctaca gcaatcaagg ggaagatttg agctcctgga 1260

gcccagcccc aagacgcagc gagtgtcctg ttatacaggg caggtgctca cagttacaca 1320

ggacgacagg gtcaagaaat tgctcaattg aacacctgct atttgtcggg ccctgttctg 1380

ggcagaggga tgtagtggta aatggggagc ccactattcc agtggaggga gacacacagt 1440

aaagttgttg gccaataaag agcacagata aagccaaatg ccaataagtg cctggaagaa 1500

aatgagatag agtgcgctgt gggcaatggg gctgggtggg gtggaggtga ccagttaggg 1560

tacatgagaa gggcctcttt gaggaggtaa catttgagct gagccccgaa tgttggggag 1620

ggaagcccct gaggatgaca cttggcacaa agctgaggag accctaagcc tcaggcggga 1680

acttggggtg gaagacttgg gggcttttct aatcctaagg gtctgcggtg gaaaatgaat 1740

gcataaagag cacatggaga gcacctgcac agcactcagg gaactgggag gtttttcccc 1800

cgctccaaaa atgattaggc agttctaaga aaaaggctga gcacttccaa cagccttttt 1860

gttttctttt caaatttggg gaaagtcggg aaacagaggc ctgcattaag aagggtggaa 1920

cacatgggtc tcagtctcag ttccagtccc ggagccagac atcctggggt aggtccccag 1980

ccctcccagt gcccctccct c 2001

<210> 18
<211> 2001
<212> DNA
<213> Homo sapiens

<400> 18
tccccttttg ctattatttt taagagcctt gctgctctcc aggcaaattc tcaggttctc 60

tgcccgtctg cagcatgtat gcatatgcgt gtgtgtgtgc gtgcgtgtgt gcgcgtgcgt 120

gcgtgggcgt gtgcgcgtgt gtgggcgtgc gcgtgtgcgt gtgcgcgtgt gtgcgtgtgc 180

gcgtacgcgt gtgtgcgtgt atgtgtgtgt tgggggaggc tgcggtgcct atgactggtt 240

gcccccttga accccaccag aagccagccc aggcccctct gcgccctctt ctgagtctcc 300

agggccggtt ctctcctggc aactccagaa agtgcctcct cacctcaaaa gaccaagaaa 360

cagggtttct gataaagtgt cccacccagt cccggctggg gcagttggtt agagaatgag 420

gggacattgg ggggaagagg agggagaatg aggggatggg aagagaggcg cagagaaaat 480

gagaggtgga gagaaaggga cagagtgaaa gagaaggaga aaggggagaa gccgggaaga 540

caatgagaat ggaagcgaga gatggaagtg gaaagaagaa ctggaagcct agagacagag 600

ggagagaggg agagagggag ggggagagag agagagagag agagagaacg ggaatgagca 660

gctgtgatgt ggccagatca gttcatagaa aagaaacaca gttcagagca gaagggagga 720

aagaaaataa gaaggaaaga aacagagaat gagatgcaga gaggagagga gggagagaga 780

gaaggaaacc gaggtgggta gaaaaatgtg acgaaaggga aggacagaaa aaaaaatgag 840

agaagtaaga cagaataaag gagaaaaaga agaagggaat catgaggatg gtgagaaaag 900

acaggagaga aagccgggag agaggataga ggggcgttct cgctgcgctg cgtgtcagct 960

ctggtaagcc gtctagcatg tcattagtgg ggacctgagg cgccagcggg aaagtcccct 1020

ccccgtgggc tggaggctga gggagggcgg gccctgggct gggatccaca ggcaggtggc 1080

cgcccctggg tggggccgcc cggccaagcc ctaggggtat aagtgggagg ccgggcctgc 1140

tgagcacctg tcaggggcag ctccggggtg caggtgccag gcaggtggct ccggccagcc 1200

cagccccagc atgagctcct tcgacctgcc ggcgccctcc ccacctcgct gcagccccca 1260

gttccccagc atcggccagg agccccccga gatgaacctc tactatgaga acttcttcca 1320

cccacagggc gtgcccagcc ctcagcggcc ctccttcgag gggggcggcg agtatggggc 1380

cacccccaac ccctacctct ggttcaacgg gcccaccatg accccgccac cctacctgcc 1440

cggccccaac gccagcccct tcctgcccca ggcctatgga gtgcagaggc cgctgctgcc 1500

cagcgtgtcg gggcttgggg ggagcgacct gggctggctg cccatcccct cgcaggagga 1560

gctgatgaag ctggtgcggc caccctattc ctactcggct ctcatcgcca tggccatcca 1620

cggggcaccc gacaagcgcc tcactctcag ccagatctac cagtacgtgg ccgacaactt 1680

ccccttctac aacaagagca aggccggctg gcagaactcc atccgccaca acctgtcgct 1740

caacgactgc ttcaagaagg tgccccgcga cgaggacgac ccgggtaagg aggctttgag 1800

tgtggggggt gtccccaagg aagactcact tccttcctcc ccaagacccc attctagaaa 1860

gttctgacta gggctgtgcc ccccaagact gggggatgct accaggcagg acagagggga 1920

gctcagcagt gaaggagagc atgggcctgg gggaagattt gggtttcagc cctggctgtg 1980

ctcctgccca gtcagtgacc t 2001

<210> 19
<211> 2001
<212> DNA
<213> Homo sapiens

<400> 19
gtaagattca ggggctgcta tggatgcatt acccaggaga taagggcaac ctcaggtggg 60

ccggggccct cacagcaacc ttacgagagg ggttgtatca tgcccatttg atggaagaga 120

aaactaagat tccattagaa ttgatgtgac gtatcctaaa tcacacagct gcagcaggac 180

taggatttga atgcgggtca atctgcctcc aacactactg ccttttcctc catgctggat 240

taggctattg gcttctgaaa gcacagacag gtgagatggg tcaaacatgg cctgaagcac 300

ccaggcacag gtgggagatg gcaagcagag gcaggttcaa aacgtggggc caggactggt 360

ctgaaagata gtcgttaaat agcactgagg agagggagtg agagagatgt gggtcttggg 420

ggagtggggt cttgagaacc ctgaaatcag cagtgtctgc ccactttgcc ctggaccagc 480

ctctctgtct gtatccttcc cccagagaaa ggaccatggg ctgacctggg ccaaggacac 540

ctggctggag ccccttctct gacattaact agctccgtga cctggggaaa gccaccctcc 600

ctcctgagcc tcacttttcc tgtctgtaaa atggcctccc cgtcatcaca cccacacagg 660

tgtcttgagg agtgaatgat ggtgtgctcc aaggccccca tggagagccc ggcacatggc 720

aggcactcgg tgttcaagca ctgtgttccc atagactctg ggctcctgcc ctcctcgccc 780

ccaaccccct tcctaggaac cagccccggc ttgtcagaga gccagctagc tgtcaacccc 840

accccagctg gccagttaat gagtgaccag gactcctgag cctgacctat aaggaggtgc 900

taggcaggga cacagatggg agacggtgga cagcggcagg gggaacccag ggagcgcgat 960

gggctgcagg gctgcatcag ggctcctgcc aggagtggcc gtggtcctcc tgctgctgct 1020

gcagagcaca cagtcagtct acatccaggt gagtcccttg gccagcgttc cctttgcctg 1080

aagggcccca tggtgggagg ctaggctgga gagggtgtac tgggaattca gaggggcacc 1140

gggggagcac ggggcccggg gctcagccca acacccatca cagcccagag accaaggcag 1200

ctggtgggtg ggggcagcca gatgttgatg gcagtgatga tggcaatgac aacaccaaac 1260

tgattcagcg ctgctcccct caaaggcccg tgctggtcac agaggtcagg gtgtggaaca 1320

gaaggctcct ccttgcatgg gaggaggcca gaaagggaag gagacaccct gttatgctcc 1380

cacagttccg tatattcacc cacatgtagg tggaggagga caggctccag aaaaattttc 1440

ctgagaagta acccttgagc tggcattgca agatgaacag gaggttgcca gacacagatg 1500

atggggaaac tgttccaggc caaggggcac agcatgtgca aagaccagat agaagactat 1560

gggggttcca gtgcccttcc agggggcatg ggagagtggc agtagtcgtc acagcagggc 1620

ctgtctgcca cgtcacaggg gatgctgctg gcctcgccca gcatagcaga gacgtctgtg 1680

gccctgggtg tcacatccct gactctgggc atggtctcct tgggaagaac aaagtgggtc 1740

ccagagaggg gtgcaggaga cttggcctcc ccaacagccc tggcctcaga gaccagctac 1800

ttggggctgc catcactctg ccatgtgagc tcaggcaggc gtactcagtt agtgattgtg 1860

ccctggtacc aggcccccac gggagatgtc agatgaggga actgaggctg agtggggaac 1920

agacaggcag ccggtcatgg gcagaggtgg gatttgaacc agggcttgtc tgtctctaaa 1980

gcctcgaacc actgtgctcc a 2001

<210> 20
<211> 2001
<212> DNA
<213> Homo sapiens

<400> 20
   ctatgctaca aaggcagtag gccaaatttg attgaattga ataattcctt attttcatca 60
   gcttctcctt tttttttttt tttttttttt tttgagatgg agtattgctg tgtcacccag 120
   gctggagtgc agtggtgtaa tcttggctca ctgcagcctc cacctcccag gttcaagtga 180
   ttctcttgcc tcagcctccc gagtagctgg gattaaaagt acccaccatc acgcccggtt 240
   aatttttgtg tttttagtac agatggggtt ttgccatgtg ggccaggatg gtctcgaact 300
   cttgacctca attgatctgc ccccctcagc ttcccaacgt gctgggatta caggtgtgag 360
   ccaccgcacc cagccagcct ctcagttttg aacatgcact accaccacct ccacaacaca 420
   caaatgtaaa tgcactttcg tatataaaac tgtataaata caaggaagct catacacatg 480
   caaggataca cacataagca cccccagatt caaccacaga aatatacgcc agtacatttg 540
   cataaattca aacacccctt tacatgtaaa aatcatataa gcacatacag ggatgcaagc 600
   aggcatggac aaatgcatgc aagcacagac aaacagacaa agctaagtaa aaaagtgcaa 660
   gctcacctat gcttacaaaa atagacatac atatacccac aaacccacac acccacacat 720
   tcacttgctc acctggactt tgatatctct accactgtat ccctgccaat atctacagag 780
   tgggtaaagg gataggcatc aggtcactgg gttgcccaag caggaagtct gggttcccta 840
   acaacttttt ctaagctaat gctcctggat gatgatgaaa aaggaggtgg ggaatggatg 900
   aaattttata acagggtgca gaggcagggt caggataaaa ggcccagttg gaggctgcag 960
   cagggtgcag ggcagtcaga ccaggaccat ggaactcagc gtcctcctct tccttgcact 1020
   cctcacagga ctcttgctac tcctggttca gcgccaccct aacacccatg accgcctccc 1080
   accagggccc cgccctctgc cccttttggg aaaccttctg cagatggata gaagaggcct 1140
   actcaaatcc tttctgaggg taagacacag acgaatgggg tctgagggtg agctgcttct 1200
   tgccttggta cttggggaag cttcaccaaa cagaatgagg cagacttcca gagtcagggg 1260
   tggcacgggc atggttggtg agtacggagc atggtgaagc atgatgggtg gtattattag 1320
   gagaaaagca tcaaattaaa tttagcagag tttatttgag caaagaagtg actcatgagt 1380
   tggacagctc cctaaaccag gaaagacacc acacggcagt atggtcaagt ggtatttaca 1440
   ggcagaaaaa ggaggtgaca tacagaaaca gcctgattgg ccacagatca cagcttgcct 1500
   tacttggtca caatctgagc agtttgcagc ctgtgtggac tgaaagccca gctgctctga 1560
   ttagccaaca cttggctact tgtcacaaga atatattcat ttgggccagg tgcagtggct 1620
   catgcctgta atcccagtgc ttttggaggc cgaggtggtg gatcacctga ggtcaggagt 1680
   tcgagaccag cctggccaac atggtgaaac cttgtctcta ctaaaaatac aaatattagc 1740
   tgggcatagt gatgcgtgcc tgtaatctca gctacccagg aggctgagga aggagaatca 1800
   cttgaatcca ggaggcagag gttgcagtga gccaaaatct taccactgca ctccatcctg 1860
   ggtgacagag tgagactcct tctcaaaaaa aaaaaaaaaa aaaagaatat actcccaagt 1920
   taggttgcag ttcactctac agagagagct ttaggtcaaa tttaatttaa ttaaacaatt 1980
   ctcccctttt ggtcagcctc a 2001

<210> 21
<211> 2001
<212> DNA

<213>  Homo sapiens

<400>  21
gtggcgtgca cctgtaatcc caactactca ggaggctgag gcaggagaat cacttgaacc      60

cgggaaatgg aggttgcagt gagccgagat cgtgccacag tactacagcc taggcgacag     120

agtgagactc catctcaaaa taaataaata aataaataaa taagtctcat gaccttgagt     180

ggggtctcgt acccctatag atttcagtct tctcatctgc aaatcgagga agacagaatc     240

atccacctct catttgctgc caggcctgag ggtgtatgat gagtccactc agatccacac     300

attttagtgt gttccctccc taccagtatt tgcattttca ctgaagacaa agtcttaagg     360

tgaagcaata aacatcaaat tggggaatgg tcagcctctt cccacaaaag ggcaaggtat     420

aaaaagaggg gatgaggagg ctgtcctctt cacctgctaa ctccatgtat gagtggtggt     480

cacaagagag gaagcgggca gcagaaatcc tcagctccca cagaaatgaa tacattttcc     540

aaaataaagt caagccaagc agccctaccc cacagaaagt cctagcaaac agaggtggct     600

tcctacctga agcaccggcc aggtgtgtcc aacagtagga gctgcagtca gagatcagag     660

tgacttaaca gctagagggc acttgatgaa taaacggaaa taaggaaacc acatcagatg     720

acacctccct tctgagcccc acccatgtct acatttgggg aagaacagtt taagtcaagg     780

gatcacagac ttgtgattag agactgccag ggtccatatg accaagtggg ggtcccaggt     840

gtgaagctgg ggttgagggt ccattatctg aattctccac tctatggatg atcattttta     900

ttattttcct tttcttgaat ttatttccat ttgtttattc ctaaattccc tagtatatca     960

cctgtggaag cttgcaactt cctgaccaag tataaagggc gaaggatttg acttacagca    1020

gagacttcag aaggagtccc ccataggagg gacttggggg caatccagtg ggaaagagga    1080

ggaggactgc gcttgagttg tgtttgaaca gcaggcactc aatctctact tattttgcag    1140

gcttctttga ggtggcctag ctcggattca aggcattctc atcaaggatg tgagagcagt    1200

tactgggata tccttggctt ccctgttggt ttgccaacga gagctcccct tcctcctttg    1260

cctccaacct ggtcttcttt ggaggttggc ttcccagtgt tctccacctt ccacaactcc    1320

aaggcctggc ctcaatctct acttattttt caggcttttt gagggtattt gaatggaaca    1380

caaaccctcc tttgccgcca gccactcttt cgcacaatca ctgccaatga ccagttggtt    1440

taaatggccc tgaggctgac ggcataggcc actcccagag tctggctggt tagcagagag    1500

accacagagc tggggcagtc catgaatgga agagctgtgg tcaaggtcac tgcagccccc    1560

atctcagtaa cggagaatgg cttctctggg aacccacctt agctgcctcc agcagctaat    1620

ggctgtcccc tagcttgagt cttctggcac cttcgtcact tctccatata acatgaaccc    1680

tgctgtgagg catttacaag tcagccctct tctagcttgg gagccatgag acagcaagaa    1740

ccttcttcaa ttcatgttta tgttctcttt cagggaatat agtaggtgct caatcaatgg    1800

ttgtggaata aatgaaggga gacatttctc ctgtgattca tggaccataa gtcaccacca    1860

cgattcagtg tctattgctt ttggctacac actaaagtca ctggtatgcc tgatagcttc    1920

acttccacac agcccagttg gtctgcctgg cattgcttct ttccaacctt cctctaatgt    1980

ttggagctaa tgccagtctg t 2001

<210> 22
<211> 2001
<212> DNA
<213> Homo sapiens

<400> 22
tccctcagaa actgaagcat gttgcccagc cagttgctga tgccctcctt ctcctggctg 60

tggctcacgg agagcatctt gtgaaagaga cgctggagtc ggcccacttc tagccactcc 120

cggggccaca gcagggagta gacaaacctt gggaaaagaa ggtcaaactt gcggaactcc 180

atgaataact ctcctgcctg tagcaggtcc tgctccttgt ccttcgtgta gccgaacaag 240

ctcaggtagc cagctgtgaa caagatgtaa tagcagaagc gaaagaggct gtcctcatgc 300

cagcaactgg catccagact ccagcctttg gacgtcagca ttacaaagga caggctgtcc 360

agcatggtct cattaagatc cttcaagcca tcccccctca gatgcttggt gctggctgag 420

tgtatcatct catggtcccc ttgcactgaa cggtatccaa ataccttcag caccagtttt 480

tttgcatatt gcccaaagtc tagttttctc tgtgtgtcct tgaggatgga gccaaaggag 540

agggggtcca tgacgaaggt gaagtactgg ccccctagct gcactgtgaa cacatcccca 600

tgcttggtcc tcatgcgctt cagaaattca aacatattct tccggaaagc catggcatgg 660

ccaagccagg gcacggtacc cttgtccaga gggggctccc atggcctgcg ttgtcggagc 720

atccctggca ggcacaggta tccagcaatg accaccagca gagctcccag cactggaccc 780

cagagaacca tggctatgct cctgcggatt aagctctcga cacgcacact gttccctggg 840

tgccagagag ctttgggagg ccttggaaag tccaggatgt aaaggtctga gcacctggac 900

cttgccctgt ggccgcaagt tatggcaaat aacactagtg agacagtact tggaagcagc 960

aaaatgttcc cctggactta acacttggct gactggtcac gtaggggagg aggaggtaac 1020

caggagaaag ctcagagaga agcttccagc tatggtcaca gctgctcacc agcttcatgc 1080

tttgctctga ccttttcaga cagctcttgc accttctctc taagcctgcc ttgagcccac 1140

tcccaccccc atagctgaca gactatttat acccccattt actcatgctt ttatttattt 1200

gccagttcag ttggtagaaa tgaatcaagc acttacaaaa tctcacttcc actctttcct 1260

catctttaga agactcaaca aatgcctcca ttcctacttg gcccccttgg tcaagtaagt 1320

taacctcact gtggctccac tccctcatct gtgaagtagg gataattaca gtgccttgta 1380

ggtgctgttg tgaggcaaaa agagataaca cctgaaaagc acttagaata gtgcctggct 1440

catgctagtg ctccatatat gttagttagt atcctaaagc cttttctctt acctttgcct 1500

gctcccctgg acctcacagc agcccgtgct tatctcctca ttctttccct agtctgtctt 1560

cctctctggt ctcaaagatg cttgccaagg gggcatgacc ttgttgaagt catgtctgct 1620

cctacctcca tgtgcacaga gtacacagtc aaaggcaagt gaagaatgac tgtatgccct 1680

tccacaacag ctctgacccc taacccaaaa aaagaggcat tgatgctggg gctttagatg 1740

ctgagaaggc tgagatggtg gagggggtgt tgactccatc ttggggtatg agtgtgaatg 1800

aactcatctg aggaggccac agagcctccc agatgatgac tcttcaagca gaggctgagc 1860

cctcagtctc tgagcctctc acccggagcc ctctcacctg cccaactgct tgcctataca 1920

cttctctgac aagaaggcag aatcctggaa ttgtaaaatg tcagagacaa tagagacctc 1980

actcaatcac ttcatacttc a 2001

<210> 23
<211> 2001
<212> DNA
<213> Homo sapiens

<400> 23
agcctcccaa agtgctggga ttacaggcat gagccaccgc gcctggccta tatacatgtt 60

taaaaaataa agacagggcc agcacggtgg ctcatgcctg taatcccagc actttgggag 120

gctgaggcag gcagatcacc tgtggtcagg agttcaagac tagcctggcc aacacggtga 180

aaccctgtct ctactaaaaa tacaaaaatt agcctggagt ggtggtgcac acctgtagtc 240

ccagctactc aggaggctaa ggcaggagga tcccttgaac ccagaaggtg gaggttgcag 300

agagccgaga tcacaccact atgctccagc ctgggggaca gagagagatt ccctctcaaa 360

caaaacaaaa aaaataaaaa taaaaataaa taaataaata aagtatgtag agttttattt 420

aataattaac cacacagatt aaaaaataga aagtagccag cttcccagaa gtcttctgtg 480

tgcaccttct aattaccatc tctgtcatgt gtcttcagac agaagcacat ataaaaacaa 540

ggttatgtat tgatcaaatg atgaaaatgt gtggtcagag gcttgcattt tccctaggag 600

caatggctca gtatttgcta atccggtgtt cacagtgact ttacagaaca taactacccc 660

caagtaatgg gaatcaactg tatttttatt actttggtgt acaaagaact ttgagctctc 720

catggcttgc cacaacaaaa gacttcctag gcaaattgtc tactgctttg ttccaagtgg 780

gggctctact tgaggctgtg ggaccaagac aaaagaagca gcctctatgt ggattaggcc 840

ccttctcctg gcagagagaa agcacaaagg agctggcaga aactcacagt gcaaaactca 900

ctatgcatat gctacatctg ctcatactcc aataggcaaa gcacagctga cagcaaaccc 960

aaagtcaatg agacagaaat gtgtcttcct tccacgggcc gttctaaaat tgcaaggtaa 1020

tggacaggag tgtcctctta aaggaaagat ggaatgaata gttgtgaata ataatataac 1080

ccatcactac tactcctgtt ttaatttttt gaaaatgttt agatacatat tatatgcttg 1140

tttaatttaa aatatcaaac tttaccttgc tcaggctctg cccctccaca ctgggccact 1200

caaagatgcc caacttaccc tacttgggct ccaacagcca tatcaggttg cattcgagac 1260

cagcctggcc aacatggtga aaccccgtct ctactaaaaa tgcaaaactt agccgagcgt 1320

ggtggcgcac acctgtagtc ccagctacta gggaggctaa ggcaggagaa tcgctggatg 1380

cattgatgcc ctcttcaccc tgcttaggct ctgattcctg acacaagaca gctctgttct 1440

gtggaacccc tcctcactat gcttggccca actccccaca gccgaccact ctaacttcta 1500

tgtatcctcc tcatctcctt gggctcagac cctccatgtc tggctaccca cccccataga 1560

caccctcctc accctccttc agttcggaca gtccacatca ggcctcttcc ctatggagac 1620

agcttcctca ctttgctcca gctctaaggc tcctccctca tctgctcccc tgcctggaca 1680

ctctgctcac cccactcagg ctctcattct atgccagtct gcccctctag ccccaggggg 1740

atgcctttct caacatgggc aggctggtct ccatgctatg ctgcttcttg cctcttcact 1800

gggcattctc atctacccct ggctccagca caccaggcca gcagccctcc tacagagaca 1860

ccctccttga cctgttccag ctctgatgcc cacacggagc cagccgtggc tcccttcccc 1920

ttggggcaaa cgcctacctt gctaaatgtg cctatctaat ggctttagga ctgactagtt 1980

cagttagtaa agaaaaggag a 2001

<210> 24
<211> 2001
<212> DNA
<213> Homo sapiens

<400> 24
tgcaggtggc cagccaccca gtgctgacta ggtgggaggc ttccaactac ctggctatct 60

aaatgcccgc tgcttcacca gctgctggct accttcctgc ttagggcagt gtgactcact 120

gacggattga caaggaggcc tggcaggacc catcacccag ttccatccct ctaatgctgt 180

gtccccaggc tcactcctct ctacccagtt ccccgacctg cttacttggg tttggggtgt 240

caacaggatc caggcatttg atgccacaag tgtcaggaca acatctcttc ttccctggac 300

actgccagtc actctggcac tcaggtttct tgtatctaag gcactgggca gatttcttag 360

gaggacagac tccagctttg aaggcttttg aagagaaagt caagaggtca ggaggaggct 420

gggtactgag gacccatcat gcctcctgaa caaagcacca cttttgaggg ggagagaccc 480

ttacctcagt agctgtccac aaagcccaaa gggtcatgcc tgcctgatct ccaggtgtat 540

tgatgggctt gattaaatta gacaaatgga caggggacgt ttcacaccca agaaaagggc 600

agcaggcttc tagtcaagac ctcccaccca agagcaaaac aaggctacac aaaggcagag 660

aggaaggctg ggagtgatgg agatggagag aggtaaacaa agataaatag aaccagtgtg 720

agagaagttg agagctaaag cccaaggcag aagcacagag acaaaatagc agaagcaaag 780

acacaaggaa gcagagtcag aaactaatga tttgagagtc gcagacaggt gaagaagaag 840

gcggagtatg agagagagag agagagagag agagagagag agagagtggg acctgggctg 900

acagcatggc tgcatagagg aagcatttcc agaagctcaa actgtcacac aaaccttgga 960

gctcagccag acatgtattc caccctcagg gaggtctccc gaaactaagc tgagggatca 1020

gagcctacag aaggggacac acccacacca caaggagacc ccacctctga ccctgcagcc 1080

cagattagac cagagtgact ccaacttaca ctttccagag ccttccacag cccaaggtgc 1140

cagagttccc agggcaagca gcaccaggaa ggggaagagg ccgctggact tcatggtgaa 1200

ggcaggagtg actctgatgg ccaatgccaa acctcactat ttattctttc accagtgggt 1260

gtggccccac cagaattctt tcggcctctc ccagctacta ttgcataagc aggaaacgta 1320

gccagagacc agaaatcctg acaccaagga gattgtttat ctttcctatg ccaggtaagg 1380

ctatgaggcc aacacagcaa ctatcagaaa agaaagggaa gagattggcc tgggaagttg 1440

gttgattaaa gaggccatct cagtgtccat ctgagcccta aacatcacaa gatcccaggc 1500

cattagcctt ataggtggag agagggaaga agtggtcaat tgcaaaggtg ctacccggac 1560

ctggaactaa aaccagacac agactccctg ctgagacttt gtgcaataca ttctattctc 1620

acattgctaa ttctgattct cacattactg gactctcagg cattgtgaaa ctctgctgat 1680

tactcaatgc agtttctttc tcaaaagttc agctcgttca accaactgat acttagttcc 1740

ctgaaatatt tacaccgtta tatccccagt gcctgttctg tgcctgacac aggaaaagca 1800

ctcacaaatg aaagctgaag aagagctccg ctctccacaa atctctctgg agctcagttg 1860

tctcagctgt gaaatgaaat gatactaaca agtcagccct tctcagaggg ttatgaaatt 1920

aagggcatga agggactctg aatgaaaaga acaaatgaat agtatccctt cccactaaag 1980

agaattgaaa tgaatctgag a 2001

<210> 25
<211> 2001
<212> DNA
<213> Homo sapiens

<400> 25
gagatctggg aaatagttta atcagacact agcatttatt cttagctgct ttcttaatta 60

aataaaaatg agtataaatt aatttactaa atgttcacaa cgtaaaaacg gacattaaaa 120

aaagaaaaaa aatgccaagg taagctgata atgtttttaa aaagtagaca ataaaataat 180

gtatgtaact taaaatttgc catgataatt tattcttaaa atggaaaatc agtaaaatac 240

tcagacttct agcataatat ctcttttttt ttatttatta ttatttaagt tttaaggaca 300

tggatgaaat tggaaatcat cattctcagt aaactatcgc aagaacaaaa aaccaaacac 360

cgcatattct cactcatagg tgggaattga acaatgagaa cacatggaca caggaagggg 420

aacatcacac tctggggact gttgtggggt ggggggaggg gggagggata gcattgggag 480

atatacctaa tgctagatga cgagttagtg ggtgcagcgc accagcatgg cacatgaata 540

catttttaaa agtgtgttac tactgtcatt tcaactgact agaaatacca ccagcacgtg 600

attgccacct caaaggactg agaatgtaat agtagtctat gaagagcagt gtgtgtgatg 660

gacaatgatg gggtagctcc agtgactgat ggcttcaggt gccactgacg ggtaattgtt 720

gatgtattca actgattttc cgtgagcacc cactttgtgc cagacagtaa ggactcggag 780

ataaaaagca gccctctgct ggatacgttt acaggctagt ggaggtgaca ggcaccgtca 840

gagaagattg taacaagata tgtatggcat agtaagagag aagagatcat ctaattctgc 900

cactgagtat ttaagggcag gtttcacaga agagtttaca tctgaattga atcttaaagg 960

atgacaggga gttcactgga tagacaaagt agaggaggac gtaagaggca aaggacacat 1020

catgtgtaaa ggcatgaaag ctgagccagc ctattatctt ggaaaaacta catgtactgt 1080

agttactaca actgtcacaa aggctattag tggagcagca gaggaaggtt aaataacaga 1140

gggaagttgg atcatgaaga accttatcca tactacaagg tgattttatc cagtaggcat 1200

tgaaaaatgt aatcgaacct gtgttttaat ggaatattga ctaggatgag gggaaaagac 1260

agaggataac aaaacactta gtagacaaaa gcaacatttg tttctatctc ctactacttc 1320

agtcataatt ataataataa tagcccacat tattgatact gacatgtaga taggtgaatc 1380

caatataaaa tgataggagg tatgaaagaa gttggtgagt ttggaatatt atttgactgg 1440

ctataatata gagtaatggg gtgggagcca gagggaaatc gaaaaggtga cctggaaaac 1500

attgttcagg gccttgaatg ctatgttgag gtattttgac tttataaaat agtgggggac 1560

aaactacaag tattttatac attgaggtat agtttatgaa gtttttttat atgtatagct 1620

tatgtaaatc tcaaaggaat gctgagaagt aggcgttatt atcccattgt acaggtgaga 1680

aagctgagtt tgtggaatat gcatacattc tacattattg agcacctgct atttgccaag 1740

cactatgctg agctatgaga ctagaaggaa gttgtagttt aatactctaa tagaggaaac 1800

aggaataaat cagcaattgt aagtagttgc tataataaag gcaaacaatg ttacgggagt 1860

gaaaggggga cttgtctata tttgcatagt cagggaaggc ttcctggagg aggtgagctt 1920

aaactgagtt tggagaaaca aatagattac agtaattgcc cagagatatt taactaggaa 1980

gcagtttacc tgaaacttga a 2001

<210> 26
<211> 2001
<212> DNA
<213> Homo sapiens

<400> 26
aataggtgtc agccagtttg cagcaacctg tcttgcccct accattcttg ggttctgtat 60

tcagacccca gctattccta acacccagtt catactccac gttctgtaca aagccatccc 120

tagctatccc agtcagccca cactggtctc catgacatgc atgaaccgaa gtttttagaa 180

cctttgacac ttatgtctgc cctggatgat ttgttcatta tcttatgttg ttgtcatcca 240

cagaacaatc tggagaatat tcatatattt agtcagcaaa tagctattta ttgggggcct 300

ttgtaggcct caatgttgag aagaagggtt ggttgttttg gacagattgt gtatttataa 360

agctctagca atttttaaat ctctagcagg aaccaaagcc ttcttgacag ccacctctct 420

gaattattgt cactagcctc cttaggagaa gagacaatat agtttatacc aagatttgca 480

gttgtctaga tattactcac ttcagctagc actgtgctag tagataacat tcaaagttgg 540

tctcaacaaa tattttctct agtgcccatg gagagtggct gagaaataca ctctaagatg 600

taaaagagtt tggtatctag atcctctttc ttattcattt caagccaata tttattaagc 660

accaactgca agctagatac tattatagtt aggaatataa agtgggccag ggatggtgtt 720

tatgcctata atcccagcac tttgggaggc caaggcagga ggattacttg aggccaggaa 780

ttcaaggtca gcctgcccaa cacagcaaga cctcgtctct acaaaaaatt aaaaaattag 840

ctgggtgtgg tggcatttgc ctgtagccct agctactcaa gaggctgagg tgggaggatt 900

gcttgagccc aggggttgga ggctgcagtg cgctatgatg gtgccattga aaacaaaaac 960

aaaaaccaac caacaaacaa aaaaacaaac aaaaagaaaa cgatactcag tctttatagg 1020

gaggttggcc agtcaatagg ttactttatg agttgctaac cctggtgagc aggaagttat 1080

gtggaccagg agagaaaccc ttggttcagc ctggagaaag gagaggttga ccctaaactg 1140

gagggtggag aggaccctgt tgtgactctc cgactgactt gtcttccttg atgtccttta 1200

agccggagct gattcgggct gctgccttat ttctgagtta gcgctcttaa gattgggcct 1260

cccagtttga ggaaggggcg ggctgctgtc tacctctgtg aatctgccct ggaccacccc 1320

gggagagaag gagggctccg gggaatctcg cacattccag gcaaaggctc ccgggccgca 1380

gcctctgtgc cacacccttg gcccgggcca ggtgtgcgcc ctcctcgctg cgagggggag 1440

cgggcggctg cggggagcga ttttccagcc cggtttgtgc tctgtgtgtt tgtctgcctc 1500

tggagggctg ggtcctcctt attcacaggt gagtcacacc ctgaaacaca ggctctcttc 1560

ctgtcaggac tgagtcaggt agaagagtcg ataaaaccac ctgatcaagg aaaaggaagg 1620

cacagcggag cgcagagtga gaaccaccaa ccgaggcgcc gggcagcgac ccctgcagcg 1680

gagacagaga ctgagcggcc cggccccgcc atgcctgcgc tctggctggg ctgctgcctc 1740

tgcttctcgc tcctcctgcc cgcagcccgg gccacctcca ggagggaagg tgagtcggct 1800

tccacaagga aacatctcag ccctgggctg agaatctgcc tttctctgca gaccgtgatg 1860

gctgaacgta cctccgagga ttcccacttt gtccagaaac ttgttagagc tcccttctcc 1920

tcccctatct ggggctcctc cagagacgga ttttaaatga gactaatata taaagctatc 1980

tgctttttaa aaatagtaag a 2001

<210> 27
<211> 2001
<212> DNA
<213> Homo sapiens

<400> 27
tcaggtgcag cgtcacagca gggggcctgc gttctgcttc ccagtctggg agaagagagt 60

gtctggatat tttcaggaaa aaaaaagaaa gttgagcagc cacagcaggg cagggcagtc 120

tacatcctca agtctccctc agcttctctg ctgctgccgg ctggcctcaa attttgcctc 180

tctttggttc tctgagaagt tttaccaaga gaagagacag gaggagagat ccagcctgga 240

ctattgtttg tggggtgtgt aggtgtgtgt gtgtgtgtgt gtgtgtgtgt gtgtgtgtgt 300

gtgtgtatgc gtgtatgtat cccgcgaggc tggagccaca gcttccctga tgaaccatgg 360

aggcgggcca gctcctgagc tgcggttgcc ctggagaccc acctaagcct ctttgcctgg 420

agcagcatta tctgtagagg gagtggctga tctgacacaa aagcctgagg cgacccaggc 480

ccaggcagtg agggagcact tgtgtccaga tggcattctg tccgcttcct cctccagccg 540

gtgctcagtg tggcacaaac tgggcaagac cattctcacc caaccctctg ctgagcccta 600

ggtgtacctg ctgttggcaa atgtttccag ctgcctggtg ggtggctcct ggatggagat 660

cccctctttc tatcagctcc ctcctacagg gaaggggtga ttctgggtgc tgaggacagg 720

gacctgtgaa gatacatctc cgcctttgcc ccaccacatc ccttgcctgt cttcctctct 780

gcttatctaa attatttcct tccctcaagg caagctcaag agcagacagt tctgactttc 840

agaaggaggg gctgtccagg gagaagagga acatggaaca gaaagcattg acgttgaggt 900

atttgagata acatttgggg atagcagaag catcctgttg ctgttctgca gcatcccaca 960

ctcatgtctg ggcctccttc ctgtctctgc tctctggtca cgctgtcact tgtagactgt 1020

tctgcctaga agccatgtgt gggccagctt gcacctaaca gacattaggt agcactaaaa 1080

cagatgcaca ctgccctgtg aggggcatgg gaatactgta atgggcatga agctgcatta 1140

taatagatct taggtaatcc tgtgctgggt gatagcgtga cttttgcaca catctggttt 1200

tgattgtgtt ggaaataaag cagaaagtac aaccaagaat aaattaaaca atggctgatg 1260

aaatggactc aggttgccca cctgcctgcc tgcatgattt cgtccctggg tgatttgtca 1320

cttcgtgctt gctctgtggc tggttccact cagctggttc catccgcctt ccctcttcat 1380

ccatcaagca ggcaagattc ttgagtcagg agttggatca tatacttctc ttgaccagtt 1440

cacagcacga cagccccaaa gattgagtgg gtcacttggg gatggaagat ccccacattt 1500

gagggagctg aagctgactt cctctgaccc acagttccca ggcggcctcc acctcagggc 1560

cagctaaggc cagagtcacc caaagcaggt aactgggagt ccccacctct gagcacccca 1620

ccactatcct ggcactttcc caaggggcgt ggcttggagg tccaagggca gaaattccca 1680

ggcatttcca cactgtaggg aagggagcag ggggatgaca agactttacc ttccctttag 1740

gaaagttgct ggaggcaatg aggagaggag ggcatggact aagtcacatg ctcatctgtg 1800

cagcacgtat ttctaaagca ccattctaca ggggcaccaa gctaggagct ggctgagcct 1860

ggctctcctc tctgcacaca tcccagccaa gcctgtgtgt gctggattgc tggatcagat 1920

gaggaggagg tggaagtctg gcaggcaggt ggggagcagg gacagcatta gggcccagag 1980

ttaaagactt ttatctccaa g 2001

<210> 28
<211> 2001
<212> DNA
<213> Homo sapiens

<400> 28
acttgggaga cagagggagg aggcttgctc gagcccagga gttcgtggct atagtgagct 60

atgatcatgc cactgcactc cagcctggac aacagagcaa gaccctgtct ctaaaaaaga 120

aaagaaattt ggaaatggtt tattttgtat taacaattta taatttacac tgaaatttat 180

tatgataaaa cttttccctg tgttaaaaag ctattaactt tatgaaaaat ttcttttagg 240

taaggttgat tatatatacc cacacacata cacaggttaa aagttagttt catgtgacat 300

aataactagc attttgagca ctacctgttt gcccagcact gttctaagtg ctctacatgt 360

attattgtta aattatcata acactatgaa ttatgtacta taattacccc agctttacag 420

atgaggagac taatccatgg ggaggttaag taacttgtcc aaggccagac agctagagcc 480

ggcttttgga cccacaccac agtctgactc cagcacccat attcttaaca atttcaccat 540

attaatatgt caagattaag cagttttaaa ggatgctatt ttctcacaaa tttcttaata 600

tgaacactca ataagaataa tcactaatat aagcatttag tattttttta acactaagtt 660

ggaagcatag tggaacattt atttttagaa atattattaa ttggctgggc tcacgcttgt 720

aatcggctgg gctcatgcct gtaaattttg ggaggccaag gtaaaagaat tgcttgagcc 780

cagtatttcc agaccagcat gggcaataca ttaagacatc atctttaaaa aaaaaatgtt 840

attaatctcc tctttttgtt aaatgtatat tatcaaaatt gttactaagc taacaaactt 900

cagaaaaact tatgatgggc aagctgcttg tgacattgaa ggtatttaag attcaattct 960

agtttggtcc tagatgacca catatccatt gttccttcaa cgagcacatg gtaaagagcc 1020

tagaacacag agacacagaa cacagtggag aaaagggagt gaaatgtctt taatgacact 1080

tactatatat gggattttgt gacaatatac aaggatggtt aagacatata aggtgatgca 1140

aaaaaacata ttaacaatta tagtgacaaa aaatgaggag catataatta tacattgatt 1200

tatacagagt accagaggaa cacagcattg agagccgtaa caccacctga gggagtggag 1260

aaaggcttca gagagaaagt gttttttgga atggatcact gtttccaaaa gaactaaagt 1320

acagtttgag aaatgcatac ttaattcatt acttttttcc cctcaacttt aataataaat 1380

ttacccaaca aaaaagttta tttttgactt gtaaatctct taaaatcata aaaaagtaaa 1440

attagctttt aaaaacaggt agtcaccata gcattgaatg tgtagtttat aatacagcaa 1500

agttaaatac aatttcaaat tacctattaa gttagttgct catttctttg atttcattta 1560

gcattgatct aactcaatgt ggaagaaggt tacattcgtg caagttaaca cggcttaatg 1620

attaactatg ttcacctacc aaccttacct tttctgggca aatattggta tatatagagt 1680

taagaagtct aggtctgctt ccagaagaaa acagttccac gttgcttgaa attgaaaatc 1740

aagataaaaa tgttcacaat taagctcctt ctttttattg ttcctctagt tatttcctcc 1800

agaattgatc aagacaattc atcatttgat tctctatctc cagagccaaa atcaagattt 1860

gctatgttag acgatgtaaa aattttagcc aatggcctcc ttcagttggg acatggtctt 1920

aaagactttg tccataagac gaagggccaa attaatgaca tatttcaaaa actcaacata 1980

tttgatcagt ctttttatga t 2001

<210> 29
<211> 2001
<212> DNA
<213> Homo sapiens

<400> 29
tttcatattc catttctctg tatgctttct tcaccaggaa gaaatagttt tagtgtcagg 60

aatgaatgag tctgcccctc aattccagcc tgctcagcac acaaggaaac aaagccctga 120

caatcagagt gactccctgg tgactaagct ccagtcctgg atgcatattt gtttagcagt 180

tctgacagca tctgacccag ccctctcttt gcatacccca ccagaacctt cttttttttt 240

tttttctttt gagactgagt cttgctctgt cggaagcgat tcccgtgcct cagcctccca 300

aatacctgga attataggcg taagccatca tgcctggcta atttttgtat ttttcatgga 360

gatggggttt tgccatgttg gtcaaattgg tctcacactc ctgacctcat gtgatccacc 420

tgcctcagcc tcccaaagtg ctgggatgac aggtgtaagc caccatgcta ggctcagaaa 480

tttcctttta taaaaatgtc attaaggatc ttggctgcac aatatcgtta ccagcttcct 540

ttaaatccac ctctggcctg ccaggaatca gggttcttca gaacctgaca ttttaaatga 600

agaggtcagg caggtcatga ggaaagcctc attgtcccca tgtctctgtc actgctgcac 660

ccctgagaca tcacagacat ggacactggg gcctgcttgt ttctcaaact gcccttagat 720

cgaaagaggg aggaaccagg atgaatgcca ctcattttcc caagaaaggc cctctcctga 780

gtgcccggga tggggctctg tccattgcct ggggccgcca attgctactc tgggttacgg 840

aagaaggaca gggtcctgag agacaccaga gacctcacac agccctgaaa acatggggct 900

ccttcataag tgtttcccat caccaacagg gagaccacgt ggaggccttg cagccctact 960

cggtgcttct ccaccaaatc ccaagggcag tgacgctgac gtctgtggaa agcagagaaa 1020

gccctggctc ccaaagccct gaagtcctgt ggagctgaca ttccctgagt gacggtgtga 1080

atggaaggaa ctcaagtgcg ggtggtaggc cacctcctgg cccaggcctg ggtgaactct 1140

gaggggacac atgtagtcac aatcccatcc tcccattctc cttctcagag gaaggaagtg 1200

ggcatccatc tgcctcatct ctctcccgtg gggaagatgg ggagtttcag gggaactttc 1260

acataaattt caccagctca gatctcctgt gaggatgggg cccaccatgc tcccggtgct 1320

gccagaggcc ctgagcccct ccagggtccc tgggtttgag ccagccctgt atcatcccca 1380

ggagctgaat gtccgagcaa tggatagaat tagatggaaa gagctctcaa tttggcctga 1440

gactgtcccc agatactcag gaaaaacagg acgtcgcaca gagtgggcag caggtgagtg 1500

gcaggttata ggtcctgagt ttgagtttgt tctcacgtga gacagaccca gcccctcact 1560

ccattcacac actgggtttt aaatggtgca agataggagg aattttctgg tcccaagagc 1620

aggaggaagg gattttctgg ggtttcctga gtccagattt gcataagatc tcctgagtgt 1680

gcattgttct ttgaggacca ttctctgact caccaggtaa gtggctgaat tctaacctct 1740

gtaatgagca ttgcacccaa taccagttct gaactctacc tggtgaccag ggaccaggac 1800

ctttataagg tggaaggctt gatgtcctcc ccagactcag ctcctggtga agctcccagc 1860

catcagccat gagggtcttg tatctcctct tctcgttcct cttcatattc ctgatgcctc 1920

ttccaggtga gatgggccag ggaaatagga gggttggcca aatggaagaa tggcgtagaa 1980

gttctctgtc tcctctcatt c 2001

<210> 30
<211> 2001
<212> DNA
<213> Homo sapiens

<400> 30
gaaaggaaag cctggctccc gctccaactg caaaaggaga aagataaagg atggggtgga 60

aaggctggga ctggaaaaat gtggagaggg ccaacaaggg gcatctggag acgggggaag 120

aagggggtta aggagagggg cggagagagg ccaagaggct gagaggttag ggcttgaaag 180

agggcgccgg cgagcaggag aaagcccaag aaggagtcgg aggttgggga aggaggagat 240

aaaggaaaaa ccccgggatg gagtgaagac ggcggcagca agcaactcac cagagcccag 300

ggagaggcag ggctggggac ggcggagagc cgcagacgcc ccttcggcca gtagtctcga 360

cttgaaggct ggcgaagccc cggcggcggc agccaacgag ccaaagacgg gaggggacgc 420

gtgagggact ctgagacagg gggcaacagg cggttacggg tgaggacaac aggaagcgtt 480

ccaactgccg gaaaagacgc gagcgctgag gggccaaggc agcccggtcc cccgccgcgg 540

agacgggctg gcgcggcagc ccggggccga gactacgcat gcgcgacttc ccggagcggc 600

cgggattgcg gaactgcgct tgcgtggccc cgccctgccc ctcccttcac cccctgccta 660

gggctggctg gacctgggtg aggccagatg tgtgagtgtg aggcggtctc gaagtaggct 720

gggctgaggg ggaagttcca ttgttttctt ttgttttact ttttacttcg aaataattat 780

ggactcacaa gaagttgcaa aattaataca tacaggttcc ctataccatc acccacccag 840

cttacctcaa catttttctt aagatacaac attctcaaag ccaggaaatt gacattcgca 900

caatacaatt aactaaacca atacaattaa ctaaaccaat acaattaact tcattacaga 960

ccacactcgg atttaacctg ttgggtatgc agtcatgtgc gtgtgtgtga gtgtgtgtgt 1020

agttctatga aatgttgtca cctgtatagt tcccacccac ccccctaccc cgtgtagaac 1080

aggtctgctg gagcccttgg cccccctcct cttccttaaa gtaaggacct tacctgggat 1140

aaggtgagct ctcataggtt tgggaggtta cccagcgggc gacccagtgt ggacagaaga 1200

aggtgctttt caaggccacc caactcttat tctgtactac ttttgggggt aaagaggtcc 1260

caagtttaat agcctttggg cacttgtcgt ttcttgagaa tggagcacaa agccctcaac 1320

tacaaagtgt caagctcctg gaaactaaga tgaacgagac atggtccctg actgcctgca 1380

aaggggccat ctagggagag aaggaagcat gtgctgcgag ttattcattc atttcatctt 1440

caaagcccag tttccttttg tctagcctct taacaaaaat cacttctcgc caggcgcggt 1500

ggctcctgcc tgtaattcca gcactttggg aggtccaggt gggaggatag cttgagccta 1560

ggagtttgag accagtctgg gcaacataag gagaatccac ctctaaaaaa atttttgtaa 1620

ttggccagca tggtggcaag tgcctgtggt cccacctact gtggaggctg aagcaggagg 1680

attgattgag tccaggagtt cagggctgct atgagccatg atcatgacac tgcaccccag 1740

cctgggcaac ggagggagac cctgtctcaa tcagtcacta catacatatc aatcaatcac 1800

ttcgcctttc attctctctg cccagaggcc cttctcccca aatgcctgtt gtcaaagtcc 1860

tacccattct taagctttta ataatagtcc aagatgtctg cataatgctt tacagtttac 1920

aaacaacttt cacttattta atctcacttg gtcctcactt tggcagtgac acacgaggtt 1980

ttgcagatga agtaattgat a 2001

<210> 31
<211> 2001
<212> DNA
<213> Homo sapiens

<400> 31
gccgagatgg tgccactgca ctccagcctg ggcgacagag cgagactctg tctcaaaaac 60

aaacaaacaa aaaaaacaaa acaaacaaac aaacaaacaa aaaaccttcc ttggattttt 120

caagaagttt tcacaattgc aaaaaaacaa aacaaaacaa cacaacacaa caaaacaaaa 180

caaaagcaaa aaaaccctat gcttccaccc aaggtaaaaa ttttagcttt aggtccactc 240

tcaatacatt atttaatgaa ctgaagttgg caaatattcc tcacagcctg ttggagggtt 300

cagcagttta ttacagaagt atgaaatgct tttatttaaa aaatgtattt tggtaaatac 360

atttttgttt aggtaaatat catcaaataa tccaatttgg aaaccaacat tcttacttct 420

ttttccaaca gttgttccta tcatcataaa aacatgttaa atttttctca tcctttcaaa 480

aaatctctgg agcttacctc accctccaga tacagcctca cctctcattc caccatgaaa 540

tcagacttct tgagatggtt tcagctggac tccatccttc aactgacaac ccaacacaac 600

tgtattcatc tctcgttaac attactagca gtgagtaaca tcagaaagtt tttgacacat 660

gttagtcttt tttgtgatga acttcacaga tacatttgac attggtatgc ctcatttatt 720

tgttgaaatt tttttctttg gcttccatga agtttctttc tcttctgtat cattctactt 780

ctatgactgc tccttctcga gtaaaacaga atgtgtctca ggattacttt aaaacaagac 840

aaagtataga gttataccta aaatttagta tttaagttat tggatcagaa aggaaactcg 900

catttagagt atgaaggcat tgtcagccac caattacttt tgtaacctga agctagtctc 960

cttcctactc cggactgaat ttcttctgta taatgcaagc gatctggcat gatgatatac 1020

aaagaccgat aaaattttgc tggggattct gaaagtaaaa aaaattgcct ttgatattat 1080

gtccccatgc taagtccctg ggactttgga cattatcccc cactgagcag gggtgaggaa 1140

gctggcattt actaataagc tatgaaaggg cagtgccttt tgacatttca gctccaccca 1200

tagcacaccc actcaaggaa catataaatg aagagatccg ctcagttctc agacaagatt 1260

tttcaagcaa gatgaagtcc atcatcctct ttgtcctttc cctgctcctt atcttggaga 1320

agcaagcagc tgtgatggga caaaaaggtg agtggagagg gtaagccttg gggaaagcta 1380

ctttaaaaaa atggcctcta aggatattca gggtgcaaac agtaacctgt tcaggcacag 1440

attcttctcc ttgatgagaa ttgatttttc tccacccaac gctgtaggct tttggaaata 1500

tcagaaattt gttgggaaaa ggtgggaggt aagagttgca agagagcttt ggagataatg 1560

aatgcataca tttctattat caattaccag gtggatcaaa aggccaattg ccaagcggat 1620

cttcccaatt tccacatgga caaaagggcc agcactattt tggacaaaaa gaccaacaac 1680

atactaaatc caaaggcagt ttttctattc aacacacata tcatgtagac atcaatgatc 1740

atgactggac ccgaaaaagt cagcaatatg atttgaatgc cctacataag gcgacaaaat 1800

caaaacaaca cctaggtgga agtcaacaac tgctcaatta taaacaagaa ggcagagacc 1860

atgataaatc aaaaggtcat tttcacatga tagttataca tcataaagga ggccaagctc 1920

atcatgggac acaaaatcct tctcaagatc aggggaatag cccatctgga aagggattat 1980

ccagtcaatg ttcaaacaca g 2001

<210> 32
<211> 2001
<212> DNA
<213> Homo sapiens

<400> 32

ctgacagtgt cttcctgaat gactcaagtg gtcaaaaacg caagattatt tttcattttt 60
ttacagagaa ataaaataca attcatggcc tttactgaag tttactgatt tgtgattcat 120
aaaacaatta gcgtatcatt agattttatt aagagctata atttagtgtt ctaggaaatt 180
tagaaggccg aattatactt tgaaatgcac tggcaggcat actaaggaag tcctgaggtg 240
aatcattttt tataaaccta catttttata tattacatta gagccctata aatatatgta 300
tacacttttt tttttttttt ttttttcaga cagagtctcc ctgtcaccca ggctggagtg 360
ctgtggtgcg atctcagctc accgcaacct ctgcctcctg ggttcaagag attctctgcc 420
tcagcctccc aagtagctgg gattacaggt gggtgccatg acgcctagct aacttttgta 480
tttttagtag agacagggtt ttaccatgtt ggccaggctg gtctcgaact cctgacctca 540
ggtgatctgc ctgcctcgat gtatatactc ttttatactt acttttgact agagacaatt 600
gagacttgaa aattagaaaa atcaagttag aattactgag aaaattagta gctctgatat 660
tctacccatt ctttattttt aaattaaaat taatcttaat gacctacaaa taggttaact 720
taaacagtcc ataccttaca tgagaagctc cgacttggga tgtttcttgc taactctgat 780
atcttgggtt tattctgaag acacttggtt aggtgatatt ttttcaattt tattctattg 840
taacaatgat cagcgaagtt atagtgattc aaatgctgtt tctgcatgca ttttccaagg 900
tgaggaatac tatacttcaa agcttggaga aataatttgg tcttctgctg catgatatca 960
aagtccctgg tttatgctcc ttttggggct gccagcacac gaatgatttt ataggtatga 1020
ttacagagta gtgggttctc agagtaacta agatcagcag ttctcatccc tccttttcag 1080
ggccatgccc cacttaggtt atgaataact tcagacagct gataacttag gctaactaaa 1140
tctaaaaacc tgtgtaagtt aaagtttcat tcaaatcata attcttggca gatgtgactt 1200
aaatgtctca tttgtaaagt taatcctatc aggctcaaac tatgcagcac tgactctggc 1260
acacataggg gttaaactaa aacaaaaaac aaaacaagaa acaaaatata aatttgcaaa 1320
taagtacaca gacttaatgg ggtatctcag gaactcaggg ctgtaaaagg taaaaagccc 1380
tttggtctta cataattatg aacgtatctt tggtatatat gtggaagata aactggaaaa 1440
gcctaattat ttaataaagg tgggggaggg aagattaatt attgttgagt gttaactaac 1500
tgccaggtgc ttttatcttg tttgtctaat ttaaatttta tatggactta ataaagtaga 1560
tattaccatg cacattttac aggttgccaa actgtggctg tgggaggttc taagcaactg 1620
ttccaggtca cttgtacagt aagctgtata gcaaaaaatc caatttggtt cggttccaaa 1680
tactggacat actcaacttc ggatttttaa tgttaaaaag gtatttagcg ctttaattaa 1740
aaacaacaaa aaacccctgc aagatgcaca aataaaatgt aaggccatct ctggtttgtc 1800
aggaggtagc ttttattaac cttgattgct aataagtagt taagagtcag agagacctgg 1860
gttgaggtct tagctcctac acttaactgt gtctccgggc ctcagtttct tcatctgcaa 1920
aaatgaaatg acaccattta ccttctaggg ttgttgttgt aaagacaaaa tgagacagct 1980
taacattaag accatacagt g 2001

<210> 33
<211> 2001
<212> DNA
<213> Homo sapiens

<400> 33
ccccagtttc cttggttaaa atgtatatgt acataatccc taaagtgctg gccaagctcc 60

caccatgaga taagaatttc aggccaacct gactctataa tggatggcca caggaaagaa 120

catgctagtt gtctgtgatg ccaaggactg cacacactaa caccacctgg gcacacacta 180

acaccacctg ggcaaaagcc attgggtgag gagctgcatt ttttttcttt tgagacagag 240

tctcgctatg tcacccagga tggagtgcag tggcgtgatc ttggctcatt gcaacctccg 300

cctcccaggt tcaagccatt ctcctgcctc agcctcccga gtagctggga ttacaggcaa 360

ctgacccatg cccagctaat ttttgtattt ttagtagaga cagggtttca agttagtcag 420

gctggtctca aacccctgac ctcaagtgat ccacccacct cagcctctca aagtgctggg 480

attacaggcg tgagccacca ggctcgacct aatttctgta tttttagtag agaccgggtt 540

tcgctatgtt ggtcaggctg gtctcgaact cctgaactca tgtgacccgc ccgccttggc 600

ctcctaaagt gctgggatta caggcatgag ccaccacgcc tggccaggag ctgcattttt 660

atgccattat aacttttcta gttatttttt tctttttctc caccagacag gagtcaaaat 720

taaagtcatc tctgtttttt aagaagtaat tccaaaaaga ccccaatcaa acctaattcc 780

ttaggattct gttttctgaa atcctaggca tgacagtcct taaaccagaa tgattgctgc 840

tactcaccat ggctgggcca tgcagggtct tcactgtcat gttgcgctgg tggcttactt 900

tgtgcaggaa gctctttctg tgaatgtatc ttcctgtgta tgcctgtgtt ctatcaatga 960

gagtacctgt tagttttata gcaatcattg cccctgtttc gattgacctg cttactgtct 1020

ttaagttgtg gttgacccgg agttactgac gaatgcaggg gttttttttt ttattccatt 1080

tcccgaaggg gaacaaaagg gggaccctaa agaaagggat gacaggaaaa ggaccaaatt 1140

tttctttcct tctaccctac ttccaccccc attgcccaca aatcagagca aaatttttaa 1200

tttactacag agatgaacca agttagaaat tgttgtcaaa tcttttttgt tcttccacaa 1260

attgccaaca cccttgtcac ggaccatatg ttctactttc cagtcacgca atatgaatcg 1320

tgatttaaat gggagaaaac tcatccttag aggagccttg gtactaaact atctttacct 1380

ccattttatg aatgggaaaa caaaactaaa gaaatgaatg acataagtga aaaattcaga 1440

aatgatatac cctggtagtt ttgttatctc cctgaactaa ttatttttta accccatggt 1500

tagactccta ctggtgctta ataaataatc actattcatt gtgttgataa aacaaactgt 1560

tttcctctgc tctcacacca caaccagcaa cacaaaagat ttccatgacc atgtatgtgg 1620

gcatttatct ccatcaacca gcaagcaatc aattctgcat ccaacgccag ctaagcatct 1680

tctaatccaa ttcaattctg acgctatctc caggtaggta gagtcagatc ccacaggctg 1740

aaggctcagt cccacaaaac tgctcccact ttaagcacca atcacaggtc caagcctctg 1800

ggacttctga ctgactggcc tcaagttcag gttcccacaa accgcttgag tttgattagg 1860

cttgattaat ttgttagaac agctcacaaa acttagggaa cactaattta tatttaccag 1920

tatattctaa aggatatttt taaggataca agtcaacagc cagatgaaaa ggtacacagg 1980

gcaaggtttg aaagggtcac a 2001

<210> 34
<211> 2001
<212> DNA
<213> Homo sapiens

<400> 34
ccaccaactg gggccgggcg cggtggttca tgcctgtaat accagcactt tgggaggccg 60

aggcgggcag atcacgaggt caggagatcg agaccatcct ggctaacacg gcgaaacccc 120

gtctctacta aaaatacaaa aaattagccg ggtgcggtgg cgggcgcctg tagtcccggc 180

tactcgggaa gctgaggcag gagaatggca tgaacctagg aagcggagct tgcagtgagc 240

caagatagcg ccactgcact ccaatctggg caacagagag agactccgtc tcaaaaaaat 300

aaataaccaa ttggacaagt tatctgactt ttaagaactt tcatctataa aatgtcaata 360

ctagctacct tatatggtga ttgtgaaagt tgggtaagta gacagtgtga aagtacccag 420

catagtaagg attcagttaa catttatgga aaataatcca gatacttagg aacttttatg 480

aaataacaca atgtatttaa actgttttct agtctgccat ttgctttaca attataccgt 540

tgactggtat ggtgtttccc agtgtctcat ctctctttta ggttactgct tgccttcctt 600

gtttcactga attggtatgt ctgacctaat gaagactttt tagattattt agaaactact 660

acgttttctg cagtcttacc tgtttcagag cgtggtggag aatccaaaca ttcaaaaaca 720

tgccatcgag gtgtctgccc tatcaatgag gaaaaaggca tctggcaatt tggacagtat 780

ccatcataaa ctggacgtat ctttggggac acgtgttggc ttttttgagt tctacagagt 840

tttcctggag tagtttcctt gtcttgggtc tgctgaatac catctccaca acttgaattc 900

tgactagaag caacagaagt ctgacaacct gcatttccaa ggggcacttc atggtccttc 960

acctctttag cttctgcggc tctttttctg tttctactcc gttttgactg gtattttcca 1020

tctgttgctt tttcaacaga ttttagaata tttttagagc cattatttgg atcaactcgt 1080

tttggttttc ttttagattt gtattcccaa atgtcttctt cggaaatgtc ttctaacatg 1140

gcaaaatgat tttatcattc aagaagtatt aatcttaagt aaactgaaag tccatttctt 1200

gtcacaaaca aaaagttata gaattatttt gctgagaaaa aaaacaaagg taacaaaacc 1260

cccaccaact caatgggaat ctgcagtgtt ggtaatgaac atattggcaa gctacaaacc 1320

ttgtacctga caacacctgc tattccattt ataccacaat gaaactaaga taaacctatc 1380

acaggagtag caactgtgaa gttctccatt atgggtgcta tttcattcaa atatcttgtc 1440

cttttccagg gcttgattca taaaaataat acttcaaaaa ttaagcgcta atggcaaata 1500

taggttcaac agcttaatct tagaacgaga tttgtaacat tgttatgaag ctactgtcag 1560

ttctttcaag tggacaaccg tccaaataat tcataaatcc aaccacagtc cctggacaaa 1620

gggcttaaca ggtatcccaa acactaaatg actatctaac aaaactagtc acaacataat 1680

caaagagatg tgtaattcgc aaaaaactgc gaagtcactg ccctcatcca cgactagtgg 1740

atactcaacc acttcagttt cccgcgacag tccatgatct tcctccagca gcaatgaaca 1800

atccctgcac agtgaacaaa aaaagttgat atgtgaaaaa agttcaaata tgtgaaagag 1860

tgggcttgta ttgaggtctg actccgtatt gcacccatgg ttccgaaggt ggccagccct 1920

ctgtccacct gactcaaaac ggggtcacac gccagcctct cccttatctt ttggcattag 1980

aggctgggag gccaggaagc g 2001

<210> 35
<211> 2001
<212> DNA
<213> Homo sapiens

<400> 35
gtgatccgcc cgccttggcc tcccaaagtg ttgggattac aggcgtgagc catcccaccc 60

ggcaatcgtc aggactttgg cttggactgt gagtgagata gggagttgca ggaagctttt 120

tcgaaaaacc acctttgggc caggtgcagt ggctcccacc tataatccca gcactttggg 180

aggccaaagc aggcagatca cttaaggtca ggaatttgag accaacctgg ccaacatgtt 240

gaaaccccat ctctaccaaa aaaatacaaa aattagccag gcatggtggt gcatgcctat 300

attccccgct actcgggagg ttgaggtggg aggatcgctt gaacccggga ggtggaggtt 360

gcagtgagcc aagattgcgc cactgcactc cagcctgggc gacagagcaa gagcctgtct 420

ccaaaaacaa acaaaaaaac ccaaaaaact tcttttggca gtgtacaatt attattacta 480

ttttttgtag taaatttaca gagatctgca gtcatgacca caatccaaat ttagaacaat 540

tctatgactt caaagggatg ccttagagcc gttccctgga accactcctc tgttctctct 600

gtatctactg gagggttcta agcagaaaag aaacaaagtc tgacttatat tattatcact 660

attgcacctt ttctgttaaa acatcataat cccagcactt tgggaggccg aggcgggcgg 720

atcacgaggt caggagatcg agaccgtcct ggctaacacg gtgaaacccc gtctctacta 780

aaaaatacaa aaaattagcc gggcatggtg gtgggcgcct gtagtcccag ctactcggga 840

gcctgaggca ggagaatggc gcaaacccgg gaggtagagc ttggggtgag ccaagatcgc 900

gccactgcac tccagcctgg gccacagagg gagactccgt ctcaaaacaa acaaacaaac 960

aaaaaaaaaa acccgtcata atcttcctgt tgctttttcc gattagatgg cgacttgaat 1020

ttttagggaa tacatccacc agtttgggca cacccaccag gcacgacagt tgaggtctga 1080

tcttccagcc actgtgagta ctgagaaaac ttggcagggg gccactctca ctggctcaat 1140

gcaccctgac cactggaagc cactgtgtca gctgctgggt tctggctctg cccactcttg 1200

gctgcatttg gggctgaagt tccctgtggg aggctgtttt ctgagggagc tgagtgttta 1260

cagccactca gccctgctct gctcagctga agcagaaaac agagaccttt tgcattactt 1320

tggttcaaga gcaaggtaat ttggaaagaa atttttcaga cgaaggtatc agctgcctct 1380

gaggcctttg ctgattaaat caagaagtga catctgaaat ggtgtcgggg aatggaaagg 1440

aggcctgggg attctaaatt atcagctgtt taggtggctg aggaatccag gggtgggaga 1500

ctgaggaaaa ggactggggc agagctcagt tcaggagagg tgaggggcgg gagaggtgag 1560

gggcgggaga ggtgaggggc gggagaggtg aggggcggga gaggtgggtc agagaaagga 1620

agtctctgag gctgcaggcc taggcctggt ggccactgca gaggctgggg cctatgtttt 1680

tccaggaagt gaaacctgaa agtcactttt cccctttaag cctcattttc tttatcttta 1740

aatggagaaa ataacattta cagcatagag ttgttataaa gatttcatga aataatgcct 1800

ctaagcactc ttaaaatgct tggtacacag taggccttca atagactgaa gctgttatgg 1860

tttttcttgt tgtgccacgt tattattgtt ggttgttatt accatatttt gttattatta 1920

ttactaagag gaatttaatg caaggaaact gaaactgagg tcacacgcag ctcccgggcc 1980

agtgcctctc aaactttaac a 2001

<210> 36
<211> 2001
<212> DNA
<213> Homo sapiens

<400> 36
ggggtgtcca tttttttggc ttccctggtc acattggaag aagaacaatt gtcttgggcc 60

acacatacat aaactacact aacactaatg atagctgatg ggcaaacaaa caaaacaaaa 120

aatgctaaaa atctcctaat attttagtta agtttacaaa tttgtgttgg gccacattca 180

aaactgtcct gggctgaggg ttggacaagc tgctttaagt catttgtaga ttacttataa 240

tacctaacac aatgtaaacg caatgtaagt ggttgtcatg ttgtattttc tttgtattat 300

tttactgttg tattattttt tattgttagt ttttgttttg ttttgtagta ttttgatccg 360

ctgttggttg actctgcaga tgcagaatgc tcggataagg aagccaactg tactcccgca 420

taccagctac ttgcttactc tctatttatt atctgttatt ctctatgcct tcattctagc 480

atatgcttca ttctagcata agtaatcacc tttctacatt ctggagtcag gacaatatct 540

agatcattgc tcaagagttc tgaactggga gattaaaaag ctgggccagc tgggcacagt 600

ggctcacgcc cgtaatccca gcaccttggg aagtagaggc gggtggatca cctgaggtca 660

ggagttagag accagcctga ccaacatgga gaaaccccgt ctctactgaa aatacaaaat 720

tagccgggag tggtggcgca tgcctgtaat ctcagctact cgggaggctg aggcaggaga 780

atcacttgaa cccgggaggc agaggctgca gtaagccgag atcgtgccat tgcactctaa 840

cctgggcaac aagaacgaaa cccgaaaaaa aaaaaaaagg ctggggaggt gattgtgggg 900

aaagacaggt ctcccaaaat gccctctgtg tgtagttgtt cacggaaacc acataaacag 960

aaaatagcta caacagctaa ttcttactaa ggtgtcaagc gttgtgcata acactgccta 1020

atgtgtgaca ttaaacgttt tcaccaggtc agccacttaa tcttcagaaa actggtggcc 1080

aactctgagg tttcacattg acgtttaatg gctgttccta aagaagtgcg tgctttttaa 1140

aaaaatagag gatgggaggt aggttggaaa gggaagataa gcggcaatgg aaaaatcgag 1200

tgtgattatt gcatttttat caaatatgtc tgtctggatt atgtggttag aatcactgca 1260

aatttggctt ctataaatac tttaaaactt ttttccaatg ttttcattat cagatccact 1320

atgcaggctt tgagtgggct gccatttagt tcagatatca aagtatttta caagtaacta 1380

atagcattta ttatctacac gtggaaaact atagcaaaga aaggtcaatc aaaagttgtg 1440

ctctaggtca tcagataaaa tgagattaat actgaaaaat tcttccacga agaccagggg 1500

tccagctaaa tgtgtggtgg tgggaaaaat gtttatctga cgtccctcta aagaaacctc 1560

agatgggaga aaaatgtccc agccccagga atggacacat ttaggttttt cctttttttt 1620

tttttttttt ttttttgaga cagggtctcg ctctgttgcc caggctggag tgtagtggcg 1680

cgatctctgc tcactgcaac ctccgcctcc cgagttcaag cgattatcct gcctcagccc 1740

cccgagtagc tgggattaca ggcgcgcgcc accaggccca gataatttct ttgtattttt 1800

tagtagagat ggggtttcgc cctgttggcc aggctggtct cgaactcctg gcgatccgcc 1860

cgcctcgcgt tcccaaagtg ctgggaccac aggcgtgggc caccgcgccc ggcgcacatt 1920

tgggtttttc gtggtcagca aaagcacgac caccccaaca cataaatctg gcgaatcata 1980

cgcaaattcc ctcatgctat a 2001

<210> 37
<211> 2001
<212> DNA
<213> Homo sapiens

<400> 37
tgtgccagga atttattaat ggagaaaccc cgcctctact aaaaatacaa aattagccgg 60

gcggtggtac atgcctataa tcccagctac ttgggaggct gaggcaggag aatcacttga 120

acccaggagg cagaggttgc agtgagccga gactgtgcca ttgcactcca gcttaggcaa 180

caagagcaaa actccgtctc aaaaaacaaa acacaaacaa gaaagaaaac ccaaaaaaca 240

aaaaacaaac aaaaacccat agatggtaca aaaagtggaa gaaaaaggaa aaaaaaaccc 300

atgaaactat ctgcaatgca gtttcctaga atcaaccaca gtaatctttt agcttttctc 360

tatctaaatg ttatacatat tttatattta tattaagtgc atttatattt atattaaaat 420

acagatacaa atttaaaaac tctttattat taaattttca aataaaaagc aacttttcaa 480

ataaaagcag cgagaacctc cttataccca acacctggct tcaactatta gcaacccatg 540

gtcaatattt cctctatttc ccacttaatg tctctactga attatttaat ttcacccata 600

aacacttgtg tgcatctcta aaagttaagg actctttttt gaaaaagact ttaatacaat 660

ttctcatctc aaaaaattag caataattcc tcaacagcat ccagatgcaa tttttcatgc 720

ttttttcagt tagtttaaaa tcatgagctt ttctccatgt ctctaaaatg taattactaa 780

tgcttctaat actattccac caggttgagt gtattataaa tgatctgatc accccttgcc 840

tcccactctt ccatcccctt ggacatattt aggctacaac atccccagga aaatggtttc 900

cattgtctgc acgtaagcaa acaggcatta ttaacctatt agctgtccac tgctccaccc 960

cacccacagg tacccaacta gtcctgtgca cctacctggc gtggactgcc aagggaacct 1020

ctggatctgc cagtcagcca ccctgtcctg ctcagacttt accagagacg agtatttccc 1080

tttccttccc tctaagagca ttttatttgc agagttagcc aatagtatct tgtttcctgt 1140

aagggagact atctttagat gctgccacag gcaagctcag atactccagg gtggaggcca 1200

gctgccaaca ggagggcctg actttataaa cctggaagat ctaaacctca gggaagactg 1260

cggagctgga gggagagagg tgccacttac cccaacctgg aggtgggggg cagggagcag 1320

gccctgcagt cagcaaagca cagcacagtc agctctcatt aatggggagc cttcaggaag 1380

ctgggagagc tcatggggcc ctcagcttcc ctgcccacga gagggagccc agagtactca 1440

ctggaccaca ctggggcaca cacagctctt ctcctcaaag tgcagtccca ggcactggga 1500

gcttattaaa gacagcacct caagccctgc cctatatctg ctgaattaga atctgcattt 1560

taatgaaatt cttagaagag tgatatgcat actaagtttg agaagctttg gtactgaagg 1620

catctagtga gtaccttgca ccttgtagat gctcggtaaa tatttttttc ctcacctctc 1680

tgccccaagg catttagtgg ctccctatac ccatcagtca aggctgagga cacagctttc 1740

acccaggacc tggcctatgc tcctttcaca gtctctcccc ctgccactct tgccctgcag 1800

tggggagcct ggtgggctcc ggcatccagc catgcttctg ctccctggcc tggccagctt 1860

ggggcgactg caacgtgaat ctcaactgcc actgcctcat caccttccga tcacttcatt 1920

acctgcctga cccctggcat atgctatata attctgatcc atccctaact agggcctggc 1980

ccatggtggc cttcaataca c 2001

<210> 38
<211> 2001
<212> DNA
<213> Homo sapiens

<400> 38
ctcatcctga gtttatttca tcttcttatt gttttcttct gaatttacat cttttcatca 60

gaacaaaaag aatacaatca gtttttcaga agtttaaaaa attttttcaa atatatagca 120

aagaatgtca tcatttttat atactctacc aggactcctg aaaccaaaca cttaagaaag 180

gttatgtctg gaaatttttt caaaatctat atgcatttca gtggtggaga aatagttctg 240

tctggaagca ggaggctgaa attatgaccc tgaagatttc ttccagcttt tgaacttctt 300

attctatgac aactcctggt agatacaggt ttcctggtag ggattgagca agaagcatgc 360

acaattactt tatgagtctt gataaagctg gaatttcagg ttgatggtta gaaggagttc 420

ggctgggtcg tgagaatgct tgttttcctg gttactggat atgccatggg ctgtatatct 480

tgtgttgtcc tgtggtaact gaagccactg ttgctgtgga aaatgatggc agtttgaatg 540

agataattta gagtgaccta tggaaatctg aggctgtctc cttaatgtac aagaccttgt 600

atgtgtggca ctgttgtatt tgaatggcac acatctcttt agttcccttc aactgaaatg 660

acaaaaggtg agaaactgaa tcgtaatttg atggcttaat attaaaaaaa aattggcaaa 720

aatgctaaat aaggaacaat tcagaagaat tgctgatcat ttcaaaaata ttatttgcat 780

agcatttata ggtagctaaa gaaagagaaa aatggcttaa tattttaaaa ataatttggt 840

caatttagcg atctggggat tctgcgatcc ccagaacttt gcatccaatt tccaaaaacc 900

aaaccttaca tctaatgtaa aatgacacaa aaaattctaa atccagtcta attcagtagt 960

atatagctta ttcccagtga tgtctcacat tcgcaagccc gttacacgtt ggttttacct 1020

gtcaccaaaa cctttgtatc actcttataa aaaaccacat cactttctat aaaaagtgag 1080

tggaagaatc taatattgta aaactgcacg tgcctcgcgt gacctgagca gaaaattcct 1140

agtttgggat tttctcacag gcatacctaa actgacctct gctcctagca ggtaaattgc 1200

tagaaggaga agcaagtgtc agtccaaaga aaacccttgt tgtgtgattt tttttttctc 1260

tttaatcgct ttttaggtgg agtacccgct gcattctcta aagtctttgc caccaccaga 1320

ttaccaacgt tttcaccaat ttgccacctc cctactcctc tcctttccaa aagccctcat 1380

tggaaagaac tttctctcgt gaccccccta ttccatctag ttccaatcac tcccagcgcc 1440

ccccatctcc atcggcttcc cctctcccgc ccagaatcca ggttcccaaa ccacttaaga 1500

gaaaattcac aaccctcgcc cccttcccgg gcgtccggag gaggcgctgg ggtgggggtg 1560

tctgtggggg acagggtttc ctggggtctc ctgggtcttc ctcgcgcacc cccaggaggt 1620

gttgcagggg gaggggagcg cgggtgggtg ggaccgggcc gggtggggga ggggtgaggg 1680

cgaggaggag gaggaggagg aggcggcggc ggaggagtgg tgtgtgtgcg agcgtgtgtg 1740

gctgggggaa gccattgcct gtttaatagt tgctgttgct gcacttccgc ttctctccca 1800

gcgagagaga gacacgagtg gccaggccca gccgcagccg cagcagcagc cgccgcggcg 1860

gcacggagga gccagacaca aagagaggta cggggatccc cccagcggcc cgccgccccc 1920

cggccgcgcc gccgggcctc ggggacaccc ctcgccgcct cccccgactc gcgccgccgg 1980

ggccgccggg ccgcagggca c 2001

<210> 39
<211> 2001
<212> DNA
<213> Homo sapiens

<400> 39
aatagccagc gtgaaagaac tgcccacata ctagggcttt tataaccctg ggatttggcc 60

tctgtgaaaa ctgatgacgg atgtcgtttt cttttttttc ttttctgaga cagagttttg 120

cttttgttgc ccaggctgga gcgcaatggc gtgatcgccg ctcactgcaa cccctgcctc 180

ctgggttcaa gcgattctcc tgcctcagcc tcccgagtag ctgggattac aagcatgagc 240

caccatgccc ggctaatttt gtatttttag tagagatggg gtttctccat gttgatcagg 300

ctggtctcga acccctaacc tcaagtgatc cacccacctc ggcctcccaa agtgctggga 360

ttacaggcgt gagccactgc gcctggcctc atagatgtcg ttttcttcat gaataagcta 420

gagtagagtt cgtttgaaaa tacacacacg caccacacgc acacacacac acgtgtgcac 480

acacacgcat acaccccccc acatacaagc atacagatgc acacacacac ccatatgcac 540

acacccacgt gtgcgcacac acacacagat gcagacatac atatgcgcat acacacacca 600

atacacatac acgcacacac agatgcatgt atacacacac acacccatag gcacacacac 660

acatgtgcac acacacaagg acaacagcaa cacaaacaac atgagccaga agctaccaca 720

gtcaggctcc ttgcttctga gcactcactt gctccaggta aatatccacg ctgcctgttg 780

aggttgtgct gatgtaagca actgccaaga atggatggac agggtgccag ctgataaatg 840

agggtgaccc tgctgcatcc ggggcttcta tctggtggtc ataatagagg gccatgacgg 900

aactcaggcc tcctcagcgc tgaaacctgc agggaaaaaa aaatgaccaa gtccaatcag 960

ttttaaataa aacaaacaat atgactgtac atggaatgac gaaaaggaag catcccaaag 1020

ccaccatcgg aagaccatcg gcaacttgaa aatgccttgc ggactgagca ggcaacctga 1080

tgcgaggaga tctccaacag ggagacatgc agaagagaag cagttagacc cggaatcacc 1140

caggctgtga cagaaacgaa tacaactcat ggccatcaaa ggaggaaaga aagaaaaggc 1200

ctgcagccta actacacacg caatcctcag taactttcat tccagtcaca ataaggacaa 1260

gttctatttc tgtagttccc tttaactttt tagaccaagt cttgctctgt cacccaggct 1320

ggagtgcagt ggtgtgatca cggctcactg cagcctcaaa ctcctgggct caagtgatcc 1380

tcccacctca gcctctcaag tagctgggac tacagacatg cactaccatg cccagctaat 1440

ttttaagtaa tttgtgtaga cacaaggttt tactatgttg cccaggctgg tcttgaaccc 1500

ctggctcagg taatcctcct gccttggcct cccaaagtgc tgggattaca gccgtgggcc 1560

accactgcct tttaaacaac attttcaaac aaaataaatg caaactgctc cagaaaagag 1620

tactggtaca gaaaaaaaac cagtactgaa tttccaataa agcataaaat caaagactgt 1680

cttttacctc tgactcacag gtggtagaag acagaagtct tcctctatta gagagtcaag 1740

aatgcacagc cgcagcagct gtgagaagtt gagcttgatc acttacgtga acttaatctc 1800

tctctttcat tcttcctttc agatctggaa aagatataca catcgtccct tttcaggcgg 1860

gttagggtga taaataaagt gacggtctgg ttactgctca cttttttaca cctcagtcaa 1920

tttggggaag ttaactggca gagaagaaca tacttgtagc cgggtttgcc accatttaaa 1980

attatacagg ctcatgcctg t 2001

<210> 40
<211> 2001
<212> DNA
<213> Homo sapiens

<400> 40
ctgttgtaga cccagccatc caggcatggc tccatggccc tctgggtgtc attgggcagg 60

ctggcattgg gcggatgtac aaaacggagg cacctctcag gcttcccatt tgggcccatg 120

gggagcaccc aaggccctgt ggaggcattg tggggcgggc gacagtggtg gacaggggtg 180

gcggctgtga agatctgcag caggttgtgg ttggccatgt tgaggatcgg gaggcccagt 240

atggctacat gcaggaactg gaaatggccc atgcttccca cacggtccag gatctccgag 300

aaggtcatgg cactggggca agacgagcca gagctgtggg cagggcatag gccagggaga 360

ggtatatttg tgcctggtag tgcgtgggtc tatggaacga tatgtgggag ggtgaaccag 420

gtggatatgc gagcacaggt gtgtggagca gagcatgtgt aagtgtgtaa agatgtggaa 480

gctcaggcat gtggagccgc agattcttgt gtggatgtct gaggaagagc ccagctgcac 540

ggagggctgt gggatcaaag gtggaagtgt gtgtccaggt gtgtggacac aaaggtatct 600

atgggtatgt gctcatttag gctgcagcat aaggacacag ctgcgtgagc tcgggtatgt 660

gttgtgagcg tgtgcacctg cggacacgag catgtgtgtg ctcagctagg ctggttgcac 720

atgctgcatg catggagcag tgcaggacac gtagcaggga ccaaggggcc cagccttgga 780

tgttggctac aaaggacaga ttagagggtg ggtggtgaat gtccccagtt gctccaggca 840

gatacacagg gttagcagag ttcaggttgt ctgctgggaa aggcctcccc aagctcagag 900

cctcctggct tctggcagtt ccctggggct gggcccacca tgctggagta tctgtaagag 960

cccgaagcag gtctcagagg gcactagggg cttggctagt cgcaagtgga gcctggcctt 1020

gggagatccc tgggattcct aggtgcctgg gctggggagg ccctccatcc ctcctcacct 1080

ggtggccggg tctggatccg atggggctgg tgatcagtgt agttgaggtc cctgctctgt 1140

cctaggggcc ggcagctgct gtagtagggc agctcagctc taacaagctg tgtttgtgcc 1200

tcccccaggg gggcttatat aaggcataag ctgtttcccg ggctaatgtt tgactttctt 1260

ttgcggatta acgtcactgg cagggagagt gctgtggacc caccaagccc agctctcctc 1320

aaagggctgc ctcagcgagg gccgctgggg gtggggcagc gtggctgagc ctagcagggc 1380

tgctggctgg acaccacggg cccaaatggg aagtctgaga cgtgcctccg ttttgtacat 1440

ccgcccaacg cgagcagccc tggcaggctc accgagggga gattctggga aggaatctgg 1500

cctgacctct gagggttcaa ggtccctctc aactgatgtt cagaggactt gatgctgagg 1560

acaaccaatg ggaaaggcac tggtgcccgt aagcaggcag cattctcctg tgtgctacct 1620

cccctgcctg gcacctcccg agtaggggaa ggggatttgc ctctcaggaa gtgttagggc 1680

acacccagac agaaaatgtc actttcatct atccctgagg acgcagggct gccatctcct 1740

gggcccctgg ggcagccagg acctccgtgt ctgtgtctct gacacaggtg aactgccccc 1800

ttccatccca ccctcaatgg acaggcttct ctcctgatca gccgcttccc cctggggacc 1860

aaatgtagtc cctgctgttt tattttcact ctgaatatta ttttaattat ataataatgt 1920

gtgtttcctg cataaaacat gggaaataca gacaagccaa acaaaacaaa tcaaaacaaa 1980

gcaaaatgtc cagtctcacc t 2001

<210> 41
<211> 2001
<212> DNA
<213> Homo sapiens

<400> 41
atggcctagc tgagtgccag ccaaggttga ctcattcaaa ggcagcaggg gctggaacct 60

tggccttttg aggcccagtg cttttcccaa taccccactc tacgttgcct ctgcccatag 120

taggcagagg aaggcacagg gggcaggcct gttgaatgca tcttgggcac cactgttcct 180

gcgctgggtg gactgctgag tgcaggggcc aggcctccaa gcagggaggg gaggccggga 240

cagcccctcc agcagtaggg gtaggggtgg aggtagttac aaggtttcag ataaagcgct 300

cctccagtcc ccaggcagcc tggattctgt cggcaggaga gggtctgccc tggaaaggcc 360

tcggggcttc tctgcaggtc tcaacctggc tccccctttg aacagaacct tccaaggctg 420

caggcttgca tcctgcctct cagccaagac ctgtctcctc agtcatctga aaccacccta 480

ccacgccaga cgctccctct gccttcttgc tattccagct ctctcttctt cccacttcct 540

attttctctc cattttgttc aaactccatg aacaagaaca acagagctca gacagcgaaa 600

gcctccccat cccagctgta tgcaaattcc cagcaggctg aaaggaccac ccgcccaccc 660

aagaccacct cccaggaagc cgaggccttg tgtgcagccc cagaggagct tgagctgaac 720

ggagaatggg ttgaggtggg tgggacccat cactgttctt cccttctcat tcctgcgcgt 780

tccctgaaga gtctctgggg cctgcttttc cccccagcag agacggatgt gtcacattta 840

tggcatccca tttccttcac agagaggcta tagatcctgt ttcattctgg tgtatttaat 900

tcactccata ggaatcaata aaaactttaa attaaaaaaa aggcaaaatc caattgcttc 960

tcattttctt gatccaactg gcagtcggca tgtcacctac gttcatgtgt gttctggtga 1020

gctggaggtg ggagaaggag cttttcattg tacagtgagg cactaacttt cctttcccat 1080

catcagagat ggggggcttc aaccttcaag tgtccagctc tgcatgaagg gggtgaccgt 1140

ctgccctccc ttgcagcacc cctggccggg ctagccttgc ctccccatgt gctctccctc 1200

ggggtcaact ttttcttttc cttttctttt cttctttttt tttttttttt ctgagatgga 1260

gtctcgctct gtctcccagg ctagagtgca gtggcgcgat ctcggctcac tgcaacctct 1320

gcctcccagg ttcaagcgat tcttctgcct cagcttcctg agtagctggg actacaggca 1380

tgtgccacct cgcatggcta atttttgtat ttttagtaga gacagggtgt caccatattg 1440

gcctggctgg tctcaaactc ctgaccttgt gatccgccca cctcagtctc ccaaaatgct 1500

gggattacag gcatgagcca ctgcagggcc aaatttttct tacctttcca gagcaaagcc 1560

accatgccaa gaggggccct gcgcccatga tgagctctgg cttgggttcc cggcccaccg 1620

tggatgtgcc agacccaccc acaacagcct ctgctttgcc aggagttgta cccacctgct 1680

tgcctccagc gtctgtgcct cagccctgcc tctgctcact gatgccctgg gaatcagcgc 1740

gggcactcag tgcgaagccc ggctgggcac tggaaggtgg cagagggccc tggaccaggc 1800

tggctctggc ttgcaccagc gttctgcttt agggcttatg ctgaccactc cccacccact 1860

ttctccaagg gctacttacg gcgtgcacgt ggtctgagtg ggtctggagg agtctctctg 1920

cttgtcttct ggtgtccggc tgatctgacg ggttcttata tacctcccaa ccctgggagc 1980

tgcccttgga gcccattaac c 2001

<210> 42
<211> 2001
<212> DNA
<213> Homo sapiens

<400> 42
ctgaattttt ccttctggtg atgagaagca atgccagaat ttctactcca gccatcttca 60

cacttgtgga gagaaattca atagtgatac atttgtagtg aggagaaaga tgtgttagct 120

taacaactga tgcagggtct atcttttaag gaagatattg tgtttgtgga ggaaaacatc 180

ttggaaggta tttagaagtg gaaagtaaga ctttaagggc aagagaaact ctgcccatgt 240

caagagacac aactaaactg ctttgtattt ggaaatcaat aggaatttgg agagagaaac 300

taaaaacatg agtaatcaac aaaagatgat ttaacctaca tgatcggaat ctgaagccca 360

agatttggaa ggagagaggg aactttagga acatcaatat ctaacataga atattacatc 420

ttgagccttt tggcagtgta aatctctaca tcccctataa ctcagtagtc ctttacacat 480

ataagagttt ctataagtag tttggcataa ctaagaaaaa gtatagtgat ccacatctaa 540

aacaatataa tgcaaaacat aataatacat attggacatt tagattatcc ctaaactgat 600

tcacactaag caggacttaa gagctgaaag tgatcataaa ttctgatgaa atttgaaaat 660

tcttgagata caggttgaac tcagtggaaa atacattgaa tcaatcttac tgatctcaaa 720

aggcagggaa atgctgttga aatttagttt acataattat tttctctttt tctggctgtc 780

cttaaagcta acacttggca tgatactaaa aacattgttt taagagaaaa tctattgtgg 840

acaaatacaa gctttaggaa taaattaaaa tgtttagagg aattaacaca tctgtgtaaa 900

attttacatg tgtgccaaac tggtccccaa gctaaaacta gtgacatatt aaaacccaac 960

atattagctc acacatttac aatgaaatac aacacttttc gtaatactat agtttgtgac 1020

agttcaagaa gtgatgaatt ggaaaacatg ctatgatagg catgggcaat ttagagctca 1080

gtctggacaa tcggtgatga ttaatcttaa gaggatttct gaagttcagg caacaaatga 1140

ttaaaaatca acttcaacaa tagtcaatga tggctgaatg taagatattt aaacatgcat 1200

tggaaaatac aatggaacta attgtagtca tggacattga gagaagactt ctggattcag 1260

acaaattttt gctcactcaa atccctgtaa cattgtttac cttatattac atgtcataaa 1320

ttgactacgt tatgaaatag gaagggtaat atctattttt aaatgtaata attataaaca 1380

atgtataaaa tatgtgacac aaagaagact ggcagtttat agtgactatt gtattattat 1440

tttgatttta gtagttgaac aggaaaaaga gagaacagca cagactgaag tatttatatc 1500

tcaataatta aacttactta atattttcta ttatacattt acctaaaaag tacagggctg 1560

atacttccat atttatatgg aataacagtt cttaaaatat ataaagcaaa atattattga 1620

aataaagaga tgatcgataa acttgtaaaa ttatcatcag agatgtaaat accacctcta 1680

caataggtag accaaaatta gtaaagatac aaaataattt aacaacacaa taaacttgaa 1740

ctaattaaaa tacaaggtct ctatcccaaa gaactacaaa ataaatatcc tttttaagta 1800

gaaaaaaaat tcactaaaaa tatatatttc aagccataag acaagtctga ataaatttca 1860

acaacagaaa tcattcatag tacaaaccct gactacaaag gaagaaagtt agaaatctcc 1920

caattatttg taaacaatgc actcctaatc aacccaagag tcaaagaaga aatcagaatt 1980

tgcacaaaac agctgaaaga a 2001

<210> 43
<211> 2001
<212> DNA
<213> Homo sapiens

<400> 43
ctgtaatccc agcactttgg gaggccaagg caagcagatc acctgaggtc aggagttcga 60

gaccagcctg accaacacgg agaaaccctg tgtctattaa aaatacaaaa ttagccgggt 120

gtggtggtgc atgcctgtaa tcccagctac tcgggaggtt gaggcaggag aatcacttga 180

acctgggagg tggaggtttc agtgagctga gattgtgcca ttgcactcca gcctgggcaa 240

gaagagcgaa actcagtctc aaaataaata aacaaataaa taaataaagt gcacttctct 300

ctctctcttt ctttctttct ttttttttct tttttctttt gagatggaat ctcgttctgt 360

cactcaggct ggagtgcagt ggcgcaaact cggctcactg caagctccac ctcccgtgtt 420

cacgccattc tcctgcctca acctccggag tagctgggag tacaggtgcc cgccacctcg 480

cccggctaat tttttgtatt tttagtagag acggggtttc accgtgttag ccaggatggt 540

cttgatcttc tgaccttgtg atccacccgt cttggcatcc caaagtgctg ggattacagg 600

catgagccac cgcgcccggc caagtgcact tctttttcag taaaaacagt tggctgactt 660

caaggacatg tgaattaagt agcattacgt atagttccta gacaggggaa aaagccaaac 720

tggtggaacc actggataat gactgaggtt tgggaaactg aggctggatg agaaggttcc 780

agaggcctac cctaaatcag gaagcagctg cgcttggtag aaatagtgtc tgtttggtct 840

gtttggtagg aggcctgcct cccattgagc caacaataat cctgttgaca taaccctgtt 900

gatcaaagcg atcccctgac tgctctgggc cagttccttt ggctcctggt ggaatgcact 960

cagagaatcc ggatccggtc agtataaaag gtctggtccc gcagtggggg agccacatcc 1020

tggaagagtg gcctaggaca gctcctctcc tgccagagct aggcaggcgc cgaagtagcc 1080

gcatggcccc gtcagaagac cccagggact ggagagccaa cctcaaaggc accatccgtg 1140

agacaggcct ggagaccagc tccggtaaga ggcggcaaag ggaccccagc cccacagaaa 1200

caccagctgg gggccaggtg cggtgcctca tgcctgtaat cccagcactt tgggaggccg 1260

aagcgggcag atcatgaggt caggagttcg agaccagctt ggccaacata gtgaaacccc 1320

atatctgcta aaaatacaaa aaattagctg ggtgtggtgg cgggtgccta taatcccagc 1380

tactcaggag gttgaggcag gagaatcgct tgaacccagg aggcggaggt tgcagtgagc 1440

tgagatcgca ccactgcact ccagcctgag caacagtgtg agactccatc tcaaaaaaaa 1500

aaaaaaaaaa aaaaaagcaa gaccagctgg gggtgggtgg gggtctgatg gcaaacatgc 1560

tacctccctt gggttgctgg gattctccag ccaagggcgg gctcgtttta tttcctgtgc 1620

tagccaacct actagaacat aagcttcctt ctgagcccac gtcgtgtttc acagcacgta 1680

gctaagagaa tggatgtgta tgttatttgg gtaactcaaa aagttacctt tttaagagca 1740

caggctttgg attcagatac aattgctcag acacctgctt cattgataag tctattggct 1800

ggtttgtcca tccctctacc ctgtgctggg ctctgagcgg ggggattgcc agggcaaatg 1860

ccaccaggta ctgatgggat tctctgaggc tgtggacaag attatctggg gagggggaga 1920

gtagcaagat gctccctgag acccaccaac acttgtgcag gctgagcctc agtttcctca 1980

cctggaaaga tggccagggc t 2001

<210> 44
<211> 2001
<212> DNA

<213>  Homo sapiens

<400>  44
taaagaataa aagtgaatga aaaaatggaa gcattgcacc gtgcttttga tttacatttc      60

cctgatcatt tagtacaacc accatggaaa acagtgtgga gattccttaa agaactaaaa     120

gtagaactac catttgatcc agcaatccca ctactgggta tctacccaga ggaaaagaag     180

ttgttatacg aaaaacatac ttgcacacac atgtttatgg cagcacaatt cacaactgca     240

aaaatataga accagcccaa atgcccgtca atcaatgagt ggataaagaa actatgatac     300

atatatatga tggaatacta ctcacccata aaaaggaatg aattaatgat gttcgcatca     360

gcctggatgg gattggagac aattattcta agtgaagtaa ctcaggaatg gaaaaccaaa     420

cattgtatgt tctaactcac agtgggagct aaattacaaa gatgcaaaga cataggaatg     480

atctaatgga ctttagggac tcaggggaaa gggtgggagg ggggtgagga ataaaagact     540

aaaaaatggg ttcaatgtgt acactgctcg ggtgatgggt gcaccaaaat ctcacaaatc     600

accactaaat aacttaacca tgtaatcaaa taccacctat tccccaaaaa cctgtggaaa     660

taattttttt ttaaatggaa gcatccaaaa tgtaatttgt ttccacattc ttgtaagcct     720

cattatgtat cccagggaca gttaatatcg tcatacgaat tcacaggtga ggaaatggaa     780

tcacagagag attatgtact ttgcataagg agagcgaggg tgaggcagga tttgcagtct     840

agaagcctgg gtctggagtt ctccactttg cactatatca tttctgtaaa cttcttactt     900

aagactgtgt ggaaatgtag agtgagacag aactaaagct cactattctt agtccacagg     960

ggagtagtga ctacccaggg cttggtaagt gctcagtaac gtttgttgaa agatgaatca    1020

atatttcaat gctggggcaa agcagtgaaa aactggggaa tgtccaggaa ccagccatca    1080

ggataaggca ggactagaat gaatgcaaca gtcctcacag gtccaccaag tcaacaacag    1140

aaaaatccaa agctaaggtg gctcttgtgt gatcaaggac ctcttgtaca ccgacctgtt    1200

aggctgtggc acagtcaaca tgggagttgt agacaagcta cttgagagtg gttaatggta    1260

gcagtcaggc atttggagct ggaggcaagg ctgattctcc tcaactagta tcatattgag    1320

cggggactta aagcctcaga agtctgagca gaaaagctga gaccaggctc cttgggacag    1380

gctgttcata tgcatgattg agtttcagtg atgagtccca gaaggaaaaa gtttatgcat    1440

gttttcctta aaaatatatt cacttatatt tagagaatcc ttcagatatc taacctaaat    1500

acgtctgtaa catttactct cattcttttt atgacattat tagtataata atatgaataa    1560

ttcccagtgg acttacttcc acacttcctt caaggattct ccattttatt tcataattga    1620

agactgttaa taaatcactc tctagctaag aacaattcaa tgctaaaaca cttagcttat    1680

ctgaccttca cattaattat ttttatgaca gcagcaggaa ctttcagctg cttttccccc    1740

ttccttatga agccatgtgg tcccaagcag acagagttcc cttgctacac ttaagggtgg    1800

gcttctcaga cctgagcata tcttaaaagc tttcccttga ctgtgccaat ccaaactcca    1860

agtttcctca gcagatttaa ggggttttaa atctacttga gcatgagtgt tatgagaaga    1920

ctttgaaatt cttacaaaaa ctgaaagtga aatgaggaag acagattgag caatccaatc    1980

ggagggtaaa tgccagcaaa c 2001

<210> 45
<211> 2001
<212> DNA
<213> Homo sapiens

<400> 45
gtccagtaga catcaatcaa actggactca gtttgcacac acctggagct caagagtctc 60

cagggggaaa acagagacac aaagtcagac agagagagag ccagagaaat ttcctgcacc 120

gtgaagatag tcagaggcag ggaagaaact ccttagcact agttagagtg atcagaaacc 180

aagaggacct gatcgctgta cctgccaggt ctcagtttct gtctccttcc aactgaccac 240

ctcttcctct gagactcacc agttctgcat ctcttgctcc tccttctgtt tctccgacca 300

cttccacctg tggctgtcac agaagggcgg atgaaggagg ggacactgga gatagactca 360

gcatctgcag gcttccaaag agaggggcta ggagatccac caacacacca gcacaaatac 420

accagcacac acagatacac acaattggtt catgtattgc taggttacag tttgctatgc 480

tacaaaggca gtaggccaaa tttgattgaa ttgaataatt ccttattttc atcagcttct 540

cctttttttt tttttttttt tttttttgag atggagtatt gctgtgtcac ccaggctgga 600

gtgcagtggt gtaatcttgg ctcactgcag cctccacctc ccaggttcaa gtgattctct 660

tgcctcagcc tcccgagtag ctgggattaa aagtacccac catcacgccc ggttaatttt 720

tgtgttttta gtacagatgg ggttttgcca tgtgggccag gatggtctcg aactcttgac 780

ctcaattgat ctgcccccct cagcttccca acgtgctggg attacaggtg tgagccaccg 840

cacccagcca gcctctcagt tttgaacatg cactaccacc acctccacaa cacacaaatg 900

taaatgcact ttcgtatata aaactgtata aatacaagga agctcataca catgcaagga 960

tacacacata agcaccccca gattcaacca cagaaatata cgccagtaca tttgcataaa 1020

ttcaaacacc cctttacatg taaaaatcat ataagcacat acagggatgc aagcaggcat 1080

ggacaaatgc atgcaagcac agacaaacag acaaagctaa gtaaaaaagt gcaagctcac 1140

ctatgcttac aaaaatagac atacatatac ccacaaaccc acacacccac acattcactt 1200

gctcacctgg actttgatat ctctaccact gtatccctgc caatatctac agagtgggta 1260

aagggatagg catcaggtca ctgggttgcc caagcaggaa gtctgggttc cctaacaact 1320

ttttctaagc taatgctcct ggatgatgat gaaaaaggag gtggggaatg gatgaaattt 1380

tataacaggg tgcagaggca gggtcaggat aaaaggccca gttggaggct gcagcagggt 1440

gcagggcagt cagaccagga ccatggaact cagcgtcctc ctcttccttg cactcctcac 1500

aggactcttg ctactcctgg ttcagcgcca ccctaacacc catgaccgcc tcccaccagg 1560

gccccgccct ctgccccttt tgggaaacct tctgcagatg gatagaagag gcctactcaa 1620

atcctttctg agggtaagac acagacgaat ggggtctgag ggtgagctgc ttcttgcctt 1680

ggtacttggg gaagcttcac caaacagaat gaggcagact tccagagtca ggggtggcac 1740

gggcatggtt ggtgagtacg gagcatggtg aagcatgatg ggtggtatta ttaggagaaa 1800

agcatcaaat taaatttagc agagtttatt tgagcaaaga agtgactcat gagttggaca 1860

gctccctaaa ccaggaaaga caccacacgg cagtatggtc aagtggtatt tacaggcaga 1920

aaaaggaggt gacatacaga aacagcctga ttggccacag atcacagctt gccttacttg 1980

gtcacaatct gagcagtttg c 2001

<210> 46
<211> 2001
<212> DNA
<213> Homo sapiens

<400> 46
agtaatctag accttcctct tcctatcaca gtctcaagga ttgtgccccc aaagcagatg 60

caaagccctg tctacccctt ctccttcccc tgaggtagag ctttatcgaa ggtttctctt 120

ggtaaacgtt agctttctga actaagggcc gtcttttttt tttttcccgc ttaagcccat 180

tgcataacaa tctcttgttt taatcttttg cccaacactg cttttcttac tgtcagaggt 240

tactacaagt gtttgaacag gtaacttcta cctttgaact tctgttgtga cagattcaaa 300

ggttccaggg gagtgtgttg gccactctca ggacagtaca cagtagcttc gggtgtgtcc 360

cagtcagtcc taggagctgt ggaaagagta gaagtgcctg aatgtggtgc tgaatcaata 420

cagccagctg tgaggggagc acttcctgga cccaggaagg gagagtcttc ttccaaggta 480

agctgcaatg atactacctg cactatattg aatatatctg tgagatagta gggaggggat 540

tggtgtgggg aaagaaagtg ggatttgtta tgctcaacct aaggctcttt aataacatga 600

aattacattt agttgaatag taatctcagc tgacttgtgt gggaggcaaa gaaaaaaatc 660

agttggaaaa ggggtggaga tatatgagct atccaaatgc tagcagggga tggaattgga 720

agggaacact gattttggta agtgtactaa agcacaccaa gccagaatgt gggtctcctt 780

catcttccat taagaaattt ctgtctcagt gtagttagaa ggatcgtgcg tcagctgctg 840

agggcagaga gtcccatttg gcagacagag aggaagggaa tcagcagata ttcgattagg 900

tgctggggac tgttctgggt agattatgta tgttacttca ttggtgtatc tcatcctata 960

aagcaggtgg tatccttccc atttcacaga ggcatagtgc gtctccaggc tgttaaacag 1020

catggcctaa gctacatagc tccaaggtgg tgtggctaga atttgagact tgattggtca 1080

gtttccacag cctgcccaat aatgcagaat tgttgtgggt tgccaaagct ttcagttacc 1140

ccctgaaatc ctgagggtgt cactcatgtg cacagaggat tgataggaat acaactctga 1200

tgggccttta gaatgactca accttcctga acttgagcat ttttactgga ggaggtcaat 1260

aaaaaaaaaa gaaataaaac ctcatcttcc cctggccctg agccctttgg tgaaggaaga 1320

cagcacccgg tgaagggctc agaggtatta gtgtggctga gattcgacac ccactctgcc 1380

atcctctgct ctcctctaga ggaagttgcc tcttggggag ctgagttcct gaaactaagc 1440

aaatgacaag tgctcctcca gccgtgagat gagccagagg atggaatgcc acaggcccca 1500

cttcgtctcc cagcccttca tctttcttcc atgtccaaag gcggctcccc agaggccaag 1560

ggctcagctc ttgtgttctc tgagactgtg gcacagctca ccctggcttc cttagtagga 1620

agcttttaag aactgctctg tagaaagaac aaaccctaat ctagctccag caaagagcca 1680

ggatcatgct gaacttcctg tgagccttcg tgggggatcg aggctggaga aagtgctgct 1740

tcattctgag gtatctgtga ggtggggaag gggcaggaaa atttgtcatt tcggcttcct 1800

aatctgagaa tggtagctcc agggcccccc tgtagccaca gccttaggcg gatcaaaaat 1860

gagactaata caggtcctgg taatactggg ggtgaccaag gcgaaagaat cgatttttaa 1920

gttaagtttt gaggtgtgac tgtatcagag ggaaagaaac catgccaatt gaacgactca 1980

cagccctgtc gttcctgact t 2001

<210> 47
<211> 2001
<212> DNA
<213> Homo sapiens

<400> 47
acaaattaca ccattccatt caatgcagca atatttcttc atgcttatgg tgtgctgtat 60

ctgatgataa aagcacaaat aagaggattt acatttctat cttggaaata cacaatatct 120

ataggcaaag agacaaaaga aaaggcattt catttgaaag gcataggtca gtggtgagat 180

ctacatattc tcttgatact ctggaataga ttgggtgggg aaggcaagat acctgttctt 240

aggaagtcac tatctcactc tggttctata ttatctgtag ttctacactg tcaaagagga 300

cattgaacct ggtgatatgg tttggctctg tgtccctact caaatctcat cacacattgt 360

gatccccata atctccacgt gttgagggag agacctggtg ggaggtgatt ggatcatgga 420

ggaagtttcc cccatgctgt tcttgtgata gtgagtgaat tctcatgaga tctgatggtt 480

ttataagtgt ttggcagttc ctccttttct ctctctctct cctgccgcct aatgaagaag 540

gtacttgttt ctccttctcc ttccgccatg attgtaagtt tcctgaggct tccccagcca 600

tgcggaactg tgagtcaatt aaaactcttt cctttataaa tgacacagtc tctggtattc 660

tttgtagcag tgtgaaaatg gactagtaca cctggtgaca tcaagacccc cagcatcatt 720

caagatttat tgtctcttga acaatatgaa tgactgacta aagtgcctca aattctgtag 780

tttgcatgtg ggatgagtca aaagggggtg agaatgttaa caaggcacat gacccatggg 840

aatttgctgg gcatatcctc agtcacagag caatgagggg agagtacagg caaaaggtgt 900

aagcagaacc agccacaatc tgtggagaag aatgagatgg gatgatgttt gcttggctgc 960

taacttgata ataagaatgt tagcctttgg cttcatttta cgtgaataca gggagttcca 1020

ccacaaagtg ggtaggatta tgtcccctaa aaatatagtt taagtcctaa tccttctacc 1080

tgtgaatatg accttgtttg gaaatagggt ctttgcagat gtggtcaaat taagatgagg 1140

ttgtactggc ttaggatggg ctctaattca gtgactgctg tctttaaaag aagagggaaa 1200

tttggataca tacatggaca ataaagagaa ggatgtgaag atggaggcag agacttgagt 1260

gatttgtcta caagtcaagg aatgtcaaag tttgctggca accaccacaa gctgagagag 1320

aggcatgaaa cagattttcc ttgagagtct tgaaatgaac caaatctgct gatatcttga 1380

ttttggacgt ttgtccccca gaaccatggg agaataaact cctgctattt aaaccactca 1440

gtctgtggta atttcttatg gcagccccag gaaacgaatt cacactacta aatgaaaata 1500

gatctctgtc tatcaatatc aacaccaata tttatataca gggatgataa tgaccttagt 1560

tttccagttc ttaatagcat actgaataat tgtgcatgat tgtatttcag gtatgtaaaa 1620

taaaattgtt tgttcactta ttttcttcca atcaccattt tctctttctc cttttttttt 1680

ggttttagat cgttataagt tttacaatgg actctcttgt tacagcaaac accaaatttt 1740

gctttgatct ttttcaagag ataggcaaag atgatcgtca taaaaacata tttttctctc 1800

ccctgagcct ctcagctgcc cttggtatgg tacgcttggg tgctagaagt gacagtgcac 1860

atcagattga tgaggtacgt gtccactagg gtgctacaca gggtcctaaa ctctgggtcc 1920

acactcaaag tcagacccta atcccactta ggcaagccaa ggggcttatt gcaccctggg 1980

cttggtcaga acccatccct g 2001

<210> 48
<211> 2001
<212> DNA
<213> Homo sapiens

<400> 48
tttattctcc ccaaccaggt ggaaatgtac cctgtttagg cccaggacct accttagcaa 60

ccttgattgg ctcacaagcc aggttgctta agacttgttt ccccagcagc tggtgtccct 120

acttcctgcc tagccctttt ccttttgctt tgtctgtgct tgggaaatac atgttatact 180

ctgtagagca gatttgctgg cagtaagaac agagatgtgt agagcccagc gtgctggagg 240

gctctctgca tctggctgtg agtggcccct ggactgggac cgtgggagga cagctttcca 300

tagtttaaca taaatgaacc acaccataag gacaatctgc tctcatttca ctcaactagg 360

atttagtgag tactgttaaa gcacagtttg cactggagta attggagaat ggagtttgcc 420

cttcacaatc tgtgcacaat ctgttagcag ctaaaaacaa atgttagcag ttataattac 480

catcaatttg tctccaggct tggatcacat tccaggtgtt agaagagatg gtccatttcc 540

ttttttttct cctttaagtc ccatgccctg tttcaagtag cttcagaagg caattgtgct 600

gtaaagattc accttgtaat gggttcagga ctctttcaca gtttgtgtct attcgttcat 660

ttgaactttg ctgttccaag ttagaaagcc cttgctgagg tgtggctacc tctaagaaca 720

ctgttgattg ggtgccatgg taacgtttgc aaagtagcta ctgctaggat acagctctga 780

cacttattac tttatttgca tatcaagcca cctgttccaa agatgaaaat atacaggact 840

gcatatccca gtctgggtaa aaggataata gctgactgtt gaagagaaca tgttgcagtt 900

tttctcctgc tagctgaaga atcaagttct gagggcctgc tcctcagcac ctttctcctg 960

gtctagagta ggtgccaggc ctggcggttc tgttgcctgc gtgggtgcca gggcttctcc 1020

ttgggaggct tgccctctgt gagagggagc agagagttga cttcctccaa gaggcccata 1080

gtccctgaag ggggtgtttt tgattctgtc taaacctagg ctatgtgtta gcggtttccc 1140

tcctcttaga aattgcctgg aagactcttg ctattggtat gtatgtggtt ccctccagtt 1200

taagacaaga tttgttccag gatcggttgt agccccttct ctgtggctaa cggaagagcc 1260

acttgtcctt ctccattctt ttccttagtc tcccttgacc cccacatcat ctcctagaca 1320

caaggcacat tgatagtcac aattcactgt caatcactct ggattattcc ctcattcccc 1380

tttcccaggc cacctgaagg agtgagtggg gattgctatt gtgtacattc cagtaataac 1440

tattggctgt aagcagacat actctaaaaa taactcaggg atactgagca cccagagggc 1500

accttctgtc ttctcgaagc ttcgccacgc ctgtttccac tgtcagactt ccagtctgca 1560

ccacagcaca catcctttct catccagagt gctcctacta gacgagatgt ttttgtctcc 1620

taagggcctc aggtgtattc atgaactcct cagctggtta ttttcagttt catcttcctt 1680

ggtgcttgct ctggcacatg gtcactgctg catcctgtcc cttccccaca gtttgccatg 1740

ggtcatccct tttttctcat tgcttgcatc ttctaactga atgaacttgt aaatatggaa 1800

ctgaaagtca atcctcatag ctcatctttt atttaatcct aataaatgac aatctattgc 1860

aaacattact ttaaaaatat tacttatcaa aagctgggtg aaaggataat agctgactgt 1920

tgaagagaac atgttggggt ttttctcccg ctagctgaag aatcaagttc tgagggcctg 1980

ctcagttctg taagaaagta t 2001

<210> 49
<211> 2001
<212> DNA
<213> Homo sapiens

<400> 49
ttaggctcat gtgcttcccc ctgctccccg ccccctgacc ttggaccccc attcaagggc 60

atccctgtct tccttgactg acggttaaca ctcccaagtg catggcgctg ggctgccccc 120

atcttgagtt ctgaacagca aagcctaatc tcagagctgc tgtgggactg tgactcccaa 180

acctggtggc aggtgctgcc ttcagccccc acctgagtca gggaccgcca cacggccccc 240

tgccccaagc cttgggttac cctgtagata ctgccctggc ttatgtgttc actggctcct 300

gtgccccaac ccaaaatgcc gccaccattg ccttcctcca agaggacccc cagtatctac 360

tgctgcttct cagagccagg agctctgagc aggagcagtc aagaggggtt gccggtctgc 420

cctcctctgc tcttgtgcta cactacagag ggataggcaa agggagctaa cagcgtcgct 480

cagagacaaa gaacacagaa gcaagatggt ttccttggcc agcagggagc tctggccctg 540

ccttctaggc tccctgggag tcctgcgcgt caggcaccct ccttctcttc cgctgcaggc 600

attgctgact ttgagcactc accctggctc tctgatccca gggccacccc ttcccctggg 660

tgcgctctcc ctcccacact ctgctgggca tcctgtgtga aaacatcatc ccttaggctc 720

tgccctgggg ctcctaagga acctcacgga agattctgct ccttgctcac agcctccaaa 780

tggccaggaa aaagtttcac ctctcacaac agttttgcag gagagatgcc attctgtggg 840

acaaaatgct gtgggagtat tttaggaatg ttctaccaac aaaccaactg cacaggcaag 900

aaatggtttt ctccagctcc aagacctcgc tttctcctta aacaaaagcc ccaggttctc 960

tgcctgggat caacctacat ctgaagttct gggctgcaga cgcttacctt gttcagtcct 1020

cgtggagctg ggctggcctg tgcctcgccg tagactgcag actgtgaggg gcagagccga 1080

ggggaggcta tgaaaactgt gtccagcagc aactggctgg taaaaataga acagacccac 1140

tgccccctcc tgctgcccct cctccctccc acccttcaca cacacacaca cacacacaca 1200

cacacacaca cacacacaca cctcacaggg cagatgcgtg ctgcaatgca cacaacaggc 1260

acataggcgg aaagccatgt gaatttgtgg ctcaggagca gaggcaaatt ctgtgcacat 1320

aggtgccacc aactgagtga aaccagcacc cacctgggga gactcgagct gcagaaaggg 1380

aaagaatgag cagccagacc aggtgtgggg actgagcaga gatgggactg ggagggggaa 1440

tgagggagag caaagggcca cagtgaaggc ccagaggggc aagtagcagc tgggggaaga 1500

ggaccattgg gctaaccctg catctcttat aaggcaatat ggaaatgagg cccctgaacc 1560

catgccccaa aagcccaaga gggctaaaga ctcagctttc agggattttt cagtggctct 1620

ggtgcacgtg ggatacgagg gtgcctggct gtggggagtg atgtgagcag tgttgcaggc 1680

tgaccccagt gagtgtttcc agggctgagc agaatggcag tggcagcagg gcacaccttt 1740

atgctcaggg aggtctgagg gtctgcagac actctgcatc cccctccaga tgggtttcct 1800

ggaatctaga tttcccaggt tccaaaggac acccgagtct catgcctgga actcagtgag 1860

actaattcac ctctcctctg ccctaatctt catctccagc cagaagccaa cagatcccag 1920

gggactggag ccacaggggc tgcacctgtt taccgggtat ttttaggatg gttgatgaac 1980

acataatacc caccctatag t 2001

<210> 50
<211> 2001
<212> DNA
<213> Homo sapiens

<400> 50
ggttccttaa ctttgcctag gtttttcttc ctgtctccag tgacagagag aaatcacccc 60

tggggttccc aaaggtgcat ccccgtcatc atcttcactc actgcatctt gaagcatatg 120

gatgaaaccc agcaggctta tcgcatagct tgtggtctgc cctggggagc caaggaccaa 180

gagccagaga gatgagaatc aagataaaaa taggtgaact accacctacc aagtagatga 240

ctacatagga atggatgtgt gggcaagtgg agccgtctgt gtgtgtgtgt gtgtgtgtgt 300

gtgtgtgtgt gtgtgtgtgt attgaagaca agggcagggg ttccagacct ccctgcattt 360

gagccccatg ccctctctta gcatacacac agcctcccag gtacaaacag tgccctgttt 420

ggaaagtaaa catgccatag caatcctggc acctctgtga agtgacttag atgcccacat 480

gcaccaccta caatgctacc tgagtgatga atgtctctgg gatgtgagtg tcagctgagc 540

acgtctgttg ggaaatgggc ccctcccaag agcctgaacc tgaatacttc ttataaaaag 600

gttctctggc tggacgctgc tcaatccact gcctagcagg tggcccattc cagttggaga 660

acgtagtgag tctttcagtg gagccagggt ctggtaagtc tcctagcagg gtctggggga 720

gagggtggga tgagttgagg gcacagggga ggagcaaaca gaacacaggc agcagataga 780

tgtggaaggg gtgtcccgca aggcacctac gcttctagtg agccaacctc ttaaggtact 840

aatgaggaca taaaggcaac ataggaggtg gtttttgcaa gggtacacag caagtccgca 900

gaagagctgt gactagaacc caggtctttc agctccctgg atggggttat ttatattggc 960

acaactctcc tttcccacat ggttgtgggg catgagaggc gtaggcaaga aagaatgatc 1020

aggacatgct tctggcctgt ggattattta gacccagcag gtgggaccgc ctgttggact 1080

tgggtgtttt ctaactgatg ggggtgtagt aactaggaag atacagtcga aggtcggtag 1140

gaaagtaaaa tgagggacaa gaaaaaagcc aagtggagat tttggactgg atcatacaga 1200

taagttgccg cagtgtgctg tttattttcc actgtggaga acttgagcag tcttcttcta 1260

gtcagagaaa atggaaatgc attgattcat tgcatggatt gtgtcctgct ctgagcatgt 1320

ttaactcact ggaaacacct gcttggattt gcttctttgc gtccgctctt tctgggtgct 1380

tttccctcct tgtcccaggc caggctgact tgtacactaa gccgaatctc aaaagcccag 1440

acagagttcc attggtggaa agaaaaagcc ggctcgcctt tgaaaggctg cctttcttcc 1500

aggtttgtcg tgaggagctc cgcgatgtcc tctcaacaga gcgccgtttc cgccaaaggc 1560

ttttccaagg ggtcgtccca gggccccgct ccgtgtcccg ccccggcgcc caccccggcg 1620

cccgcctcct cctcctcctg ctgcggctcc ggcaggggct gctgcggcga ctcaggctgc 1680

tgcggctcca gctccaccag ttgctgctgc ttcccaagga gacgccgccg acagcggagt 1740

agtggttgct gctgctgcgg gggcggcagc cagaggtccc agcgctccaa caaccggagc 1800

tcaggatgct gctccggctg ctgagaggcc cgcaaccccc agcgctgcgc tagagaaacc 1860

cgcccagccc agagcgggcc cgccccgctg cggctcccac gcggggctgg gcctcggagt 1920

ttgccccgta aagcgaattg cactttgatg ttcagaaacc cactttgttc tcagccacgc 1980

aaaactccct gaccccgatg t 2001

<210> 51
<211> 2001
<212> DNA
<213> Homo sapiens

<400> 51
gcttccgcag agtggggagg ggccgccagt gcgcgccact tttcgcctgt ccgggcactg 60

cgccagcgcg gggcggcccg gaggcggggt cgcgtctctg cgcctgcgcg gcgggaagcc 120

agtggggcgt tgtttcgtcc gatatccgcg tttcagtctc cgcccatacc cctccgggtt 180

aggcggctgt agcggagctc gaaaagagtg gcgcagggtc gcgcggcccc gcctccttcc 240

ccgcccagcg aagctctctg accacccctc ttttctagag ttctgcctcg cttcccggcg 300

cggtcgcagc cctcagccca cttaggataa tggcgacagc tgagggtgag taacagggat 360

ccgggccctt cacggtctcc ctgaccccca ggccgtgagc tcgcggccac gtgcgagccg 420

cgaaactctg tcccgcctcc ctgcagcagc gcctctaggc agccgcgcgg gcgcacacgt 480

ggcccaggcg ctcccgggcc tccccgaact ttactgcctc ggggatgggg agaaggagca 540

aagtagagga gaacgagaaa tggccaaggg gtggaaaggt gcctggaagt attatttata 600

atcaatagct attgcaaaat acaagcaaat cagtcttgat tcgtcaaaac ctgagtcttg 660

ctttgcccgt taggttaatg acctttctaa gaaggttaat aattgaaact tttgaaagac 720

ctgtctagag gccgggcctg gtggctcacg cctgtaatcc cagcactttg ggaggccaag 780

gcgggcggat cacctgaggt cagtagtacg agaccagcct ggccaacatg gcgaaacccc 840

gtctcttact aaaaatacaa aaaattagct ggacgtggtg gcgcgcgcct gtaatcccca 900

gctactcagg aggctgaggt aggagaatcg cttgaacccg gaggcggagg ttgcagtgag 960

ccgagatcgc gccattgcac tccagcctgg gcgacaaagc gagactccgt ctcaaaaaag 1020

aaaaagaaaa acctgtctag ggggcgtgtg tgtaagtagt ctttgatgcc atggtgtgtg 1080

tgtgtgtgtg cacgcgcgcg tgaagtagtc tttgatgcca cgatagcttg gggtgtgtgt 1140

acgtgcgtgt gcgtgtttaa gtagtctttg atgtcatggg tgtgtatgtg tgtgcgcgcg 1200

cgtgttgaag tagtctgatt ccatgatggc tggggagggg ggctgttttt aggtagtctt 1260

tgatgccatg atggtgtgtg tgtgtgtgtg tgtgtgtgtg tgtgtgtgtg tgtgttcgtt 1320

ccagtagtct ttgatgccac gatggctttg aagaactcca aaacctttag gcaaaaaagt 1380

tgcactatca ggaaagattg ttcttgctat ttatgctgga aaaaaacaaa tgctggtttt 1440

ttccacacac ccagttgcta tatgtatagt gaggcaaatt gcttttgtgg ctgctgggta 1500

cattcttttc cacatagaaa gtaactgaat tcagatttta aaaagctgta gactcaggcc 1560

gggcgctgtg gctcacgcct gtaatcccca gcactttggg agtcccaggc gggcggatca 1620

cgaggtcagg agatcgagat catcctgacc aacatggtga aaccccggct ctactaaaaa 1680

tgcaaaaatt tgctggacgt ggtggcgtat gcctgtaatc ctagctactc gggaacctga 1740

ggcaggagaa tcacttgaac cggggagtca gaggctgcag tgagccggga tcgcgccact 1800

gcactccagc ctggcgacag agagagactc tgtctcaaaa aaaaaaaaaa aaaaaaaaaa 1860

aagctgtaga ctcagcaaaa aagattcaga tgatattgag tatcaactcc ctagaatcaa 1920

ggcaaggacc agaatttttc ttgcaatcat ggtacctgta cctctaaaat ggcaaatctc 1980

taaaatcttt gctcttccac t 2001

<210> 52
<211> 2001
<212> DNA
<213> Homo sapiens

<400> 52
accctctcaa ctctgggtca gggtggctgg tccccactgc tgtgtggaca tgcaagtgag 60

ggagtctctg ggtcagctgg aatcatgaag acgagagttc agggagggtg ggagtggtgg 120

agcctcagca gagaagttga cggtcattga tgttgaggaa gtagattgtg tggagggagg 180

agaatggcat ggtgtttgcg ctccccacca gcctcactta cacttgtgga cactgcggcc 240

ggtgtcctga ggacgtgtgt tgggaattca gccacgggga atggaggtcc cttggttcat 300

atagtcacag gctctgtatt ctaaccccag ctttgtaatt ttccagctgt gcaaccctgg 360

gcagtggctt agcatctctg agcttgcctc cagatccgga tctgtatgat ggggttatta 420

atgcttcata aagtaggtgt gaggagggat tgaggtacta ttgggagaaa ctttgtaact 480

aaaaggcaat tgtatcattt gcattattac cagtgacatg agacatagtc acacaaaaat 540

tgaaatgtca tttgttttaa aaccagaaat tgcccaactt agggccctcc tgtctctcac 600

tgggtgccct gcaggggacc aagagccaga gcaagactga gaacctgggg cagaagcgca 660

gggaggaagg aggtggggcc tgcttaccaa ccaggcctcg gccccttccc ctttcaatcc 720

caggcccctg catggcaggg agaggccagt tccaccatgg caggttcttt ctaggctggt 780

gtgcagggtg ggggcctcca aggagcaggg gcatggggaa aggggctgca ggcgctgggc 840

ctaggaagag aggctcttat cttgtgggca ggaagtgtga ggtgttgagc agctagggaa 900

ccctggggag gcggcgggga gcaggagggg tgtgtgttgt ggtgaggagg agcggaagtc 960

ggaagctcca gccgtcacag ccacattcac tgggcaagcc gactgtgagc caggaagtgc 1020

tcttggggag cccaggccaa gccatccatt cttgggtcct ttggaggtga gctaagtggg 1080

tctgcctagg ttggggctgg tggaacctgt gggagcaggg aatgtggaga gtcacatgtg 1140

ggtccttgtg gcaggggctg gggcaggtgg gcaggggaga ggcgagggct ctggcagtgc 1200

cgtgtgcacc tgccatgagc ctgggctagg ctaggcccct ccttccccct tcctgctctg 1260

tgtcccgtcc ccctcctccc tcagcctacc cctcctcatc tgacccttct ctgattccct 1320

gccctggccc cacggacctc tgttgtttcc cttccctggc tccccgcaga gcctctggag 1380

gtctgcaata gtgaagggtg aaggaagctc cttgctaatc ctcttgtgag tgtcactgcg 1440

tccccagtga ctgctctggc tgagtaccca gtgggggctt ttgtggccaa aggtcccaag 1500

tgattctcga ccctagactc tggagagagg gagtctggaa ggcctgggtt tggggtgggg 1560

gtggctctgg gtgtggttgc tgcaggagct cagagtcaac ttggccatca gggggctccc 1620

agcttcatct tgccttccag gaccatcctg gagaagctcg aggagaggta aggggaccct 1680

ccttactggt ttagctctgg cttaggcctg ttgtcctgat gggcaacagg aaccactgaa 1740

ctctcaaagt gtgcctgtgt aggaaactaa ggcccactgt agtcattatt gttatagtga 1800

aggtagctgc atggagggaa agtcccctga cactgccatt ccccccttat tccagggcct 1860

aaaattccac agttgaagtc actcctctgc tagaatgcag ggaagggtgg cctgtcatta 1920

gctgatgata aatactcctc cgttcattgg accttggacc agctttaaaa agtggtcccc 1980

aagcaggcct gtctcccagc t 2001

<210> 53
<211> 2001
<212> DNA
<213> Homo sapiens

<400> 53
cctcacccac cccagcctca ggatgtcctg gggcccctgc tcacgccatc atgatgacac 60

tgaagtccag ccagttccag gggtcccgga ggaatgtgaa gtcgtcgaca cagaagcctc 120

gggccagtat cttgatgagg gactcaaagg tgtagatccc tgtgaaggtg tacctggggg 180

ggagagggcc ggccgggaca ggcatgtcac ctgggtaggg gtcagtgtgg caacgacaga 240

cccccagaag ggagggacac agaaatgaaa tatgccactc aggtggctaa ggacactagc 300

gagtgatagc atggccaccc cagggactca aggtgtggat gagggtcaca atgacagtgt 360

gtctccctga aagacaagag cagcaccaca cagaggtgca aacacctgag atgggctgtg 420

tcccagggct ggggagctca gggagcaggg agacttactc cacattcttg gaccagggag 480

gcgggtcact catggtcatg aatacgcagt tggtcaagat ggtgatcatg atgaacatgc 540

tgaacagcgt ggggcagggt caaggaaagt gaggaagcag ctaggatggg aggtgataga 600

gggtctcctg ggccacagca ccccatctcg cccatcccta acccctcccg cctctcccat 660

cttgggcagg atatgcatgg atgagcacct tgatggcccc gcgcctgact acgctgaagg 720

ggctcagcag gtagagagca ggtgtggcgg agaagcggaa gatggccttg cccttgttga 780

gtacgatgaa ggtctaaggt gggagagagg ctgtgagacc cagggacaga cagacaggca 840

gacagatgga tggatggcag acagacagag gaagccttcc caggcccaga ccttcttatt 900

gctgtagtag ggatccaggt cctccagggg gatgccgatg acctccggcg gggggtctcc 960

gtagatcatg ggtaggttct tgccagcctc caagtcactt cgtggcttcc gttcgggctc 1020

ctcaatctcc atctgcttat tccgctgcag ccgggcctcc tcctccaccg cccgctgttc 1080

tatggctgcc agtgactccc gggtgaaggg gcgcaagcac tcagggccca gaggcaccag 1140

ggtgcacaga gatggtctgg ccatcctcgc atcctgggct cagagaccag aagggtggtg 1200

ggtggcctgg ggagctgcag tgcgcagccc cggggtgctg ggcggccgcc cctccctggg 1260

cgggccgtcc actcctcacc tcctgcttgc ccagtggccg agccggggag gtgggcgtgc 1320

agactctatc aggactggga gatgtgcctg caaggtgctg ctgctgcaca ggtgctgggc 1380

acagagcatc acccctggcc cccccaccac actttcccca gaagtggcag cggctgccct 1440

caggaccgac ggcaaaccaa ccagattctt tgtggccttg ggacttgggg tcaacacccc 1500

agtgccttcg actgcctcct gggcccaagc tccgggaact gtgtggcttc agcctaatgg 1560

gacagagaga gaaccacagc tggctgtgct caggcgacct ctggataggc caggatgagg 1620

ggcctggttg gggggttaga tcaaagtggg actggcacag ggacttcacc ctgagggccg 1680

acgtggatcc tgatattggc cacctggaga tgtaggtgga gaccatgaag gtggcagcca 1740

ggctctgtgc aaaaaggttg ctgtgattga ttagtgatgt ctgccagggg cgcaaggagg 1800

aagaccgtgg tgcctctgcc tgtgtttgcc atccctggcc tggagggaag ggaaaagaaa 1860

gatgggggtg ggtgtggatt cctctttcct gatctttggg gtacttggtg ttgggctggg 1920

agagggaaga agaaagggcc ctgggcccgg caggggtctc ctagcagttc cctcccacag 1980

ccctgggggc tctagcccac t 2001

<210> 54
<211> 2001
<212> DNA
<213> Homo sapiens

<400> 54
tgggactcaa gccctgccat tcagattctt ttgttccaca gggtattccc ttgatgtgat 60

gttcttcccc ttcctctagg aatggacttt cctggaagcc agactacagt gctcttctgg 120

gtctagccac ccagtgggcc taccagactc tgggctggtg ctagggaatg tctgcaaggg 180

atccagtggt gttacctgtc ttcaagactc ccagcagtga ataccagcac catctctgac 240
agaagtggga ggggagtgat gcaaactctg caagattcct tggttgtaga tagacttaga 300
gtattgactt tctcaaatgc tggttataat tatagtgaac ttatcacgtg gacagacgca 360
ggacttctgg ttagccaggg tgttgtaagc agtggtgata gctgagaata tgcagccgtt 420
ttctccttcc tgggtacagt gctattctgc ctagaggtga gtttttgcct gtctcacaga 480
gtaggctgca gcctgccact tctttcaaaa gttctgtgga ttctttcagc tttcctgttc 540
agttcctgca ttcgttcctg gagaaaaaaa aattatagtg tgaatctcta cacgctattc 600
tgtccttcca gatgggagag gcatgctaat gctgcctcca atctgttgtc ttggggggaa 660
agaaagcaat ttgtttttgt tttttttttt aatggatttg ctgggtttag taacccagag 720
gtcattggga accttggcga aagttatttc agtggagcag taggaaggaa gccagttgca 780
gtgtctttgt tgagcagcag aggggaggtg aggaagtgga gagagcagct gtggtggctg 840
aagggtgcta ggacagaggg agcatgataa agccataagt aagatgtggt agaagggaaa 900
gagccaggac agaggggaga gaaagcagga tcctcaagga ccagagaggc agtgggtccc 960
acagtcaggt gcggggattc aacagaaaca gaaggaggga cgcttctcac tctgaccccg 1020
gaaagaagga agaaactgcg tgcgagttca gacaagtccg ttggcggggg ctgggatgcc 1080
tgcatcttcc ttgttaaaaa aggaagtgca ggttcaaaac attcagagac agaaggtgga 1140
tagacaaatc tccaccttca gactggtagg ctcctccaga agccatcaga caggaagatg 1200
tgaaaatccc cagcactcat cccagaatca ctaagtggca cctgtcctgg gccaaagtcc 1260
caggacagac ctcattgttc ctctgtggga atacctcccc aggagggcat cctggatttc 1320
ccccttgcaa cccaggtcag aagtttcatc gtcaaggttg tttcatcttt tttttcctgt 1380
ctaacagctc tgactaccac ccaaccttga ggcacagtga agacatcggt ggccactcca 1440
ataacagcag gtaggtgact tgttcagtct gagatgctgg caacatattt ctaatggaag 1500
agatctggaa acttcagcct cctctgaggc ccctgagaac tggggtgatg aagtgtagaa 1560
gcttagagct aaagacaact aagtcttcac ctagacagtg ttgcacaaaa gaattttctg 1620
caataataga aatgtcctag acctgccctg cccagtatgg tagccactag tcacacgtgg 1680
ccacggagca cttgaaatgt gactagtgtg actgcggatc tcaattttta tttcatttta 1740
ttttatttta tttttctttt tttgagacag gctctcactc caatgcccag gctggagtgc 1800
tgtggcatga tcttggctca ttgcaacctc aacctacagg tctaggtgat tctcccacct 1860
cagcctccca agtagctgca gctacaggca tgtgccgcta cactcagcta attttttggg 1920
ttttgtttgt ttgtttgttt tgtttttgtt tttgtaaaga cagggtttcg tcatgttgcc 1980
caggctgatc tcaaactcct g 2001

<210> 55
<211> 2001
<212> DNA
<213> Homo sapiens

<400> 55

ttcattcatc catccattca ttccattcag gaaacacact tcggacactg aatgtgccag 60
aacctgtgca ggaagggggt tgggggacaa gctatatggg atgctgtctt tgccttccag 120
gaggaagctc acagcccagg cttgagggca ctgaaatgca gaagagttct gggtccaact 180
ggggacaacg tcataaattt gcttcaggaa ctcccagtta gctaaggaga aacattcctg 240
cctcaggaga gacccctctg caatggtgaa tacatagttc ttgtcccagg aagaccccct 300
tctgaggagg gagcctcagt gtgtcccagg atagtttcca gtcgggtggg gaggaccctt 360
ggtctcgggg gaattcccag tctgacccag cccttgccct aagagatctc agcctgatgg 420
gggatacaca aagcccactc tctgggagct aacagtcaga tgggagaggt aggctgaata 480
gatccactct atacacaggg ggaggaacag gagaccgcat gtggaccgaa actaacgaac 540
cactcactca cactaaactg agttagcaag tcctgttggt ctttctgaaa agcaaagaca 600
ttttgaacag gtgaaagcag ggtgggttga ggtgttgcgc agaagtggaa aagggaagga 660
actttcttcc cctgttcctt ccccgctccc tccagaaagg tatttctgga actctccagt 720
tggtctggga tctgggcaac tgctgtgtgt cccagtctgt cagcccctca gctgtcactc 780
tgctcccaaa aatagcaatg gttgagggga gggagaggaa gctgtttatc tgggatagat 840
gtggagaggt gcattagagc tgtgcccagg gagggagtct gctggggggt tcctgtccct 900
tctggatctt tctgtcactc tttggaatgt tagtgcctaa gccccccacc aggataaccc 960
agagtggggt gcttgggcgg aggctggtca gcacctccac gtggtcatga ctgaccagtc 1020
tggggagggg gagggccctg agtatcagtg acagactcca ggggcacctg cccctgtggc 1080
caaatatggt ccagagccac tggctgaggc aagaaaatct cttgggtggg ggctgggagg 1140
ggcagactga ggctggctgg ctggaggttg acacaggagt gctcagggga gcagcatcac 1200
aagagggcag atcgaaagca tcgtccttgc tgaaaaagtg agcatgggcc tggggtgtgc 1260
cctggactgg ggagggagac ggcaaacttc taagacctgc aatgagagtt caggcctttg 1320
gcacaaacta agcagcttcg cctgctcagg ctgggaggga cagagagtga ggcacaggga 1380
ctctcaactt gggccaagct ctgagcaaat gctataatgt cctcatgggg caatagattt 1440
gggggttctg ggggtgggtc ccttccaggc agaatcccca ccatcctctc tggtgtgagg 1500
ggaccctgga gctccggacc tcaggccagg agcccagccc tgggtctgca gtgaggggct 1560
gaccagacag agcttctcct gtcccagcag atggcagagg tggaagcggt acagctgaag 1620
gaggaaggaa accggcattt ccagctccag gactacaagg ccgccacaaa tagctacagc 1680
caggccctga agctgaccaa ggacaaggcc ctgctggcca cgctttatcg gaaccgggca 1740
gcctgtggcc tgaaaacggt ctggggcagg gcagggcaca gggtgggagt gaggcagagg 1800
ggctttgggg ctgagtggca gccccttcac cctgatagaa attgcccttc atcccagccg 1860
actctcctgg atgcctgggg ttggagaagg gtgcctccaa ccccctgacc catgccccgg 1920
aaagacagaa attcatccag agcagaatca gcccaatgcc aggtgctttc atgtgttatt 1980
catggcatct tactaatggc c 2001

<210>  56
<211>  2000
<212>  DNA
<213>  Homo sapiens

<400>  56
agggagataa tatctgagaa gggacaaggg cttttgagat gctgctgaca ttctgtcttg      60

gtctgagaag ttattccata ggtggtgttt tctttgtgaa tattcattaa gctatacaat     120

taggatttgt gcactttttc tgaatatata tttatgcttc aatttaaaga aatgttaaag     180

aaaaagagag tgcaaaatca gaggatagga ggaagaaaga gagatctctc tggatttgag     240

agcagcagcg actctggaag cccaccaggg aggtggtaac agcctcgaaa gaaaaagcgc     300

tggagcctag ggggatctca aaatgaggtt tcctgtctca gcaataattc ctaagccaca     360

ctaacgaacc agaacactag ttggcgaaca ctagcaaaac caaagggagc acatgagctg     420

ggacactggg gatctcacca atgtggatgg ttgaccaatg acctgagtat tcctccccac     480

cccaacccag cctcctggga tcacactaac tattgggaaa gttctgaact tgaaacaatc     540

acgagggaga taggatttat gctgataatg gctggagtta tcctttccaa caacccagca     600

gaatggggac ttgcagtcat aactcaagtt gagttctagg aaataaaaaa agatctctta     660

catatctaaa tttgaggata gagattctta ccagatacat ggaactaaca agtctgggtt     720

ttgtttatta gtttttattt ttttaacaga cttcctcctt cttctctcat cctttcttct     780

cagtttcttc ccaatcgtca cacttgaaac agtagggcct aaagccacct atttggtatg     840

cctgtaagga tggccctgtg gatataaccc aggacttact attcccccca aatttgctct     900

tatgtactaa aataagcata tgtcaggagc tatctcctcc ctgcaaagac acatgtacct     960

ttctgtactt cactggcacg tctgggatgt ttgccagtgc gttcattttt ctgtgcagag    1020

ttgtccatgt tagcaaaatt ctgtgacgtg ttccatttgt ttgatgaaaa gttctactgc    1080

atgttctcct aagcacctga aacagcaaaa cacagcattt gtagagatca tttcacattg    1140

gttaattatt gcaaatgggt tctgttctat ctacagggta aactgagaaa tggtgtgggt    1200

gccggctgag atctaccaag tgcagtgacc caaagtccca agcacattgt gctggtgctg    1260

cctgcagtag gaactgcgta agaagtcact gttgatgttg cttacagaac tactgaaggc    1320

ttggatttta tttttatttt ttattttttg atacggagtt ttgctcttgt cacccaggcc    1380

ggagtgtaat ggtgtgatct tggctcactg caaccgctgc ccctgcctgg gttcaagtga    1440

ttctcctgcc ttagcctcct cagtagctgg gattacaggc gcccaccatc tgtaatttgt    1500

atttttagca gagatggggt ttcactatgt tggccaggct ggtctcgaac tcctgacctc    1560

aggtaatctg cctgcctctg ccatccaaag tgctaggatt acaggtgtga gccaccatgc    1620

ctggcctatt tttatttaaa tttagcttta ctgagacata atgtatacac aaataaaatt    1680

catcagtgtt agttgtacaa ttccaagttt tggcaaatgt ttacagccaa gttaaatacc    1740

accacaatta agatattaat ttatctatga tgctgagaag ctccctgtgt ccctttgcag    1800

tcagcaccct cccaaccact gctctgctgt cacaattgtt gcctttctag aatgtcacag    1860

aaatgcaatc atagacactc ttttttcact tagtgtaatg cttttgaaat tcttccatat    1920

tgttgcatag atcagtactt tgttccagtt gttctacatc ctcaccaaca tgtgtttgct 1980

caatcttgtt aattttaggc 2000

<210> 57
<211> 2001
<212> DNA
<213> Homo sapiens

<400> 57
cggggcctgc ctcctgcatc cccggcccaa aagcccgggc caagaaggac acaggcttca 60

atggctgtca tgtgttgcag acaacatggt gttgagatct tgcatggtgg agggtgacgc 120

tggtccctga agggagatgg aggaggaggc agagctggga acaaagggtt aaagggcgcc 180

atgtaagaga gctctccatt cccaccacgg agacatccag accccagcag aggcccaaac 240

tgactcacaa acacacagcc ccatctttcc ccttccaaag aactaccttt tcaagcaatt 300

ccaggaagct ggactcatag gaggaatttg tcagaaaaga ctccttcagc ttcagttgca 360

aagtcatacc cggccctgcg gatccagaag tacagcttag gacccagcag tcagggctga 420

ttcctccgga gacacagaac cttcctgctc acgctccccg gcacagttcc tcttccccag 480

caatgcccct ccagagcctc ttggaagctc aggactgggg tgtctctgtc actctcaggg 540

accatgaagt cccacccctt ttctctggcc tcttcttgcc acagggaccc aggagtcctg 600

ccctctagcc ctcacctgtt ccatgtgagc tgccaaggag ggtcaagagg aggacaaggg 660

gcagcccaga ccccatagtg gccactgcgc tcctgggatg gaggagacac tgaagtcccg 720

gtggcctctc cttaacaggc ctgtttctac accccactca agccttagca taagtgtttg 780

aggggaaatg ggaggaggaa tctggtcaac tggattttcc agttctccca gtaagagagg 840

acccaggaga ggaccattca ctgtgctttt ggggaaaata gaaggaagtc ccctcttttc 900

cactctgtgt cccacccctc ccttgtgtct ccaggtttga gggagtgagt gcgggtccta 960

cagacaggga aatggagagg gaagcgggga ccagggaggc gtccctcctg caggtgtctg 1020

ggcccccacc catgcccggg cctcccaagg atctttagag attaattatt aactgcagct 1080

aattttcatc attcttgaca ccgaagggct caggaatgtg ggcccaaaag ggaggggtgg 1140

attaagccaa gtttcttcca gaacccaggt gtcctgctcc ctcaggtttt ttttttttaa 1200

acactagtca agtgcagtag tgaaaagagt agaaggagta gaacaaggag ttgggtctat 1260

aatggactgt gaacactgct tccccagcct tggtggcttt ctagatgaga aatctggtca 1320

tgggaacttc cattgctgaa cattctgttt tacttgctct aacgcatcct ggattgtcag 1380

ggggagacac gagtttcctc agacatcctc tcactcctgc cttgtgcacc catgaaaagg 1440

ggacacccac ctactcccgg ccgagatgcc tacaggcaag cgctgggaca ggcaagcaca 1500

ggacagatgt gcagagggag ttactgaccc tctttagaaa ctaggaaagt aaggcacaga 1560

gagggtaagt gacctgctaa ggtcacccag ccaacaagta gcagagagat tagaacccat 1620

ccctctagcc agacaaagag acacaccagc catggaaact tgcttgaaca cacaagggca 1680

caggccagca tcttcctatc tgccacttcc ctatgcagcc tctccacctt ccccaaagcc 1740

agcgggactg agcacagaga gacagaggca gagagagcac tgggcagagg tggggagagc 1800

aaaagcaaga tggggaaaca gagatagagg cctggtctgc actgtaagtg taaagtgagg 1860

caaccaatga ctcccagact tgcacaggac aacaggaaga ccacagtggg aagaaagcat 1920

ggaggagaag agcagcaggc acaggaggga cgagcaggag gtagaattgg gaacacagag 1980

agatcttcct cggagaagag t 2001

<210> 58
<211> 2001
<212> DNA
<213> Homo sapiens

<400> 58
gtcaggagtt caagaacagc ctggccaaca gggtgaaacc ccatctctac taaaaataca 60

aaatttagcc aggtgtagtg gcaggagcct gtaatcccag ctactcggga gactgaggca 120

gggagaactg cctgaacctg gaaggtggag gttgcagtga gccgagatta taccactgca 180

ctccagcctg ggcgacaaag cgagactgtt tcaaaaaaaa aaaatttctt atggctgagg 240

agtctgtcct tatcttgggg aatcgagaag aaagctgtgg ggatattttt aaatgacggt 300

ttccccttga caattgtcca caagattaat aaaggaagat tctgagccct taccctgtgg 360

gaccgtgttc tcataactgt ccagcattgt gtctctgtag caatcctttt gagagccttc 420

taaataattc caatctttgg tcatattttc aaatgctgtt gcttcctgaa acaacattgg 480

ctgctgccac agggaccata tagtcttggg gcctgggcta aagagcagct tcagcaggac 540

tggggaagag acaagagagg gcaccaggaa tgaaggcatg cagcataaag gactctgaaa 600

gcgagtcagg tgaataaatg gaagaaataa gaaataggtc tcacatctaa caagatgagt 660

acagcttcat ggatgaggga aaggagtttt gctttcctgg gaggtctgtt ggtctggcca 720

gttttgacgc tgccagagct gtagcccatt tattaatctt tccagaataa agcagagtcc 780

aagtgaactt gggcgcaggc ttgatgactc ctaacagggg tggcaggatc tgtttccaat 840

ggaaatatta tatctgtgta cataaagata tgtcaggaga ttatcctgca gaaaagagat 900

accccaaatg gagtctacct gccactgtga tcagataatt ccttagggag agaacttgat 960

actcagttgt ctaaactggg attccttagt cagaacagcc gtaagacagg atggtgatga 1020

ggccagggga gttcacatga ggactgcagg aattccagca agcttaggaa aacaggatcc 1080

cgagatccca gctgtcagat gcagagcgga ggggcagggc agaatcctga gaagcagaga 1140

aagatggaaa cttttaaagg ctctgagcaa gagtctcagt taagtggggc ctgcttccag 1200

gtccggaagg tccttcacta aaagggcttc aaactcccct cctcagcatt gctagttaat 1260

catgaatatt cctgattcta agacattact gattgtaaat cactaactta ataacaactt 1320

tcttttgaga ggaaaaacag tgtgtattaa atgaatgtat tgataagaca catccaaaat 1380

ttaaaatgtg aaaaatatgc attttcattc tttttctttt tttgtatttt tttatagaga 1440

tggggttttg ccatgttgca aaaaacccag gccggtctcg aactcctggg ctctagtgat 1500

ccacctgcct cagcctccca aagtgaggtt ttgccatgct ggccaggctg gtcctcgaac 1560

ttctagcctc aagtgatctg ctcgttttgg cctcccaaag tgttggaatt acaggtgtga 1620

gccagcacac ccagccggtc ctactagttc taacagactt gaaaccacaa ggtattcaat 1680

ggaatccaga aaaagcacct tccagattcc tttctgaaac cagcgtctct cagaacctga 1740

gtgctgtgtt ggttgttatt tgagtccctt caggggaccg caaaggaagg aaaagcctca 1800

tcttcattct ataaaataat gaaggcaact gggcatggtg gctcatgcct gtaatcctaa 1860

cactttggta ggccaaggtg agaggattgc ttgaggccag gagttcaaga ccaacctagt 1920

caacatagtg agaccctgtc tctaaaaata aaaaaaagaa aaaaaagaat gaaggcaaca 1980

ggctagaaac aatttataat c 2001

<210> 59
<211> 2001
<212> DNA
<213> Homo sapiens

<400> 59
tctgtgttag taattaagga aaattcaccc agaaacacag attgtgtcaa cacagatagc 60

agacagttaa acaatgatct atggaccaca gaccatgtta ttgagaaaaa gagatctccg 120

ttgcttggga ttaactacat ggtgtgtaac atcaggaatt cactggacat gtgtatcttt 180

caatcaaaac atggtcacag cacatttatt cattgattca gaattcagag gtcatagtct 240

tgtcaaagag ttatttccca aaacctgttc catacaagtt gcctctttac gggttagaat 300

ctggtaaaga gacatcttaa tgattgggtt tgtttctctc ttctccttcc ctgacatttc 360

cattccctac cccagcccca acatcctact agtggattcc acagatggct ctcacctctg 420

cctctcgttg tttctcgctt ttccactgtt gctcccccag ggtcacaggg tgggctagaa 480

gtgtcttcag atcctcctgc ttctctaaag taacagcagc ttcatactct ccatctctga 540

aatcctcagg aaggaattcc cctgcctccc cattgccatt cttctttcca gtgacctatg 600

agggaagaag atggcagcgt cagaagcagc agcctcgtcc tgggtcattc ttcacagaca 660

atgccgcagg aggctcttgg tccttctccc ctgggaacag ccacctttga gcagtgcact 720

ctcagatctc aagctgcgat gcacactgct acaattgtcc cagaattacc ttcagatgct 780

tgtcctactc tcttggttaa atcaaagctt gggttaggtt gacagattaa atctactttt 840

aatctgctta atcacatttc ttttaggaaa atcaaaaacc ttctctgctt aattaaaact 900

caactttagg tacttttgat gagtttcgcc ttattgtaaa gtttccgggc ttacattctt 960

tcaataacac ttcttcccta agacattttg ataactacac gtgattccac actgtagtaa 1020

taaaaccaaa ggagaagata ttaaatagga aatatgcaga gaattcatta ctggtagctc 1080

catttttttt ttccaatttc acttgtttca tcacatcaac ctttttccat atatgacatt 1140

ttcagagttc ccttgcatta cacctgaaag caaagggcat ttcaattttc aaataaaaac 1200

caagatgaac cagttttcat tttataaaat taatgcatct tacataccag ttcctctact 1260

ttcagtacca tcatgttggc tgaaggagtc ttgtatgttt ttctgtcgtg gaaggaatcc 1320

ctggagttgg ccctgctggg cccctccaca ccggtcagct tatatagctg gggccgggtg 1380

agataatctt ccaacctggg aaccgaagta acactcccac ccccattagg agccaactca 1440

gaggcaggtg aattttcatt ccagactcag tgtctgggaa gctgaagagg gaggggagga 1500

caagctaacc ctgctgaata tgtgaatcag gaagaaatgt gagagggcca ttcctttggt 1560

ggtgatcaca tcgctcagca atgcaagtca tcctatttgt caagagtcag gggacagctg 1620

tctgttgaca ttgcaccaca tcactgccct tttttctttt gtcagctttc atatgactac 1680

ctatcaagaa aatgtagatg ccctacatat cacaccccca gtaatatctt tctgataagc 1740

agacttatca acacttcact taggggaaac ttgtcccagg acatcctatt ccctgggtaa 1800

acagcctgag gggaagggat ctgggcccta aaggcacttg tctatatctg tttggaattc 1860

tttggagtga tgtctggggc taaataggcc ccatgacatg ccaccttacc atcattataa 1920

gaaattgaaa gcattcagta atgcactttt cttttatgat gtcactggcc ataattagca 1980

catgacatgc ttaccgtgtt c 2001

<210> 60
<211> 2001
<212> DNA
<213> Homo sapiens

<400> 60
agaggtaatt atccagttct atatcaaggc aaaaagacag cagtggatag aaagatctta 60

gaagtcccac taggttcatc caagccacca tacacatagg cagaaaaatc aaaataagat 120

atgagcctgg acagggtgag caatctggga aaagatgaac acagtatgct aggacccaga 180

aatcatcaag tctatgaaaa ctaagccaga acacaaatgt gaattccata agatcaggaa 240

cataatctgt cttgttcatc caggcatggt aatctgccag aaatagtgct taactgcaag 300

aactgaatat ttgttagata attaaaccat caactaaatg agattcatgc aaccatgaaa 360

aatgctgcta taggtacaca atattgatat actagaaagt taaaaaatca agttggaaat 420

tagactattc catttctgtt tgtgtgtatg tatctacaaa taggtggaag gatataccaa 480

aatgtcaaca gcagttacct ctgggtggtg aggagtaatc ttaaccttgt tatttatccc 540

tatatgttca tttgtgaatg aatatttatt acatcattat aaaaaggatt tttaaactat 600

ctgtatgttt aagagtatat gttgctacta tgtaagagta tatgctgtta ctgtaaagac 660

attgcattac tactgttgac ctcagagcac gagcctcttg cctaattcta ggactcctaa 720

ctaagtcttt ggagtttcag ctggaagaat gctggaggaa tacggaactc ctcccatttc 780

tcacagccac ctccaactct taaaaacgct tccaactgcc tcccagcaca caaccaaggg 840

agaaaactat tctgtcaaag agacggtgcc aaaaggcaaa aacaaaggta aggatgatcg 900

ctggggaaag aagctgaaaa ggaaaagctc agaactctag ctggaaattt ggctcacatc 960

cctagtatgt tactgcatag tctggctttg ttcaatgggt cgcttttaaa tattaaagct 1020

agatgtaagc aaggtttgca acaaagtcca taagaaactc agcttttctc aaaggcaaga 1080

agagagcagg atttttgact ggctctttat tcaatagtgc tgcttattaa attaccactg 1140

ctacaatgtt taaagccaat tacctgagca catcataagg attctcttac cggttgtccc 1200

agttaagtaa tgttgattga tcaactcctt gacaggagct gatggcaaag aaggtagctg 1260

tgattggagc tggggtcagt ggcctaattt ctctgaagtg ctgtgtggat gagggacttg 1320

agcccacttg ctttgagaga actgaagata ttggaggagt gtggaggttc aaagtaagtg 1380

agattttctt gggtcttgaa caggttgtgt tgttatttca gggtgaatca cagttactga 1440

tgggtcatat tgagaaattt attaaacaac tctgatcaga ttttatttct acttattgat 1500

gtggccataa tggaactgaa gtcataggct ggcatctctc ccccagtcaa tactaaccca 1560

acccaggtag ctgacccagg catgtaaaag atctcttctt ttggattcag caattgtctt 1620

acagcccata cttctgtcat tctttaatac gctaatatta gagaacattt tacaaaaata 1680

gaagtaacag ggattcttct caagatatca cttctgtttc aattattaaa ccaaatgctt 1740

ctttagagac catgctctta tcattactat ttttctctga caaatgaagc atgtttgttt 1800

actgagcttt atcaatgaca ttctagtata actgctgtga aactctttgt taaatatgtt 1860

ttattaaatt tattctatta atcaaaccaa aatattgata atgctatttg tctgtattag 1920

tccattctca tgctgctatg aagaaatact gagactgggt gatttataaa ggaaagaggt 1980

ttaattgact cccagttcca c 2001

<210> 61
<211> 2001
<212> DNA
<213> Homo sapiens

<400> 61
aactggggtt tctccatgtt ggtcaggctg gtctcaaact cccaacctca ggtgatccgc 60

ccgccttgac ctcccaaagt gctgggatta caggtgtgaa ccaccgctcc ctgcgagaat 120

ttcttgaacc cgggaggcgg aggtttcagt gagccgagat tgtgccattg cactccagcc 180

tgggtgacag agtgagactc agtctcaaaa aaaaaagaat tttagctggt catagtggca 240

catgcctgta atcccagcta tgtgggaggt tgaggcaaga ggatgacttg agccaggagt 300

tcaagactgg cctaggcaac gtagcaggac cctatctcca aaaatttttt taaatgtaga 360

aacattaggt tatagcttga ctgttacaaa taaccatgtc ttggtaataa gggtctcacc 420

tctccttctg gaattctcag tggcacactg ggatttagca gcattggggg ctggtcctgg 480

tcccacccag aacactgggc ctcaatatcc tcatctctac agtgagtggg ttggcagaga 540

tcatctctaa tgttacttcc aaccctgtcg ctataggtcc attgcgaata ttattatcat 600

cactattttt caggtggaga aactgatgaa ccagttaaat atttgaataa aaacatctgt 660

tggcatggaa aagtgattat ttcctaagct actgagtaaa acacagctgc actggcagtt 720

ttaactctgt tttcttgtca atttttgttc tttcaggaaa ccttacagaa acatgaagcc 780

ctcaaccatc tgctactcag ttattcgggg ctgacggcgg cttctagaac atccaggtgt 840

tctgcagatg cgagaactca tcctgtagtc accagatgga gtcccaaaca gccaagcaga 900

tgtaaggcct gtgctgtggc tctgaggccc tgaatacaga agggtcactt tcttagtggc 960

caaagagcag ttgttgacat tgatgtctaa ttattgaaca cgaccagtca ttttactgag 1020

ctgcggtgag gaaacactga ccatagaaga tcaagccaaa tgagggattg caaatttcct 1080

gattcttttg aattaggatt ccagatgggg gcctcatttc tacagccccc aacattccta 1140

tagccgttat cactgccatc accactgcca ccagcatctt cttgcagatt ccacccctgc 1200

tccccagaga cttcctgctt tgaaagtgag cagaaaggaa gctctcagaa aaatctctag 1260

tggtggctgc cgtcgctcca gacaatcgga atcctgcctt caccaccatg ggctggcttt 1320

ttctaaaggt tttgttggcg ggagtgagtt tctcaggatt tctttatcct cttgtggatt 1380

tttgcatcag tgggaaaaca agaggacaga agccaaactt tgtgattatt ttggccgatg 1440

acatggggtg gggtgacctg ggagcaaact gggcagaaac aaaggacact gccaaccttg 1500

ataagatggc ttcggaggga atgaggtgag tcttgagatg ccaggccagc ctttctttgg 1560

atgtcttact cccgttcttg agaggggaag gggccgtgca aagcacttaa agagtcattg 1620

atggacccat gctgatttag ttaatttatt aattaatttg gtttgaagcc agcatagcat 1680

cgtgaagaaa tccagccatc tagcacccat ccatctatct agtaacattc attatgtgtt 1740

aggagctagg aatacagagg tgaacaagat aaaacatttt tgccctcaag ttattcattg 1800

tctggtagag aagacaggtt aaaaggaaag taaggggcta ggtacagtgg ctcatggctg 1860

taatcccaat gctttgggag actcaggtgg gaggttcacc tgagcccatg attttgggac 1920

cagcttgggc aacctggcaa gatcccactt ctacaaaaaa taaaagcaaa ttagctagcg 1980

gtggtggcac gctcctgtgg t 2001

<210> 62
<211> 2001
<212> DNA
<213> Homo sapiens

<400> 62
tccagacaag ggggtgtgga ggtcagagtc cacaggagag gggcctgaga ggaaataatg 60

gaaatgcggc tagcaaacag ccactaaatg gacagaggag ggggtgcccc cagagaaagt 120

aaatgggggg tccagaccat gccagaatcc atcctcagtc cttcctaaag ctggaatggt 180

tggaattcca gaggaaccca ctgtggtcaa gacagtcagc cctcaggccc cagcaccagt 240

acacgggttg gcagggatga tatcaagtcc ccgtgattcc aaagcacctc tgtttagttt 300

gggagggaat gccagggctg agaaccttcc tggagccaga ggcaaggagt aaggatgcag 360

attgttctgg gatttaccaa ggggctgatg tgaacgtccc caacacgggg ccaggctctt 420

gttggtgttc agtaaaagtg ggatggatct gacaactgat aaggatacgt tcccacccct 480

cctgccctgg cccagccctg gcagctgatg ggagagcaga tcgtccaatt caagccttcc 540

tacggtctct ttagaagaca tgggctgaaa cttggggtaa tagaggttgg ctggcatatc 600

catgcaggat gattgtcttc acatgtatct attaccttgt agaataaggt agaccctgat 660

tttggaacct gaagaccaaa gtgcaagatt agctctgcta cttccatctg tggaccattg 720

ggcaggtatc tctgggcctt cacttactct ttgtgaaatg aggacagggg caatccctac 780

cctaccaagt cattgggagt gaagacatga tgacacggtg attgtgaaaa gattttgtca 840

atcgcaccag cattaagggt gcccatctcc aggttccccc aggcctcaag gctcccaagg 900

cctgagtggg caggtagcac ccaggtatag accttccacg tgcagcaccc aggacacagc 960

cagcatgaac tgggcatttc tgcagggcct gctgagtggc gtgaacaagt actccacagt 1020

gctgagccgc atctggctgt ctgtggtgtt catctttcgt gtgctggtgt acgtggtggc 1080

agcggaggag gtgtgggacg atgagcagaa ggactttgtc tgcaacacca agcagcccgg 1140

ctgccccaac gtctgctatg acgagttctt ccccgtgtcc cacgtgcgcc tctgggccct 1200

acagctcatc ctggtcacgt gcccctcact gctcgtggtc atgcacgtgg cctaccgcga 1260

ggaacgcgag cgcaagcacc acctgaaaca cgggcccaat gccccgtccc tgtacgacaa 1320

cctgagcaag aagcggggcg gactgtggtg gacgtacttg ctgagcctca tcttcaaggc 1380

cgccgtggat gctggcttcc tctatatctt ccaccgcctc tacaaggatt atgacatgcc 1440

ccgcgtggtg gcctgctccg tggagccttg cccccacact gtggactgtt acatctcccg 1500

gcccacggag aagaaggtct tcacctactt catggtgacc acagctgcca tctgcatcct 1560

gctcaacctc agtgaagtct tctacctggt gggcaagagg tgcatggaga tcttcggccc 1620

caggcaccgg cggcctcggt gccgggaatg cctacccgat acgtgcccac catatgtcct 1680

ctcccaggga gggcaccctg aggatgggaa ctctgtccta atgaaggctg ggtcggcccc 1740

agtggatgca ggtgggtatc cataacctgc gagatcagca gataagatca acaggtcccc 1800

cccacatgag gccacccagg aaaaaaggca ggggcagtgg catccttgcc gtagcagggt 1860

ggtgaggagg gtggctgtgg gggctcagga agctcgccca ggggccaatg tgggaggttg 1920

ggggtagttt ggtccctggg tcctgagcct caggggaggg aggttgatag ctactgggga 1980

ttttgtatat ggcaacagta t 2001

<210> 63
<211> 2001
<212> DNA
<213> Homo sapiens

<400> 63
gacctcggtg tccacgattc cccggcggcg gcggcgctgc ccaggtcagc cccgaagctg 60

cacactcacc ccgtgggcgc cgggcaccgt ctgccgtctg ctgcccgcgc ggccgcgctc 120

cgctcgcctt ccgcccgggg ccgggctggg gacgctggtc cgcgccctcc cgccgcgcgc 180

gccgtctgca caggccgggg aggctcggcg ggcgccctcc ccaacgtgga gttatcctgg 240

gagcggctcc agggcccagc cggtgagggc gatggcagaa tcccaatccg tccccacccc 300

ctctccgcgc cctgggcgcg tcgcggccgg aggctggcgg gcgagagcca ggcgctcctt 360

gagagcgccg cgcgttcgca ggtgcccgga gcaggccctc gcggcgcccg gcaccccctc 420

cacgcgcgcc cctcttcccg gcttcgcgct cgccctcctc actcgcttct ccgccgctct 480

cccaacctcc ccagcccact ccggtcgcct cctcccagtc tgcatgccac gccaaccccc 540

tccagtcccg ctgccatttt aactcctgag tggccagagg ccccctctac ctaacgcaca 600

cacacccggg aggaaggagt tgggtggggc cagacgctaa cgcttgagct gtccttgtgc 660

ctagagacac aaggcttggc tgagagaagg ggcacagccc ttgggtccgg gaagagaagg 720

gttggcttct ctcatactgc ctcggaccct cttgcaaagg ccaggagacg aatggggaat 780

tcagcagctt tgtgaggaca ggtgcagctc acagtcacag catgcaccta agatcacatt 840

tttgctttgc atggtgctac cctcccgaag ttaacagtgg actaaggaat gtagtggggt 900

gggtgagaat ttgtcactgg gcccaggcac ctctcaccct aaagaatgga gtggaagacc 960

cctttagttg tcatgctttt ccagcattgc atcctgagcc gtgcagctct agcattctgg 1020

aagattccgt cctggctctc aatgaaggag gtcaatttca gtcaggaact ggcaggccca 1080

gttaggggtt tggcttcagg gttccccact ctgccagctg gacatcatga agactggggg 1140

aggaaagtaa gaaggtgcca aaggtgggtg ctaggctgct gcctggtggc ttagaaaagc 1200

tgggtgactg ccactagagg agggcaaagg cccaatgccc aacacccatg ccttggtggg 1260

gacttcagcc tggaccactc agaacagaca tcacaaactc ggctccatgg caggccacac 1320

cctactgatg ggtctggacc agagcctgca gaaccaacaa caggggaatg gatgaggtgg 1380

aggccaaggt gtggagtcct gtgaggagtg gcttccctgg gctgcctgcc gtcatccagt 1440

gcttgagctt ctggccctgt cttttccaag ggtacagcca gtggtgactg ccatgcctgg 1500

tggcttttgt gaagttggct tgaaggtctg agttctgtat gcacagaggg gattctcacc 1560

ttcatatatt gaatgagcac atattaagtt cccactaaac cacccccatg ttcccactct 1620

ccacccctgc tacggagcat aatggcctca aggagctcag agtgacaggt tggaactata 1680

tgaaaccaat tttaatattt caagacgatg gaagtgccat agaaatacaa aaagaaacca 1740

ccttaatgga ctagcattat tcacaaaagg tttaacggag gagctgagtc tccaactgag 1800

cctagttgga gggataggat ttgaactgga agagaatatg gggaccagca aaggcaggaa 1860

agtggagaca agcagagtag aatgggttgt ctggagggta gggggcaagg gaggaaaatt 1920

atagagctac tattagatgc cagccttttc tcatctctgt catcttagac tctgagttga 1980

ctaatatccc cttttaacag a 2001

<210> 64
<211> 2001
<212> DNA
<213> Homo sapiens

<400> 64
atggtgctgg aaagggccac tgtggccagc tcagatgagc ttcaaggatc ccctggttag 60

gagcatgaac tctcatctgt gggcagaggg atctcaggtt aaattttagg aactgtagca 120

agctgtggcg aggagtgagg ggactttttg ggataaaatt gtcttatgag ctcaattgca 180

tcccctaaaa attcattggt ttaagcccta acccccaata tcccagaatg tgactgtaat 240

ggagacacgg cctttaaaga ggtggcttag gtaaaatgag atagttagag tggaccctaa 300

tccaatacag ctagtgtcct tatcagaaga ggaaatttgg acacagagag agatgccagg 360

gatgcatgcc atgtaagagg cagcaagagc atggccatct gcaaaccaaa gagagagggc 420

tcagaggaaa acaagccttc cccaatggct tgacctcaca tttctagtct ccagaactgt 480

gagcaaataa atttctttag tcaaagccac ccagttgcag tgttttgttg tggtggcaga 540

gcaaacagat aaagtagtaa gtccctccag caccctagga ggttctgctt gttgccattc 600

cccaaaggcc atcctagcca tgatgagggc aggtgcggtt gtttgggagg agaccactgt 660

ggaacaaaga gaaacaaggc ttgcttgatg cagcagggag tagactacag attcaagaag 720

ggctctagaa aggtccagca attcagcttg tcagggaacc ccgagctgta tgtagttctt 780

gctctcctgt gccctgcagg atggcttgac ccatttctgt tctcatgtca gaccctaagg 840

gcagggtcat ctgggccaca gacccaggcc cggtgggcct ggtgggctga cctgtgggct 900

attcttatgc cttcaggaaa cccaccttta ccacctggct gttgtgactg gcatcgctga 960

ggctgtttct ttgtgtataa aatcgaataa ggacacatca cgtgctagca atccaccagc 1020

atagggcgaa ggctcagcaa agagaagttc tctcctcctc tgctctttta ggtcagcagc 1080

aaatgcagat cggggtgggg acaaggtaaa cacataactt gggtggagat gtgattgatt 1140

aataactcat aaatcatctg aaaccatact ttccttttga tagtcaaccc tctgtaaaca 1200

ctcaatgtgt tctcaccttg ttatcattcc caatgatgtc gaatgcgtgg ttgccctctc 1260

cagtataaaa gtttgatgca gctttgcctg gatgtacctg tctataagga gtcctgctta 1320

tcacaatggt aggtaactgg ttttatatat aaaggctaga aaagcaaaag agaatatgta 1380

ttcctatttt aatatgcgtg tgcttctacc acatagcatt gaatggtaaa tgtgacctag 1440

ttaggaacct tatgttatta cctgcaattc aatgtgcaga gtatgagagt ctgagagctt 1500

gcgaaaattg taatgagttt agcactagtt ctgttctcta agatctaagt tgtttttcct 1560

tccagtaatt ctggggttca attagatttc ataagtgtct ctaattcttg ggtcctatgt 1620

gagtaatttg aaattaaaaa taatttcaga gctacatgac tgcatcgttt ttgagatgag 1680

ggaactgagc ctggggaggg gaagaagctg ccactggggt ctcaccgcaa gcctgtgaat 1740

gagctgagat aggaacccag gcattcctct ctccctgcca ggggctccca aacttggctg 1800

tgcagtggaa actccggggg aattttgaag aacactgaca cccatcttcc accccgacac 1860

tctgatttaa ttgggctgca gtgagaacag agcatcaatt taaaaagctg cccaggtcat 1920

tttaatactc aagtttggga accatttccc taggcccttt tcactgtatc atgtgttaag 1980

gaaaaatctt gggtttgggg c 2001

<210> 65
<211> 2001
<212> DNA
<213> Homo sapiens

<400> 65
aaaactgttt agtacaattc tgacaatata atagtggctt aataaatgac agaaggagca 60

acctttgttt ccagtttcat ttgtccacat ataccccaac tgagatttgt ttccgtgtcc 120

tgaccaaaaa atcacagatt gcctctgtga cccagcctac tgcaggttgt ttctcccagc 180

aggctcgaac ccaagccaag gccttcaaca cgcccaggca ctgactgagg ttgatacaca 240

caataaggat cctaaacaag acacaatgtt tccctcttcc tgcctcccta ccccttgaat 300

aggtggctct ggtatatgaa gtccatccca tgtaaagagg caaggcgtac ctttctgcca 360

aagggaagaa cactgttctt tgaaaccaaa agccacttcc agtctggtct gtggcctgga 420

cttacaaaga aaggcatcac tcccccatgc caattcttgc atacacactg gaaaaaaaca 480

attgctctgt tcttacctgg agagtctgtg ccttgagtca tggaggaatc cagtcgggtc 540

agattcacta gcagagccac cacccagggg acccacccag aacccattct ggagaaagga 600

aaaaaatgag atagtaaaat cgtcagcctc ttcagaatga gctcataaaa ttcagtcaga 660

aagtacccat taagagtata aatcgctgtt tttctggctt ccccaggatt ggaaactcct 720

cagattgaca accaatcaag atagaagagt tttgcatcat caggtactgg gtaggatact 780

ttcacaaagt tgtgtcatac aactcaacca ttgtttgcct gcagaatcac tgacagtaat 840

ttaggtatac taaaaatggg ctgggagaag aagtaaaaat atatgtttga catattatgg 900

ggccctagaa gaactaggca gactgtttat tatgtattct tctattccct ggcctgttct 960

gaccaatagg tctcccttct tattgggtgt tgacattgcc gacaggcagt gtgtaaatta 1020

agaaggaatt aagagtatgt aaattaagca ttctgaagca tatgcctcaa gaagatttag 1080

taaagggatt atcagagaag aaatagaaga catagagaac atttggagag tacgatttcc 1140

tataagccaa aatgaccaca aaaaaagaaa agaaaagaaa acatggaaag agaattctca 1200

aagaataaaa ttttgcaaaa aggctcacca ggatacaatc taagagagca ttattgtgtt 1260

ttatggttag tagggcacca ataaccttca agaggacagc agagtgttaa aggtagaagc 1320

catatttcaa aagaatgaat tggaaacaag aaaacaaagg ccgcagatta aaacagtctt 1380

tcaagaattt tgaaagtgaa aacaaaggaa aaaatgggat acactttaga aaaaaacaac 1440

actaagaaaa aatacttttc ttgggttttt taaattgaca cgtaataatt gtacatattt 1500

atggggtaca gcatgatgtt ccaacacacc tatacattgt gtattgatca aatcagggta 1560

attagtatgt gatatatata catgatggaa tactattccg ccataaaaaa gcatgaaatc 1620

ctgtcatttt caacaacatg ggtgaacctg gagggctcta tgttaagtga aataagccaa 1680

gaacagaagg actatattac gtgatttcac tcatatgtgg aatctaaaaa agttgacctc 1740

atcgaagtaa agagtacgtt aggagttacc agaggctggg gagtcagggg tagaaatggg 1800

tagaggttgg taacaagtac aaagttacag ttagacagaa ggaatgagtt ctggtgtttt 1860

gggaaaagaa acttttaaga caggagagac ttcaacaagg ttgcaagtga agtgcaaggt 1920

atctgtagaa aggagaaact gaagagaaaa aagagaagaa aggggacatt ttctccaaaa 1980

tgctgtggtc tgaatgtcac t 2001

<210> 66
<211> 2001
<212> DNA
<213> Homo sapiens

<400> 66
aaatggcatc tttggctgat acaaaaaaat tagccaagta tggtggtgcg tgcccgtggc 60

cccagctatt caggaggctg agatgggagg atcacctgag cctgggaggt ggaggttgca 120

gtgagccgag attgtgccac tgcactccag cctggacgac agagcgagac tctgtctcaa 180

aagaaaaaaa aagaaagaca gcctgtccaa tatggtgaaa ccccatccct actaaaaaat 240

acaaaaatta gccaggtgtg gtggtgtgca cctgtagccc cagctactta ggaggctgag 300

gcaggagaat cgaggttgca gtgagccgag attgcgccac tgcactccag cctgggcgac 360

aaagtgagac aaaaaacaga aagaaaaaaa aaaggaaagg aaaaggaaaa ggcatccttt 420

tagatactaa ccaggcctgg ggtcagatct ggacttaaat cctggcgtga ttgcagactg 480

gctggcttga ctttgggctt gttctttctc ctctctgggg ctcagtttcc tcatctatga 540

aatggggagc tatagtacct gcttctgaag gttggctggg cttttgtgta aagcactcag 600

ccctgggcct ggcacatagc aggggctcac tagatgggcc atattaactg tctgctccaa 660

ggctggaggg tgttataggg aaaacagtca gaaaatactc tccagatgca aggtggcctt 720

aatatgtcag gatgtggaag gcagagagaa gcctcggggt ctgggatctc ccatagggga 780

tgagtatttt ttcctggaat ccctgccctg ttgccacatc actaccctga ccatttgccc 840

agcccagccc tacatgcttc aggtgaaagc agagagagag gggcgggaga gaaaagccca 900

tcaagcagca ggcggagcct ccctgcagga gtctggcctg gctggaaggg gagggggctg 960

tggggccagg gacgtcagat gccaacagca ccaggggacc gttaaagttg ctgcgccgcc 1020

aggtgggcca gggtgtctgc agaggtcccc ggcctcctgg gctgccgcct gccaccgctt 1080

gggctgcccg tcctgggtac gcaccgttgg agcctcctgc ctggtctctc gtgcttctct 1140

gtctcttctc taagtcgatt tttcgctctt tcttctgttt gtgcccctct ctctgtctat 1200

ttctgtcctc ccccagccac attgttgcct ctctctcatt ctctcttatc tctttacctt 1260

tctatttctc attctttttt ctctgtaact ctttttttct gtcccctttt tggtctctct 1320

tttttctctg cctctttcat tctctgccat tgggttttct ctgtgtctca ttctcaattc 1380

ctaaattgca gcaaagcagg gacaaaagga ccttgcaata tgacttgggg taaggtgggc 1440

cctgccctcc gagggcattg actgagcccg ctccagcagg attggtgaag aggatgtcta 1500

gactggagag ggctgctgtc tgtctcctgg gctggaggga cagagcactg gctttgaagt 1560

ggtggcacca tctcacccca gggccatgtc tggattagcc actgtctggg catttcttcc 1620

ccttgcccaa cccactcagt cccctggggt ggaaaggaag ggtgctgggg catctggacc 1680

agagctgcat ctggaggtct ttgccaagtt cccaggaggc agcatcctct gggtcggcac 1740

tgttggagaa cgctcagggt ctggtgctct cgtgcctgcc ccttccaggc agccggctct 1800

gattgcacaa ggcagacctg tgactcctcc atccagctat gcccctgctg cccagcaccg 1860

tgggcctggc aggcctgctc ttctgggctg gccaggcagt gaacgccttg ataatgccta 1920

atgctacccc agccccggcc cagcccgaga gcacggctat gcggctcctg agtggcctgg 1980

aggtgcccag gtaccgccgg a 2001

<210> 67
<211> 2001
<212> DNA

<213>  Homo sapiens

<400>  67
tcatgagaga aaaaagaaag gacgtattat ctcactgtga taaatctcat aatattggca      60

tactccctaa gttgggcaac tttgatcagt tttctggaac catcataaaa aggcctttaa     120

ttggataaag tctcgaactt ttcttctgtg gatgtgaata tcttgagaag tactggaatg     180

aaacaatttg gctctggttt cagtcatctt cacatgtttc ttcaggtgct ttcttaagct     240

attagggcaa aataagctgg tagaaaaaag gtgttacttg taggatgaaa gaagcaacta     300

aagagaagaa actttctcta gaaaaatgtt acatttacac atttataatt gttaacaact     360

tttttcttct tttctttttg ggagactaaa taatgggatg atacaggaag aaatatgatt     420

tagagaaaac tgattgagcc aaatttttca aacggtaata tgggtaatca taggctatgg     480

aaagttaaac tgagtctcca atgtttatga ttatgatgtt tggctaggat ataaaataag     540

gcttcagaat ataaaatatc ctgcagattg agaagtttta aaagtagtta catacaagtt     600

ttatttttcc aacgttcctg ccttttctaa tgtctaagga tgaagcagtg tacagagagg     660

aaagaatgtt atatgtgtgg tctgcttgca agtgctggat gcctcctctg cctgctccaa     720

ctaaactgtc tgcttatgag aaagtcattg ctcctctgag ttcctgcctc cctatatatt     780

caacattaga attttgccag gttatctttt agggtcgttc aactctaaca tatatatgag     840

tctgtgacac tgaactcaga cacttcagaa caggaaccct ggatttctct ggatgtggaa     900

atctttaaat ctatacctga ctggtaggac aaccaatctg tcttcctttc ctctcctctc     960

ccacttatga agggagtcca gtcagaaaca tgtttgtcta cgaggtagta tatctggtat    1020

ttctgaggaa ttcggttatt atataggatt atagaatatt caactttgag tagggtctgt    1080

ggactccatc aattgcgttc tgggggaggt accatgaaag aacattccta ctttagaaat    1140

agatattctc tgttcaaacc tgacaccctg acaggatcct aaaggatgga agctaaagtg    1200

gtgtggtata taagtcccat gaccttgaac aactggtttt atgtgggtgt caccagtgct    1260

ttataaaagt aaactgcatc gccccgagtt cagtcatact gcaccagctg agcaatgcat    1320

ggagtggacc tgtaggcgac ttgcatcgtc ttcaacatga agatagccac agtgtcagtg    1380

cttctgccct tggctctttg cctcatacaa ggtgagcaat ttgtgtgtaa tctaagcctc    1440

ttgccacaca tctcaaagcc ctggggaagg gacttttctc cccctactcc tgccaaaaaa    1500

tgtttacgta tgcatgtata atcctgaaat gtcttgccag acacagagtt ctgaagattt    1560

atatatgtgg ctttataatt atgttgttat ctttagaaat ggcaagtagt ttgagataat    1620

ctgaattttc taaccatgag aaattcttcc attaattctt tattaattca gaaaactgaa    1680

tagattcttc ttcatcttcc tcttgctaaa tgtatcaatt cataatgcat attcttatat    1740

atgtaataga catttaaaac ttaattgtaa ataatatatg tattttaatg ttataggtat    1800

aaatgaaata ttttttaaaa ttcaatcatc tgttcttatt ttaaagcagt ggtatcttct    1860

agcttatttt agtcttgtgt tttcaaatgc taaatgggtt gaaagcattt ctcccaaaga    1920

gtgagggact tttgtgggag ctggaaggta gatttttata tttgctattt aaccctaaga    1980

gaacacaaat tgcctagact t                                              2001

<210>  68
<211>  2001
<212>  DNA
<213>  Homo sapiens

<400>  68
gttatagcag ccctagaaaa ctaagatgga actcgccggc tggtcactgt cttccctctt      60

atcacactac cttcccttac tgcagctact tatttaactg tttcccacca aaagacccta     120

acatccagga gtgtggggcc cacatctgcc taattcgcca ctacggccca agcccacctc     180

tggcacactg gaggtgttca agatgtgagc tgacatatga aagagctgtc ctgactagtt     240

ctagaatctc aggcatgtga ctccccctcc cagaatgtca atttccccat ctctctgcag     300

agggaaaaga aaataattgg agtagatgac ctctgtgagt ccttcgggct caaaagatgg     360

ccgggactca ccactgaagg tgtctgcatc catcatggtc agatcaggcc atgagaaacc     420

ccactagagg tgctaaacag agggaatggg tcacagaggt gtgggaagcc tgagaaacca     480

ctgcggcctt cctgctggac ttcagagaag ggctggcctc attttatggg ctttctgaaa     540

cccatattca gagaaacaca cccaattcag catggactgg caatgcctat ccccaaggaa     600

actccaaaag gggtgagtct cctgggcagt ggtcactgag ttactacaag gcctttgggg     660

ttggggtggg gatggtgggt caacaggctc ccctagaagc cctgtgccct gacaaagtaa     720

acaatggcaa aacatgtgct tcccagcact cacctcccca cctccttcta acatcagaac     780

tgggtttctg atgatgggag aaaatgtaaa tgaaatcaac ggaaggatga tcctgtgagt     840

aatctgacaa gggagaacca gcttcctctc ccatgaaccc cagctgcctt ggctttcacc     900

cagacccctg gcccacccgg tgtcctcccc agacagtggt actcacccac actgctgagg     960

gagctgctgc tttctgagtc tgagggcatg gtggacagga cgctgtaggt agaacctggg    1020

gaaagagggg agaccatgtt actaacccca aggggtcact cgtcttcacc ccaagccagc    1080

tgatcatcag tcaatgccag gccatgagag ctacatgcac caggcactag aaatccacat    1140

ccacgggcca agcagaggag gcgcgctcag cagatctgga cttcggtgtt tctgccaggc    1200

ctcccacctc actttatcgg cccctcctgc tctgggcagg cgggcagagg ggaacttgga    1260

gtcagtcagc tggcttcttg ttgggacatc tggggaaagg cttagcaagg gagtgacatg    1320

ccccaggtaa acatggggtg tgggggtttc ctccacatct ctagcaatat gattctggct    1380

tccatgcaga gggtaacaga gcagaaatta aagaacaact gaactatctg ttctttctct    1440

gactgttctg ctctttaggt tctaactgta ttcaagctca gttcttaata atttacttag    1500

tatctgcccc cttattaaaa gttactccta aaatgatgag caattgtacc atcaccaaag    1560

ccagagggta gcactttttt ttgtccacac ttaatgttaa ttcctggttg tggacacaca    1620

accactgtgg tttggtttga attcaaatat cttcaaaacc agatgttacc ctcccagcac    1680

cgggaagccc agtttctgaa cctagctcta tgtaggtgcc catagctggg tcgtgctgag    1740

caagtagctt ttgttttctg gaactctgtt tcctggtctg caaaatgaag gagttcaacc    1800

agatggctta taaggtccct accccatccc ccatccccca tcccccatcc catttcagca 1860

atttccatga ttaatggaaa cagaaggtac actttgctca tcaagactat ggatgacata 1920

aaattaaaag ggagggcaaa ctgactcaga gactaaattg ggagtggaca aagcataaca 1980

cacagagatg gcaggtcaga t 2001

<210> 69
<211> 2001
<212> DNA
<213> Homo sapiens

<400> 69
aaatgattgt gaaacatttt ttgcaactgt aagagggttg atacttttcc tgaatattat 60

tcattcaaca ttcatttatt tctttgacaa atatttaaga gatgctcatg atgtcctagg 120

cattgagcta agaactcaat gttgcaatgt ggataagatc cagtccctgt gtgttcatac 180

ttatttaata ctattataga caaggccttt aatacatacc atagtcatat atcaattttt 240

aattagcaat atgaaataag agaacaatgg gactttccac tcaatttgac aggtggctaa 300

attttcagtt actgaattta ctattttagg gtaatctagc acatgaccaa aatttaaatg 360

cacagaacag aagatcagag taagaagagc ttccatttgg attatcactt tttctacttg 420

gagtcacata ctgtacttta aattgattaa gaacatgtta aaggtccagg tggtgatttc 480

cagctacaag tccaaagtat agaagaatgt gtttaaatcc tacgtttatc agatccataa 540

atatagttcc ttctatgtta agtggcttta tttctaagaa cccagccctc ggtcctgggg 600

gtgcttccca gcccgctgct ggaattcctg atcgtggcag tctgctgaaa catgagctca 660

gttaccaaat atagctgagt ggagtaagag gacagatgag agtctgaggc cacagttgcc 720

tcaaagtgac cacggaccag gcagtctcta atgaataggc aaggccacaa cctccattct 780

cccagaaaag aagaaatgct catctgaaat tcatcacctc tctggagtct tcaaactgac 840

caagcattga aaaggtctgt aaggtgtatc ttaatatact aactagtagg gcaacatttt 900

aaggacttaa aaaaaaaaaa actaaaaata caaaagtttt tctgaagata tgatactatt 960

tacaagatac cttaattatt cttttgcttg gagaggaaac gtaatgagaa tgttacggtt 1020

ttgccaaaaa cagataatac gtaagatttg cctgcccact tcatctctgc ctttgctgtc 1080

agagaattac actttctgtt ataagcttaa gcatgctaag atggtgctat caattccaaa 1140

aggattaaat gtatttatgg agccagcttt tgtcaagatc gtgtatgtgt tatgtttcac 1200

agaaaaggaa aacgcagctt tctagaccct cacccatatt taagctagat aagtgatgat 1260

ggcttaaagt gatagacctt agatttcagt aacattataa attgttaatt acactttcaa 1320

aagattctaa aagtaactca ctggacataa ttaaatgagt tgggatagta acagcaggta 1380

ttttaaatta tattcatgca taaaatttac atacatattt ttaataagat aacgcttact 1440

aagggaaacc acagtgcaat ctcgagtcca gtcaagttta aacatcacag aataattttt 1500

atcatgttgt tttaagttgc acatgaaatt gtcttgaatc caatatagga ggttgacata 1560

aaatgatttc aggctcatat tacttattaa catttgctaa aaatactaat tgccttctag 1620

tatgaataat gtggtgacat ataaagccat ttcttggcaa tataaaacta ttacaaatct 1680

attcaatcac atatatgcag aaattaaatc ctcatttcat gataaatgtg ccttccaatg 1740

ttttttctca cagaaagaac ttgaatactc acctcaatta ttcagatttt tttttcagat 1800

ttttaaaatc aaagtctctt tgacaagtta ttgctaacag cagaaaaggc cgatgaggtt 1860

tatctggcca ctaactgccc tgtaaataca tgattacaca ttcctagaat ataaattagt 1920

cctctggcat cctagaatct ggtatggatt agggtctgta tcccaggggt tctgtgtcag 1980

ctcttcctct gggtttctct g 2001

<210> 70
<211> 2001
<212> DNA
<213> Homo sapiens

<400> 70
cctctttttg gggaaaagga cttcaagctc aggttttctt tggtggggtt gggtattgct 60

tcttccatat tccaagtctc aggaccctca ggggcaggtg ccaagtgaga gtgaccccca 120

gaggtagaaa ggacccagca tcatttctga ggacacctgg tagaggagga gcctggacac 180

ttggcctccc atcctggctc agggtatggc cttgacacat cttcatcctt gtttcctcct 240

ttggacaatg gagatgttca gactaggtgg tctctgaggg ccctgccagc tttagccatc 300

cctccccaca ccagctaaag ggactaattc ctccgaactt tcatgggtca gatagaggca 360

gggaagggac agccccagcc ctgggaggca ggtctcctta ccttcttcca gttccttcac 420

aggaatcaca cacttgaagc cacagtgcag gtaacaacac ttcctttccc ccagacagtc 480

ctggtctgtg tgacactggg gaggatcgga cttgaagcag cgtacgttgt cagctgggca 540

aacccctgct ttctctatac ctagtggagg aagccacaag gtgatatgag aactccagcc 600

ccagagggcc cctcaggacc tctccccagc ccagccccac ccagctccac catgtctgct 660

caccctcttt aactccttcc acagccacca gggtcacaag aacgagagac accatgagga 720

ccaagaagct gctggacccc atgttgccag gcaggtgctt ggctgggagc tgggctgagc 780

ctggctttat agtgttcctg ctgggcatgc cagtgggtgg agctgaaggc tcaaaatggc 840

aagggaggct gcacttccca gctctatttg gaaatggccc cttctgagga gggacttggg 900

gctttcccta aggctctgca ctggaataga tgaagaaatc caagagcgac cctcgttctt 960

cacacgagga gaagaaatgg acacgtgatt gaccattagg cgccaccagg gccaaactat 1020

cttatggaag gaggaaaaga agcacagaaa gggcatgaaa ttactcaagt tcacacacga 1080

ggcagtggca gggccagact tcctcaactc caccgtgttc tctatcagtc cctcttcatg 1140

cctgattcac ctgggaccaa aatcttccct ccttcctgtg ctgccccgga cttgttctca 1200

gttctcacct caccccaagg aagaatctga atggcagtgc ttaaaatgtc tgtcctgaac 1260

attcactctc agccttgcgc tccagcaccg tgtgctcctg ccccccgcca atctcatgtg 1320

gcctggagga aagcagattt tggacctgtc accagctgtg tgactttgaa agcagcttca 1380

cctttctaag cctccgtttc ctcattcata caatgagcat aataattata ctgtatcatc 1440

ttcataggat ttgttttccc tcctctgccc ctgcatcccc caaattcctg gatttggaag 1500

aagacatgga cgtgtgtgcg tgtgtgtgtg tgtgtgtgtg tgtgtgtgtg tgtgaccagg 1560

cttttggggt cctttctttg aatgagtact aaagggagga aaatccttcc tgttggaaaa 1620

gttgcagata taagcccaga aactccagaa atttggtcct ggccctggtg agagagaaat 1680

tctgagaaga taaatctaag atgctgataa aaagagatga gagatggaca gggagtaatt 1740

tagaggatat ctagtccttc actaaacttt ttcctgagat cagccccact catacccagg 1800

gttatgttac cacccaatac attcaccttt ctgcctaagc tggtctgaat tggatttctg 1860

tcatttacta acaggacccc tgagtaatgc aaagatttgg gtttggggtg ttccacatcc 1920

tgaagtgtgg aagggactga gttggggtta agtggaggca gggaggatac ctccattcct 1980

ggaagtgagc ctactcctta t 2001

<210> 71
<211> 2001
<212> DNA
<213> Homo sapiens

<400> 71
cctctttttg gggaaaagga cttcaagctc aggttttctt tggtggggtt gggtattgct 60

tcttccatat tccaagtctc aggaccctca ggggcaggtg ccaagtgaga gtgaccccca 120

gaggtagaaa ggacccagca tcatttctga ggacacctgg tagaggagga gcctggacac 180

ttggcctccc atcctggctc agggtatggc cttgacacat cttcatcctt gtttcctcct 240

ttggacaatg gagatgttca gactaggtgg tctctgaggg ccctgccagc tttagccatc 300

cctccccaca ccagctaaag ggactaattc ctccgaactt tcatgggtca gatagaggca 360

gggaagggac agccccagcc ctgggaggca ggtctcctta ccttcttcca gttccttcac 420

aggaatcaca cacttgaagc cacagtgcag gtaacaacac ttcctttccc ccagacagtc 480

ctggtctgtg tgacactggg gaggatcgga cttgaagcag cgtacgttgt cagctgggca 540

aacccctgct ttctctatac ctagtggagg aagccacaag gtgatatgag aactccagcc 600

ccagagggcc cctcaggacc tctccccagc ccagccccac ccagctccac catgtctgct 660

caccctcttt aactccttcc acagccacca gggtcacaag aacgagagac accatgagga 720

ccaagaagct gctggacccc atgttgccag gcaggtgctt ggctgggagc tgggctgagc 780

ctggctttat agtgttcctg ctgggcatgc cagtgggtgg agctgaaggc tcaaaatggc 840

aagggaggct gcacttccca gctctatttg gaaatggccc cttctgagga gggacttggg 900

gctttcccta aggctctgca ctggaataga tgaagaaatc caagagcgac cctcgttctt 960

cacacgagga gaagaaatgg acacgtgatt gaccattagg cgccaccagg gccaaactat 1020

cttatggaag gaggaaaaga agcacagaaa gggcatgaaa ttactcaagt tcacacacga 1080

ggcagtggca gggccagact tcctcaactc caccgtgttc tctatcagtc cctcttcatg 1140

cctgattcac ctgggaccaa aatcttccct ccttcctgtg ctgccccgga cttgttctca 1200

gttctcacct caccccaagg aagaatctga atggcagtgc ttaaaatgtc tgtcctgaac 1260

attcactctc agccttgcgc tccagcaccg tgtgctcctg ccccccgcca atctcatgtg 1320

gcctggagga aagcagattt tggacctgtc accagctgtg tgactttgaa agcagcttca 1380

cctttctaag cctccgtttc ctcattcata caatgagcat aataattata ctgtatcatc 1440

ttcataggat ttgttttccc tcctctgccc ctgcatcccc caaattcctg gatttggaag 1500

aagacatgga cgtgtgtgcg tgtgtgtgtg tgtgtgtgtg tgtgtgtgtg tgtgaccagg 1560

cttttggggt cctttctttg aatgagtact aaagggagga aaatccttcc tgttggaaaa 1620

gttgcagata taagcccaga aactccagaa atttggtcct ggccctggtg agagagaaat 1680

tctgagaaga taaatctaag atgctgataa aaagagatga gagatggaca gggagtaatt 1740

tagaggatat ctagtccttc actaaacttt ttcctgagat cagccccact catacccagg 1800

gttatgttac cacccaatac attcaccttt ctgcctaagc tggtctgaat tggatttctg 1860

tcatttacta acaggacccc tgagtaatgc aaagatttgg gtttggggtg ttccacatcc 1920

tgaagtgtgg aagggactga gttggggtta agtggaggca gggaggatac ctccattcct 1980

ggaagtgagc ctactcctta t 2001

<210> 72
<211> 2001
<212> DNA
<213> Homo sapiens

<400> 72
aaaaaatacc taaaactcat caccccttaa ttatgtgagt acatttttct attatttgtg 60

ctgtcgaggt tacttacgcc cattatgtct gcatgatggg actactcccg tacagtggtg 120

agcgaccttc ccatgtcccg gttcaaagac actgtagtgg tggtttgaaa gcagccctga 180

tgggaccact gacatcacag aaattggcaa atgctgcaaa gtagggctat cccaccccac 240

ccagagatct aactgttaca acatttacaa gcctgaaagc caatttgagg caaaaaggct 300

gtcatggaag aagattctca ctgtggagtt tgcgcactca gatttgagtc caggctccgt 360

ctccacccac tgtggggcct gggctatttc cccttgagcc tcgcctttcc tatctgcaaa 420

ataagactaa aagtacagca gcttttgcaa attgagcatt tgccgagtgt ggtggcttgc 480

ccctgcaatc ccagcaactc aggaggctga ggcaggagga tcacttgagg ccaggagttc 540

aagaccagcc tgagcaacat agcaagacgc tgtctctaca acaaataaat acataaataa 600

gcaagtcagg caggggggag cacacctgta gttctggcta cttgagagtc tgaggcaaga 660

ggattgcttg aacttaggag ttggaggctg gactgagcta tgatggtacc tctgtactcc 720

aacccaggtg agagcataag atcctgtctc tcttaaaaaa aaaaaagagc atttactacc 780

tgtcaggtgc tgaatccatg ctcttgctaa atctgccagc agccctgtga ggtgggtgca 840

gtcacgtccc ttgcgaagat ggggatttgg gggcgtgaag aggataagta cctcatcaaa 900

ggtgacagat cagatttgaa ccacactctt aactgctgcg gtttattggc cctacaataa 960

tagtgcccag ttcacagggt ggtttggggg attacacgac gtaatgtgtg ttaaagggct 1020

tagcacagat tcagctccag aggaacatag taaatgttga tttaccgaga ggcagaagag 1080

cgggatccac atccacccac gtttgaatct caacttggct attttctgat tgcgagatct 1140

ctggagtcaa gtcattttgc ttatctaagc ctcatgttcc cccagtgaaa atggggataa 1200

tcatagctac tgaagagctg ctcagaggat ggagatagag gatgtaaagt atctgccgag 1260

aagtgggtac tttatgggag tgagtgttcg ggattatctt ttattttatg tcacgcagac 1320

agcaactctt cccagccctg tggaggaggc cggaggttgg ggccggagcc ctggaagcag 1380

ggccttgctc gggcagcttc tgaccctccc ctcctggcca ggtcagcccc tcagtgggcc 1440

ctggccctag aaggtctcag aagcctgggg acgctgttgc taggcaccag ttgctaggct 1500

acacattgtt tctcctgctt ggcctcctcc aggagggctg gcttctctgc agagctctgc 1560

ttcctgcagc ctctccccga cctcagagcc tctgcagagg aaaggtgagg gtggggccgc 1620

tgtggttggg cctcggggag agtgcagcca attcccaggg gaggcaggca aggccagagg 1680

cgggcaaggc catggagatg agggagagag aggagccggg ggctgggagt gagcgcctcg 1740

gcctgacttc ttgggtgggg gccaagaatt ccccaggtag tgggttgtcg gctcattcat 1800

tccgagaggt gggtatgtaa ggttcaggtc aggggccgag tagtcaaaca cctaggtctg 1860

aatcccagca ctaccaccta ttatggagca aatcctttat cacttggaac ctcactgtcc 1920

cagtggtaaa tggggacagg aagagttaat aagaaggtgt gggaaagtga gaggagacat 1980

gaggactctt gggataagtg t 2001

<210> 73
<211> 2001
<212> DNA
<213> Homo sapiens

<400> 73
caaagttgca aggcactact atagatctcc catttcttcc ttttgttcac cagaacgtta 60

acaagagata caacacctag aaaacagaga gcagagactt ttctggatca aacagtacct 120

tttttttctg tttctgtctc cttgtgactc aagggagaaa tgatcctttc tttctaagtg 180

atcttatttc tctctgtcaa gaatccgtgg acatgttatc tggttagagc tcagcctgca 240

gccaccacgc aggtaactca ctcacgttag actataactc acctaactcg tttgtgtccc 300

agccaaatgt gtaccagagt aaaagttcag acctgaccat gggtaagttt aatgattaac 360

ataagccact caggcttttt cctcagggct tgttagtaat gaccaaagga aaagtgtctt 420

acatttttct ccacttactg catgtgcccc agataagaat tttaagtaaa agtccaccca 480

gtgagacaaa caggcattca acattgaaag aatttgccag caaatcagcg attgaacaga 540

gggctcaaga gaaaggacca ctagagggca gatccccatt catgaacagt ctctcactct 600

tcatcccgaa gagtgactcg taaataccag gttatgggct gttattcata tacccactgt 660

aggggtatgg gtctaaagaa gtacagaagg gaagtaactt ggtccaagtc ttgctgagaa 720

agggttgatt gattctcttc atttttctac tcctcatcct ccctcttgac cttgcttttt 780

taaccaagag gaggtccttg acttgtactc atcaccctcc agtcgtaccc agaccatggg 840

cctcagtcat cggttgaact ggtgggccta ggaaggtcaa caacaccttc ttacttggtc 900

catgtctgac ttctctgttc ctgtcccact gcccattctg attcacatcc cccaattcct 960

gatcatgttt gtctacgtct ggcgatacct gaccagaaga cgtccttatc ttctatcctc 1020

tccctttcct ttgagggaga agtcattttg gtaaaatttg atagccctga gtctccacct 1080

tcaaggacca gcagtagacc tggagaagca tctccagcaa aaccagatta aagccatgga 1140

aatacgacca caactctgat gacagtagta aaactatcct ctagaaagtg tggaagttta 1200

catcaactct gctgtatatc acagtttggc aacccgtgac aagctgaaaa atttttgtag 1260

ccctgaagct agtggtgtag agagagggtg cttggccaga ttggggcagg tagggggtca 1320

gtcaccccac caccctgcct gcctgctgaa cctctctcag tttccactct ggatctcttc 1380

catgcaggtc aacaagcatc atttgaacca catttgtgtg cctggcatgt gggtaatggg 1440

aaggaggggg aagactagtt tgggggtaaa ggagatttca acattattag taacttttat 1500

ttttataaaa atgatatgaa tcacttagat aattcaaagt tatattttta aagagtatga 1560

cacagtttct gccttcaaac tgcttaccat ctggcagagg agacagaaat ataatcagat 1620

tattttatta tattgtagag tctcctgata agtttcctaa gacttatgtt ttatttcttg 1680

ttatttcaac aagaggtttc cataaattag ccccacatct atccttggct aaaaggccta 1740

taataaatat ttataagtat ttttggcagc ctagctatca aggccgcatc ctaaaatcac 1800

cccaattttc ttatgaggag acagccacct ctctactatg gactaaaaga atcctaatga 1860

cacccaggac tttggtttct gtgctctgtg ttataggtat ttcgtggtgg acaatcaagc 1920

tctattgtaa aagaaataaa ggacaatgaa tcatttcttg ccttgaggcc taaaaggaaa 1980

tgttgtagaa ctctagagaa a 2001

<210> 74
<211> 2001
<212> DNA
<213> Homo sapiens

<400> 74
ccaagaatga gttttcaaaa cccgagttca gaagaaggat tctgagtggt tggttctcag 60

atctctgagg aagcggaagg atgaccctag ggggagggcc accgcatgtt agaacccagc 120

agggggctgt gacagacagg ctggtgctcg gagcttcatc tctttctgcc gttttcctct 180

cagtttttgc tctggttatg tcatatttct cattttcaac accaacatga gcctttgtac 240

agacattcct ccacctggaa ccccatttca gctcatctac ctgttgcttt gtatttattc 300

ttcaaaactc tgccgtcttc cctgaccttc cggagccggt gtttgtggcg cctcctgctg 360

gtcactgggt tcaggcagtt tggagctggg tggaaggaac gggtagtata ttaagtcttg 420

ggccctggac cttgttggct gtcctctccc tgctgaccta aatgacaaag ggaggggtgt 480

ccagtcagag tgtgagggct gtacccgtgg gcagctgagg gagtgcgagc cacaggccag 540

gcactctcct tctcccatta gctcagccgt ggcatcggac ttgcagcttc attttgggct 600

gccttagcca tgaagctcct tttgctgact ttgactgtgc tgctgctctt atcccagctg 660

actccaggta acctgaacct ccttaaggaa ggggcagggc ttagagacta aggcctggtc 720

agagaatccc aagggctaga aggatgggct gcaggttgtc atctagcacc acgtatagga 780

ggcaggaggc gcctcaccag cagcaggtgc cagctgatag atgacaacta caacatccag 840

agctgctctc tcagcggctc catccctcct cttcctgccc agctccaggg agcatgtggg 900

ggtggcctag tgtgtgagga ggagtgggag gggtgagcaa gagaagcagg aaagaggaga 960

ggcaggtatg gcccttttgc aaaatagtcc ccagaatgaa cgaagctttg tgaagatcac 1020

agctacctta atgggagaga aagctcattt gtacttagct gtctctgtgc cagactctgc 1080

gctggaagct aggaccacgg agataaatga catggtgcca ggggaactaa ctggaggaga 1140

cagttaaata gcatttataa cccagggtga cacggctgtg gcagtgggaa accagggggc 1200

tgggtacaca tggtccagcc caggctgagg gaaaggagag tcctcctgga gggatatcct 1260

gagtcctaaa aagtgagtcg aaaatagaaa aggaaggaga ggggaaagca gaagggtgtc 1320

ccaggcagag aggtcagcct atgcaaaggt tggatgcggg agagagcatg ggagtgtcgg 1380

ggacctgcat aacatggagg ggtgggttag gagagaggcg ggggtaaagc tgaggaagca 1440

ttcaagctcc aggtctcagt gggcctcaaa tgccaggtga gggacccatg ggaaggttta 1500

aacagagaaa gaccctggtc agacctacca cccaccaatt ccagcatgag ccacctatcc 1560

cttcctgctc atctggcaga agtttccacc caccctaagg ctgtcggcta acctttctac 1620

agagtcagaa acgtgtgaat ttcctaaaca agctgacctc cgtccttgtg tcttcctgag 1680

gccctaaaac accactcacc tggagtaggg aggaaagccc agaccccggg ttaaaacctg 1740

ccccatcatt ttctgactct gtgagcccca acaagaagct cagctgctca gagtatcaat 1800

ttcctcattt ctcaaactgc aataacatca agtgtgtaat aatatcaggt tgagggaaca 1860

acattgaaca cactgaaatt ttggtctgca aaagaaatat ggcttctcta ttttctctaa 1920

ctcaccaaac tttcccagga acttccatcc tgactgtggg agttagtggt gggaggagca 1980

ggactacaga gataaatgac a 2001

<210> 75
<211> 1920
<212> DNA
<213> Homo sapiens

<400> 75
cccttggtag gtcctatctt atgctatcct tggtctctca tgctattaga aattccacat 60

ttttttttcc cgatcctttc tggtgggcat ctctctagtg tgactcctct cttgaggttt 120

tggctctgga gggctctttc caaccccaga acatgttcct agtcagagcc ttttctccca 180

gtctctcaca tcactgccac atggtggttg cctggattca gtggagaagg tggctgcaaa 240

ctctagttgg acactgacta cacagatggt ttcaaccaaa cccagactgt gcactgcctg 300

cctccccata aagcctgtga ggatggggac catgactctt gctcacccct ggatcctgag 360

ccccaggaac agtggcttgg acaaaatact ctagaccctg ctcagtggcc aaatcactgt 420

gtggtataga aagagttctg tgccatccac gagacccact tccagacagt tttccagcac 480

cacacttatt agccctgcct tgatggagac agcatctcct tggcatttaa gcagttattt 540

ggcttcaaat gccctgagcg cccacacagt ggtcctttgc tcactaggtg atattatcac 600

tcagtgtgga aggctctgcc cctcgttctg cttttgtgtt ttcagttaat ctcccccatc 660

agtctggtag ctcccggtcc catttttgtc tttctgtctg tgtcctagaa gtatgactac 720

ctttcccttt ggcctgggaa cttctgtggg caagttcttg cctttgactt ccctgtcagt 780

gcccagcctg agttctgaac atgagtaggg gttcaggaag ggtgtgatcc aggacgacac 840

ccaccttgtc gatgaaggcc gcgaatctgc tgttgaggga cttgatctgc tccttctcct 900

cctgcttcac gcactgcgcg ttggggtcta tctccaggtt gaggggcgtg aggaggctct 960

cgttgaccga cacggtggtg atgcatgggg ggctgggtcc gcacacgccc ccggagcggt 1020

agccgaagct gcgtccgcag gagccggcgc ggaagccccc gcacacgctg tggctgccaa 1080

agcccccggt gaggccgcgg tagcaggaga tgccgcggta gggggcggcg gtgatgcagc 1140

agcggcttgg ccggggcccg caggcagaga cacagctgaa gtttccaggg cggaacccac 1200

agcctatgga gttgaagcca caggtcatga cggaggttag gaggtgtctg aacagtggag 1260

tagatggcag aggatggaac caagggcctg tgctccctgg ctgatggcag gcccttttat 1320

gtgccagggg cagctggtgt taaaagaggg agcaaagaga caatagctga attttattgg 1380

cttggcatca ccctggcctc ttaagctaga accctgcttg cctcttcagt gtggctgcat 1440

caacaatccc tggccacggg ccctgtttca tcttcaaagc cctttataag cttcctcctt 1500

gctcctgccc ctcccactgg cactgctgtg gagcggggag aggggttcct gaaagcatta 1560

gttgcattct gcggtccaag cgaggggaat gggtgcagtg acctggggta aagcctgctc 1620

tctgaaccct gttctgaaag gggcagagct aggctatggg ccctggaccc atggccaagc 1680

cctggggtgg agtggctggc ctgtcccaag gagggggctg cctctgagac aacctcttgc 1740

tgtaggcttt cctgtctttt tctcaaaggc gggttccttt cctcattgtc aagattggcc 1800

gctcctatcc tattcctgct cagagcctgg tcaaggatgg gagtgggggt agagatttgg 1860

ggtgggagga gggcatgttc tttgcacttt gggtattcct agtgcaggga caccatctgg 1920

<210> 76
<211> 2001
<212> DNA
<213> Homo sapiens

<400> 76
cattattatt cccattttac agaggaggaa actgaggtaa cctgatcatg accacactgc 60

cccacagtag aggagccaga atccaaaccc aggcagcctg ccttgccacc agagccacgc 120

ttttcaccct caagctgcag ttaagaagta tcacgccgtg gctaggccca cggcctctgg 180

cttcacatcc cactagctct gccatcttta gctgtgtgac cttgggcaag ttacttgtct 240

gtgcccgagg gtcctctgta aagtggggat agtagtagct gcttcactgg gttcccttca 300

atgagttaat agctgtaaca tgtttagaat ggtgccctgc acatagtcac cagccagtac 360

atatgggcca ttattcctga accctggata ggcacatgtc cttgaagagt gtagaattct 420

tcaggattct gaaatgccag cctgtgggat cctggaatcc tccaatagca ttctaacagc 480

tctcagctgc agaactgtac cctgttttct agagctggaa ggtgtgaggc tgcctgtcca 540

cccagctcaa ccccacagta ggcaaatgag gtgatgtcaa agccttggct ggggcccagc 600

cccggtaacc tcccactctc cctcccctgt gtgctccctc gctcagattc acacacacac 660

tccaggccag ccgggcccag agggaaggtg agagggcaag gagtaaaggt ggctgggtgt 720

gggtccgttg aagcgagccg cctccagccc tgttgaactg gtgggcccag ggactggagc 780

gggattgaaa gggatcttgc tctcccttga agccttgagt tgcagcgatt tcagtgtctt 840

ctctccctgt gtaagcctgt ctcggtgttt aggctgaact acagccaccc cctctcccgg 900

gggtgtgcag gccagggact ggccaggcag ccatggctga cgagaagacc ttccggatcg 960

gcttcattgt gctggggctt ttcctgctgg ccctcggtac gttcctcatg agccatgatc 1020

ggccccaggt ctacggcacc ttctatgcca tgggcagcgt catggtgatc gggggcatca 1080

tctggagcat gtgccagtgc taccccaagg taggtggtag tggggctggg tggggccagg 1140

tcagctgggg ccaggagggc tggacactgt gcctgtatgc catgcctcac cccctatcct 1200

ttagttgtct tttcattctt ctcattttga ctcggtcttc tctctgctct cctgagcgtc 1260

tgtcccatcc atccatccat ccatccatcc atccatccat ccatccctta gcctaagggt 1320

tgatcagaag gcccctggat ctggagccct gagccggaag ctatggtata gacacagcaa 1380

acacaacaca ccatgtgctt gaaacaaaag ctaatgagcg ctgaccccct gccaggcact 1440

cctgcgttca gggtgcagtt cagccctcat cctaagcctt ttggtgcttt ctgtagttcc 1500

accttctgag cctttctgag gtccagaaag agaggctgca cagctaacga gcaaggtttg 1560

gagctcagat gcgtgtgact tacacgtcac ccttcacctt gggtcttcta cctctgggtc 1620

ccagagcgac tgagagctcc agggtaggtc aggtctaacc ttggtggcat gcactgggca 1680

gtatccttta gagtcttcat gtattctcag ccttatgtgt aatttagtga tgggaagagc 1740

actggtttgg gagtcctgag ttctagttca agtcctgttc caaactctct atgacctttg 1800

tctttctgag cctcattttt ttcatctgca aatgagatcc ctgcccctat tttccacaga 1860

gcttcacagg gtcattataa agatcagaga agagataaga tataggaata ttttgtaaac 1920

tgtaaggtgt tatttataga tgaagggcta ccactgttac ccatactgat cctcccaaca 1980

gctctgtgga atggaaatgc c 2001

<210> 77
<211> 2001
<212> DNA
<213> Homo sapiens

<400> 77
gtagcagagg agctttcaag catgaaagga gcctatggga gggagcaaag gcaccaaaag 60

atccctccct ccccccaggc acccacagga agaaccagca ggccatgagg tcccacacca 120

cccattctgt agtgtggttc cttgagagat cagggccaca gaagacttag aagcgcctga 180

gcagcttgcc aggaagcagc tagggacagg tctgacccct acatcctgat gctatttctt 240

ctggttagtg gaaatccctt caccaacacc ctcccagtct ctaagaccct ttcttcagac 300

tctccccttc tctgccactg cagctggagg taagatgtgg ggaagaaagg ctagtctttt 360

cttaaataaa tcccagcttt ggctcagtcc ccctcctgac agttgttgga accctcaggt 420

ggagaccacc aaggactctg gggcaccagg gactagagga caaggtgaca gaaggcataa 480

ggtacaggct acaggcagct ctggaaatgc catggtggga ggggagtccc agtgccatcc 540

tgccctcccc ttcccccgct ccacactcac catgcagaca aatatggctg agctggtcca 600

caagttccaa caggttcatg accttcaagc ccttgaggac aactttcaca gcctcctttt 660

ctccatactt tgaaatcagt aattctgcca ggtccaccgg aatcaggccc tccaactccc 720

ctctggccag tgggggctgg ccctcagaca gggtcatatc ccgtaagtag aactttaact 780

tcttgaaatc gttctcctca aggtcactca aggcccagag caatgcctcc cggggctttc 840

tggccttggc catggccatg gtgatctggg ggaaggatca agtccagacc agaagaccag 900

tgacctggag aaagaggcaa aaggagaggt ccactgctga gaggcccacc ctgctttctg 960

ggggcttggc caagttatag agcggaggcc agggagagac gagatacatg tgcaaagctc 1020

caagaaagac aagcagatga aacgcagggg aattttccta gtcattcccc tctgcctctt 1080

agtcaggtga cacgtgactg tgttggcaaa tgggtaggaa ggtatgtagg tgtgttaatc 1140

tctcaatctc tctctctctc ccccaaactc tatctctgtc tctctctctc cccccactcc 1200

cttcctcttt ccctctcaac acgttcttta ttaggctctg tatgcctcct tagacggatg 1260

gattaagaaa aaaaagtcta agttttttat tttatttttt ttggagacag agtctacctc 1320

tgttgcccag gctggggagc agtggcatga tctcggctca cctcaacctc cacatcccac 1380

gttcaagcaa ttctcctgcc tcagcctccc aagtagctgg aattgcaggt gcccaccacc 1440

atgcccagct cacttttgta tttttagtag agatggggtt tcaccatacg ggcccggcta 1500

gactcgatct cctgacctca ggtgatctgc ccgccttggc ttcccaaagt gctgggatta 1560

caggcatgag ctactgtgcc cggccaagtt ttaagtttta aaagtgagac tgtccctggc 1620

tgtgtgaacc tatagaaagc tggagcacat ttgctggaac tgctgtggta tggcaagaag 1680

gtaaaaaaaa tctacactca acccattggc ctcacatgtc agcctcctgg cctcagtcac 1740

acagggattg caaagctaga agtctcctaa ttcaaataag gaaacaaatc cagataatga 1800

taggaagagg aaccgccacc tgtgagtgct gactttgtgc caggtgattt acatatcctt 1860

tttgatcatc tcagaaatcg tctcaggaac tgttccacat gagaaatttg gcagtcagag 1920

aaaaatggtt tttcagagtt ttccagagct aggccaccgg gcaaagaggc aggatttaaa 1980

ctggtctatg ccctgggtgg a 2001

<210> 78
<211> 2001
<212> DNA
<213> Homo sapiens

<400> 78
aaattaatag accataaatt ctgtaatgtg aatctttaga caaagactgc taaatctatc 60

atctgatata tgaagtttta ttacaacaaa tgtttatata ttgagttaaa aattatctgt 120

acccatacac acaacctgca tttacttaat ttaaaaggag tgtcaatatt aaaattgagg 180

attttggcca ggtgcggtgg ctcacgccta taattccagc actttgggag gccgggtaga 240

tcatttgagg tcacaagttt gagaccagcc tggccaacac ggtgaaaccc cgtcttgact 300

aaaaatgcaa aaattagccg ggcatggtgg tgtgcccctg taatcccagc tactggggag 360

gctgaggcgg gagaattgct tgaacccggg agacagaagc tgcagtgagc cgagatcgtg 420

ccactgcact ccagcctggg caacagagcg agaacccatc tcaaaaaaaa aaaattgtta 480

ttttgttgtt attgttgttt ttgaagacag agtctcactc tgttgcccag gctggagtgc 540

ggtggcatga tcttggattg tgttcttcca gggttgcaga gagaagtgca acagcgtctc 600

attgcaacct ctgcctccca ggttcaagca attctcccgc ctcagcctcc caagtagctg 660

ggattacagg agtgcaccat cacacccagc taatttttgc atttttagta gagatggagg 720

tttcaccatg ttggccaggc tggtctcgaa ctcctgacct caggtgatcc gcctgccttg 780

gcttcccaaa gtgctgggat tacaggtgtg agccaccatg cccagcctaa gaataataac 840

tttataacta tgaaaaatgt gttacatatt caccattttg ccagcattaa tctcaaagca 900

taagttttac cttttgggga taaagagata gattgtgttt tagccatatg accaatgtag 960

aaacaagcta aggttaactc ctcatctaat gaataggccc gataaaaaag ttggttgacc 1020

acaacatgaa taaataaata accataagct tggaaatgct acctgtagta tttcagagtg 1080

tcgatcacat ttgtagtcat cacttaaaga attaatgtct gccaatgtta tcatctaaaa 1140

tgtatttaca gaggtcagaa gagctatgtc tgtcctcctc tatgcaaaga ggtgatgaca 1200

tcaaggggta tgtcacaagc caaagtttag ttagcctaag ttagttagct ggtttgcagt 1260

ttttatccac taagtctgat cttctgaccc ctcagtttta caactttcca ctctctgtgc 1320

tgcatagcac ctctccactt ctgagttaat catttctttg ccattatctg gcaagaattc 1380

ttatctcttt tgctgtcact ttataacagg gtcctcttat caacctgcat agagtcatgt 1440

gatgagtcaa agcctatcaa aagtttccat tcttctgatt agaaaccatc atgaaactgg 1500

atacatggtt tacagcaggt cactaatgtt ggaaaaagta cagagtccag ggaaagactt 1560

gcttgtaact ttatgaattc tggatttttt tttttccttt gctttttctt aactttcact 1620

aagggttact gtagtctgat gtgtccttcc caaggccacg aaatttgaca agctgcactt 1680

ttcttttgct caatgatttc tgctttaagc caaagaactg cctataattt cactaagaat 1740

gtcttctaat tcagatactg gggatttaca agagtcttta aagcacggac ttacacctat 1800

tggtaagtag gagtttctct attgatcatc tatttattct gctactacat acatataatt 1860

taatcttgtt gatggatttt gcattttaag tcatggcttt tcctattatg tttattaaaa 1920

gcacacaata agcctatgta tatatatcac acatttatct ttcttgcagt tcaaaaaaag 1980

caaatggata tttcaacata t 2001

<210> 79
<211> 2001
<212> DNA
<213> Homo sapiens

<400>  79
aatggaacta ataacatttt aactacatta aaattcatga atcaaagggc ccatttttga      60
atagtaggaa gaattgttta tctaaataaa taaatacaag ttataacagt catgaagcat     120
ttcttattgt atgtcttttg ttaggtattt cttagtttta aatacttggg ttagttccaa     180
atctgtgatt tctcacctta ttcttaagat cctcaattgt taggtgatat ctgctatagt     240
catgatcaag tccacggcaa gatccaggtc cgtatttttc ataccaaccc ttgatttttc     300
tctctaattc agcatttgcc tcctccagag ctcgcacatt atccaggtag gatgccaagc     360
ggtcattaag attttgcatg gtcaccttct catttccaga gaggagtccc ccttcgcttc     420
cagcaaagcc aatacaagca gcatttccaa gggcaccacc agcatggctc ccaccaggaa     480
cactgcccaa gccccctccc aaggcacagg aaaattcact tccagcaaca gagccaccac     540
atgcactgct gcctgcaaag cctgcacctc cattgagggg tctgacagat ccagctccag     600
accttaagca aacatgcctg gatccattag aaaattggag agacatggtg ttttgagaaa     660
tgttcacctt gtctatgcaa aactgtaatg tcccaagaga acagaatatc atgcactgat     720
aatttctttg ggtttttata tatcattatt ggggcgttcc tatttgagtt atacatgcca     780
aaagcaaaat ggcatcactt agattagcat aaaattatgt ataattgggt gattttaaaa     840
ataattaatg tctaacatcc ctcggtgttg ctttgggcta tttcttatct taaatataat     900
agggtttgtt ctatattaag tttattaatt taacttctaa ttttgagtga gtaatccttt     960
cctttcatct agaccctagg catatactgt cattatctgg cgccaggtat tatcaaattt    1020
tttataaagt taagtaagat tatgatagag ttctgtgagg tacagtaggg tctcaattac    1080
catggagggg tataaaggac tgctaattac agaaacagat attttatccc accagaaaaa    1140
aatgtgttat ttagaaaaca gaggctagtg acatctctgt tcttaccatg attataacat    1200
ctatttatta tcttggacaa tgacacaaat attgacctat tattttgttt aaaatagcat    1260
ggttatgatg tttggtgctc tatgctaagt gctaaaatga aagacatagt gttaactaaa    1320
tgcagaagtg aaaaatgcgt gattacaatc tttaagaaat ctattgtcag gtccagacag    1380
gggctggatc atggttagct ttctgtgctg gtctaaagag tttgtaatga atcctaagta    1440
aaaagcagag ggcaaaatga tcaggttgaa atgaaacaag gtggtcaatt acaaggttgg    1500
aataatggtc caggtgagaa ggatgaaggc agtggggatg cagagaagat ggctttgaga    1560
gcatatggta atatgattga atttggagag tagggaataa ggcagaattg gggataactc    1620
ttaggtaatt aattttagat tctgggaact cataatattc actgagatgg ggaatagagg    1680
aggaggaaga cactgagttt aggtttggac caactggatt tgaggggttg ccagaatgtt    1740
ccagatgaag gtgactgata gcaagaatga ctgtttagaa tatgtatact gaactggaaa    1800
aatacttgac tagcaatgag taaaagaaaa ttggagggtc cagctctcat ttataatgat    1860
gttcaactgc atatttggaa gtaatcctgg tgcaggaata gaaaggatga tgatcagatt    1920
gatgtgggtt ttgggatttg aagacaggtc cagaagaagg aaatagtggc aaagaaattt    1980
ctggtgttct tgagaaaaca a                                              2001

<210> 80
<211> 2001
<212> DNA
<213> Homo sapiens

<400> 80
ttttcatgca tgccacgccc gccgccccca tccagtcagg agggttggcc taaaagctta 60

gtctgtatcg aaacacaaag atgggatgtt gtgtccatcc tcctcccccg atttccttca 120

cagctgctcc ctgaagaaga aatgagatat tgttttcgcg cgcgcgtgtg tgtgtgtgtg 180

tgtgtgtgtg ggtatgtgtg cgagagagag agagagtatg tgtgagagaa agataaagta 240

tattttcatc caaaacattt ttttttttgc tatgtggatt ttctaattta atttttttgg 300

tcagaatgca cttttaagtt ccagcaggac gtgttgcaac atgttttcaa acatctggta 360

tttatttttc atttttgtgt aaatgaaaaa tcaacaagct catttcctga agtggaacat 420

ggagttcctg actccactaa tgggagtcag gtaatggcgc gagggttatg agtcaaagct 480

ggctttctcc aggatcctgg gcagccccct aacttctctg agccacagtc tcctcgttgt 540

aagtaggggc tgtcattctt tctccaccag agccagacgt gtaggggaga taccagttct 600

ctaacaactg gggctaaaag ttgagaatgc agaagacatc ttctgtcgct ctgttctctg 660

tcccttttat gacccttgca gcgagaagtt caagacagtc tgtcactgct tgggggtggg 720

ggccggggtg gtggggagtg tggacttaaa cccaggggtg cttttggtcc agccttatct 780

ctcctggtgg aatgaacaac tttgactctt gtgagtgaga ttctatgtct ttgtgatagg 840

caggaggtat gtttagagac atttagataa ggcagtgcct tcgtagtaga cttagactcc 900

taaatgaaat tcattctaaa atatgtaagc aacagagata catttacagc actgagtcta 960

taaacatgaa aacaacaaca cagatccgta acatcccaaa cgttgtgagc gctttcttta 1020

agatgttctt tgccttttca gaaacataag aaattcctac taccacccag ggcctgcctc 1080

tcgctgacct cctgtgctgc cctcccctaa gagctctgtg tagctccctt ccccactcca 1140

ggtccctgat cagatgacac ttttccctaa aaagcttcct gacctttcaa aaatagcact 1200

cttgtcactg tccctttccc tgcttcatta tatctttacc tgaaattctc tcctttattt 1260

atacattatc ctgcttagta tgttttcccc aggaggatag gagcttgagg cgtgccagga 1320

gtttgtctct ttgactctgt gttgtgcccc agtggctaag cacatactaa gtgctcagta 1380

aatacttata aattaaatgg atgctatcca aagaacttaa tgagtttcta agatatacaa 1440

aaggccccag aggtaatatt cacgcgattt tgatttcaat tgtatttctg gagaagcact 1500

agcctgtatg tttctgtctt cttcacctgt atcacatttg ccaccaagaa ttgcctgtca 1560

ttgaagaaat acacagttag cctcttttca gcctattaca aaaaattctc tctcctctaa 1620

gcagttgcac agttttccac aaagcactgt tgcctaagtt tccaaatatg tatgttggag 1680

ttggagatat ctatgtattt tagccctcag cactgaagtc ggcaaccatg gagctgaagg 1740

tccaggtcta ggagtcacag cgggagaaaa gccacacgat cttcctaggt ctcagttcat 1800

tttttctgta aatattggta agtgatatat tccttaggga aatcctctct tcacatgttt 1860

ctggaatgta tagccagtaa caaattctta agcagcataa aacaaatcag acctatcttc 1920

taagcaagtg tttattttta agtcccaaaa taaatacaca tttgttcaaa acaattaaaa 1980

tagcaccaaa atgcaggact c 2001

<210> 81
<211> 2001
<212> DNA
<213> Homo sapiens

<400> 81
ggtagagttc cagaaccggg gagatagcag caaagaccct cccaggtttt ccctcatgaa 60

cctcagttct tgggaggctc ccccttggac agtggccttt atctgttccc agggaagctg 120

tggcttgggc ctggagctgg aggtggaatg atctttctgg gagcagagag gcccagagag 180

tgtgcatctg gggcccctgg ggactttgtg ctgaaaggag agaaagttag tgagatttgg 240

gtcacctggg ggcttaccca ctacaaagag agtcacaaga gagaagacca aggtcacagc 300

cataaatgcc ggggtagcct gaacgagcct cggtatcttg ggggctgcag gagccattgc 360

cacagagtcc acctctgcag gggagaaggc agtgtccaag gtgagagggt gctgtgcaag 420

gtaagagggt gctgtgcagg ctgagacaca gcctctctga ttctgtttcc tgggttgtct 480

aatggggatg ataactcctg ccatgcttat catgcaggtg gttatggaga aggaagacaa 540

aacactttgg gctgttcacc agagctagat gtggatatgg tagcaacaca gagctgagga 600

gctctgagac cgagaccacc gatctccaaa tctcaaggag gctttggtgg tgcacagggc 660

acagaacctg gcacagagca cagagcaaca aaagtgtagt ttgaatgaaa gaagaaagga 720

agggagggag gcctcacagg accagctcag tgtgcagagc cgggattcca agccaggacc 780

atctgaattc aaagttcatc ccctttctct tgccaggtct gcatccccaa ggacaagggt 840

ctggggagag caataagaca gcaataagct gccttatggc agagggccaa aggacaggca 900

ggctactccc tcactgggca agagctgggg ccccagtttt ctcaccttgg gggtgcaggg 960

agacacactg gttatctgtg cagaagcgga ctgcctcacc gtccatctct gcttccttga 1020

tcctccagca ctgctcccag ccactggctc ctggaagggc cgtcccgtgg accaatggca 1080

gtggaagcaa agctgagaca cccacactta tatgctcctg accaccctcc cagctcttcc 1140

tccctccctg cctgactctg cagaaacccg ccccagtggg cccctcccct agaggctccc 1200

agatacccat ctgccctatt ttcttcctct tcccattagc accttcccgt cagttctcag 1260

ccccatcatc ccagcctctg ccgcatccag cataacccca aacacacctc ttcttctctg 1320

ggggctggga ctggcaccta ggaagggggt ctcagctttg cacagaaaac atccttatga 1380

ctatggctga cctttaattc tagcctcaga acctcttaag taaaaggagt caggtaaaag 1440

aggggccagg tcagtgcggc cgatatttgc aggtcaaagg aggaagcggg gtgaccagcc 1500

acttggggac tcacaactga attatagtat acaggaggat gtgcggaggt tatataaaaa 1560

tactacatga ttttgtacaa gggacttgag catctgtgga ctttggtacc catgggagtc 1620

ctgaaacaaa tcccctgcag ataccaaggg acaactctgt aaaaaatata tctggtcttc 1680

atctcagttc ctgacacaga actcctaaaa cgcttagagt ttcctgggtg acaggagtgt 1740

tttttgttat tcataataag ccccttagga ctacacttgg gtttatgcta atgaggtgac 1800

acagggtgga cccttagata atttcaggct ggaggctggt cccagaaaga ccaaacatgg 1860

gattagactt tggaagactg ggactttcag ccacccactc ccattcccta ctcccagacc 1920

tccaggtatg ggagatggcc tggggtctgg gctataacga ctcttaaaaa atgagattca 1980

aggagcttag ggattggtga t 2001

<210> 82
<211> 2001
<212> DNA
<213> Homo sapiens

<400> 82
cagtgccctg gagccggttg ggttgagcgc ccctgcccgg ggaaggtagc ctgcgggctc 60

ttgccccgag cccgcggagc aggaggtctc ttttccaagc gcactcacat tattcatgca 120

aagttaatcc ccgccgcgtc acggccgccc gcccgaggat gtgcgccttc cttggccaag 180

ccccgcagtc tccaccctcc tccgccccct cctcccggcg gcggggtccc ggggagcgcc 240

ggcgactggg cgtcgcgcga gtcctgcaaa atgtcaactc cttgggcgaa gagaggcggc 300

cgcagccagg gccgcgccgc acctccccgg tctctccact tttataggcg taggaagcta 360

ctgcggctgg ggcaccagag tccccgcctc tccgggtccc ccgcgtgccc ggcccgcccc 420

ggcccgcttc ccgggcgctg tcttactccg ggcccggggc gcctgctccg cgccgcgtct 480

gcgaaccggt gacctggttt cccctccagc cctcacggct gtccgacttg cgcggcggtg 540

gcggcggcgg ccaagagcag gcaaacccgg ctccgccagg ggcgcagcga ggaaatggcc 600

tcctggcgca caccccgccg ccgccgccag ccatcgccac cgccccgggc tgcccagccg 660

gtacagacgc cccctgccag gctccgggcc agcgctccga cgcgctcaca gcgcccggct 720

tgccgacccc gaccccgacc ccgagtccta cgcactcgag gtccccgggc cggccgcctc 780

cgcacctgcc tccccaggtg tgggcgctcc ccctcctcct aatcccggcc gtgccctccc 840

agggttggtg acggtgctgt gggtttggtg gccgcgcgtc cacccacccg attgcctgcg 900

cgcataggtg cgggtttggg cgtcattact ctagttggag aagaggaaag aatggggaac 960

gagtgacacc gggaccggag ggcgagtctt ccaggagcac gtctcggcct tctttgcccg 1020

gcccgaccgg cccgacccgt gccgcagcgc tcctccctcc gctcctaaac cacttgtggc 1080

gcgtgtcggg aatacagggc gctctggctc ttcttggggc ctccgctggt ccgaccatct 1140

ggcagctctc ttaaccacca cggccacccc acgaatggaa gtttgggacg tgcctgggtt 1200

gcccttaagg aggacacccc cgcccccacc gccattatgc atatcgtggg agaggcgcgt 1260

acagggctga gacactcctc aggtcccaga acggccgccc tattaatttg tctttgccct 1320

ggaaatgtat agataaaacc aggaagacaa aaataatgta tactcttcaa gagatctttc 1380

ccgggcaaat ataggtctct caaatgcccc cgaccttaag tcctggagac caggaagaga 1440

cttgctttct gagctagaag gatacttggg aaataccgag tcctcactca gctaaaaggt 1500

agtcaataag gaactatagt tgcaacaaaa ctactttgac aacagagtac aattaatttc 1560

ttatagacag tattaaaata attccgacat cataatttaa tattcacatc tattcgtgta 1620

acacgcagta taatagagtt atgacgatat ggatcctcag ctgcctgaca atgtataggt 1680

tatttattgg aaggttctct agatttccag ccagtgacat gtatgtttta ttctcgcttc 1740

ttctgagcgt cccagggaaa ggggtgagag gccccccacc cctgctgaca gcattaacaa 1800

ccaccccgct ggataagcag ggtttcactg atgcactttt aagaaagttg caggccccag 1860

agaccccatt tccctttggt cctcggtgag aagtccgtgc tttcttctct actcttaata 1920

agtctgcacc ataggacttg caggcttctg tagttggaga gctgttctcc tttgcaggga 1980

agttcctggg ctttgctcca a 2001

<210> 83
<211> 2001
<212> DNA
<213> Homo sapiens

<400> 83
gtacacactc acatgcatac acacacatat gctcacacac atgtacacac acacatatac 60

acaccacaca ctaacatacg tacaggcgca cacaccgcta cacacacaca catgtacaca 120

cacacacatg cacacacttt ggatcacagg ctcaatttct agaccacacc tggacctcgt 180

taaatgctgc gggagttaag tgatggaaac tgacccttcc acctgtatgg gtctgcacgt 240

cattccctct gctgcctcaa accggcagcc atcctgtctc ctcagcagac tgggacctcc 300

ccaagggagg acagggctgg aggcttctct gggccgtctc aggcctcacc caccttgtcg 360

atgaaggccg cgaacctgct gttgagggac ttgatctgct ccttctcctc ctgcttcacg 420

cactgtgcgt tggggtcgat ctccaggttg aggggcgtga ggaggctctc gttgaccgac 480

acggtagtga tgcatggggg gctgggtccg cacacgcccc cggagcggta gccgaagctg 540

cgtccgcagg agccggctcg gaagccacct acagctatcc ggggcccgca ggagcccagg 600

ttgcagaggc tgcggctgcc gaagcccgtc agccctcggt agcaggacac ccctcggtag 660

ggggcggcgc tgatgcagca gcggttgcca gttttggggg ccacagctga gcaggagctg 720

aagttcctgg tgaccccgca tcctgagctg atcctgtagg agcggcacga catcgtgtga 780

ggctgagcag agtctgagag gcagcggaag gtggtgcggg cggcagagtg cgaggctcag 840

gatccttctg ctctctctga tcctccctgg tccttttatt ggtggggaga gctggggttg 900

gctgcataaa aggagtggaa agccatttta tgttgtttat gggcttgggg agtttgccag 960

caactcccca cggactcatc ttaaaggtga gctcagcagc gactctgggg ctccctgaag 1020

tcttgaagat gttattaagg agactgcatt tgaacttttc atcttcaaag ctcgtttaca 1080

tacattaagt aattaatttt ctcacagtct ttctgtgaga tggcctcaac ccacacttgg 1140

agacggattt gaactccaca acagcctggg ggtggctggg tttgggatac aggcaggagc 1200

cattgtctca agggagaggt tggagggtcc tgggttaggt gagacttggg ggatcattgc 1260

attcatctat ctggttccac agaggacaca ttctcaagta aatcattgca tttcaggggc 1320

caataggtca ggcgaatccc accccatcta ggcagcattg cttggaaggt ggttcaggac 1380

cagagcggtc attttgacca tgcccgtgcc aggataaaag gctggggcag atgatctctt 1440

ggaaccttct ggattccagc ccagaaggcg ctggtccaga gctttcactt ctcacccagc 1500

aaaagtgcaa accacagaac tctcagtggc cacaataatg gctacttgcg gattatgggg 1560

agactccctc tagccacttt cttccattcg gtcaaaagga ttggagaact caaccttggg 1620

ataacctggg ggctataaat ttgctgtggg cacctttgct ttgctcttta agaagaaaca 1680

ggtcatgtga gtctcccttc ccagaccacc agctacaaga ggtattcctt ctgttggcca 1740

gcccttgaat tgtcatccct agaggagtgt ttagagactg cattgcacaa agctggaagt 1800

gggatagtgg tttattcttg tctaaggtgg ctatgacttc tggctcctgg gggttctggg 1860

agccccatcc ttctagtttc tgcttccctg tgtgtccttg ggacacttat taggataaca 1920

agtgctattc ttttaatgtt tatggagtac aaaccttggg gatgtgggtc tgcccccagg 1980

aaggttagcc agacacccaa g 2001

<210> 84
<211> 2001
<212> DNA
<213> Homo sapiens

<400> 84
tgccaggcac tgctccaaat aagtgtttga agagtgtttc ttcatttcat tctcacaaca 60

agcctgctac tatacacatc tttcatatga gaaaacagaa gcacagagac attaagtagc 120

atgcccaggt gacacagttt gtaagtggta aatctgggac ctgaagcaga gaatctggct 180

ccaaagcctg tcctcttact accaagtgac gtttgtgcct ggcacagaga aggtgctcat 240

taaatgtttg ctgattcaaa tcattttacc aagaaattaa acatgcttga gcttattgtt 300

ctggacttaa tgccctgact actcttaaaa agcaaacaaa aatgcttaca agtacaccac 360

aatgaaacaa aacctggaat tatcagaaca tggtaaatgc tttattagat tttctgattg 420

accaagggaa attactctaa gctttcaatg tcttcttttc ctacaaggga actaaatttc 480

caggaagttc tgtgacaaag ccacagagct gagaagtaaa ttagtctgtg ctggacctag 540

gatttccctt gttcttccac ctctgtcccc agcaattgct ttgctgccta ttctcagggg 600

aaggcattgc actagccatg catgctccct ggagaatgac aacttgaact gcaaaatctc 660

aaaggttgtg cttcttttgt tcttcctctc atctagttga taggcagaga acaattctga 720

ttgaatattc tatttaatta tgtttaatca tactaagaat caaccaatgt taattattaa 780

aaagggagaa aatggccaaa acatcatgta caaccatcct gactttaggt atgaagggtc 840

aacattcatt cagctctgag ccaggcagtt aaacagcaaa cagttttaat aaaggacaat 900

taaataagtc aggaaatcca ccagtctgtt ctccaggttc actcaccttg gtgtggagga 960

cgatgggcag aggtagtaaa accaacacag tgaaaaccac gaagagaaat cggcgataaa 1020

ccagaatgta actgaagaat ttcattgtcc tgagcaggtg gcttcaatag tgtcctccaa 1080

ataaagcaga tgttaaataa ccaacttgga ttaatatttt tccagcctgt caccttcact 1140

cactgcccta gaccgaccaa cacaagttaa aattaaatct cattttttat tgttttacta 1200

tcacaattaa catgaagaga taggcccaag acttttcatg agaaatcaaa gaccatttgc 1260

tcctctgggt tttcaaggaa aaactgttct ttccctgctt tctttgatca cattcactga 1320

aatacagact ggacaagcct aggagaaggc aaaaccatga agctggttgt ttgttcacaa 1380

tgaggcactt ccaacccttt tgatgtcctc tgacttccag actccctaac tggagtagtt 1440

accacattgt catggtgcca tgagtgagtc tccacaggca gcctccatgg ccctttctaa 1500

ggaagtatgt tttcagtgtg taaattgaaa tagcaactaa tagttggaat ttcagtgtat 1560

gacagtgaaa gggaaacttt tttgtttaga gatggatttt taacggactt cttggacaat 1620

gtattgcaag ggtcttctac aaaatttata gcaaatgtcc ctgttggtta gaagcaaagt 1680

tttgtgtatt tcttggaccc tattgtctct tttgatagaa aaccaaaata accatgaagt 1740

gttatataaa ccctctattt taagaccttt gaaggcttga tttgatactg ttgtgctgat 1800

acagactaca taattatcag ggaatctgca taaagatgag tgattaggga cattcaagtg 1860

gtgtgtgatg tgtgtgtgtg tatggtttgt gcacatgagc acatgggaag ttggagactg 1920

ttccaacagt gatatggagg atgtattttg aaaatcttca gggataagga ccctggtttt 1980

tttcacgttg tgttgtaaat t 2001

<210> 85
<211> 2001
<212> DNA
<213> Homo sapiens

<400> 85
ggagaattgc ttgaaccggg aggcagaggt ttcagtgagc caagatcatg ccactgcatt 60

ccagcctggg tgacagagca agactccatc tcaaaaaaaa ttaaaagaca aaatgtggac 120

acacacatgg ccccagcaag caagattgat tactaacttc gtggtggcgt gcacatgcac 180

acgatgttcc acaagctcat catctattcg agtcgagagt attcagttat cagcctcaga 240

aatccagaag gaacctcaca tcccaggagc ctctggcatt tcagggcctt tcactccaag 300

gagccgcctc caccgggcct tccccagcag gatgtatatg gccctgtctg tgtgctagga 360

ggagaagggc aggcccgtgt gctgtctggt gctaggtgtg agaccacagc ctttctggaa 420

aggctttgta ctgtgctgct ggtgtgcaga ccccgtgcgt ggtaggggct tcaagaatac 480

gcatcaaatg aacccgaacc caaggacgct tgctcacctg tgtcatctgg gggtctcctt 540

ccacaggaag cacatctggg cagagtgaag gctcccaggg ctcaggtcca gaaagagctg 600

gaggctcctg agagcaaggg gccctactcg gccatgagcc cagaacaatg cgagcttcac 660

acaccgcagg tgtgtgcgtg tgggagcctc ttcctgaagc cttggggtca ggggccgggg 720

cccagcaggc tgggtttatt gtccattgtc tcaggccggg acaggaagca ctttctgttc 780

ccaattgctc gggtggatta atttacagtg gggagctcag agtcgcccac ggcacctgtt 840

ttcccctctg aggggccagc ttccagcttg gagggtgttt caggaggggt ctcctctgca 900

gagacgtggc ctcctccaga gacccttggc cacagcccct gcccgctccg tgctgggccg 960

aggccaaggc cctggcagcc ctgcctgctt gcccgggtca cgctgcccac ccacggggaa 1020

agctgagccc tgtggagtgc acaccatgct gtcagcagtg agccatggtg aggcagcctc 1080

agcaatgtgt cttctctttg gttgggaatc cgctctctct tccaggttca ggggcaggtg 1140

atgtatgctt gttttcattt ggtgaagatc gtgagtccca ttttacagat aaggacgctg 1200

aggctcagca aggttgacta cacaggtcgc gaatgaagtg gtgggatgca aacccgagtc 1260

tgctccaggt cccagagctc tcaacctcaa atgtctcagg agccaacgaa agcttgtgac 1320

tgagtgtcac cagctggtgt acaaaggggg agtggcagga ggtggaggag aacccagggg 1380

tgcaggcagg ggggaccctg tgccatctcc aagggcagca ctgctctacc cagcctgcca 1440

tgccttgaag taaggtggac cttgtgtggc cacatttttc cttgttagta actcagaaga 1500

tacatttcta gacacggggt aggtcacaaa agcctgtctg caggcggcac attgctttcc 1560

acctctgctc tgtgccacca tgccaacacc aacagtgagc acctgctata tgcaggtgct 1620

gcccgggcac ctccacaacg gggacccatc ctgtgtcttc aggccccccc aggcagcagg 1680

cactgtgctt cctgtggaca cacaggggac tgaggctcaa aagagcaggc tcctgaccct 1740

gggcaccagg ctggtctcgg cttgctcact ccacccctgc atccctgcat cggtttttca 1800

tcaccactaa tgcaggaggc tgaggaagaa ttctggagtc tggtggagga tggggaagct 1860

gaccctattt ctataggtac ctccctcatt tttgtaggat ggctccaagt cagccaccga 1920

gactgccccc tctgcactaa ccattagcca gcactaccag gattggccag cttgctctgt 1980

gccacttgtt ttatttattt g 2001

<210> 86
<211> 2001
<212> DNA
<213> Homo sapiens

<400> 86
gaagtggccc tccccaagat gttcattgga gttgccatgg tgatcactat acacagggct 60

ctgttacctc agagggctga taggcccacc cgtagggaaa agaggccagg agaaagccag 120

gattgaggcc aggaaaaggc aggaggggct tcaattattg ttcactctct catccactct 180

agaatgtcag ttgagccccc actacatgcc aggtgtgcca ggcacagggg gctcagatgc 240

taccaagtgc aaaagatgtt cttgggagct caaagcacta agtagggaga agaactagct 300

ctgtgtagga aaaaggagga aggacttatg aaagagccat ttgagttgga tcttgaagga 360

tgcctaggaa cttaacagtt ttgaagggat gagaatatta aaggcatgga tgtgtataac 420

agcataaatg taggtcgagg tcaatgggga aggagaaaga tgcatctgga gctgtctgat 480

ttggacaccc agggatcagg gacctgggag taaaactaaa ggaagggtcc tctctcctat 540

tcctaaacaa caagggctac agaaggagat caacaaaatt aagtgacttc ttttctgcag 600

agaagaagga ggcagaagtc attggtgcca gcactggccc attcctgtct ggctgttgct 660

cagcctggac aaagagtggg tatagtgaac gcctcctgca agcccagcct ttcaggattt 720

ccccaggaaa cagagagtgc tgtggttaaa cttggagtcc ccagaatctg ggcctgtcac 780

ttattagata agagactggc caagtccttt agctgaactt ccttcacatg tgaaacagat 840

atcacccaac actaaggata agggtgatgg gcgaataaaa caattttttg caaaatgtag 900

cacattgcct tgcacagaga gagacatcac aaaactctaa gctacttccg caagcaaccc 960

atcagcaagt ccttcgaagt acactagatt tggatgcctt cgtccacctc cacaactccc 1020

cctactccct ggtccaagcc accctcatct cttgcctaag tattatctat tgctgcacga 1080

caatcttgcc ataaacttcg aagcttaaca tacaactgtt atctcacagt ttctttgggt 1140

cgggtgtcta ctcatggctt agctgagttc tctgcattgg ggtcttagca ggctacaatc 1200

caagtattgg ccagggctgc agtctcatct gaggttccac tagggaagaa tatgcttcca 1260

agctcatgtg gctgtgggag gattcagttc cttgcagtct gttagactga gggtctcagg 1320

ctctctctgg ctgtcagcca gaggccatcc tcagttcctt gctatgtggg ccccccaaca 1380

ccaacatgac actgacttca tcaaagccag caaagaggga gtccccttgc aagatggggc 1440

attacaatgt tgtgtaccat agtcacagaa gtggcatcca tcacctttgc cgtaggctgt 1500

tggttaaaag caagtcacgg gtcccacccg cagtgaaagg gagagggtca cacggggcat 1560

gaacacgagg gggcagataa tatggggacc accctggagt ccaccatatc ctctgacagt 1620

cttctcactc aacagcccag caatcctgtt aagacctata ttagatcata tgtcacccct 1680

ctaaacaaaa ccttccagct caaccacagt aaaagctgac atcctcccat ttatctgcaa 1740

ggccctaccc aacctgtcca ccatttttct ctgacctctg ctcctctcac tttcccaagt 1800

gctccctggg tccctgccat gccaggctct gctgtttttt gggcacacca agcacagtcc 1860

ctcctcgggg cctttgcacg ggctgttccc tctcctctcc tgcaacttcc ttcccccaca 1920

tgcctacatg gcttgcctct tcacctcctt caagactctg ctcaccttcc cagaccacct 1980

tttcttaaaa agcatcccag c 2001

<210> 87
<211> 2001
<212> DNA
<213> Homo sapiens

<400> 87
gccactgcac cccagcctgg gcgagagtaa gactctgtct cagaaaagaa aaaaaaacaa 60

agaaacttta aaatagttta atgtcaatgc tactttcaat cttctctaat tctaagtcct 120

acacctctgg tgaatttaat acttttaaaa agctgagtta tgaaacctgt tttatttttt 180

atctttttct attaaagtgc taaaaccttt cagtctattc acagtagcag attttcaaag 240

gagatcttat gcttggacaa ccaaatgcgc tgatgctaca ttataacttt tcatactttg 300

cagattttca ttcattataa aaataataca aagcacttaa tcttttgcta tatgtactta 360

ttctttccat ttataaaact aaagaaacat aaatctctta cctatgtttc tatctgtatt 420

aaagctgtta gatctacaaa ttttttaaaa gaaaagaaaa acattactca ttagtataat 480

cataaaaatt aacatgtaag atgatataag tgcaattttt aatggcatac tacagttaag 540

tccttcctcc aggtaggaag aacaaagaaa ttactcatta caaagtctag taaatccaag 600

tctacttaga gaaaacagaa gtacttactt cgctcaaatt accacattta cccaaattta 660

agggagttgt tttttccaaa aatattaacc aagagagaac agtgtccacc aaagtctctt 720

gtcaattatt ttgctgacat tagaagacac agcctcaata tataaatata ttaattcatc 780

attcagtaaa tacatactaa gctcttattt accaggcaca gttctaggta ataggattat 840

agagataaac agaacaaagt tccaacctac ataaatctaa tgaggaagac agaaaacaat 900

gatatataac agatcatgat ggttttcagt gctataaaaa gagacataag agtatagagg 960

taaaaaaaaa aaacagagaa acctcaatca taaggaaata cgtgaagaaa tgtagaatga 1020

attatgcagc ttactgggga aagtgagaga acagcaaatg cagaggccct gaagcaggag 1080

cattcatgga atattcaagg tacaaggatg gatgttcact caatgctcct ttgaaatgat 1140

atagaggtca agagatggaa tcaaagagga ggcaaagaaa ttaacagtca ttattcctgg 1200

gggcatgacc tcataatttt aagtgctgga tgtcttttaa agagtactta tgaaaacact 1260

gtagtgaaag tatgtatcac agtgatcata aatttagagc cacaaaaaat gaaagccaca 1320

actgtctgga tactatgtca attggtacct gtggctatag atggtttcca aggattattc 1380

acctacataa aaggaccgta aatggaagag aagataattt gatctggaaa actgacaaag 1440

aggtcaacct caaaaatgta catatatgtt tcatgatata tggttttaga acagcagtat 1500

ttctaaatta atagatatca tacatgactt caaatatgta tacacaccca aagtaaaaaa 1560

tgagcagaca tttgaccatc tcacctacat ccctataaat atacttattc catttggcta 1620

aagcattttt agaatcaaca catggggaaa aaaaatggta actgcttcct atttggtcac 1680

cttatactag agcttttaag aaatactcag taacacaaga acagaaaacc aaaaaaaagg 1740

aaagaaatca tcaggccaag tgtggcagct catgcctata atcctagtgc tctggaatac 1800

caaggcagga gaactgcttg aggccaggag tttgagacca gcctggccaa cacagcaaga 1860

cccatttcca tgaaaactaa aaataaacca gacatgatgg tgcatgcctg tagtcccagc 1920

tacttgggag gatcgcttgc acacaggggt ttgaggctgc agcaagctat gactgcaccg 1980

ctttactcac tacagcctgg g 2001

<210> 88
<211> 2001
<212> DNA
<213> Homo sapiens

<400> 88
tggggggcat ctgactgctt ccccccaccc tctgccaggc attgctggag aatgttaaga 60

cggcgatgga gatgccatca accccaccct gcagagcatc accagacacc accagaccaa 120

attcactttc cagccccttc atgttgaacc tgaaacttga gctagtgtct tgggagaaaa 180

gggggaaatc tctacgaggt acccatcctt ctgcacctta ggtctgaggt gcttggcccc 240

ctaggaagcc ctacatgaat gggacagaag gtccttaaca acactggaga tgaagcagcc 300

gatgctgttt tggacaaatg aaacagcgtc ccctaaccag ccctttctat ctcattgttc 360

tgacttggac acgccatggc tcaccgctcc caaagtcccc actatgtctc cctagctgag 420

gaaataaaag cagagagggg tgatgaaaca gtgacgatcc tggggaaaca gctgaggagg 480

ggagggaggg ggaagaagcc actaaaaaag tgaaatgtgc ttgggagaat cggcctgcct 540

gcagggtaga tgccctttct ctctgctggc cagctctgcc cctcagtgag aaactttaca 600

tattgctaag atgcctggcc aatgaaacag ttccagagac tttatgtccc ccagtagaaa 660

tatgaataga aatcaccctg tgggcaatgg tcccatttta aaatatgctg tcccattgtc 720

ccctagagcc tactttaact tgtcagacca tgtattccac ttcatatgca agaggcatgc 780

actgagccca taggtggcta ggcaaacacc caatagctcc ctgaaatggc ttcattatgc 840

agcctcgaca gccaccccaa ccctcccact ctcacactga aacacccaga cctagagata 900

gctagacaca cccagacacc cgccaagccc ctcacataca gatatgtgca caatgataca 960

cagcaaacgt acacagagtt cagtacacac aaagagctca cgcccacgtg cacacacccc 1020

tcagttggga cagagttgac caccaccacc tttctcccaa acacatggct tttgaactgc 1080

ctttccttgg atccagttca aggggatgga ggagcagtga gagtcagccg cccttccact 1140

ccaatttccc agcacctccc ttatctctgc ctcacaagtc acccagcccc cctctcttcc 1200

ttccttgtgc ttgaagaatc tctccttgct ggaaagcccc ctgttttctc aatctccctt 1260

tccacttcgg taaaatctct acttgctgga aagccccctg ttttctcaat ctccctttcc 1320

acttcggtaa aatgcccact ttctggtccc cacctttttc ctgagtgtag tcccaaccag 1380

ccaaatccaa cctcaaaaca ggaagaccca aggccagtga cccccatagg cctgaggctt 1440

gtgcaggcag tgggcgtggg gtaaggcttc ctgatgcccc ctgtccctgc ccagaacctg 1500

atggccctca ttagtccttg gctcttatct tggaagcaca ggcgctgaca gccgtcccag 1560

cccttctgtc tgcgggcctg aaccaaacgg tgccatgggg aactgtctgc acagggtgag 1620

tatggggcca ggccccagag tcccttatcc ctatgcccct catttcccgt gctgtttgcc 1680

cctcagtctt tatatctctt ccttttcctc ctcatctttt ctcccttcct gcttttttcc 1740

tcttccttca aagtcttttt ccttctctcc ttcctatgct agcctcctag ctccctcttg 1800

tgtccctccc tttgcctttg agtcagttcc atcctggtct cttggtgcct tttccttctg 1860

accttgcact gctcctccag ccccagctgc cctggcttcc ccaggactgt tcctgctccg 1920

gctcttcagg ctccctgctt tgtccttttc cactgtccgc actgcatctg actcctgcag 1980

agaccttgtt ctcccacccg a 2001

<210> 89
<211> 2001
<212> DNA
<213> Homo sapiens

<400> 89
caggcgccct ttgagccggg accagaatgg atgagaggga gcagctgtgt aagcatcggg 60

gggaagagca tcccagacag agggactcat tgcttgcctc tgcctctaga acaggccctt 120

aggaaaatcc cggcagtcgc ctgtgctcag ggccaggctt tccatggttg ccacaacacc 180

cagggcagct tggaggggaa ctggcctgag ggccaaagtc tgaggagcag gtgggcaggg 240

tctctgacca tgtcctagag ccaaggccct ggtgcatgcc ccctccccac tgccccctcc 300

acccctcact cctgttccac atgtcctcac cccaaccctc acacttagct gcctggctgc 360

ccacagcccg tagctctgca catccaggaa caaccagaca gaagggcagg attcgggcat 420

tcttcagagg ccatctcccc tcgatcttta agggtagggg aagcggaggg gagcaggggt 480

ggaagggaga gaggcatctc cagcaccatc cgctctccca gcattcgctc agccaccctg 540

gcccatgtct gagccatgag gtttgagcat agctgcttct ccacatggct ctcccaggcc 600

tggttatcct gaagaaaaag gaagcacgct acccgctcct tgctaaaagc attccacggc 660

cttcacctcc ctccacaggt ctgcacagct ggtgggagag ggacagtgac aggttcctag 720

tcttggcact taccccctcc ctggatggaa tgggtggagg ggttcccgcc tctttgttct 780

ccatccttgt tcaggtggga gaacaggtgt gctctacctc ccaagtgaca cctgctctgc 840

gccagccacc taaagaccgg ctgtttctcc cctgggccta cctggcttta tgatcctttt 900

gctctctgga ccctgctgac tttccccagg agcaaattct ccttggaaat gttgataaga 960

cacacgttgc cagtgcacaa atcatacaca gcacgaattt cgtctttgca cccaccaagc 1020

cccaagccga aagctcccta gttgcaccaa ggagagagaa aaaacattgc ctgacattgg 1080

tccgcagaaa tgaggacttt cagagaccca gtgaggctgt gacctggatc taggatgctt 1140

tttctgtgtc atttttccat gtatgcctct tttttttttg agacagggtc ttcctttgtt 1200

gcccaggctg gagtgcagtg gcgcaatctc agctcactgc aacctcggct tcctgggctc 1260

aagctattct cctgccttag cttcccaagt agccgggatt ataggcacgt gccgccacac 1320

ccagcttttt tttttttttt tttttttttt tgaggtggag tctcgctttg tcgcccaggc 1380

tggagtgcgg tggcgcaatc tcagctcact gcaagctctg cctcccgggt tcacgccatt 1440

ctcctgtctc agcctcctga gtagctggga ccacaggtgc ctgccaccat gcctggctaa 1500

tttttttttt gtatttttag tagagacggg gtttcaccgt gttagccagg atggcctgga 1560

tctcctgact tcatgatctg cccgcctcgg cctcccaaag tgctgggatt acaggcgtga 1620

gccaccgtac ccagcctttt tttgtgtgtt tttagtagag atgaggtttc accatgttgg 1680

ccaggctggt ctagaactcc tgacctctag tgatccgcct gccttggcct cccaaagtgc 1740

tgggcttaca ggcatgagcc accgcacctg gctttttttt tttttttaac ttttatttta 1800

ggttcagggt atatgtgaag gtttgttaca taggtaaact cgtgccacgg gggtttgttg 1860

tacagattat ttcatcaccc aggtattaag cccacttccc agtgtttgtt tgctgctcct 1920

ctccctcctc ccaccctcca gccggaagta gatcccagtg tttgctgttc ctttctgtgt 1980

gttcctaggt tcttcatata t 2001

<210> 90
<211> 2001
<212> DNA
<213> Homo sapiens

<400> 90
tattttcttg ctgtcaggta gttctcaagt tgccttagaa ataggggtca cctctgagga 60

cagagagaag agaagggcaa agagttaggg cagtgcttta ggtctttagt gtttatttct 120
ttacccagag aagtacagga aaaatataac aaaaatgtta tcattgtaaa atctaaatgg 180
tgaacatttc aatgttcgcc atagtactta cagcattttt agtacattta tattttgtaa 240
tttaaatttt tggggttttt tttgcctccc aagtagctgg gattaaggca cctgccgcca 300
cgcccggcta atttttgtat tttttagtag agacagggtt tcaccacgtt ggccaggctg 360
gtcttgaact cctgacctca gatgatccac ccgccgcggc ctcccaaact gctgggatta 420
caggcgtgag ccaccgtgcc cagtcaattt aaatgttttt aatttcacaa cttacattct 480
agcttcaaaa atatatttta cattattacg aaccactcac caagatttta tggccagata 540
cccaaattca tctcagtctc caagtgcaaa ttttaaaact aatttcttcc atcattcaat 600
tcaagcagaa tggtagagta gtattttaga acagatagaa ctagtaaatc ccagctctgt 660
gaacatactt tctctgttac cttggccaag ttatttgcta aaggcatttg cttcccatcc 720
tgtatctgta aaatgggtat ttcctattgc aaaaggtaga taagtagcat gtgcttggtg 780
ggttagtgtt tttccctctc cagctggaaa aaaaaaacct ggtgtggggg aagaagggga 840
acagtttgtg caacagaacc ctaagaggaa atcagctgct catcaagata agggctgagg 900
cataaaactg ccagagggtc tcaaggcagg caaagaagaa ccatgaggct catcctgcct 960
gtgggtttga ttgctaccac tcttgcaatt gctcctgtcc gctttgacag gtaaatctta 1020
cttcctgtgt tccagtctct gtgtagaaga gaattaaagg ttgtttcctt acagttcact 1080
tttttttttt catttgcctc cacaaaggga gaaggtgttc cgcgtgaagc cccaggatga 1140
aaaacaagca gacatcataa aggacttggc caaaaccaat gaggtaagca tttagaggga 1200
tattttatct tttcttactg ttctctaaaa gatgcttctt cttattttac tgtcaatgcc 1260
catgggacta caaaagacta ttgaacataa ggggaaagca ggagctatct ggataattca 1320
gccaatggcc aatacttcac ctgttctggt tttggtcttg gtttccaaga ctaccacttc 1380
tgtttttcct cccaaaagct tttcaaacac cagctttttc aaggtaaaac tatgaaatca 1440
ggagctgttc ttggcctcct tatcagggca tctttctctt gcctacgggt gccattccct 1500
ctccattctc cccactccga ccctcatctc ctctggtgcc ataatttctc ttgagtacta 1560
actgaaaacg ttgaggcact ggctaatatc aagatgtgac cctttccaag agcaggtaca 1620
ttttcaaata agacttttaa agttagatca atcctttaaa atccaacagc ctaatataat 1680
tttatcatgt attcagctgc tcctatttat gttgcaccag ccaccttata tatgccattt 1740
caaattctcc agcagattct caatgtcagg gttataatgt aactcatttg aaacagaaga 1800
gctaaagtgc aaagacaata aattaaatga ccaagattac acatcaaatt taaacccaga 1860
actgggtctg ggatttattc cagtcaaaca tacagttacc tctacatcaa atcaatcaaa 1920
ggccctgcct attctaactc ccaaatgttt ctcaaatcca cacattgccc tccattccac 1980
tgccactgcc aagttcatgt t 2001

<210> 91

<211>  2001
<212>  DNA
<213>  Homo sapiens

<400>  91
cagctcaact cgccgagatg acctgggcac ctctgcgttg aatcggcaaa tactgatcaa      60

gccgcattta ttctgctctc aggaactcta agtctagcag agaagatgag gcggtagaag     120

ttcatcagtc agtgcccgaa gagaggtcct aatgggaatt cgaagaaagg agttaatcaa     180

ggaagtgtat ttttttgttg ttgtttgaga tggcgcccac ctgtaatccc agctacttgg     240

gaggctgagc taagaggatc acttgaactt aggaggcaga ggttgtagcg agccgagatc     300

ataccactgt actccagcct gggcgaaaga gtgacacttt gtctcaaaca acaacaacaa     360

caagaaaatt agccaggcat ggtggcctgc acctgttgtc ccggctactt gggagactga     420

ggtgggagga ttgcttgggc gcgggaggtt gaggctgcag tgatctgtga tcgtgccact     480

gcattccagc ctgggcgaca gagcgacaac atgtctcaaa agaaaaaaag aaaaacagaa     540

agaaatccta ccttacatat acagccaaac agttataacc actccatatt taaccattac     600

aaacgtaaat ttttatttta ttttattttt tgagacagag tctcgctctg ttgcccaggc     660

tggggtgcag tggcacaatc tcggctcacc gcaaccttcg cctctcgggt tccagtgatt     720

ctcctgcctc agcctcccga gtagctggga ttgcaagtat ccaccatcac gcctgggtaa     780

tttttgtatt tctaggagag atagggtttc gccatgttgg ccaggctggt ctcgaactcc     840

tgacctcagg tgattcgtcc atccggcctc ctaaagtgct gagattacag gcatgagcta     900

tcgtgctcag cctacaaaca taaattctta agattggctg catcttatct tcccataaga     960

tttgactgta ctgcttattg caaattattt cataaagtgc gtcctaaatt cacctgataa    1020

ttgaagtagc agtttgtttt agctaattgc ctttatctga aggaataata aaatttctct    1080

tatttctatg acaagcaata tcatttcatc tcagaaaaag atgcctaggt tgaattccca    1140

caatgacaag gagatagggg ttttattttc ctagatgttt atatttgaga ggaattactc    1200

gcagaccctt gaaaaaacat gtcagggtta taaagctgac aagggactta ctcatcttct    1260

aattgggttt ttttcagatg gcttggctgg aggacaagca aattgaggac attggcaacg    1320

gagtgatcaa aatgatagat catgaggcct aaaatgaata aggaaagaag agaagtggca    1380

gaggctgaga acagaaagag agggtggagg ggctgtaaat cttgaagatt agggtataat    1440

atgagtatat gggtaagaat tggaagaatt gtgtaggagg cagtagtcaa aaagtagaag    1500

cagtttggaa gagtagttac aaatatcaag agccaggtgg ctaaaaggtg gagctatagg    1560

tcattgaagc tcaagaaact gagtctctag ggcattggtt aagtcatctg tctagacttc    1620

aaagttgtct aggatgataa ttcagaagac tgatctgtgc caaagtcaca ggtaagggaa    1680

gtgaacaacg aggaggggaa aagggagggt ataggctgat ggaaggagcc tcagagaggc    1740

aaagagttta tatcctgttg tgggagtggc atcaggtata aaggaacaca aactccttat    1800

tttttattga tttgcttctt tgtttgctat ctgtaatgtg attggttact tcctgcagaa    1860

gatttttttt ttccaaatga attatttcct ttttacattt gcaggttttt cacgactgaa    1920

aacaacatag caaaataagc caagatgtct gtggatccaa tgacctacga ggcccagttc 1980

tttggcttca cgccacaaac g 2001

<210> 92
<211> 2001
<212> DNA
<213> Homo sapiens

<400> 92
gcaacttggc cgaattttga taccccatgt gggggtatga attcttcttc tttttttaat 60

tactcaactc cattctctac ttttctaagc ctgaatagat gaggccactc agcctcagaa 120

actatgaaaa cttcataagg aacaaaagga tctcaaagaa taaaacctct taagcatata 180

ccagccaaca cctggctaaa gtcccagtgt ttccagcctg tttcagagac atttctaagt 240

gaccagatgc aacatcttag tttgtcgaga tgccctcgag tttggctgga tgctcctctg 300

tcctccagaa gatatcaaca ctgtcggaat ctatcccacc atgcagctcg tcctctggct 360

tggccttgct ctgcagcaga gcaacccata aagcatacca caatgcctga ggtaggcact 420

tgaaagatct gaggtcaagg tcaagtcccc agaaccgggc cagttctttt taagggaaag 480

agcactctag cgcattttgg ctgtaatatt cctagaccca gagattcagc tatcataggt 540

accttgtcaa tgaaggaggc aaacttgttg ttgagggtct tgatttgctc cttctcatcc 600

ttcttcaccc tctgggcatt ggggtcaatc tccaggttga ggggggtcag taggctcttg 660

ttcacagtca cagctgtgat agatggggct gctgggactc caacccctcc aactctgtaa 720

ccaaagccag ggccaccaaa gccatagcca atgccactgc cagctccaaa gcccagacca 780

acacagctgt cagccctagg ccccagacca acacctctcc catcaccaaa acccatccca 840

cagccagcac caaagcggac tccacagtgg atgggccgag agcctacagc tgctatccgg 900

ggtgagtacg atccaaaggt gatgacactc cgactaccaa agctgccaag gccccggaat 960

ccaggcccac tccaacagga gacagagttg gcccggaagc gattcaggtt ctgtggtgtc 1020

attgctgaac aagagctgaa gttgcccacc cggtgaccag agctgactcg gtaggagcgg 1080

caagacatga tggcttcctg gttgggagca aaagagcaag tgtagaatgg gtgagctgga 1140

gctgggagtc cctctgaggc cagtggaacc cttgcacccc ttttatgtgt gtggtgatct 1200

gaggcttggc cccataaaaa tgaaaaaatc catttgcagg catttatgag cttgggtagt 1260

tagcagaaac tctgcatgcc tataacctcc ttgacaatga gttaactcag acacacctag 1320

tctcattggg atgagggctg taacagcaaa ccaataaaca ttaaaaacac ataaacatat 1380

aaacattata gtcttttttt tccctataag tctaccacaa ttgccattaa ggggacatta 1440

atttgctttg gtcataaatg caataggttt tttcactgtg agtgtggcta accaccagaa 1500

agttaggatg gcgacagctc cagcaagagg ctcacatggg atgcatccat gaagatataa 1560

ccccctaaag aggtgggagt gtgtgcacat ctccaagtaa gaagtgatat ggagaaaata 1620

cagtttgttg catttctgca aatcaattaa atcatcaaaa aaccttctgc catgttttat 1680

ccaatatggc tgtttagtat acaagagttg aatctacagt aaagctcatt tgggctagtg 1740

tagacacatc tgtgatttga gacactcaga gatattcatt cattctacag ttttagagtg 1800

cctgctatgt atccagcagt gtgcttgctg ctgagggtac agagattaca aagacagtct 1860

ctatcttcat ggtagtaggg gaagatttat cttaaatttt tttttttttt ttagagacat 1920

gatctagttg cccaggctgg tcttgaactc ctgggctcaa gtgatcctcc cgcctccgcc 1980

tcccagatag ctgggactac a 2001

<210> 93
<211> 2001
<212> DNA
<213> Homo sapiens

<400> 93
tctctggcct ctggcttcca ctggcttttc ctttggaaaa agccagttca aatggtgctc 60

actctaggaa tcgcttcaac tcggagccct ggaggtgagc agggccgggc acgggccggg 120

cacatacacc ccctctaggg agcagggaag ggcacaggcc aggggaagct ggagacaaaa 180

gaggagacag ggacagagat aaaaacaggg actgtgacaa gaacacagga tgggaagggc 240

agagacagaa atagaggcag gcttaaggat ctagacaggt catgccagcc caggttcaag 300

gcagaggggc gtagggggac agagagagag acacaggaag cacagagggg agaaagctct 360

gcttagcaga cttgccaaag ctgaagaccc cactgttgcc acaaaggggc ccacaggaaa 420

gctaggggtt gcaatcgcct gtcaggaccc acattttcac cctgagctgt gcacactttc 480

caacggcaag caaaaggagc acaatctagt taagttagga ctggcaagat tcacttctga 540

tttaaaactt aagacagcaa caataaagat actcagctga aatgccaccc aacaaagcca 600

gctcccctgg aaagttaaag tttagcaccc ccatcccagc accctgtacc tttaaggaag 660

agaaggttcc ctccgcttcc cagccgaaga ggcaggttat tctaggatat aggggaggct 720

tacctaaaac tgagttattc tcctttcctc ctctggccag cctctcttca ctctggggag 780

cttttaccat ttaaggtcaa tgttacatct ggctccggag ctcagagccg agaagcctct 840

ggctgcagaa cctcggccgc acgcctcctc tgagaggggc tgggacttag agccgagagc 900

tcctctggga gccccactcc ggcctggctc gggggataat ggcagggaaa gccgaccagc 960

tggcctcagg gctcagggca ggctgggcct ttggggcagt cgctcaccct gggcccctcc 1020

gatgggtatc cacacagcag atggcagtcc ccatggcaac tcgtgccact ggacactgtc 1080

ctggccggac gggaacaaag ggaccctgca gatctcccca cccgcctgga gcctccagca 1140

agcatgtgag cctggagaag accctgggcc agcgccaaga gaaactggga atggttcctg 1200

tcctggggat tcgggggcag gggctgctcc cactgcgtta aactcttggg attgccagag 1260

agaatggcct gggagattca gctactccca gacttggcac cagccccagg gatgtcaacc 1320

ttgctcctcc attaaacagc ctgtggtctt aacatcgtgg agcctcagtc tcctcatctc 1380

taaagtggaa ataataatga taatgcccga ctcactaggt agtcagggga ttaaatgaga 1440

tgttagtcaa gtgtttaaca cactgccagc atctcataag agctagatca gtatgaatga 1500

gcttcctgcc cctgcccagg aaatccatac cctgtccata cacactcttc acctgccttg 1560

ggtatagggc ctacccaagg ccaggatcat ggagctccaa gctgggggca agccacatgg 1620

ggacttcagc cccttctgtg gttagagcag cacagagaaa ccctaggtaa agttgctaga 1680

taaattacag gacacccatt taaatttgga tttcagataa acacaggaca tacttgtatt 1740

aaagactgtt atttatctga aattcaaatt taattagtca tcctctattt ttgtttgata 1800

aatccgggaa ccctagcttt tggccatcat tagccaccca gaaaacattc agtagtcatg 1860

gtagagagca ctagggattg gagctcccag gcctatggcc tcaagctctc ctggcaaaca 1920

cagtccttat gctcagctcc tggccacaga tcagttgtgc agcagtggca gatgctctcc 1980

actgagcctg gcccactgag c 2001

<210> 94
<211> 2001
<212> DNA
<213> Homo sapiens

<400> 94
actacaaaga agaaaataaa taccaacgga aatcacactg tatacttcta ccatgtgggg 60

gggcacttat tcagatatct ctctttcatt ctctgtccac ttaaagaaat atgcatacag 120

tttttaaagc atggagtaat actctacctg ctgtttctgg agtagtattg ttgtagacag 180

agtgacctct ttcaatggca gatgacttga ttcttggtgg taaagcaagt ggttggttat 240

aagtatgggg tctcattgtg cctaggttaa catttctttt ccactcatat gtgccattga 300

gtaagtgatt taacccatct ctctgtgcct cagtttcctt atacacaaaa gtggggacga 360

taatattacc tacctcagag agttggtgtg aaggttatat cagatcatat atttaagtac 420

ttacttaaga cagtacctgg aacatagtaa gtacttacta catttcaact attgttctta 480

actaaataag ttttaacttc atagtaacaa taaggggttt ttaaatagaa aagtggaatc 540

tttttaaaaa tacttttcac catatttcaa agccattctt ttaacataaa ttattactct 600

ttttgcacac agctatatac gttatccact gaaattagta tttgcaggta actacccttt 660

tgcggaggcc tacttaaaaa ctatcctgtt gaacacaagg tacctttaag tgagacagtt 720

ttacatgggc ttcctttata acacattagt ccaactaaaa atccttgtta attattaaac 780

ccttaggtat tacaggaata cctgacactg cagattgaaa acagacagtg tttgtctctc 840

aagttaaacc aacaagccga tagaaaaagg tagttatcaa gagattttta aaacttcaac 900

cctttttctc ttatagttag tgaagagagt agaatatctc cagttttggc tgacatctct 960

acaacctgaa caattggctt aaacttcact tgggattccc ggttgcttgt tttagcatgg 1020

caaaatttgg cgttcacaga atccttcttc tggctatttc tctgacaaag tgtctggaga 1080

gtacaaaact gctggcagac cttaaaaaat gtggtgactt ggaatgtgaa ggtaagtttg 1140

cttcccccgc ttcttctcct cctaaattga ggctctgcaa tgacataaaa tcttattgtg 1200

gtcacgcatg ataaaaatga gtattgatac cattgagaaa tatttggatt atgaagtaca 1260

gcttggtata aaaaagaaat gttggaagca aagcattttg tggtagtatt taatacaaat 1320

atacaaatag caaaaatcag tctgctatag aaatatatca gaagttaaaa aactagtcag 1380

tccttttact atttttaaag attaatctag gagtctctga gcctattctg gctcagaagg 1440

ctgcccatta aaaaaaaaag ataaatctaa ttgattcata taaaaaaaga catcaggcta 1500

atttgatagg cctttattta tgaaggcctt ttacttttta gtttctgttt cacactcaga 1560

actttctgat tctcatgacg ttcctaaaaa gtgtttttat ttcaaaccat catgatttat 1620

tcttctaaac tttactaaat gttaatcatt tctgtgttat ttattatgta aaaatactga 1680

aatcaggact agaatataaa ttttttattt atatcattga atataaagct ttctttactg 1740

taaatggaaa ttaatttcca ttctattgtt aaagaactta tttctgttat tagttttatg 1800

gtgcaaaata aattacatcc caatatatta gagttgcttt aaataagtta tttttccttt 1860

tgtttttatt ttataataaa tacatatttt ttctctctca gcagaatcaa aaattgcttt 1920

aaataaattc tacctttttg gttttttttt tttctgagat ggagtcttgc tctgtcaccc 1980

aggctggagt gcaatggcac g 2001

<210> 95
<211> 2001
<212> DNA
<213> Homo sapiens

<400> 95
agggctgtgg atttaggagg gggcaaccga taaactgtag aactgaagag tttggaatat 60

ttgcataggg gagcacttgg gctttggatt tggggtctga gagtgagaat gcaccccaaa 120

gttgtggatt tgggcattcc aagggagagg aatccaaagg tctgggtttt attagggtga 180

gatggaagtg tggctgtgca tctggaggtc ttctgttgcg gaggatgcag ggttaagggt 240

ctcaggaggg gggactccag gcatatggac ttgaaagtct cggggctagg aagacccagg 300

gctgtgggtg tggagtttct gggaggagga gtgtgtagca agccagcagg tgctcccaga 360

aagcccagca gcccagcaat gggcccaggg gctgacttga ttttgctttt gcccagagct 420

atctcttgat gcccagaatc ctcattggaa cctcagagtg aggccaggat gggtgccatg 480

gaacctccac tgcccatctc tggttgggaa aggggtggaa gaccagggcc atatttcagg 540

ggaactttcc ccacacctgg agaagagtac ctggagttag cggttggcag agggattggc 600

taggtctgga gaaatagtaa taacaacccc caaatggtgg aaaaatctca agtgtcccaa 660

gggttgtccg ctggtttcca acccagatcc tcagggaaac ccactagatt gattgtatca 720

tccctattaa tattatgatt gttgtttatg tttctcattt tatataaaga tctacagagg 780

acagaaaaca gacagtgggc aggggacatg tgtgagtggc cctgaactcc catctctcct 840

gctctgtgta cttagttccc ctttctggat ttggttttcc tgccagtgga aagggagtgt 900

ttgggaagag aagatcacat ttgagggtga gttaagtctc ctttaagggc gattcctgct 960

ttgtcagggg ctactactat tagcaggcag agggaacagt cgcagaagag attggggtaa 1020

actccagtgt taggcacaga ggcagatcct ggggtgggtg tgagtggaga agggatgagt 1080

agccctcctg tccttccttc agggaaattc ttccttagta aacaggacag ttaccctttg 1140

gtgagcatgg tcatatatgg gaagattaag tctaggtgtg tatgatctgg ttgaatcttt 1200

gtaacagtga ggttggttca tcgtgatcat cctcattttg aagatgagca gatctagact 1260

ccataaaccc tcttgagtga ggtcacacag ggagtaagtg agggagctgg gatgtgaact 1320

ttctcctcgc cccccttcat tgactggcct agatcaccct gggagtcaag tgcgggccag 1380

ccttagcagg aggtgagtgc tgagactggc atcagcagtg gacacactga tgacattggc 1440

gggttctgat atcagcatca ggtatggcca gggagggaaa ggcatcgtgt tggctttggg 1500

actgccccca ggtgtatgcg aggatgggaa acaagttggc actgaacttg acaccaggag 1560

aatattgaag tcatgtttgg tgccaagagg gctctgtgtg ttgtctcagg tgtcattgag 1620

ggcataagag tgtcctgctt caggagggca taagggtggc accatatagg gtctgaggtt 1680

ggtcccagtg ggctctgaca tgagcatgag gcaggtgctg atattggtcc tatgtgggtc 1740

tgagatgagc ctcagccaga ccctgagagg ggccccagat ggccacagga caggctctgg 1800

tggttggacc atttagggtt gcacctgtcc tctgggtcac caactttgga caagtggagg 1860

tgggtgaggt tggcaccccc aggactgaca tcttgctcaa actgttctag gatgctgagc 1920

tctgagacct gtgtggaggg aacaaaggtc agagaacaag gcagagagag gaaagttgtt 1980

tgttgatttt ggaggggaag a 2001

<210> 96
<211> 2001
<212> DNA
<213> Homo sapiens

<400> 96
ctttccttgg tagctctcac ttagggactc tgaagagtcc catctcagac cccccaggat 60

gtctcctgta ccctcatgcc ccgccagggc caagcctgct ttgcacacct ctgtagtcag 120

tgcttctccg agggtacaca ataggtgctc acactgctga aagacgaaag catgacaggg 180

cattctacag cagccctggt cttgggccaa aaccacagtg gcatcctcag ctccttgctt 240

cctcccactc cgcatccaat tcaccagcag agctgatggg ctctacctcc agaacccacc 300

agaatcttac tggccacacc ccctcctagc cccgttctgc tccaggcctc cggtaccttg 360

cacctgatgg ctgcagcggc cacctctttg ggcttcttcc ttccaccctg cctcctacag 420

tctatgctgt tgaaatcgaa tcacgggtgt ctgtctctgc tcacaacctt gcagtggctc 480

ccatgacact cagaacaaaa gctgaagtcc ttccagtggc ccccccccca gctgcatggc 540

ctgccctgct gtcacctctc agacctcccc gcacccttgc tccctctcct ctgctccagc 600

tacacaggcc ttcgccccat ggttctccag gcacacaccg ctgcggatgc attccctcct 660

cacggtcaaa atacatgcag ctccctttgc agaacgttcc ctcaaatacc tgcctggccc 720

actctatcat ttcccttgtt ctctgggttc aaatgtcacc tcctctgaga agtactctct 780

aatgatctga cctcaagcag ccacccaggg ccgccacttg ccctctttct tcttcatttt 840

gcttcaccat ctgacatagt agattatctc ttactgcctc tttcccctac tagtccagag 900

ggagacatac tatactgtcg tatccccagc acccaggaag gcttggtccg ttcagccctt 960

ggtcagggct gaatggattt ctgttggatg aatgaaggga cggcagttaa agagccaggt 1020

tgccagctga cattgcccaa tcttgctgtg tggcctcaga cccacccctg cccctctttg 1080

tgcagctcag agacagccct ctgtgctcag ggaaggcctc ttggatggac actccacctg 1140

ctgattgtga cagaccccaa agccgccctc tgccccctgc aaacctccca tgccccgtac 1200

cttcttcagc cactgcgtgt tgttctgcag tgcctgctcc agctgtttca cctgctgggt 1260

gggcaacttc cccaggtgca gtgggttggc cagtgattct ctctggaggg tgttggagtc 1320

cctggagacc tcaggccccg gagggcaggg ctcagacttg ggcagcaaga aggtgtagct 1380

acagtggccg tgctggacta caagtgtctc gcagccccta tccgcctcct gccttgtctg 1440

ttgagccaca gacatggtgg caaccacaag gaggaggctg ccctgcagca tggctagctg 1500

ggagagcatc tgaagatgtg tcaatggcga gggatgtctg ctcagagccc taggggctgt 1560

gcctgggatg tcctgctgca gccaacagtg gccaggcttg cctgcagctg cagctacaaa 1620

cctctgtctg gccgagctct gtccaggcag ctatttatac ttgctctgaa taccacggct 1680

gctctgcttg cttcctctca ccgcctccct ccggccccac ccgacaccct ctcacctcta 1740

ttcttcactc cttcccttcc tccttcctca gtcttggtgg cacgtcgccc ttaccctaag 1800

cgtgcaccaa ccttgctctg tctgcctcac ctctgagccc tctgattatc cagtgaggcc 1860

tggcttccct gctggagggg agggaagaaa acagcacaca gacacgcgca cacactcatg 1920

cacacactca cgagcacaca ctcacacgca cgctgacacg cacacacatt catgtacaca 1980

caaacgccaa gctccaatgc t 2001

<210> 97
<211> 2001
<212> DNA
<213> Homo sapiens

<400> 97
ttaatatgtt catgctattt tcatttgtga ggtctgtaat ccatcagaac aacctgacag 60

tgattaagta aaatcaaaga gactttgtaa acatctgtgt gcttttattg ttacaaatgc 120

ttcattataa aaagatcagt caaaagcata aaagtaccct ggtttaaact ttttctgtgt 180

ttgaatgaga atgtttctct tggaacatgc tcatatcagg cttccacttg gcaaaatcac 240

gaattggctg atgttgtttt agaggctttc tcatgtcccc ttccaaacat cactcttctc 300

cttctgtgga cccctttctg ttatctagga gcccctcatg ctgccttcca cattctgcct 360

actgcacagt catgttctgg acccctcaga atccccctct accattacaa tatgtttttc 420

ctaagactta cttgcattga aaggcttgcc agggaagtga ctaatcttat catcttgtac 480

taaatgttaa attaatcctc attctctcta gaattgtttt ctaagcaggg attcctagag 540

atgctatcaa tatctcagcc tgaaattgca taggtattct ggtgaatagc aagagaaaat 600

aacgtgagta taactggacc ttttagaaaa gtgaagtcta ttaggtcacc ttagttggcc 660

tagggagttg atgtaggaat gctcctctcc ctgaaataat cttccaggag aggaatgtat 720

ccccaaatag caagtgaata tccaatggct tctcattgca cttaaactct atattccttt 780

ttatgaccaa aaaggcctgg cttctcccta actgtctctt ccttacacat tacacctctt 840

ccttttgtat gtgaatgtgc caagctacca cctcatttct accttaaggc ctgaagtgtt 900

atgttccagt tcttggcatg caaattactc ttcatcactt gtttcccgac acaaatgcca 960

cctttccaga caatctaaag taacctcaaa atcatttgct cgcacatcac tttgttttat 1020

ttctcctggt attaatcact atgtgaaatt ttctggttca tattatttgt cacctgcttc 1080

ttccaggcct cctcttactt cattgtctat catgttcatt accgtatccc tagtgttttg 1140

cacagaatct ggtacatatt cagtgctcaa taaatatgtg ctaaatgaat aaatgaataa 1200

attaacctaa acataagttt tacacttaag taattgagca agaagtcttt gttgtactaa 1260

tattggaaaa ggcaggggtg ggggtggtga cgtgattata accaatgtaa ccagcttgca 1320

tttataagac ttgtattgat tatgaactat agcatttttt cgttaaaaac attatcaata 1380

ttggattgta aaagattcca ttggcctttc tgaaagactg tccttttgat gagatgtcac 1440

aaatgaatgc taatgtccct tgagaaatcc tgccaacaga tcttattctt ctatcaatgt 1500

taagaaactg catttctctt tggccaattt ctaggcttat ggatagtctc tcttgctagg 1560

acttcagaag ccaatgagca acttgcctgt cacaagatcc gacgaatgat ctctccctgc 1620

atgcggtgta gctaagcaga gaagcagctc ttgtaagaga ataaccaaca cagcatggaa 1680

tattgctaaa tctacagtgt tttatccagc aaagttcacc ccagtagcct gcccctgcca 1740

ataaagccta gaaatttttc ctaccaggca gtcccagttt agttggttgt agcttagaag 1800

cagacatctg agagcagttc agcaagcctg cagtcagatg cttttgtaga gctctcctct 1860

taaaaggcaa aattgaggca agtcaataga ttacaacttt ctgggtttgc ttctgtcatc 1920

tatcaaatat cacaattcat cctgcttcgc taaagtgaaa ctccaaggat ttgtttttaa 1980

aggtgtcctt ctttctgtga a 2001

<210> 98
<211> 2001
<212> DNA
<213> Homo sapiens

<400> 98
ttttaaatta tctctttttt attacatttt ttggaagtat ttctcaattt ctctctattc 60

acattttctg ttcagccctt ttccttattc cgtatcttca ttttcttggt agtcctcatt 120

ttgaagtcat tcttcagtta aataatgtca taaaaagatg gagattaaac aatatgtttt 180

tcatgagagt tgattaggat caaattattt gaaatttcta acttagatgt tttcaaaatc 240

aaaaactaac taaactacct tgtattttct taagtatgga ctacaactgg tgaacaagac 300

tcttcaataa gaccccctgt ccccacccca ctcccatttt tcactgagca gcacatgcca 360

gatgccaggg caggagggta cgtggtcacc aacctgctcc ttccaggaat tcaagatgtg 420

ccccagtctg taggcatagt agtccttgct ctgttggttg aatgtgtgga cagcaaactc 480

cactgtggca gggaggtaac gagccatgac attgtgttca tcacagtccc tttgctcgtg 540

gaaatgccag gcatagatca gcaggatctg ggagcctaag agaagcagca gcagcgccca 600

ggacagacct cccttccacg gcaggcccag catggtgctg actgtaggca ccgctgactt 660

tgctcttccc agcccccgtg cccacaggag ttaattgagc acagacccag cccttaatct 720

tatctgctca gacagccctg tgggacaaac ttgtctcacc ccacactggc aacaggcact 780

cctacaggac aggaaggaca ggatcctgga ttaaagggag gcagctctgg atgtggagtc 840

actcatctgc catccttctg ccctatactc tctaggccag cacaagccac aggcaggagg 900

ggagctaccc tctgccacta gctttgaagg ctgctccttc agaacccaca ggttctggtt 960

gtcctcaggt gcttggtgtc cttggatgat tctgtgcctt cgcctcccac agcgaactga 1020

gataagacct ggggacatct cgactctcac tctcccctcc ttcaccccgt cgaagagagg 1080

atctccgttg ttcagaaaaa taactaagat atcagtttgt tcttttgttg tataatacct 1140

caacagttgt actctgtgaa ccaagggaaa aaatgaaaaa aataaatggc aatttttatc 1200

aaccattttg acctgggatg gacaatgagt attgtgagaa aagggcagga aagacagaga 1260

cccaagtcct gaggccccag cctggtcatg cccagcccag ccctgcttgc atttctaaac 1320

cttgtcagcc ctggttccag ctgagccaga gccaaggttt ctacagggcc caccgcccta 1380

catctgcagc cccaccacac acactcatgt tttattagac tggaatatca ttctagacca 1440

aatttttaaa cacttctctg ggagatcaca tgtttctgcc tcagtgagat agaggttgga 1500

ccattcttca gccccttgat catttttgtc tccatctgac catccttctt ggtgttctag 1560

agtgagctat tttgccagca cttaggtgat actaggtggt tacttttaaa tcaatttcct 1620

ggcaataact aacctgaaag aggaaataat aatattttgt tgctgtaaga atgaacatta 1680

aatagtgcag gacaggtgaa gtatgcaaaa gtgagagctt tgagtttggc tgagaagact 1740

atggtgctgc aatataggca agaacctagc attgttgcca gataacagaa tagaaagttc 1800

agtcccaagt cagggagact gattctacaa gaactgttct gactgtgtcc cataccatgt 1860

tcccagatca cagcagggcc aggcctggga agagcatcac tgcatctgtc cacactagca 1920

cagcactggc caggtcaaga cagctgcagg tgaagagagt catgctgcct gacccaacac 1980

tggggtggtc ccgagtgtct c 2001

<210> 99
<211> 2001
<212> DNA
<213> Homo sapiens

<400> 99
agtgataccc tcctgccatc ttaaacggct ctctgccatc tgcttcctcc tcctaagtgg 60

ttttccttct gtcctccctg tttaatgttt aagttgtgca catcctgtga catcctggag 120

acccatgttc ctgacctact tacctattca accatctctt ctcacacatg gggtagcaat 180

gagaagcaca gggggtctct gcaagggagt agggaggtgg agagagcggg ggcagccaca 240

catcacactg ctgttctcag tggcctcctg ctgtcaacaa gctaacatta ataaaacaag 300

cccactcttc tgctaagaga ctgtgacagg gtaacattta ttctaggtag cttttcaaga 360

aaacagctct tgctcccacg gtcccctcgg agggcctgca gagctccagc gatgtcagag 420

ggcatctgtt tgtttctcct ttgcttataa aagggacctg gaaatcaaag gcaggctctt 480

agcccttagc agtgaggatg acagctctgc accagccaca cgcaggcccc tggctctagg 540

aaccacctct cacccagacg cccaggcaca gcatggtggg gagagagtac aggacacagg 600

gcctaggcct gctgccccgc ccacagcccg ccccctagcc ctttctctga ggccaggtgt 660

actcacctag cctccagggt cccctgctca ggtggcccca gaggcccaaa cccactgctc 720

ccactttctg agacctggga ccctcacctc cccaactccc accatagggc gacactgaaa 780

agtgtcccct tccccccccc gtttccaagt caccagcttg aggatgccca aggctcaaag 840

gctcctccga gtggcacccc gcatcgggct gcagaggtcg gagaggggag gggatggtgg 900

gtgcaaaggg tggcttcttc aggaaaggga agcctgtccc tggctcccag cacctgtcac 960

ccctggcgat ctcaccccgc ggccatcatt cccgtcctca cggcccattt cagggacact 1020

gaggctcaaa gagccacgtg atttattcaa agcagctcga gccaccactt ggagccacaa 1080

gaaagccctg gtccgtgcct cctcgccttg gtccctggcc cttcactctt cgtccaggct 1140

ctctgacctc tttccctctg cgcccctgcc tcttccccag gcctgggtcc ccaaggcact 1200

cggccacgcg ctgggtccgg ggaagaggcg cggccgcggc cgaggcgggg acagggaggc 1260

ggcagcccct cctgcgcagc gacggcgcgg gcgggcggag gccggggcgc cccagccccc 1320

cgcccccgct ccctccggcc gtgacgtcag agccggacgg ggtaaactga gcggcggcgg 1380

cggggcgctg gggcggagac tgcgacccgg agccgcccgg actgacggag cccactgcgg 1440

tgcgggcgtt ggcgcgggca cggaggaccc gggcaggcat cgcaagcgac cccgaggtaa 1500

cacggccccg gttccctgct cgcagggcgc ggtgccggag gggagcgggc ccgggggagg 1560

cagggaggca gggaggcccc cagagctacg cgggcggggc tgcggggacc ccggggcagc 1620

cggggcgcag ggcgcagggc gcaaggtgca cggggccgag cgtgcagttt gggaggtggc 1680

acagttgggg gcggcgggga tcagggcagc gtcctggggg ttctaggttg ggtctccaga 1740

ttgggtcccc gacggcgctc gcgggcgctc tttccaggga gcgcgggctc tcgtggggac 1800

gcgctcgagg caggtgagcc gaggctgggg cgcccggggg tgtccccgga tgccggtgcg 1860

cgcggctccc tgacccgccc ttccgcggcc tgggggacgg gagaatgggg ggtccacggc 1920

cgccttgggt cctcaccgcg caccgcgccc gcagcggagc cccggagcca tggccctgag 1980

cgagctggcg ctggtccgct g 2001

<210> 100
<211> 2001
<212> DNA
<213> Homo sapiens

<400> 100
ccttccaaaa atgtgtccac ctgggcccct gccctgggac tcatggattt ggagttgtgg 60

ccacacggtt gaggggtgca gtgtccagtg gaatggggca attgcgggcc tgggggccct 120

tggcctgtcc gtggcgggag catctgcaag gaggagcccc agagtccagg gagcactgtg 180

gggagctcct tagagctgaa ctcacccggc gtcaactcat caaccctcca cccatggaca 240

ggggtgcccc cagcacagga gaggactcag ccctctgccc ccacgcacgg tgggtgcctg 300

tcaccctgtc ctgcccagcg gcccgagggc agcagtgggt gtgagggcag cccccggcct 360

cccaagagca gctgagagga tccctgcggg aatccgggct tcgggtgcat gcgatctgat 420

ctgagttgtt tctgacagtg acagagtgac aatctataag tatctcaaga tcaaatggtt 480

aaataaaaca taagaaattt aaaacgatta aaatatgcct cagtcttggg aatcctggtg 540

tctgcaacaa aaaaacttga ggctgtcctt gcaaacggtc ctcatggtgc tgggggtggt 600

gccaggcccg gcgtttgcct ggaacacaga gcatgcgaga aggagcagag gacgtgggtg 660

ccagggaagg accctggcgt cagagccagg gcctggacct gagttaccca ttaacggcac 720

tccactggtt ttcctttaaa gaaatgaata aatacaactg cagtggggct gcttgtggtt 780

tccaaacgtc ggcagaggct ggagacggct ctctagtgct gggtgtggag tgaggcacca 840

cccttgccct gaagcctggg gcactcagtc accatggctc atgccccaga accggacccg 900

gccgcctgcg acctcgggga tgagaggccc aagtgggaca acaaggccca gtacctcctg 960

agctgcactg ggtttgccgt gggactgggg aacatttggc ggttcccata cctgtgccag 1020

acctatggag gaggtaagca cccacctgcg tcctggggca gacccctaag caggggaggc 1080

cgtctccacc ctgacctgcc aggcgcctgc cacgcatccc cagtgcttgg gcagagtcac 1140

tcctcctgga aaccagggtg caccattgcc cacggcagag tcagtcccct cagggcccac 1200

ctgcagtctt ctgggacctc agggcagctt caccactgcc cacagcacgg aggatccagt 1260

cccctcgggg tccactatgg tcttccggga cctcagggca gcttcctgcc aggcgtcagg 1320

ttgaatgggc cgccctgccc gacacacaca ccctacaaac agtgagcgct cttccatctg 1380

aatgtcgtca gttctttaaa gaacacacaa tccgctctgg gtttcctcac cacagccttg 1440

gtgtgtccag acatgtgctc aggtgccctt ctccgaggtt ctttgcagga atttgggggc 1500

ccacccctcg cctccctggc tttggttctc tgggagagcc ctgtgggggg tgccaggatg 1560

ttcagaactg tctgggtgac tttagtgacc ttggactcac cacccccaga agtgcattgg 1620

tgactgaaat aggctcccac gcacctcgag cctcccctca ccctgtgcct gcttctcttc 1680

atgcaggggt cacactctgc tctccatgtg gcgacacagg atgtagggac aggcatgaat 1740

cactgctctc agggagccga cgtacaacat cacgcatgga cacccaggtc ttccccaggc 1800

aacacgggca ggggcttccc aggccgcagg gtcctcctca ggccaggccg ggctgcttgg 1860

tcagcctgtt ccccgggccc tcagctcctt ccctgagagc cccgcacaac tatcccttgt 1920

tataaggtta ctcctttaaa gtgtttttcc tcctgcttaa gaaaagattc tcaacaggca 1980

gaaaatgcag gaaatgcgca c 2001

<210> 101
<211> 2001
<212> DNA
<213> Homo sapiens

<400> 101
tctccagctt gggggtctcc tactggattc ctctacccag ggctatgtag gactccagcc 60

cctccttctc tccatcccca gctcctgtct tctcccactc ccctctccag gcacctgagg 120

ttgcaggaac ccttcagacc cctggggact tgggtaccac tctacagttt tcgagtgggg 180

agaggcccag ccagcccctt ctcccattca tcaatgaaga tgcagatatt cagaatggcc 240

acatagatag agtgacctat taaggtcgcc tagcagcagg tggctgggtc tgactgaaaa 300

tcaggtctga ggacccgcaa agcgtgcctt ccactggaca ggctggtctg atgccatttt 360

aagtcacaga gaatccagtg caaatgtgag acagttagga ggcagctgaa ttaggaagag 420

tgactgagaa tggaagagag agggatgcgg gagagggggt aagacagaaa ggggaggaga 480

gcaaagggga aggaaagcag gtggaaagga ttgagaaaag agagatgcag gataggcaga 540

gacagggaga gagggctgag gaagagggag agagaagaaa aggatgggga gggagatgag 600

gaatgcctga aagggttaga gatgaaggag caggatggag aaggatggcc cagagatctg 660

ggacatgggc gaggggctgg aagccagctc tggacaaggc gtgtagagcc actggggagc 720

ccactgcctt cggagctgtg cctcagctct ccagcctctc ctcgccaagc tcagctcacc 780

tggctccccc tgcgcacctt cacccctccc ggggccacaa agacacacag acacacacag 840

acacacacag acacacacac acacacacac agacacacac acacacacac acacacacac 900

actcttaccg ccccagttgc aaagtctctc ccaaagtggt tcagcagctg cttatggctc 960

tctccttgtg tccccacaat ggtcagtgag atggagtccc gtgttcccga caagaggtcg 1020

gtgcctgtgg ccaccctgac tttgtaggtg gccatggctg ctcttcagga ggcaagaggg 1080

gcactcagtc ccagacaccg tggaggggcc acacgaaggc ccaaggcagg caagagaagc 1140

caggcacaga gtggagtgga cagggctggc ctccgaggtg cagtggtgag gtggcgaggt 1200

ggggtgacta ggcctgccag ccaaattctg gaaaagctgc tgcccttggt ggccggggtg 1260

ggtgccgggc aggcccaggc ggagcccagc tggtggtgag tgagcaggtg tctgggctcc 1320

aagccttctg ggagctggtg gggaggaaga gcaggcaggg cactgggcct gcctctgaaa 1380

gggcggatag aagatgccgt ggatgaaggc gccttcaggg tgggggagcc agtccagcct 1440

gagccagggc cctagggaag ttgcgggagg gggcagggtg tggtccccag atctttatca 1500

gactaataag ctccttcctc ctgccagtcc ccattggcta cagtgccagg cacccctact 1560

aagcagggcc atgggcagtg agtgacttgc attggttctg aggccactgg tgggcggggg 1620

agggtagggg gaggggcact aagtggcagc gcagctggcc agcctccagg ccattcctct 1680

ctgggaaatg ggacaggtag tggccacagt gagaaagctg gcctgtcctt ctgccccagg 1740

gcccagagtc tgtgactgga agggtgggag gtgttgaagc tagggctcgg ggaccacatg 1800

ggtgcccaaa ggcctgaaga tgctgcgggg aggaacgatc agggtaggag aaggggatct 1860

gttgcttcct gtggggccag gatccactgg tgagggattg gagttggata taaaatcaga 1920

agcccaaaaa aaaaaaaaaa aaaactaaat tagaagacag agtgaaatct ggggcataag 1980

aattaagtta aatagctggg c 2001

<210> 102
<211> 2001
<212> DNA
<213> Homo sapiens

<400>  102
agcccctccc cgagcctggg gcttccccac cctaccctcc actcccagcc ttgaccaggc      60
gaatgcagcc tccccctcct cccagcgcgc gcgcgcgcgc gcgcgcgcgc acacacacac     120
acacacacac acacacacac acacactctc tctctctcac acaccctctc ccccgacgcc     180
tgggttcagg cttggcccca ctgaagaagg aggtggccag agttcctctc acctgatctg     240
gggtcgccaa ggctgcaggg agggtgtagt aaccttccta gggagccaga atggggccca     300
gatggccaca tctttcatca ctcctctttg cgtcagtttc tccaccaaga tcagggtggc     360
tggaaggtgc ctttgacaag atgaggatgg agccagacag tctttgggct tctcggtgtg     420
ggacagtcac atgtggcacc cacagcctga gcgcacccgg ggtactcaga gcgggcagga     480
gaagcacacg tctgggcagc ttggttccca ccctcctcca agccctgcgc ctgccccttt     540
ggaggggaca gctttggcct cagagtttgg ccctagcaga ggttcaagta gcaccaaaga     600
ttgtcagcct gccccgatgc ttccttgcct ggcagggatg cttgccaaca gaggtatcct     660
acccttctcg ggattccccc ggtctgcact ttgggtggcc tggattcctg ctccaagctg     720
tagacttgct tcttatggga tgaggagggc cttgggatcc aaaattgagc caagcccaag     780
ggcagccggg aaagagcctg gtcttggggt gcacaggcaa aggcaaaccg ccttagggag     840
acccagtggc agcgaacggg ttaggcgcca ccgtgctccc gcctgactca ccctcgaagc     900
tctaaccact gtgggctatt tttgccagag aggggcccca ggctttccag ccgccccccg     960
cccctgccag accgcagccc aaggcacggc tggccccacg cgccccaggc acattgactc    1020
agactgcccc caagcaagca gaggaacagg gatgataccc acaaccacag tcacctcttc    1080
cttggtggtg gcgttgcagc tctgctgggc agccagcctc cagtctgtgc tgctggaggt    1140
cctgcccgtg ctgcaggggt cactgttgga tagcgggcac agcagtctga gacaggagat    1200
cgagcaaact tacatctgtt gggatattta cagatcacct ttacgtgcca ggctgtgagc    1260
agtctgggga ggtgaaagtg ccatggtccc tgacactgga cagcctggac cagactgctt    1320
ttctctgatt ccctacctct gggatgggga agtgggagca ctagagttcc tgaccctcct    1380
tcccatttag aggacaagag ccaaaccatg tgtggttttg gaggtcacca gaatctgcaa    1440
gccccatctc atagttttca tccttacacc tggattcatg atccccacca gtgtccttat    1500
cctttttctt gcagtcaccc tgggattcct gccaggtcct tggaagaggg gaatgaggtt    1560
gcctaagcct ctcttctcca tggggacctc atggacattt gtatttctcc atgtgtctct    1620
gggtccccct taggtttggg attatttttt gccttctgat tctacccttg tcaggctcca    1680
gcatacagcc aggtgccaag aaggggcgag gggcagaact actacaatag gtttcaactt    1740
cctggtgacc ctaggtctcc ccaagcagga ctgttccaaa tccaaggacc cagagtcctg    1800
ctccttctct ctactgaagc ccaactctgg gacaagaggc cacaggccgg cagactgggt    1860
ggcctgagct ggcccgcagt gccctcagcc cgaccggctg gcctgagcac tgacccgcag    1920
gcggctagag ggcggtggga caggcggggc ctggagaggg ctgtgggtgc acacccattg    1980
cctaataccc caagcacgcg g                                              2001

<210> 103
<211> 2001
<212> DNA
<213> Homo sapiens

<400> 103
cacaagttgc cctactgctg ccgttgatgt tctcatgccc caccagcttc accttgggtc 60

tggttcccgc aagggtgagt tggagatcct tggtcctgag tgagaagtgc tttgtcagag 120

gcagcagagg gcgccatggg agcacacagg aagggcgcct ggggctgtgc agggactgca 180

gaacaggctg cactggcaca cagggttggc tgctggctgc tggctgctgg ctgctggctg 240

ggtttcctct attagcttaa acaatggaag gtggacttgg ataggaaatc tacctcatgt 300

attcctgagc tgtgtctgca ccgttcattc attcgtaggc gactcatgta ttcaagcact 360

catttattta ttagatgctt gctgtgtgct tagcactgtg ccaagccctg tccttagcta 420

ccaagagttt aaaacgtaat ttggaagaca ggatataaga ttatgtctcc cctttgtatt 480

gcgactcact catcaagttc tcccagcatc tagcattcat atctggcact cagtaggtgc 540

tcaacaaata tttgttgcat aaatgaatga aaagattatt agtaataata ccttttattt 600

actgagctct tcccaacttt ccatgtattg tcgcaatcct caaggtaagt cctgatatta 660

tcctatttta ttgataagaa aagtaaggca cagagaagtt aagtaaactg cctaaggtca 720

cacagacagt ctgaagtctt aaccactact ccaggtgcat ctcagcagtg tgaggcaggc 780

catgcttagg gccaatgctt agcacacaca gagattgcct caggctttca gaggacaaag 840

cagtcaggag gggctggggc aataaaggaa ggcttcatgg agacagaaca aaataaccag 900

tgcaggtaga agaagagaac catctttcct gatagagaga gggggcagca agcaagaggg 960

agtcccaggg tggatcatcc atactggctt ccaggggtga cgggaaagga tgccagcaag 1020

gacttcagca aggatcaatc agcaaatccg agggtgtctc tcttgccaag ccccgggctg 1080

tgtgcatagg agaaggagaa catgaatgag aacctacctt tgaggatcta cagccaattg 1140

ttggacagag gcaaaatatg tatataattt tactccagga aagcaagtgt tgagagccag 1200

aagtggtatg agaatcatag tcaggaagac agaaactgtt tccaaatggg aagcacagag 1260

cagtctcctt ggaagagagt gcattagaac tgagccgtgg gggcgggcag gatatcactg 1320

tggagtaggg gaagggcact cctggggtgg caaggtggga ggtgggccct gtgttcccac 1380

agtgggcagg gaggtagtga aagggaagct ggccggacag gcagggccat tccaagaggg 1440

ctttgtgcgc agggctaagc caagctttct ccataggcaa tggggagcaa ctggaggttc 1500

gtagcaggag aaggacacat cttgcccacc aggaggctaa gtaaaaacag ttgtctccca 1560

agttataagt tcctggaacc cttgctggga gcaggattta gaaaaatgat gctgagagat 1620

gctagaaaca tattcgccct gaggctctct cactcagact gcaagaggaa ggtatcatca 1680

gaattgccct taaccaggaa ccagaatagc tgggtcccct tcctgccaag tcagcaacca 1740

gctatgtgac cttgctcagg tccatctccg ggtgtcagtt tcttcatcta caatgcaaga 1800

gggttgccca cctctgagaa cccttctaac cccaaatctc accctatgaa tctaagaaca 1860

ccaacccctc gccatcctaa gtatcacaga gccaggcaag catgggtgag agctcagacc 1920

atccttgttg gactaaaagg aaggggcaga ctgcccatgg ggggcagccg agagggtcag 1980

gcccccatag gtcctcagcc t 2001

<210> 104
<211> 1925
<212> DNA
<213> Homo sapiens

<400> 104
ctctttcaag aaatatgata gtggggggag gggaaacggg aagatggtgg aaggaagggc 60

tttctgggga gatggaggtt gggagcgact tgagcagttg tgagccagaa tgaaaggaga 120

agcggaaggc aggagtttga aggcttcaaa gcaaggagcc tgtccctaag agagctccag 180

agggagctcc gagggacaga tggagagtag ggttggtggg ggctcaatgt ggctggactt 240

ttccaggcct gtgcaatgga agaggtggaa gtgttcatct ctgtggcccc cagcagctta 300

atagtctttc cttcttcccc ttttcctctt cctaccttcc tcccttcctc tctagccaca 360

aacatttatt gagcacatac tatgcaccaa gtcttgtgct aggttatgtt gctattataa 420

tgaacaggac agtgtatatc atccttacaa ctaagtgagg agacaaatat tatacaagaa 480

gatggacaaa tgtgtgtagg tctacatatt gtataatact aacaatgggt aatgttcatt 540

gggcactttc aaggtatcaa gacctttata agcatttctt aataaccttt tacctaaaaa 600

tccatcccct acattaaaac ttacaagata ctactgatat taccttttgc agatcaggaa 660

actgaggcac agagggatca gatgactggc caaaatcaca caacttagat tcacctgttt 720

ccagacgcaa gcacttaact gccacacggt cctgactatg ttgagagctg cggacaccag 780

tatgaacaag acctgcagag tgtctagggc tgggaagaag ccacgaattc atttactgcc 840

ttttcctcaa ggtgtggtcc ctggaccaga catatcagcg tccctgggga ggtagtcaga 900

cacgcaggct cttcagccta ctgcgaccta ctagaccgtg atgtgtagtt taaacaagac 960

ccgcaggtgt ctggtgtgta gttcaagttg gagaagtctg gtctagagcc cattcctctt 1020

attagaaaca ggaaaccgag gcccaagcat ttgaaggtta tggacggtgg tagaactttg 1080

gggtttggtg agtggagccg gcctaggccg gaatgggcgc ttccactctt ggcccagcag 1140

gccagtgggc agggccgcgg gaaggacggg cgctgcaggg gctccgcgcg ctcaaggtgc 1200

tcgcgggctc ccgggcccag ccgggctgag gtggcgtggg cgccctgcgg gccgggcctc 1260

cgcggcgcgt cccagtccca gtctccgcgc tggtctggcc cggtccctca gctccacccc 1320

ctgcctcccc ctcccgcccg ctcgcgcctc ctgaaaagcc agctcgcccg gggcggcggc 1380

gcagagccgg gccggcgcac gaggcagcca gcgcggccat gacggcggag aaggcgctgc 1440

ccctgggcaa tgggaaggct gctgaggagg cgcgggagtc cgaggcgccg ggtggcggct 1500

gcagcagcgg gggcgcggcg cccgcgcgcc acccgcgcgt caagcgcgac aaggcggtcc 1560

acgagcgcgg ccactggaac aacaaggtgg agttcgtgct gagcgtggcc ggggagatca 1620

ttgggctggg caacgtgtgg cgcttcccct acctgtgcta caagaacgga ggaggtgagg 1680

tgatagtgag gagaagggga gggggcgcca accgcccggt gggggcgggg agccaggggc 1740

gagcgcgaga ccccctcccg cgcctgcgtg gagcggaacc cgagcggaga acctcgactc 1800

caggcacctc gcgtgtgagc tcgccccgga gcgcggccca cctgtgccag tgcgcacgcg 1860

cacgtgccaa ttcgcacctg agggttccac ctgccagcgc ggggacttgc ccgcgttctg 1920

tcccc 1925

<210> 105
<211> 2001
<212> DNA
<213> Homo sapiens

<400> 105
tttcagccag ttttgcctct tgcactttgt ccctgcctgt ccccgtcccc gtctccctct 60

ctgctccggc cgccttggcg cagccctaac tcgggcatcc gaagctccta gtgggcgcag 120

aagccagcgg ctgctgcctg cccttcgcct ttcggaggct cacgggaact tggttccttt 180

gctggggcct tgaggaccgg cctgggaagg aggggtggat aagagcagta agcgacaccg 240

gggcctggct gggcaaggga ggaccctttg gctacaggcg ggagggaggc cacccccacc 300

ccctaggtct tggccccaca ggcttggcgc gtttctgggc tggagttctg aatacctggt 360

ggaggtccac ctgagccaaa gggcaaggtg gcagagatgc acccagggag gcctggtcct 420

gcacccccta cacacacacc cttccgtgaa aggacaggta gcagggcata gggccagagg 480

ggatattcag catcccctgc tcctttgatt ccacagcaga aatatccatg taccatagcc 540

agagccctca atctagcagg gacccaggcc aggcctctgg aagcctcccc tctctcactc 600

atggttctac ctcccactgt gcccattatg gcctgatggg gactgtccta gctagatgca 660

ggcccaccta ggtgtgaaaa cccctgttct ttgtggtccc tgaggagcct gcctggccct 720

tcctgcaggc ccgcctagcc acagctgcag ggggcttggt gcctacccca tctctcatac 780

caccagcacc tgtgaccaga gatgtcccca ggccaccgca gcctcctcca cctccaccgc 840

atccccacag ggagattcct gggacccctt cagaaccctg ctggggctta ttcgtgcccc 900

tggaggggac ctgggaggct tgagggctct tctctgaggt gcagaaaggg gggcacttgg 960

gggtcatcaa gtggctttgg caggaccgga acctcagctc cgcacccctt gagaggatct 1020

ttgttcctcc ttgggcctgc agcctgcttg ggcagcgcca cccagacctg gaccttcctg 1080

ggctgcagct cacagcttcg agatgcaacc cggctgcctc tgggtcctgg ggtcccccag 1140

atgagcctgc agatctggtg ccaaatagac atcgttggcc agggtgcagg gtgcagggtg 1200

caggggacct agaacccatc cctcacttgc tgtgttgtct ttccatctct aggtccctga 1260

ctccccattt gcaaaatgac atgggaggtg ggctttcctc cctcagcctg gggccaaccg 1320

aacacaccct ctggggaagt gagacccagg cctggagacc cacctgtgcc agctgacctg 1380

agcagaggga ctctgatgtc ttcccccggc ttttgatagg atgaggctcc tttcttccca 1440

ggactcccac agcaagctca atttagggct tcccaaggac ccctccctgt ctctcctatt 1500

tgctgtgagt tcagcaaagg acgctgcagg cagctgatat atctcccaga actgcaggcc 1560

cacccacaca ggataggtgc ttgtggtgac tggggactga cagccagcag ggcttttggg 1620

agctgggaat tcccagcaga actgatctgg aatctggctt ccactttgct ccgggtgctc 1680

cctgagcaca ctctcaacct ccgcaggccc tggcctcctc agctgtgcaa tggaggcaac 1740

aaaagcacag gtaaggaaga attgcggatg aaggatgcac caggaagcac tcacttatct 1800

gcatctgacc cctttccgtg cccctggccc ttgacctggc ttcccacctg cttcctttct 1860

gctcctgtgt tcccgccatg ctgaccgcgt catctctccc cttcccactg gatgaccccc 1920

ggccccagat cttcctgcca atgacttatc ccattcaggt ttgcctgaat ggagaagcca 1980

ctccattgga gtagccactc g 2001

<210> 106
<211> 2001
<212> DNA
<213> Homo sapiens

<400> 106
cagggtgaag gagatggggc tggagaggct gagcagtccg ggttcgctgt ccgccacttc 60

ggcgcggaat cagagcagga cttgctgagc cctcctaccg ctccctttcc ccctccccct 120

ctgtcttccc ttctgtttcc ttttccctcc ccctggagct gtagcggcag cagcagcagg 180

aagccgagcc gggttgagcg actcggaggc gagcggagga gctggaatat ggggagtcag 240

cgaggacggt ggggccagga gcccttggga gggcctacgg agggagcggc cccaggcgct 300

ttctagagcg tgagcggtgg gggagcaggc gcagggtggc acgagcggag gcggggcccg 360

ggcgtggggc acggctgggg aagctgccgc ctccggccct gcccggctgc ctccgccgcg 420

gccagtggct atggagcagg cgggcaccag acctgcggcg acagagcatc cacggctccg 480

gcggcccatg ccctggctgc tgctactgcc tctcctgctg ctgttgctgc tgctgctacc 540

tggccctgcg gcctcccagc tgcgatactc tgtgccagag gagcaggcac ccggcgcgct 600

cgtgggcaac gtggctcgcg cgctggggct tgagctgcgg cgcttggggc cgggttgctt 660

gcgcatcaac catctgggtg cgcccagtcc gcgctacctg gagctggacc tgacgagtgg 720

agcgctcttc gtcaacgagc gcattgatcg ggaggcgctg tgtgagcagc ggcctcgctg 780

cctgctcagc ttggaagtgc tggcgcacaa ccccgtggcg gtgagcgccg ttgaggtgga 840

aatattggac atcaacgaca actcaccgcg tttcccgcgg cccaactacc agcttcaggt 900

aagcgaatcg gtggcgcctg gagcgcgctt tcacatagag agtgcgcagg accccgacgt 960

gggcgccaac tcagtacaga cctacgagct cagccccagc gagcacttcg agctggacct 1020

taagcccctg caggagaaca gtaaagtgct tgagctggtg ctgcgtaagg gcctagaccg 1080

ggagcaggca gccttgcacc acctggttct cacagccgtg gatgggggca tcccagcccg 1140

ctcgggtacg gcacagatct ctgtgcgtgt cctggacact aacgacaact ctcctgcctt 1200

tgaccagtcc acttatcgcg tccagctacg ggaggactca cccccaggca cattggtggt 1260

gaagctgaat gcctcagacc cggatgaggg ctccaatggt gagctcaggt actccttgag 1320

cagctacacg tcggaccggg agaggcagct cttcagcata gatgccagta ccggggaagt 1380

gcgagtaatt ggggggctgg attatgagga agcctcctcc taccagatct atgtgcaggc 1440

gactgaccgg ggtccagtgc ccatggcagg tcactgcaag gtgctggtgg acatcgtgga 1500

cgtgaatgac aatgccccag aggtggtgct cacggacctg tatagcccag tgcctgagaa 1560

tgctacaccc aacaccattg tggccgttct cagtgtcaat gaccaagact caggccccaa 1620

ccggaaagtg agcctgggtc tggaggccac actgcctttc cgactgaatg gctttggaaa 1680

ctcctataca ctggtggtga gcggcccact ggaccgagag cgggtggctg tctacaacat 1740

cacggtgaca gccacagatg gggaataccc gcagctcaca tccctgcgta cactgaaggt 1800

tgagatctct gacatcaatg acaatccacc aagcttcctg gaggactcct attccatcta 1860

catacaggag aacaatttgc caggtgtgtt gctctgtact gtgcaagcca cagacccaga 1920

tgaaaaggag aatgcagagg tgacctactc ccttctggag agggagattc aagggctgcc 1980

agtcacctcc tatgtctcca t 2001

<210> 107
<211> 2001
<212> DNA
<213> Homo sapiens

<400> 107
cccgatgcct ttcatctgct gtgcctgtgc tgagaccaca agacaacata ctgaaaccag 60

gaagagctgc cccaaattct cagacactgc tctgaggaaa aaactgtccc ttagagtgag 120

gaaaaaagga ggaaaaaggg gctggtcggc caggcctggc accctgaagt ggggcgggca 180

gagtccgttg ggggaagagc acctcagggg accaaccaag gcactgacct tgaagaggcg 240

aaggatgttg gccaccatga tggagaccga gctccctgaa gcaccgatga cacccaccac 300

acgttcaggc ttggtgatga tgggtgggcc gccactgcca cagcggacct ctgtgccatc 360

cttctcgatg agcgcctgca caaaggtcag cgactgctcg agggcatggg tgtccctgga 420

gcaggtgtcc agaatgcggg cgcccagcgt gatgttaggc agcaggtccg ggtcgttgtt 480

gatgcgatcc agggcgaaca gcatggcctc cagccggtgg atgccctttt ccttcttaag 540

ttctccacag ggcttgccct ctgagccccg gccatgcacc gggaacaggc ctcccagtgt 600

gatgtcccca tctatgcgga tggaattcat gtgagggtgg cctttgggct ttcccaggga 660

ggaaggcatc caggggccgt aaaggctgag gagcaggcaa aggggcagcc gggcccacca 720

ccagcccaag cctctcttcc caggcatctc ggaaatccct agagacccat gaacggcagg 780

cccactccta gccctggcag gcccctggcc ccacggcctg ggtgggcatg ggcagggcag 840

cttcagcagc agggggactg agggcagcca accgcgtagc ccatgctgct accctctccc 900

acctccttgt cactcgggcc aagcacagtt gcccgcacag tccaggccca cagacagcag 960

gcagtggccg gggttgcagg aaggctctga tccttgtccc gtcctgcagg cctgttctcg 1020

gtggatgact gtggaaaggg cagaatgctc ctagcttggc gtatcttggc atcaaggaga 1080

aaacagctcc aatcctctcc tcctaccaac cttagaaggg gaagagagag agagaatatc 1140

aaacagaagc ccaaagaaga cattagctag tctcatctct catcaggagc ctgagagaag 1200

gagatgctag aggttatcgc cgaagattcc ataaatctcc tgaacttggt ttcctctccg 1260

ccattgctca aatctggctt aaatgggaga gtgtatggca gaaattaggt catgtttgaa 1320

gcttcctaag gcctgtaccc actgctatca gtagcccggg catcagagag cacaatcccc 1380

aggaacagcc tcgaactggc ttggaaggaa gaggggcctt cctcaacaca tccacatgaa 1440

ggagagcaag aaacaggtcc tatgggccca atcccattac agagatgagg aaactgaggc 1500

ccagagcagg agagcatttt gcctgagtca cgcagggagt gtgcagttta attacaagct 1560

cagataaagc ttggacaatg cctactgcag cccagctttc ctgctgtcag ctctggaacc 1620

cgagacactt tggctcttcc gggactcaga gacccaggaa cacaaataag gccccctcaa 1680

gaaatcatcc tggaacgcag tggtgtaggc tgggagctgc tcactctgaa ggccaaggga 1740

aggccaggga ggagctgagg ctgtgggggt tctggatttt catcctgaat cagggagcgc 1800

aaggtggacc ctgtactctc ctgcttccac cccagctaca cagctacccc aggcagagca 1860

ggggaaaccc agcagcccct gcatgaggag caggcctcca ttcagcttgc acatcctccc 1920

tccctccctc cctccctgac tttcttcatc tctccatctc tccactgctc tttctcccca 1980

cccccacttc ctccctctga g 2001

<210> 108
<211> 2001
<212> DNA
<213> Homo sapiens

<400> 108
atttgtattg gaatagcttt agatatactt caatgtaaaa aatattcaag attcaactct 60

gatctgtcct gtgtgtagtt ggttccaaaa ggtagtgttt tagtcagttt ttagtcatta 120

actatttata gacacactat tcaggagcct aaacatttca aaagaaatgt aaattattaa 180

gtccattcca tggatatttg caaatatgtg gatgtttaga aataataatg gaatattttc 240

accttgcaat ccaagttgtg tggacttaca tgagtattta ccttggtcta ttaatatttg 300

caacatgaac ctatctttcc cacataactc tgaatgggct tagtttatga agttttatac 360

aaatttgcta caaactaaca gctttagtta tacattctac agatacatat taaccatttg 420

ctctctgcaa ggcactattt actgccttat tttacatact tctttctctt tagcccctct 480

taagagattt ttggccaata agggaataat atatccccag tgcttttttt tttttttttt 540

tgggacggag tttcactctt gtagtccagg ctggagtgca gtggtgcaat ctcggcttat 600

tgcaacctcc gcctcccagg ttcaaatgat tctcctgcct cagcctcctg agtagctggg 660

actacaggca tgttccacca cacctagcta actttttttt tttttttaat ttttttagta 720

gagacagtgt ttcaccatgt tggccaggct gacctcaaac tcctgacctc aagtaatccg 780

cccactctgg ccttgcaaag tgctgggagt acaggtgtga gccaccgcac ccggcctcaa 840

tgcattttaa aagcatggta tgctgttgct aaaaccaata ttaaagggta accatagtga 900

taaagtatac aagtatcagt catgatttta tagtttaaat tcttccacat gctaaccaaa 960

attaaaacca attcccaaag gcaatgagta cttacatgac gattgcccat tttccttttt 1020

ctgagatggt ttttctgtat ttcatcaaga gatcagtcct cctacaggca aagtttacaa 1080

cagttttccc cccggacata atatctcttg tgtttaataa gtaacttttg gcactgaatt 1140

tgaatacatt gttggggatg ggtagggagt ggctctagct acagataaac ttagactgcc 1200

caggtttcct tggtacccat gtttttaaaa taatatactt cttaataaat aaagtttagg 1260

cttttaaagt tgaatagctt cttcccacct aaaagactaa acctgcctca cttctgaatt 1320

gttgagttat tctttcatat tctggtgttc atttaaaata ttatgccttt tagtaatgaa 1380

agcattaacc aaatagaaaa atattactat tttttactaa cattgttatt attattacag 1440

agaatttttc gaaggctaca ttgcattaca ctagatcatt taaaaaattt tactgagttt 1500

tgaatttaaa aagtaatatg tgcagatggt aaatttgttt ctaataatac ataaaggtcc 1560

tgtgaaaagt cggcctactg acttcctttt tccttccccc agaggttttc cgtgagtcct 1620

tcctgaagtg gtccatgcat attcagctat gtccgtcggt ctgcctgtct agttacctac 1680

ctctctgttt atcttgatcc aacttgtctt ttcttcctca ccaaatgggt ttgtacaatg 1740

tattctgttc catgccttgc atatttcttg tctttccttt ttttttctga gattattaca 1800

acatgtttct tttttatttt gtacaaactt atgtggtaca taagacattt tgttacatgc 1860

atataatgag tagtgatcaa gtcagggtat tgagagtgtt tgcctgagta caatagattt 1920

ctgttgagta cagccaccgt actctactat ccaacactga atttattcct tccatcttag 1980

tgtgtgtttg taccctttaa c 2001

<210> 109
<211> 2001
<212> DNA
<213> Homo sapiens

<400> 109
agctttctgc ccctccgggg aggcggatcc cagcccccaa cccaccaccc tcatccgccc 60

agcgccttcc ctgtcctggt cccgaactga cataaacagt aggttgccgg acacggtgaa 120

gtccttcaga accgcccctg gtcgggggag aggtgcggca tcccgggaca ggatcctgcg 180

gaccagctcc gcttccatgg gcgacgagaa aggcatcgtg atgtgcctgg tggggaccca 240

gttaaggtgc catctcctag gatttggccg gtgcccatct catcccctca ggctgtctcc 300

ccacgcactg gcccaggtcc tgcagcaacc ccacccagct gtcttgattc cccactttcc 360

tctgttgcac ctcaagctga ctcctgcgtg cccttccaag gccctcccca ccatgccccc 420

tgccgcatca gccgggacct ctccccatcg cttgacacca cccccatcca gcccacaccc 480

cagccaccgg ctcccacctt gcacgcttcc ccccaactcc tgatcccttc acacacccaa 540

gaacccctgt ctcctgtccc cctgcccctc accccatcgc ccagcaccca actgtgcaca 600

cgctccacct cccatcccct acctactccc ttcctctgag ccacccctcg acctccaact 660

cccagccccc acccccaccc cacccggccc cttaccccca tctcccaaac cccaccttcc 720

gcacctggcc ctgccctcac ccacctcctg gccctgcccc ccacctcttg accctgttgt 780

ctccgtctca cggcccccac ctcgccacca gaacctccta gaacagaaca gaacaggata 840

caactgaagc aggcggctgt ccggcctcct ggccccgcag gggcaccttc catcctgcgc 900

agaagcggca ccgccatgcg gaccccgcgg ggcgcctcct ctcggccccg aggcccttgc 960

tgcccctgcg ccccggggac ctctgccgcc cgtggcaccc gcctctcctg ggccgccagc 1020

attgccccct gggccatcag gaatgccagg gcctcctggg ccatcagcat cgcccgtcga 1080

accccctgtg ccctggcctt cggcctgcat ggctccggag cctctgcccg gctctcagag 1140

agaaggtcag ggcccacgag gatgcggagg cagagaggct gcaggaagtt ccgccccctg 1200

gcgtgagatg ggcagcccgg gatcctcagg gcgcctgcgc acaggggccc tacttccggc 1260

cctgggagac cccgagtgag ccccggagca cgtgaccggt tctcaccaac cccgcccctc 1320

cccaagagag cccgggccgg aaggtggccg caatgccagc ttggacccct cacccgtgag 1380

cagccggctg tccgccggac ccctgtcccg ggagccctgc agggagtcag gcactgcggg 1440

gcccagcctg tcccatcccc cgggtctccc tcacatcgag gagcaagacg ggcctgggaa 1500

cacggggccg ggactgtgcg gccatcgtcc cggaccctgc ctgccctgtc cgtccttggg 1560

ggagcgccca ggacagaccc cggggggcag gcctctaact gggctcagca gcctccgtcc 1620

ctgtcctggt cgcccagctg gtggggtagc tggaactgca tgtctggtgg gcgtggcctc 1680

gcgccagcca aggtctccca ggcgccaggt ccagccaagg tggacgggag ggcccagcgg 1740

gacccggggc cggatctcac tcctgggttc aacgggttct gtccctgtcc tagtcgcccc 1800

gcctggtggg ggagctggag ctgcgtgtgg ggtgggttgc gggtggcctc ccaccagcca 1860

ggggctcccg ggctctaggc ctggcccagg tggacaggcg ggacctgctg gacctgaggc 1920

tgggtcgggc ctggctcctg ggttcggcag tatcagtccc tgccctggtc gctcttgcct 1980

ggtagggagc tgggaggtga c 2001

<210> 110
<211> 2001
<212> DNA
<213> Homo sapiens

<400> 110
cttgacaccc cctccgggac tgagccctcc cagccagagc atcaatgtca ttaggtcagc 60

ccatcagccc tgggtagtcc tcctctgccc tctgggtcct ctctgatggc atcaccagag 120

tccacagatg tctgactcca gccctctcgt tctaaggctg gggaatctga ggcccagcca 180

cggggggcgg tgggtggggg gagtttgccc caggccacac caggagtctg ggtggagaag 240

caggaatgca tgctctcagc cctacattcc acagctgccc tgcctccagc acccccaccg 300

cacccccctc cccgggtcac tacggacagg ggagatgtag ctctcccttc cagctggctc 360

atcctgctgc atccctctat cctccaacct cggtagctgc cactcacctc gggtgggggt 420

ggagggctgg agcctgcact gaaaggctgg gctggtggga agtcctgtcg ggaagtgagc 480

ctggaggtgg gatggccacc tatataggct tctgctctgg ctattttggg aggtggcact 540

ccccgtagcc ccggccccct ccccttccgg tgtcaggtac tccccaaccg tggtgctggg 600

tgtctgtatc acattcagcc tccctgggcc catcaggcca agactattaa taactcttgg 660

cccctgcttc cctccagcac ccaaaattga ggcccaggca gggctcgcct cttccatgaa 720

gccttccgtg accactgtag atggcagcga tgcttccttc ctccatattc tgccaggaaa 780

ggaagagaaa cagcaaggaa aagaatgaaa caagaactgt cctctgtgca tggggctgag 840

ccccattcct atgctgatat gatctctcac tacagccttg ccaggtagac atagctgctc 900

tcgcacagat gaggaaacag cataaagacg ttagatgtta agtgacctgc tgctcaaagc 960

cacgggcctg gggttgggag cggactcagc acttttgtgc gaggatctag tgcctggtag 1020

aaggaaatgt tccgcatatt ctcagacaag tcccttcctc tctctttctc agtctttcca 1080

cctgtaacat gagctcaatc agaggtaaac tgtgccctat agatggtcta gggcgagggg 1140

aagatggacc ccctgaaatt gtatgcgtgt gacattgagg cacaaaggcc caagcccttc 1200

atgatttgca aagagatctc tgacattgtc tgttttggat tggagaggac cagctgaggg 1260

atgtgttttg agaagcatga atgaccaaat gaattcaata aatacgtagt gaatatctaa 1320

tctgaccacc atgaaatgtt cctctcctca tccctaacct gaaaagaatc tccagggcac 1380

gaagattgtc aatggtgggg tctagttctt cacagtgaca cgaggaatcc caggctccat 1440

gcaaaggctg tttggaagct ccatggatag aaagggccga gggcaggctg catgggggaa 1500

gctgggactg gggcctcctt gaggtgtggt gctggggcct gagctaggtt gctggtggtg 1560

gcaacaggaa ggagtgtgca gataggggag cacctcttgg agagaagctt ccagaattgg 1620

ggctgtggca tgaacaagag ggacaaatcc aagctgaccc taaatattca aggtggggtt 1680

tgctgaagaa ggaaggtgtt gactttgaag gcagggagtg tagggaggag gtagtgctca 1740

gtcgaaggga tgatagagta agtggtgttg aatcctagga tgttagggct gggacagcct 1800

ccgtggtgat cggctacaaa cttctacaaa tgacatcatg ttatagatga agaaaccaag 1860

gccagggtga cttacccaaa tcccccagag agtagtggaa gagccaagag ctgatgctgg 1920

gtttagggga cctgaaggac accctggcgg agatgtgtct gaactctgga gtgaggtcag 1980

ggctggaccc tgggggcttt g 2001

<210> 111
<211> 2001
<212> DNA
<213> Homo sapiens

<400> 111
caggatttcg gcctcctctg ctccgtgtgt tggctacagg ctaggttcaa atcccaattc 60

tgctctttac tgctgaaagg ctttgggcat gttcgttaat ctcattttct cttctgaaaa 120

agagacaatc ttgaaaagat tgaccttccg tgaagatcaa atgaagtaat gcgtgtagtg 180

tgtctggcac gtcgtaagcg catcacacac gtgagctgtg gcaatactaa ggagcagtgg 240

caagagctgg gggaggagtg gagacgaaca gacctggaca aattagtgac ctttctggga 300

cccctacaga actcttctgg agaaattaat tctatttttc catgtacttt taagtttgct 360

tttcctagca tttcttctac attcctgaat aattttccac tcaaaaattt cactggttct 420

ccctgactcc catattaatc tagacataat tacttcccca gaacagttct gtgggcaggg 480

agggctctac actatgcacc ctccaaaatc cccaaagctc ggaccgtcct ggcctgggtc 540

cccactgctg tcctgcccat gggctcccag gctgtgcttg ctcttctccc accccaaccc 600

catcaccaca aaaagcccct cctccaaccc ggggactttt gtaatttcct acctgaaatc 660

atccactccc tacctctaac aatctgtgat gagcagagga caacatcaag tttgaattta 720

gaggtaggga atgggtgatt tcaggtagga aattacaact tcactttgaa gtttaatttt 780

gtaataaact tcacttaatt ttgaagtaaa tttgcaaagt cagcaaaatt ctttcaaagt 840

caaccttgct ctggcctagt tgcttattta ctgctaagct ggatgttttc attaaaagat 900

taactttgag acttcagcct cttctttagc ctgaactgtg ggtagtgatg tggtgtgttt 960

cctaacacct ccggcagtga gccaggcttt gagtggctgc gtctaaacac ttctttccct 1020

gaggactgga agacattaga ataaggtttg tcattgagtg tgttctgtgt gcttggaact 1080

gagaagggtg atgaagtgcc agataggatg ggaatggaag atatcgtgga agccaactat 1140

gagtttgcaa ggcaaggacc ttggcaaaga tggtcctagg gagacaggtg ttagggaact 1200

cagagggcat gggcaaagga agatttggtt tcaaaaagga tacagacaga gaaggttaag 1260

tctaggaagt tgagtcttag aattgtatgg tggaatattc gtgggtgtcc ataggaggaa 1320

aaatgggtta aaatgttttc acttgacagc gttctgaaaa cctaaaaggt attgctattg 1380

ccttcaggga tatatttagt attctgacca ctgcttcccg gccacaagca aaagaaaaca 1440

gtgacatggc tatgctccta aaaaagtaaa gaagttaatg tgtctcaaac aatgtagttc 1500

ctttgagcaa taagcttatt tggaacaaat gggttacaaa gtcccactaa tgaagagcct 1560

ctgattgtgt taagttgaat ctacagatca acctgcggag aactgccaag aacgctaatg 1620

aagacaggga ggtactggta taaagataga taacagatca atggaacaga ataaagcgtc 1680

cattagcagg aagcaaaatc cgcaaacttt tattcttaac tactactata ttctgcattt 1740

tacaagatcc ttcaattctc acaagatccc tatgagatag acagtaataa cctaatttaa 1800

agatagatga gaattggagc ttgtctagaa tagattgagt ggaacatcac ttgtggtata 1860

tcatttgaga ggtaatggga atattgccaa attcccaggt aactctcccc tcaaagggac 1920

tcctgttgta ctctaaatcg tatcagctat caaatgatga gctatgaaag tctgtcttct 1980

tcgactgtaa gcttcttgag a 2001

<210> 112
<211> 2001
<212> DNA
<213> Homo sapiens

<400> 112
cttttgacac cctaaggttt acaaaaatcc attaaaacag ccatcagaga tccctgtcac 60

aaattaattt ttccattata taagacatat ggctatagcc taaagtaatg cctcccataa 120

attatgatga accatatgga gtaaattcat agttatcgaa cactttagct tcaaaacaca 180

cttaactatt aagataaagt gatttctttc ctaatatctt aaccaaataa tttttaaaga 240

ctcgttcaag taatattaac tatccaaaga gttagtttta tagaagaata aagatgctac 300

aattattttt aactttacac aaggaattac tcttcaacag atgacttcca cagccaagga 360

cacaaaggat gatactattc taagccagtg ctaataagcc tgacatttct tcaagcttca 420

ctattaaact ctgattcaaa ttcgtttgta aataaaagac aagatcataa ttaggtacat 480

acaggtaact tcttctccag acgaaatctt tccatagtaa tatagaaatg ccttgtttct 540

gatgatgtaa tgcagtaact cccttgaaga ttccagggac agtgagcaca tatctgattt 600

aggtcactgt gctttgcaga gatgaattct tctaagttac ttttatggta cagccaatca 660

ctgtgttcta agttacctaa tcttccaagc taataactac aacttacaca gggtcacagc 720

ataacagatt tggaaagtaa tatacacatt tagttataag taaaaatacc aagaggaatg 780

cagcaatccg ttttcaactg aaacctttct gcaatgttag tacaaagagt gcatactcta 840

agaatatatg tggatggatg tgtgagagta tggtttataa ttatcattgt aaccaaaaaa 900

caaagtatta atatggacaa tagctagaaa aaaaaaagag tagcacccct ctaactactg 960

gacttgggat tctctacatt cttaaaaatt cctgaagacc gaaaaggacc ttggtttata 1020

taagttacca catcaggatt aaaacagaaa acttcaaagt acttattcag aattatttac 1080

acaataataa agccattaca tgttaatata cataacattt taatgaaaat aacttttcaa 1140

aaacaagaca aaacttagag gaatacctct gttttacatt tttacaaatc tcttcaatta 1200

tctaacttaa tagaagacaa ctggattctc atatctactt ctgcagtcaa tctgatgtga 1260

tctgcccttg gttaaagtat atttaaaaat ccatattcac acagatattc tgctggaaag 1320

gggaggcata tttcaatatt ctcttcaggt agttatggac attcctctga tagttcatca 1380

aactcaacaa gtatattgtt tcttaatagt tgcaacgtag aatctgaaac cattccaaat 1440

gaacatttta cacattgtta catcaaaatc catttgtcta ctatgcactt tgaatggatc 1500

tctcacattt ttggaatgta tgtgttcaca gggtattaga gcttttccaa atgtcacatt 1560

tcattaaaca atatcaaaaa tttttttaaa tcacacttgt taataatatc accaacaatc 1620

tcataagtcc aagtattgag aaactgtcaa agtcaggtta gcagatgcaa gttttccaaa 1680

attctaagtt ttgcctgaaa gtctgaatta ttatcatggg taacaaacac tatcagttgt 1740

ttttcttgaa gtgacaagtt cacttcattc atttttgaca aaatatctgt caaatgccca 1800

agtatgatta accaagtctg tcattttttc tttcaggtaa aaatggtgtt ccatgaaaat 1860

aacagctagt tgagttcaca attcaggcaa ttaaccagtg atttttctcc aaacaatcat 1920

tctatttgga tatgcagcag aaatgttttt acaaatgctt attttgtcat tcagaatatt 1980

aaagcaatac acaaaggcaa a 2001

<210> 113
<211> 2001
<212> DNA
<213> Homo sapiens

<400> 113
ggagccagag ttgcctttag ctctgcaggg attctcagcc aagaatcaga attttctgtc 60

tgtgattttc ctcaaataaa cccagaccta ttgaatgaga atctctgctt atcagacctg 120

tgcatccttt tcgcctctta catagcccca caggtgattc tgaggcccac gcggcccgct 180
tcccctccct gcgtcaggcc cggccctagc tctggaggtg gttggattgg ttcaagggct 240
gcagccccac ctcgcgtctt cccaccgcgt cggaggacgt aggcgcctgg gatcctggaa 300
ggcggccagc ctctgcagcc gggcccagcg gctcctgaga ctggggcgga cctcacacct 360
gaccccggag cggctaatcc tgcccaaccg tcccacctgc cacgtgccct cggggctgga 420
acgcggcgcc ccgggagagg cctgctggtc agggggcgct ggtgcgcagg cgcgcggggc 480
cgccgtcgca gctaccttcc tgcgccgatt cggagagggc cccctaatct ctactcggcc 540
cgcaccagca gcgctggcgt cagggcgcgg tgggggcaag acgggtgact ggcagcaggg 600
cggtgccggg ccgagaaggg cgcctcacct ggcgcctcct gccttccttt ccacctcagg 660
ttgcagagag ccgccagggc aggtggaagg gccacgcccc gctcccgtga gggtcaccga 720
gagacgttgg gggtggggca gggaccccaa ccctagacca ggtagggggc gggcgaagat 780
tggtggtacc ttcaggtaag agggagcggg gagcgccccc tcggcttggg gtgccagagg 840
tgtgcggcgg gagcgccttt tttcgtgggg ccctcgccct gcagggcgga ggcggcattg 900
cgttggcctt agttcctagt cctggttccc gaggagcccc tgaaggcttc ggatgggaag 960
tttgcgttgg agtccaacac ccgacaggaa ccccagttaa cgcctccgca tggccagggg 1020
cagagttctt gacacctgta gcttaatcaa cgcaggtgga gattcagggc ttgaatcgcc 1080
cggccacgcg caagccgcag ggcaccctca tactactact tctgccttat tacccttaaa 1140
actggagtct cagttatttg tatcccacta ttggtttttt tttaaatctt ttttcgtggg 1200
tttgccccct tttcctcagc tgcgtttctg tcttcagtcc tgtagggcac aatgtctgaa 1260
caagagcgta tacaggaatg tctgcggaag gaaataaggt cacttctcat ttccaccaaa 1320
gatggtttga gcccacagga gttggagaag gagtaccttt tgatggttgg caaccatcta 1380
ccactccgaa tccttgggta tcggtccact atggagctgg tattggacat gcctgatgtt 1440
gttcgtgtct gccccggtgc aggtggtact gtaatactga aaggtaggtt taagattttt 1500
gaaggtctat aaactttgta ataaaaacaa ttgcttagta aataattatt ctagttctgc 1560
agttattatg catcccagcc agtggatttt aatttggtgg gggagggaga ctcataggtg 1620
cttttgaggt tgtgggcaaa attggaacct ctgcaaaatt aacaaaccat tcccccaaac 1680
aatcgtgcac tcacaaaagc tacattttta catatgattt caggttcttg caccttaagt 1740
cgagagaaac aaaaaaagga cttcttaatt ataaaaacat gttttaaatt tcgtgaagca 1800
caagaaaaaa ctattgtagg gaagtttgca gttcatccca agagtttcaa aaaatctgtc 1860
aggggtcaat tagttgtaat gataatacat tgagttaact tttcttcagc tggcaggaga 1920
gctagcaagc tgttgcccaa aatgcaagaa atcgaggaac caagagttac tgtttatcgt 1980
gctgcctgga taaacagctc c 2001

<210> 114
<211> 2001
<212> DNA

<213>  Homo sapiens

<400>  114
   ggtagctacg gaaacgtgga gaggacccac tcactccgtc cagcagtagg acttgtcatc      60
   atccacacga acaacaccac aggtagggtt gtttgtgatc attcacgcac acacacattt     120
   agccctccag aatttgacca ccaaggaagt accttaccgg ctccctcggc ttcttccttt     180
   gtctgtgcac agagttgtct ctgcagtgtg tgaggatgct ttaccctagg ttgcctgctg     240
   gtttcttttc acattgctga gagctcaggt tatttcttgc actgggtgag tcctgatttc     300
   tcacccctga ggccatcaca agagggtggg gcatacctcc tcaggagaga gaaccagaga     360
   ctgcccctga aggggaatgt aatcacgagc aagcccccaa attgttataa ataaagtttc     420
   cccccagtgc cgcaaaagaa atagcactcg aatataaaat tttctttttt ttcttctcag     480
   caaggcaatt tacttctata gaagggtgcg ccctcacaga cggagcaaag gtgagcgcac     540
   acctggacaa gggaggggaa ggtggttctt atgcctgacc cacgtgattc ctgatgctgt     600
   gtccttcccc tgttggctag ggttagacct ataggctaaa ctaattccga ttggctaatt     660
   taaagacagt gagggggtga gtggtttggc gggaaaaatg gttaccgcag agcaggaaat     720
   cggaatgaac cacggtgaag aatgagtgag ggaggagcag gtaatcgaaa aatgttgctt     780
   tatgaggaag ttaagtttaa aagtagaagg caaagaattg aagatactga catattgatt     840
   cttttaagat aaatttagaa ctcatatcta acagctggca tgtctggcat ggggctccgg     900
   ctggggcgca gtcttcagca tttggggctc tggggtgcag ggggcagagg acggccgggg     960
   ctgtggtgcc caaccggtgc taaactcaag cttgggaggt cgcccacgag aaacagtcgc    1020
   ccttccccac ccgcctgctc ggcttggcgg ggagcgggat gtcgctgggg gcgatgccag    1080
   gcgggccgcc tctgatagcc tctaggctct ggcgcgcttc cccgcccctc ccgcgcaggc    1140
   gggcgtggga tcccggctga tccagtccgg gttttgcggc ggagcgggcg ggctgcgcgt    1200
   gcggcggctt caactgtcgc ggtaggcagc agcaggctga agggatcatg gtgcagtacg    1260
   agctgtgggc cgcgctgcct ggcgcctccg gggtcgccct ggcctgctgc ttcgtggcgg    1320
   cggccgtggc cctgcgctgg tccgggcgcc ggacggcgcg gggcgcggtg gtccgggcgc    1380
   gacagaggca gcgagcgggc ctggagaaca tggacagggc ggcgcagcgc ttccggctcc    1440
   aggtgactgc cggagcgtag tgggatgggc gcggcctgag ggtactcgca gcggcacttt    1500
   cagccgccgg acccgccgag ggacagggga tggggcgggc agagcagatg tgtaactctc    1560
   cagaatctct ccttgcccta agatattccg cactttctga gccaggtaga gcacggggga    1620
   agaaaggaaa cggtagtgtc aaggcaggtg gtggaaggcc tttccctcac tgggtcaaag    1680
   aggagttttg ctttgtgcca aagaatctca cgattgctgc ctattggaat gcgaaaacgc    1740
   aagcgtgaca accagtggga tgccaggggt tgaggacttt ggaatcgcag agtgtaatgg    1800
   ccaaggggat ctggagagct tagagttttg ttttttgttt ttttttgttt tgttttgaga    1860
   cggagtctcg ctctgactcc caggcaagag tgcagtggtg cgatctcggc tcactgcaac    1920
   ctccgcctcc cgggtgcaag cgattcttgc tgcctcagca tccccagtag ctgggattac    1980
   aggggcccgc cactacgcct g                                                2001

<210>  115
<211>  2001
<212>  DNA
<213>  Homo sapiens

<400>  115
acattaaatg gtctccttat ggttaggata acaaaaacat gaagaaagca tataagattc        60

caactaattt gacgttgtga attgtgaatt ctgaactgaa accaagtaag tcccttatga       120

tcaggctcca aggttttggt cagcctctca aaattgagag gctgaaaccc tttgaccaaa       180

attaacacat ttgccccttc cctccagctc ctggaaacca ctaccctaac tctgtttcca       240

taaaatagta aactatttta gagtccacat ataagtgaga tcatgtagta cccaccgtgg       300

gcttatttta aggctctgag taaatcacca tctctctgtg ctagggtatt ctgagtcagt       360

cttgttataa aatattttgt ttaggtaaca tattgcaaaa cgtttcataa ttcaatggtt       420

cctttcccag aaaaaaagca gtgaaagttc caatagccaa aaagtgtcta actcaactga       480

cctttgggaa aaaatatccg tataattcca gatagtccta acttctgaag gtaatatagc       540

ctggcctctg ttgataaaca aacaaaaaca aaaaaaccac acacacacac acaaaccagc       600

ttcttgtctt taagtgtgca agaatgttag cacttaaaat ttttaagata cacaaaaact       660

aaacagaact gttcaagttt ttaaagggag cttggaaatt tgaagaaagt aaagttaaaa       720

atattgcccc atgagtcata taattatttt tctttttatt tctcatagct tccctcctat       780

ccattattta ggcaattttg tatccaaatt taataattta acaccttatt tgttacaaga       840

acacgttact atgttatttt aagctttttt gggcaaatta cacataatta gataggctgg       900

gttagggctg tctacaagat cccagcctag agtgtggtgc agtagcctat tttctctagg       960

actcttgctg caacaggata tgggagaagg gaaacattaa cgttagcaga atgggaggaa      1020

tcagattatg gagacatgat ccttgccttc tggcacctct ccagctgatt tagaagagga      1080

cacagaggga gacagacata tggccatttc tataattata aactctgcta catgctgaga      1140

ggatggcttc tcaaagccag ttagtgtaca agcagtggag gttgggactg ccctacttct      1200

cctgcctagg gtcacacagt ctggcagatc tggaaagact tccctgagaa agtgaccttg      1260

gaactgatac cttagtggaa ataactaggc attgggaggg ggtgaggtga ggagaaggtc      1320

aagagctttc atgtagaggg aactttgtgc agagacctta gtcaggaaga ggttttgcat      1380

agtggaggaa ctgaaagcta acagccaggc tacagtacag agcaaatgat gctggggtgt      1440

gaggtgatgt caaaaggtag acaccagggc tagagggcgc ataatcttgc aggccgtgct      1500

aatgattttg ttttctaccc aagaatgcta cttcaggaga tcagtcaaaa ggttagggtg      1560

atggtgacgg tggaaatata gaaaaaggaa tttattcaag aggtattcaa aaagaagtga      1620

aatagagatg agttggagat agattggata aaaagagtga gggagaggga ggtgtcaggc      1680

tgcagcccag gttctgagtt gccaaagagg agtcttttcc tccaggtgag ctaaggctta      1740

gaaccacgat ttttggtcac tttttcttat tttcagtgtg agaagtggcg agttggctgt      1800

ccaggtacac actgtcatct tacacttact gtttaagaca gaggcgcata tttggaaagt 1860

gaatgagtca ggagccagag gagaaggtat gccggcagag cctagggcgg agaggaggaa 1920

gagagtctgg gggcgcgtcc ccaaaaagga gacagggaag acagagcaga ggccggggag 1980

gggagagcaa ggaccatgac a 2001

<210> 116
<211> 2001
<212> DNA
<213> Homo sapiens

<400> 116
ccgggacctc atccttaacc ccttcctggg tctcagctac ccccaccctt tgtctctgtc 60

ccctgcagcc actggagggg tccctctcaa acttcactct atgtggatct ctgcattttc 120

caaagtgctt ctagtaagct cccccttggc cagaattact ggcaaaacaa aattcatatc 180

cctaaaaaat aggctttcat gctgcgttaa ttatgaataa aaaattaata aaataagctc 240

ttcctcagaa gtaaaaaaga ttaaagacac aaataaatat aaatattttt ttttaaaagt 300

tctaggagct gggagaatgg cttgagccca ggagtttgag accagcctgg gcaacatagt 360

gagaccctgt ctctaaaaaa atgaaaaaaa aaaattagct gggtgtggtg gcacgtgtct 420

gtagtcccaa ctacccagga ggctgaggca ggaggatggc ttgagcccag gagtcacccc 480

agcctggatg acagagtgag accaatcaac taacaggggg cacctcccct actcaagcac 540

catccatggc tccccactgc ccaccagatc aagatccagc cattcaaact ggccttcaca 600

gtcaactcct gcatccctcc agcctccaca cctctcacac tccaaggcca gggaaagcca 660

tctgctaagt cacaccctcc agtttctctg tgcttgagct ctaccccctc tcctgcactt 720

gttgaaatcc aacctctcca tgcagtccca gctcaagtgt cacatcctcc acgtcgcctt 780

ccctgacctc atgtctgcct cctccaagca gcaaaacttc aattacttgt gtctcctctt 840

cttcaccagg agacatgccc cacacctccc acgccctctt tccaagggcc cagcaccaaa 900

tgagagcgta ttcttagaaa aaagagggat aaggaagtct tctaagttta ccacctgctg 960

gggcaagtgg agattcttgt cttctcatct gtctgcccac gtgctaaggt cagccacaca 1020

tccacccacc gactccggag gacagacaca tccagaggcg gagctgactc tgtctccaca 1080

catctggagt agacgggttg gccaaggaca acatcctggt cagagggtca aggtgtctgg 1140

cagtgggtgg ggaggagtgg cggagggcgg gttggcgggg agcagaacca gtctggtgct 1200

gcttgttgag gttggaggtc ttcctggagg aagaggctgc agagcctcca ggggtgggtg 1260

ccggggaagc ttaggcccag gatccagaga gaggggaagg gctggttccc aagggctggt 1320

ggagccagag aattcctgag ggccccagag gaggcttctc gggggggtca gtggggcccc 1380

agggggtgat ggtttagaga tcgctgtcca agcttgggag caaacctgga agtaagggcc 1440

atggggggtg ttggtgtggg aggagtgtgg tccagagcgg gacttgggcg ctaatgcggc 1500

ccagcagtgg caggagggcg gggagcctga tggtttggga cttctaggtg tccaagtctc 1560

tgtctctctt catctctcca tctcagtctc agtctctctc tctttccatc gctctccacc 1620

cctccacacg cctatttctt gttctctcct tttctagggg gcagcgctgg agtccccgcc 1680

cagaaccctg cgggagcccg gggcgggatg tccgggggcg gacccctcgg cacggccggg 1740

cggggttaaa gggttgggcc gtgggacggc gcggtcagat cccaggtagt ggcgccggtg 1800

gatccggatc gagggcagga ggctgagacc cgcgggtgag tgcgcgcgtg gtgagggctg 1860

taacctgagc gcggccgcgg cgctgcgctg gaacccgagg ctcgcgcggt tcggtgctgg 1920

cggggcaagg gcgaaggtct gcgcgcggcg gcgggcgggg cgcagccggc cacggggacc 1980

cccccaggac ccaggacggg g 2001

<210> 117
<211> 2001
<212> DNA
<213> Homo sapiens

<400> 117
ctccaggatg gacttttata tcttgataag cctgggggtc accatgctgc gtagggaggc 60

cggagacctg ccactgacgg tggttatccc atgtcatggc tttcttcatt cccactttac 120

ctttttgagg ctggaaggta tttaggggat gacagaaaat caagtttgaa ggtcaatgga 180

aatattgggc acccactaga atgtcccaca tctgtaaata catgtgcatc tttgtaaaca 240

cttaacagct gttttgggga gtgcttgttt aaatccacag atggcatttt gccagaggcc 300

tccttcagtt ccctatttct ctcactcaag gtatcctagt ttggagatcc agaccaggtg 360

cagcccatgt accctgatga accccaggtt cccctctcca acatctttct cacccgacag 420

gtgcggccct gaaatgggtt tctactgtct cctcacacag tccccatgtt cccctcctcg 480

ggttagtttt agggtagtct agcctgggaa ggaatttggg actgatcatg ccacgttccc 540

tggaatggga ctagaaagga atcctgggct tcgtgcggac caaggcccca ctcaggttgt 600

catggaaaat gaggctgaga gcccctctct gcagcaccct gtgggacgga acccgaggtc 660

tctgctggtc ccggtgggca cagacctgca gccaacccgg tgttccgtga agccctgatt 720

tcccaatgaa gcccatggaa gccatcactg gtttccatgg gagcggacca tggccacccc 780

tgctcctgtt ccctgtggga aaggaccaag gccgcccctg gtcctggtca ctgtgggaag 840

ggactgtggt caccgctggt cctggtcccc ttgggaggga ctgtggtcac cgctggtcct 900

ggtccccgtg agaatggtct gtggtcaccg ctgcctgttc cctgtggaag ggaacgtggt 960

cactgctggt cctagtcctc ttgggaggga ctgtggtcac cgctggtcct ggtccccgtg 1020

tgagggactg tggtcaccga tggtcctggt ccccgtggaa gggactgtgg tcaccgctgg 1080

tcctggtcca ggtggaaaca gaccgcggct accctggtga tggaccccac ggggcacacc 1140

cgcgccaccc cgcacccagg gctcgcgccg cccgaggccc ccggttcctg tccccctccc 1200

cgcctggcgc cggaacctgc gagctggggc gcggcctcgg ggaggggcgg gcgggacaga 1260

cccagccgcc ccggctcccc cgccgtccgc gtctgcgccg gccccggggc ctggtcggcg 1320

gcggcggggc cggtcgatgg cccgggcggc ggcggcggca tgcggctcct gttcctggcg 1380

gtgctgcggc cacacaccgg caacgcggtc acggcccagc gcgttcggta ggtgcagggc 1440

gccggggcct acgaagcctg ggccgggggc cgggggcggg ggacgcggga cccggggacg 1500

cggggcgctc agccaggccc cctccagccg cgccggggcc gtcccgagcc gcgcgcacaa 1560

acggatgggc cggtgccgcc tgccgggcgc gcgggggtcg gtgccttctg cgtggcgcgc 1620

gtgtccccgg gtctccgtgc ggccggcgca ttggcctcgc gctctccgga ggggactgag 1680

caggtgaaca ggcccggagc ctgtcgtgga ggggcccggg aaggcctcgc tgcagaagca 1740

ggatgggagc aggatccgca gggatgcgca gcggggtccg cggagctccc ggcgggggcg 1800

cgtttccagg ccggggaccg cggtgccagc cctgccctcg cggatcgggt ctcggctgtg 1860

tgccggcgtc cggggtcctg tccccgaagt ccctttccca cctcccctct gccctgtcta 1920

ttctcgtctt cctggctggt tagctccttc acactcatct ttttctgcct ttgtcttatt 1980

tgtttacttg ttggtctgtg t 2001

<210> 118
<211> 2001
<212> DNA
<213> Homo sapiens

<400> 118
tgtgtgagcc ggggccttgg ccaggggaag gacgctgctc agagctcaca gacctgggtg 60

cccgggccct gacgactcac acaggcccgg agctgggaga ggatatggtg cagggtgtga 120

aggggctggt ccaagacatc ccccagggct gggtcagtgt cagcggtggc ctccagaacc 180

ttcagcgtca gggccagctc agcctccaaa gccacggggc gctccctcac ctgaggagag 240

gtgagaaaga gcaggtgagg ggggaggtga ggggaacagg ttgggggagg aggatagaga 300

ggaacaagtg aaggtgacag gcacagggga gagggcacag ccagtgtggt caggtaggag 360

cagagggaag gggtagcagg tgtggggaga ggagagaggg acaatggaga aggagaaggt 420

gaaggggcca ctacagagcc aggtgagcag ggctgggagg gcaggggtgg gcctgactcc 480

ccctctcacc tgcagctgcc tcaggtccca ggtcctgggg aagaggcggg agcggcactt 540

gcagtccttc agcagaagcg actcttccta gacagcaaag gcacaggtta gccccagcag 600

gaggggtgga ggttagacca ctctgatggg attggaggat ggctgacaac aggatagggg 660

aggttaactg ctgggaggat ggtagaggac cctcttcttc aggaaaacat gagtcagtcc 720

ctgcagtagg agcatgagat agcccactgc agggagggtg gaggctagcc cagtgaagga 780

agctgggagt gggaaagcat ggtgaccctt ggagtgcggg tggaggctag tccatggcag 840

gagggcaggg ggagactcac taaggcatct ttggccctct taaaggcctg cagctcctgt 900

ggagacaggg acttgaactg ggctatgtgg cagccccttg catccgggag agccccgcgg 960

agcctggcga caggaactgc tccagtcacg gtcagcactg cggccatcag caccagcact 1020

ggcatgcagt ccccggtcat gtctgtgtca cagagagaaa gggagctgag ggaatgcaga 1080

ggctgcccac tgagggcagg ggctgcagga gctgagcacg gacagagatg tgggactcac 1140

ctagtttcat tcctgatctc tggtctttgt cagcagaaga aacactctga ggctgtcacc 1200

cagggtctgt ttgggtcttg tctggagtct ctgttctttt cagtcccctc ttctggatct 1260

cagactgtgt cctggctttg actctttctg tcagttttga gctctgtctg ggatgtaatt 1320

cctgcctgag ctccatgggg cagcttttat ccctgacaga agggcagtcc cagctgatgt 1380

aggaaaagtg aaaacacagc ctcaggtaag acaccggcca ccaggggagc cccaggctgg 1440

gaaagcccag agcagggcag ggctagtgag ccagatgagc agctggaggg aaagagaaac 1500

tgatggagac tcaggggtaa cctacaggaa ggtatgttcc caagaggatt ccacctgctc 1560

tggttttgtt gttgtttaag acagggtctc actctgtcac tgcagtgcaa tggcatgatc 1620

acagctcact gcagcctcaa actcctgggc tcaagccatc ctcctcaccc agcctattaa 1680

agtgctgaga ttacaggcct gagccaccac tcctggcata tatatatata tatatatata 1740

tatattttga gacagggtct tgttcggtca cccaggctgg agtgcagtgc aaaatcttgg 1800

ctaactgcaa cctccgcctc ctgggttcaa gcaattctct tgccttagcc tcccaagtag 1860

ctgagattac agatttgtgc caccattccc agctaatttt tatattttta gtagagatgg 1920

cgtttcacca tgttggcaag gctggtctca aattcctgat gtcaagtcat ctctctgtct 1980

aggcctccca aattgctggg a 2001

<210> 119
<211> 2001
<212> DNA
<213> Homo sapiens

<400> 119
ggtggatcac ctgaagtcag gagttcgaga ccagcctggc caacatggcg aaacccccct 60

ctactaaaaa tacaaaatta gctgggcgtg gttcgggtgc ccgtgatccc agctacttgg 120

gaggctgagg agggagaatc gcttgagcct gggaggcaga ggttgcagtg agccgagatt 180

gtgccactgc actccagcct ggcgacagag caagactctg tctcaaaaaa aaaaaaaaaa 240

aaaaaaaaag caagacaaaa ggacttctct cctgtcacag actctgccct ttctgagagt 300

ctgggaccct actattgtca ctcctaaatt ttgtttgttt gtttgtttgt ttgtttgttt 360

gtttgttttt gagacagagt ttcactctgt cgcccaggct ggagtgcagt ggtgtgttct 420

cggctcactg caagccctgc ctcccgggtt cacgccattc tcctgcctca gcctccccag 480

tagctgggac cacaggcgcc cgccaccacg cccggctaat ttttgtattt tttagtagag 540

atggggtttc accgtgttag ccaggatggt ctcgatctcc tgacctcgtg atctgcccgc 600

ctcggcctcc caaagtgctg ggattacaga cgtgagccat cgcgcccagt ctgtcactcc 660

taacttctaa tctgctctag ggagagtccc aatagtccat cttgcatact gaagattaag 720

tgaatttcac agccaacacc acacaatacc ttctctattg agctgtaagc tgcctctgga 780

ctggatggtg ttttgttttt atttttaccg tcagcaccag tgcctctcac atagaattta 840

gccaagccgg ttaggtgtta agtgctgctg ctgcgctgtc agaagggcac acggggctgg 900

ttctagaccg gcctctgctg cttactcgtt cagtaatctt ctacagcaag ttcttgaact 960

tctctgggcc tgttttctac ctcgccgagt ggttaactgc gtttaggatc ttggaagcac 1020

ctaagcacga aaagcacata ggccagaagt taagatgtcc cttggggacg caaataccgg 1080

gaatttctcc ggctgttccc accgatcaag caccagggct ctcaggtcta gctccttcgt 1140

ctccccaccc ccacatatac cctcactttt tcttcctttc cctatgcctc gcagtccctc 1200

aacagggtct gcggtcagac gtcactccca ggtgactggc aatcagggat gccgcctcgc 1260

tcacctggcc acccttgaag attgtggatc ctgcaccggc tagccggctg gcagagggcg 1320

cgccaacagc cgccagccga agtgcccccg cgtaggtggg aggagtgact gcctctcacc 1380

atcccccggg gatgcacctg tggagagacg gcccgagagt tccagggtga gtgcgcagag 1440

gagcttatct gcctgcctgc gggtcggtgt gtcctgccgg ctgctgggcg ctggcctgcg 1500

tcgcctgctt gtctcgctcc ctgtccctcg tccgctaccc cctctgcccc ccctccctcc 1560

agcagccggc ctctcggtca tcccgccggg tgggtgcctg cgaacctcac tcctccctcc 1620

tctgcaggag ccaactggcg agccgcaggc cgccgggctc ggcctgggag cccataaaac 1680

ccgcccgagc cgcgccgctg cgctccagcc gctgctcacc aggcagagcg agcgggcttg 1740

gctggggctt cctgccctga gcgcgacacc gactgccccg gaccgcgcct ccaagcaggc 1800

tgaagggctt ccgctcttgg cttccagaaa agtttggaga aagagaattt gaggcggatt 1860

ggagggtggt agcccctccc cagccttctt tccctcccag aagcctcact ctgcacagcg 1920

tcccccattc ttcccgtcct gattccccat cttcctgacc cctccttctc cctctcctgg 1980

gtcgatccca gtcacatttt c 2001

<210> 120
<211> 2001
<212> DNA
<213> Homo sapiens

<400> 120
gtagcgggga gcaagtcttg ctctgacagg gagcagagca gcaggcggca gagtccaggg 60

ccccacaccc tcatgctcac ttccccgctg cccatgcaca gagcatagca catgcacctg 120

agccacctgg cactcctcaa tctcagcaaa gattttcaag ggccacgacc ccagtgagta 180

caagggagcc tctgccctgc ggcttttctg tatctggaaa caacactgct gaggcctcca 240

gagctgggtc tgctgcttat tatctgtgtg accctgagca agtcacctca cccctgtgtg 300

cctcagcttt ctcattcata aaagtaggag ggattggacc ttcctcatct ggatgctgcc 360

atgatgaaat aaagcatgtg ggacagtgtc gggtgcaaag atgccgctgg gctcccaact 420

cagtgctcat tctctaccaa aatgaatagg aggagggtga ccataattga tgctcctaac 480

tttggggcat ttttgaaagt aaaagggggc attgttaatg atgccagcac tgcaaacata 540

aaccagtaca gtcctgggca aacaccatgt ggagccacca tagtgccagc aatatcatct 600

ctgtggagac aggactcggg ccccacccta agatccaggg actcccaagg gaggctggaa 660

accctggggt tttccaaaag aggctggggc actgcaggct tttattagtt cctagtcagg 720

acacactagt aaataagaca atgtgtcctt atccccaggg cacagcccag ccccacccca 780

gccgcagacc cacctgcttt ggcagaggct atttactgca atggtatttg taatagaatg 840

aaataaaaaa ctagaaacag ccttaatttt tatcagagta ggactgataa aatatattac 900

agtacatata ctattcagct gtttttaaaa tgagagagca aggcagctct atatcgactg 960

atgggagaaa tgtcgtattt tgctaagtga aaaaaagcaa cgtgcagaag agtgtagata 1020

gcatacttcc acaggttaag actatatagt tgaatatgaa cagacgattc aggaaggaaa 1080

acaagaaact agggaccctg gtgttcaggt aggcagctcc ggttgcagtc tgcccctctg 1140

agtcactcca ggcggtgacc tcccccaacc cgccctcatt cctgaacttc tttcctggca 1200

caggactcac tgtgcccctt cccgctgtgg gtacaaggtc tgccccccac cccagctctc 1260

caaagcccac cggcctccct ggaggccgag gtcgacggcc cgtcgcaccg ggaggggggg 1320

ctcccagggg tgccccacgc acggtcaagg tcccgcgcca agcggggacc gggctgggcc 1380

ggaagcgggc acggtactcg cggcaaacta gcgtgggcga gtcctgattg cagtcggacc 1440

tgccgccgcg gcacttaaca gtttgcagag tgcttcccgc ccctgatctc attggagcct 1500

tcggacagcc cagcccatgg ccaccgatgc ccccatttca cgcctgagga agcggaggct 1560

cagacgggcc accagcccct ccggaggctg gcccgggagc gcctggcagc gtcgggtcta 1620

ggagccggct ccctcctgct ccctcctccg cgccgcccgg ggtgtgcccg ccgtctgtgt 1680

gcaccactgc tgagcccagc tccggcgccc tcgcctctgc tgtgggcccc ggggacgcgg 1740

ggtcaggcca ccgcgttggc caggccgctg caggtaagtg ctggacgggg cggggcgagg 1800

ctgggcggga ccctcagggg cgccaagcgc tcctggcgac cccctgcggc agggctgggg 1860

cttgagcctg ccagtgcgct gggatggggc gcgaagcggg ggtgcgggga gcccgccacc 1920

ccgtacctct caatcctccc tctcgcctct caattctggg cccattaggg gtcccggctg 1980

cgggaaggtt ccgagacacc c 2001

<210> 121
<211> 2001
<212> DNA
<213> Homo sapiens

<400> 121
tatgatgggg tcttgggtca caccatatag cctatgcaaa tagtgtgatt tgtagctagg 60

gctgtcttag ttcatttgtg ctgctataac aaaataccca agactgggta atttataaat 120

aacagaattc tttctcatag ttaaggaggc taggaagtcc aagatcaagg tgacagcagg 180

ttcagtgtct actgagggct gccctctcca cttctagatg gtgcctagct gctgtgtcct 240

cacatggcag ggtcggaggg cagatgggag ggagacagct cccttgtacc tctttcataa 300

gggcattaat cccattcatg agagatctac tctcatgact taatcacctc ctaaggtttc 360

accttttttt tttttttttt tgagacagag tctcactctt gtcacccagg ctggagtgca 420

atggcatgat ctcagctcac tgcaacctct atctcccagg ttcaagcaat tctcctgcct 480

cagcctcccg agtagttgag actacagacg cctgccacca cacctggcta atttttgtat 540

ttttagtaga gatggggttt caccatgttg gccaggctgg tctcgaactc ctgacttcag 600

gtagtctgcc cgtcttggcc tcccaaagtg ctgggattac aggcgtgaac caccgaacct 660

ggccaggttt cacctcttaa taatatcaca ttggtgatta catttcagcg tatgaactac 720

ggggacacat tcagagcata gcaggggcct agggccatgc gatatcagct tgacctctgg 780

aggggctaag actcaaccag tcacatttgc acttgaccat gtctccaaga caaatctcca 840

gtggaatctc tggacatcaa ggcccaggtg agcttccctg attggcagta ctccagctgt 900

cttttcatac atcttgctga aagaagtaag agctgtccgt acaactcccc tgggagacga 960

cagctggagg cccctggctt gttgactgct ggaccctgcc gtatgtactt gtgccctggg 1020

ctgactgtag tctgcatttt ttgctgtata gactctaacc atgagtataa gtggcttgac 1080

tgagttctga gtccttctag tgaattgtaa acctggggat ggttttgggg atccctgacc 1140

ttgcagttag tgtcagaagt gatggtggcc actgggaggc cgaggcaggc ggatcacgag 1200

gtcaggagat tgagaccatc ctggctaaca aggtgaaacc gtgtctctac taaaaataca 1260

aaaaaaaaaa aaaaaaatag gcgtggtggt gggcgcctgt agtcccagct actcgggagg 1320

ctgaggcagg agaatgatgt caaccctgga ggcagagctt gcagtgagct gagatcacac 1380

cactgcacac cagcctgggt gacagagcga gactccatct caaaaaaaaa gaagtgatga 1440

tggaaccctc aaactttgta tttcataaag cctaaaatct atttattctt gcatcagcta 1500

cttaaaagtt gttcctaagt tggtgcaaat aaggcttttg agagaatctt ttcttattac 1560

ttctccttgg agaatgtaat tgttgttgtg taattggtat gaaggtttag gcctggaatg 1620

gactattttt agtaaattag atataactct tttctaaatc ttcttctttt attcaatgtt 1680

tgaaactttg atttgttaaa cacacaagaa agtggttcac tttttgaaaa gagcttgact 1740

taacaagtga agaagctggt agagtgtggt aagtaccctc tcccatctgg ctacaatggc 1800

aatggagaat gttatagaac aatcactcgt tagcacagtc tgagtttgca ccaatttgaa 1860

tactgaattc aaatactcag atatacctga agattcctct cctagaataa aaatgtcaga 1920

atctctgcaa ggaactaagg atatcagagc tctggtccaa gatgaagagt ctatcaccaa 1980

gtgcctcctc cccacagaaa g 2001

<210> 122
<211> 2001
<212> DNA
<213> Homo sapiens

<400> 122
gtcacctccc agctccctac caggcaagag cgaccagggc agggactgat actgccgaac 60

ccaggagcca ggcccgaccc agcctcaggt ccagcaggtc ccgcctgtcc acctgggcca 120

ggcctagagc ccgggagccc ctggctggtg ggaggccacc cgcaacccac cccacacgca 180

gctccagctc ccccaccagg cggggcgact aggacaggga cagaacccgt tgaacccagg 240

agtgagatcc ggccccgggt cccgctgggc cctcccgtcc accttggctg gacctggcgc 300

ctgggagacc ttggctggcg cgaggccacg cccaccagac atgcagttcc agctacccca 360

ccagctgggc gaccaggaca gggacggagg ctgctgagcc cagttagagg cctgcccccc 420

ggggtctgtc ctgggcgctc ccccaaggac ggacagggca ggcagggtcc gggacgatgg 480

ccgcacagtc ccggccccgt gttcccaggc ccgtcttgct cctcgatgtg agggagaccc 540

gggggatggg acaggctggg ccccgcagtg cctgactccc tgcagggctc ccgggacagg 600

ggtccggcgg acagccggct gctcaggggt gaggggtcca agctggcatt gcggccacct 660

tccggcccgg gctctcttgg ggaggggcgg ggttggtgag aaccggtcac gtgctccggg 720

gctcactcgg ggtctcccag ggccggaagt agggcccctg tgcgcaggcg ccctgaggat 780

cccgggctgc ccatctcacg ccagggggcg gaacttcctg cagcctctct gcctccgcat 840

cctcgtgggc cctgaccttc tctctgagag ccgggcagag gctccggagc catgcaggcc 900

gaaggccggg gcacaggggg ttcgacgggc gatgctgatg gcccaggagg ccctggcatt 960

cctgatggcc cagggggcaa tgctggcggc ccaggagagg cgggtgccac gggcggcaga 1020

ggtccccggg gcgcaggggc agcaagggcc tcggggccgg gaggaggcgc cccgcggggt 1080

ccgcatggcg gcgcggcttc agggctgaat ggatgctgca gatgcggggc cagggggccg 1140

gagagccgcc tgcttgagtt gtattctgtt ctgttctgtt ctgttctgac agttctggtg 1200

gcgaggtggg ggccgggaga tggggagggc agggccaggt gggggaggag gcgggggaga 1260

tgcgagtaag tggttgggtg ggggtggggg tgggggttgg gaggtgggga tatgagaggc 1320

cagctgcgag gaggggagga ggtaagggct gggaggtaga gggggtgggg gtgggggaga 1380

ggggctgggt gattgggtga ggggcaccgg gtcatgcggg gaggggagca ggggccagga 1440

gataggggga cccaggtgta ggaggggatc aggaggtggg gggaaggggg caagatggga 1500

gcgggtggcg ggaggtggtt gggatggagg gtcgagtgat gaggaggggg ccgggtgatg 1560

ccacaggggg cagggtgggg aggggtcttg taagggcatg gaggagtcag cttggggtgc 1620

agcaaaggaa agtggggaac caagacagct gggtggggtg tgctactggg ccagtgcctg 1680

gggagacagc ctggggggga tgggctgggc aatccggggt aggggggagg ggtgggggag 1740

tgggggaggg ggaagctggc ctggggtaag cagcaggccg gagccacagg ccaagtccta 1800

ggagaggatg ccttaactgg gcccccacca gctacctcgc catgcctttc gcgacaccca 1860

tggaagcaga gctggcccgc aggagcctgg cccaggatgc cccaccgctt cccgtgccag 1920

gggtgcttct gaaggagttc actgtgtccg gcaacatact gactatgtca gttcaggacc 1980

aggacaggga tggggcctgg g 2001

<210> 123
<211> 2001
<212> DNA
<213> Homo sapiens

<400> 123
gacgcgcgct cccctgcgcc cccggggcct cccgtgggct ccgtgcgggg acaaagccag 60

cgccagcagg aagagtgcgg gcaaaggggc gccgggctta aggggccgca tgttcgcaag 120

ccgggaggag agagcgggag actccgggag gatcccgacg caggtccgga gggtgcgcgg 180

cccaagagaa ggccagcggg accacagcgc ggctacgcgg ccggccgcag tcttcaccgc 240

gcgcctgccc ttgtctacgt cccgggggtc ggctggagct gcactgggac tcggtcctca 300

gtgtgccgaa gcctaagcgc tgcggggcgc ggggcgggaa cgggaggcgg tgcctggggc 360

cacggggtcg tcccccagga tgagggcgtg tcccagcgcg cgggaccctc ggaagtccgc 420

gctgggccgg gcgggcacca gcctcggact cagcgggtct cagggctccc tgcgcaacgc 480

ctgcctcgga tccggacccc gggctcgctc tctggtcgcc gtccccggga ggacccagta 540

gggtaactgc cgcgtcgccc cggcggttct ccctgggctc tgtctcccgc cgcctccacc 600

ccccgagcct cggggtccgt cacggcttcc cctggctggc ggggtcagta gaacccgcgg 660

cgcctaggtc cggacggaaa aaagcagggc cggggtgcgg cctggatgag cggagatctc 720

cgcgccttgg gctcaaaggt gcggggtgcg ctctgctgcc gagcccctgc tcgctcagga 780

acactggcca cgccgtcacg ccagccgccc ctgccccagg tctggaggcc cgacctgctc 840

tcctaggcgc agcaccgcgt tctcttccgc gtgggggagc ggcgggcgga agaggtctgg 900

ggctgggcac cggggacacg cgcccagctc ccctggcctc cctgggggga gtggccggtt 960

tcagtgcttc cccaggtgaa atcgccaagg ttgaccagtg cgcccaaaaa agcagccggc 1020

tggatttttc ttcaaattag ggaactctta taaagagttt tagtgttaac aacttaaaaa 1080

atgaaattaa ggccagtcgt tctgattaaa gtgcgcctct ctgatcaaac cgcgctctgt 1140

aaactgttgt tgttttagtt gtttcagccg cctgctttat tgctttatgt taagtatatt 1200

gaaaaatgtt gaaattgctc gctaatcaat tgactactaa ttatctcctc atatgcaaag 1260

tgcaaacagg tgaaaggaaa aaggcaggaa taactaaggg aactgaggct ccattactcc 1320

ttaggcggtg cccaacgtta cattactcta caaaaagaaa tcttgtgggt cacagatcct 1380

gaataatgac ctcaactcct tcaaatagtt tatctaggct tggcatgtcc aacatgccta 1440

agaaattggg aagcaaagca agctttgcag gagccctgtg aaatcgtgcc tttggatgaa 1500

gctcttttga ggaagttgtt acacaggtga agactgcttc ttccaatcgt ttcactgtta 1560

agcacgcata ggaatctggt atttcctaag caaacacagc agtgtaagag aatggcttcc 1620

accaagggag acccacaaga gtcttcctag aaaaacaggt taagggtctg aaggccctac 1680

ttaggtggaa taaacgcttt ctcacagctc tactgaagtg aaataagaaa acattagaag 1740

ttcacagaca ttgacaggca gttcaaaaat taattttaat tggaaaataa aaaaataggg 1800

actagtccga aaaacgatgg ggaaatgcag tatgaaataa tgctggagaa taagtgatac 1860

tgaaaccatg acaaatggac tttttaatca accatctatc cggagcaaaa gacagcaaaa 1920

cctggaagct gaattatttc cctcttcctc tacgttgaaa gctagaggaa ataaacaaag 1980

caatgaagtt ttcaagcctg a 2001

<210> 124
<211> 2001
<212> DNA
<213> Homo sapiens

<400> 124
agagcattcc tggagctcaa gctcctctac aaagaggtgg acagagaaga cagcagagac 60

catgggaccc ccctcagccc ctccctgcag attgcatgtc ccctggaagg aggtcctgct 120

cacaggtgag gggaggactc cctcggagtg gatgggagga gggagcacag agactggcta 180

gggtctcctg gggaggacaa ggctctgaga ggagacagag ggcttttgtt gaagcctgag 240

gaaacagaac accagagagg gacaggggtc acaacaggaa agtcacacta aactgggatt 300

gataaaaagg gaggaaaatc aattgatcat gttttccaag ttaatcatca tttgtcatta 360

ccatttgaaa aaaaagaaaa atgatagaaa tcagaactgc attaggatga cactccaaat 420

aaaaatataa caaggaaact aaatgctgcc cttactcacc aatcagaagt tgaaaaataa 480

ccaccagata cactcattaa ctcatccaca agcatttgca atcaatttta gtcaatggca 540

tacaacaagc atcagacaag tctcagtcat cacagagctt atgctgtcat gaagaggaaa 600

acacacacac aaagagatat agaatgtgag gtcaggtgtt gacaagagcc ctggaaggaa 660

cagagcaggg aaaggtcaga aagaaaagac ccagggtctg tagagggggt gtcagggaag 720

ggatctccca agaatgccct gatgtgagca ggacctgagg ccagtgggga gggagccatg 780

cagacccctg gggaagagca ttccacacag ggaaatgcca aggtcaaagg tgctgaagga 840

atgggggtgt cacactgctg actttgactc agtaggacac acacacacac acacacacac 900

acacacacac acacacgctc caacgtggag gggtgaagag acctgctcag gacccagggc 960

cctgtttttc caccctaatg cataggtccc aatattgacc gatgctctct cctctctcct 1020

agcctcactt ctaaccttct ggaacccacc caccactgcc aagctcacta ttgaatccac 1080

gccgttcaat gtcgcagagg ggaaggaggt tcttctactc gcccacaacc tgccccagaa 1140

tcgtattggt tacagctggt acaaaggcga aagagtggat ggcaacagtc taattgtagg 1200

atatgtaata ggaactcaac aagctacccc agggcccgca tacagtggtc gagagacaat 1260

ataccccaat gcatccctgc tgatccagaa cgtcacccag aatgacacag gattctatac 1320

cctacaagtc ataaagtcag atcttgtgaa tgaagaagca accggacagt tccatgtata 1380

ccgtgagtat ttccacatga cctctgggtg ttgggggtca gttctacttc ccacatacgg 1440

gattgtcagg cctgggttgt gcctgtggcc ctctctgcat tacatcctgt atcagggttt 1500

ggacatttag tgcaggacac acacggggga gacaaacttc cacagatcag aattcctttc 1560

ctggatccag accctgcaga cactcgctgc agaggaagga cagtctgatg gggggactca 1620

gcagggcgag gtcagtgtca gacaagcacc ccatggtctc cccatggacc tgaccctgag 1680

aaagaccctg gagaactgga tcagggcctg gcctgagggg tccccctagg gacccctcag 1740

agagaagctc aactcttccc tgaactgagc ccctgtacaa agccctgtcc ctggttcctg 1800

attccaggtg atcctgggga gtctgtgcca gggctgtgtg gtggcctctt gggcagggct 1860

ggctgggagc aaggatgtga gctatccaag ggccatgact cctggagttg gtcaccagcc 1920

aggtccgagc acccagagcc tcatctagct gaggataggg cctcctcctt cacttgaggc 1980

tcagcatgga aaggacagat g 2001

<210> 125
<211> 2001
<212> DNA
<213> Homo sapiens

<400> 125

tgggcccttt ctggttcctt taaagcttcc cctgaattgt cctggggtgg tcctgaaatc 60
aagccagagc ccatttagta taatctcatt gatccattcc tggagtggag tctgtgagaa 120
tccgggtgac ctccacacct caaggccgcc ttgccaatgc tgagatacct ggatactggc 180
cagaaccacc agtcactcag gatcagggcc aaatgggcag tttccacacc tgccctggta 240
gacttgaaac catggggagc caagcagagg aaagaaacca ctgacacaga agccacggaa 300
atgtgctgga gaggccacat ggaggccaag gccccagggt accacagaga ctgcctaacc 360
tgtcgggtgc ttctggcagt cctggaacaa tatggcctga gagtttagtt catactaagt 420
gaataagaac aggctcaaag ggcatgtgac tgtgaccaca ctagccccag agagggcatg 480
accagctctt aaaaaccagc agaggaagcc tcctgaccca gtcacatggg gaaccatgtg 540
gaggccgaga tataaaggaa gaggaggaag gagcatttca aggaagaaac agtcccgcct 600
aagagggtca ccctaaaaac aaggaagtag gcagtccagt cctgacctgc tcggagttga 660
agcagtcggc gttggaagag cctgaacacc cggcctacca cttgtccttc catctctggg 720
ccttcaggac agagcggact cccctctccc ctaacacaga cagactggtg acagtaagcc 780
tgagaaaggg gctgcttcgg tctccagcca caactctgtg aagccaagcc acgcgctgtc 840
ttccagagag ggaatagaag tttccatcct gtgcacccag tggttgagca agaaggatgt 900
gacgggaccc aggaaggcag cagaagccag caccaacaga gtcagcatcc tggcactgca 960
gcgcagatct cagctcctcc caagagcccc agggtgtggc gagagaaaag gtcatgagca 1020
aacacctctg gctgaaagta gccggaaagg cctggctact tatgggctaa gggttttgaa 1080
tctgggctgc agattgtgca gagagttaat ttacaacctt agaacagtgg acaatgcttg 1140
atagtgtcag actgtgggca ctggaaaccg gctggtctca ggatagggat cctatacctc 1200
caacacttgg attgctgacc tttcacttct caacttggtc aacggaagtc aaaaacccca 1260
gcaaggccac ctgctgaatt ctagaacttc caggacttcc cgacctagag cacatggcca 1320
aacactttct gcagccagga tgtcactgcc ctcccctgat taagataaat agacttggcc 1380
taataattat ccaaatagat taattcttcc aacaaaagaa agagttctgg aagtcaatac 1440
ccaatagcct tctttcctga tggtgcagta gagagcaaaa tccatctcta agcccagagt 1500
tcgtgatatt ttggaaatag ctacaatggt gaatttcacc ccctggagag ttctgtcact 1560
ggcacttgtt ccagggttcc agcagagtcc ctcttctcaa ataggaagca ctggctcaga 1620
agactgtccc cagcataagg taggggactg attgcaccag cagcctgagg gtgattccat 1680
ggcagtggaa aacatctggg tgatctccac ccaaccccca cgcctgctca gccttcctcc 1740
gggtcaggcc aggtcagctg ttctgcccaa gctacaggca cagctgctag cccactgctg 1800
tgtctggaga ctaggtaaga gagaaggaat agcaaaaaaa tcaccttgga ttctcacaga 1860
aacccctgaa tgagtaaata cagggaaggc ttttaccagg aagctgcctc aaagggaaag 1920
cagggggcaa aagggtttga cgccagtgtt tgccactcct gctaagaata agcacccctc 1980
tggaagagag tggggtgcta g 2001

<210>  126
<211>  2001
<212>  DNA
<213>  Homo sapiens

<400>  126
   aattaaagaa cagcaagtca gtgccactta aacggtaatc caaccaccgc cagtcagtga      60
   acagcttttt actgctccat gaacaggtaa gtatagaaat tgtgagtaag catttagaaa     120
   taaaaacaac aacaacaacc aataattgaa aaactaattt tatacttttt ggcatactca     180
   ttgtacactt gtaataaaaa ctgggcttgt agtttttgtc tcttttctta tttgctagca     240
   ggttttgatt tgtattttac agaaataatg gtccatgtca gattggaaat aataaaaact     300
   tgtcttttac ccttgatagt ttgagaaaaa agtaattcag tggggttaga tatggcatcc     360
   gagagatgat tcgggaaagg tgggccaggg ccatgtttat gtggaccctc caatgcctca     420
   gagttcacac ttgtttcagt gggcaacagg actagtaagg tttagaggga ctgttaaccg     480
   aaggtgagtg gactttgcac attttatggt attttttttt tcccagtagg catgtgctta     540
   ttctttcttt tcttttctaa ttaaaagttc tgtttggttt ttttagttaa gaggagagac     600
   tggaactgct cagtgtttca caaagataac tgtttgtatt tgataagagt ttcgttaaac     660
   aaaggaattt gcacttatca aaacataaag cattgaaaac actgagcaca tactctgtga     720
   ccagaatgcg tgtgacttgt tgagataagc cacaccgggt aatcagatga gtctcctttc     780
   ttcttgggca ccctcatata attcaggcac aaacgaaaga gaaaacgctt tttctgatct     840
   ctcagttcgg gtcatctctg ccttccatca tcaccgccta acaatcctac acctggcagg     900
   gtgggaaaac ggtttaataa tctcttccct ttgggaggcg tttgacagct cagaaagcat     960
   ggcctttact caggctcaga gacacacaaa aacctttcac gttttccttt cagcttcgca    1020
   gccccgtcct tctccacctg cacgggcacc cactgaagca caggaccctt ctgccaagcc    1080
   atgagccttc tcaggcccct gccgcccgcc tcgcctgagg ctgcgcttct gaaaagcacg    1140
   cggccctggc ctgcgcacca cccagtctga aggcgacgcc cctgcacgaa ggagtcaggc    1200
   ctaggggctc gggacccagg gcgacactca attttctaac aggagccaca gcccagaagc    1260
   tgagaagcct tccggccgat ctgtggccct gaggtcgccc ttggagggcg agtgagaacc    1320
   ccccaggcgt tccggaggtg gcccccagcc cgaacccggc gcccgccccg gcttcccgag    1380
   tcaggtcttt gccccgcctc tcggccccgc ggcctggccg gcaagcaggg ctgcagtcac    1440
   ggggcggcgc ggagggcccc agcccagtca ggggtgtggc cgccgccacc gtaaggctag    1500
   gccgcgagct tagtcctggg agccgcctcc gtcgccgccg tcagagccgc cctatcaggt    1560
   gtgtttgcag ccgaactggc agtgcccgcg ggcggctggg ccgaggcctt ggctcctacc    1620
   cgcgggggtc gggcgggctg aggctggagc aggccagggg cgggggcaga gggggacgga    1680
   cttccagcat cgctcattcg ggggcgtgac gggaggacct ggctggggag cggcgggttg    1740
   ggagcctgtg gctgcccctt cggtgacagt ggagtcaccg agcctagaaa tggggaagct    1800
   gtgacgtggg ggtggtggag ggtgctgcgg gacagcagcg agcacggccc tcagccgtcc    1860
   ttcccaacac ggtttcactt cccctcagct ccagggctca gccccaagcc gcccccttca    1920
   tcccccatct gcaaacaatc ctggtccccc tgagcctcca atatgcctta tgcagttctt 1980
   ttggggttcc tttcgcattt t 2001

<210> 127
<211> 2001
<212> DNA
<213> Homo sapiens

<400> 127
ggccccaatc agcagtgcca aacttttctt ggagatgtgc tgccggtgag gctcttcata 60

gggactctgc catcatgggt atgggggtat caaacacaca ctaatgaata aacctctgct 120

cagattcata gggcaggaac ttcccccacc ccacaatgaa tcagaccagt tcctgctccc 180

tcctcttact tccccctgta cttatctcag ggagtcagca aagggggtgg gggcgtctgt 240

gtctgtctcc ctcttctatg tgtctgtcat ttacctgggt aactttggtg cctggcaccc 300

tttaagtgct taataacagc gagtgcttaa gaaaatgcct gctgaatgga caacctaact 360

cataaacccc attggtggga acctgcattc ctcagtaatg tcggatcagt gtttctcaac 420

tcctccccac cctaccccat cacccgcccc taaggagctt tgaaaagatc ttttcttcct 480

attctagaac caattccagc ttccacacct cccaccttga gaaggaatgc attagatgaa 540

ccaagacttc tgcctatata catgggctgc tacagttggc caccccggca gtctgcggcc 600

tcccaagcgg gggcctgggt cttaatcctt acttctctct aagctgcatg aagggtgccc 660

ccattccatc cctcttcact tctccccagc atagcgcctc cataaatgtt tgtgaactgg 720

atgaatgact gttgagcgct tggtatgatg agctgcttac aataggtgct caaccaatct 780

ttgtccaatc tatgaatgaa tgaatgaact tcaccctttt ccttccatac ctccgggctg 840

agcctctctc ccacatggaa accctaaggc tgagaccacc accttcctac cagttcctgg 900

gggtgcccag tgaactgtct cagtatcccc agtgcctagc acacagtagg cgctcaataa 960

acgcttgctg tgttcataat gaatgactca atcaggccca cggtgggggt gaaggggggc 1020

tccatactgt gcctaaggat acagtgaatg agcaagtgac acacactccc ccaccccaat 1080

ccctaactct ccttcctccc aggatcgggg gagaaacctc ggcccacagc cgggatgcgc 1140

ccccgccggg gtcaccgcgg cggcgaacgt cccggacttc gcatcccggt gaccccttgg 1200

gatgaaccga agccggaccc gcaccttggc cgtaacaagc gcgctccacg ccgccacgca 1260

ccagcggtga ctcccgagtc ctctgtcgcc ctggcctccc cgcaggcggg tttcgaggcc 1320

cccgcaggga gttcagggcc acggccacgt gcagccgccc ccgagcccga gtttgcaggc 1380

tcggggtccg gttcgcgctt tggtggcgcc cggcgaggct gcagcgcgca aagttcccac 1440

ctcaagttcc cgtcgcggcg tccccgcctc ggagttggcc cggccgcggc ttccgttccc 1500

cccggctgcc cacgggtccg gggcgaggac gcgagagggg cctggaagtc gcccggggag 1560

cccgctctgc ctcccggagc cgccgccaaa cttcggtccc ggggcccggc tcagctcccg 1620

ccctcggagc cacggccgcc ccgtctctgc ctcccggtcc ccgccgggct ccgagtgcgc 1680

cgggagcgcg gcgcagcagc cgccgaggga gggagcgagg caggaggcgg gacggaggga 1740

ggggactgag cgcggggcgg ggagaagggg cgggcagccg aaagcgcagc gcctggcctc 1800

gcctcgcttc gggacagtgg cagcggcgcc accgtcacgg ggcgtccagg cgcccaatcg 1860

ggaccccgca ccttggatcc gggggccctg gcgcgcctgc tggattgcgc tcctcgcggc 1920

cagttcctga gcccgcgtcc gggactgctc gcgctacgcc ctccctcccg ttgccttctt 1980

ccccgtctgc tctcccagga g 2001

<210> 128
<211> 2001
<212> DNA
<213> Homo sapiens

<400> 128
tttttaaata acttcctcct cctgccttca actctcagaa gattatcaca aaaacatcca 60

tattatgaat agttttatag gtcagttccc ttccgtacca ctaggcatac actgatgatc 120

atttccaaat ttttccagac tgctttgata tcatgtgatt cattttaagt agaggcagtt 180

ttgtgaattc cctagccaaa ctttttagat tgtggatttt catataaata aattgacaga 240

ccggtggggg tggaggggga aatagaaaaa ggaaaaaaat gtttttgtaa actatcttac 300

tcctaatctg ttattgagtg agccattttt ggctctaagg caccctagcc cgttaattca 360

aggttacctt cggtcattgc catggctcca gtgcttatta tcattgcctc atccacatct 420

tttgagaaca ctctgagaat ctacacggct ttaacacact tcttcgcact agtgagaacg 480

cccgcaatgc cttattcagt aaggttaaat gagtttgttt gctgaaacat tcgccacatg 540

atcccaacgc tcctccagct ccatcccgtc tataatcgca gagcgaacac tcactcacct 600

tgggcgtgtc aataattgat ggtctgggga tggaagcgtg aaacaccact gcttcacagt 660

ccgccccatc atccagggac ccgggcacat cttcaggcgt ctccgcgggc tgggccgcag 720

tgcggtgact tgaattccga ttcgcactgc catttctctc tgcatgggtg ctcgaatctt 780

gccttttctc tggagctcct tctgcgcttg ggtgtcttgg ctgtctgcct gctttggcca 840

caggctgcac acggacggtg gaatgagtat cagccacgcc ggactgattg aaggaagcac 900

tctccctcgt attcccaaaa caggcgagca gagctgagca cggaaatgga tgcatgcttc 960

tagagggaga cttcagtctt ttggtgagtc ttgctttcca cgtggacatg acttgcagtg 1020

aatcattgca aagttcaaac ggggcaagga aattttccag gggtccacat cacctccact 1080

ttgcttatag gaactctttc caattgcccg aagagaagaa cttgctagtg gttgccccta 1140

aatagaagtt gggatggcaa ggccaccttc cctgagagtc acttagccgc tggggcccct 1200

ttgaaagtgt ccatcctggg caatttcgcg ctcttccttc tgtgatgctg tcagtggcta 1260

aagaaagctg ttggggaggc atgtgagtgg ctccggttac agttcctagg cagggcagac 1320

gcttcagggt agggtagtgc caagtcaata gacaattgct tcaggttacc taggaggcat 1380

aaagataatg tagcctgata gcatctgcca taatagcgtc agcctgcgta tcaataacat 1440

cataacagca gcaaaacctt ctttggttac cccccttcaa atatttattc ccccaaacaa 1500

aacaaaaacg acccagttgt ccatcacaaa tgaagacccc tagaagggcc acgaccctag 1560

cagctgtttc attattaaaa taggaggtag actcaatctt tcgcctggag agatgcggtg 1620

tgatgtgttt tctgaatctt tctggacttt tcgggatctt tccggaatct tcccggaatc 1680

ttccccccac cctcaaaaca agtttcctgg tttcaggatc cacattgcct tttctgataa 1740

ctataaatag gtctttggta atttgatttt ttttttttta ctagctaggc taagttcttg 1800

attagttttc aacaatacca aaaagaaaaa agagagccct acattaacaa ttatccttat 1860

gtattatgtt attatgtgtg gaggtattat gtacatgtga agtattctac ctatatggta 1920

tatatcacct actttcagaa agtaactcct ttttgaacat agatcttctg tttctcaaca 1980

cattaatatg tgaatggtga a 2001

<210> 129
<211> 2001
<212> DNA
<213> Homo sapiens

<400> 129
aagtccatga caagcaacat gccgtcagcg taaatacagc gcgggtcctc tagcacaaca 60

gaacccctga catccactcc attccactac tttggggagt gatgaagaaa gggagagggt 120

tggggtcgtc agcggcggat ccacccctcc atcaaccccc gcccccgccc cgctccaggc 180

tccctggccc gacaccgccc acaaactaac cacttgtttt gtccacgccc cctcccatgg 240

gcacgcccca ggccttggcc ccgcccacct actccggctg gacttgtgcc cggccctgcc 300

ccttgcgacc ggccctcgcg ccggcctcac gttcaggctc cgcccccggc cgcgcctccc 360

tccttccctt tccgacagcg cgggaacggc tgcggcttcg ggcggccggg tttctccggt 420

ctcccaacgc cgaggagagc ttgtaacagg cgctggagct ggcccgccac cgccgcgtca 480

gggacggcgc tggagtcctc cgttcccctc agcctctgag ctgaggcccc acaccagagt 540

agggggcggc gcggcacccg tgccccggca tggcgacacc ggacgcgggg ctccctgggg 600

ctgagggcgt ggagccggcg ccctgggcgc agctggaggc ccccgcccgc ctcctgctgc 660

aggcgctgca ggcggggcct gagggggcgc ggcgcggcct gggggtgctc cgggcgctgg 720

gcagccgcgg ctgggagccc ttcgactggg gtcgcttgct cgaggccctg tgccgggagg 780

agccggtcgt gcaggggcct gacggccgtc tggagctgta agtcctcgcc cgcggcccct 840

tagcaggtat gggaggcggg gggctgtcgg gggaacgaca cacacagagg tgaaataagc 900

cccttcagcg acggcctgcc gcgcacccta tgagcagggg cacccttcac tgagcacttg 960

ctgtgtgcgg gacatcgagg atccgggaac tcgaggcaca cgccttctgt tcgctgtaag 1020

gtatagtgat gataagtgga ggcaaatcac ttttgatcgc gtacttccat ctctaaaaaa 1080

ctggactcgt acgtccagtg taagtatact tatgtataaa gtgtacacat gtgttaaaat 1140

acgtacattt taaagtacaa aaatagatac ttaaaacgtg atggtggaat acgtatccgc 1200

tatcctggtg gctgtatgct ctgcttttca aagcataacg ttcactttaa ggcaagattg 1260

ctttctctaa gaatggtaac tgtgaatcca tattttagtg ttcttgataa tttctggctc 1320

ttggatgaca ttgaactttt tttttaacct ctctgtggct gacgaaggat tcctactagg 1380

agcagaggtg tcggccctgc cattttattt ggttgtgttt ctgttattga tattatctcc 1440

aattttttgg caggcattct tttaagccac gtgcttattt gtgaggacaa cttttgctta 1500

aaaaaaaaag agtacagtat ctacagatgc acttaactag caaagttaac taaaacacac 1560

agaaaaatga gtgggtgagg tgccatgttc agctcctagt ggggccagcc ttccactcct 1620

cttggatacc cctcccccat aatttatgct ggaaaccatc cagatgctaa atatttctaa 1680

tgcttaattt cttatgtttt agataaatag gggaaaatgg gagttccaat attttcttcc 1740

tacactccac tttgaatttc tttcaggaga tgggtcagga gaacttgtct gaatccccaa 1800

attggaagct atagaaatgt ttgcccagct atctggctta ttctggatag agtaaggaga 1860

gggcagggcc ttaacctggg gcccctggat agaagagtat ttcagcacaa ttgatttcct 1920

ttataatcct agatatttta tcaaatgcat ttaacactca ttctgatcac aggtctctgg 1980

cactaaaaca ggttaaaatt t 2001

<210> 130
<211> 2001
<212> DNA
<213> Homo sapiens

<400> 130
atggaggcag gttggtgcct ctggcttctg aagaccttac ctctcagtca caccaggtaa 60

gatgaagaac atccagaagt gaattccgaa cacaagaatg acctggaaga tgacctttcc 120

cagtacagtc ttcctgaggt agagggctcg gtccaccacc atggttccaa actgaatgag 180

gaccatcacc aaaaacggcc ccgggacctg gtcctctgac agtgaagagg tgatgtctgc 240

agctgctgag tgtttctggg gaagagacaa caaattcatg cccaagagag gcaattcatg 300

gcaggaagcg ccatcgccat gggtcagggc gggtgctgtg gcctttgccc caacctaccc 360

caaaggccca aaagccgaag acaatgatga tgaagtccac agtgtcagcc aggaacatga 420

gtacatacac gtcagtcacg gcgctatact ccgggtggat gaggttgtaa aagaactgtt 480

tgatgggcac atagatctcc agcgtcctgc aaaatggaga cccccacccc caacccactt 540

gtttcaggaa gggcaggatg gcagaaatga ccagacactg ccatcatgcc actcctctgc 600

attcctcttt ccagtcaggt cccaccttcc tctctctgcc tcaggatagt cccgctgtgg 660

acagctcctg ttctaacgga gagcgtatac acatttactg ccaagatagg tactgcagcc 720

ctctctgctg atggcaccac agccctctcc tcttgcttga agcataatag aatggtatga 780

tgccctggga acgaggtgtt gggtttgtag cctgattcaa ttgctcacca ccggcgagaa 840

ccatgagcat gctatgtaac ctctgtgttt agttttctca tctgtaaaac tggagtgatg 900

agaagacctg atgcctaggg ccaaggtggg aagtaaatga gggaatgcat gagggcgctc 960

agacttggag tgcttggtcc atgcgcgttt gaatgtgcaa cgcccctgct cctcctgttt 1020

tagggcctgt gtccatgctg gctcccacat actcccctgg ccttttatct atgctttgct 1080

ctgtgcctta gtgtggtgtc ctgttggctc ttgcttgtct gtctaacctt tgccctatct 1140

ttgctctggg acctgccttc agttcaacgc cacgttcagg tgccactggc tctcctgctc 1200

tcagagcctc tgtccagggc tccactccca gctggcagtc ccagtcccat gcaccacttc 1260

catccccagc aggtggaggg acctgtgtag tgtgctgggt aacaggtgta tgaccatgga 1320

caaagcattt ccccaccatg cacttccatc cccttctcag gataagagtg caggtctaag 1380

gcccttccag cattgacact gtattcctct cagtctggct ggacttcacc atgataatgc 1440

ctaagaatac aatccatgag cccttgaaat gacccattta ctctgttcaa atagaaaaag 1500

catgtgcata aatgcaatgg atataaaatg aatcacactt acagaaaaat gatttttaaa 1560

aacaagacaa aaaggggtaa tttcattctg ccttacgcag ataacatttg tgacacgaga 1620

agttacttct ctgctctgct cctggtttat aggaaataat aaagcccaaa accctacaat 1680

aaagcacaca tgccatatgt tctcatggac tcacttcttt atggtaaagg cttttgcttt 1740

gattaaatgt tcttgaagct tttccatata aagttccctt ttgccttttt gcttaatact 1800

caaaacactg cttccttttt gactgctgtt tcgggtgctt gtgctgccta gaaataaaaa 1860

gagatcataa gatgggtggt ggagcctggg gtttgttcac ctaaatatcc aagaagcaga 1920

acccagagcc cacatggtgg agggatagct caggactgtt tgggaggatg agtatgcacg 1980

gccttgggat ttgtcctcaa c 2001

<210> 131
<211> 2001
<212> DNA
<213> Homo sapiens

<400> 131
gtaaactgac actcggtctc tgtgtgtggt atccaggcag ggtttgatgg aaaaagcctg 60

cttccactca acctcaggtc actgtctggg gccagccccg gcccaccccc ctcccacata 120

cccctcccct ccccacccgc cctctctgca tctacagccc caccagcaca ctcagccagg 180

agcagtcccc cggcactcca ggggcacaca gggctcctgc cgccccaggg ggcctgccta 240

ggctgtgcgg ctgtgaaggc tctttgcctg gtgtcttcct cctgattact gcacaacctc 300

cctttctcct gcctcctgca actccccagg ctccccagca ggcacacccg tgcacctcgg 360

tcctttcagc ctagacgcag cggggtgccc agctctacct cctgtttggt ccgctggtct 420

gcagcacctt ctttctgggc ctcccccttc agcccttggg aggccccgcc agctccggcc 480

tcctgctctc ctcctccctc tcccagctcc tatccctggt cggactacat ttccctgcac 540

gtgtaatcca ataactaact gtcgggtctc attggaagtg attgctcccg agggcacatc 600

tctctcagag cctgactctt acttagggag gaattagcca caactgggga gaagtgttct 660

cggacaggag ttagctgaga cctggcacag ttttgggtgg gcaatgttaa gacggaggat 720

gtaaacctag tgacagctgc acaccccttc aggactgact ataaatatag ccatctcgct 780

tatctcaggg tctttctttt tctttctctt tctttctttt cttccttcct tcctttttct 840

tttgcacttt tttcttacag aacactaact ggggtgcggg ggtgggggga agaagtggaa 900

gcaaatgcaa ccctaagtcc catgagatcc aggtttccag agtctttgaa acttggccta 960

ggtttctcag tgtaaatccc tctgctcaaa gttccgaaaa cgtccacgat cgccagcggc 1020

ccgcaaccct gcggcggccc ccagtcgccc taacaattgt cggggagcct gcaggccctg 1080

ccagaaagag agaggattgg cacgttacct ttgggcgtct ggcaccccgg cgtgtggctg 1140

agggcggcaa agtctaaacg aagttgacag atggagtcag tatcggccct ggggagggcg 1200

gccagggagg ctccgagcag ccgcacccgc agcagcacca ctcggcccgg cgggccagga 1260

ggaggaaggg ggcgcggagg ggcggggctg ggtcctggga tgggacgcgg ggcttgctgc 1320

ggggggcaaa cagaaagaat atttcatgaa gtcagaggtg gtcgagtttt gcgaggggtg 1380

ggggtcgtcc ccctggcaga accggagtga gtctgtgatc cctcaattgg aaagagggtg 1440

agtgagggaa aggggaggag gccgcgagag gaatgaagag cgaggagacc ctggagagcg 1500

gggagcgaga taagagaggg aggcggccgc caggtcggga gggggaggag tgagcgcggc 1560

ggcgcggagc gaggaggggg cagggcgggg tgggggggga gggagggagg gagggagcga 1620

ggagggcggc cggggagggc gagggaggga ggaagtccga gagacagagc gaggagcgct 1680

cctgaaggga gacgtcgggc tcgtccccgg ggtccaggcc gcggtcccac tcgcgggcgc 1740

agtcgcccgg cgccggccgc tcggccccgc ggcctgggag gccgttgcgc aaggcagggc 1800

ccgcggctcg ggagggatgc gccgggcgtt gcctcccgcc cgccgccgcc cgccgccgcc 1860

agaagcccca gaggagctga gggaggcgac gccgaagcgc tggcccggag tggccacggc 1920

tgcagcccgg gcggcgggct agggcgctcg cagggcttct gggccgaccc cgctccggcg 1980

cctccgcttc ccgcccgggg c 2001

<210> 132
<211> 2001
<212> DNA
<213> Homo sapiens

<400> 132
ttctccctgg cccagtctag aagcttcttg caggcaggta ccacagtttg gtcaaggtgc 60

catgcgtccc aggtcctagc caggccctgg gctgccgctg gtgaacagga agggggtctg 120

tgccctgcca tgcccccagg tagagctccc ctgggagcat cctccctggc ctgggacctc 180

ccagacccca ccccccggtt gagtgatggc actgccaggg gttgaagacc ctcagccctc 240

gacgttgtcc tctctccatt ggatgccgcc tctctctagc cacccctctc tccctctctg 300

ccccttcgag ctttttctct caatatgcaa ttttctcttt tggtcttccg cactcttggc 360

ccccagttct attgcagatc tgtttctcac tccatctaaa ctcttacccc tgtgtctcag 420

gagctgctct tgctgaggga agaaggggac actacgggac aggggggcag tgtcgtacta 480

aggacctggg ctctagccac tggaggaact ggactcattt gggccctcag gaagcggctg 540

agtcttggtg gggtaacccg gttagccccc gtaagtgacc agcacagggc tgagcccaga 600

ggaagtggcc acccacagag tggttctcat gtccgagggg acctgcaggg attgagcaag 660

aagactgact cgctggatcc ttcgtctctg aatcagttca gggcaggcaa gctggggagc 720

cccctgcccc gtcctgccac caccagccgg atcgggccct ctttttaagg gaagaaagtc 780

tggagtggaa gggagggcac agggcgccag gagcctacat gaagtccttc cagaaatcca 840

caacagctac ctctctgatc ctggagaaac cacctccttg cttaggccca agcaggttcc 900

tggcaggctc aggaccaaat tccaggggcc actcatgggc ctagcagccc aaggccgcct 960

ccccctcgtc tttcttccat ctctctttcc tctgcctggc gagatgccag ccagcacctc 1020

agtgtcccca tctgggcagt ggaaagtttg actctctggg tccttgtttg agtgagtgcg 1080

agtgtgtccg ttcctttgct gtctgcccca ggcgggggag ggggggggag gtggtggggg 1140

cgagggggcg ggggctcagc tagtccagcc gtctacaaga aaattgctcc ctttgaagct 1200

gccagggggg ccgggaagcc tgccccctcc tgctcgcccg ccctctccgc cccaccagcc 1260

ccctccctcc tttcctccct ccccgccctc tccccgctgt cccctccccg tcggcccgcc 1320

tgcccagcct ttagcctccc gcccgccgcc tctgtctccc tctctccaca aactgcccag 1380

gagtgagtag ctgctttcgg tccgccggac acaccggaca gatagacgtg cggacggccc 1440

accaccccag cccgccaact agtcagcctg cgcctggcgc ctcccctctc caggtagggc 1500

tggcttcaag ctgcctcctc agcaacccag agatgcccct ggctctgctg cctccgctgt 1560

cccaagccct ggtcctgctg tccccagtgc cgcgagggtg tccacagatt tccccggtgc 1620

tctctgtagg ctgctgatcc acgccccttc atcgccaccc tgcggccccc ttggtccctg 1680

tcaggcttct gcctcgtctc gccgccctcc aggcaccttt ccctcacccc ttcctctccc 1740

tctcccttct gaccttgctc tgcttcatcc acctcttgtc tctctgcctc ccgctcgggg 1800

tccgtcttct tggctaccac cctagagcgt ggctgggtga ctggtacccc agctttgcca 1860

atggccctgt ttcatcattg caagtcccag gcgcatgctc cactccctca gcctcgctct 1920

gcccaggcgc ctccttgctc cagggcttgg cgcctggccc gggttgggtc ggatcgggga 1980

ggaccgccca gcgcccaccg a 2001

<210> 133
<211> 2001
<212> DNA
<213> Homo sapiens

<400> 133
ctgggactcc tccctctgtt gaccaagtgt ggaaccctgt aataacttct tagcaaccct 60

cagaccaagg tcctcacctc cctgagaccc ggcaggcaga agtggggagg ggcaccacac 120

gatcacacct gcatgggatt cccaaagctg acagaaggag cagacatttg gcgggggggg 180

gggggggggt ggtgggcagg ggagggggac aagacgggga cttcctttat ttgggtgtcc 240

tccaggtatt cagatttact cccgacaggg cccagccctt tccctcctgc tgaatcgtgt 300

atgggtcttg cacgccaaag ccaccatctc cctgggatcc ccggtgagaa agtgggggcc 360

acttctcttc ctcaccgtcc tgcctggggt tccagagctg ataagaggga cagatttcct 420

agttctgaga tgcggggatc tgctgagtcc tccctcaggg ctctgggact cctccctctg 480

ctgacctgag gctccactcc tcagaccata gccctctcct cattgacacc ccaaaggcac 540

agcaaagaca catcacatgg gccaccgtgc ctgaaaccac ccaggctgaa ggtccccact 600

gatctggact ccaaggtccc ttgagtcctt tctcttcttc ctccccttga ctcttggctg 660

ggttgttctc ctctaccacg ccccgccgca gacctgagtc accgtccctt ggattcctca 720

ggccgagtga agagacatct cggtctgaga cagaggtggg gtgggcaccc tgtcctggga 780

tcccttcatg cctccctcat ttcccagcag ggcccgggcc ttccctctgc tcccctgaag 840

ccgcattctt ggtaccaata tccccttccc tacgtcagcc tcagatgccg acgtcagaat 900

ttgcgttcct ggtcgccatg accagggctt cccagggccg acatcagggg cagagccctc 960

cctgagcgtc ccgacattga tgctggcaga acctaggcgc ggctcccgcc gaaccagccg 1020

ggccctggtc acaccaagct ctgactccca gagtcccctg cggcttaagc ggcgggagag 1080

agtgggggcg ggggtggggg gggggtgaca gcccagcctg agaagtctgc cctcaggtgg 1140

tccagagctg gcagcagagg cagcgctgta ttatttaggg ctttctatct gggttggggg 1200

cgtcctcagt cctccctcag cctctcacct tgcctcctca cagagcctgg gcccgctctc 1260

ttcagccgat ctcaagcctg tccctcagaa cgagaacctc gcttctctct gatccccgag 1320

gcgtaagtta gtggcgtcac atccggtccc cggggcttcc ctgggttgac agcaggggcg 1380

gagtggattc tgccggaact ggggtgggag ttggggtggg agtgagggtg ggagtccggg 1440

tggggtgggg tacgggtgag gatggggttg gggcaggggt ggtggtgaag atgagtgtgg 1500

ggaggggcgt ggaggtggga actgggatgg ggatcttggg gctgtgggtc gtggtggggg 1560

tgggggtggg gtctttcagt catccctcag tgtctcgacc ttgatgcctg gaaaagcctg 1620

gacccctgtt ttgtgctgag gatgtcttct ccccttaaac caggtctctt gactcggagt 1680

cttccaaggt ggcagtgagg ggagggttgt ggtaggggtg acaacattgc cagggtcttc 1740

cagggctgac aagaggggct cagctggatt ctgtggcccc tttgtgaggg gtgggtggac 1800

cctcggtcct cactcaggag ggtcttcctc ctggcttttg tctctttgat gaagtgatgg 1860

gtgggagatg ctcagtttcc gtcacctctg atcatttgcc cagctgcatc ccttgcagag 1920

catgggtgcg gttcccaggc cagatggtcc ctggggagct gcaacacccg tgattgtaat 1980

gacctatggg agaagacgca c 2001

<210> 134
<211> 2001
<212> DNA
<213> Homo sapiens

<400> 134
tttaacttct gtatggtgag atcatggata ttaataatac ttttccatat tttccaatat 60

ttctataatg accatttatt tctcttataa tcagtaacaa aagaagggaa aacttggtct 120

aaacacgaat ttagggactt aaactagact tggagaaaag ctttctatgc aagatttatt 180

agatactgga aaggatgagg aagacaaatt taagtcaata tgatggaaag agtggtggat 240

ttggaatgag gaaacttaga aatgaatctg ttttctgggt gaccttgcgc aagcctttgc 300

taaaatttgg ttttctattt tgaaaaacgc agctggctgt ttaaggaaga taatatccac 360

cagggcgcct ggcccagaaa aggcgctctg taagtggtag gtggatcctg tctaatcaac 420

gccaattata accaggcatc tgcaagcttt gtggagggcg catctttcac tgctaccaga 480

gcctacagtt ctaataatga aaattgcctg gaaaagaacc acaactctta aaaaaaaaaa 540

aaaagtccaa aatcacaaag tggcttttgc agcggtcctt gcctgtgacg ccgggccatg 600

gctggagctc cttgctccgt acctctcagg agcctccatc ctgtaccaat ggccagagag 660

ccggggggtg acagacaggg gggccggcag ccgagccagg caggacgctg cgcgccgcgt 720

tactggagcc ggtgtcccgg caaccccgga acgcgcctct gtgggagaag caggcggctg 780

cggtgtccct tttgccctag gctctcggtt ctcaggcctc ggcgtgcggc gtgcgttcgg 840

cagttcagtt gccagttggt tcgttggtcc gtggaggcct ctgagcacag acccgggcct 900

cccctgggga agggggtggg aagaccccga ggtggaaaag aaggagcacc ttgagctgga 960

tgggggctgc agcgagcctg gttcgggctg ggggcggact cgctggggct cccagggctt 1020

ccaaaccacg aaacgcccag gtggagggag taaggctccc gtttagaggc cagtcctgcc 1080

ctcctgactt tcggtggctt gtggaatttg tttgttttga ctgatacctt tcttacgagg 1140

gactttcttt gtctctgggt tgcttctaat atttaaaatc atcatgggca cagatgagtc 1200

catccatgct cactggaaac agtccacggg gctgccgtct cccctccatc tcaagcacga 1260

cctgcggcag acagctggag aaggttccag aaaaaaggga ctctggcatg accgaggtgg 1320

agaggaccta cagttgagtt ctaagaaatc caggctgtgg agttggtggc cacatccaag 1380

tatactacac ccgggggcgg gggcaggtgg ggggcaccat ttgaagttta gaaggaagct 1440

agtttggggg catgtaaaaa gagctgcttg aatagtagct tatgtttgtg ctaacccaag 1500

atttgattag gcagagagca ccaccaaagc agagagtctc ataaattacc tcactggatc 1560

tcacagcagt ggagaagtat attacctatg agtaaaggga gccagagatt tgacctgccc 1620

agtgcttggg ttaataaaag gtgagcctgg gatttgacat gagcctggga tttgacatga 1680

gtctgccata ctttcgagcc tgtgtccttt catctttcct ttgctcagat attagtaggt 1740

ttgtgtcatt gtttattgga tgggacagtt caggtgagaa ttatgaacac tctaaaaatt 1800

aaataatggc tggtagctcc ataattggtt aaggccgagg agggtatttg ggggcacgtt 1860

tgcctttgga gaacagtgtc atggcgctga acagccacag tctcccagaa aagctcagag 1920

tgcgcctgcg ctcaagttct catttacctg ttatgggtga gtggcctgag tgctgcaaaa 1980

aggggttgtg aaatatccag a 2001

<210> 135
<211> 2001
<212> DNA
<213> Homo sapiens

<400> 135
tataggacga aaaaaaaaaa aagtctagca atcaagggag tgcgggagta cggatgcgtg 60

tgtgtgtgtg tgagtgcgcg tttaaagaac ataaaacgcc acaaataagc acttaatatt 120

ttactgagtc gtcatacagt aactcatttc taatgagact gttaaagctg ttaccaaatt 180

ccaacaggtc atgaaaaaac tgctcagcac ctctctgtct tctgtcaggt tagggctccg 240

cagacggacg acagaggatt atccgtagac ttagatgggg gttcctccgc ccctctctgt 300

cctcctgcca ccacagccag gctggacgca gaaccgcacg gaagcctccc caactcctgc 360

ctctgggagt gctcctgcct gcttcaagga atctgggcag cgggaccctc tgggtttagc 420

gtcctcgcct ggggaagcaa ctttggcctt accgtagaag ttacaaaatt ggcgggggta 480

gggagaaaga ttataatata tatttggagt cctttcttcc agtttaataa acacgtactt 540

ttctcattgc cctagtcctg gtatgcctaa tggcggggaa agcctcaaat aaactcggtg 600

ccctctggat ctccccaacc tgcctttagt ccttgcgaga ggcgtgagag aacttcctat 660

tatcatccct tttgttattt ttattacatt atttaatagg ctccctctac ggaaccaaaa 720

atagccttaa tatgcttact ttctccctgt actccgcccc aaactgttag aacagcagaa 780

aatttttttt tggggggggg cggtttgggg gaaggcagaa ggagaagaaa atttgattgt 840

gatggtggtg gattttttcc aaatttcccc acagctgtta tcaaaacatg caaatgagat 900

tttccaacag gatcttaaac aaatctggag gagttaatgc cgaagcaaaa taatcagcac 960

gtttgaagtg actgtgagtt tcagtttgtc ttttctcccc gtgatattat tgcaggggaa 1020

tgataattag acctttagaa atccaaaggg gctctcttgc tctcttccct ctctcatttc 1080

tcttctctct ttctctctct ctctctctcc cttcctccct ccctccctcc ctctctccct 1140

ccctctctct tgttccctcc ctctcccttt ctctctaact ctgatgttgg caaagggggt 1200

tttcttaatc agactgtttt ttggtcccag ggaaaggagg aagaaggaga ttgtgatgga 1260

gaaagggggt ctgtgaaacg tcaggcacag cacaaaggct ttgccacgta attacaggct 1320

cctattaagt cgagatctgc cctcccaggg gtctccaatt ttcttgtatt ccctacaaag 1380

cctcctctgc atgccagttt gtgccttttg aagtgccaga gagcttcttg atccaactga 1440

gaaggaaaaa ggagcccagc aagaagaggg ggagagagag aaggggaaag gggggaaccc 1500

accagcaccc tccgtcggac tcttgaagcc tttttttttt aattcttaat tttttttttt 1560

actctttaca aaaagtaaag tgagaatcct gctctctaat acatctgcaa gacatcaccc 1620

tctcctcctg aaactttagt cactcctgag aatccacagg agtgcagaga ggggggaaca 1680

cgttttcttg aagatgtttt aaagctggaa caagccttct tctgttggtg cttgaactct 1740

tgcctgggaa taactttttt aacctttaaa aaaaccattc actttgattc ttctctccca 1800

ccccttcttc tctcttcttc tgtttgccta actcccccgc cctgctggcc tccgctttcc 1860

tctctccccc ttgttattat ttttagtctg tgcgtgtgga cacttttgga gagttggaag 1920

ggattttttt ctcctgactt gaacataggg tgacttttta atattgtatt ttactgtgga 1980

ttatctcttt ggaccgcgcc g 2001

<210> 136
<211> 2001
<212> DNA
<213> Homo sapiens

<400> 136
atatctagag tgatatttta cctgtttctt tttcttcatc tggattcttc atgggacacc 60

ttgatatcac tctagatatc aagagtgagg agaattctca ataaactggc ttaataggat 120

tctttgctaa agcagggttc aggaggtcaa tgtcaaggct tagttcagaa gaggagcctg 180
actaaagttg ggtcaaagag gaagtctttg tctttgttaa tctttcaaat ttgaattcac 240
ccactaataa ggaccaattt agaatgctat atgtacttta caagttcttt tctagacctg 300
gaaaataagc tccatcatta ctcataatga cattataggt aactgacgcc agctgtcttc 360
ataagaacaa aattatctag cagaattgca cacacactga caatttggtc caaatgaatt 420
ctaggaaatc tgattaaact cagagggtaa tgaaaatatt tgtagtattt atattgtgct 480
tagtatttgc caggtactgt cctgaatcat ttgcatggtt taatctctta gtcctcccaa 540
cagtctattg ggaggtatta ctaacagtct gtagggaagt actattacta gccccatccc 600
aggattacag acagagaaac tgagacacat agcagtttca aagcttgtct gaagttactc 660
aattataata tgtcaggttt gtgattggaa cctagcacta taacttttga gatgatgtgt 720
ttactacagt gcattcctac ttctgtaaat ctcttctcct tcctttctct gagcaaacac 780
ataaaactgg gaggtaggta ggagaattgg gatgacattc atttgtggaa gtccatatac 840
tgtgtcatat tcttcatctt cagcactttt aatctctaaa acatagtgca gtatttagca 900
ggtcatcaat aatttttgtt gttgggtaaa tggctgatcg aatgaattaa caaaaggact 960
ggatactcca tttctgatca ccatagagat aaccaaatta cgtttctcaa actctgtcat 1020
gctgagggct gcagtgacaa cattttagga ctagaacaca ttacttggat ttcacaatac 1080
aaagatgatt gcagttgtcc ctggtaagct cagcccacag agcctttcaa ttcagcttgg 1140
ggtgagctgc acttcaggca tggggatata atagtgtgat gaaccattct tctgaaggtt 1200
cttctgaaaa gctgctggtc ttgggatact gtgagagccc ctctcattgc tctttagaag 1260
taatttaaat ttttctgcta cattgtctgc tcagctcgta ttctggtcat agaggaacct 1320
gaagccagag aaactagaca aaaaggtaag agaaaaccaa gcaacaaatg cagtctcagc 1380
tgttgctaac gttattcaca ggagtgacaa actgaatgaa tcttagggtg agagactggt 1440
ctttttacct catttacact tttccttggt ctgcactgta gatcagcaga actcatttat 1500
cctaagaaat cgatgactaa ttttctgtga agcagaatca agaaatctgg tctacattgg 1560
ttaaaaataa gtacttggtt caccttgtgc tgtgaagtcc aagccaaata attgttgcaa 1620
gttgttgaag catttgagtg aagaaaaact gaccaagttc actatgactg catgaaatga 1680
aatgctgaac tttttggggt tgagggggtt ggaaggatgg atggaggaag acagttgttt 1740
ccagtctctg aaattcaatt ctgcagtaag tttctgtatc accaagtgtc actttggcat 1800
acctcgtgca tatttatagt ctaaggcttt tcatatagga ttcaaacata gattaaagta 1860
gcagggaact tgagagctcc agggaagaat atgatacttt aattgcattt gatcatttta 1920
ttgaatttgc ctaggaaaat caattaaaac acaaaactta atttttgtac ttacagcaga 1980
gattatacat tccatcttgt c 2001

<210> 137
<211> 2001
<212> DNA

<213>  Homo sapiens

<400>  137
gaacgggaag acgcctttca ctgacccggg cgcgggacct cgggtcccgg gccggggcca      60

ggggccatac cctggcgcgg ggggcgggcc gcctagcggg ggaggggggc acgcccccga     120

ggtgggggca gcggctcgcc cctcaggtta actcttcgct ggccgaggcc aggcgcgggc     180

atgctccctc gcacccggcc aggagaaaaa gggcagcggg ggatgggcgg gcgcgccggg     240

cgttgccagg ctacggcccc ccagggagcg cgggggcgcg ccgggccgcc aggggccgtc     300

aggggccgtg ggccgcgcgc ctccccgccc gcggggcccg ccgctgccgc tgccgcctcc     360

ggctcctgcc catggccgcc agccccggga gcccggccct cgcgctcggc gccgcggccc     420

ggcccgcgac aggggggcag cggcgcggcg gcggcccccg cccgcggcca ggccagcgcc     480

cgcgctcccg cctcgcccgg ccgcgctctc ggagctcccg gcgcccgctc ttcgtccagc     540

agccgctggc cgtctccagc tcccgcccgg gacgacgggc acgcgagcgc gcccacggcg     600

gcgggggagg ggcgcgggca cggggcgcac gcgcgccggg aggggcgggg aggggagcgg     660

ggccggctgc gggagggagc ggcgggggcg ctggagccgg cgcgggaagc cgggggcggg     720

cgctcgacgg aagcgggcgg gggtcctagg caccccacga cacccggcgt ccggtcctga     780

ggggcgggca ccaggcgcgt tgtctgcgcg atgccgctct gggagagagg ggtcctgcca     840

ggaggagcag tctcctgggt cccccagaga gagtccgcga tttcagcccc tctccccggt     900

cttggacgca gcagccctca cgcctctgct ccttcctcgg gcttcgcctt aaatgactgc     960

atccctcctt caactcagcc attccctgct gtctttgaaa cggacacctt tgtttaaaac    1020

aagaaagaaa gagagaaagg gaggaaagaa aagaaagaga gaaagaaaaa aggatgagcg    1080

aagcgtgggg agggggtggg tcgtggggag agtggcctcg gaggccttgg gacccgggcc    1140

tgcacacagc actggccagc ctgggcccgc cgcctacctg ttcctcagcc ccagccctca    1200

cccggtcccc gctttcacag cccttccatc tcttcctctt ctctcccacc acctccccgt    1260

cttcttggac aagcctggag ggagaaggcc tttgcctgga aagcagcagc gggtggaagt    1320

ctgcgttccc agaggctgcc ccggctgcag gagatcactt ggagaaattg ctacatgctt    1380

catttccagg tttgggcaag tgagggaacc cccagccaca cggggctgtt gaatcggagc    1440

ccttcctttc ctccctggct cctcgttaga agagaaatca agaatggcgg gcctgcccag    1500

aatgtggaga agagaggcga gtttcttatc cagggcttct cccagaaagc agaacttgag    1560

acaagggctg gtgtgcaggt tgcaacctgc cacccaatgg ctggctagtc ttgaggaatg    1620

gcgtcatatt gagggctcag catcggtctc tgcagctggt ggatggaaga acattcacta    1680

gcggcagtgt ctagatgagc cgtggtaaga ggaagccctt gccgttgggc ttatacagag    1740

cctccagccc tcccaccatg gccactggca tcgtgggtcc atcgcatcct ggggagaagc    1800

tggctgacct ccaccagatg gtcccctctg ttcgctagct tctggcgcgc cccctccaca    1860

ctgcatgctc cccagcggca tgggcgcgaa gacgagcacg gggccccaca ctctccctga    1920

gcactcccgt aggtacctct tcgccaaacc tgtctcttct tccaatcttc ctccacccgg    1980

gtccctgacc agtcagccca g                                              2001

<210>  138
<211>  2001
<212>  DNA
<213>  Homo sapiens

<400>  138
tcctattcct tggaaagttc cagtatctga gggaggaaca gtgacttctg gtcctgacct      60

gagtggagac cgagggactc agaagagctg gaatcagacc cccacacaca ctgagtatga     120

gtcagagaac aaggcctgag agaaaaagtc acagtgccca agactgctgc aggggtcaaa     180

ggggactgct gatcagtgtt ccagggattt gctccagatt aatctgagtc atggaaaaac     240

tgcctttctt ttgtttctaa acaaatacct gcaaagacaa aagaccacat aaatccccag     300

gtgcacaact ttttcagatt taaagaaaaa acaacccccg tctttccctc cattcccagg      360

agaagctcac tctgtggcat caagctgcct gggtgagctc tcttctagaa gagtccaggg     420

ggacaggcaa ggaatgggag gcaggaagtc cagttcaggg agggggattc tgggatgaaa      480

agtgaaggga gagggactga gcccatgctg agggtttttc tctggtttct cagacagctc     540

cggggccaag attcggggag acattgagac agagcgtttg atataaaaga agggggtaag      600

agccaagtcc cagggcccgg agcttggctc tctgggcttc aggccccgag ggcggtgcct     660

gggatgggcc ggctcagctt tggggtttcc cgagctccgc tctctctctt ccacctttcc     720

caacctgtgt caggtccttt tacctggata ctcatgatgc ggcctcagtt ctcacttcca     780

ttgggtgttg ggttcctaga tcagccaatc agtgtcgccg cggttcctgg ttctaaagtc     840

ctcgccggcc caccgggact cagattctcc ccagacgcca aggttgcggg tcatggagtc     900

ccgaaccctc ctcctgctgt tctcgggagc cgtggccctg atccagacct gggcaggtga     960

gtgcggggtc gggagggaaa aagcctctgc gggaaggagc gaggggcccg cccggaggag    1020

taaatctgca catatattta attacagatt acaattacaa tcaaggcaga aatgatctca    1080

tttttacatt acaactctgg aaaaggcaat agactgagat gcaagtgtgc ccccaagtga     1140

tgggcagaag gagagaaggt gttttggatg cattctagaa cacaggtaat ctaaggagag    1200

ttgatcaagg ccgtggaaga gtcctccagc cactattggc catcaaagga gtcctctgtg    1260

tcccaggaat ggtcctgctt tggtgtccct ggtgtgaccc atcacccgct gggaacagcc    1320

tgagagaagt agggcctctg caccaatgct gctgaggatg tcagagcaca ggaacgaggc    1380

cttgggaaat tacctggaaa tgcgactgaa atcttccttc ctgaggggtc tgggctcttg    1440

gaaatcaaac cctctcaggt tgggtggctg gacgattctc ctcacactta caatgggaca    1500

aggggaacca ggaggccccc aaggggatcc ctgggttcca cacgaactcc tcctaccctc    1560

attgtgtgac agcagccatg cctcctcctg gggatcagga tctattacct gtgcctggag    1620

aggaggggac tcctcttctc acccgctggt ctctggacac atactgtcca attcccctgt    1680

ggcagctgta atgtgtagtt caatgggcac tcatttgtcc ccttttaagg gtaccctcct    1740

ttagaatcca ggaccttcta ccctgcagag tgtggttttg ggagagaagt gcaaaatccc    1800

acgacaggtg agttgaagga atgggatatg gagccacatc cacttccacc ccttggtatc 1860

tggacccacg tgttcttcct actgagatta cagaactgta gagatgtctt tgatttttaa 1920

aatgcaccat gtcctgaaag atggcaccct cccacccgca gagtgcttcc tgcaagctgg 1980

cgttgagctg tgcctataga a 2001

<210> 139
<211> 2001
<212> DNA
<213> Homo sapiens

<400> 139
tctctgcctc agctttctcc actgttaatt gggcataatg gacacaacaa tggtgagggg 60

ctcttgcagg aatgaagact aggatgagga tgaggatgag ggatacgtcc ataaagcccc 120

tgcacacctt ccatttcctg agcactccca ggaagtcttt gctgaccacg ccaaattaga 180

ttatctttgc ttagccatta ggccgctgcc ctatattggg aggcaaatat acctctcagc 240

ttcggtggcc aggtcccctg gcacccactt agtcctcagt ctgcacctaa aagcaagcct 300

tccgagggtc ttgccttgca ggtgtagctc ccacccccca cgctggggtg gtccggggac 360

cctctgtttc ctcagtggtg gggaagggac ccgtgggttc gtgggtgctg accgacgaat 420

gtgcggagga gggcgcgccc cagcatgggg ggcgggggtg cttcttagga tggcttggag 480

gggggcgcct gcagggctgg acggcggacg ggggacactg cactttctga tgcggtcggg 540

gaagccggga gtcagcctac tggagtccgc tgccatccct ggccagaagc agccagcgcc 600

accccaagct ccccagcccc tcgcccgtgc cggggccgcc ggggtccgag ttaggggaga 660

gtggggggcg gccaccgctg caccgcgcgg ccttcgaagg actgccctgc ggcgccgcgt 720

tcccccgagg ccaccccctc ccgccgtcaa cactccccgg ggcgggggct cacctgtgtg 780

gtctgggtcg cctccgaggc cgagtccctc gttgccaagc ccagcaggcc tcgctccccc 840

gtgactgcac cgccggcccg ctccagcttc ccgagtaccc cacctgtctc cccggcttcc 900

ttccggccct ttgtgttttc ttctcttgct ccgccccctc gcctgcccgc taagcactta 960

agctggtgaa gtcaccctac tgccacctgg cagtcacttt cgctgttggt ggcaacccag 1020

gctggaaccc agctcttgcc ggaacccctg ccccatcctt gaggcttgtc ccgacaccga 1080

gtaggttcta gttcctttgt gccttccaaa tgactccctt cctccagcaa tcacagccgc 1140

aacagccatg tattgcgccc ccgccctagg ccaggcgtgg gcatcacctt tgcactcctt 1200

tctgcagccg tgtgtggtgc ccactgtgac ctgcacttca cagacgggtc atctgagttg 1260

tgcaggccac taagggagcc tggagtcaac cccaggcctg cttggctccc aggccttctc 1320

tttccattgc tctttgtcgg tcccctgctg cctcctgtcc ctccccaact ctgacttgtg 1380

gagggaagcc tttgacatgt gggggtacct ttaataccga tatcacttgt taccggagag 1440

gggtcccgat ccagacccca agcgagggtt cttggacttc tcgcaagaaa gaattcgggg 1500

tgagtccgta gagtgagagc aagtttatta aggaagtata gaaaacaggc cgggtgcggt 1560

ggctcacgcc cataatccca acactttggg aggcaaaggc aggcagatca cttgagctca 1620

ggagttcaga ccagcctggc caacatggtg aaacactctc tctactaaaa atacgaaaag 1680

ttagccaggc atggtgctct atgcctgtaa tcccagctac tcgggaggct gaggcaggag 1740

aatcgcttga actccggagg cagaggttgc agtgagccga gattgcgcca ctgcactcca 1800

gcttgggcga cagagccaga ctctgtctca aaaaaaaaaa aaacaaaaaa aaaacaaaaa 1860

acaagaagaa caacaacaaa aaagaatggc tactccactg gcagagcagc tgtgtgggct 1920

gcttagctaa ttatactggt agttatttct tgattctatg ctaaacaagg ggtggattat 1980

tcatgaattt tccaggaaaa g 2001

<210> 140
<211> 2001
<212> DNA
<213> Homo sapiens

<400> 140
atcgggttcc cttagagcta agaataaggg acagccagac tctgtggcat cccttctttc 60

tgggccgggg gtatccccag tcctcatgaa gggggcacac cttgggtccg gcagatcctg 120

ggattcctcc ctctgctgac ctgaagtgcc actcctcaga ccacagcccg caaatctgtc 180

accctgaagt cgcaatgagc atggggcaca ttaggccact gtgcccagga tcccccagcc 240

caaggttgcc actgatctgg actccaaggt cccctcagtc ctccctcttc ttcctcccct 300

tgactcctcg cagggctggg ctccaactcc ctgctgaagt gggtctgtat ccctaggatt 360

cccgcggccg aatgaaaagg tggctcagtt tgagacaggg gtactttggg cccctgtcct 420

ggcatgctgg gagccctccg gactccatta tcttccagga ggggcttggg cccatcctct 480

gcttcccaaa ggccgtactc acgataccga cccccttccc tccctgagcc acagatgtga 540

acgtcaggat ccgcgtgctt ggccgccatt cccggagctt ccgtgggttg attgcagggg 600

caaagctccg attctgccag gatgggtggg gatgggaatc ggggtttggg ggtcccgttg 660

atgtttgagc gatggtggtg ggtttggtgt tgggggatgg gggtggggtt ggtgttgggg 720

gatgggaatg gtggtggggg tgcggggggg ttaccctcag tcccctcttg gggtcctgac 780

cttgattcct ggctgagtct gggccctgag cacttctaac cagccctgcc ctggtcacac 840

aaagctctta caccagagtt ccctgcggtt aaagcggcgg ggggtggcgg ggtgaggtgg 900

tggcgactga gcctgagaag tcttcccctg cagggtggtc caggcctggc agcagaggca 960

gcgctggatt attgggggcc ctctgtctgg ggtgaggccc gcctcagtcc tccctcggca 1020

tcttaccttg cctcctcacg gagcctgcgc ctgcttccct ccaccgacgt caggccgttg 1080

ctcagggcga gaatctcgct gttctctgac ctccaacgcg caagtcagtg gcgtcacatc 1140

cggccccgtg acttccctgg gttcacagca ggggtggaac tggattcttc ctggatgggg 1200

atccagatgg aggtggaggt aagcatgaag atggtgttgg gggtaggggt agggataggg 1260

acggggatcc tgaggctgta gatcctggtg gggttggggg agttgggggt gggaggggcc 1320

tttggtcatc cctcaaggtc ctgacttgga tgccaggctg agcctggacc gccatcctct 1380

gccgattgtg tctgctcccc tcagaccaag tctctgactc tgagtccccc tgaagtggca 1440

ctgggggagg ggtgcggtcg gggtgacatt attgcctaag tttatcaggg ctgacaagag 1500

ggactgagct ggattctgtg gtcccactac gtgggggagg gtggaccctc agtcctcact 1560

caagagggtc ctcttcctca ctcctggctg ttggatgaag tgattggcag gagatactct 1620

gtttctgtaa cgtctgtgca tttgcacagc tgcacccctt gtagagaatg gctgcgggtc 1680

ccaggccagg agctccctgc agggcgggag ctcccacgat cgtattgacc tctggaagaa 1740

gacagacact ttcccacggg agctcctctc cagccagagc tacacttggc aaacctttgg 1800

tcctaaatga tttattcact gaattgaaga aatacggttt acatatcttc caagtatata 1860

tgtaggtagc gatatatata tttatttcta cgaactaaca cctaccctta gtatgcatcg 1920

ttcactctta ctatcctaat ctacttctat tccagtcttt gtgttctgtt gaaggaattt 1980

tttttaaac gtaattcctc t 2001

<210> 141
<211> 2001
<212> DNA
<213> Homo sapiens

<400> 141
tgacactgac tttttaaaaa ataaacttac ccttttaatc aaatgtgtga attgtttaaa 60

ggaaaaacat tgtgtaaata atgttttcag ggggtagaat ccagacgtga gttttggaaa 120

cagagtccag tcctgcttct gacatttgaa ttgcttctcc agcatcatca ccagaagatg 180

ggctacctgt gctcacaagc ctcctgtaac acggagctca ccacctctca aaggcacctt 240

ctgtcaggtc tgcctggaaa tgctgctccc gcagactccg cacctgaggc caggtctgca 300

gcgaagctca tccacacctg aggccaggtc tgcagcgaag ctcagccccc ttccttcaca 360

ggagaaatac ttctgggtgt gcaccttcca ctcctggcag ggccgtgagc ccctgtggca 420

gtgcctcacc ctctgggagg gaccctctcc atctgggaac aatgtgggta cttttttctc 480

ttaaagggca gtgtcaggaa tgaaagcaga tacccaaaag cagtgggtct cgtggcctcg 540

tgtctgcttc cagaagtgga gggcggcctg ggacggcggg agccctgagg acaccaccgg 600

gggggtcccg ggacctgata cgagtgggca ggagccccaa gccctggagg aaggaaaacc 660

atttcataaa taactgcatg gggttgtggg ccttttctca caagccgcag cacctccttc 720

gtctctccac ggtgaacccg cccgccacgc tatgccacca ggccaggtcc tgcagcaccg 780

cctccgtggc taggacttcc tcaccagcct ggggccgtga ggtggtcatt tcccccctta 840

cctgacttat ctcaggattc acacacacac acttgacttt cggaaacttc ttttaaatta 900

atttcactga cactaaaagg gaagctttgt ggagtgtgat tgtggttacc tgttaatact 960

tggcaacaat tatgagccct gatatgagcc aacttcttcc gtcttatccc ctttgaggac 1020

gctctgttat cgcccccatt ttgcaaacga ggaaactgag gctcggaggc ctggagcaat 1080

gtgttccagg acagacggca ggaagccggg gtaggaattc caacagggag ttccctctgt 1140

ggaagggaac gtggctggca cctccctgcc ttccaccctc agagctagtg tgaaaaagaa 1200

gacggggtta tggaaaaggc aacgggctgg agattccccg gattcccagg ctagggcgcc 1260

ggtctcactg tgcgcgcagg tggggcgcgt gccagaacag ggcctggggg tcaggccgag 1320

accggcgggc accttcacca gggccgcccg tggcgttccc ccagcccctc tgccgcgggc 1380

cccacgtggc cccgaccctg cggcagcctt tcggaagggc ggagctgaaa aacggccgct 1440

tggctccact cggccccaca gcgcccagga gcgaatcccg agtccgttcg gaaccgcctc 1500

gccgcgtggc ctggggcggc gtcgccaccg cgcgttctcg gcctcccggc tgcggcagcg 1560

atgccggtcc ttggagctgg agcggggagt ccaggaggcg gcagcgctcc ggagagagac 1620

gccctggtcc cccgccgcgc gttcccgcct ctgtgccctg ggggggagga gggggcggtc 1680

gccgctgcca ttggccgtgg ggggtgaggc gggccccgcg caggaaaccc cgcccctccc 1740

tctgcggcgg gggcggcccc agcggatgaa tgaagcggcg cgtggctgcc ggggaggcca 1800

gagcgtggag cgctgcgcgg cgcggcggcc gggccctcga gacggggacg gtgagtgcgc 1860

ggggagcggg gagctggcgc gggtgcacag ggcccgacct gctggcgtag gagcgcggca 1920

ggtgccgcca tcgtgggcag gggctggagc gccgggggga tgcgcgcggg ggcgggaggc 1980

ctgggcaccg gccgagcgcc c 2001

<210> 142
<211> 2001
<212> DNA
<213> Homo sapiens

<400> 142
agaatcactg aatgatccag ctcacggtca tcacaaacga gcagaggtaa cagggcacaa 60

actcttcttt gaactaaaaa atccaaccaa agttagttct gttgtcattc ctgagaacca 120

cggaaaggat cctggctggg ctgtatgggc ctccgcgcat gtgggagagg tcaagctcct 180

ccacccttca acgccaatgt cagctatgtg tccttatgcc accaagaagc ccccttcctc 240

ttccggggcc tgaggttttc ccaactgatg gcgaagctat tcctatatgt gtcatctctg 300

tcctactaga acataactgg cctgtatttt agacaacttg gtaatcccag tgcccagcac 360

agggtttggt acagaataag tgctaatgtt tgatgaatga atgaatgaat gaatggagta 420

gattaacacc aggaaccatg aggctcctga gcagcacctg atgaaagatc ccccacactg 480

aaaacagaat ccagaatcgt tgtctcactt gggcagtatt attcccatca attttttcaa 540

caaatatggc ctggctgtct actacaagcc gggccctgtg aaaagtcctg tggggataca 600

gacacggtct cttgttttca aggaccaccc agttcggcaa gtggaggtaa gtcatggaca 660

aacatggtgg caatgctcag tggcgagcta tccagcacgt cagaagtcca gcccacgtgc 720

tgaggaagtc ccaaagagag caagatcact tctgtctggg tgctgctctt ggaaggcctc 780

atggtcttcc aagaccccca tctgacctct gacccttggc agccaacctt ccccaggcca 840

ccccagagct tgctggccct tcccacttat gcacgccgcc tgggcaggag aggtcactca 900

ctctgtgtca gggtgaccgt ggctgcggct gcggtggccg agggtccgag ggccacaggg 960

tggatctctg tggtgctggg caggctgatg atggtggcct cgcccagcag gttgcagata 1020

cgttgttgca tgggggtcag cagcactggg gctacagctg ggccaccgcc accactgcca 1080

ccgcctgtcc caggcccccc agctccgtcc tcctcagtgg gccccggcgc ctcaccaccc 1140

tccacggcgg cccggacctg ggcaaccttg cgacggacct cggtcttgag gtcagaccac 1200

ttcttcttga cctcaggcag ctctctgcgg caggtggcca cggcgttgac ccttctcagg 1260

atgccgtgcc aggccgcact cttggcggcc aggggtaccc cggcgttgaa gtggttcacc 1320

agcaggtgct tcttcagctc cagctcctcc acgatgatct ccacctcccg ctctgagaag 1380

ttcatcttcc ttttcttggc tgggacggcc atggcctctc ccctcccctc tttttaaaga 1440

aattataatc ccctcaagcg ccccaattcc ccttctcttc ttcctcagcc tcgcccctca 1500

cccaccccct acaggggata ggccaggctt gctcgaggcc aagcactagg cctttggtaa 1560

cccccctcgc tacgcaaagt gcgtagggcc cagccccgac cggcccggcc gctgccagcc 1620

agcagcgtaa tggcttcctg aatctgcctc ttccgccgag gtgtgcggag atccgcccac 1680

ttcccctctt cccgcctccc agcgcttctc cgggaaaacc ggccttcccg gttggtagct 1740

gctcaaatat cacgtcgatc actggttctt tcgatctgtc gctcagctaa agagcacgcc 1800

tgcttactgt aattcgcctg atcattggtc taaaattgtg tccgtcgccc agagggcccc 1860

gcccacctaa cctttattca cttccattgg aaaatcattc tgtcgttcaa ttgtcagtat 1920

tacgactatt aagccgttaa attcagctat tttattggtg gtttagggag ttagattacc 1980

aaaaccctgc ttcctgagga t 2001

<210> 143
<211> 2001
<212> DNA
<213> Homo sapiens

<400> 143
gaatgaaaag gtggctcagt ttgagacagg ggtactttgg gcccctgtcc tggcatgctg 60

ggagccctcc ggactccatt atcttccagg aggggcttgg gcccatcctc tgcttcccaa 120

aggccgtact cacgataccg acccccttcc ctccctgagc cacagatgtg aacgtcagga 180

tccgcgtgct tggccgccat tcccggagct tccgtgggtt gattgcaggg gcaaagctcc 240

gattctgcca ggatgggtgg ggatgggaat cggggtttgg gggtcccgtt gatgtttgag 300

cgatggtggt gggtttggtg ttgggggatg ggggtggggt tggtgttggg ggatgggaat 360

ggtggtgggg gtgcgggggg gttaccctca gtcccctctt ggggtcctga ccttgattcc 420

tggctgagtc tgggccctga gcacttctaa ccagccctgc cctggtcaca caaagctctt 480

acaccagagt tccctgcggt taaagcggcg gggggtggcg gggtgaggtg gtggcgactg 540

agcctgagaa gtcttcccct gcagggtggt ccaggcctgg cagcagaggc agcgctggat 600

tattgggggc cctctgtctg ggtgaggccc cgcctcagtc ctccctcggc atcttacctt 660

gcctcctcac ggagcctgcg cctgcttccc tccaccgacg tcaggccgtt gctcagggcg 720

agaatctcgc tgttctctga cctccaacgc gcaagtcagt ggcgtcacat ccggccccgt 780

gacttccctg ggttcacagc aggggtggaa ctggattctt cctggatggg gatccagatg 840

gaggtggagg taagcatgaa gatggtgttg gggggtaggg tagggatagg gacggggatc 900

ctgaggctgt agatcctggt ggggttgggg gagttggggg tgggaggggc ctttggtcat 960

ccctcaaggt cctgacttgg atgccaggct gagcctggac cgccatcctc tgccgattgt 1020

gtctgctccc ctcagaccaa gtctctgact ctgagtcccc ctgaagtggc actgggggag 1080

gggtgcggtc ggggtgacat tattgcctaa gtttatcagg gctgacaaga gggactgagc 1140

tggattctgt ggtcccacta cgtgggggag ggtggaccct cagtcctcac tcaagagggt 1200

cctcttcctc actcctggct gttggatgaa gtgattggca ggagatactc tgtttctgta 1260

acgtctgtgc atttgcacag ctgcacccct tgtagagaat ggctgcgggt cccaggccag 1320

gagctccctg cagggcggga gctcccacga tcgtattgac ctctggaaga agacagacac 1380

tttcccacgg gagctcctct ccagccagag ctacacttgg caaacctttg gtcctaaatg 1440

atttattcac tgaattgaag aaatacggtt tacatatctt ccaagtatat atgtaggtag 1500

cgatatatat atttatttct acgaactaac acctaccctt agtatgcatc gttcactctt 1560

actatcctaa tctacttcta ttccagtctt tgtgttctgt tgaaggaatt tttttttaaa 1620

cgtaattcct ctcccactat gttggtttca ggttgttatt agcagaactc tggagccatg 1680

ctgccttaac aaacccaatt ttcctggtgt cctgctatta cctagggctc ttggcatcgt 1740

gttttcagga gctgcctcag attgtgcaat gaagtgaagt ggaaatttta aaacttttat 1800

tctacccatt ctgttatctg ccgtctaggc aggctacaaa cctccccaac atccctgccg 1860

ctaaccccct ctaaccgaca cggcaagtgt cacacacaag taccagctca acaaggtctt 1920

cagtctcacc ctggactggc cctgttttgt tgtgtagatt ttcttgctaa tactggtttt 1980

ttactttctg tttccattct a 2001

<210> 144
<211> 2001
<212> DNA
<213> Homo sapiens

<400> 144
ggtccccggc gctgcgtcct cccgacccgc tccccgcggc gcccgggtcg cgccttgcgg 60

cagtcaccga gctgagggca gggcgtccca gcgtccctgg ccccggcccc gctccgcagc 120

gcgccgcacc ccgaggctcg ggccccgcac agctgcagcc ggtgccctcc ggccaagccc 180

ccgcctggcg tccccggccc cagccccggc cgcccgccgc tgaccttttc ctgcgcgcgg 240

ttgagtcgct tctggacgtt cttggcgaag atgcccgtct tgatgtcggc catggctgcg 300

ggtccgggga gctgcgaaga gcagagcgcg cagcggggct ggcggcggcg cggaggagcg 360

ggaggaggag cgggaggagg aggagagggc cgagcaaggg aggagcggga gaagcggcgg 420

ggagccgcgg agcgcggagg ggtgagggcg gggcgcggcg gcgggaccag cttaaagcga 480

cagagcgggg gcgggatgcg tcgggactgg ctgtgacatg gggagaaggg gcgggggaag 540

aaagagaaag aggccaagaa aaagagaggg aaaagccaga agccagattg tctcctcgat 600

gatagaagag tttcgagagg agaaaaggaa ccaagcctag cagaagtccc tgggcccagg 660

ggcagctcgg gggttctttt aatgcagttt gtatttgggg agagtgaggg gacctgattc 720

tttccagcaa aaaaagaaat aggggagaca aaaaagggaa gaaccctagg gatgtggaag 780

ccagctggat tggaactgct ctgaagtgga gctgatgggg ctggtccctt gctgctccca 840

aaagtttcca gctaacagag gcccctccat acaccctggg tgttgcagaa gaaggtatgg 900

aggtcgccct accctcagga ggctcacagc ccagtgaggg gccctggctg ggaaaccaac 960

gaagtcagag ctgcaggaga gaggcgtgtg gagagcaaag cggggagaga ccatcgctga 1020

cacttgttta aactttaaaa caagagttag cagggcgggg aggtccgcgt tgagaaaggt 1080

cagcttccgg aagacaggcg atgtgttttg gtcatctcca aaattcctgt gctgggcaga 1140

aaccttagaa tagactactc ttcaagaaat ggtgcatgga acagtcaagt cccttatagc 1200

ttcgcatcaa ccatggaaga cccagtcccc cctaacttgt tgcttccttg gaaacctttt 1260

ggcaacctta acagggactc accatcccta ttattcttat taatgcttgg gagaagtacg 1320

aataataggg ataaacctgc cctacaccta gagctcttgt tggctctgac cagtggtcaa 1380

actattcata ttccattctc actgagagtg tgaggagaag aagtcaggtg gtgccctgca 1440

aacagaggtc agaagtggtc actcacagtg aatcctatga gtccaggtga gcatcctact 1500

gtacctctgg gggaaactgt tacattggta gacctcagcg agctgccacc tggtgttcac 1560

tcttgtgtca ccctgtagtc ccctcacctt gattctagaa tgatgctatg gccagcttta 1620

atcaataaga tgtggtgaaa gtgacgccat ggtaattcca gtcccccccc cacccaccca 1680

acctagaaac caggcaagtt ttgccctctg gggattcctg tgacaccata taggaaattc 1740

agctaattca gctaccctgc tgcaaaaata aaataaaata aaataacaag gagaggccat 1800

acagaaaggg agaggccctg agattacgca gagagagaga gagagagaga gagcagccaa 1860

gatgtcccag ctgagcccat tctccaatca gttcactggc tggctgcagc cacacaagca 1920

accatcagca agaccagcag aagaaccact cagctaatcc cagccagagt gcagaatgtg 1980

agcaaacaaa acagttttta t 2001

<210> 145
<211> 2001
<212> DNA
<213> Homo sapiens

<400> 145
ttccccagaa gcacgcactc agggacagcc ttgggttccc tcccaacctt agccacagcc 60

ccagattcct ggagatggcc ccgcactcac tcttcttgca ggaggtgcat ttgcactctt 120

tgcacttgca ggagctggcg caggtgcagg agacacctgc tagaagagaa aaagccagtg 180

aacggtgagt gagatgcaga aggtacagca gtgggtcaat gagtgtccac ttcccccttc 240

tctgtgcaga gccagccctc agagctcctt tcccactcag agcagaggaa gagaagcccc 300

atgtcctctc cattcatgct gggaaaacca agcaccctcc tatttctact ccaaaggaaa 360

gtcccaggct gcagacatgg cccaggaaga cttcgacttt ctgtcctgaa cctggaagag 420

gcaatgtccc ctcctatcca atgtctccta tccaatccgg tagctcagag tcaggttaga 480

gaattggggc tcagccagca gccctgtgac caaccaggtg acgtggagca ggacagcctt 540

gagcctcagg accctcaact ctgacataga ggaacaggga ggaggaaaat gccctcagga 600

gcttggccaa caggaaagca ctggaccaat aaaggagtcc cctttacctc tgatagcaaa 660

atacgacctc ctcaaaccca gggacactct ctatggtgtc tgggaatttg gcatcccaag 720

gcacagaacc cgggcgtccc ttaccagcgg cacaggagca gttggggtcc attgcaaccc 780

gaggcgagac tagagttccc aagcgagaag ggaagaggca gtgggtgcac gtggaaggcg 840

gagtggagcc caacagccag cggctgtttt tatagtcagg agacggggcc gggcgcagac 900

gccccgcccc gcccttgcac ccagcccgct gagtccgcac cgcccgcggt cccggcctgg 960

gctgtgcgca ggagatgggc caagtgcaag gtcccttgag cgcagctggg cgcacaccgc 1020

aggacggccc ctttcgcacc ggctcgcgag ggaggcgctg tgccccccgt gtgcggcttc 1080

tctcaccctg ccaggccttc ccagcttccc tgaggttgcc tgctacaccc gccccttggc 1140

gttccgccta ccccagtatt ctggctttcc cctcaccttc accaacgccc aggtctctag 1200

tcaatagcta agcctttcct ggagtcgagg gcaaccccag tcttgtgctc cactttctgg 1260

atctgccaaa gcggggagca tagcaaggga gtagtgtgca aagggcaggc caagggtgcg 1320

aacttcgcat ctctggcagt atccgccctg tccccgttcc cacctcagtc ctttggtcac 1380

gcttctcctg tgctcagtaa cacggaagaa acagtgatct gcctctctct tctattctcc 1440

actcttgagg agctgaatgg gcccatttgt ccaggtcctc ctttgcataa atctctccag 1500

ggtctcccca cagcctagcc gggtacccca ctacaggatg gcagtttgga aagtgtggca 1560

agtccctaga agagggatca ttgaaggtga tatgaagatc ctgcattaaa ttagaaagca 1620

agagcgctgg ttatttttcc ttccatattc ctattactct atgttacacg ggtctgtgtt 1680

tggccctgtt taatcacttt ctgtctttga accctcacag caaccctaaa gggacatgag 1740

tccttttcac gaacccgtat tcctaatctg gacaatgacg aggagttcaa gccactagcc 1800

cagggtcagg gagctgggaa gtggcactgc ttggtgtgaa tcctgtcagc ttccctaggt 1860

agagaggtca gtgatgaagc ttctctatgg cctaggtatg tttatctctt cactgcttga 1920

aatctttcaa caaaaccaag cattacagtc agtcctggaa gcaaaacaga gacatagagg 1980

gatatgaata agtatacttt g 2001

<210> 146
<211> 2001
<212> DNA
<213> Homo sapiens

<400> 146
ggggtccccg gcgggcgggg ccggagtcac ctggcagctc gggactcagg cgctcccggg 60

gtcacacggg gccgcgcaca tcagccccgc cgacgggcac gggcggctca catcgatccc 120

ctcgcggcca cgctcacgcc ctgagttgtt gggatcgcgg gcagaagtca tcccgacagc 180

cacccgtgcg acgacacagt ggcccctcac tcgcgtcccc ctgacagcct cgcagtcatt 240

cacaagccgt cacagtgacc cacagccaca cgcaggcggc caggcactgc ttacacggtc 300

ccgccgtccc acgcaagtca ccctgacagt cacttataca caacgacacg caaccactca 360

cccgcaaaca gcctcaccgc cacagacgag tcacccacag acgctccgat gccccacaca 420

ccgtgacatg cccccacccg ctgtcacacc caggcacaac gggggacagt cacacaaaga 480

agtcgcccga cagtcacact ctcgcagcca caacgcctca cccaggcgca cgctgtcaca 540

cacagccacc cccctggacg gtgagcctca gacacagatc gtcctgacgg ccacacagtc 600

acacgcggtc acacccaggg acggccacac ctcggtcatc cacactcgcg cgtcgaggtt 660

taacttctca gcgccgcagc acactaattg ggcagtttac taattggccg tcactgcctg 720

gcggggcctc ggattccgag taggctgcgg ccaatcgagg cctgtgtcct cccgggagcg 780

gtgagccggc gtccctccag gctggggctg tagtgccgga caccctccct cctccatgtt 840

tccaggatct atccccccac cccagccagg tcccagctgc gctgcgccgg gcgcattccc 900

agtgatggag gcttgcgtgc ctggaacccc gaacccgagg cgtcaggtcc cactgccggg 960

tctggcccag cctggatggg ttgggtgcct ctaagcagtc cgcgtcccct tcggtcgccg 1020

gagagggaac tttcaaactt agttaggaag ccagactgtc cgcgcgtccg ccagtcggtg 1080

cgtcggtccc gggccggctg gagccaagcg cgggttttcg tcactcggag cccggattga 1140

acagcgcgcg tgggtttccc gcagccctgg cgcagacgcg tgggctccgt ggcgatgcgg 1200

ggtgagtgcg cgtccagtgg ctggatcggc gccccccagg gtctctcccc aacctcgcag 1260

gcttttcgtc aggcccctgg tagtggggat gaggcaggag agaggcgtcc cggcgcggat 1320

cccggggcct cccgggctgg gcctccctga gtgcgccctc aggccccagg ggagcggctt 1380

ctggcaaagc ctccctgcag ttcgccaggg gcagcaagca tccaggccac tagggcctgc 1440

gcgatgtggg gcggtggtgg gcgacaagtg aggtctcgcg aagagtggcg gtggttctct 1500

tttgtccagt gacctgcaaa gttagaagcc gatgaggggg gatggtagcc tcaggagaag 1560

gccgggagct gcctccagtc tgatggagga aaatagatta aaagtagcaa gtggagtttt 1620

cagggaggag aaggttgggt gtgggggcag ctggtctgtc ttctgccctt ggctttgtta 1680

tttttcaaaa tgtgaagttg gcgaggtagg taccagcctc tggtttgcgg gtgtgggtgg 1740

gctgggcatt tgtgcctcaa agtctgagtg ggtgggggtt tagcctggag cgctccgttt 1800

gagggtggtg tttctataaa tctcgccagg gtggggctgt cccaaatagg ggcctttgga 1860

ggcaggactc agccgaggcc cctgctttat tctgatgctc tgtaaacctc tgactcagca 1920

ccagaacctg tgcgaatctc gccgggaagg gcccgtcagg gagggaatga gaagccggcg 1980

attctggttt ctttctgtgt g 2001

<210> 147
<211> 2001
<212> DNA
<213> Homo sapiens

<400> 147
accccatcaa aaattgggtg aaggatatga acagacacta aaaagaagac atttatgcag 60

ccaagaaaca tatgaaaaaa cgctcatcat cactggtcat tagagaaatg caaatcaaaa 120

ccacaatgag gtaccatctc atgccagtta gaatggtgat cgttaaaaag tcaggaagca 180

acagatgctg gagaggatgt ggagaaatag gaacgctttt acactgttgg tgggagtgta 240

aattagttca accattgtgg aagacagtgt ggtgattcct caaggatcta gaaccagaaa 300

taccatttga cccagcaatt tcattactgg gtatatactc aaaaatcatt ctactataaa 360

gacacatgca cacgtatgtt tactgcagca ctagtcacaa tagcaaagac ttggaaccaa 420

cccaaatgcc catcaatggt agactggata aagaaaatgt ggcacatata caccatggaa 480

tactatgcag ccataaaaaa gatgagttca tgtcctttgc agggacatgg atgaagctgg 540

aaaccatcat tctcagcaaa ctaacacaag aacagaaaac caaacaccgc atgttctcat 600

tcataggtgg gagttgaaca atgagaacac atggacacag ggaggggaac atcacacacc 660

aaggactatc tggggatccg gggccagggg agggatagca tttggagaaa tacctaatgt 720

agatgatggg ttgatgggtg cagcaaacca ccatggcacg tgtataccta tgtaacaaac 780

ctgcatgttc tgcacatgta ccccagaact taagtaatta aaagaaaaag gaaaatcaag 840

tgattttgct tttgaaaatt cctgctaatg tttttcatgc aacatctctt tcgtgtgtgg 900

ctcaggggta gaacattgca tcaggatttg ctcatgggtc tcatgtctct tcctcctccc 960

ccagcaaatg acggctgctc ggaaactttt ataaaggaag cggttgcttt gtggctgata 1020

gatctgaagg tgaagagctt ctggccaaaa tgattcagca gcaaaacact cccctcccca 1080

gcaccacgca ggcagccccc tagccccgcc cctgccccat cccacagtgg tttgcagggt 1140

ggggttccat gctgtggccc tggcacctgc ccctgggctg ggacagccca ctgttccatg 1200

ctgcccaaga aggctcagca caggcacaaa ccattgcccg gcactggccc gtgctgcctg 1260

agaaggattg gcacgggcac agaccactgc ccccacctgc cctgcgccat ctacccaaga 1320

aggctcggca cgggcaccaa ccactgcctc caactgcccc atgctgcctg agaaggcact 1380

gcacggccac ccccaactgc cccgcactgt ccctacccgg gcagccatgc gagcggctgg 1440

aactctgctg gccttctgct gcctggtctt gagcaccact gggggccctt ccccaggtaa 1500

gtggcgttct cccctgtccg ctccccgggg gtctctccct gagattggcc actggtgcct 1560

tgggtcttcc ccagggcact ttcctagctt ggagccccca caggaccatg cggtggtgag 1620

aggtgctggg agtggtgaga ggggcaaggg gctccatctg gctgcagcta cctttggggg 1680

tagcaggcta cagggatgaa gggtttgggg gctgccccag aaggctaggg gcaagggaag 1740

catcttctat tctctggctc ttcacagagg tctgagaaag gtctaagtaa ggcaggtctc 1800

ccgcagctcc tctgcgggac ggaggcgaca gggcagccac atgcctgtgg ttagccgctt 1860

ctatctgatg gcttgtcatt ccttcctgct ggctgttgtc ctgtgacttg gctccaggga 1920

ccctaacgat cagactcttt cacccttcct gaaatccact ccctgagggt cagcactcag 1980

ggggaggggg gacagggagg a 2001

<210> 148
<211> 2001
<212> DNA
<213> Homo sapiens

<400> 148

agtgatgaaa aaaccacagg ctggtgatgc aggtcagagg aggagatgct ctttcacatt 60
cggacttgag agagcccgag agtacccata ggcatgggtc aagagagcta cagtggagct 120
cttgggggac agaaaggagg atgtagaaaa gaaaggggca gagactgtgc ctctagccac 180
ccatggatac ataaggcatt gaatccatta gtgaagagaa ataagtaatg aagcatctgg 240
ggccccgtgt ggctcctatt ataatgaatc atgtatgttc tcatattagt tattaagtac 300
acctggcttg gtatatccga tggcctattt tgattaattt atctaattct tttttttttt 360
tatttcctag gccttgggtt aaaagactca gctatcaacc tggaggataa ctgattacct 420
agtcagagtt ctggtacctg gagacaaact aactttgatt aaagcatcag atcaccaggt 480
cttaaacact gggcaccaaa ccggtcctgg gacaggggct aaaccttgtc agttatgatc 540
agggagcagt cctagaagaa aatagcaccc aaaggagaac ttgctctttt gggaatgaga 600
aagagctaat gttttgcact atggattcag tcaagaagag aaatgctttt ggggctttct 660
taaaaggcca aatgtaaaaa gtacccccat catttcctaa aactcaaacc tctaggactc 720
tacctcttgg gctaattgct catgagccac caatctgttc tgtcttgcac tgtgtgttct 780
gagagccagc aggttaggac agagggaaca tgacggggtc agaggagggg ctcacctgac 840
tgcacagctc catgttttct gagtacacag caatctgtcg tctcggctgc tgctcctctg 900
acagatagct ggagaacagg atgaggacat caaaaccaaa cttgtctgta aatgctttca 960
agtcactggt aatattgctg tgaaatagac aattctgaaa cgacaacagg aaatttgata 1020
ttaatgtcaa actgcccagc tgcggggagt tgttccacaa atttgtccag tcttatgatg 1080
tatttaggag agatatttat tatttaaggc aagtgaaata cagtttgctt tgagaaggag 1140
tggaaacttt catgctatgg cttaaaatac actttgcttg tttgttttcc caaaccatgt 1200
aagaaaggag tttttgccat tattaataat agtttagaca aagaacagat atcctaaaca 1260
tttttttcta aaatatttat tcagatgctt aatttgcaaa tcctattttc cctcttgtcg 1320
cttataggat ctatatacga atgtctgcta ttgttacaat ttatcacaat aagaatagga 1380
ggaagttatt tcttcctgta ttttttttct aatgattatt attaactcca aggagaaatt 1440
cctacgaatc aacatagctg agtttactgg gtgatcttta ttccagtgta gaattctggg 1500
agatttttaa aagagctgtg atgtcagatt tgcttgttct atgtggcagc tgtggaggaa 1560
tgaataattg cagaaaggga tcccaatacc tcagattacc cagatgtggc tcatatcatt 1620
tgcaaaaatg ttctggctgc ttcaagtttt atttactgta tgtatgtact tatttattta 1680
tttttgagac agggtctcac actgtcaccc aggctggagt gctgtggcgt tatctcggct 1740
cactgcaacc tatgcctcct gggctcaagc aatcctccca gttcagcctc ccaagtagct 1800
ggggctacag gtgtgtatca ccatgcccag ctaatttatt gtattttttt gtaggcgtca 1860
ggttctgcca ttttgcccag gctggtctcg aactcctggg ctcaactgat ccacccactt 1920
catccttgca aagtgctggg cttacaggcg taagaccgca cccggtccgg tctttgagtt 1980
ttattaacta caatgagagc c 2001

<210> 149
<211> 2001
<212> DNA
<213> Homo sapiens

<400> 149
acctatggct tgcctggttt acactttctg tttcaggtca gccagaaaga cgtccatcct 60

acttcctgag gtttttcgtg taatgagaca ctcacgtgta cccttttcca cgagcgtttt 120

attcccactc tccaaagcga ggtcttcctc ccctctaggt ggtgacctac aaagtgacct 180

aaaatcctgt cagttcctat ctttgaatac tggagtttct ttgatccaaa atacatttgt 240

acccagtgct caaatggctc atcgaaacat cagtttgaat aaatcatgaa ccaaattgta 300

tccccgacat ccagggccat gggtgacaga gccttgttgt cttggagcat aagggaactc 360

aggccagtgc ctgacactgg catcataaaa gattccattg tattccagag agaaacgctg 420

gcctccccca atttggaggc tccttcttct ccagtcccat ggatgctctg cagcctgcac 480

gttccaccct gacccctctg cccccagctg atgtttgcct gggccacatt ttttcagcgc 540

tagcatcacc caatctttct cagtgctgtg gactggccct agctccctct tctgcaaccc 600

tgaagcagaa gcggcacctg agaacacctc ttgggctgct tacctggatc tgcagcttgg 660

tgtctgagag agatggaagg ccagtttctg caatcactca gctcccgggg tgtggacctt 720

atacagatta caaaggggtt tggcttccaa gaggaaaagg gtgggaggag catctggagc 780

tcagttcaaa taaaatcaag gacgtgagga aggagtggtg aatcacccag gccgccgtct 840

ttctagggct gctgaagaaa gagcaaatct ttgcctacgt tagtctttct tcctatgtga 900

accacatctg tctgctgatg cacaaggagc agcagagaaa gccactacag cactggtgcc 960

caaacctggc acactaggaa caaagtcctt gttacttctt cgtgggcgtt ttcaccaagt 1020

agacttggag gttaacaaag gacgcaaagg agaggttcta atgggagata gggaattctg 1080

gccaaagcta ttccagggct cctagggagc aaaacccaag tctctttcta ggagggcatt 1140

ctcccaacgc tacctctccc ggggatggga ggcggggtcg gggggaatgg aggctgtcct 1200

gctgccccca gctggaaggc ttcccagatc tggagaagaa agggggagta actgaatagg 1260

gaggaggagg aggcctctag ggtgcgctca cccagttctg gccacttccc acccaccctc 1320

cacccctgct ctgggggcat cgagaggatg gggctgttct tatctacagt aaccactgac 1380

cctgtaggtc ggaattgagt agaggaacta aagataaact taaatcccaa atctcaaatt 1440

ctttttgggg aaaaaataca aagaatatca tatttattag cagcagaatg ccctcctaga 1500

aagagacctg tttttttgct gagtaacaat ttggatacct ctaagaatta tcaaaatatc 1560

ccttcgtgct gtgtattcag attgtttcag cctttctgtg taaaaagtgg ccatagtgaa 1620

attatctgac ctaccttgtg tgattgtagg tcgtaagatc ctcattccag agagggtcct 1680

gccccccata cccagaaaaa aaggacgaat gctcagagag gccaagaaga atctagacag 1740

ggaggccttg ctgggtttcc ccactcagtc tattttcatt agatcgccct gttgtccagt 1800

catatttcta cacagctgtt catactttgt tgatatccaa gtgtaagaat gcccactttc 1860

ccctgttatc tttgggtctt ctgaaggctc ccgtgtcgtg taaaactacg atcaaatcaa 1920

tttgtatgcg ttttttccta tcaatctgtc ttttgtcagt tgattttcag caaacctttg 1980

gagggtgaaa ggggaatttt t 2001

<210> 150
<211> 2001
<212> DNA
<213> Homo sapiens

<400> 150
ggactgtgga ttctttctct ggggcaaata aaaatgtaga atcagaaaat tcaggcactt 60

tgcactcctc atgggacact ccagcagcac tcacgtgacc atcctgagaa tggacaggac 120

acctgaggtg gggaagggag cacagaaccc agacaccagc ctggacacag gcacctggga 180

taatctccta ttccttggaa agttccagta tctgagggag gaacagtgac ttctggtcct 240

gacctgagtg gagaccgagg gactcagaag agctggaatc agacccccac acacactgag 300

tatgagtcag agaacaaggc ctgagagaaa aagtcacagt gcccaagact gctgcagggg 360

tcaaagggga ctgctgatca gtgttccagg gatttgctcc agattaatct gagtcatgga 420

aaaactgcct ttcttttgtt tctaaacaaa tacctgcaaa gacaaaagac cacataaatc 480

cccaggtgca caactttttc agatttaaag aaaaaacaac ccccgtcttt ccctccattc 540

ccaggagaag ctcactctgt ggcatcaagc tgcctgggtg agctctcttc tagaagagtc 600

cagggggaca ggcaaggaat gggaggcagg aagtccagtt cagggagggg gattctggga 660

tgaaaagtga agggagaggg actgagccca tgctgagggt ttttctctgg tttctcagac 720

agctccgggg ccaagattcg gggagacatt gagacagagc gtttgatata aaagaagggg 780

gtaagagcca agtcccaggg cccggagctt ggctctctgg gcttcaggcc ccgagggcgg 840

tgcctgggat gggccggctc agctttgggg tttcccgagc tccgctctct ctcttccacc 900

tttcccaacc tgtgtcaggt ccttttacct ggatactcat gatgcggcct cagttctcac 960

ttccattggg tgttgggttc ctagatcagc caatcagtgt cgccgcggtt cctggttcta 1020

aagtcctcgc cggcccaccg ggactcagat tctccccaga cgccaaggtt gcgggtcatg 1080

gagtcccgaa ccctcctcct gctgttctcg ggagccgtgg ccctgatcca gacctgggca 1140

ggtgagtgcg gggtcgggag ggaaaaagcc tctgcgggaa ggagcgaggg gcccgcccgg 1200

aggagtaaat ctgcacatat atttaattac agattacaat tacaatcaag gcagaaatga 1260

tctcattttt acattacaac tctggaaaag gcaatagact gagatgcaag tgtgccccca 1320

agtgatgggc agaaggagag aaggtgtttt ggatgcattc tagaacacag gtaatctaag 1380

gagagttgat caaggccgtg gaagagtcct ccagccacta ttggccatca aaggagtcct 1440

ctgtgtccca ggaatggtcc tgctttggtg tccctggtgt gacccatcac ccgctgggaa 1500

cagcctgaga gaagtagggc ctctgcacca atgctgctga ggatgtcaga gcacaggaac 1560

gaggccttgg gaaattacct ggaaatgcga ctgaaatctt ccttcctgag gggtctgggc 1620

tcttggaaat caaaccctct caggttgggt ggctggacga ttctcctcac acttacaatg 1680

ggacaagggg aaccaggagg cccccaaggg gatccctggg ttccacacga actcctccta 1740

ccctcattgt gtgacagcag ccatgcctcc tcctggggat caggatctat tacctgtgcc 1800

tggagaggag gggactcctc ttctcacccg ctggtctctg gacacatact gtccaattcc 1860

cctgtggcag ctgtaatgtg tagttcaatg ggcactcatt tgtccccttt taagggtacc 1920

ctcctttaga atccaggacc ttctaccctg cagagtgtgg ttttgggaga gaagtgcaaa 1980

atcccacgac aggtgagttg a 2001

<210> 151
<211> 2001
<212> DNA
<213> Homo sapiens

<400> 151
accattcatc tgcctccaga ctttgctagt cctcagagac actgctgcga gggtagtggg 60

cagtgggatt agcctcccgc agagctctgg cctcctccag agcttctctg tatcgggagg 120

gccaggcctg ggggtctttc ttaaagaccc tggccaggaa ctccatgatt tgcatcttgg 180

tgatttcgcg gctggcccgg gagccccaaa agaactcgta ttcgggcggc tccacgtatg 240

ggacgcgctg gtacttcagg taattcattt ggacgaactc ctcagtgatg agcttccgca 300

cgtccccgaa ggtggagtgc ttcttccagg gccgcagccc caggatgcgc agcacgttcc 360

agacggcgct ctctctggcg ccgcggccct tcacgtagat gaggctcagg atcatgagca 420

ggaggcctgt catgggcatg cggttgctca gcgccaccct gtccagctcc tcgggctcca 480

gcgctttgac cagcgcaaac tccatggtgt gcaggctggt tagcctcagg tgcagcccga 540

acacccgggc gaggatgagg ctggtgcgcc ggaggatgct cctgcaccac ttcttgtagc 600

tgccgatgac atctttcacc atgtctggaa accagatgat catcttcttc tggtccttga 660

ccagcacgta ccacatgagc tcgtgcgcct tctgcaccag ctgcgccggg gctggcggtg 720

ccgggcccgg ctgggccgcg ctcggggcct ggtgggcgcg gccctcctcc gcagcctgct 780

gcagggcctt cgggtcgccc tcgtcgttcg gggcctgggg aggcggcgcg gcctgcggag 840

cggccgtcgg gcctagagga gggctctgcg gctctgccag ggtcgcggac ggaggaaccc 900

cctccgaaac cccagggctg ctgtgcacct cggagttggg ggcctcggct gcaaagttag 960

ggtcgctcag atccttactt tgttctgaca tgtctgcgcc gtctggcaag ggcagggcct 1020

ctgcgtccag gagctcttcg agcctgcgct ccctccgcgg attcctggag aggaagtgcg 1080

cgttgctgcg cgcggcgcct tccgcagcga cgggcagggc ggggcggcgg cgggacgcgg 1140

cactgcgcct gcgcgaccgc ggccttggcc agcggctggg ggcgggcggg gcagcgcggg 1200

gccccgacgg ggagggtcca tggcgaggct tcacctgctg ggagagcgat cccggggttt 1260

cctgtttggg ctgagaagat ctagggcagg agagaagtga gatggatgcg tactggagaa 1320

ggggccagtt taagggaaag ataccttaag tttttgggat gttggggtca agtttcttgg 1380

gaaacgctta cctgggtata tccagaatta agttaaatat gagagtccca gagaaaggtc 1440

tgggcggctt tgtaaacatg agctttatgc gaaccagtga gaggaacagt cgggcttgtc 1500

caagaaaatg tgtaaactgt aaagtggatg tctgaggttg gaatcctgag aaattccaat 1560

aaaaaatgat cgactcccta ccaaatcaga taggctataa gaaaagggga aatgcagtgt 1620

gtcgttaacg taatttccta tcaacccatg tgaactatga actttttaag taggcagtgg 1680

acttagagca tcattaatgc tttaacgggc ataaatttgg gttttacact tcctttgggt 1740

aactttgatt ccattttatt tttttccata cttagtccat agcagaagga ttgctatatc 1800

ttttagcaca aacttgtatg ataatgtctt ttccctctgc ccagcaccct cattcttcaa 1860

ttcaccttct ctcaggaccc tctctgaagc tactgagtcc tccacgttgg aagccacacc 1920

caagtctttc ctgagattta aaaaaaaaat aggtcccatg ggtaatatct ggtagtgtct 1980

ctgagatgac caaagggtag c 2001

<210> 152
<211> 2001
<212> DNA
<213> Homo sapiens

<400> 152
ctgggatctg acaactgaga agggtctcag cccagcctgc ccctcccctg ccgaccccac 60

aatcaccctc ccatccttta gcctcctcct tccctctctg tctctcaatg tccccttgcc 120

tcttctgctc ctcacccaca tcccccacac atcctcccca acccggtagg ctgaggggag 180

gggagagaaa gtggaggggg tgctagaagg cacatactac aagcaagaca gccactcagc 240

ttatctcaag cgaccccaca gcatccccag ggcataggcc tcctcccaga tgaataagct 300

gaggttcaga gaggtgctgt gacttgccca aggccacaca gcggcaccaa gccagtgacg 360

acagcccagg tcagcctggg gacaaaggct gaatttcagg tttcccactt ccctcctgca 420

tccacagagt aaagactgag gcttgcctcc gtgggagacc tgcccccagc ttttggggtg 480

caggtgagga gccctgggca ctctcaccca tagtcctgca ccctccaccg agatggcttc 540

tggggtgggg tggggctgaa gagggtgaag ctcccctcag cccacccccg ggtgctccct 600

gtgggccctc ggagaaggaa gcgtcccctc caagctgcct tccagggacg cctagttctg 660

cagggccagg ggcttcccca ggggcaccag ggcggaagga tctcagggca tcccagttgg 720

gggctgtgag gaagtccagc caggtaccta cccatctccc accaccacac acttgatggc 780

ctgcatcgtg tccggagcct ggagagtgtc ggtggtgaca gctcagggcc aggcgcgttt 840

ctgcgggcgc aaggggtgtg gaggctggtg aggcgcctgc tgaggagcag cggtggtggg 900

gcaggaggaa acggaagggg aacttactca agctgctctc cccacggtcg tttgcagggg 960

gcggagcatg cggtctggca gagcagctgg tgggaggggc gctcaggtgc tgggctgagc 1020

cctggccatc cccacaggcc ccctgcgtgg aggctccagc aggagacctg ggggccctct 1080

gtgctgggag gctcacttcc tgtccacggg gcgggcccag aggctgttct ccaagaactt 1140

gagggttcca agaattcagt gcacttcaat tctaagcaga gcatttaatg agtgcctgct 1200

gtgtgccagg cacagtacca ggtgctggtg atcccaggat gcaagagaca gctggtccct 1260

ggagtttata ccctttgcag agagggaggc atgacttcat catgggagat cataacagag 1320

acaggaagtg attgaggcaa cgggggaatc tacagagagg acctcagggt taccgagatg 1380

gagagctggg ggcctgcaag ctagcattgg agctggctct tgaaacgtgc gccctaaaga 1440

gggaactcca ggtgggggga gattgcttga gccaggaccc agaggctccc ccaggcaagg 1500

gcctgtgtgg gagtccctgg gtgggaggat gtggggcgat gaggcaggaa gggaaggtgg 1560

ggtccgctga tggagaaggg cacattgagg ggaagtggcg ggcagtgccc cacaggagcc 1620

aagcctattc tgaggccgcc ctgggggacc aggcaccctg gagctagaac tgtggcccat 1680

ttccctgatg cgaaaactga gggtctgaga agtcatttga ccacctagcc tgaaaccagc 1740

cctaggctca ggtgactcca agctcttctc caagctgcag ctattggctc cggctgctgt 1800

ttgaagggac aaaacctgag ttctgcagct tgcagggcgc tcccttcgcc acgagggctc 1860

tccacgtgga ctcgggtgaa tattcgacca ccccagttgg caccaagttc actgttcatt 1920

gctatcactc cccacccaca cacctttcca gctccggggg gaagggacgg aggggcgggg 1980

agaggaagag ccactggtca c 2001

<210> 153
<211> 2001
<212> DNA
<213> Homo sapiens

<400> 153
tcaggaggcc agtcacttgc ctgtagccca cagttagtgg tagcactggg attccaactt 60

gggtcccctt catgtcgcct gtatttggtg tcccatttag gcgtccatgc aggttttggc 120

gtgtctgaag atctgtgtct ccccaagtaa gtgtccccca tggatgccca cccagccgtg 180

tcctcctgtc tctgaggttt ctccaggaat cgagctcctg gcctggcttg tctgagtgtg 240

tctgtgggcc agtagcatgc ccctgcccgt ctgggtccct ctgcgtgtct ctgcttgtcc 300

tagcctgtgt gtgtcagtga cgtctaagag tgtgtgtgcg tgcgtgcgtg cgtgcgtgtg 360

tgtgtgtgtg tgtgtgtcct tgtccgagga gccgagagag tgagggggaa tgaagggcca 420

aggaggcctg gctggggctg ggctgggggt ggaggcgggg agaggggcgt cacggcctga 480

gctgagaggg gagtggagtt ctggaggaat gtttaccaga cacagagccc agagggacag 540

cgcccagagc ccggatagag agacacggcc tcactggctc aggacagggg gcacagccac 600

cagggtcccc ttccccctcc tcagctccct ccctggcccc tttaagaaag agctgatcct 660

ctcctctctt gagttaaccc ctgattgtcc aggtggcccc tgggctctgg ccctggtggg 720

cggaggcaaa gggggagcca ggggcggaga aagggttgcc caagtctggg agtgagggaa 780

ggaggcaggg gtgctgagaa ggcggctgct gggcagagcc ggtggcaagg gcctcccctg 840

ccgctgtgcc aggcaggcag tgccaaatcc ggggagcctg gagctggggg gagggccggg 900

gacagcccgg ccctgccccc tcccccgctg ggagcccaac aacttctgag gaaagtttgg 960

cacccatggc gtggcggtgc cccaggatgg gcagggtccc gctggcctgg tgcttggcgc 1020

tgtgcggctg ggcgtgcatg gcccccaggg gtgagtgatg gggctccttg gggcagggga 1080

tcccctcgga ggggctgggg caggggtagg aggtggggga tgatggcagg tgtggggggc 1140

ctgggctggc ttagggagtt tccttgggac cacagaaaag ggacttcaag ggagggagac 1200

acagactcag agacaccaag tgcggcagag ggagagaaag actgagagag gaactgaagg 1260

gcagggggga cagaagagag gacccggaga gacagacaga caaggagaag ctgagggcag 1320

gggtgggggc tggacctgag aagggggcca tggggagagg ggctgagctg agggtcgcca 1380

ggaaggaaga aatctttccg aaggggcacc gagtcaggca gtgggatgag gagggctctg 1440

gagccctggg ggaaaacgct tctccctgcc aacaccaccc ccctgacccg ccacttgtcc 1500

ctaccccacc agtccactcc atctctgggg aggaggctct cactttgtct ctctgtctct 1560

gcctccagct ctgctatctc tgtgggtctc tgtttctctc tctttcacag tctctgcctt 1620

tatctctccc tgctctctga gtctctctct ctcttctcag cctccctctg tgactgtatc 1680

ccccctccgc ccccaccttt tcttctctct tgagggctgt ttcttcattt tttctgtctt 1740

gccttgtatc tgtgctcctc tctgtctcat ctcccctgcc tttctctggc ttctccctcc 1800

gccaccaccc ttatctcccc ccaccaccac ccttatctct ctcccccaac ccctgattct 1860

tcccctccat ccaccagcac cagcgcgacc tgttaagtct ctagagacct aagagaggac 1920

ggagggggca ggggctggcc ctgccaggca gtgcagggcg tgggaggctg ccagatcccg 1980

agcctccttc ccgacgggat g 2001

<210> 154
<211> 2001
<212> DNA
<213> Homo sapiens

<400> 154
aaggtaaggc cttttgaaaa agccacgatg cccaggtgag ggttgaattg atcactgaga 60

accctacaaa tgcgctcatg gcaaggctgg gaatacccta cacttaccct ctcggtcatt 120

tacttcagag tctgcggggc attctaatcc gcgttgtcat ttgctttgtg ccacattcct 180

aagaggtagg cagggcaggt tttatcatcc tctgtttaca gatgaagata atgaaggtca 240

catagctcaa gtgagctacc cagggccaca gacatacaaa gtggtagaac cagaatttaa 300

atcttggttg gtctgactcc aagtgtagta tttgtttcac tttttctttc tttctttctt 360

tctttttttt ttttaactgt ttctgcattc tgggttctga ccaggtgacc ttgaatcata 420

aagattcagg gcctgtcttg agctgctcaa tatggaaggc atcttttctc taaagagtgt 480

gagctccctg ccaagcagca caggcgctta ataggctttc agagcagcaa ttctagaacc 540

tcccttgtgg ctttctgatc tcccacagcc cttgtgtttg atggtcagga aaggagaata 600

cccttggatc gtcttatctc cagaccaccc cgaggctggc tggacttaag gggtgaggaa 660

agacaagatg ggtgagcagc tgctttcggc tgaaaaccag aaagaaatcc cagtgttttt 720

gctttatttt ggtttaaaaa tcaagtcgta tgacaatgga aacattttcc tggttgtcct 780

ctcctctctg cccatgttga cagaagacgg acttgctaat tctggctggg gtttttccct 840

gcctgggctg tttttctctt ttatcttcat caattgaaga gggctgctgt gtctttcaaa 900

ctgtcggaag gtatctgtat ttttttttct ttcacataat tctgtaggtc ttttcccaga 960

tatgtatttt tctctctaac ttctaactcg ctgaagtttc cgcccatgtg acttccagcg 1020

tgagttacca gaaaccacaa gagagagaga gagcgtgcaa gccccaaagc gagcgacatg 1080

tccctttggg gagcagtccc tctgcacccc agagtgagga ggacgcaggg gtcagaggtg 1140

gctacagggc aggcagagga ggcacctgta gggggtggtg ggctggtggc ccaggagaag 1200

tcaggaaggg agcccagctg gtgacaagag agcccagagg tgcctggggc tgagtgtgag 1260

agcccggaag atttcagcca tgcctcacag ctccgacagc agtgactcca gcttcagccg 1320

ctctcctccc cctggcaaac aggtagagtc ctcctttttc tctctcctac cttctgattc 1380

tcctggggga tggaaagaga gccaggcttc cttgtcctgc ccagggagct gaggatggag 1440

gaagtggctc gttgcacggg cactctgtta gacttaggac atggaatttg ctactaagct 1500

gtgcatattg gcagagatcc tcatccttcc acccattctg ccaaagcccc tttttctctc 1560

cattttccaa ggctgcctat cacctctgcc tcactggggt tgccacccta aaaagctttc 1620

taggaagcaa agaggaggat gaacatcaaa gaatgcagag aaaagagtct actgttctcc 1680

aaggctgtag aaaagtgaga ggagttttca agtgggccgc tggcatgcac agctcaaatc 1740

ctagatatgc agtttcccag gagacagcca atagggaagg ggagaaaagg ggttagggaa 1800

aagttaaaaa aaaaaaaaaa gaagaagaag aagaggaaga aggaggagga ggaggagaaa 1860

cccaggctag catagaagga tctctctact ggggcactgc ctctgttgct agcacagcca 1920

gcaaggggag atgctagagg tccagccaat atctctttgc ttgtggcttc tggataggag 1980

caaggaaatg aatgaggagg t 2001

<210> 155
<211> 2001
<212> DNA
<213> Homo sapiens

<400> 155
gctccccatt gccaggtgct tttcttcctc ctcctcttct tctttttttt ttttgagaca 60

gagtctcgct ctgccgccca ggctggagtg cagtggtgtg atcttggctc actgcaacct 120

cctcctcccg ggttcaagcg attatcctgc ctcagcctcc tgagtagctg ggattacagg 180

cgtgtgccaa catgcctggc taatttttgt atttttagta gagatggggt ttcaccatgt 240

tggtcaggct ggtctcgaac tcctgacctt gtgatctgcc cacctcagcc tcccaaagag 300

ctgggattac aggcgtgagc caccacacaa ggccctttct tcctcttctg tcatctgtgg 360

atttcccatc tgcctcagtg gagtccttga ccttttcctc cctggcccag cagcacattc 420

tcctctcctc ctttctccca agtcactgac ctagtattta gtgtgtgttc tcagcctcac 480

cattgacttg ctgtgttacc ttggacaagt cactctccca ctctgacctc aattttcaca 540

tctctaaaag gagactgcta gactagacgg tgagtgaagt cctgctgagc cctccgagtt 600

cattctccct tgtccagtca gtccttccct cccaccctcc cctctgggct ccctctctgg 660

ggccctctcc cccaggggag gcctgctcca gccccagggg agctgcggcc ctggctcctg 720

ttcccagtcg gagtgaccag ccctgggcct ccttcccagc ttcggccctg gctccatgtg 780

gcccatctga gaggctggcc tcaagcccgg cggcaactcc acatttctgt ttttcctttt 840

ttttccctct ttcccggagt taacaagaag cagatgtggc gcacgatggt tggagaggtg 900

gggggaggaa gggggaggcc ggaccgccag ccagacaggg gggaagggag gggagccagc 960

agggaggagg aggccagggc ccgccccaca gccactctcg cgcctccgaa cagccacagg 1020

ggcaaagccc tgtcaccccc aggatccggt catcagggaa agaggacagg gagaccagaa 1080

gagggccagc tgggacgagg gggcggacgc ccaggaggca acttctgaga cgcagctcct 1140

gagaggggca gggaccaggc gcgggaggcc agagggggca cagagaacaa accccctcag 1200

aagtgaagag gagagcggaa ggaaccgaga ggggacggac aggagctgag gaggaaagag 1260

gaggggagag gggtcaggcc aggcagccaa ggagaagacg tgtggccggg ggctatcaga 1320

aggaaactgg gacggacggg ccgggctcgg gctgtcctgt ggagcagcag catccccggg 1380

gccggcagag gcgccagtgg ctgggcggga tgagtctctg agggccactg tggagcgccc 1440

cgccatggcc ccccgcaccc tctggagctg ctacctctgc tgcctgctga cggcagctgc 1500

aggggccgcc agctaccctc ctcgaggttt cagcctctac acaggttcca gtggggccct 1560

cagccccggg gggccccagg cccagattgc cccccggcca gccagccgcc acaggtaaga 1620

gtctggatcc cagcccgagg cttgggtggt gaggaagggg tcagaatgcc acctctgcct 1680

ggctctctgc tgtgtccgct aatgcagggc agagggggag tgagaagccc atccatcagg 1740

tggctcctca catgcccacc ttcccacggg gccttttcca ggaccccctg aacacatatg 1800

ttccttgggt cacctagggg gtccgctccc acatgcccca gggaagctgc cctgagggga 1860

gcgggccagg ccttcctgcc agctgccctc tgtatcctag ccccagagcc tcagggagaa 1920

cttttgtccc cttctagggc tcccttgacc ccatccagta atccataatt ctgacaccac 1980

tccagctctg ttccctccag c 2001

<210> 156
<211> 2001
<212> DNA
<213> Homo sapiens

<400> 156
cccaacataa aagaacgcaa ctagtaacca taaagtgttt aaaagtctgg gcttctcccc 60

accaagaaaa gacgagttgt aatacctgga ctgtgggtct ccagtccgac aagaattggc 120

tctctgcttc actggtctcg taaggtcact ccctagaatt ggctttctat aggaccattt 180

aattgagtct atttgaatct tactcaagtc taccacctta taagtatcaa accgaattta 240

gtaggattcg aaatggtaag gactgcatta aattatttct ctaattcagc agtaaggaaa 300

gtgaacagaa gtctcggtgt acaaccacgc cccgggttga catctttcct tccacgaaga 360

cactgggtac aagccttgat gtagctgtta attgccctcc tgtttcctag aaagcttata 420

gggcagcagt aggaaacggg ttaggtatgc ctttcctggt aggagccgag ctttgggtct 480

tatctctgcc acttgttcgg catctgatca gtaaaagggg accatgatgg gaggcgaggc 540

agtagacagc actttgtgac ggttcctaac cagtcgctgt tatcactgag cacattgtcc 600

tcgtaatcca gaattcatac tgccatttga atattttttg tatttgcaaa gaaaatttta 660

accacttaac tcaaggctag aaataaaagc aattagtgat tgcaagagaa atccaccggc 720

agaaggcagg gggagacgac ttaagcctct gattattttt ggaagcttta tatgctttct 780

agcctttgtc attgaatgga ttaatgtatg tgcctggcat agactaagtg ctttgcttgt 840

taaactgccc gcgggtcata gcgagcaaac tgggggagcg cccagggaca gtggatggag 900

tgagtggaga ctcgtgtcct tttaagaaga ggctccattt cttcctcgca tgtcaatgtg 960

tgcgtgtcat cagttgttcg aggaagctcg cgcatgacgc gacacttgcc gtctgggctc 1020

tccaggttcg gcctgctgct ttcacagccc agatcttcgg cgtccccgag acgcagccag 1080

gcgcccggcc cggcccggcc cggccctctg cacagcccca cttcaggcca cgccgtttcc 1140

ttgaccttcc cccggcaggg tctgaagcgt caccgtccgt ctagagcaga gtcgcgcagg 1200

cccctgccct gccggtgagg tcggcgccgc gctccggaga gtcggttcct cccgattcag 1260

acccatatgg cttcagatgg aaccatcatt tctgttccgt catgaccggc ttgcaaggta 1320

gcaaagcgaa acaaacgtct cgttttcagc ctgtccccgc caccttgaaa taaaaccggc 1380

aaaaataaaa agagcattct gggaagtact tcatcatttc aacgctcacg aattcaaaac 1440

ataaacaaag gcttccgagt gccccggcca ggggcgcggg gcgcacggcg ggcccggggc 1500

aggtaagcgc aggtgcgcgc ccgcccccac ccccggctcc ctccccatcc gctccccgct 1560

ccccttcccc tttcccttcc ccgcccgctc ccagccgccg ccgccgcccc gcgcacggcc 1620

tggagcggag gctgcgcagg gcgcggggcg gcgcgggccg ggcgcgggcc gggcggacgg 1680

ccgcgtcttt cttctcctgg cggtgatgtc attgggcgac ggcggccgag gccggggggc 1740

ggcggcgggc gcccgcaggt tcccgagccg ctcctgagaa ggcgcctgac agcgggccgg 1800

ggcgcacgga gaagcgggcc gggccggacc tgctgggccg cgccgagcca atcgccggcg 1860

ccggccgctc gatgggcgag gcggcggcgg cggcggcggg ggccgcgggc cgggccgccg 1920

ctccgaggtg aaggcgcgcg cccctccccg cctgcctccc gggccgcagc gatgaattcc 1980

gccgagcaaa ccgttacgtg g 2001

<210> 157
<211> 2001
<212> DNA
<213> Homo sapiens

<400> 157
cgggagttcg tgaccagcct gaccaacatg gagaaacccc gtctctacta aaaatacaaa 60

attagctggg catggtggca catgcctata atcccagcta ctcaggaggc tgaggcagga 120

gaatcgcttg aacccaggaa gctgtggtgg cggtgagcca agatcgtgcc attgcactcc 180

agcctgggtg acaggagtgt gaaactctgt ctcaaaaaag aaaaaaagac ttgatcagct 240

tatagccttt atcatctagt cttctattct ctggagagtc agtggggact cttccgagtt 300

cttgtagcct tttcttaagg cctataggaa gtcatctgtg ctgagcctgc actttctata 360

acagatggaa aactgaagct cagacatctt aagtggcatt gacttagact atagagctag 420

ttagtggggg gccagcacca tgacctggcc cctacctcca gcaggcttca cccacccttg 480

cacccttgca ggaagcactt agaggcttga ggttgccagc tgtggggggt tgggcctgtg 540

cagtgcctct cagccgccca cgctggtggg gctctgcagt gttagcttct tgtttctcta 600

ttttctacct aggtttcagt ttttcacttg ggtttgtttc ccctttgtct ctggttcagc 660

ttccttttcc tgtttggttt taagtaggct ataaaaatca agttgctgtc ttcagagggt 720

ctgtggtcct ctgatcaaca taggctggtg ggagtacagg actcgcctcc tcagggttcc 780

ctgtgctgcc acttttcagc catggccaca aggtgagtat tggaaccagt gcaggggaca 840

gagggatgtc aagaggaaac ctagaatctt aacggtgaat gggctgtaga aattatctca 900

ggtcccctta gtatactgat cgagaagtcc atgaccagag ggcagggggc ttacatgagg 960

ttacacagca ggttagtgac agtctgctcc ctctctagcc gctggtcttt ccattacaca 1020

ggcctgtgtc tcttgatgcc tgtgaccttt aatggcttct gagtcaagga cttacttaga 1080

aattagcacc tgcctctcat tatagggagg agggtctgag ccatctaggg gaattgttgt 1140

ttagctcaga ggtacatttg gtgagtcctg gcccttctct ctagaatgac ccgagactgg 1200

ttgttttttc taaatatagg cagagagggt tctttttatg ataggaactt catgaactta 1260

atagcaagag tcctgaccct ccacttgaga gacttgtgga cttaggctat tgctgttgag 1320

cacatgtgtg aataccagcc agtctacaaa ccgcactcct cattgagatt gttctctcta 1380

aaggtgagat tacattttgg acctgcctct cccctgccct acaactctga aagattatag 1440

atgataggac agacagactt ttatcctgtg gggtttgggg catttgtttg attgactcat 1500

gacagctttg ttttttagca taaagtaaca atccctcact ttgaaaatta tttcacaatt 1560

tccaaagtgc catttcgcca gttttcttat ttgaatcttt tagccgtgtt agattgatga 1620

gaacgctgtc atcactcctg tttcgtagct gagaaaacct ctgaaaagtt gtggggtttg 1680

tccaaggtca catggcaggt tggggaagga atggaaccag ggtttaacga ggttggcctc 1740

atccggtggt tttcgcagca cgctacacag gcacagaagc agcttgcagt gagttgttaa 1800

aattgacgtg gtgataggta gaacttgtcc agggctgctt attttccagt tggctttatt 1860

tctgatcctg tcattttagt aactttcctt taactttaga ggactcgtga gtggagcttt 1920

agaaaacaga atgcttttca tttcaaaggc atatcaactt ccaaagtcat tttcagttgt 1980

aaattacatt gtgaaaactt c 2001

<210> 158
<211> 2001
<212> DNA
<213> Homo sapiens

<400> 158
gctggtctca agctcctggc ctcaagcaat cctcccatct tggcctctca aaatgctagg 60

actacaggca tgagccactg cacctggcct ggacctgtca ttttacagat gaggaaactg 120

aggcccccag gggacaagac agcctgaagt cactgggatc tgacaactga gaagggtctc 180

agcccagcct gcccctcccc tgccgacccc acaatcaccc tcccatcctt tagcctcctc 240

cttccctctc tgtctctcaa tgtccccttg cctcttctgc tcctcaccca catcccccac 300

acatcctccc caacccggta ggctgagggg aggggagaga aagtggaggg ggtgctagaa 360

ggcacatact acaagcaaga cagccactca gcttatctca agcgacccca cagcatcccc 420

agggcatagg cctcctccca gatgaataag ctgaggttca gagaggtgct gtgacttgcc 480

caaggccaca cagcggcacc aagccagtga cgacagccca ggtcagcctg gggacaaagg 540

ctgaatttca ggtttcccac ttccctcctg catccacaga gtaaagactg aggcttgcct 600

ccgtgggaga cctgccccca gcttttgggg tgcaggtgag gagccctggg cactctcacc 660

catagtcctg caccctccac cgagatggct tctggggtgg ggtggggctg aagagggtga 720

agctcccctc agcccacccc cgggtgctcc ctgtgggccc tcggagaagg aagcgtcccc 780

tccaagctgc cttccaggga cgcctagttc tgcagggcca ggggcttccc caggggcacc 840

agggcggaag gatctcaggg catcccagtt gggggctgtg aggaagtcca gccaggtacc 900

tacccatctc ccaccaccac acacttgatg gcctgcatcg tgtccggagc ctggagagtg 960

tcggtggtga cagctcaggg ccaggcgcgt ttctgcgggc gcaaggggtg tggaggctgg 1020

tgaggcgcct gctgaggagc agcggtggtg gggcaggagg aaacggaagg ggaacttact 1080

caagctgctc tccccacggt cgtttgcagg gggcggagca tgcggtctgg cagagcagct 1140

ggtgggaggg gcgctcaggt gctgggctga gccctggcca tccccacagg ccccctgcgt 1200

ggaggctcca gcaggagacc tgggggccct ctgtgctggg aggctcactt cctgtccacg 1260

gggcgggccc agaggctgtt ctccaagaac ttgagggttc caagaattca gtgcacttca 1320

attctaagca gagcatttaa tgagtgcctg ctgtgtgcca ggcacagtac caggtgctgg 1380

tgatcccagg atgcaagaga cagctggtcc ctggagttta taccctttgc agagagggag 1440

gcatgacttc atcatgggag atcataacag agacaggaag tgattgaggc aacgggggaa 1500

tctacagaga ggacctcagg gttaccgaga tggagagctg ggggcctgca agctagcatt 1560

ggagctggct cttgaaacgt gcgccctaaa gagggaactc caggtggggg gagattgctt 1620

gagccaggac ccagaggctc ccccaggcaa gggcctgtgt gggagtccct gggtgggagg 1680

atgtggggcg atgaggcagg aagggaaggt ggggtccgct gatggagaag ggcacattga 1740

ggggaagtgg cgggcagtgc cccacaggag ccaagcctat tctgaggccg ccctggggga 1800

ccaggcaccc tggagctaga actgtggccc atttccctga tgcgaaaact gagggtctga 1860

gaagtcattt gaccacctag cctgaaacca gccctaggct caggtgactc caagctcttc 1920

tccaagctgc agctattggc tccggctgct gtttgaaggg acaaaacctg agttctgcag 1980

cttgcagggc gctcccttcg c 2001

<210> 159
<211> 2001
<212> DNA
<213> Homo sapiens

<400> 159
ggggtctact gctaaaagtg atgtgaaagc cgccatgttg taggcttcca aggcctaaat 60

cccgtccctg ggaagccagt gaccaaagat gaaatttagc tttgaaacca aatgcagagg 120

aaagtgatga aaaaaccaca ggctggtgat gcaggtcaga ggaggagatg ctctttcaca 180

ttcggacttg agagagcccg agagtaccca taggcatggg tcaagagagc tacagtggag 240

ctcttggggg acagaaagga ggatgtagaa aagaaagggg cagagactgt gcctctagcc 300

acccatggat acataaggca ttgaatccat tagtgaagag aaataagtaa tgaagcatct 360

ggggccccgt gtggctccta ttataatgaa tcatgtatgt tctcatatta gttattaagt 420

acacctggct tggtatatcc gatggcctat tttgattaat ttatctaatt cttttttttt 480

tttatttcct aggccttggg ttaaaagact cagctatcaa cctggaggat aactgattac 540

ctagtcagag ttctggtacc tggagacaaa ctaactttga ttaaagcatc agatcaccag 600

gtcttaaaca ctgggcacca aaccggtcct gggacagggg ctaaaccttg tcagttatga 660

tcagggagca gtcctagaag aaaatagcac ccaaaggaga acttgctctt ttgggaatga 720

gaaagagcta atgttttgca ctatggattc agtcaagaag agaaatgctt ttggggcttt 780

cttaaaaggc caaatgtaaa aagtaccccc atcatttcct aaaactcaaa cctctaggac 840

tctacctctt gggctaattg ctcatgagcc accaatctgt tctgtcttgc actgtgtgtt 900

ctgagagcca gcaggttagg acagagggaa catgacgggg tcagaggagg ggctcacctg 960

actgcacagc tccatgtttt ctgagtacac agcaatctgt cgtctcggct gctgctcctc 1020

tgacagatag ctggagaaca ggatgaggac atcaaaacca aacttgtctg taaatgcttt 1080

caagtcactg gtaatattgc tgtgaaatag acaattctga aacgacaaca ggaaatttga 1140

tattaatgtc aaactgccca gctgcgggga gttgttccac aaatttgtcc agtcttatga 1200

tgtatttagg agagatattt attatttaag gcaagtgaaa tacagtttgc tttgagaagg 1260

agtggaaact ttcatgctat ggcttaaaat acactttgct tgtttgtttt cccaaaccat 1320

gtaagaaagg agtttttgcc attattaata atagtttaga caaagaacag atatcctaaa 1380

catttttttc taaaatattt attcagatgc ttaatttgca aatcctattt tccctcttgt 1440

cgcttatagg atctatatac gaatgtctgc tattgttaca atttatcaca ataagaatag 1500

gaggaagtta tttcttcctg tatttttttt ctaatgatta ttattaactc caaggagaaa 1560

ttcctacgaa tcaacatagc tgagtttact gggtgatctt tattccagtg tagaattctg 1620

ggagattttt aaaagagctg tgatgtcaga tttgcttgtt ctatgtggca gctgtggagg 1680

aatgaataat tgcagaaagg gatcccaata cctcagatta cccagatgtg gctcatatca 1740

tttgcaaaaa tgttctggct gcttcaagtt ttatttactg tatgtatgta cttatttatt 1800

tatttttgag acagggtctc acactgtcac ccaggctgga gtgctgtggc gttatctcgg 1860

ctcactgcaa cctatgcctc ctgggctcaa gcaatcctcc cagttcagcc tcccaagtag 1920

ctggggctac aggtgtgtat caccatgccc agctaattta ttgtattttt ttgtaggcgt 1980

caggttctgc cattttgccc a 2001

<210> 160
<211> 2001
<212> DNA

<213>  Homo sapiens

<400>  160
atcggcctcg gagacaggca gctctgagtg agtcaggacc ctgcggctct ctcacaactc      60

ctgaaacaca gagattcctt gtctttcctt tttgctttct cccctaaaaa ttagtaacta     120

cattttaata agtggatgaa atgaattgat tgaaaatatg ttagtaaaaa catggtacac     180

ataattgatt tttaaaatca aataatacaa aagagccttt cctatccaga ggcatctatt     240

gatctattgt gaacactttt ttaggtatcc tttcagaatt tttctctctg gatctaagca     300

cgtttttttg gtttgttttt gtttgtttgt ttgttttgtt ttttttttgt tttttttttg     360

tttttttttt ttggagacag ggtcttgctc tgttacccag gctggagtgc agtggcacaa     420

tctcaactca ctgagacccc cgccctcgcg gggctcaagt gatcctccca cctcagcctc     480

ctaagtagct gggccacagg catgtgtcca gctaatattt tgtatttttt tagagaccag     540

gtgtcgccat gttggccagg ctggtctcag actcctggac tcaagtgatc tgccttcctt     600

ggcctcccaa agtgtcagga ttacaggcat gagccactgc actcggccac atttttaaca     660

caaacaggaa ctgactctcc acctttgccc agcgtgctct tttaaaaact aaagactaca     720

tctcaggaat tgttttataa caacatatca gatgagacag cgatgattct atccattttc     780

cagaggaaga aactaagact caacatttcc ggtgcctatc agaggttact ccagtgtccc     840

tggtatagct ggtatttaac ccactcccag gaccctaagc ctccgccctc ggcatccgca     900

gcatccccca accccccaac ctcctgcagt ggactggctg gtagtgggca agagactaat     960

ctcattccag gaaaacgggc ccctagattt tgtgttctcc gtgtttcttt tccagggagg    1020

cattttgatg acccccactg gcccttccag gagcctctgt ctgaagggtg gacttggttt    1080

atcaccccga gaaatgggag cgcctggctg cttggattta gcctgcagct ggggtccccg    1140

gtggtgtgtg cagggcggcc ttgagcctcc aagggtgcat ccacaaactg cccttccccg    1200

agtgacccat gggagctcga tggcgtacgt gctgagcacc aggatgtgtc ctgccctgtg    1260

actcccaggc cccgcccttg cgctcagcac gccctttgtg gatggatttg agggcccctg    1320

gcctctgatg tgccactccc gctgaggcca gcgaaggagg aagaaggaaa gagcgttgcc    1380

tgccttgttt tcggggcact cactttttct gggagacagc ggacccaggt tcatcaatgg    1440

acagcaggat tttcatgtta ttaaaaaggt cattgtaggg gaaatccctg ggggatctgg    1500

tgagatctcc ctgggggatc tggtgagatc tccctgcctt cccactgctc ctagtaccca    1560

gaggccaggc caggtcgtgg tgcagggctg agttccagga gggtgcaaac accccaggaa    1620

cagactgctg cccctccggc cctcatgacc tgctggggaa ggacccccgc aaggagcttc    1680

tgcaggtgct gtggtctcca cctgctgcac tcaaaattgc ctggagtggg gagaatatca    1740

atgcctgggc cccgccctga caggtggttc tgtcgcctgt gattcttctc tgcggccagc    1800

accgagaacc ctgctggggt ttgcttccgt ttaactagtg aatgttgact gagctcctac    1860

tgcctacacc cagggtttag ggctggggaa ggaataacaa aggtgacaca ttcttgtcct    1920

tgaagagtcc ccccgcaaag ggagacggga tcatgtcaga gttaagatgg tgaagccaag    1980

actaggttct gtgctgcctg a 2001

<210> 161
<211> 2001
<212> DNA
<213> Homo sapiens

<400> 161
gagacagaca aatagcaagg tggtagtggg gttcagaata cacaaaggag agatttccgt 60

ctaagttttg cctcctgatc atttcttcga ggcgttcttc tctttcgtgc ctaaattctg 120

gtactttccc agatcttgtc agaagaggac aagggaaaat aggacaatgt ttcctgtttt 180

ctgagacatt gtatatctcc tcattctcct tatgcatttc ttcttccttt ttctctccct 240

tccctcctta ggcaggaaac tgccctaatc ttaaccagat gctgcgggca aggtttttta 300

tttttcccct aacaacaaaa acccactgtg gctgccgcaa aggacaagaa gcctcagatc 360

caaggtttgt tagaagaatg ttcagactga aaagcaatgt tctatgatga tgaaattcac 420

agcttttcat aagtttactc tagttagctt cactcatttc ttgcattggc tttcttaatt 480

acaaatcctc cattttaaga acaatttgca gcctgcagca tgacatatgg cttacacaat 540

tgcttatagt ccatttacat gtaattttgc aaactggcaa aatgtccagt aggacattta 600

aaaactagag tgttaagggc tttgagatgt ctcagtgaac aggaagcact gagctactac 660

ctccccatac cactctcctc ctcctcacta aggagttaac cgttttgatc cttgttggcc 720

aaagtagaaa acactgcaga aaggccactc ttgaaggatg gggaaatgat tccctatata 780

ctttgctttt actaacaatc atgtatgtat atatataaat catacaagga attctacaga 840

tttccaggca gattttattt agccagggaa tgacctaaga agtgcatctc tgttttcatc 900

tgcagaggct aaaagtttat tgtgatcctc aagtcactac atccctaata ctcagagaat 960

tgacaggaat gaaaagctaa aagatctagg aagttgcaaa cgttacctgc tgatggaaat 1020

ctgttgtctg gcttcgtttc ggcagcaaag tgtgggcaca ccatcagggg tgctagattt 1080

attccatcgg gattgcggaa gtcagctcca gtgatcggct ggaaaatttg ggtaagagga 1140

ggaggcgggg atagaaatgc tgagagaagg aagggtaggg aggagggaag gaggcagtgg 1200

atttgcagtg tttctgtgtg tatgcagcat gtgtgccagg gtagggaatt actagctatt 1260

tagtctcaca agactgtctt tggtggcaaa taacaagacc tcaatatcta ttgactcaaa 1320

taggagctta ctttctcttt tggtgagtaa aaggaaggac acagccctag tctctcccat 1380

ccttcttctt caatatcttt attttaatat tgggggatta tatcttttcc tacccctttc 1440

ctgttaggat ttacctctcc aacattgaaa cctattttca tatttgtgtc tgactctcac 1500

ctctgggatg cggtaatgaa cataaaaggc tataaaagaa taatgtaatg gaagccatag 1560

gctccagaat tctttgtatt tttataagct tgttggttgc aagttgtcat tacttccagg 1620

cagaatagtt ttcccagcct aatgtggttt tgctgaagga ggatatgccc agaaagtcca 1680

gatgaaactg tctttaaaag taaccaaaaa cagcagtcct ggttatttat gacagcactt 1740

gaatcaatgc cgtaagttct gatggactca catgtgactc tgttgctaaa actctcaggt 1800

ggtgggatgc ccgataaaca tcatctgtct caattaaaat ccccaatcta ggacaagtca 1860

catgagcttc tccaaatgct tccccaacat ccaagcatgt gacagatcat aagaatctag 1920

ctgattcctc aaaccacacc ccaaacttca ttttcttcca taaacagatg tggggaagga 1980

gaaaaaaagg cttcttggaa t 2001

<210> 162
<211> 2001
<212> DNA
<213> Homo sapiens

<400> 162
caagggcctt cttgctgtgt catcccatgg tggaacagca aagagagggc aagagtgtgc 60

caaaaggggg gcaaactagt ccttttatga aaaatacttt gataacaaac ccactcccat 120

gataacaaca ttaacccttt cctgacggta gagtccttgt ggcctaatca cctcttaaag 180

gtgccacatc ttaatactag tatgatggca attacatttc aacatgggtt tcagagggga 240

ccaacattca aaccatagca gtctcaaaca ctgggctggc agtggaagag gaggctgatc 300

tttaaagaat ataaattttt tttttttgag acggagtttt gctcttgttg cctaggccag 360

agtgcaacgg tgtgatcttg ggtcattgca acctctgcct ccccggttca agcgattctc 420

ctgcctcagc ctcctgagta gctgggatta caggcatgcg ccaccacgcc tggctaattt 480

tgtattttta gtggagatgg ggtttctcca cgttggtcag gctggtctca gactcctgac 540

ctcaggtgat ccgcctgcct cagcctccca aagtgctggg attacaggcg tgggccacca 600

cgcccagcca gaatatgttc tttaaaaaca ggcacaattc catgaagtaa actcacattg 660

gaatggttaa caggtaaagt gatccagctg gctgaaacaa taggcattca ccggcatcca 720

aagtacataa agatgagcac tttgagactg cgccactgca ctccagcctg ggcgacagag 780

tgagactctc tcaccaaaaa aaaaacgtga gcactttcac ctcaagagtc gctaggctca 840

gatgctgaat gcaccctgaa ggaactctaa agataccagc ctctggagtt ttcacagcca 900

acagcattcc taggtagact ggttgagaac acggctagtt ggagggtaaa aagaatgact 960

tgtgaatgct cactaggtct ttgaatatgt aagtagacac gacttcaagc cagttgctca 1020

gccttgaata tatcctagag tccatcacct tgggctgtga gtggcttgtg cggtcatctg 1080

tccatctctc agctgggcga gaaacttgat gtcctttctg agaggtggcc ctggctctgc 1140

ctgggacctg ctactcttct ccaaagtgca ttttttgtgg gtgggttttc actgtgggtc 1200

ctgggcaggt gagtgctggt gcccagtgtt ggggagcagg gtagatagag gtgtggctgc 1260

tacctggctg ggcccggact tggcagcctt ccttcatggc cctcagccac agcctgttcc 1320

atagtttgtt ctcgatgtgt cagctttgtg ttcccatgag aacaatgggc tccttgagga 1380

caggcattgc atcgtacctg tggtctcttc cctaacgccc cccagtacgc ccagcataga 1440

cagcacaggg tgcaagcagt cagatgagca gggctcccgt tcccagcacg ggacccagcc 1500

cgttacctcg ctgctatgtt tcccacgtaa acggagcaat acccaattct aaagaactgt 1560

tctgaggact aaatggaata gtgtaaaaaa taaattgaca tgaagtgaag gtcaattaaa 1620

atgcactagc ttggccaggc gcagtgtggc tcatgcctgt aatcccagcg ctttgggagg 1680

ccgaggaggg aggatcgctg gagcccagga gtttaagacc agccagacaa cacagcaaga 1740

tcccggctct agcaaaacat taataaaaat tagccgggcg tggcggtgcc caccggtggt 1800

cccaccttct cgagaggctg aggcgggagg atcgctcgag tcctggacgt cgaggctgca 1860

atgagccgat ttcgcgccat tgcgctccag cctgggcgac agcgcgagac tccttctcaa 1920

atatagaatt ttaaaaaggc catctcgact gccgcggggg tcgcgtcctc tccatccccg 1980

accttggacg caaccccggg c 2001

<210> 163
<211> 2001
<212> DNA
<213> Homo sapiens

<400> 163
agccttccca ggaggggtct gataccaaag gccaagttaa gaaaatgtct gaggccgggc 60

acagtggctc acgcctgtaa tcccagcact ttgggaagcc aaggcaggtg gatcacctga 120

ggtcaggagt tcgagaccag cctggccaat atggaaaaac cccgtctcta ctaaaaatac 180

aaaaatcagc tgggcatggt ggtggacacc tgtaatccca gctacttggg agactgaggc 240

aggagaatgg tttgaaccca ggaggcagaa gttgcagtga gccgacatca tgccattgca 300

ctcagcctgg gcagcaagag cgaaactcca tctcaaaaaa aagaaaaaga aagaaaaaaa 360

gagaaaacgt ctgagaaccg acggcttgta tttaaaacgt gtgagtgggt ggtggcagga 420

gaggtagaca gggagcaggg ccggcttggc ggaagctttg ctgtagagag gggcagagat 480

ggctcggtgg gtggagggag gggcaaacgg tgtttaagaa gaaaaaacga cagcttctgc 540

gtaggagagg ggcctcctgt ccatctccag ggagaacatg ggttctgtgg ccacaggtct 600

ccccagggcc tggcatcaag gaggcgccag gcacgacctg cgggatgaaa gcacgtggct 660

gaagtcctgg tggctgtgtc tgggtgaggg gtgtgagggc gtggcctgag caggtccact 720

ctcaggggag aggggccgag gccaggaact gggtctgggt gtccacctgg tgcctgggtg 780

aggtcctgca ggcagcaggt ctgggcttca gtggatgcag ccagtaaggg atgggctgat 840

cagggaaaac cgtattttca ggggaggagc ccaggaggca gggagctggg cggcggtgga 900

ggggcctgga gggcgattgt ccagatcaag cccctcgagt aaggcccgag aggctgtggc 960

ttcagaaatg aaggtcatgg gcgactcggg ccaatgccgc gtccttgacc tgggcgaagt 1020

gagcggttgc actcacgagt gtggacggcc cttccctaca ggggaccttg gattcgctgg 1080

gggcggagtg ggggctgggg cccagagaac ccgagtccgg acgctcccgt ccgtgaccct 1140

ggccgggttc tccgctcctc gcagcttcag tccgcaagtg tgagatgtca ggggacccct 1200

ggacggcggg gccctcgggc tggcagcgcg cgccgcagga gtttcgattc ggagggtctg 1260

ggcgaaggcc gagaactcgc gtttccagcg agttcccggg ggcgccgctc cgtcccagag 1320

gccggggccc ctccggttgc cgggcagagc cgcgccgcgc gcggtcccat tggcgcaggt 1380

gcgccgcgcc ccgcccaaca ggtagccccg cccgacaggt agccccgccc gcgcctcgcc 1440

attgggccgc ggccgggtgt cccccgagga ttggcccagg tggcggcccc gcctcgccct 1500

cattggccgc aggtgccacc gcccgccccg ggaccgcccg ccccgcggat tggccgctgg 1560

ctccacctcg gtccgccatg ttcggacacc gcccccgcc cccgccggac ggtcccgccc 1620

cgcgcgcccc ccgctcccgc cccgcgcgcc gccggagttc cgcggagttg accgggtcgg 1680

cgccgtcggt cctgagcgct gccttccgcg ttccgccgcg gccccacctg gagcccccgc 1740

cccgcgccat ggccggagct cccggggcgc agcgctgacg gcggcggggg gagcgcgcca 1800

tgcccagcag gaccggcccc aagatggaag ggagcggcgg ccgcgtccgc ctcaaggcgc 1860

attacggggg gtgagcggcg gagagggcgg ggagcggcgc gggtgaggca gggagggcgg 1920

ggagggctca gccgtcgggg ctcctgcgcg agagggagag agggaagggg cggcgaggtc 1980

gctgcgggcc cggggctgtc g 2001

<210> 164
<211> 2001
<212> DNA
<213> Homo sapiens

<400> 164
cacacactga caatttggtc caaatgaatt ctaggaaatc tgattaaact cagagggtaa 60

tgaaaatatt tgtagtattt atattgtgct tagtatttgc caggtactgt cctgaatcat 120

ttgcatggtt taatctctta gtcctcccaa cagtctattg ggaggtatta ctaacagtct 180

gtagggaagt actattacta gccccatccc aggattacag acagagaaac tgagacacat 240

agcagtttca aagcttgtct gaagttactc aattataata tgtcaggttt gtgattggaa 300

cctagcacta taacttttga gatgatgtgt ttactacagt gcattcctac ttctgtaaat 360

ctcttctcct tcctttctct gagcaaacac ataaaactgg gaggtaggta ggagaattgg 420

gatgacattc atttgtggaa gtccatatac tgtgtcatat tcttcatctt cagcactttt 480

aatctctaaa acatagtgca gtatttagca ggtcatcaat aatttttgtt gttgggtaaa 540

tggctgatcg aatgaattaa caaaaggact ggatactcca tttctgatca ccatagagat 600

aaccaaatta cgtttctcaa actctgtcat gctgagggct gcagtgacaa cattttagga 660

ctagaacaca ttacttggat ttcacaatac aaagatgatt gcagttgtcc ctggtaagct 720

cagcccacag agcctttcaa ttcagcttgg ggtgagctgc acttcaggca tggggatata 780

atagtgtgat gaaccattct tctgaaggtt cttctgaaaa gctgctggtc ttgggatact 840

gtgagagccc ctctcattgc tctttagaag taatttaaat ttttctgcta cattgtctgc 900

tcagctcgta ttctggtcat agaggaacct gaagccagag aaactagaca aaaaggtaag 960

agaaaaccaa gcaacaaatg cagtctcagc tgttgctaac gttattcaca ggagtgacaa 1020

actgaatgaa tcttagggtg agagactggt ctttttacct catttacact tttccttggt 1080

ctgcactgta gatcagcaga actcatttat cctaagaaat cgatgactaa ttttctgtga 1140

agcagaatca agaaatctgg tctacattgg ttaaaaataa gtacttggtt caccttgtgc 1200

tgtgaagtcc aagccaaata attgttgcaa gttgttgaag catttgagtg aagaaaaact 1260

gaccaagttc actatgactg catgaaatga aatgctgaac tttttggggt tgagggggtt 1320

ggaaggatgg atggaggaag acagttgttt ccagtctctg aaattcaatt ctgcagtaag 1380

tttctgtatc accaagtgtc actttggcat acctcgtgca tatttatagt ctaaggcttt 1440

tcatatagga ttcaaacata gattaaagta gcagggaact tgagagctcc agggaagaat 1500

atgatacttt aattgcattt gatcatttta ttgaatttgc ctaggaaaat caattaaaac 1560

acaaaactta atttttgtac ttacagcaga gattatacat tccatcttgt ccctgactat 1620

agatttttgc cttttgcttt tcattaaata atcaacttca aagtactcag aaggccgatg 1680

gttctattct gctgggtatg ggcgaactgg aacagggcct tcattccaca aacattttac 1740

ttgtttcttt ttcttcatct ggcttctcta tgggacatgt tcaaattcta tacataccaa 1800

agcagtgact tattagacgt atgtaatgtt ttaaaaagat aatagtgtat ttaaattgca 1860

aatggaatta ctgcaataat atgaaatatg catgattata tggaacagtt gaagagttcc 1920

caagtgaata ttaattgcct tgtatccttc cttgaggcaa aggcatataa aaataacatg 1980

aattcacaag tagttagagc c 2001

<210> 165
<211> 2001
<212> DNA
<213> Homo sapiens

<400> 165
ggagaaacaa acgagactgc ctcccagtaa gcaaaagtga tcacattttc acaaatgaaa 60

atggtaaaaa gaagtttaaa ttgttaaaga ggaaagaatg aggagccaaa gacaaagaaa 120

aatcaaagct atcagagttg ctgcagaggt tttactaaac aaagaatttt atgaagcagc 180

tgaagttcag tacaaaaaga ctccacccta cccagcttct gccgcgactt ttccttgctc 240

tgaaaactcc agcccacaga acagagctcc tctaactaaa atgcctttag tgctccgttt 300

taagcattca ctcttttctt attttaaacg tcccactctc acactcagcc aaaaggactt 360

attgtaggct ttcccagaaa aatttagtag tgtactctct ccaacggtgg atctagaagc 420

tccggattta caaacatccc aattgtcttt caagtcgttt tccaggtgca aatccgtcag 480

cccccttctc aaacttcaaa ccagaacctc ctcttcacgc tcttcctgag gcttaacatt 540

ccacctattt cccaaaccgt gcaataaaca ctacaagcaa agcaaatggc aaactcacca 600

tcctcgtctt cttcctgggc ttttattgag acaaattgcc ctaataaaac aataagaatg 660

aggagaggtc ttgcttccgc ccagtttgcc atcatgtcta aatattagac atgtgggttc 720

tcctgagagt gaaaagtaga ttctgaggtt attgtagcac catgaagtca gctgtgggct 780

cttctttcag caccagcccc agggcagcct ctgcaaaccc cctttttcag caccagctcc 840

agcacagtct gcgaaaactt ttttcaagcg atgcagcttt aaataggaag aatgctgcct 900

ccacttcttt tcctgcccct tttcagcttg ttcaggctca taaccatcca gtgtctgcca 960

agcaacggtc tgattgatgg taaacaacca aatcagaaca cgcgtctctg tgcctgaact 1020

ttccctattt catttaactt ttaagcatag atggggctta ataacagaaa caaacatttg 1080

tttctctttt cttttatttt ttaagaaagg ctgttagaag aaaagggttc tattttcccc 1140

tccctaaccc ttccccccag acacacaccc tttaactttt cccctataag ctgaaaaaga 1200

cattccgaag gaaatccagc cttttaccct agttttttta catttagata attcaagact 1260

ctcttctcca ccttaaatct ttaaacaatc acattttaac cctttctttt gactgaatag 1320

ggagtattct tatccccttc tcagattcgg tttcttttcc catactctgg aaggagatta 1380

ttagaatgtt gcccaggtaa agtccaggtt agaaaaccat tttggataaa tgaatttgtg 1440

atacattgta atttgaatta agaaagcatg tcctttccta atttctccac atctcttgtt 1500

aaggggccac atcttcaggt tcctcttgtc ccagagagtt ttaccatgtt gaaaaatagt 1560

ctactaagca gtgcagagga atgggaagta ctctaccttt ggatgaaaaa aggcttgctt 1620

ctgccttttg tgagtggttc tgccactcaa taattttgta aatgtgcata tagtccttta 1680

tctctgtgca ctccaatttt ctcatttgag aaaagggctt taccctgacc agtgtacagg 1740

gtttagtgac actaaaatga gtaatgtatt attaatgaag ctccaaattg caaagagcta 1800

cataaaagca gatggtggta ttattagctt taagccagct ttgcagggaa tatgatagcc 1860

ccagttgaac atgcatttta aggaaaatta tcgatgaact tttgtgccac cctcacactt 1920

gccaagatct taatgacctc agagagttct atacaatcta atgtatttgc caccttgagt 1980

ctccctagaa tcaggatgga t 2001

<210> 166
<211> 2001
<212> DNA
<213> Homo sapiens

<400> 166
gaagtataca agcgagggat ctaggttgcg tgctccctat aagaatctaa tgcctgatgt 60

tctgaggtgg aacagtttca ccctgaaacc atccctcccc atcccctcag tccgtggaaa 120

aattgtcttc catgaaactg atccctgagg ccaaaaatgt tggggactgc tgttatacag 180

agctgtagga gagaagaata atgcagaggg aaaaggagtg cctgtctgtt gagactttca 240

gggggaccta gggactggtt ttgttacagt tgactgggaa ttgtcttgtt tattttattt 300

tatttttcat ttaaaaaatt tttttgaaat ggagtcttgc tctgttgccc aggctggaga 360

gcagtggtgc catcttggct cactgcaacc tctgcctccc agataaagtg aagctcctgc 420

ctcagcctcc tgaatagctg ggattacagg catgcactac cacacctggc taatttttgt 480

atttttagta gagatgtgtt tcatcatgtc agccaggctg gtcttgaact cctgacctca 540

agtgatccac ctgcctcagc ctcccaaaat actgagatta caggcatgag ccaccgtgcc 600

tggccaaagt gtcttgtttt atttttaagt ctttgatcac tctcatttac agtaagtgtt 660

tgaagagtac attctaaatc atttttctct cttcctttac aggaagtgct caaatgttga 720

gcacacacac acaaaaaaag aggaaacagt tggataggaa ctcagaaatc atgtgactga 780

tgactaagtt aaatcttttc tgcttactga aaaggaagag tctgatgatt agttactgat 840

cctctttgca tttgtaaagc tttggagata ttgaatcatg ttaccatttc tgtttttttc 900

caccctgttt tcttccatat ttactgaagc tcagaagcag tattgggtct gcaactcatc 960

cgatgcaagt atttcataca cctactgtgg taagtaaaac cgcaaaacaa ataattgtag 1020

catcaactat tttgagggta agttttcacg agaaccgtac actgttgtgg ctggaacaca 1080

cgaaacatca gtgtgttcca gctgctgtgg cggacgctgc cagcaggaaa agcaatagct 1140

ggcagctgcc ccatgagaat gttctcgacc actcacagag cttgtgagtc tctaaactgt 1200

gttgtgcttg acctccaagt gcttctgttc cctgcgtcat ctttctctac cctgagccca 1260

gtttttggtt ctgcccattt ttaataaaac catctgcttc tgagctttca cctcagcctt 1320

gacacttact agttcttctt cttgagatgg tagagcagtc ccagctgctt cctggagttg 1380

atttctgctc taagctgtga ctcttgagtg ctcttggaat catccccctc actggatgaa 1440

agcctggttc tgtcactagc tgtgtgacct taagcaaagt actccagcct ttttagcact 1500

gaagtgaaag tgattttata gtctacttga taagggtgtt gtgaggtgaa gttttttagc 1560

atagggtcac atagagacaa taatactctt aatttttttt tggagtcagc cataccctaa 1620

tactattcac tggctgtgtc actgtcccct gccccagctg ctcattagca agtgtcctgg 1680

gatctgaagg cacagtgtca ggcctgacag ctgttttggt tcggtacttt ccaccgtgct 1740

gcttaccatg ggttggctgc ccttttccac ccactagcaa tagctgggag gtttaaagtg 1800

gtgcttccca aaataccagt tctttgagat tcttttggga aaataggatc tatgacctaa 1860

tcatctggga gagatttaca gatgtacttt gggttctgag aagttctgca ataaaggcac 1920

ctgtttaacc acattttcaa tttcatttgt tgtttgtttg tttgtgtttt agtctggagt 1980

gtatgttaac tctcttgaga a 2001

<210> 167
<211> 2001
<212> DNA
<213> Homo sapiens

<400> 167
tggccgccat tcccgtggct tccgtgggtt gacagcaggg attctgacaa gatgaggttg 60

ggggtagggg gtaggtatgg gaatgtgagt ggggtggcgg tggagatgag gagacaggtg 120

tgggtgggga accctcagtt ccctcccagg gtcctgaccc taatgccggg cggatgcctg 180

gaccttgatg cctgggccct gccctctcct gaccaaccct gccccggtca cataagctat 240

gactacagat tgccctgtgg gttaagtggc agtgggacgg gacggggggt ggtccagctg 300

agacatctgt cctggccggg tggtccaggg ctggcagcag gggtggtact ggatcatttt 360

ggggccatct acctggggtg tgtgcctcct cagtcctccc tcagcatctc accttgcctc 420

ctcacagagc ctaaaaccac tcccctcagt ggatctcaag ccgcccctca gaaccattaa 480

atgaacctgg cttctctcag atctgtgagg ttcaagtcag tggtttcaca tcgggccatc 540

cagggcttcc ctgggttgag agcaggggca aaccggattc tgccagaatg ggggaggcga 600

tgggagtggg gcacatgaga gagatgaggg tggggttggg ggcagtgagg gtgaggtggc 660

agaagggttg gggctggtgc tggggctgga gggtggtggt caggtgacgg agtaggagta 720

gggatggggg tggaggtgta gctgaggaga tgggggttgg gtggggaatg ggaatgcgaa 780

tggggtggga gtggggattg gagtgcagtc ttgggggccc acagccctcc ctcttgggtg 840

ctgatcttga tgcctggcag agcctgtgcc ctgccctctc ctaaccagtc ctgccctggt 900

cacagcaacc tctgattcca gagcttgggg agaagaacct ggtgtctctc aaggcttttg 960

gagcaggggt ctgggggcag cccaggctga gacgtcttcc cgcgggtggg ccagggctgg 1020

cagcaagggc ggcgctggaa tatttggggc tctctatttg gagtggcggg gtcctcagtc 1080

ctccctcagc ctctcacctt gcctcctcac agagcctggg tcaggttcct tcggccgaac 1140

tctacgccgt ccctcagaat ggaaacctcg cttctctctg ccccacaatg cgcaagtcag 1200

tggcgtcaca tccgggcacc cggatgtttc ctggcgggaa ccggatcctg cctgggtggg 1260

ggtgggagtg ggcgtggggg tgggggtggg ggtgagggtg gaatggctgg gcggatgggc 1320

atcagggttg agggatgggg tgggggatgt ggggtggggg ttggaatgac aatgaagagg 1380

gggttgggtc acgggagtga gcattggagt gggggttggg tcacgggagt ggggtcttag 1440

ggaccctcag ccctccttga gaatcctgag catgatgtct ggcagagcct ggacgctgcc 1500

ctctcctaag cagcccttcc ctgatcacac gagctctgac ttcagagtcc ccagtggttt 1560

aagcagcaag gggctggggg gtggcccagc ctgagaagtc caccaccaga tggtccagga 1620

ctggcatgaa gggattattt ggggtcctct atctggggta agtgggtcct cagtcctccc 1680

tcagcatgtc accttgcctc cttacagagc ctgggcccgc ttccctccac cgatctcaag 1740

ccgcccctca gaccgaaaac ctcgcttctc tctaaccact aatgctcaag tcagtggcgt 1800

cacatctggg cacctgggct tccctgggtt gagagcaggg gtggaaccgg attctgcctg 1860

gcttgggctg ggggtgagcg tgaagatgtt tggatgggtg gcgtaaaggg agggattggg 1920

ggtggtaggg ggagggatgc cagtggtgcc ccggggctta agtcgtcgtg ttggtggtag 1980

tggggttggg cgtggggttg g 2001

<210> 168
<211> 2001
<212> DNA
<213> Homo sapiens

<400> 168
cagggcacag ctggcaggca gagcctgaga ccccaggagt gacaataggt gggtagggcc 60

gggtgctgtg gggtctcctc tgtttctggg ggtagggccc ttggtacaca gccagggtgt 120

gcactcacct tgactcctgg cactgcctgg gcctcctctg ctgtgctgac tttaggctgt 180

gtgcctcaca cccagttcgt caccacctgg ttcctggagc acctgcaaga ggaagtgagg 240

gagcccctca ggctaaagac tgcaagtagg gtcttgggcc tgctagggct gacagctggg 300

gctggccaga atcccagagc ctcatagttc tggactgcat ggccccctca gaatttaatt 360

agcaacttca cggcttctca gagagggccg agcctccacc actctgctgg cctaaggcaa 420

aacctcagag caaccgccac atccctgaga gcagtgggag gtagggcggt ggcagccacc 480

tcccagcact ctaccatggg ggcagtcggg gtagcctcac gtccgttctg atgcacatgt 540

gactcctcag tcttatcccc agcagggcct gggaatcttc cctctgccaa ccagaggcag 600

ctccccgtgc tgacttgcca tgggaccctc agaccaaggc ctccgcctcc cctacgccac 660

tcacccagga ataagatggc tgccaccccg cctgagctga ctggggagct ctacgagggc 720

tgatacttga ggagcagggt gggaatctct gggaatctgc cctctgaggt gggggttccc 780

ccagccctcc tcagtgtctt cctatttatt ccctagaggg ccggcacctc ctcccttctt 840

ctggggccat ttccctgtct gggacctacc agtctcacgg cagcgcagta caggccgggg 900

tttcttctct ctatggtggg aggtccctca gttcttcctc agggccctca ccctgaggac 960

tggccaggtc taagactctt ccccccgcta acttgattcg cgcctcctca caaatgcctt 1020

cgatccgtca gattcccaag atggccgtcg ggaaagcaca tccgggaacc ctgccgtggt 1080

tgtccagggc tgacgcagcg tcaatgtggg gcggggcctc ctctctgagg aggaaccacc 1140

ccccacctct ctgctttttt tccatccctt cctccccacc tcccagctgc cctccaaccc 1200

ttcctgctca ctccctaagc cttgcggacc tcttcactcc cacctcccca gtccttgaca 1260

ccccactctc ctttttccag gggtgtcagc agggagtggc ttggtgcagt ctgcgcctcc 1320

cctccgtgtt gggaggtcac cgtccttcca catctctctt gttttttggc cagttctggg 1380

acttctttct ctgcttaact gaagctgccc tcctcagaga caacagcctc cccttctgct 1440

gactctcaaa atggaagtca gaaaattgta catctgggga ccctgatggg gtcctccaga 1500

gctggcagca gggacagggg aggggcacta ccagtcatgg gtgttccctt ccagccttcc 1560

tgagaggctg tgcctggact cctgatactg cctgggatgc ctgaccctgt gcatctgagt 1620

ctttcacctc cgaataagcc cctcccatcc ctgagacctg caaggtgcaa gagagggaga 1680

atcacatcca tccacagtgg gatagggcct cctagcccta aggcaggcct ccactgccct 1740

ctgaaatggg gtgggaatgc ccctctgtct ttctcaaggt atgactccta aaagagccta 1800

tactccttcc ttctcctgtc cagacagcaa catttgttcc ttcgacctct aaattccatt 1860

ccaagttttt ccactttact gctgtgagtg aaattagtgt catctctccc ataccctgtg 1920

ttaaaggctt ccttattgtt gcttcttttc ctatcctagt ccctgaaaaa aaccactgtc 1980

ttttcagcaa cccgtgaccc t 2001

<210> 169
<211> 2001
<212> DNA
<213> Homo sapiens

<400> 169
gctcgtaatc atcctctgaa cgcttctttt tgagagaaac cacaagaagg tattcaaaga 60

agtgctgccc cccagcaaag ttgggcaaag agtgctcctg ggccctttct ggttccttta 120

aagcttcccc tgaattgtcc tggggtggtc ctgaaatcaa gccagagccc atttagtata 180

atctcattga tccattcctg gagtggagtc tgtgagaatc cgggtgacct ccacacctca 240

aggccgcctt gccaatgctg agatacctgg atactggcca gaaccaccag tcactcagga 300

tcagggccaa atgggcagtt tccacacctg ccctggtaga cttgaaacca tggggagcca 360

agcagaggaa agaaaccact gacacagaag ccacggaaat gtgctggaga ggccacatgg 420

aggccaaggc cccagggtac cacagagact gcctaacctg tcgggtgctt ctggcagtcc 480

tggaacaata tggcctgaga gtttagttca tactaagtga ataagaacag gctcaaaggg 540

catgtgactg tgaccacact agccccagag agggcatgac cagctcttaa aaaccagcag 600

aggaagcctc ctgacccagt cacatgggga accatgtgga ggccgagata taaaggaaga 660

ggaggaagga gcatttcaag gaagaaacag tcccgcctaa gagggtcacc ctaaaaacaa 720

ggaagtaggc agtccagtcc tgacctgctc ggagttgaag cagtcggcgt tggaagagcc 780

tgaacacccg gcctaccact tgtccttcca tctctgggcc ttcaggacag agcggactcc 840

cctctcccct aacacagaca gactggtgac agtaagcctg agaaaggggc tgcttcggtc 900

tccagccaca actctgtgaa gccaagccac gcgctgtctt ccagagaggg aatagaagtt 960

tccatcctgt gcacccagtg gttgagcaag aaggatgtga cgggacccag gaaggcagca 1020

gaagccagca ccaacagagt cagcatcctg gcactgcagc gcagatctca gctcctccca 1080

agagccccag ggtgtggcga gagaaaaggt catgagcaaa cacctctggc tgaaagtagc 1140

cggaaaggcc tggctactta tgggctaagg gttttgaatc tgggctgcag attgtgcaga 1200

gagttaattt acaaccttag aacagtggac aatgcttgat agtgtcagac tgtgggcact 1260

ggaaaccggc tggtctcagg atagggatcc tatacctcca acacttggat tgctgacctt 1320

tcacttctca acttggtcaa cggaagtcaa aaaccccagc aaggccacct gctgaattct 1380

agaacttcca ggacttcccg acctagagca catggccaaa cactttctgc agccaggatg 1440

tcactgccct cccctgatta agataaatag acttggccta ataattatcc aaatagatta 1500

attcttccaa caaaagaaag agttctggaa gtcaataccc aatagccttc tttcctgatg 1560

gtgcagtaga gagcaaaatc catctctaag cccagagttc gtgatatttt ggaaatagct 1620

acaatggtga atttcacccc ctggagagtt ctgtcactgg cacttgttcc agggttccag 1680

cagagtccct cttctcaaat aggaagcact ggctcagaag actgtcccca gcataaggta 1740

ggggactgat tgcaccagca gcctgagggt gattccatgg cagtggaaaa catctgggtg 1800

atctccaccc aacccccacg cctgctcagc cttcctccgg gtcaggccag gtcagctgtt 1860

ctgcccaagc tacaggcaca gctgctagcc cactgctgtg tctggagact aggtaagaga 1920

gaaggaatag caaaaaaatc accttggatt ctcacagaaa cccctgaatg agtaaataca 1980

gggaaggctt ttaccaggaa g 2001

<210> 170
<211> 2001
<212> DNA
<213> Homo sapiens

<400> 170
aacatttcac aggggtttgg gctgtgagaa acatcctgcc taaccacccg accacaaggc 60

agacaaaggc ccaactaaag aaacatccct ctcatatctt cctgggcaaa ggtccaaggg 120

acaccatgat gacatcccac tggaacaagg gccaaaccgc ctcatcatga gaacatcttt 180

atatcaatat cctgctaggc agcaacccat actgcccaga cccctaccgc ccatacctat 240

aaattgccca gtctgtaagc agtggtgggc actggcatta ggctggttcc ccacttctgt 300

aggttattat gctggacata aagcctgcgt ttgctgtcga gccgccctct ttctgtgtgt 360

gtgtgtgtgt gtgtgtgtgt ctttctttaa cccttgcctt cttttcaaaa cctaacacga 420

gtagacaaac gttctttctg aagagccaga ttgtaaatat tattgtaggc tttgcaggct 480

atagctcttc tgcccttgta gtaggaaagc agccacagtc atacttaaag gaatgagcgt 540

ggctgtgtcc ccataaagct ttctttacgg acactgaaat tttaatttca cataattttc 600

atgtgctaag aaaatataca tatatatttt gtgagatggt gtgtcgctct cgtcaccctg 660

gctggagtgc aacggcgcga tctctgctca ctgcaacctc cgctacctgg gttcaagcga 720

ttctccagcc tcagcctcct gagtagctgc gattacaggc gcccgctacc acgcctggct 780

aatttttgca tttttagtag agacggggtt tcaccatgtt ggccaggctg ctctcgaact 840

cctgacctca ggtgatccgc ccgcctcagc ctcccaaagt gctggggcta caggcatgag 900

ccaccacgcc cggccaagaa aatatagttt ttttattttt tccaactatt tcaaaaggta 960

aaattcattc ttagctccca ggttgtccaa aaacagacgc gcaggatggc caagcaccag 1020

agtttatcgt ttgcccataa taatcgggtt gattcggcca aggtttccct cccccaacac 1080

ggtgcgcact gggaagcgcg aggaccacgt cccttggttt accactgcgc acctcacaag 1140

gcgcccagtg catccgggta gcgggatgcc ggttggacca acgcgagggt gggcggggtc 1200

taagagctaa gcgttggggg cggggcggga cagtggggaa gaggacgggt cgagtgctgg 1260

cttccggcgg aaaagcgcgc gagcaagatg gccaccacca agcgcgtctt gtacgtgggt 1320

gagcaggagg ggttgctagg cggagtctga gtgaacgcga cccccaaggg tcggggcgtg 1380

gggtgggacg catctctgaa ccaggaggac ggcgagctgc tgtcaaggcc gggtctctgg 1440

cggtagtgct ggcgatagct ctggctgtgc ttaaactcct tccaaggttt cccactgtcc 1500

tcaggaaaaa gtgcagactt cttagcagta cgcacttctc cagcttcttt gctttgcttc 1560

ccgattcgct gggtctcccg aaaaatgacc cacttgcccc caaggttcag gttatttcac 1620

acctttgggc ctctgctttt gcgactccct tgcagtgaac agccttgcca cacacctcag 1680

gtgcgtgaac ccgagacgga atgattattc tgacttccta gcccgctgag ctccagcggg 1740

ccccgaggag cggtaaagga gtattatggc ccttcggatc ctctcgttca tttgttttac 1800

aaacattcag tgcagcgacg gctcagcccc agcccctgcc ataaaggaag gagttcccag 1860

actagccaga aaaaaagtgg aattacctca gtagcacact tttgataagc gctgtagtga 1920

ggatggatca tgataatatg ggcctggctg cgggacaaat gacccatcca tctggagaag 1980

cgagggcaaa ttctggatgc g 2001

<210> 171
<211> 2001
<212> DNA
<213> Homo sapiens

<400> 171

actttcggac gagcacacca gctgtcagtt ataaatgata attaaggcat cttaattatt 60
aagcacagtg aaaatatgca gtgttcattt tttataaaac ttgtgtccta aggtagaaga 120
atcctcatac caaccaaatg ctctttctct acctttgatc agcaatttgg ccttttttct 180
ctctgaggct gataaattgc agcaaccttt gagatactag accctccacc cccaccccag 240
gtcttccccc aggtctcctt agttataaag gtagcagaat gtgatggttg aaagctgcat 300
ttgtaaccag cttaccttcg caacaatagc aagagtaaca aagaatccat cctaataaaa 360
gctctgtgca cagtcagctt aagcaacgta aagtgttcct ttattctgct tagtagcttc 420
tcccccagag gttaaccatt ttaaagctgg cactcagcct gagaacagga acaaagcatt 480
actagaaagc agggatcttc ctccaggatc agcatctctg ctttccgtaa ttcttaagaa 540
atatagaaac tcaatgaaaa ggaagtgata cctttggatg ggcacattcc agcacaggaa 600
aatcgctgtc ttgtcggctg ctgtgcagaa tgagttgctt gggcaggtat tcctctattc 660
tcagctcccg ggtctgcctt gtagccgcag ccagcctagg gagcatttac ggcatgtacg 720
tggtgttcat ccacaataaa aggagactaa ccagctcagc ttggcgctgc tcgcctggag 780
gcatcagatc cttggaccct ccccatctga ttggctgcta cccggtctag ccgaactcct 840
ctgccttaac cttctgctgc gttcaataca ttttccctcg ggatacagtc agagaagagc 900
tggttaggga atcagtggca ggttaaattg agctggcttt aaaaatcagt gacatgattt 960
tctatgaaga tgtctaatgt ttaattttcc aaagttctac gtaatccaca aggagagacg 1020
taataaaatg gatatgattt aacaggagcg gcttcatgat tcctccctga aaatacctgg 1080
agtttaatgc atgcctaaca ccctactgaa gtgatcaggt ggctttttgc agcactgcgt 1140
gtctgtataa tcaaccttaa atcaaaacag atggggatga cagaatatat taagaggctt 1200
attaaaagtg ccaggagatg cagttgaata aggctgctga agccatctca agaaagtgtt 1260
ataaatcatt tacacaaaat gctcttaaga cagttatggt tgattttaga tagctctgat 1320
aataaaaaca taaagaccaa tgacagacaa aaaggtaaag cctgacttag catctctaga 1380
agacaagtaa cagaaagaaa aatcaaatct tttactctac aaaagtagtg aaatgagaaa 1440
agaacaaaag tacaccttca tcatctcctg tccatgtact ggattcaatt tggtcaagga 1500
cctagaacag ggtattgaaa agtccccaat ggggttttgg tttgttttgt tctttctgtt 1560
tttttttgtt ttttgttttt tgttgttgtt tctttgtttt gagaccgagt tttgctctgt 1620
tgcccaggct ggagtgcagt ggcgtgatct ccacgcacta ccccctccac ctcccaggtt 1680
caagcagttc tcctgcctca gcctcccgag cagctgggat cacaggcacc tgccaccaca 1740
cccagctaat ttttgtattt ttagtagaga tggggtttca ctatgttggc caggctggtc 1800
tcgaactcct gacctcaggt gatccgcctg cctcaacctc ccaaaatgct gggattacag 1860
gcgtgagcca ccacacccag acagttttgt tttgttttgt tttgttttta aaaatgagag 1920
gcatcaaatg gaagatagtg aattgaccac atacatttaa ccctttctcc ctcacaaagc 1980
cccactaaaa tgacaataaa g 2001

<210> 172
<211> 2001
<212> DNA
<213> Homo sapiens

<400> 172
cctagagtaa gggacgcatt ttacactgct atccaaaact catcatagaa acatacacac 60

acaaaaccaa agcacacata tacaactgag caaatatttc atgacataac actttctctt 120

actaagggtg acgcgctgaa attttgtatt ctgtcctatt tcatttttta aaaatggtaa 180

ccatgacctg ctaaattgat ttcattgtcc actaataaat tatgacctca gtttcaaaaa 240

gattgcttta ggtaaccaat catcttctga gatttataca gattgctcat aattctctcc 300

tattttttaa aaacatgctg cagtgaactg ctttacactc attttatgac tacttctgag 360

accaagatcc cggattatgt aattgttatt tacttaaaat tctggtaaaa tgtagccatt 420

atactggaaa actaaatttt aatcttggat ctgtcaccac catgatatat aaactttggg 480

caagtccctg cacctctctg gacctcaatc tccccatcag caacctgctg atcctactcc 540

caggagtgtg ctctaagttg aaagtagatg ccccaccccc tgagtcagcg ccggcaggac 600

ttctcaccaa gcccttctcc cccttttccg ctccctgttc ctggttccta ggaagcagcc 660

caaggagaag ggaaaaggca ggtctgggca ggagggagca atgaagggcg gggcagaggg 720

agggcaggag ggaggccggc cccctagtag gaaatgagac acagtagaaa taacacttta 780

taagcctctt cctcctccca tctcctggcc tccttccatc ctcctctgcc cagactccgc 840

ccctcccaga cggtcctcac ttctcttttc cctagactgc agccagcgga gcccgcagcc 900

ggcccgagcc aggaacccag gtccggagcc tcaacttcag gatgttgaca acattgctgc 960

cgatactgct gctgtctggc tgggcctttt gtagccaaga cgcctcagat ggtgagtcgg 1020

gggcacatct cctgcctcag gatggttctg gagaatctca gtctatctgg gcacatggca 1080

agaccacagg agagcttatc tcacagcatc tgtgtctgca gctggctaga tctctctaca 1140

gggcaggcag agtcttgggg actggttcgt gtcccaaagc caaggtgagt tagtacattt 1200

aagcccctga aaagggggag atgaaagagg ctaggggaaa caggatgact ggaaacatga 1260

gaaagaaacc agcagagagg gtaggagaat cagccccagg gagaggggag aaaggggaac 1320

tgagggtgat ggtagatagg ggtacatcta ggggagacgg gaagaggctc agaagagaag 1380

agaaatggag ggaatgggaa gaccctggga aaactgatgg aagaagtggg ggaagagtgg 1440

ggcagagaga ggttagggga ggctagggaa aatggaagga gactggtcgc agctggtgga 1500

actggggaga aagagatgct gtgcctaata gaacttatgg gcgatcaggc tactgaagtg 1560

gccctgttta agcagaaaag ggagttatta ccctccatta taattgcaca ggggcctcct 1620

ttcccctctc tcacaatccc cgtaacttca gtctccccct cagagaggca gcaaataata 1680

accagtattc aatgagtgct cactatggtt aatacatgta ttgacccatt taacttgcac 1740

aaacccctaa aggtgggtaa tattattact atctccattt tatgaggagg aaactgggtc 1800

acagagtagt taaggaccat gtctagggtt atccataaat atacttattc acatctgcag 1860

atacaaagca caacttctca aatgcaaaca cagacaggac ccactcacac acacagattt 1920

acaaccccgg actcatccaa atgtgctctg ggcatcaact ctgtgccagc ctcttttctg 1980

ggtgtaggaa gcagagatta c 2001

<210> 173
<211> 2001
<212> DNA
<213> Homo sapiens

<400> 173
cccgtccctg gggtccttga cccaggctct tggggctagc ctatcttctg aggagcacaa 60

ggtccctggg ggctcaggga agagaaattg gagaaagggg gaggaagccc ccaagatgga 120

tcacccattg cctggtttcg caggagactg tccgccttca gttctccagc agctcgggga 180

tcatggccca ctgaaccccc aagcgctttc acccgaaccc aaggaggacg accaggaaag 240

acgggaactc gcgtagacac gcccggaagc ccttgtcatg taaatagctg tcggggactg 300

gtgtattgtc gccgccccag ccggcgggac ctggggcgaa tccacaccca ttgtctgctg 360

cccaaggggc ctccggctgg ggggcgcggc tgcggagttc aaaaggggta tgagcaggag 420

gggtgtactt ttagttcatt aagttttaat tacaggagtg ctacaagaac acattcttca 480

ggtttaaaaa gatattaaaa tattacataa gagacctccc ctccctggcc cacctccagc 540

ctcttaaaaa tttagtgtgt cgccttttag acactttctc aaagcttcac ttatttaaca 600

ggcacttaag gagcacctac ctgtgccaga aactctccaa atattaactc aacctgacac 660

cgactcagtg tggccgaata ttactctccc cattttacag agcgggcagc tggtcaagga 720

agtcgcttgt tgaaagtcac acagtggtgg agcctgtgtg ccaacccagg accctgggga 780

gctgcctccc cctctcccac gtagtcctga ttctttaagt gtccacatat tcctgtaatg 840

cctggagttt cagtaattag cagggactta gtgtgttcag agaaaaaaaa agcttttaaa 900

aattattgtt actgtgtttg taacagtttg gatagagaag gaaaagctgg aatttgggaa 960

gtgaaggtgg cctcggggta gaacttacct agaccagagc gaattcatcc tgaagaactc 1020

agagaaagcc ggtgcaggaa gtgggttccc gctctccctg cacaggcaca gtgatgctgc 1080

cagagctctc ccagaaagac caggaggctt gttctggaga agtcaagccc agggatgtgg 1140

ctcaggctgg tccaagctct ttggaggagt ccaagcgtgc ccagcccaga gggaggttca 1200

gaggcactga ccgtcttctg tttgggagga gaagctcact cttggagcca cagccagcac 1260

taggtcagga cccaggcccc ggcccaggag tggggcaata cccagcgtct accccagatg 1320

gcaccctgct gtgaactggg cgccctcagc ccctgccttg aggaaggggc aataccacca 1380

gcgtgtcttt tatcagggaa gatattgctg cagtttggcc gctgcaactt aagagaaaag 1440

ctaaggggtc ccccagcatc ccttggggtg ccactgcaaa tactggctgg gcctggagat 1500

gacctgggtc ccattcactt cctagggtga aggaggtcat cattaccacc cctgctttca 1560

gccatttctt cattcattca atcaacaaac tggctgagct gcaaccctga gccggggaat 1620

tcagccactc cagacacagc ccctgccctc cgggaagtct cgggagacct ggctagtctg 1680

gctgggagaa gtcacacgtt gattgtcttg gaagtgagat ggcatttaca caatggaggc 1740

tgcactgcca gcaggcaaaa ataaccagtt aattcagtgg cttaaagaaa ccaaacctac 1800

ccacaacgct tgacctccca ttgatccatc tgcgacaccg gcagtggcta ccatttattg 1860

agtgctgatg gtgtcacctg ggattgactt agtggtctct ggcgctagtt ccgaagttga 1920

ttctgtctgg agagcttaat gcagtgttca gacctcaggg tccgaacctg agggtcaccc 1980

aaagatgagt gggacatagc t 2001

<210> 174
<211> 2001
<212> DNA
<213> Homo sapiens

<400> 174
acacacacac acacccagca cacacctccc cacaatcctc agctgtgggg ggcggttgta 60

ccaaaggctt caagaagcca ctgggagcac agagcactcc caggctctgc tgtccccgcc 120

cagctggcct ctatccaggt agggacactg gccttcccca tgtgatcagt ggcagcaggt 180

gtggctgtcg ggggccctag ccttatctcc taggatcaac tctgaaacga caaccaactg 240

cactgctacc ctggccgcag aggtacagat gccactgttt gtgcctttca gtgcagtctc 300

tggtctacag tgacaaaagg actcaagagg aggatgagaa aagttgccgt gtaacttttg 360

cagggatgag cccaacagaa gcccttcacg gtctccctga cccctgccag caaaaggagc 420

actcctttag tccaccttta gtccacctaa gacctctcaa gtagctgcat ggctacaccc 480

ccgttcaatg tgacaaaacc accacaaatg gccaagggta tgacaattgc tagagaacta 540

caacaggacc tgtctctcac aacaaacccc tctcctctcc aaactaacaa gagactgctt 600

gccctcacag tgacctgatc ttcctggaga gaagcagcag caagcttctg ttcacaatta 660

cagggcaagg aatgtcgacg agggtgagaa tcattcctcc ccaggatcga caaaaggcac 720

agtggaattc tgcagagtcc aggaagtttg gagttgctaa gaaatttgct gctgcagaca 780

caagccacat agggccttac cagggtcaag gcatgaatct gtgggactga aggtgagtga 840

cagaagagag acacctgtgt ccaggacacg ctgcacagga atccctggga ctctctctct 900

gtcatgttcc tggttatcag gacggctcac cagctttctg ctgaggccag ctggacccag 960

gggagcagcg gggaaccaga aggagcaagg gcatggccat cgggacagac tactcacaag 1020

acctggctga gctgatggaa ctgctccaga gcctggcgac accagcactg ttgcttgtca 1080

ctgctgcacc ctgcacacag cgggctctgc aggagccggt agtaccggcg acgggcatac 1140

cggctgtcca gctccccttc cagttccggg tctgtgcccc cttccccctt cctcttactc 1200

tgcatataga ctctcaagaa gcacccatac agacgctgga tcatctcttg gaaggttctg 1260

ggggtgctaa agaacaagac tccgcgcaac atagtgtgga cttcttctcg cagcccctga 1320

gcaccagtgc ccatcagcaa gcccaggcga gtccatttct ccagcagctc taggctacgc 1380

aggtagggat ccaggcggct ctccagcagg ccaaaagcgt caaggagtag caaaaggcac 1440

tggggctcat ccgcagagtt ctcgcattgg gagatggcat tccagaactc aggggagatg 1500

ttggcctgca gatcgttctg cagcacctcc acgaaccact cctccaggac cgagtgtagc 1560

ccgtggcccc tcagaacctc caccgccgcc cggagctcct cttcctttgg cgggactgca 1620

ccgctggtcc gggaagacac ctggggtgca gaaaaccgtg aggcgagggg aacacaacat 1680

agaggtgggg agaggcgggg aagggaagaa gctgaggggg aggctgcaaa aactccgctg 1740

ccccctgtcc gtgctgcccg gacgttgggc cgaagctatg gaagggctgt cagcggagct 1800

gaacgaaacg aaacggaaag gtggtggaaa atggcaggac aggacagagg cggatgatgg 1860

acagagccgt atgaacccga gggtagcggc cccaaagcgc gtacggtccc ggaaactccg 1920

cgcggggccg cccctctctt cccaggcctc accagcccca gcgcagccgg cggcaccagg 1980

ccggtgctca cggtgttcca g 2001

<210> 175
<211> 2001
<212> DNA
<213> Homo sapiens

<400> 175
cgaggctgtg tccaggttca gtggggaaaa agtgccttga tttgaccatt atatcgtgta 60

ctcagggcct agtttatttc ccacaagcag ctatttaaaa catgtctcat ccaaggcagg 120

gtcggggtgg ggaacctcag aggatcaatg atgcagcctg tattgtcacc agggctcatc 180

ccaaagcaaa tcctgctgaa gttctcctct gaacaaccct tatcactcat gctaatatgt 240

tggatcctgc aagtcacaaa tacgaaggta tgagcattgg ccgaatccca acagggaaac 300

ttcaccggga aggtgaagac ccctttccat taagataaca aacagaacct agaaggcccg 360

tagcatggtg gcttagaaca tgtgggcctt ggatgggatc ctgccaaggg gtgtgtgacc 420

tctccgaagc ctccagggat gatactactc cctggggtgc ttatgccaac cacgttagag 480

acaatggttt ctgtacccat tgcctggggc tgccataata aagtgccaca cactgagtgg 540

cttaaaacaa cagaaaccta ttgtctcaca cttccggggg ccagaagttt gaaacccagg 600

tgtgttagga tcctgctccc tctgaaggct ccagggaaga gtgtcctctg ctccctccga 660

aggctccagg gaagggtctg tcctcttagg cttctggtgg cttgcaggtg cagccctcca 720

atcctcctcc ccaagcggcc ttctgcctat aaggacacga gtcatactgg atgaggggcc 780

cactaattga tggcttctgt aaagtcccca tctccaaata aggtcacatt gtgaggtact 840

gggagttagg actccaacat agcttctctg gtggacacaa ttcaactcct aataacgtcc 900

acacaacccc aagcagggcc tggcaccctg tgtgctctct ggagagcggc tgagtcaggc 960

tctggcagtg tctaggccat cggtgactgc agcccctgga cggcatcgcc caccacaggc 1020

cctggaggct gcccccacgg ccccctgaca gggtctctgc tggtctgggg gtccctgact 1080

aggggagcgg caccaggagg ggagagactc gcgctccggg ctcagcgtag ccgccccgag 1140

caggaccggg attctcacta agcgggcgcc gtcctacgac ccccgcgcgc tttcaggacc 1200

actcgggcac gtggcaggtc gcttgcacgc ccgcggacta tccctgtgac aggaaaaggt 1260

acgggccatt tggcaaacta aggcacagag cctcaggcgg aagctgggaa ggcgccgccc 1320

ggcttgtacc ggccgaaggg ccatccgggt caggcgcaca gggcagcggc gctgccggag 1380

gaccagggcc ggcgtgccgg cgtccagcga ggatgcgcag actgcctcag gcccggcgcc 1440

gccgcacagg gcatgcgccg acccggtcgg gcgggaacac cccgcccctc ccgggctccg 1500

ccccagctcc gcccccgcgc gccccggccc cgcccccgcg cgctctcttg cttttctcag 1560

gtcctcggct ccgccccgct ctagaccccg ccccacgccg ccatccccgt gcccctcggc 1620

cccgcccccg cgccccggat atgctgggac agcccgcgcc cctagaacgc tttgcgtccc 1680

gacgcccgca ggtcctcgcg gtgcgcaccg tttgcgactt ggtgagtgtc tgggtcgcct 1740

cgctcccgga agagtgcgga gctctccctc gggacggtgg cagcctcgag tggtcctgca 1800

ggcgccctca cttcgccgtc gggtgtgggg ccgccctgac ccccacccat cccgggcgag 1860

ctccaggtgc gccccaagtg cctcccaggt gttgcccagc ctttccccgg gcctggggtt 1920

cctggactag gctgcgctgc agtgactgtg gactggcgtg tggcgggggt cgtggcagcc 1980

cctgccttac ctctaggtgc c 2001

<210> 176
<211> 2001
<212> DNA
<213> Homo sapiens

<400> 176
gattctgatg ctacaaatga tagatatcgt tttagcattt tcttacgggt cctagcgatt 60

ctattcattt ttctttcagt ctctttctct gacttgttca cattgaacaa tttccttttg 120

ggataggttg ctatttctgt tttcgcaggt ggtttacctg tcttcccagc cagtcacagt 180

ggtccttgtc cccatggtgg gtccggggca agagagggcc ctgggttggg ggtggggttc 240

agttgaagat ggggtgagtt ttgaggggag cactacttga gtcccagagg cataggaaac 300

agcagaggga ggtgggattc ccttatcctc aatgaggatg ggcatggagg gtttggggcg 360

tggcgctggg aacggcagcc ctccccagcc cacagccgcg catgctccct gggctcccgc 420

ctcagtgcgc atgttcactg ggcgccttct gcccggcccc ttcgcccacg tgaagaacgc 480

cagggagctg tgaggcagtg ctgtgtggtt cctgccgtcc ggactctttt tcctctactg 540

agattcatct ggtaggtgtg caggccagtc atcccggggg ctgaagtgtg agtgagggtg 600

gagagggcct cgggtgggtc aggcgggtcc cgcttcctgg tctgtggcct ccgagggaga 660

agggccacga ggtcgtcctc cttcccttca caggctgcga ggccaccggc ggcttcgtgg 720

tcgtgaaggg gcctggacgg ggaggaaggt gggccgtgga ggggaggcgg tcaggggctc 780

aggtgaagac ggggtgagtg ctgttggggg gatggaagtc ccgaggtgcc gggaaccccc 840

gacgacacag ggcagattcc ctgaatgggg tccccggcgg gggcgaggcg ggcggtgaag 900

aaggggcctg gcacctggga aggctgcggc ctggtgagcg cccccgcagc ggtgtggagt 960

gcggagcacc cgagtgagaa gcactgcaag gtctcacctc cgccatggaa ggtccgaaaa 1020

cagtgggaag gagtgggcga ggcagtgcgg tccaaccaaa cttgttgtga gggggggtga 1080

atggctctag gaagtgggag tgtgcccaaa gcagcaatca cgagaattgt gattcactag 1140

ggttttcgtg gggagtgcac ttgtgaaact aaacctcatc agaaatgacc tctgtctgcg 1200

gggcgcagtg gcgctcgcct acgtagtccc agttactggg gacactgagg tgggaggatc 1260

ccttgagcgg gaggtggagg ctgcagtgag ctgtgatcac gccgctgcac tccagcctga 1320

gcaacacagc gagaccgcgt gtccaaaaga aatttagaaa aaaatgtcct ctgccttttg 1380

ccacacgcct taagatgatt gctctgccag cctggccagc agaagtggct ttgtaggcac 1440

tcagacagcg tacacacgta tgcttaactc tgggacttat cttgagagta ttttcaaaag 1500

taaaacggca agtttacatt tatccatgga agtgatcgaa tatagcagcc ctgtggagcg 1560

cacgttccca atcacggttg tctgttttca gtgtgaaata tgagttggcg aggaagatcg 1620

acctatcggc ctagaccaag acgctacgta gagcctcctg aaatgattgg gcctatgcgg 1680

gtgagtgctt aaacgttaat tcgatgtttt ctattagtag aaattaattt ttgtgatagc 1740

gtcgttgcat tagtgtggaa atgctgataa aggtctttcc tgctcataaa aaatgatgat 1800

ggcatctcat gaaggaaaca ttgattctgg aggatttttt ttttcctctc gtgttcttca 1860

gcttttgccc atgacttctt tctccggctt tgtttgttaa tgacagattg tacacatgta 1920

ttccaacaca gagtacaata gcctccaaag tcctcgtgcg tcacttttct cacagtaacc 1980

tccctgtggg tggagtaacc t 2001

<210> 177
<211> 2001
<212> DNA
<213> Homo sapiens

<400> 177
   taaatttgat caaattctct ctcgtgcagt tcaaagacat ctgccagctg ggtgcagtcc 60
   acaccgaaac gtacatcgat acctggcaaa gatagtttgg gggcgcaaaa tatagacagg 120
   aggatgcctc aagacatgga acagaaaaac tcttcctaac ttaaaccgtg cagaccagcc 180
   caagtctggg cacaaagccg gctttatgtg aatatccgtg ggacgatcaa gaacaatctc 240
   cacgtgggtt acatccccac gcccccaccc tttccacttt gggacgcgcc ggccgctggg 300
   cggggtttcg cagaggggcg ggctcgctac ctcgctcgcg caggcactgc agattctccc 360
   aggccagtgg atcccgagcc aactcggccg ggcgctggag gcaggtggcg gtaagttgag 420
   tgctctgatc cagggtttcg ctcagagcgg cggcgaagga gaaattcccc tccccaacca 480
   acaggaggcg ccgaggggcc atggcctcca cggactcccg gctcgcgttc tctgtggcgc 540
   tcgttttacg tcacttcctt cacggatctc tctccgcccc gtcttctccc gtcactagtt 600
   gatgacgcac ccatggcgcc tggttgtcta gacgagccgg tgtaccgact ccccggtgga 660
   tgataactat gagtgcaaaa gcaagacgcg cactttcctg tcacctcgcc ctttcacccc 720
   ctcgggaaga tcaaagtgtc tttagcccaa gcatggcctt gagcgacttc atcacacctg 780
   catgaggtca caagcagtca ctagcgcagg ggggtgaggc gagttctggg tgaaagaaac 840
   ggggcggggc tgaggccaag gaagaggttg gactgtgtct cgcatgctcc tgacgcagaa 900
   ggttttgaac cttttttcac ctcgtctgaa atggctgcct cccagtgtct ctgctgctca 960
   aaatttctct tccaggtgct taaatctcct atcttctatt cgttcaagaa agagtattga 1020
   gtgtctattg cgtgccaggc cactgtgagg gtggttacga tatgtggatg aacaaaatgt 1080
   ggcctatgat ttcaatgagc ctgcagtctg gtggggaatg cacagagcaa aacaggcaac 1140
   tctgatatgg cagggtgtgt gctctgggag cacacagaag aacgttacca agtttggggg 1200
   cgaagagggt cagagagtcc ttggatgaag taaacccgag ttgaaagcta acagggtaaa 1260
   agtgcatctt aactgagtca agaggcgtga agaggcagaa ggaacaatat atgcaaaagc 1320
   ttgatggaga aacagaatag ttgagtttgg ctgaaacaca attggaggga gagtaacaga 1380
   tgagactgaa gagttaggca ggggccaggt catagggagc gttgtaagcc tttaaaaaaa 1440
   atcaactttt attttacaaa tgggggtaca ggtacaggtt tgttacgtgg atatattgca 1500
   cccaggtagg ttgcaggttt gctacatggg tatattgcac ctaatagttt ttcaacccac 1560
   accacccgcc ctccccactc tagttagtag cctggagtgt gtgttgtttc catgtttagg 1620
   tccatgtttg ctcaatgttt agctcccact tataagtgag aatgtcagag gtatttatac 1680
   cagagcaact ccgtcttgaa taggggctag gtaaataagg ctaagacctg ggctgcattc 1740
   ccagtaagtt aaggcattct tagtcacagg atgagatagc tcagcacaag atacaggtca 1800
   taaagacctt gctgataaaa cgggttgcag taaagaagcc agctaaaatc caccaaaacc 1860
   gagatggtga tgagagtgat ctctggtcat cctcattgct acactcccac cagcgccatg 1920
   acagtttaca aatgccatgg caaagtcagg aagttaccgt atatggtcta aaaagtggag 1980
   gtatgaatta atccacccct t 2001

<210> 178
<211> 2001
<212> DNA
<213> Homo sapiens

<400> 178
tgggatgcaa gacttatgag ggcaaagatt ttgtaaaatt cactatgtat cccaacgttt 60

actgaccaat agtcataaga tgttttctta gttcattcac tcacctgctt ttctaataat 120

tatttcatat cgtctgtttt ctcaaattct gttcttttac atcttcacat tctgctaaat 180

taccgtgctt atttcactga tgaagttgaa gtgtttagaa tggaactcca taacctccta 240

ccatgatatt tttccactta ccagcatctg taccaacact ctgcctttgc tgtttctgta 300

aatgaactat tcatgcctct ctttgcagcc agtctttgtt tgcatgttca tcctgtagat 360

cccatcccct ttcactttca caaggagcca ttcttccctt tctgtcctat atcatcaatt 420

tccccatgcc tgctggatcc ctcctgtgag cttacataca tactgttatt tcttccatct 480

tgaaaaacaa acaaaaaacc tctcttgacc acacttcccc tgccagctac catcatattt 540

ccctcttttc ctttggagct aaactggttg gcaaagttta ttactttctt caattcctgt 600

tgttctctct gaaacccact ctaaacagaa gtttgatctt tccacactac cgaaactatt 660

tttataaaag tcactgataa tctctgcttt gctaagtcca aagatcaatt ctctgttctt 720

attttacctg acaaattcaa taatttgaca gatttatcac tctgtctttc tgaatatatc 780

tttttcatat agattccagg acaccatatg tttctcggat tttcctatgc tttcctgatc 840

actcttcctc agtctccttt gatgccttct ctctctctct ttttttcaac tacttaccta 900

acactggagt ggtccaaggc tcattccttg gacttctcag tctattctcg ccacttctgt 960

catgtcagtc agtcacacag ctttaaatac catctgtatc gaataatttc taactttttg 1020

tttctagcct aaaattccct cttgcattcc tgactcttaa acacaagtgc atctttgaca 1080

ttcctatctg gatgtttaac agacatctca agcttaacat gcctaacctg ctcctgatcc 1140

tttccccagg ctgggccctc cagtgaactt ctgcatccag ttggcagctc cagacttata 1200

gttgtcaaca aaaacactgg aatcatcctg cattcctctc tttctttgac acaccataat 1260

caatctgtca ggaaatcttg ttgcctttac tttcaaaata tattcagaat ctgacctttg 1320

ccctgcacct ccagctctac caccatcatg caagccacta tcatctccac ctgattttct 1380

ataatagctt gttaattgtc tctctgcctc tgtcctttcc tcctcttccg cctttgtcca 1440

ttgtcaaaag catggcctgg atgattcttt tgaaatgtga gtcacatcac atcactcttc 1500

tcttcacatt tctgtggtag cccccaattc actcagtcat ggcctacaat gctgtgccct 1560

ctccgtgacc tctctgatct catttttatt tattttgtct ttgcctaact ccactccagc 1620

cacactggcc tcttggatat tactcagaca agagaggcag cactccacct cagggtgttt 1680

gtactagttg ttccccctgt ccaaaattat cctccccaaa atactgacat ggaggacact 1740

ctcacctcct aaaaatctct tctcaattag aagttggatc ttgtaccctc aggactctca 1800

gttcctctta tcctactttg atttcttttt ccacagcatt tatcaccttc tagtatacta 1860

tatcattaaa gaatttatta tgcttattgt ctcttgtcat cttagtagca cataagagcc 1920

gtgagagtaa aaatattaat atgttttcat ttcattcgct ggaatatcca ataattgctg 1980

cccacactta ctatgtgcaa g 2001

<210> 179
<211> 2001
<212> DNA
<213> Homo sapiens

<400> 179
cagcccaccc gcccgaggcg gcggcgacag gacactcgcg ccaaccgaga ccccgccccc 60

aggggcccgc ccccccaggg ctccgccgcc aaccctgcgt ccccgacgcc cgtcaccgta 120

tgtccccgcc cacccccggc ctctgccccg cccgcgcagt ccagcctggt gtgggctccc 180

tgcaacccca ccccctaagc cgggtcccgc ccccacatgg ccccgccctt atctcttctc 240

agaccccttc catgggttcc aggcaccttc tcgccagggt ggcgaggaga aacaaaacac 300

atctgtcaca cagccacatg gcctcctctt acttggacag acaagcgtcc ccagcaaagc 360

ttcccgtcct ctcgataggt gactcgtcct aaatcagctc tgaattcccg caaagacttc 420

tggaagctgt gctggggttg aaactggtag accaaaacca gaatcaagtc tgttgggagg 480

ggcctttccc ctgggacggg aattccagtg gtaatgtctt cctggggctt ctccggaagg 540

tccaatttcc actccaccct gctcattctt tcaccatgta tttctcacaa acactttgag 600

ctggattgaa ggggaaacat ttgataatgc aatcattaat ggagtatctg ggtattcggc 660

aggaagtcag tatatctttc acactactgt ttagacttat tcaaaagaaa tttaaaagaa 720

acatgaaaaa gcctcatcta ctgttcaaca ctacttctta aaactatttt tcgaagtttt 780

ttcctagtgc caggcacaga gcagtttctt cataactgtt cattgcatga aaacaccaag 840

tgtgtatttc tgacaactca cttgagggga agggtaagaa tcagttctgt aagcacaact 900

ctgagctcgc acagagcccc acgatggggg cggggagctg aagactccta ttctaaggtc 960

tccaggagca aggccaaaag gaggttgtca gcagtatgca cgccgtgaag gaattccaga 1020

cttaagcaac agactgagaa gcatgaaaaa atgttttgtc aactaccata tctgccagtg 1080

agaacctgtc agagtaaaaa agaatctgag gctggccagt ccacccggac accttggcct 1140

ggatcatttc ttcttccata acaccagatt gatgagctgg gtacatcaac ctgaggcata 1200

agaaagctct ttcccaagca ccgttgaaag gcgcttcaca gtgcttcaac ctgtagcttc 1260

agcttcatca atctaccagt gctgcacagc tgtctttggt attgaagata gccaagcttc 1320

agcttctttt tcttcattca aaaaggacag gtcctcaatc caactttttc ttccaattct 1380

tctcagccaa ccatctcacc acttccttac ctatcacaag gccaccttcc cctgtgcact 1440

gagggccctt gacctcttta aggcgcccag tgtccacttc ctggacacac cttcccttac 1500

gaagttgaca gtatctccta agaattcagt gtacacaatg gtgccggagg aaatggcata 1560

acagaaggcc taagagacgg atggattcat gaaactttgg cttcacaaaa gatcaagttt 1620

ttcaacacac aggagattaa gtttaccaca tgattcattc aacactagta attttatttc 1680

aaaatataaa tatttcacat taataaagaa gttacatcag taggttgtca aagcaagtta 1740

gctaagaaac agcatttgtg tgagaacaga gggtatttat tgtattcgaa tattcttttt 1800

tacttttgaa ttgcctcctt cacctgagaa cccagggatg atacattttc aaagagttat 1860

gtaagcaatt acaactctat atcaaatctt agttcaaaat caaattgtac tggaaataca 1920

cttcagttac ctaaataagt ggcactgcaa agggcaaaga agggttttct ttgggatggc 1980

tacaatgaga gttacatctg c 2001

<210> 180
<211> 2001
<212> DNA
<213> Homo sapiens

<400> 180
gattcctcta ctcaagtact ttaagaaaag ttcctgattt ttccccttgt taagcccgag 60

aaggaggtgg gagggagaat gcctcccctc tgctattcct tggctccagg tttctcttta 120

agaaatgcaa tcagaccaca tactcctcag catctgagaa cacttcctct ggagtccagt 180

tctcctttcc aggcagctga gggaagagag actttaaaac tggcagaggg catcaggggt 240

gaaaggcaac cctgagagta ggaaaaggcc agggagacag gccgattcca gcaggggaga 300

ctggcccatc tggagcgcca gcacatgcct ggggatttcc tggcctcacc tgcagacacc 360

ctcccctccc tgagggttgg gatgctcaga gatcacaggg actctcaacc cactgctggg 420

agtggcccct ctcagacagg catttttatt atcaaatgag caatgagaga ggggagaggt 480

gaggaactct atgagaaaca tgcagtcttt tatcacgtca tcagttttca gtgagaaaat 540

ccagaccttc ttacttttac agagatagaa ttcagttctc agggacttgt gaagatgcag 600

catcatcaaa tcattgtgtg tgtgtgtgtg tgtgtgtgtt gtgttttcct tatcatccac 660

cttagaaaat attctccaca ttcccatgtt aaaatatttt ttcctaaaat ccatccatcc 720

acatgccaac atcctaaaga cagtaatata ggttcattgc agaggatttg gaaaatacag 780

aattgtgacc agaacaaact aaactgcatc cataatttta tcatcgaatt tgatcaacat 840

tttggtgaat agccaccagt ctgttttcta tctttttagg gaatataagt tccaagaggc 900

agggatgtca ttcacttctg tgactcagtg ctctgaatca ggcccagtga agactgacgt 960

gcagcctgta ttggcagaat gaatgaataa atactacaca cgcatatttt gaattgaaat 1020

aagttgtcag ggtgcagcag agaataacag aaatagaatt gcttggatgt gctgacctag 1080

atccaagctg ctactggtaa cgtactgtat ctccttgaat aagtcacttc acctctctgg 1140

gcctgagctg cttcttcagt aaaataatcc ttgtagctaa gatttgttga gcgctcacta 1200

tgcacactgc catgagcccc tatatgtgta tttctcatca tagctctatg agttacatac 1260

ctgccatctc cattttatgc atgagaaaat aggcatggag aagttaagtg gtttgcctaa 1320

agtcacacag caaatttgtg gagagctggg cttgaactca ggcatctggg cttttactct 1380

ctccagtgga atgcctgggc tgtgggatga gatgacttca caggcctctc ccagctccag 1440

agttgagaga gagagaggag tggtgcaggg aggtagtagg gatgattatg ctactggcca 1500

agttcaagaa agtcaattgt agaacaaggt ccagctgatt attacagaga gcaaagttct 1560

taactagagg acttcctcct ggaatgtgga gagtccacac gggcactagc agagcataat 1620

gaaagaccag gaattctctg ctgttcaatc agtgctccat cactcagagg cagggctgca 1680

agagctccta gtgtgtcccc ttcgccttca gggcctccct gtacagccac ccacaggaca 1740

gtgcaccagg gtcaggcagg gaagtaaata aaaaaaggaa tctgataatt cctgacagtg 1800

agcagcagct gatgctttta atgggggagg aagactggga atctcgcttg atgatgacct 1860

aggacttcag tcagcaggac actggcccct ttcctccccc tcccaggctg aaaccttgtg 1920

ttttgcctta tgtccatggt aaccaagtct cctttttggt gaggaggaat gggcttcagt 1980

aaacagatct atttgtgtga t 2001

<210> 181
<211> 2001
<212> DNA
<213> Homo sapiens

<400> 181
tgtagcagtt gccctgcccg ttcttgcagg tgaccttttc ctggaaacag acattctgga 60

catctaccag gggctcgtgc acaaaggtgt tcactggttt gcaccgcccc tgtgtcatat 120

tccggcgcct catcatttgg ttacagtagg tggagctgct gctgggggaa ctgtctgagt 180

ccatatgctg ccgctggaat ttcttggccc gggattcctt gcccagggaa ggctggaccc 240

agcccagcac cagcagtatc aggacaagca gaaggagccg gacaagagac ttctccagag 300

ccatggtggc ctcactttcc cagaaaagcc taattgagaa aaggagagag aaggaaaaga 360

gccctcttag aaaaagaact ccagctccca cctatggctt gcctggttta cactttctgt 420

ttcaggtcag ccagaaagac gtccatccta cttcctgagg tttttcgtgt aatgagacac 480

tcacgtgtac ccttttccac gagcgtttta ttcccactct ccaaagcgag gtcttcctcc 540

cctctaggtg gtgacctaca aagtgaccta aaatcctgtc agttcctatc tttgaatact 600

ggagtttctt tgatccaaaa tacatttgta cccagtgctc aaatggctca tcgaaacatc 660

agtttgaata aatcatgaac caaattgtat ccccgacatc cagggccatg ggtgacagag 720

ccttgttgtc ttggagcata agggaactca ggccagtgcc tgacactggc atcataaaag 780

attccattgt attccagaga gaaacgctgg cctcccccaa tttggaggct ccttcttctc 840

cagtcccatg gatgctctgc agcctgcacg ttccaccctg acccctctgc ccccagctga 900

tgtttgcctg ggccacattt tttcagcgct agcatcaccc aatctttctc agtgctgtgg 960

actggcccta gctccctctt ctgcaaccct gaagcagaag cggcacctga gaacacctct 1020

tgggctgctt acctggatct gcagcttggt gtctgagaga gatggaaggc cagtttctgc 1080

aatcactcag ctcccggggt gtggacctta tacagattac aaaggggttt ggcttccaag 1140

aggaaaaggg tgggaggagc atctggagct cagttcaaat aaaatcaagg acgtgaggaa 1200

ggagtggtga atcacccagg ccgccgtctt tctagggctg ctgaagaaag agcaaatctt 1260

tgcctacgtt agtctttctt cctatgtgaa ccacatctgt ctgctgatgc acaaggagca 1320

gcagagaaag ccactacagc actggtgccc aaacctggca cactaggaac aaagtccttg 1380

ttacttcttc gtgggcgttt tcaccaagta gacttggagg ttaacaaagg acgcaaagga 1440

gaggttctaa tgggagatag ggaattctgg ccaaagctat tccagggctc ctagggagca 1500

aaacccaagt ctctttctag gagggcattc tcccaacgct acctctcccg gggatgggag 1560

gcggggtcgg ggggaatgga ggctgtcctg ctgcccccag ctggaaggct tcccagatct 1620

ggagaagaaa gggggagtaa ctgaataggg aggaggagga ggcctctagg gtgcgctcac 1680

ccagttctgg ccacttccca cccaccctcc acccctgctc tgggggcatc gagaggatgg 1740

ggctgttctt atctacagta accactgacc ctgtaggtcg gaattgagta gaggaactaa 1800

agataaactt aaatcccaaa tctcaaattc tttttgggga aaaaatacaa agaatatcat 1860

atttattagc agcagaatgc cctcctagaa agagacctgt ttttttgctg agtaacaatt 1920

tggatacctc taagaattat caaaatatcc cttcgtgctg tgtattcaga ttgtttcagc 1980

ctttctgtgt aaaaagtggc c 2001

<210> 182
<211> 2001
<212> DNA
<213> Homo sapiens

<400> 182
gaatgggtag aataaaagtt tgaaattttc cacttcactt ctttgcacaa tctgaggcag 60

cctctgaaaa cacgatgcca agagccctag gtaatagcag gacaccagga aaatttggtt 120

ggttaaggca gtatgactcc agagttctgc taataacaac ctgaaaccac catagtggca 180
gaggaattac attttttaaa aaaaaaaatt ctttcaacag aacacaaaga ctgaaatagg 240
aggtcactac aaccgcggga gctgccgccc cgccctgcag ggagcacctg gcctgggacc 300
cgcaggcatt ctctacaagg ggtgcagctg tgcaaatgct cacaggtgac agaaacagag 360
catctcctgc ccatcacttc atccaacagc cagaggtgac gaagacgacc ctcctgagtg 420
aggactgagg gtccacaccg cccccccacc ccacacacca tagagggacc acagaatcca 480
gctcagcccc tcttgtcagc cctggtaaac gcaggcagtg atgtcaccca gaccacaccc 540
cttcccccaa tgccacttca gggggactca gagtcagaga cttggtctga ggggagcaga 600
agcaatctgc agaggatggc ggtccaggct cagccaggca tcaacttcag gaccctgagg 660
gatgaccgaa ggccccgccc acccaccccc aactcccccg accccaccag gatctacagc 720
ctcaggaccc ccgtcccaat ccttacccct tgccccatca ccatcttcat gcttacctcc 780
acccccatcc gatccccatc caggcagaat ccagttccac ccctgcccgg aacccagggt 840
agtaccgttg ccaggatgtg acgccactga cttgcgcatt ggaggtcaga agaccgcgag 900
attctcgccc tgagcaacga gcgacggcct gacgtcggcg gagggaagcc ggcccaggct 960
cggtgaggag gcaaggtaag acgctgaggg aggactgagg cgggcctcac ctcagacaga 1020
gggcctcaaa taatccagtg ctgcctctgc tgccgggcct gggccacccc gcaggggaag 1080
acttccaggc tgggtcgcca ctacctcacc ccgccgaccc ccgccgcttt agccacgggg 1140
aactctgggg acagagctta atgtggccag ggcagggctg gttagaagag gtcagggccc 1200
acgctgtggc aggaatcaag gtcaggaccc cgagagggaa ctgagggcag cctaaccacc 1260
accctcacca ccattcccgt cccccaacac caaccccacc cccatccccc attccccatt 1320
cccatcccca cccccacccc tatcctggca gaatccgggc tttgcccctg gtatcaagtc 1380
acggaagctc cgggaatggc ggccaggcac gtgagtcctg aggttcacat ctacggctaa 1440
gggagggaag gggttcggta tcgcgagtat ggccgttggg aggcagcgaa agggcccagg 1500
ccctcctgga agacagtgga gtcctgaggg gacccagcat gccaggacag ggggcccact 1560
gtacccctgt ctcaaaccga ggcacctttt cattcggcta cgggaatcct agggatgcag 1620
acccacttca gcagggggtt ggggcccagc cctgcgagga gtcatgggga ggaagaagag 1680
ggaggactga ggggaccttg gagtccagat cagtggcaac cttgggctgg gggatgctgg 1740
gcacagtggc caaatgtgct ctgtgctcat tgcgccttca gggtgaccag agagttgagg 1800
gctgtggtct gaagagtggg acttcaggtc agcagaggga ggaatcccag gatctgcagg 1860
gcccaaggtg tacccccaag gggcccctat gtggtggaca gatgcagtgg tcctaggatc 1920
tgccaagcat ccaggtgaag agactgaggg aggattgagg gtacccctgg gacagaatgc 1980
ggactggggg ccccataaaa a 2001

<210> 183
<211> 2001
<212> DNA

<213>  Homo sapiens

<400>  183
cagcggtccg gccgaatctc gccggggtct cctcttcccc tgcaccagcc agcgcctcct      60

ggctggccag tcccaccctg gctcaccctt cccgaagagc ctgccgagac cactcatcgc     120

gagctcgcct ctcccgcctc cgcccctcag cgtcctccag acttccgcct tgctcctggg     180

agggtgatgt ctcatcacac agggaccagc cttgcccaat ccgtcctcag ggcgccgccg     240

cgacatcaag aggcgcaaac gagaccaccg gtgtcacgtg acccaagcca ccagccccag     300

accgacgcgc tcctcttaaa gctgccacat cattttctat taagtaaaaa gacagttcta     360

tctccacctg agtacgtgct tgttctccgg aaggaaatgt gcagtgtgat ggcacctcag     420

tgtatggggt cagtgggaaa acttcatgct ctttacagag ctcaggaaag tggggtggta     480

taggttcaag cacttggggt tgccctaagt ttagatggca aacctgatgc agtctaatgt     540

ctcacattct ggtctctgca cctcaggaca accaacccca tttcacaggc tagaaaaata     600

gaaactcccc acactgtggg taaaatctct tctgtcacac acagatgaac tttaataaat     660

tacaaatgca cctgaaaatg ccttcttgat ttcctttcag tttaggcctc aaatgggctc     720

tcctcaaggc tggacctcaa aggcccagtt tgggccttcg caaatgtctc taacccttga     780

cttagagttt gaagattcat tccattctgg atgtgaatgc aggtaacacc tagaaagata     840

agaagtcaca tttcattaag ctttcagggt tctccattat tactctgacc tttgtgagtg     900

ccacctatag ggcaaactat ccaacctgta tgtatctacc taatccctcc tagggacccc     960

aaatggctgt tcctcatcac tcagtaccca gtttgctgac gtctacaata ttccgcctct    1020

catcatcaaa gaagatcatc tgggagaaag gaattccagt cttctgctgc aacctattca    1080

agacagggca ggagtaacca agctagtatc ttggtttccc aggcctctct gatactttct    1140

caccctgctc ctcccttatc tccgcatacc tctcaaagtg tgtgatcttg ctgcctggat    1200

agatttcccg atgaacaaag tacctgaaga ggtcaaagag ctccagtagc tggttggccc    1260

cttctatctc acttgtcctg caaaacggtg taaaagatgg gattagcaaa gtgaagggcc    1320

agggctacgt taacttatta agcaaagtta gtaagtataa ccagggaaat caacttgctg    1380

tttttccggg atggacaggc atcctcagcc tctgtttaac tccattgcct tcctatttga    1440

cacaattggt gctgggccaa tgttttagct tttgttttga gacagggtct agctctgttg    1500

cccagactgg agtgcagcga cccaatcacg gctcactgtg gtcacgacct cctgggctca    1560

agtgatcctc ccacctcagc ctcccaagta gctgggacta caggtgcacg ccactacaat    1620

tttttttttt tttttggtag agatagggtc tccctctatt gcctaggctg gtctcaaatt    1680

cctgggctca agtgatcctc ccacctaggc ctcccaaaat gctaagatta caggtgtgag    1740

tcactgtgtc tggccccact tttttttttt tttttttttt tgagacattg tctcactctg    1800

tcacccaggc tgaagtgcag tggctccatc tcagctcact gcaacctccg cctcccgggt    1860

tcaagcgatt ctcctgcctc agcctcccga gtagctagga ttacaggtgc gtgccaccac    1920

gccctgctaa tttttgtatt tttagtagag acggggtttc accatgttga tcaggctggt    1980

cttgaactcc cgatctcgtg a                                                2001

<210>  184
<211>  2001
<212>  DNA
<213>  Homo sapiens

<400>  184
gaccaggaca cggaatgtgt gggcttgcca atattttgat atataaatca actgttttca      60

ttatggaagt aacaaaacct caagggaaat agaaaaaaaa attccctgta atcatcccac     120

ccagctagga ggtctactgt gtgtgttttt gacattttgc tgtcctcgtt ggtgcaaggg     180

cagatgtgac tgtttatgcc tgtggagggc agaggggttt ttcttcttct tgtctttttt     240

tagagacagg gtctccctct tttgcccagc ctggatggaa tgcagtggca tgatcccagc     300

tcactgcagc ctcgaacccc tggcctcaaa ggatcctcct gcctcagcct cccaagtagc     360

tgggactaca ggcacctgcc accgtgcctg gctgatttat tttttatttg tttgtttaga     420

gatggggtct ccctacattg gccaggctgg tcttgaactc ctggcctcaa atgttcctcc     480

tgtcttggcc tcccaaagtt ttgggattat aggcgtgagc catgttgtcg tcattacagc     540

tacattgaga tataatttat gtgccacaca atgcatgcct ttcaagtgta cagttcagtg     600

gcattgaggt atatccacag aaccgtacaa ccatcaccac attgagaaaa tttttatcac     660

cctaaaaaat cctttacaaa atcctttacc ctttagcagt caccccatgt tttgattttt     720

gctttacatg attttacaag catcttttcc tgttgctgga aaatctcctt acccatcctc     780

tttggtgact gtcagcaaag gctttcgggt ggtgaaatag cgtggccagg agtgctctag     840

aacaatggtg ttggtgaggg ctgcagaagc tgaggaccca caggcagaga gggagggatg     900

gtagaagcag gtggtgagga gggactcggg gcaggacttt gggggtaaaa ggaagcaagt     960

ctctggaagc agaaccagca gcactgctga tcattgggac gtggagaaag gcgaaggctc    1020

agggtgtcag caaacttcct agggtgggtg agggttgcca ggtcaataac gaacagggtc    1080

ccaggaaaag gcggcagcac aggtttgggg agaattagag atgagcttgg ttttggtctt    1140

gttggatttg ggggcaccag tctctctcct ggcgagagtc ggcagtgggg tgttaggatc    1200

tggccctcag gaggctgtta gggctgaagc aagagatttg ggagttaagg gtgtgagggc    1260

cacgtgggag gcctcgggag cctgtgccga tccagggtga gtgtgaagga aagaaaaaag    1320

agggcccggg gaacactggt attttcgggc ggtggacgag gatgggacgg ccacgagctt    1380

gtcttaaggt agaaggcaga gcggggccgt aggggcagtg gcgcgggcga gggtggagaa    1440

cagcgctgag cctcgctggg aggcggccgg agggggctgc agcccccgag ggctccttgc    1500

agtccggccc cgcagcctgg agacagctcc ggtggtgctg cgctgcccga gactgctgca    1560

cgagcgggga ctgcggcctc ggctccctgc ctgcccggca gccccccatc cagtcggcct    1620

gcccggggtg cgcagggtta gcctggagtg agctcgcccc gcccggggtt gcgcggccct    1680

ggggggtcct ctcgcggcgg ctcctgggac ctgctggcgc cccgggcaga gcccggcccc    1740

atcccaggag tcgcccggac tggaaggcag tgcgagcact gggacacagc ctccgagctc    1800

gagcgtgacg ggaagttggg gcaatttgtt agttatccgc cgccaccaag acgcggcacg 1860

gcgcctggac cggaggggcc ccgcgcgggc gcgaactttg ggctcgggcg agtgggtggt 1920

gctccgccca gcccgagacg ggcgggcgcg cgggccaatg ggtgccgcct cttggccgcg 1980

gggggccccg acccgtgggt c 2001

<210> 185
<211> 2001
<212> DNA
<213> Homo sapiens

<400> 185
cattcacaac aacccattaa tagatgaaga aattgggctc agagaggttg ccatctgtcc 60

aaagtcacac aggatgagat ggaactgggg tttaaaacca ggtctgttta tcctacaaag 120

gatggaaaag aaccaaacca aggtgctttt cccagtctca tgcagcactc acacttctga 180

cacccaaagg tgtgagagtt gtttctccat gcaccaagca agtttctaac agacaccaag 240

tgagtgtcct ctaattcagt tccgatgcta tcttctggat gatagcatca gattccacaa 300

gttaaggcac aagactgccc cctcctcccc ctcaccactg cttcagatgc tgaccacaag 360

tcccaggttg tggcctgtgc ttctgaccaa ccagctgtaa atcaggttcc cacaaaaccc 420

ccctcaggtt tgattaattt gatggagtgg ctcacagaac tcaggaaaac acttagttta 480

ctggtttgtt aaaggatatt acaaaggcta taaatgtcta gccagttgga agagatgcac 540

agggcaaagc atgtgggaag gggtgtggag cttcagtgac ctctccaggt ataccacccc 600

ttaggaagct ccatgggttc agctgtccaa atccagttct tttagctttt tatagaggct 660

tattgtgccg gcctgattga ctaaatcatt ggccatttgg aatcaactca gcctttaggc 720

ccccacccct ccctgcgggt catgaggtgg gtggggacaa aagttgcaac cctctaatca 780

catgattgat ccccctggca acaagccccc atccagaggc tatccaggag cccaccaaaa 840

agtcacctca ctggaacaaa agatgctcct ctcacccggg aattatcaag ggatttagga 900

gttctgtgtc aggaacgagg gtcaaagacc aaatatttga acaaaagatt agcaccccta 960

tctacgaggg ttttaggagc tctgcgtcag aaactcaggc gtagagacca gatacatatt 1020

tcttactata tcatcagacc acgtctttaa agcccgtttt ctttgtacac actctactac 1080

actctaattg ttagctgttt aagtattaga aaatcattcc ttagaggcaa aatggtgtag 1140

tatggtgaca gtgtaagcct gtgctctctt caccttccta caactaagtg gtgatgtcaa 1200

gaggaaaggc taggtgtagg tttgagatcc gcatttggca gcaggggaag ggaagggaca 1260

gagtgagccc aggaaaaatt gtgaagtggg cagagaatct tcactcccct tcagactctt 1320

tccccttctc aggcagctcc cccacctctg cactcatttt gccccttggt cactgtagag 1380

accccaccca atgcttcgga catgatctag acttatctgt tcgcactgcg tttggttata 1440

tctgtcccca cttgccaagg tccttcacct ttgtgggcct tgaggtcatt gtctctacca 1500

gctggtggcc tcaggcaagt gactcctctc agggtatttg agcttggagc ccctgaattt 1560

tcaatactaa tgcccaaaga acatgagtct aaaatggggt tgtctgactt ggagataaga 1620

gcaaagaaag aatcctttgg tctcccaatc tacagagctg tggatagaaa catttactaa 1680

tagacagcca tgcttcaaag cagtgtttct catcattttg gctggtatat ggacaaccct 1740

cctccccatt gtcaatgcca gcagatggaa gtgacacagt ccatctgttg gcccacttca 1800

ggggccggct gtgtcttctt actatatctt ccttctaaaa tacactttct attggagact 1860

gctctagtga aggtgacatt ctgaggtgag gtggactctc tactgtccct tattccacca 1920

gccctgaatc caggagacct aggagggggg tgctctgaag gtgtgaattt tttccttgtt 1980

gcaggcacat ggtaattgct a 2001

<210> 186
<211> 2001
<212> DNA
<213> Homo sapiens

<400> 186
cctacgaagt aagcaagctg tgattccaac ccagatttgt gggactccaa agcccactac 60

taatccactg gaccgtgttc ctggaggcca caaatgctct ttcctcagct gtcttctcag 120

atcctagaac gggctgatat atggctcaaa agttctttaa cggccaggca cggtggctca 180

agcctgtaat cccagcactt tggaaggcca aggcaggagg atcacctgag gtccaggagt 240

tcgagaccaa cctggccaac atggtgaaac cctgtctcta ctaaaaatac aaaaattagc 300

aggatgtggt agcaggcgcc tgtaatctca gctacttggg aggctgaggc aagagaatcg 360

cttgaacctg ggaggcagag gttgcagtga gccgagatca caccattgca ctccagcctg 420

ggcgacagag aatccgtctc aaaagaaaaa aaaaagttgt ctaacatatt aatacaactt 480

tcaagccaca tgcctcggtc taaaagtagg tgcccccagt acacttttac cccaggtagc 540

aacttccact caccttaaag tcacatacct ggaggaaggg gatagggagg aaatctaaac 600

atcttatact tgaatgtaag ctccgttatt tcacggattc aaatgtggtg aaaggaacta 660

tttttttttc cttttttttt tgagatggag tctcactctg tcgcccaggc gcgatctcgg 720

ctcactgcaa cctcccgtct cccgtgttca agcaattctc ctgcctcagc ctccagagta 780

gctaggatta caggcgccca ccaccacgcg caactgattt ttgtattttt agtagagaca 840

gggtttcacc atgttggcca ggctggtctc aaactcctga cctcaggtga tccgcccgcc 900

tcggcctccc agagtgctgg gattacaggc gtgagccacc gggaccagac ggaactattc 960

ttttccacaa aagaacaaac acatacataa cttttgccac gtctgaagac gagggttcta 1020

atagggcgcg ggtggtgtct caggtatttt gaaaggagct acccaagaat ctcagcttgt 1080

caggccagtc attccatttg accctatctg gcgcgtattg gggccccgac aaggtaaggg 1140

gttggcatga agctgcacag tgagataaag ctggaccgga gctagaaccc agaatcaaag 1200

ccttgccctg tgcggtttct catcgcctac gcagtcgggc cctggcctgg catctccagg 1260

caggtggcct tggacggaag cccggcctac tggcttcatt tgtgggaccc cgggggtcct 1320

ctcctccgcc catggagcct ggttactccc acttcctcaa gcctagggcc tggagaagcc 1380

tttcctggtt cgcggcctca cggtctgcaa ggtcctctta cttccctgaa gccgctccag 1440

ccccacttcc cgaaggtccc gcttccgggt ggctcgcttc gccacctgcg gtcccctgcc 1500

tcggcttggg aacgccaagg acatccccca cggaagcctg ggcccacatc cccatgccct 1560

tgccgcacgt caccgccccg ctggccccga atacgcctcc gccctgcact tcctctctgc 1620

agcgccgtct ttccggccgg gctcccttct aattggccag ctcagagctt cctcgggtac 1680

tcggccactc ggggcatcgc ggcggccttt ctagccgctg tcccaagggt tggtctcgcg 1740

ctttcggctg cgagctctct gtggtgctgg cagcgacatg tggcgcctcc cgggactcct 1800

gggccgaggt aagggacgtc caagcaacca gcgtcactct gggccgcaaa gaccgaactg 1860

gagtgggggc gggcccgagg aaacagccga agcgatcgtg ggccgggtta gagccaagga 1920

aggcggggct tgaaagaggg cggggaagtc aggggctgct ggaatttgag atgtggatgg 1980

gcctagctag aagagggtgg a 2001

<210> 187
<211> 2001
<212> DNA
<213> Homo sapiens

<400> 187
cgtggcatat caccaggaat gccactgctt aaactcgtct gaggctggga agcccaccca 60

gaccaacagg cgggccctct ggcttccctg gagggagctc tgtccccagc actcagagaa 120

cagagtcccc acagcaaagg ccctcagggt gcaacggccg tgtcggaggg ccccttccca 180

tcttctcttc ttcagcacat gtgcccagct cctgcaccca atgcccaggg gatgcctccc 240

agatgccagc agcatggtga caggggcagg aggtggcatt gtccttgttc tggatatccc 300

tgggtgaccc cggtacgtga ctcccctgaa ctccagcggc ctcatctgtg acacaggggg 360

ccgggccaca tcatcctggg cagtgctgct ctctgcaatt attgagtctg gagtctcttc 420

ttgtcagaga ttctctttag ggagtttatt ttccatagac taaagactaa acaatagaga 480

acagagagag gcttctagat acctactgca accttagctt ctcagaaaat acaggtgctt 540

gggactcttc cacaaaatca ggaatttaaa tggaaaacgt gggccccaag acagctcctt 600

tagtcaacgg gtctgagtac acattaaagg agggtggagg gagaggggac atagagaggg 660

aggaaagggg aggagggcac caggaagggc ggagccaagg gagtcctggg ggtggatacg 720

cacctgcctg taaggggccc tgtggtcctg gcacgtgcct ataagaggcc ctgtcatcgt 780

gtgttaggtt gtgacacagc tctgccctcc ccaggtgcct tttctgaacc aggaacctaa 840

gaggagctct cccaccaggg ccagggaagg agggcaccag gacgggcaga gccaaggggg 900

ttctggaagt ccccggccct ggtgggagag ctcctctctt aggttcctgg ttcagaaaag 960

gcacctgggg agggcagagc tgtgtcacaa cctaacacac gatgacaggg ccccttacag 1020

gcacgtcgta tccaccccca ggagcacaca gacagactct cgtccacatg ccctgtctgg 1080

ggacaggagc ccagcaccca atgcttgttc ctgcttccac ccctttgctg acctccccac 1140

tcatcagggc tgcgtgccag gtgaccaggg ttctaaccct ggcttcctcc ctgacccctc 1200

ctcctggaaa catccttcct gacccagcct cagtttacct cattgaaggg tgaattttaa 1260

ggacccattc agcttttatc tcctgtcact ccatagacat caagacttaa catcccatcc 1320

ctcccactcc cagctgtcat tccccccccc caatcttcgg cccactccct gtccaggggc 1380

ccaggtcccc agcgcgggca acccctctgt ccccattccc gggacctgga gcccgcgagg 1440

gctcaggccg gccaggatgg agttctctat cccagttcaa tttcccccgc ctttttgatg 1500

tcacatcctg tcccagcctg cttccccgcc ggccgccgcc ctcctccccg ggagagagcg 1560

aggcgcgcgg gtccctctgc gccacccccg cccccgcccc ttccgagcaa acttttggca 1620

cccaccgcag cccagcgcgc gttcgtgctc cgcagggcgc gcctctctcc gccaatgcca 1680

ggcgcgcggg ggagccatta ggaggcgagg agagaggagg gcgcagctcc cgcccagccc 1740

agccctgccc agccctgccc ggaggcagac gcgccggaac cgggacgcga taaatatgca 1800

gagcggaggc ttcgcgcagc agagcccgcg cgccgcccgc tccgggtgct gaatccaggc 1860

gtggggacac gagccaggcg ccgccgccgg agccagcgga gccggggcca gagccggagc 1920

gcgtccgcgt ccacgcagcc gccggccggc cagcacccag ggccctgcat gccaggtcgt 1980

tggaggtggc agcgagacat g 2001

<210> 188
<211> 2001
<212> DNA
<213> Homo sapiens

<400> 188
ctccggcagc ttgtcgcccc acgcttggga ggccagtccg cacgctcggt cgcgggccgt 60

gcgccctcag atgaagcccg tgcgggccgg ccccagcggc gccccacctc cgggccgcct 120

cccaggcccg cctcggccct cctgcctctc agcgatcgcc acctcgcgac ccagcctgtg 180

gacgtgccgc ggcgcccgcg ccggcagccc ggggcctcgg accggttcct tcccgccgcg 240

tgaggctgac gcaccgacca gcttcccggc tcggccccgc ccggtccgct gcgcccgcgc 300

ccgtccccgc ccccggctcc ctgacgcccc cggcctggag acgcaacccg acgcgcacaa 360

ccccagaccc tgcgcgggag tcgggggtcc ttccagttcc cagagcgaga gcgaaagccg 420

gaggggcctg tgttttcctg cggggttggg gggcgggggg tgcccgcccc gcgccgcccc 480

gcccagcccc agcccgctca ctcccggcca atccttccgt atctcaatgc gagacaatca 540

aatcgtacga attacgtgtc caccgtgttc ccggcggagt cttaaagctg aaaaggatat 600

tagagagcat ctagaccagg gcagcatatg tcatttatat ttttagtagc cacagcaaaa 660

gagtaaaaat aacaggtaca ttttattttt aaaatatttt tatttaaccc cgaatgtgca 720

aaatattatt tcaaaatata accaacatta aatttattca taagatattt gacttttttt 780

gtctttgaaa tccagtgtgt attcgatgcg aactagaccc atttcatagt caaatgtggc 840

tggctagtgc ccactgtatt agacagcgag ggtggaagcc acccgctcat atcataggaa 900

gaaacaggcc tagagaagtg aggtaaggtg agcaaggatt ggaggtcctc tgatacccag 960

tcctggtctt ttaacattca gagttgggac tagtctatcc gctttttttt tttccagaca 1020

gggtctccct gggtcaccca ggctggcctg caatgatgcg atcgcagctc actgcaacct 1080

cgaactcctg ggctcaagcg atcctcccac ctaagcctcc tgagtagcat gaactacagg 1140

cgtgcgccac cacgcctggc taattttatt tttagtagaa acgaggtttc accatgttgc 1200

ccaggctggt ctcaaactcc tcaagagcga ttctcccgcc tcagcctccc aaagtgctgg 1260

gattactttt tttttttttt tcgaggcgga gtctcgctct gtcgcccagg ctggagtgca 1320

gtggcgtcat cttggcttac tgcaatctct gcctcccagg ttcaagagat tctcctgcct 1380

gagcttcccc agtagctggg attacaggcg taagccacca cacactcggc taatttttgt 1440

attttcagta gagaaggggt tttgccatgt tggccaggct ggtctctagc tctgagctca 1500

ggtgacccac ccatctcagc ctcccaaagt gctagaattg cagacatgag ccaccgcacc 1560

tggccttact ttttgttttt tgtttgtttg tttgtttgtt gttttaagaa gacttttttt 1620

tgagacactc tcacttcctc acccaggctg gagtgcagtg gcgcaatctc gggtcactgc 1680

aacctccgct tcccgggttt aagtgattct tacgcctcag ctacccgagt agctgggatt 1740

acaggcctgc gccaccatac ccagctaact tttgtatttt tagtagacat gtggtttcac 1800

catgttggcc aggctggtct cgaactcctg accttaagtg atctgcctgc ctctgcctcc 1860

caaagtgctg ggattacagg tgtgagccac ttcaccaagc ctaagaaggt agtttctagg 1920

tgaaactggc agctttccga catcttgcat gcacattaaa agttgaaata gttcaaatga 1980

cttaaactca tgtattttac t 2001

<210> 189
<211> 2001
<212> DNA
<213> Homo sapiens

<400> 189
cgtccgcggg cgtcaatagc gactttcagc acaaaacaaa gatggcggcg gcggcatctc 60

ggaaatgccc ggatgagact gctaacccct ccgacgcgct cggccccgcc cccttgggaa 120

cggtctctcg ggttgataag ggacgcacgc ccgaagaact gcctttaacc agcactgggg 180

acagcggcac gcaggtggcc ggggttggcg aacagccact gcagccgcgc ctgcggccgg 240

gaagcgagca ttttcgcgcc ctgcacaccc agcagccgca ggaggcggcc cgaggcgcgc 300

cgcgggggag ccgggggcgg ggcgacagtg ggaggcgacg cagccgagcg ccggcgcccg 360

ggggtggagc ctccgcccgc tcccaggctc ccaggctgcg gggcccgccc tcgggcgcca 420

ccgcctccgc ccgcgccgca gcaaaatgag ccgggccgcg cgccccagac cgccgcgcgc 480

tcggccgccc ccactgccga gaggcccgca gtcgggccgg tggcgcacga gccgggcggg 540

cgaccgcggg aggcgtccgc gtcatctcgc gtcacctcgc cccgcccgct tcccaggctt 600

cgagtcccgc cctgcgcgca ggcgcaccgg cgctgctcgg atcctccctt ttcggagatt 660

tgaatttccc ccagcgaggc gagtgaggcg aaatacccgt atggtgatag ctggcctttt 720

cgcgccaata ctgtaagtgc tcaccgcagc cctgggaggt gggtcccggc ctcgccccca 780

cgtttcgcct cctctaggag gccgcccggg cgcggcggcg gcggcggcgg cggcggcatc 840

cgccctgcca cgcgcaggcc gcagcgcggg gacccaagct gctgcccggg agcggcagtc 900

gccagtagaa atggacagac cggtcctgtc agagcctgtg gcttttgttg taaacgtcta 960

gaagttcagt taaattacaa gatctaccag atctaccggc gggcagaatt attactttga 1020

agttactgaa gttatgcgtc ctgctggaag accttgtaat ttaatgcgtt agtaaagaag 1080

cacacgtcac cctctcacac attttcaaca atatttttaa agtcgcctct caacatatta 1140

catgtatttt gtaatcgtct gtgttttagt tcatgcagtt ggaagtatcc atttgagaag 1200

ggacaaagag aagaattctt gcctgtctgt gccaccacct gctctttgtg accttggcta 1260

gttacctcac atatgtcccc aagtccccca cccatggtaa ataagaaaac taaggcctga 1320

tgcgttggct catgcctgta atcccaacac tttggaaggc tgaggtggga ggattgcttg 1380

agcctagccg gggcaacata gcgagacccg gaggcatggt ggcacacgcc tgtggtccca 1440

ggtacttgga acgctgaggt gggaggatgg cttgagccag ggagttcagg gctgcagtga 1500

gctgagatcg cgccactgct ccagcctggg cgacagagca agaccctgtc tcaaaaacac 1560

acacacacac acacacacac acaaaagcag ctgctggttt ccaagggcct tgcctggaat 1620

cagtccagaa gctaaattaa tgttaattga tattccttta tccacagaaa gacagcaaga 1680

aaggtcatgc ggagcggggg acataactaa ccataaatgc taaaggcctg gaggtgacag 1740

tgcctgctgt ttaaacgcgt gcgggagagt ctggggcctt tagccaggag attgtcaggc 1800

ttcccagggc ttccctgaaa gaccccagac ccaggggcca gtgctgttga ggtgttggct 1860

ttcccatgcc aggcgctggg ggaagctgtg acgagatttg agtgtcacat aataaggatc 1920

atggcaattc aaaggtacag ggaagctagg ctataaggta accagctcgg ttttcctggg 1980

ggtcttggac aggtgctttg a 2001

<210> 190
<211> 2001
<212> DNA
<213> Homo sapiens

<400> 190
tgggattgca ggcgtgagcc accacgccca gccctattat tatttttgac aacaacagca 60

aagatctgcc catccgactg caggaattta atacacagcc tttgtcagtg gtagaaattc 120

tatcactgta tgactcatga gtacataaca atgtgctata ccatacttcc tctttgaggg 180

ataaagcccc tgcactctaa gggaggattt cattatgcat ttctcttata tcaccagaaa 240

attatttagt ggaagacagt ttaatatttg acagctttga accatttttt tttctctttc 300

aaccagtctt tttacattgt ctgcttaaaa acaggaaacc acatatctcc accccagcac 360

aacccaccat ccaccagcac agaccgtatc attttggatc tacagtcttt ttcttccaag 420

cctggccagg tgcctgctcc tactttgcag gaaacccctc caggcactcc cggtgcgtgt 480

tctcagagcc cttctgagaa tattcaggat tgcacttcct gggctgcagc gcaccctgta 540

ccctcctttt ttaagcctcc tcccctgagt cactgctacc cttccctcct ctaacttccc 600

cttctctgca gcctccttag caatttctta tcataaagtt tgttttaaag aagaactgcc 660

cctctaaaaa aataataaac tgtcgagggc ggtagccccg tctagaggtt tccgactttg 720

ctctgtgccc acctgaagcc tccccagccc gaggtatttg aacttacatt cagtgcttca 780

cgtctctgtc cggggtgcct ctgtctctca actctgactt ctgagtactg gcagttagaa 840

caggaaggaa gtcaatgaga aaaaaaaatg ttttagacct acttgaagag gaaaccctgg 900

gctgtctcaa atcctgttcc tttgcactgc ctatctcttg atcagccaac ttgagcagca 960

ttcttgtcaa ttaatggcac atgtgcgagc atgacagccc gtgtgacgtg gagatgcatg 1020

aatgtacacg caagagtgtg gagcctatag ctatgggtat tatgaataat tgtgagggta 1080

tttctgataa tcaggtcatt gcagtgcata agcctatgga ggtaaggagc ctgaaaccag 1140

tagcaataaa ttcacatcgg ctttactttg taccactcac ttctgttccc actgggtccc 1200

cgccagcttc attatcaacc agatacgttg aaagtttgct ggcagtgaat gtagagtgct 1260

aagaaatgat gattgttaac tgcatatact acggtcctaa atttaaaaat gtgtccttaa 1320

acagattttt ggagttttgt tggttataat ttcaagacgt agctttgtat tgcactacaa 1380

acagtcaaat attgttttta atagttttgg ctgatttttc ttcaaatgaa acaaacaagc 1440

aagcgaaagt actgtctttg atgaaaccta ggaaagaaat tacaaaagga agtaaaactt 1500

cattaattca tagtcttcca actcagaatt tgtgacaact taacaagtgc caaaatagac 1560

tttgtaccat atttgaggaa atggtttatg aagtaattta aatgggaaca agtctcggag 1620

gccatgttta aaccatctga ggaacagttt ttaagcagcc aagtcacatt catgctgtat 1680

attcccgtag ctgttaccaa taataaatgc ttatttaggt taatatttca atattcagta 1740

tttacactac atcaatatat ttagatctga ctaagcacac aaatttggag aaactgacgt 1800

ttattttgtt ttcacctaaa tgtactgcat cttcttcatt tccctttgta aattgatacc 1860

acagggtttg ccttctactt agagtcaatg aaatcggaat aatcaaggga gtcatttcac 1920

gtgagaagag attagcctag aaaaatatga aaaggaaaat agtatataat taaaatgagc 1980

aaaaatacta aaagcctaat a 2001

<210> 191
<211> 2001
<212> DNA
<213> Homo sapiens

<400> 191
agccaaaaga ttggacaccc ctgattgaga acaatgcctg gaactttgta gctctcaaaa 60

gtttgttcaa tgaatgaatg agtgaatgct tccaatacaa aaccttagaa caggcaagtc 120

aaaatgtcac caaaaggtgg gaagaagagg cattcacggt actggttgag tgcatagtaa 180

ataaggtact tgcacaacta tgggcaacaa agagacggct tgggaattat caaacaccaa 240

ttttgatttt gatgagaaca gtcataaccc aatatttgaa gattttgatg gttgcttctt 300

ctttgcccct ctaaccaccc cccccacccc agaacgatga agggttaacc aaaacaccac 360

caaaggcaac tacttattac ctttgttatt cccacataaa aaattgagca gacctattgt 420

ttaagcagaa gaggcagtcc ctcaatgaag caatatcctg tttgaataat ttaggatggg 480

gcataggcca gcccacacag cgcatttgtg tgacagggcg tcccactcag acgtctccat 540

ttccggtatc tagagccggc tgccctctaa aaatccccct gttacagtcg ggatagaaaa 600

caaaaagctt aatttaccag gaggctaaac agattgtgcc ttcttaggca aatgttccac 660

ctgcttggcc aattagcttc ctctcccgaa agactcgttc aacaggtttc ctttttctct 720

tctctgcctc ctgggttggg gaaagaggct gtcacttcgc ttcgaccctc cccttccctc 780

cctctcggct tttccacagt gggtcggacc aatcgcgccc gctccgacgt gtccaggtcc 840

gcggccccgg gagcttggcg cggcccggcc tggatgcgct gggcggaggg tgcaggggag 900

ggcacggcgc cgcttgcttg gcctgcgcac ccggacctag aagccgggac gagccggggc 960

agagccaggc gcgcggaagt ctgaaggtgc cctccagaca cggccccgat gactgaacta 1020

cagcaagatg tggaagacac aaagcctgcg aaagtgctcg ggaagaggga gagcaaactt 1080

ggctcagccc agtgagtatt ctcagctggg acccatccaa ggaggtggga ggagcgcagc 1140

ctaaacttct ctgccccggg acgcattttg ggtggatgcg gatttctgga gctcctatat 1200

ggacaatctt gtggcgcgct ctctctgtct ctcactctct ctctctgtct agatattcag 1260

ttaaggagat ggtttagatt gattgaaaag tctactgtag gatttaaaca aattctctta 1320

attcaaaact cactggatgt tgggaaaata attttgtaga aaataagcct aaccacattg 1380

aagctcaaag tcccaacaca gcccttcgtt tagaatgacc attttcacaa attcatttta 1440

gttcagcaaa taattgagta tctacttgtt caataacgat gggcaataca aggataaatg 1500

ttattttgga tctgcccttc tggaacttca agagcagagt ggggatgggg gaggcacaaa 1560

tgaatattac tgtacttcaa gggactttgg tgaccattgc cacattaaac acacagacaa 1620

ggtatgataa agagtttagc ctataattga gtcttctcta cttttaaaat atttatctat 1680

tttgcagtct ttggtaaaat tggtttggga ggagaggttg tttttttcct acaaagtggt 1740

attttctaag cctactgaag tcacttctaa ttcagtctat gattttagac actaaccctt 1800

ctgagctcta aaactcttgt tctggtatca ggatatgagt aatttaggaa ggagggatat 1860

gtattatcat aaggttatac tgatgtgata attccacttt gaaatcattt taagttgcca 1920

aatttttgtg cttagaaacc cggtctaact tctgttttga acttcataac ttacattaat 1980

tatcactagg ctcaaagtct t 2001

<210> 192
<211> 2001
<212> DNA
<213> Homo sapiens

<400> 192
aattctctca acagtatttt gaggtaatgg cactggccga tccagtatcc cggctttttt 60

tcttaagagc caggtagcac caacattttc atgctaaata aacattagga aagctaaaat 120

tcttgttgac ttatacctgc catgcaaggt ggaataaata tgcatcaaga gtccaaatga 180

aacagaaaac tggggagaga agaagattaa gagaggcact ttgcaattta ttaaactgtt 240

aatttgcctt acatgccttt ttaaaagaaa gagctttggc tcaaatgaag aaagaggaat 300

ataagaatga agattaaatg ggaaggaggc aaaaacttga caggtgaaaa tgacttggaa 360

atgatagata cataaaaaca aacaaacaaa aacctacaaa catgatcata gctaagaggg 420

aggaaaatga aaggctatgt actaattcac atatattgac aaaagaaaaa agggtgagtc 480

cataaacgct ctgacccttg ttgtagatgt gaagcgttcc acttccagaa cttgtgcttt 540

tattggacag ccggagagat acgtctgtat attaggctcc ttaaatcctt gagtgttata 600

aatagcagag aaagggatat acactaaaaa aaaaagtaaa taaagttaat gcaatatatg 660

attagcataa cttgtctttt acaggcattc ttttaagaat cacgtgttaa aacaagtaag 720

cctcttataa aatgatctga gcccggtgtt acatctgcaa ggaaaatcaa tcatttgctg 780

gttacttcaa atttgtggtc ttccgaccaa ccgagataaa ctcttttctt ccccgctcag 840

atcatccgtc tttaccttct tgtatcttgg gatcgctggg agacacgtcg tagtctacct 900

cctctccctc aaagtggagt tcccgcgtgt ccagattttc tatgatatta ctcatgtcag 960

cagcaatttt ctgcagtgca gaggtattta cccgtggctc gtttttcagt gacatgttaa 1020

ctttggacag agtaaaagca aaggggctag gaaagaagaa aacggttaca aagacttagc 1080

attttgataa cataaaatgc tattccacaa tttgaatcca gacttagata cagcatatgg 1140

aaacaattca gcctgctctg tgtgagcctc agttatccaa aacaaccttt tagtttacgt 1200

tcctgtttaa catttaaagg attcctaact tgtaaattaa aacagagtgc ttaattgaaa 1260

acaagagaaa aaaaaaagaa aaaaaaaagc aacaccacat tgaatgccaa catttctctc 1320

atccaacttt attaacttgg ataagtaatt caagggagat tggtttggga aagcttattc 1380

agagaaagaa atagattatt tgctgtaata aatacagctg aagaaagaat atcatgaaaa 1440

ataagcaact ctaaatattt cctaaagaaa atttagagta tttcacatat tgaaatatat 1500

agatttagaa atatttttat gatagcataa tatttctaca ttttctctgt aggtgatatc 1560

ccaagcaaag tgtatattac ctcttcaaga aaggagattt tggccatgac tttacgtgag 1620

gtataatgac gtatgtgtct tgaaaggaaa ttgattaata tacctattcc gaaagacatc 1680

tgaggtgcta gaattatact tccctccttt ctttttcttt aaagaaaacc agttctattt 1740

cattctgaca tttaataaca caagtcgttg cacatcagta cccagggcag aagagttcac 1800

atggatatta tttttcatgt aaaatattga gattcaagct ttgcacactg tcatcagagt 1860

tggatgaaac atctgttttg cctgatgtag aaataatctg ttaaaaccag gccctagaat 1920

ggaaggacgc tcataccagt aactctttgg cacatgttcc tttcttactg caaatcacaa 1980

acactgagca tccaccacat g 2001

<210> 193
<211> 2001
<212> DNA
<213> Homo sapiens

<400> 193
acttttatgt ttttatatgg ttaaagtaaa ttcaaagaag gataatattt catgacatgt 60

gaaaatgata tgaaactcaa atttcaatgt ccgtgaataa agtgacgact gtttttgtca 120

ttttatatat atatattttt tttgcagagg gactggcttg cactttttaa acattagtta 180

tattattctc agtacaaccc tataagataa aattcattcc cattttactg atgagcaaac 240

tgcagcttat gtttcagcca aagtcagaca gttcattaag ggcagagtca tccatggcaa 300

tctgcctgtc cagatattac actttccccc ggggtaaaag cactggctct aagaatagtg 360

agaacaaaac atacttataa aagggagttg ggggctggga atggtggctc acgcctgtaa 420

tcccagcact ttgggaggcc gaggcgggtg gatcacttga gatcaggagt tcgagatcag 480

cctggccaac atggtgaaac cctgtctcta ctaaaagtac aaaaattagc caggcgtggt 540

ggcgggtgcc tgtaatccca gttattcggg aggctaagac aggagaatca cttgagtcca 600

ggaagcggag gttgcagtga gtggagatcg caccattgca ctccaggctg ggcgacagag 660

cgatactctg tctcaaaaaa aaaaagagag ttgggaagtg ttttgaatgc ttagagggca 720

gcttgggaac tcaattagaa gaagggacgg tttaagcaga catatttcta agagacttat 780

atgagttaac tcatttgatc ctcagcactc ctccatgagt tggatgctat tattaacaac 840

ccaatattag ataaaggatg accttcttaa agacctcttc ccaggaaaaa gactgtgtgt 900

gggtactctg aagctttggc tcttctggtg gggacaccac tgggtttcca aaggctcatc 960

cagccaagcg aagtatctat gacctcgtcg tgttcagcgc gcttagggaa ggattctatg 1020

gactgcactt ttaccctggt gtcctgcatt cttgctccaa gacttctctt accactctca 1080

ttcccctaaa tgcagcttct gacaccttct agctacgagt tcttgggctc ctccttcgtg 1140

acttcctttt aagataattt ctgagttaca acaaatcagc atttgaagaa atgcagcaga 1200

taagttgaaa attcaagtgc tgaggaatgt aaaggcccaa aacaatcccg tagaaagcac 1260

agaggcagat gacagtcttc cgttaaagat ttcactgctg tagatttctg agagtgctgt 1320

gtagctgtcc tctttatcct ggtgtttgcc acttcgaaca gtcactttcc cacttggaac 1380

ttacttgtgg ctttgctgac aaaacaggaa gtgatttgaa tacagtttgt ttttatgacc 1440

tttcaacctc aggaatttga aaagttttag cagtggattt acagccttgc ttaatacttc 1500

tatttcttac aaaacaaccg tataggggtt aaagtatcag tgcaaagtcc cgttcaaata 1560

ttgactcagt aatgagctct acaagccagc ctttgtggca tgttaaattc aaaacaagct 1620

tagcattttt tttttttttt ttggatagaa actggtgggg gtggggaaag aaagagggaa 1680

ctgggaagag aaggagggga acttaaacct tgcttcctgc tctgtctttc tcagaaaacc 1740

aaatatggca tcttccatga ggagcttgtt ttctgaccac gggaaatatg ttgaatcttt 1800

ccggaggttt ctcaaccatt ccacggaaca ccagtgcatg caggaattca tggacaagaa 1860

gctgccaggc ataataggaa ggtaacaaaa gggacgttgt tgtcaaaggg acaagcctca 1920

gactggctgt gcgtcagtga tagatgggtc tcgctcagcc tcgcaggctg ggcacaggga 1980

taagggcgaa ttactgatag c 2001

<210> 194
<211> 2001
<212> DNA
<213> Homo sapiens

<400> 194

ggcaatgaat tcagggccac ctgatggaaa acttgccttc ctctgtcccc aacacaacga 60
ccagtacctc cctgacccac ccgctttgag aacctcggct tcacgcagtc tcacggcatg 120
ttcttcccat ccaagcgcac cacaaacgca ttcccggcta ctccctcctt ccccggggac 180
tccggcgcct ccataactcc tcccacctcc agtcccagct cctggaggat gtcagagacc 240
cccagagtct caggagttcc tcagctccag ggccctttgt tccttctctc atcagccaca 300
tgtttccaca ggtcccctca gccttggtca tcaccgggca ctgtcccacc acccagaaca 360
acgttcagaa actcccctct ctctgaaccc aacctccctc ctcaacttcc acggcgttca 420
ctcctttttt ctctcaccgc atgtcctccg accacacgca ggcctcagtc acctcctcct 480
gcctcggtca cccccctcct gcctcagtca acccctctcc tgcctcggtc acccccatcc 540
tgcctcagtc agcccccctc ctgcctcggt cacccccctc ctgcctcggt cacccccctc 600
ctgccttggt cacccccctc ctgcctgtgc tctgcctcag ttaccccctc tcctgcccat 660
gttctgcctg ttaccccaac tcctgccctt ggtcaccctg ctcctgcctg tgctcggcct 720
cagtcacccc cctcctgcct cagtcagccc ctctcctacc tatgctctgc cttggtcacc 780
ccctctcctg cctcagccac cccctctctt gatttcatcc tcacccagct tggatgtcac 840
tgtccctcat ttcagctgat ttcctcctcc ctacccccca ctgtcatgcc agcccagcag 900
aactccagcc aggcgtgact tcaatcatcc acctgctcca ggggacattc gggcagacca 960
ccctgtgcaa aggctgtgac gttgccactc tggaataaca cggtcaccaa cgtgtagaga 1020
accctcagcc tgcagggcga tcccacaggt cagcatggct tcccactcgt caaacctcct 1080
ctcctctctt taaacttcac acctcctccc ctgtcccctc tgtggtaact gcttcctaac 1140
gagcccttga cgatcccagc ctcccggtgc tcacaccctg tgtaggcccg acgcacacac 1200
ggaattagag ctgacctgtg tgaccagcag aatatggctg aggagacagc gggggtgggt 1260
gcaggggtga ctttcaggac caggtcttgg aaggcactac agctgcttcc gccttggtct 1320
cctggatgtt gctctagagg acaccagttg ccatggtggg aagacgctca agtggccctg 1380
tggagaggcc tatgtggaga ggaactgagg cctcccaccg acggccagct ctgactcgca 1440
ggccatggag tggcccccgg tacccgttga gccttcagat gatgcagacc cagctgacag 1500
ccttctgcaa gtttccagcc agagccttcc agccaagctg ctcctgaatt cctgaaacac 1560
aaaaacagtg agagacgata catggtcatt gtttcaagtg actaaggttt gagatcattt 1620
gttaggcagc aagagatgac aaatcatctc attttcagca gacaatcccg atcctgcttc 1680
tcagggtaaa gatcatgagg aggaatctac tttcctgtgt tgctgccagc actgcaacct 1740
gtgccccacc cttctgcctc ctcccatcgc agacaaggag ctgcctgcct tctccgcagg 1800
gcttgctggg cagttctggc ctggggtcca ccctcctgct tttgaggagc tgtctgtgct 1860
cgaagctctc tcctccgccc tttcccttct agctctcccg tgctgctaga cccttcctca 1920
tgcccctcaa gcctgatgtg gcatcttctg tagtgcgatg agcccatcct tggggtacag 1980
ctttctttcc ggttactgcc c 2001

<210> 195
<211> 2001
<212> DNA
<213> Homo sapiens

<400> 195
gtgtgtgtgt gtgtgtttta aggctccccg ggtaaccgtg atgctgacca gctttaggaa 60

ccactgtcaa tgaaagcaga catagtacaa ggtctaggaa gccccaggtt acgtgtatat 120

aggaagaaat atatcaggtg aagccttacc agtcccctgc caggggcctg cctttgctct 180

ggcgttggga gtctgcacac agaatgtgga acttggaacg cacgctggaa cctggcactg 240

ttgcttcctt ctttgtacag atgacccttg cagtctgacc aggcagcttg aatccaaaga 300

gccaggctgt cactggggaa ggctggccaa gtctgccaag aggaggctaa aagggatttc 360

cttggcaccc ggggccatga cgggggttat ccttgcacac tcagaatctc agttaagttg 420

gcacatggta gcctttagct ctggggcggg ctcaccttaa caggtatgca tggtgaccca 480

ggcagccagt ttgctaattt actaatgaaa aatacagctg tcatgttggc ggatcatggt 540

ctcctttccc tctatgtgag ttgcacgtga agccaaatct gaaggttgtt taataagaaa 600

ccagcacaag caaaattcac cagatggtct ttaagagagc cacacccaca acaggggatg 660

ctgtgttcca gctcatgagg gagggcatcc tcttcatgcc cagacagggc ctaatgggca 720

tcaccagctt ccacggtgtc ggcgtggact ctgcagtcag actgacggaa ttcagatcct 780

ggttctgcac ttaccatttg tacgactttg gcaaagcagt atacccctca agccctcagt 840

ttcttcatct gtataataaa aataatagca gctacatcat tggattattg taaggattaa 900

atgaaagaag tgagacgttt aacacaatgt taggcatggt taatcttcat gtctggtagt 960

tattattatc atcatgacca ctactactat tattgtcctc gtctcctctg atttaggcct 1020

cactcttcat catagttgtt ctgtgtgtac aggataccta tgaatgatta ggtttccact 1080

cactaaagtg gaaacatatt ctagtcttgc ctgtttgtaa taaacttagc agccctgcct 1140

ggagttggtg accctcctct ccacaacact tcatggtacc tttgcccaca gtctcaggtg 1200

accagtcata tcctttgtga tggtggtgga agctcctgaa tcccaggatt ctgaactggg 1260

gatgaggtgg aagtcatctc acagcctgtc acacatcctt gtgcctgaat ctgcatcctg 1320

agttcttgtg tcccctccct gcactcccac ttctaaatag ctcattagtg ctgggacatg 1380

aaggcagctg cagggcaggg cagggttaaa aggcagggat tgagcaagtc cctctgcagg 1440

aaagcgtgtg cccatgtaat gtgtctagca gatgtaactt ctcgtctgtc tgggatggag 1500

attatgagcc cacatgggcc tgtgacttga gcaaaacaac aaaacaaacc aagaaatgtg 1560

aaacgcacat gcctgtcctt atacgagggc tcagacactt ctaccctgct gaagcaggtg 1620

gatgctctaa gtggcaccca ctccgcctgt gctggggaca gcagaagggg ccctccaagc 1680

cagactcggc tctcgtgaac ccttcacgac tggggcctcc ccgctaaaac agcctcattc 1740

ccacttaaca gctttggctc atgctgttgt cttggctata atgcttccct ccacttaata 1800

aatcctgccc atatattcag gcccagttta agccccacca ctccttgatc tctggactcc 1860

tcctccaaat gacagcatgg tctgttttcc agatacaatt cagagctgga ttcggtgttt 1920

gtttttccag ttatttttgc ttcctctagg aaacttcttt gaaaatgaga ctctccttcc 1980

ctagcagggg ctgggtgcag a 2001

<210> 196
<211> 2001
<212> DNA
<213> Homo sapiens

<400> 196
acagatttct ctttcccttg ctcaaatcag cattgcattt tcttcagcaa tgaagtatca 60

tagtatcaga agaaggaggg gaggtcctaa aggaaaactg aggcattaaa gggaatcttc 120

tttggagaca gaaagtgttc ctttgatcct tatccttcct gaattaccat ttcatctgta 180

ctggttttct gtctaagttc cattgctaac caggttaagt aaaggaattg gtcacctcct 240

cgggcctcag tttccacatc tgtaaagtga agggtctaga ctgtggtctc tatattcact 300

ttctgtctgt agattgctca tatttctgca ttccttctat tggctgtcca tttccatagc 360

acttttctgc attgcttctt gtacttttca cctcttattt ctgaattgtg gtcccactat 420

tttactgaaa ttaacctctc agacatcact gcacatctct aatcatcatt tcttttattt 480

tttcccctat accctcttgg ttacattcaa tactgacaac ctcagtctga tgagtctgat 540

cgagtttcag acagttgctt tctaagaaat tttctacatc tcatgttgct ccccttttct 600

cttccactta tagctcctgt gccctacctt tgtcatttct tatagttttg tgcttggtat 660

ttacgactct ctctcttttt tttttttttt tgacagggtc tcgttttgtc acccaggcca 720

gagtgcagtg acatgatcac agctcactgc agcctcaacc tcccaggctc aagtgatcct 780

cccacctcag cctaccaaat atctgggact acaggcacac gccactatgc ctggctaatc 840

tgtgtatttt tttgtagagt tggggtttgc catgttgtcc aggctggtct tgaatgcctg 900

ggctcgacag atcctcccac cttggcctcc caaagtgctg ggattatagg caggagccac 960

ctcactgtct taatgtaaat tttcccgcac tgacttgtcc gttctgtttt actacttccc 1020

tctttcccat ctctttgcct ttgttcatgt tattctttcc acctggtttt cttcttcaga 1080

ctaaaaatta taacaaatca tttgagatct tgcatttgcc aagaatgatt gacatcactt 1140

tcaattacct agattgaaaa tgaagttggc aaaaatataa attttcatga cttaataaat 1200

gagcttgcgg aaaaaaagcc agaaagattt gtcaatcaat ttaatacatt attaaagtat 1260

tatggtttat tgttttacat aaaattagga caccaaaaca ttatttcttt gctgtttgta 1320

gtttatgctg ttatatatgt gtacccgtta cctcattaca tgttataaat aataagactt 1380

tttagaagaa aaagatttat attttactat ttcttttttt ttttttttga gatggagtct 1440

cgctctgtcg cccaggctgg agtgcagtgg tgccatctcg gctcactgca acctctgcct 1500

cccgagttca agtgattctc ctgcctcagc ctcccaagta ggtgggatta caggtgcctg 1560

ccaccatgcc cggctaattt ttgtattttt agtaaagacg gggtttcacc atgttggcca 1620

ggctggtctc aaactcctga cctcagacca cccaccttgg tctcccaaag tgttgggatt 1680

atagacttga gccaccgcgc tggacctatt ttaatatttc taactgaact ttttttctga 1740

cttttcaaca ggagacctca cattttcatt ttgcactggg ccttgtaaat tatgtagcca 1800

gcccgaattg ttgggtctct ctgaggaagc tgaagaatct cagagaaaat acctccagat 1860

agaattgttt gagtgttctc cacaaaaagg gagctcactg tacaaccact ctggaaggaa 1920

acacagacac ttcacacaaa gctttcaaat gtttaaggtc ttactcaaaa cgaacataat 1980

ttaaaatatc acgatacatt g 2001

<210> 197
<211> 2001
<212> DNA
<213> Homo sapiens

<400> 197
aagcatttca gactttagtt ttggtagtac aactcttgta accattcaag tgctgctttg 60

ctgtcatttc aactcttaaa agaagggaat gggggagggg caagagagtc ttctaagtaa 120

acaaagatcc caaatccatg aaacctagga tttatcatgg tatgtacatt atgaaattcc 180

atatattgta aactgtgtag catgtcaaga tttcacatta gcaggtccta gtgtactctt 240

caatgcacat acggttttta tatgcatatt aataaaggcc catctttggg agaagaaaaa 300

aggctccctt tcaaatgaaa tcaagcccag gtaaagctga aacccaaccc acagagtact 360

ggcaccgttt tcaggccaaa agaagacaag ggtttcaatt tcatcatctt gtaatgctga 420

taaatgcgat gctgtgactg gcaaaatttg cagattacat aggtctttct aaacgtgtgt 480

aaatatgcaa atatgtattc caatttatgt gcaattccaa acaatttgct gtttgaaatt 540

aaagaccaac aactcttctg tgagacaata attcgggtca tttacatgaa aagtcttaaa 600

cagcaaaaat gctacttgca atttacatca tacagtgctc ccatcacaga gaacaatggc 660

ttcctcaatg gaagttaacg cagtagagag gtatactgag attttttttt taacttttca 720

gcacagtttg caaatactaa ccaatgaatc aaatttaaaa taaaatagaa atgtttctta 780

tgcttggtca tcactgctac ttgagtgtta tttttgtgtc ttcagattcc tatcaaacag 840

gagtgaacct ttttaacagg ttatggtgac attttttcag ctccttccat tttgtcctat 900

ttccagtgtc agcacaatta gcttgacttg ggggtttctg tctggtttta aaaacctttc 960

aatggagtgc acgtctgtga acaataaagg aacgaggaca cggggctggg aaaacagcat 1020

acctcatctg gaatggaaat ctatcgtctc tcgcccttgc acgggtcacg attatacacc 1080

ttcgtgacct ttagcgggct tacatatgta tctttttgtg ttccgcaggc tatgaccacc 1140

tgctgtatgt acaatatgtg cttgttacat ttgtacacca tctcccttct agaatttaaa 1200

gacctagcta ttacatcttg tttactcaat atcattcccc cattaaggaa aaaaaaaaac 1260

ctacatgaac tgaacatgcc atgtttcaat ctggccccag tggctttttc tctgaaagca 1320

aacgtgtgtc ttttacacca gggctttctc cccaccccag ggggtgtctt ccatcctttt 1380

gtggctcagt tgaaggcgaa aagggctcca aaccactaac taaccagagg agagcccctt 1440

cttccacctc cagggagaat ttcagattta atttgtccga agatagcgtg ctctcttctt 1500

actcatttgc catcattacg aggaaaacaa accaccacct tggcttcaag atcctgggta 1560

gaggctcacg gtcttttcaa ccatctttgg cgaggccttg cttccttcca ctcgagtaag 1620

tcgcggcttg gggccacgga aaggactggg tgagagcccg caatgtcgtt ggaaaaaagc 1680

agccctccct gcgccccctc accagcccca ccaccccgct ccactcccgg ggcgaagccg 1740

cgagccagcg gccccgtatg ggcgagactt ggcaggtccg cggggcgcct tctcgccgcc 1800

gccggactcg gggctccgca gcggccgggc gtcggcgccc gccggccgcg gggcttcgcg 1860

ctgccgccgc ccggtcccgg agctcccttc ccccaccggc cgccccctcc ccctcgcccg 1920

cgctcccttt tgttcgctcg ccgcccgggc cgagccgccg ccgccgccgc cgcccggccc 1980

ggcggcgagg ccgctccacc g 2001

<210> 198
<211> 2001
<212> DNA
<213> Homo sapiens

<400> 198
tgtatgtccc attattgctg gccccatact agacatgtgt ctgcactcaa ttgatagctt 60

tataatggat gaatgattaa gcctttttat ttaaagctag ttatgttccc acctgagagg 120

ctattcgcag gggaaaaaca tttgattcaa tcctctttaa gaaagcagtt tggccgggtg 180

caatggctca catctggaat cccagcactt tgggaggccg aggcaggcag atcacctgag 240

gtcaggagtt tgagaccagc ctggccaaca tggtgaaacc ccgtctctac taaaaataca 300

aaaattagct gggcatggtg gcgtgcccct gtatgccagc tactcaggag gctgaggcaa 360

gagaatcact tgaacccagg aggtggaggt tgcagagagc caagattgtg ccactgcact 420

ccagcctggg tgacagagca agactctgtc tcaaagcaaa aaaaaaaaaa agagagaaga 480

aaaaaaaaag tagtttgatc agagagttga tctatgttgt tcatattatg aaaagacttt 540

aaatcgtttt gtgagaatac aatgttgatc aacttatatg aattcagatt caagaagaga 600

ctgcagagga tcagggttat taaatagctt cctggcagtc aaagctgttc tggatgcaat 660

gggctgcctt agaaagtagc ttctgtccct agtattcaag ctaagaggac aatcacttgc 720

ttggattatc tgcaacatct ctgccatcag atggattctc agactggata atctttaagt 780

ttcttctaat aatcaatttt atgagtctgt gctgtgaaga gtggtgtctc aagaccaagg 840

gaacaataga ggaagacgta ttcagctttg gtcttgtgcc aacatttcct agttttaggt 900

tagcaaattc agaaatctgt taaatgttta ttgagtagct tctatggtcc atacactgtc 960

ttaagtgctg gggaattaac agtgaccaag aattttagat cgtgacttca tggagctcat 1020

aatgctaaca attgacattg gacaagtaac tacaagtttg atgaatctta ccaaaggaat 1080

aattgaagga gcctataata aggcgatttg gcaaagttgg agtcaggagg gttggggaaa 1140

agctatcaag gagagatatt taagatgaga ccttagttct tcaaagattg gtagaatctg 1200

cttagtgaag aagataatga agtgagtagt tcttaaattt caatttatga gtataaaacc 1260

tgagggatta gaattttttc ttaaaggtga accagatttc agtctgagta taaaacgttt 1320

ttatgtaata aatctgttta acaaataaaa ttagatccag caatcccact gttgggtata 1380

catccaaagg aaatgaaatc agtatgttaa tgaggtatct gcactccacg cttattgcag 1440

cactattcac tagagccaac atatggaatc aacctaagtg tctatcaatg gatgaatgtg 1500

taaagaaatt gtagatatat acacaatgga atactatcca tccttaaaca aagaaggagg 1560

ctgggcaagg gagctcatgc ctatgatcct agcgctttgg gaggccaagg caggaagatc 1620

ccttgaggcc aggaatttta gaccagccta agcaacatag aaagacccca tgtctacaaa 1680

aaaaattttt aaacatgatc agggcatggt gatgcatgcc tgtaatcctt gctatatgtg 1740

aggctgaggt gggaggattg ttgagcccgg gagtttgagg gtgcagtgag ctatgatcat 1800

gccactgcac tccagcagag accacccaga atagagatgc atctactccc agccaggggt 1860

aagagagagg gaaccagcaa aacttaacca gcaaaactgc ccttaacaga aaatagtgca 1920

tcctagccat cctccaggcc cactgagtgc aaagccactc agctaggtaa gaagagtttt 1980

gacacctccc catcctcctg g 2001

<210> 199
<211> 2001
<212> DNA
<213> Homo sapiens

<400> 199
ggtgttgttc ttgaacctcc cggtgtttga gagatggtgg gctgccctgt caagggcact 60

ggctggaagt tgtggcttct actcctggct ctatgtgtga tctgaccagc tcatcaggct 120

ccacaattag ctttctgcaa tgtgggagtg gattcttaca aaatgtagaa ctctactttc 180

taccctgctc ggggtctgca tgaaagatgg cgggggtaag ctttggctgc aaaatggtaa 240

atatttcctg agtgtctact gtgtacagtg cagtgaggct ggtttctgcc gtacatggta 300

tatagttatc caacagacac tagctggcat gggctcttac tgtgtgactt taggcgagtt 360

acttaacctt tctcttgacg ttcctttttt cacttttttt tttttttttt gagacaaggc 420

cttgctggag tgacaagctc attaggttcc acaattagcc ttctgcaatg ttgtccaggc 480

tggagtacag tggcacaatc atagctcact gcagcctcaa actcctggga tcaagtgatc 540

ctccagcctc gggatttttt tcacttttat tattttattt gtttgtttgt ttatttattt 600

atttatttga gatggagtct cactctgcct cccaggctgg agtgcagtgg tgcgatctcg 660

gctcactgca acctccgcct ctcaggttca agtgattctc ctgcctcagc ctcccgagta 720

gctgggacta caggcacacg ccaccacacc tggctgattt ttgtattttt agtagagaga 780

gggtttcgcc atattggcca ggctggtctc gaactcctga cctcaagtga tcctcctgcc 840

ttggcctccc aaagtgctgg gattacaagc atgagccact gcgcctggca cttttaaaac 900

agagacaata cttacctcat aggtttattg tgagagtggc aacagcttgc aatgggatga 960

ggcacatggt aaggtgctca atatgtgtga actattataa cggtcatagt taccattaag 1020

ggaagaccta tctgtgctac attaatgggg gataataatt actttattcg ctgggcgtgg 1080

tggttcatgc ctataatccc aacactttgg gaggctgagg cgggaggatc acttgaggcc 1140

aggagttgga gaccagcctg ggcaacatgg c[?]aaacccca tctctactaa aaatacaaaa 1200

actagctggg cgtggtggtg ggcgtctgta atcccagcta ctcgggaggc tgagaattgc 1260

tggaacctgg gaggcagagg ttgcagtgag ccgagatcac gccattgcat ttcagccagg 1320

gcaacaagag caaaattctg tctctaataa taataataat aacaacaaca acaacaacaa 1380

caacaacttc attcaaccaa atacattgta catctcacct ggcctgccca taagcaatgg 1440

agacactgaa ataaacacga ttcctgccct caaggtctaa tggaggaaag agatgtacac 1500

agctattcta gagggaaatt gacatgaaca gaagttctca tcctgtgctc caggatcaca 1560

ctggaggagg gagatgctcc cagagggtct tgggaaggct ttccaatctg gagctggaag 1620

aatgtttgcc caagagatgt acacagttat tctagaggga aattgacatg aacagaagtt 1680

ctcatcctgt gctccaggat cacactggag gagggagatg ctcccaaagg gtcttgggaa 1740

ggctttccaa tctggagctg gaagaatgtt tgccagagat gaaaggaggg gaagggaatt 1800

ccgggcagag ggaagggcgc aggcaacagc taggaggcgc agatgcaggc tgggtgtgga 1860

gggtgcgagt aggctggagt acaccaggga gttcccagca ggccactagg agccacccag 1920

gatgcccggc aacctcatga gtcagacaag cagggctgtg tctatctgga agtttccagc 1980

caagctaggt aaaacacatg g 2001

<210> 200
<211> 2001
<212> DNA
<213> Homo sapiens

<400> 200
gatccccggt gagaaagtgg gggccacttc tcttcctcac cgtcctgcct ggggttccag 60

agctgataag agggacagat ttcctagttc tgagatgcgg ggatctgctg agtcctccct 120

cagggctctg ggactcctcc ctctgctgac ctgaggctcc actcctcaga ccatagccct 180

ctcctcattg acaccccaaa ggcacagcaa agacacatca catgggccac cgtgcctgaa 240

accacccagg gctgaggtcc ccactgatct ggactccaag gtcccttgag tcctttctct 300

tcttcctccc cttgactctt ggctgggttg ttctcctcta ccacgccccg ccgcagacct 360

gagtcaccgt cccttggatt cctcaggccg agtgaagaga catctcggtc tgagacagag 420

gtggggtggg caccctgtcc tgggatccct tcatgcctcc ctcatttccc agcagggccc 480

gggccttccc tctgctcccc tgaagccgca ttcttggtac caatatcccc ttccctacgt 540

cagcctcaga tgccgacgtc agaatttgcg ttcctggtcg ccatgaccag ggcttcccag 600

ggccgacatc aggggcagag ccctccctga gcgtcccgac attgatgctg gcagaaccta 660

ggcgcggctc ccgccgaacc agccgggccc tggtcacacc aagctctgac tcccagagtc 720

ccctgcggct taagcggcgg gagagagtgg gggcgggggt gggggggggg tgacagccca 780

gcctgagaag tctgccctca ggtggtccag agctggcagc agaggcagcg ctgtattatt 840

tagggctttc tatctgggtt gggggcgtcc tcagtcctcc ctcagcctct caccttgcct 900

cctcacagag cctgggcccg ctctcttcag ccgatctcaa gcctgtccct cagaacgaga 960

acctcgcttc tctctgatcc ccgaggcgta agttagtggc gtcacatccg gtccccgggg 1020

cttccctggg ttgacagcag gggcggagtg gattctgccg gaactggggt gggagttggg 1080

gtgggagtga gggtgggagt ccgggtgggg tggggtacgg gtgaggatgg ggttggggca 1140

ggggtggtgg tgaagatgag tgtggggagg ggcgtggagg tgggaactgg gatggggatc 1200

ttggggctgt gggtcgtggt gggggtgggg gtggggtctt tcagtcatcc ctcagtgtct 1260

cgaccttgat gcctggaaaa gcctggaccc ctgttttgtg ctgaggatgt cttctcccct 1320

taaaccaggt ctcttgactc ggagtcttcc aaggtggcag tgaggggagg gttgtggtag 1380

gggtgacaac attgccaggg tcttccaggg ctgacaagag gggctcagct ggattctgtg 1440

gcccctttgt gaggggtggg tggaccctcg gtcctcactc aggagggtct tcctcctggc 1500

ttttgtctct ttgatgaagt gatgggtggg agatgctcag tttccgtcac ctctgatcat 1560

ttgcccagct gcatcccttg cagagcatgg gtgcggttcc caggccagat ggtccctggg 1620

gagctgcaac acccgtgatt gtaatgacct atgggagaag acgcacacct tcccaacggg 1680

gctcttccca agccacaagt agacttggca aactttttgt cctaaagggt atattttcta 1740

caagaaagag gctgagcatc ggggtgtgga gaggtcctgc tgttttgagt ttagctgata 1800

ttttatagtc caatgcagtg atgtctggtt ttctaaccct cattcctagt aaaaatatgt 1860

gttttaaatg tctgccaagt gtatattgaa tacatatata acatttcatg aaagaatacc 1920

taccttacgc tgtgtggttc actgcttatt ctatttctgg tctattcttt atattctatt 1980

ataataattt ttctcttttt t 2001

<210> 201
<211> 2001
<212> DNA
<213> Homo sapiens

<400> 201
tttatgggac aggatcacac acagggtggt gctactttct agagccaaat ctcaagtaag 60

gtgtccaggg gtcagttgca acagaaggca cattagccac agcagtctca gagaacactg 120

ctagaagaag aacctacaca tggccatgca atgaggctga ggggcaggcc taagtggaag 180

cagaccaagt aaagaggtca gagagcccat ggaaggagaa gagggaaaga cagttccaag 240

agtaaagcag cctaggttaa ggtgcagaaa attcagcatg gatactttta agagagctgt 300

gcaaggctct ccttacctaa aatcacactc cgtatacaga catacccaca aacaatcaca 360

tcatgttctt tgctcaagat tgaacttgct gaaactgtga tgtgctacta ctcctaccgg 420

gggtagaggg tagtctgctg ctgtacttct cctctctggt tactgatgca attatctata 480

gaagtataat gccgctctct gtaagcaatt ttaggtaaaa tccatgggat attttcacag 540

tgagctagcc tacagagagg gcaaaagttt ctgggttctt aaagcagctg ccgccaatgc 600

aagctctgta caggaggaat ttcttttttt tttttcgttt tattattata ctttaagttt 660

tagggtacat gtacacattg tgcaggttag ttacatatgt atacatgtgc cacgctggtg 720

cgctgcaccc actaactcgt catctagcat taggtatatc tcccaatgct atccctcccc 780

cctcccccca ccccacaaca gtccccagag tgtgatgttt ccctacactc cacctcatgc 840

acaagcccaa atatatcaca gcttctctac agaaatgcat aattcactgg cgaagttaat 900

agacattggc caagaggatg atctcctcac tatggaatca gctacagtct taggtagcat 960

gcaaactcca gtctgactcc aatcccttat atcttccatc gtaaagtatt attgccaact 1020

ttgtttcttt ccaatttttg tatgttctgc agtttatgta aaggctagca aaaacatgaa 1080

tactttatta tacatatggc aaatagtctc aaattgtaat gactcatttt tatcctctta 1140

tactgttgta aaattcagaa caacatgtat tcactatagg agtatgaaaa ttctttttac 1200

caatgtacat tttaaataac attgtggtaa agagaagcta catatttcac attctgattc 1260

aactcactgt atccaacgag taaaagatcc tagaaagaat cttacatttc taaaattctt 1320

atagactaag agtgagcgat aggaacaata aaaatatttc tgtaagtgct cttggtaaac 1380

agtcttaaag tcagaagaac acttatccaa aatgccctac attattgtgc aatcaaagta 1440

gataacttta acttcaaaaa caactcagtc ttccaaccca attcttaaaa ctctaagctc 1500

ttcacaaaaa atttttagcc aatttaaact tatttctaca ctcagttatg tttaaaacaa 1560

cataatgttt gcataatttt ttaaaaaaca ttctaactca atgtttttat ttatctaata 1620

ttaattacca gatataaaat ctttaaagta gcccaatatt tttcaaaata atttctgcat 1680

agatgattgt cattcagttt ataaaggttt atttataatt ttgtgatagg aataagaaaa 1740

gacaaaacct ctgaaacaaa ctcacacaca aaagcaaaat acaaaacaaa acagaaaaaa 1800

tacagtgaac ttacttctgg gaaaatggaa gcaaggaatc tagaggtatg gtttatccac 1860

tgagctcctt gtttagaaga aagttattca tattttctcc tggtctctac catgtatcta 1920

gaatttgctt taactatttg acttctgtga aaagtttctg tgaagactgc tttcagagtg 1980

gaggttatga tctcaattct g 2001

<210> 202
<211> 2001
<212> DNA
<213> Homo sapiens

<400> 202
gtgttcctct tatttgttct cccagctgcc aggtgaactc ttgaactgca aatcacattg 60

ttttcctctc ctgcttggaa tctgctcctt ggggcccact gtttgtggaa tggagtcctt 120

gctgtgtcta cccaacccta ctccactcac ctctctccta tgtcaactta ctgccctcat 180

ccctggcctc cactcattct cttcctaccc tttgcccctg ctgttctctc tgaacaaagt 240

tcagctccct cgttccctgg gtgctctagg gttagggaca agttcttatt tgcttacatg 300

ttcctcccac aaggcctggg aggccagctc tgcatttcct agcttgcagc aggtatgttg 360

taaatctctc cggcaggctg ccatgctttt gatccccttc cttcctgacc tcaacctgac 420

agatgcactg ctgttattac cattttatgg agaggatgtg ggccttggaa agggaggaga 480

ctttataagg gtcatgcaac cacatgtagc aaatgattca tatgaggctt tcaaaccaat 540

acccccagca gggctgtggg ggtgctggca aggggcccca tcctctgccc cccggagtcc 600

tgcaagtgcc cctagtgcgg ctcctctcgt cctgccttac ctcttttcct tgctctgctg 660

ccactgcctc cttgctgttc tgaatgctcc aggcctccct gccactgcca gcatttgcta 720

gggcatctcc atctgcccag aacacccttt cttcaaatgt cactagggct ttttgcattg 780

aacctcattt aaccttaaag aggccaccct ctttaaaacc ccaagccccc tcccccggca 840

ttccctgtcc tcctcactct gctctccttt ttctcttttc acttatcccc ttctgacatt 900

tgagatcatt tactcatttc atatgttttt gtttacttcc atctccctgc tactaacaca 960

gaaactgcaa gagggccggg atctttgtca ctgtcatgcc gtgaccctaa cacttagtgc 1020

agggcctgat ttgcaggagg tgcccagtga agaaagcctt gctcagggtc tctcggagct 1080

gcggacaggg tcatcatggg ggcagccctc tcctctgggg tctcccagtc cctccctgcc 1140

caaaaacctg gctcacctgg ctcccctctg tcctgtgtcc gcactgtagg ccgggctggc 1200

ctctccccgg ctgtaggccg cagcctctgg caggcttgtg gtcctgccct tctctcctcc 1260

cacatagggg ctggaacaca gaagctgaga gaaactgctt ggccagctct caagcccttg 1320

tctgctctca agcccttgtc tgctctcaag cccttgtctg ctctcaagcc cttgtctggg 1380

cttatggagc tcctctcctt cctccctctc ttcctgcatg ggtgtgacct ttgtgcctgt 1440

cgctggggct gccctgggag gacttctggg aggttggagc ccttacatct caaagccagg 1500

gattcctttg acactacttg taccaaaaag ctgtgtaccc cagcagtgtt tccaaagtga 1560

tatcctggtc ttccctgatc tttcactgtg tgcccttcag gcactcaagc tgttaatatc 1620

acacgagctt aataggctcc cctgagatgg tcctcatgtg aaaggattgc tctgtaaggt 1680

gtacgttttt cctctctccc catgtttctg ctctaaaata tggcttgtct tatgcttagc 1740

atcccctgac attccctgga aaaatatttg tgaagcatcc atctgtagtg tgtgggatcc 1800

agatgagagc tgggtcttag tgagaggaat gagaggctta gaggttccct ccctgcataa 1860

ccaccttgga aagtcagatg cggctcccca ttttgcaggt aaggaaacag gctcagggag 1920

ggatgtcact ttgtttgggt ttgcacaata agtagattac gtaacacatt tgtgtaagac 1980

tgggaaagaa atcaaggggc c 2001

<210> 203
<211> 2001
<212> DNA
<213> Homo sapiens

<400> 203
tcctatggct aaagtgctca aaggggaaaa aggagtttca aaaatgttct tggaaatacc 60

atctctcacg aattcttcgg cctctgctgt cccaatgtca cttgtctgag atgtaaacag 120

aggagttctg agaaagaagc tgaacttgca tttctccctg tttctatttg ttccaaactt 180

gtggcatttc taacaggatg aagcggaaga gaaagggaaa gagacaaaag tgtagaaaga 240

tggaagatcc cagctgcaaa tggccatttg cagttagatg gaacagctgc tgacgttcag 300

ggaaatgcat gtctctcttc agatgggaag gagcagtgga aaggggtgac gagttcctgg 360

ctggccacca atcatcccat ctttctgtgc cggttcctca tctggaaagt gggagtgata 420

cttgtgcttg cttttcctac ccacaaagat tattgtgaga gctataatac ggtgagatac 480

agaatcctgc ttttaaaaat acaaagcaga atcaagatgt caataataag gatagtaatt 540

gtgttagtta tctgcaatca tctattatag ctagtcgtct aggatcctgg atcgttctcc 600

tggttttact acagttttgg atcagctcac ccccaaatcc cttgctgaag ggtggagctc 660

tgtcagccat gggcagggaa ccacttcctc ttgcctttct actttctgtc tttcaaacat 720

gcccagggtc tttgcacttg ctgttccccc tgcctggtac ctctctcctg tggcttgccc 780

cagagctgat ccttgtcttt gtccacttct cagcgaggat ggcacttcag ggagcccttc 840

ccttactatc gcagagagag caggccctcc ccagtcatgt ccaacccaga actctgtttt 900

gttttcttca tagccctagc atcacagaaa atcaccctgt gcattcatgg atgtccacgg 960

gggcaagggc tttgtgttgc ttaacccagc atcctgaacc gtgtttgttg aatgaataca 1020

gaaccccgtt tgctctggga gagcacagaa aacagtcttc tatcatatat catagccagc 1080

tgcaaacagc agatggcttc ccatatccca gagagtaaga accagagaga gagagaaaga 1140

gagagagttt gggtctttct cctctgtgcc tgctctctcc agagaaactg gaggggtagc 1200

agttagcatt cccccgctgg ttccaccaag cacagtcaag gtctctagga catggccacc 1260

cctcacctgt ggaagcggtc ctgctggggt gggtgggtgt tagttggttc tggtttgggt 1320

cagagacacc cagtggccca ggtgggcgtg gggccagggc gcagacgaga aggggcacga 1380

gggctccgct ccgaggaccc agcggcaagc accggtcccg ggcgcgcccc agcccaccca 1440

ctcgcgtgcc cacggcggca ttattcccta taaggatctg aacgatccgg gggcggcccc 1500

gccccgttac cccttgcccc cggccccgcc ccctttttgg agggccgatg aggtaatgcg 1560

gctctgccat tggtctgagg gggcgggccc caacagcccg aggcggggtc cccgggggcc 1620

cagcgctata tcactcggcc gcccaggcag cggcgcagag cgggcagcag gcaggcggcg 1680

ggcgctcaga cggcttctcc tcctcctctt gctcctccag ctcctgctcc ttcgccggga 1740

ggccgcccgc cgagtcctgc gccagcgccg aggcagcctc gctgcgcccc atcccgtccc 1800

gccgggcact cggagggcag cgcgccggag gccaaggttg ccccgcacgg cccggcgggc 1860

gagcgagctc gggctgcagc agccccgccg gcggcgcgca cggcaacttt ggagaggcga 1920

gcagcagccc cggcagcggc ggcagcagcg gcaatgaccc cttggctcgg gctcatcgtg 1980

ctcctgggca gctggagcct g 2001

<210> 204
<211> 2001
<212> DNA
<213> Homo sapiens

<400> 204
ggtccccgag gggccccgac cgttggcagg agccgcgggg ttgcctctga tgcctggaca 60

aggagggaga agcaatggga gtcattccat cccgaggtgc cacccattcc tatctccgga 120

cgcctcttcc caaatcctgc ggcctctgac aaatgcctca caatgtctgg gctgacctcg 180

aaaattcagg tacgaccggt cccattctac agcggagggc accgaggctc agagaggcca 240

acggggctgt tttcgggtaa catctacctg atccagatcc cgcacgtgcc ttccccctgc 300

cctagcctcc gtcccgcatg agagaggggg tacttaatca ccgggcttcg gtgccaaagc 360

ctgcgtcctc gggcactggg cggacaccca ggccccgccg ccagccactc cagaaccggc 420

ccaaaacacg gcggccgtcc atctcccgag ccaggacagc gcgcatgtgc gtccagaggg 480

gcagacccgc gctccagcca gcgcgctgat tggtcagctg gagtgtcaat cggaatggtg 540

accgggcggg actcagagcc tgaggaggcg gggcttttcc tccgcccagg aaggaaatgc 600

agaaatgcct tcctatgctt ttagggagag cgctgtagca gaagaatttg atctgatcaa 660

aaaacctgtc ttagcaaaaa acctgaagcc gcctttgcaa aaattatatt acaaaattat 720

gacagcgaaa gagatctgat ctaaccaacc cgcagcttgc ctttaacctc taaacggtcc 780

ttggtcattc ctgggcttgg gccaagctaa ctttgggaga aattagttcg tagtctaaat 840

gataattgcc cttcccccga actaaactgg cttcatagag ttaatgaaag gccaccaggt 900

taggagtagt agagggacct gaattctgct aagggttaga ggtaactgat taccagcggt 960

tattccggag gtcacagtgt ttgcagcttc cccagctcac gattgtccaa cctgagattg 1020

gcctttggag atgtctttcc aggcttttgc atttctgacg gccggatcac cccacccgga 1080

ccctcaactc ttggctctac tagtcctgtg gcccctcgaa gtcaggagat cgagaccatc 1140

ctggctaaca cggtgaaacc acgtctccac caaaaataca aaaaaattag ccgggcgtgg 1200

tggcgggcgc ctgtagtccc agctactcgg gaggctgagg caggagaatg gcgtgaaccc 1260

gggaggcgga gtttgcagtg agccgagatc gtgccactgc actccagcct gggcgacgag 1320

cgagactccg tctcaaaaaa aaaaaaaaaa gaaaaaagaa aaagaaagta gaagaacaca 1380

gaaaaaaaag aataatataa tgactgccca cattcctacc atcagaatta gtcattgtga 1440

aacaatatat atatgtatag gtttcctgtt tttcttgttt aagaaatgaa agggataaaa 1500

gtaaattccc ctttactgcg tgcttccagt tttcactccc tccccagtct ccactttgag 1560

tttggtgtga acattttcct aatgtgtttt ggtttttgtt ttttggagac agagtctcgc 1620

tctgtcaccc aggctagagt gcagtggccc gatctcgact cactgcaact tccacctccc 1680

gggttcaagc gattcccctg cctttgcctc ctgagtagct ggaattacag gtgcggtgcc 1740

cgccaccaca cccggctaat tttttgtatt ttagtgacgg ggtttcacca tgtcgcccag 1800

gctggtctcg aactcctgag ctcaggcaat ccgcccgcct tggcctccca aagtgctagg 1860

attacagacg tgagccactg tgcccggcca ctaatgtgtt tttttgcttg ctctgtcatc 1920

caggctggag tgcagtggca caatcacagc tcactgcagc ctcaacctcc caggctcaaa 1980

cgatcctccc acttcagtct c 2001

<210> 205
<211> 2001
<212> DNA
<213> Homo sapiens

<400> 205
ctgcaactct atataccagg caactagttg tcaaatgcta attactgtca tcatccttaa 60

agacttgggc ggcaatgttt ccattatttt cacgatgggg aaacgcaggt tccagaggtt 120

gagcaatggc cacgaagcca cactgctagg acctgagggt gtgagcttag ctgcaacctc 180
cctctttggc ccctgctcat accaagccag gtgggtgaac tgagagtcca ccgagacgcc 240
cagcacttca cagcccagct tgcggaagtc ctctgcacgg ttgctgaacg cgatgatctc 300
ggtggggcac acaaaagtga agtccagagg gtagaaaaag aggaccacgt acttccctgg 360
ggaggaggga cacagaggag ttagggccca gcttctctca tgcacggccc cgcttctacc 420
tgggccccct ccgggcggcg ctcacctttg tagtccgaca gcttcacctc tttgaaggcg 480
ccatcaacca ccgctgtggc cttgaagtca ggggctggct ttccgatgcg cgcgttaccg 540
gaggccatga ctgaaagctg agacccccgc ccggtcagtg cgcccgggaa gacactttgt 600
cctcccaacc caaggtcgcg ctgcgtgctg ggccctatga ctgagtcagc acggcggagg 660
cgacagcact aaccctcacc ctcccgggta cccggtctac gaggcccgga ggctgccgga 720
gacccgctct cctgaccaga gcacgagtga cccagccact gccaccagca ccccacgagc 780
gagtcggccg gagggcggtg tcgcaagccc ccagcccaga aggagacagt gaggcccgca 840
cgagcagggg gcgggaagtc gggcgatcag gcctgcgctg caaggctgtg gcctcggttt 900
cccccgcaag gacaaaggag gccactatag cggatggaaa gcaaaggcgg ccagcactaa 960
agtcaggatc gggccaaagc agcctgaagg gggtcctccc gccaggtgca ctccgggtgt 1020
tcatacctgc gtgggcaaag gctagacgca cggacgatca cacgcgtgga cccgcgttct 1080
cagcgccaag tgagccctgg gccgcgaagc cttttatgcc cggcccgaac caagacgtgc 1140
acggcgcggg ggagggagcg gacgagcgga gcggtcgggt ccactgcgcc cgcaggtgcg 1200
ggggagcgtg cggccgggtc catgcgcctg cgggcggcgg ggggagacgc gttgccttcg 1260
gccgggacca ctgcacctgc ccgcgtgggt aatgcgcccg ccgcagactc cgcgcacgac 1320
tccgcctggg agcgcgttgg gggccgttgg agtccagcat ggcgcggacc ccgggggcgc 1380
tgctgctggc gcctctgcta ctcctgcagc tggcgacccc tgccctggtc taccaggact 1440
atcagtactt agggcagcag ggcgaaggtg acagctggga gcagctgagg ctgcagcatc 1500
taaagggtaa cctcaggcgg cggggatgac gatgcccaaa ggctccccgt tgccctactt 1560
ctcctgctgg ctgcaaaggg accttcctcg gcgggctgca aatctccacc cagctccgca 1620
ctggaggcgt ccccttgggc tggttacttt tctctaaggc tcggtttact cttggatata 1680
ttgaaataga tactgcttta aagcagctcc caggggaaga gagggtgtgg gcaggacact 1740
gaagctgtca ctcggggata ggtgccagga agggcagaga cagagaaaga agggggcggg 1800
caagcttggg aaatcagaga gggtccgatt gcagaagacc cgtgaccagt gcagcgagaa 1860
cgacaagcga aagtcccata tggaaagatt tggggaaaga atgtccaagg aagagggaat 1920
agcaaatgcc aaggtgaaaa gtcctgaagg agaaatggaa agaggtcggt gaggtgtggc 1980
agagacatgg gtgtgggagg g 2001

<210> 206
<211> 2001
<212> DNA

<213>  Homo sapiens

<400>  206
cccactggga tcccctgccc actagacttg tccccatcct gaggcagcct gttcctcctg      60

acccccgaaa gcttcccacc catctccctt cggcctgaag ccagcccccc tcctctctcc     120

cttctgttta cctcccaggt ttgggctcta ttttctactc cttccacctc ttcctgagta     180

acaaggacac tcccttcatc cacaccccat gcctcctgcc ttgcttcctg taccttcaag     240

tagttggaag aggtcatggg tgagaattcc ttctccttgg cctagtctgg cccaggtctg     300

gagagaaggc aatggagatg tgaagtggag gccaggtgtg gtggctacgc ctataatccc     360

agcactttgg gagtccgagg tgggcggatc acctgaggct aggaatttga gaccagcctg     420

gacagcatgc tgaaaccctg cctctactaa aaatacaaaa attagccagg tgtggaggca     480

tgcacctgca gtcccagcta ctcgggaggc taaggcacaa gaattgcttg aacccgggag     540

gcggaggttg cagtgagcag agatcgcgcc actgcactcc agcctgggtc acagaatgag     600

actctgtctc aaaaaaaaaa aaaaaaaaaa aaagaaattg aagtggaaaa tgagctggca     660

ggggtggtca ggcagggggt gagcaatgag ggctcctgag ctgaggaagc aggggcgggg     720

ggaggatggg ggctagggag tcctcagaag ttcccggaga tactccctca ctggacagag     780

tggggttccc gatgacgtta cttgaggcct gggagacggc ctctgcccct cactgctcct     840

ccccctccta aggtcaacat ttatgtgtcc tagacctaga gaggtcccag gacacgccac     900

tgtcccgcct tccccattgc ccgccccact ggccagtccc cacgcccaca cacccaaggc     960

tgccccatct ggcgctgatt atcctgctgc tgccgccacc gctgctgctg ctctgcaaaa    1020

ttcagctgct gcctctgtct tgaggacccc agcgcctttc ccccggggcc atgctgcctg    1080

cagccacagc ctccctcctg gggcccctcc tcactgcctg cgccctgctg ccttttgccc    1140

agggccagac ccccaactac accaggtagg tctcttggca tcttccagac agctccagtg    1200

aacttcagac ccccgattcc tttggggtta tgcctggaga tattttgggt tcccttgctg    1260

acacaccact ccccagattc cccaccgcct cacccttctc tggcccccaa atcctcccca    1320

tctgctgcaa gaccttggtc tcctgcaggc caccactgct cccattcccc caccttcgtc    1380

ctctggtctc ttaagtctcc ccacccaccc agatccttga ccccccatag aacccaagag    1440

agagccaagc tcctgaactt gcctggtagc tggattccag gaaggaattc gagagggatt    1500

gtagggggag gagggggcag tgagtggaga cttgggagtc agaatctgaa gtagtttgga    1560

acaggggcag ggctaaggtg tggagctcgc ggagcctggc ctccaggttc aatcagaggg    1620

ccatcgccac tgcagctccc tccatctctc caccccctcc cccccaccgc ctcgtggctg    1680

tgtccacatc tggaagtagt taggcggctc ctgtccctgg actgccaggc agagtgaggc    1740

gggagtctgg aaggggggcc gaggaggatt ccgggaattc ctgttggggt gggggtgggg    1800

actcttgggg ttggagggat ggtcgagttg ccaggcccac ttttccctta tctttgtggg    1860

agtctgggac accccagccc tgtcctggct ggtggaagct gccccaggtc tctcaaggcc    1920

tttccatttc atcccccagc ttccctcctt ggagtttcat cctcatcacc tcatccccat    1980

cttgccagtg ctcccccgtc c                                              2001

<210>  207
<211>  2001
<212>  DNA
<213>  Homo sapiens

<400>  207
tgactgagaa ggaatagctg atacagaggg gagggaattt ctgaaggagg gagtgaatca      60

tcaacaccat cacatggctt agaaaagtgg accctggatt tggcaatgat ggacataatc     120

caggtggact ttgagcctcc tcctgccccc agtggccact gcattctgca gccctgaact     180

ctacctctac ctctgctttt cttctgcaaa atgaacctct tatatacaat tctgtttcct     240

acatcagtta ctaaactttg aggcatttga gccaagggga cccgtactcg caccaagttt     300

catcacaggt gtctaatgtg attacagggg cagcacggtg ccctgcacgg gaaagtactg     360

gggtcagaga gaccagagtt caaatcgtga ctctttaccc ctgcgatccc tttggtgcaa     420

atgacttaat ccgtctctgc ctcagctttc tccactgtta attgggcata atggacacaa     480

caatggtgag gggctcttgc aggaatgaag actaggatga ggatgaggat gagggatacg     540

tccataaagc ccctgcacac cttccatttc ctgagcactc ccaggaagtc tttgctgacc     600

acgccaaatt agattatctt tgcttagcca ttaggccgct gccctatatt gggaggcaaa     660

tatacctctc agcttcggtg gccaggtccc ctggcaccca cttagtcctc agtctgcacc     720

taaaagcaag ccttccgagg gtcttgcctt gcaggtgtag ctcccacccc ccacgctggg     780

gtggtccggg gaccctctgt ttcctcagtg gtggggaagg gacccgtggg ttcgtgggtg     840

ctgaccgacg aatgtgcgga ggagggcgcg ccccagcatg gggggcgggg gtgcttctta     900

ggatggcttg gagggggcgg cctgcagggc tggacggcgg acgggggaca ctgcactttc     960

tgatgcggtc ggggaagccg ggagtcagcc tactggagtc cgctgccatc cctggccaga    1020

agcagccagc gccaccccaa gctccccagc ccctcgcccg tgccggggcc gccggggtcc    1080

gagttagggg agagtggggg gcggccaccg ctgcaccgcg cggccttcga aggactgccc    1140

tgcggcgccg cgttcccccg aggccacccc ctcccgccgt caacactccc cggggcgggg    1200

gctcacctgt gtggtctggg tcgcctccga ggccgagtcc ctcgttgcca agcccagcag    1260

gcctcgctcc cccgtgactg caccgccggc ccgctccagc ttcccgagta ccccacctgt    1320

ctccccggct tccttccggc cctttgtgtt ttcttctctt gctccgcccc ctcgcctgcc    1380

cgctaagcac ttaagctggt gaagtcaccc tactgccacc tggcagtcac tttcgctgtt    1440

ggtggcaacc caggctggaa cccagctctt gccggaaccc ctgccccatc cttgaggctt    1500

gtcccgacac cgagtaggtt ctagttcctt tgtgccttcc aaatgactcc cttcctccag    1560

caatcacagc cgcaacagcc atgtattgcg cccccgccct aggccaggcg tgggcatcac    1620

ctttgcactc ctttctgcag ccgtgtgtgg tgcccactgt gacctgcact tcacagacgg    1680

gtcatctgag ttgtgcaggc cactaaggga gcctggagtc aaccccaggc ctgcttggct    1740

cccaggcctt ctctttccat tgctctttgt cggtcccctg ctgcctcctg tccctcccca    1800

actctgactt gtggagggaa gcctttgaca tgtgggggta cctttaatac cgatatcact 1860

tgttaccgga gaggggtccc gatccagacc ccaagcgagg gttcttggac ttctcgcaag 1920

aaagaattcg gggtgagtcc gtagagtgag agcaagttta ttaaggaagt atagaaaaca 1980

ggccgggtgc ggtggctcac g 2001

<210> 208
<211> 2001
<212> DNA
<213> Homo sapiens

<400> 208
tgaaggatgg ggaaatgatt ccctatatac tttgctttta ctaacaatca tgtatgtata 60

tatataaatc atacaaggaa ttctacagat ttccaggcag attttattta gccagggaat 120

gacctaagaa gtgcatctct gttttcatct gcagaggcta aaagtttatt gtgatcctca 180

agtcactaca tccctaatac tcagagaatt gacaggaatg aaaagctaaa agatctagga 240

agttgcaaac gttacctgct gatggaaatc tgttgtctgg cttcgtttcg gcagcaaagt 300

gtgggcacac catcaggggt gctagattta ttccatcggg attgcggaag tcagctccag 360

tgatcggctg gaaaatttgg gtaagaggag gaggcgggga tagaaatgct gagagaagga 420

agggtaggga ggagggaagg aggcagtgga tttgcagtgt ttctgtgtgt atgcagcatg 480

tgtgccaggg tagggaatta ctagctattt agtctcacaa gactgtcttt ggtggcaaat 540

aacaagacct caatatctat tgactcaaat aggagcttac tttctctttt ggtgagtaaa 600

aggaaggaca cagccctagt ctctcccatc cttcttcttc aatatcttta ttttaatatt 660

gggggattat atcttttcct acccctttcc tgttaggatt tacctctcca acattgaaac 720

ctattttcat atttgtgtct gactctcacc tctgggatgc ggtaatgaac ataaaaggct 780

ataaaagaat aatgtaatgg aagccatagg ctccagaatt ctttgtattt ttataagctt 840

gttggttgca agttgtcatt acttccaggc agaatagttt tcccagccta atgtggtttt 900

gctgaaggag gatatgccca gaaagtccag atgaaactgt ctttaaaagt aaccaaaaac 960

agcagtcctg gttatttatg acagcacttg aatcaatgcc gtaagttctg atggactcac 1020

atgtgactct gttgctaaaa ctctcaggtg gtgggatgcc cgataaacat catctgtctc 1080

aattaaaatc cccaatctag gacaagtcac atgagcttct ccaaatgctt ccccaacatc 1140

caagcatgtg acagatcata agaatctagc tgattcctca aaccacaccc caaacttcat 1200

tttcttccat aaacagatgt ggggaaggag aaaaaaaggc ttcttggaat agaaaggata 1260

ataaatggga tctgtaatag actcagtttc tagctggatt ccatgcataa accctcaaaa 1320

atacaggctt atgcctctgg attttaagag ctgctaacag gctaggcgca gtggttcacg 1380

cctgtaatcc caggcctttg ggaggccaaa gcatgtggat cacctaaggt caggagttcg 1440

agactagcct ggccaacata tagtgaaacc ccatctctac taaaaaaaaa aaacacaaaa 1500

attagctggg catggtggca cacacctgta gtgccagcta cttgggaagc tgaggcagga 1560

gaattgcttg aacccaaggg gcagaggttg cagtgagcca agatcgtgcc actgcactcc 1620

agcctgggta acagagcgag actccatctc tcacacacac acacacacac acacacaaaa 1680

aaaaaaaaaa aaaaaaaagt tgctaatgaa ccaacatagt ctcctagagg gaaaccaggt 1740

ccagacttga acagtttaaa tttcactcat tttaatccag aaaaggacat actgggggaa 1800

ggcatccaga tgtgccctag tggttttcaa gtcccgactc ttcccctctc tcccaagaac 1860

ccaggcaacg cccaccccaa aacccccacc tcatggtcag agcattcctg ctgatgttac 1920

tcagaatgtg tcaggatcag gaggacaagt gaggggggag gcgtgcaatg tgatcagaac 1980

cctatgattt ctgattcctg a 2001

<210> 209
<211> 2001
<212> DNA
<213> Homo sapiens

<400> 209
tattcctatg tttccaaata gaaatgaaaa ttctgaaatt tcagataatt cccccctcca 60

aatcccaatt ctcaatttag cttttctgag ccttgtccct accgtgaaat cttactagct 120

caccccgaaa cgccggggtc ttggtcctct gggggactag tgagtcctgc tcctctcggc 180

tttgctccgt ccctgccccg gcccctccgc gggagtctgg ggtcgcggcc cggtcaggcg 240

ccaagccggc aggggggcgc ggtccacttg aggccacagc tccccaggtc cagggctcgg 300

gccatccgcg ctgtcccttc cgcgggctct tgctgttctt cgccaggagg ctttgcactc 360

cgggccgcat gggtcctgga taaggacctc aagaggttgt agtggcgccc cttcgctccg 420

gcgtactgtc tgaaccctgt gcccaaagag ggctgtggca gttcctgcac caaccgcctg 480

gagcccatac ctaagccctc tgggcacgag ggacctcctc tcccccgctc ccccctcccc 540

ccatcccaac tccagcccca aaggaagcaa tgcgcgcgtc cgagagcagg agcgcgcgtg 600

gctgaggaaa gaaagggaag gcaaccgggc agcccaggcc ccgccccgcc gctcccccac 660

ccgtgcgctt ataaagcaca ggaaccagag ctggccactc agtggtttct tggtgacact 720

ggatagaaca gctcaagcct tgccacttcg ggcttctcac tgcagctggg cttggacttc 780

ggagttttgc cattgccagt gggacgtctg agactttctc cttcaagtac ttggcagatc 840

actctcttag caggtaggtg ccgcagaccc tgcgggttaa gaggtggggt ggggggcagt 900

gcttgccaag gccctaaact gggagcgctg ggtgagggga acaacccact ttggagggtt 960

ctctgagaga tagatacacc ccatatcctg ggcccagctc gtgcacacag ctggaggtcc 1020

agagacccag tcccctctgc tccgtcagcc aagttccaag aagttgagca gagaccctct 1080

gggagcctgg cggggtgcag cggcctcccc tgcggggcct gtcacccggc cggcgcgtgc 1140

aaacgcctct ggcgcctctc tgcgcggagg gagataagcg tctgagccag ggaaagcgcg 1200

ggctaaaccc gcctcgccgg ggcccctgcc cgccctccgt gccccgcccg ggcggtgcag 1260

ctggcccggg tgctcacgct cgactctctt tcttcttttc cagggtctgc gcttcgcagc 1320

cgggatgaag ctggtttccg tcgccctgat gtacctgggt tcgctcgcct tcctaggcgc 1380

tgacaccgct cggttggatg tcgcgtcgga gtttcgaaag aagtgagtcc gggcagcgcc 1440

ttcccccttg ctggtacctg gcaggcaagg ggaactgacc gttggtcccg aaggtctaga 1500

agtgaatggg agcagggaca ggcctgggcg tcacctgaac gcacgcgaat cgggtctgct 1560

tgtgttttcc aggtggaata agtgggctct gagtcgtggg aagagggaac tgcggatgtc 1620

cagcagctac cccaccgggc tcgctgacgt gaaggccggg cctgcccaga cccttattcg 1680

gccccaggac atgaagggtg cctctcgaag ccccgaagac aggtaactac gccctgtgct 1740

gtccagggac gggagggaag gaaggtgtgc gggaggagtt ctctgtctcc actcccctgg 1800

cccgggggat cgtcggggct ggaccgcagc tcagatggcg cgagcagttt ccagctccct 1860

ctggctctag aatggctccc gttcccggtg ttggggccaa agctctgctt gatggggtct 1920

caagttgcct ttcttccccc tccccccgcc cgcagcagtc cggatgccgc ccgcatccga 1980

gtcaagcgct accgccagag c 2001

<210> 210
<211> 2001
<212> DNA
<213> Homo sapiens

<400> 210
tatgaaaaac atagtgaagt gaaagcaaac atctgtccag aagggaatag aacaataaaa 60

atgcccaata ataaatttaa tttgaatgtg aagaactaat atgaagcaaa taacacagtc 120

attgtgaggg atacataaaa agacttgagg aaatgaggag tggctttttc ttggatagag 180

aaattctaaa atagaaagat agatattgtc cctaagttaa gatgtaattt aaaaataata 240

cctagtgtct cacctggaat attttagcac ctgatcaaat gattctgaag ttcatctgaa 300

agacaaatgt gtcagagtag acagaaaagg aaaaagcaat gagggagggc aagcattatt 360

tcgcattaca aatataatta agctacagca attaaaatag tatactagtg gtacaggtac 420

aaacgactca atggaagaaa attaaaataa gagagttaca tacaaaattg agtggggatg 480

taggtgatga taaaagagcc acttcaaaac agtgggtgaa acagggattc aacaaatgat 540

gtagagtccc taccacttaa ttttgttcag atggatcaat tttttttttg tttgtttgtt 600

ttgttttttt tttttttttg agatgaggtc tcactttgtt gcccaggctg gagtgcagtg 660

gcacgatctc ggctcactgc aacttccacc tcccgggttc aagcaattat ccagcctcag 720

cctcccgagt agctgagact acaggcgcgt gccactatgc ccggctaatt ttttgaattt 780

ttagtagaga tggggtttca ccatgttagc caggatgatc tcgatctact gacctcgtga 840

tctgcccgct tgggcctccc aaagggctgg gattacaggc gtgagccact gcgcctggcc 900

cggatcaatg attttgaatg tacaatcatg caacaaaaac tatgaatgtc ttatcatatt 960

ggagtgggta agatcattgc aagcatgaca gcagactcgc gaaggcacaa gaaaatgatt 1020

tatgaaagca gatataaaat gggtattaac atatataata tttatttcta tgatggcgaa 1080

tttgtccgaa acacattgtt ttcttagagt aacctgggcc tcatattctc caacgccatt 1140

ggaagtgaaa ggaaggttgg gcgggggaaa aagaaaagtg ctttgtaagt tcattttcgt 1200

tgtcagaaga gcttgtggtt taggttctcc acagaggcag gaaaccttca gtcacgtggc 1260

aaatacccca ctgagtgcgc atgctgcaca gatctgttgc tgcgcatgcg cctttctgcc 1320

caccttctgt ctaggcagag ctctgcaagg agaggttgtg tcttcgttct ttccgccatc 1380

ttcgttcttt ccaacatctt cgttctttct cactgaccga gactcagccg gtaggtctgc 1440

agagtggtct tcctggtaat ttagttgtga gtgaatgtgt ggaggagcca gcgggcttag 1500

gacaggtccc gtggcacagt ccgtggcttt gagggaaaag ggcctcgcgg tggtcctccg 1560

ccttccccca ggtcctgatg caggcgccat gggccggtaa tcgtggctgg gctggaacga 1620

gggaggaagg taggccgtgg agggggtaga tcgcctgaag atggtgcgag tcctgggggt 1680

gggtgctgtt agaggtgtct gagtcccgaa aacgcctgga actctctgag ggaggacagt 1740

ttctggactc ttcgttaggg atgcgagaag ggatggtgtg ggtggtaagg aaggggccag 1800

gaaactggga acgctgtggg ctggtgacta cggccccgag gtctgtagag tgcctggcag 1860

aggtgtccag tgaggaacat aaccttaact ctgtgtcgca acttctcatc tcagccatgg 1920

acctcatgga aaggaatggt tgaggctgtg cattccaaca aaacttgatt tttcggggag 1980

tggggaggag gtggtggtgg c 2001

<210> 211
<211> 2001
<212> DNA
<213> Homo sapiens

<400> 211
cccaaacatg ctttactgga acttacgccc tggcataagg gccacaacaa aagagagagc 60

gaattctctg gcttgctgac tccttggaaa aaaccggtag ggattttttt attaggcaaa 120

gcacaggaat tgacgtcaga ggcaggatgt gctgctgggc aaagcatacg agaagtgggg 180

tatgcaggtc agcattactt ggttgcaatg gttatcttga ggaatgggcc aactggtggt 240

ctggccagtg gcaacaaggc tgtaaatcaa ttattcagca ttccttccca aggtgggaca 300

cccggcaaca ttgtttatct cctaaggcca gttcctggaa ttaagtgaaa ggatgactaa 360

tggacatgtt gtcagtgagg tagtggtgtg ggttttgtga ccagtgggaa tgcacgaaag 420

aatgctttag cggggagtga gctgaagcca agccccatcc ctactctgtc tcaaagtgag 480

ttcagaaaag gggatttaaa gaattctttt tttttttttt tttttttttg agacagagtc 540

ttgctctgtc gcccaggctg gagtgcagtg gcgccatctt ggctcactgc aagctccgcc 600

ccccgggttc atgccattct cctgcctcag cctcccaagt agctgggact gcaggtgcct 660

accaccaagc ccagctaatt ttttgtattt tttttttagt agagacgggg tttcaccatg 720

ttagccagga tggtctcgat ctcctgacct cgtgatctgc ccgccttagc ctcccaaagt 780

gctgggatta caggcatgag cctccgcccc cggccttaaa taattcttaa aggaagtaaa 840

gttaactttg aaagaactat caggatttgg attgactgaa aggagtgggg aagcttaggg 900

aggaggtgct tgccagacac tgggtcatgg cagtggtcgg tgaagctgca gttgcctagg 960

gcagggatgg agagagagtc tgggcatgag gagagggtct cgggatgttt ggctggacta 1020

gattttacag aaagccttat ccaggctttt aaaattactc tttccagact tcatctgaga 1080

ctccttcttc agccaacatt ccttagccct gaatacattt cctatcctca tctttccctt 1140

cttttttttc ctttctttta catgtttaaa tttaaaccat tcttcgtgac cccttttctt 1200

gggagattca tggcaagaac gagaagaatg atggtgcttg ttaggggatg tcctgtctct 1260

ctgaactttg gggtcctatg cattaaataa ttttcctgac gagctcaagt gctccctctg 1320

gtctacaatc cctggcggct ggccttcatc ccttgggcaa gcattgcata cagctcatgg 1380

ccctccctct accataccct ccacccccgt tcgcctaagc tcccttctcc gggaatttca 1440

tcatttccta gaacagccag aacatttgtg gtctatttct ctgttagtgt ttaaccaacc 1500

atctgttcta aaagaagggc tgaactgatg gaaggaatgc tgttagcctg agactcagga 1560

agacaacttc tgcagggtca ctccctggct tctggaggaa agagaaggag ggcagtgctc 1620

cagtggtaca gaagtgagac ataatggaat caggcttcac ctccaaggac acctatctaa 1680

gccattttaa ccctcgggat tacctagaaa aatattacaa gtttggttct aggcactctg 1740

cagaaagcca gattcttaag caccttctga aaaatctttt caagatattc tgcctaggta 1800

agtctgttgt ctgcatgtct ccccactaat gtgagtcata tagatggagt ctcagggcac 1860

gactgggttt tgtgtctctc gttgttgctt cacagccctt ttggcatcac ccatttattt 1920

aactaggata aaaacgaata ttggtatagc gattccacag tttacaaagt gcttttgtat 1980

ccactgtctc acttgatcaa g 2001

<210> 212
<211> 2001
<212> DNA
<213> Homo sapiens

<400> 212
   gaatgggtag aataaaagtt tgaaattttc cacttcactt ctttgcacaa tctgaggcag 60
   cctctgaaaa cacgatgcca agagccctag gtaatagcag gacaccagga aaatttggtt 120
   ggttaaggca gtatgactcc agagttctgc taataacaac ctgaaaccac catagtggca 180
   gaggaattac attttttaaa aaaaaaaatt ctttcaacag aacacaaaga ctgaaatagg 240
   aggtcactac aaccgcggga gctgccgccc cgccctgcag ggagcacctg gcctgggacc 300
   cgcaggcatt ctctacaagg ggtgcagctg tgcaaatgct cacaggtgac agaaacagag 360
   catctcctgc ccatcacttc atccaacagc cagaggtgac gaagacgacc ctcctgagtg 420
   aggactgagg gtccacaccg cccccccacc ccacacacca tagagggacc acagaatcca 480
   gctcagcccc tcttgtcagc cctggtaaac gcaggcagtg atgtcaccca gaccacaccc 540
   cttcccccaa tgccacttca gggggactca gagtcagaga cttggtctga ggggagcaga 600
   agcaatctgc agaggatggc ggtccaggct cagccaggca tcaacttcag gaccctgagg 660
   gatgaccgaa ggccccgccc acccaccccc aactcccccg accccaccag gatctacagc 720
   ctcaggaccc ccgtcccaat ccttacccct tgccccatca ccatcttcat gcttacctcc 780
   acccccatcc gatccccatc caggcagaat ccagttccac ccctgcccgg aacccagggt 840
   agtaccgttg ccaggatgtg acgccactga cttgcgcatt ggaggtcaga agaccgcgag 900
   attctcgccc tgagcaacga gcgacggcct gacgtcggcg gagggaagcc ggcccaggct 960
   cggtgaggag gcaaggtaag acgctgaggg aggactgagg cgggcctcac ctcagacaga 1020
   gggcctcaaa taatccagtg ctgcctctgc tgccgggcct gggccacccc gcaggggaag 1080
   acttccaggc tgggtcgcca ctacctcacc ccgccgaccc ccgccgcttt agccacgggg 1140
   aactctgggg acagagctta atgtggccag ggcagggctg gttagaagag gtcagggccc 1200
   acgctgtggc aggaatcaag gtcaggaccc cgagagggaa ctgagggcag cctaaccacc 1260
   accctcacca ccattcccgt cccccaacac caaccccacc cccatccccc attccccatt 1320
   cccatcccca cccccacccc tatcctggca gaatccgggc tttgcccctg gtatcaagtc 1380
   acggaagctc cgggaatggc ggccaggcac gtgagtcctg aggttcacat ctacggctaa 1440
   gggagggaag gggttcggta tcgcgagtat ggccgttggg aggcagcgaa agggcccagg 1500
   ccctcctgga agacagtgga gtcctgaggg gacccagcat gccaggacag ggggcccact 1560
   gtacccctgt ctcaaaccga ggcacctttt cattcggcta cgggaatcct agggatgcag 1620
   acccacttca gcagggggtt ggggcccagc cctgcgagga gtcatgggga ggaagaagag 1680
   ggaggactga ggggaccttg gagtccagat cagtggcaac cttgggctgg gggatgctgg 1740
   gcacagtggc caaatgtgct ctgtgctcat tgcgccttca gggtgaccag agagttgagg 1800
   gctgtggtct gaagagtggg acttcaggtc agcagaggga ggaatcccag gatctgcagg 1860
   gcccaaggtg tacccccaag gggcccctat gtggtggaca gatgcagtgg tcctaggatc 1920
   tgccaagcat ccaggtgaag agactgaggg aggattgagg gtacccctgg gacagaatgc 1980
   ggactggggg ccccataaaa a 2001

<210> 213
<211> 2001
<212> DNA
<213> Homo sapiens

<400> 213
gccaggggat ggggaaatgg agtcaggtca cagccatgcc tggaacctgc actaacgccc 60

tccccatccc agaccacaga gagccagcca ggaaagtcat tccacaccca tgtttcagaa 120

aacattcaag gtcagatcct ttttacctaa atcctgcttt catttgggtt tgcaccaggc 180

tcatccccct cctccagcaa gccttcctgg atttattcac tcagtggatg ccattccact 240

ggggcaaacc tttccggcac ctctcctgac ctaaggcaca gtcctgcccc aatcccctta 300

ccctcctacc cagatcactc ccaagttaac cacttgagtg atccacagca ggctgtcagc 360

cccggggact ggcttccagc tcctgcccaa gcccaaaaca ggaaatgggg ctccaaggct 420

gaagccttga tcttaacaca ggcaaggtta aatgctgcca aaataaatga tttaaaaaaa 480

ccctggccca ctgaggtgga agtgaccggg agataatctc ctgctgttca ttaaccatgc 540

cgctgcctct acctcaggca gggcagggca ggcaggcagg ccactggaag gctgactcaa 600

gtgtttcctt ccttaactta gaagctcctc ctatcctcag acccaggaca caggacacag 660

agggacagga tgggggtggg ggggtgacat ctctagatag tggagggcat cttgagagca 720

agaggcagat gccccctgca gccagcttct acgtttagcg gggtaatcaa cattcaacct 780

cgccctggag gaagacacct ggctttcctt cccccgtagg gcaaggctgc gcctctccac 840

tcccctgccg gacaggcgcc cgcagttccc ctcccctttg aggaaaggga ggcaggcccg 900

gaggttctaa gaacggcccc aagatttccc ttcttgaggt ttccatagcc ccaaagcctc 960

tgaatctcat tcctaacttt acttgacaat ctactcctaa cgttcgccac cacttaaacc 1020

tcccccaggc ctcccaggga ccaccagtgg ctgagcccca cacagcgcac gtttgggaag 1080

cttgaagacg accccgaaat gcgaagaacc aacgtttctg tcggtttacg cccttaagca 1140

cgaggcaagg gctcactggc ccggctgaag ttcggggtcc tgatccccca ccagccccgc 1200

cctgttaggc ctttagccgt ttctgtgccc ccgcgccccc tactcactgc gtgtgctggg 1260

cgcggcctgc gccgaagacc tcgtccagcg ccaccgtggc ctggcccagg aacttgtcga 1320

cgccgatgag cgagcggtgc atggtggtga gcaccagctc gcaggcggcg gcggagctcg 1380

cggcccaggg cgccgggccc gcgtcggcct cctgcgcccg cagcaggcca tccagggccc 1440

ccggcggcag ctcgaaggag cactcctcac gccactcggg gcagccgtgc gtcttctcca 1500

ccaccgacgt actgtacttc tcgcggccca cctggatcac cgtgtacgcg tcgctggtgc 1560

tgcccgctcc cgagctcttg ccccgcagcc cgcgggcccg cagcaccgtc acctggacgt 1620

gcgtgggcag ccagcgggaa ggccccgccg ccggctccgc gccccgcacc agggccatgg 1680

cggagaagcg gggggcgagg tctggccgag ccggtgcaga ccccaggacc tggtgacaaa 1740

cccggccgcg gcagaaggcg gtcaggaacc aactctgagc gccgccgcag ctgcgggctg 1800

ggctgggccg ggccgaccgg ccgccgcctc cccgcctcac cgggccgctg gccgcgggcc 1860

gcgccgcgac gtcagcgccc cgccccctgc cccgccccgc gcccgccgcc ccaaccggcg 1920

aggcggtgcg acagggatgc gggcacgcag ctcgcgcccc ggcagccgca gaccagcccg 1980

gagcgggaag gggcggtgcg c 2001

<210> 214
<211> 2001
<212> DNA
<213> Homo sapiens

<400> 214
gtttgctgtt agttatctca tacctaaagg gctattatca ttattaatat ttttattttt 60

tgagccagag tttcgctctt gttgcccatg ctggagtgca atggcgtgat cttggctcac 120

tgcaacccct gcctcctggg ttcaagtgat tctcctgcct cagcctccca aataactggg 180

attacaggga tgcaccacca cacccagcta attttgtatt ttcagtagag atggggtttc 240

accatgttgg tcaagccggt cttgaactcc tgacctcagg tgatctaccc gcctcggcct 300

cccaaagtgc tgggattgca ggcgtgagcc accacgccca gccctattat tatttttgac 360

aacaacagca aagatctgcc catccgactg caggaattta atacacagcc tttgtcagtg 420

gtagaaattc tatcactgta tgactcatga gtacataaca atgtgctata ccatacttcc 480

tctttgaggg ataaagcccc tgcactctaa gggaggattt cattatgcat ttctcttata 540

tcaccagaaa attatttagt ggaagacagt ttaatatttg acagctttga accatttttt 600

tttctctttc aaccagtctt tttacattgt ctgcttaaaa acaggaaacc acatatctcc 660

accccagcac aacccaccat ccaccagcac agaccgtatc attttggatc tacagtcttt 720

ttcttccaag cctggccagg tgcctgctcc tactttgcag gaaacccctc caggcactcc 780

cggtgcgtgt tctcagagcc cttctgagaa tattcaggat tgcacttcct gggctgcagc 840

gcaccctgta ccctcctttt ttaagcctcc tcccctgagt cactgctacc cttccctcct 900

ctaacttccc cttctctgca gcctccttag caatttctta tcataaagtt tgttttaaag 960

aagaactgcc cctctaaaaa aataataaac tgtcgagggc ggtagccccg tctagaggtt 1020

tccgactttg ctctgtgccc acctgaagcc tccccagccc gaggtatttg aacttacatt 1080

cagtgcttca cgtctctgtc cggggtgcct ctgtctctca actctgactt ctgagtactg 1140

gcagttagaa caggaaggaa gtcaatgaga aaaaaaaatg ttttagacct acttgaagag 1200

gaaaccctgg gctgtctcaa atcctgttcc tttgcactgc ctatctcttg atcagccaac 1260

ttgagcagca ttcttgtcaa ttaatggcac atgtgcgagc atgacagccc gtgtgacgtg 1320

gagatgcatg aatgtacacg caagagtgtg gagcctatag ctatgggtat tatgaataat 1380

tgtgagggta tttctgataa tcaggtcatt gcagtgcata agcctatgga ggtaaggagc 1440

ctgaaaccag tagcaataaa ttcacatcgg ctttactttg taccactcac ttctgttccc 1500

actgggtccc cgccagcttc attatcaacc agatacgttg aaagtttgct ggcagtgaat 1560

gtagagtgct aagaaatgat gattgttaac tgcatatact acggtcctaa atttaaaaat 1620

gtgtccttaa acagattttt ggagttttgt tggttataat ttcaagacgt agctttgtat 1680

tgcactacaa acagtcaaat attgttttta atagttttgg ctgatttttc ttcaaatgaa 1740

acaaacaagc aagcgaaagt actgtctttg atgaaaccta ggaaagaaat tacaaaagga 1800

agtaaaactt cattaattca tagtcttcca actcagaatt tgtgacaact taacaagtgc 1860

caaaatagac tttgtaccat atttgaggaa atggtttatg aagtaattta aatgggaaca 1920

agtctcggag gccatgttta aaccatctga ggaacagttt ttaagcagcc aagtcacatt 1980

catgctgtat attcccgtag c 2001

<210> 215
<211> 2001
<212> DNA
<213> Homo sapiens

<400> 215
tatttagtgt gtgttctcag cctcaccatt gacttgctgt gttaccttgg acaagtcact 60

ctcccactct gacctcaatt ttcacatctc taaaaggaga ctgctagact agacggtgag 120

tgaagtcctg ctgagccctc cgagttcatt ctcccttgtc cagtcagtcc ttccctccca 180

ccctcccctc tgggctccct ctctggggcc ctctccccca ggggaggcct gctccagccc 240

caggggagct gcggccctgg ctcctgttcc cagtcggagt gaccagccct gggcctcctt 300

cccagcttcg gccctggctc catgtggccc atctgagagg ctggcctcaa gcccggcggc 360

aactccacat ttctgttttt cctttttttt ccctctttcc cggagttaac aagaagcaga 420

tgtggcgcac gatggttgga gaggtggggg gaggaagggg gaggccggac cgccagccag 480

acagggggga agggagggga gccagcaggg aggaggaggc cagggcccgc cccacagcca 540

ctctcgcgcc tccgaacagc cacaggggca aagccctgtc acccccagga tccggtcatc 600

agggaaagag gacagggaga ccagaagagg gccagctggg acgagggggc ggacgcccag 660

gaggcaactt ctgagacgca gctcctgaga ggggcaggga ccaggcgcgg gaggccagag 720

ggggcacaga gaacaaaccc cctcagaagt gaagaggaga gcggaaggaa ccgagagggg 780

acggacagga gctgaggagg aaagaggagg ggagaggggt caggccaggc agccaaggag 840

aagacgtgtg gccgggggct atcagaagga aactgggacg gacgggccgg gctcgggctg 900

tcctgtggag cagcagcatc cccggggccg gcagaggcgc cagtggctgg gcgggatgag 960

tctctgaggg ccactgtgga gcgccccgcc atggcccccc gcaccctctg gagctgctac 1020

ctctgctgcc tgctgacggc agctgcaggg gccgccagct accctcctcg aggtttcagc 1080

ctctacacag gttccagtgg ggccctcagc cccggggggc cccaggccca gattgccccc 1140

cggccagcca gccgccacag gtaagagtct ggatcccagc ccgaggcttg ggtggtgagg 1200

aaggggtcag aatgccacct ctgcctggct ctctgctgtg tccgctaatg cagggcagag 1260

ggggagtgag aagcccatcc atcaggtggc tcctcacatg cccaccttcc cacggggcct 1320

tttccaggac cccctgaaca catatgttcc ttgggtcacc tagggggtcc gctcccacat 1380

gccccaggga agctgccctg aggggagcgg gccaggcctt cctgccagct gccctctgta 1440

tcctagcccc agagcctcag ggagaacttt tgtccccttc tagggctccc ttgaccccat 1500

ccagtaatcc ataattctga caccactcca gctctgttcc ctccagccct ccagccagac 1560

acagatcaga ggccggagtg aatggggcca ggagtataga actgcaatcc ccaagagggg 1620

caggctcatc aatggcttat tctctactta accccaagct ggcctgcccg tcttctcctg 1680

gaggggtggg gtggaagcca gtgttcagat cacccacagg gcccgggctg gctatgcgca 1740

cacagcatgc caatctgggg actgagacag gcccagaaag ggacacacat gattgtgtac 1800

attcagaggc tccccgggac cccaccacca tgaggattga ctgctctgtg gttttccatt 1860

ctgcgcgtct ccccatcttg aaaagtcttt atctgctcct gctacacagg aaagcgtggg 1920

ggatagggag ggtggcgatg ccaaagccct tgctctcact gatccccaag ggttctgcat 1980

ctttggagcc ttcttcagcc c 2001

<210> 216
<211> 2001
<212> DNA
<213> Homo sapiens

<400> 216
ttttgaatta tttgccatga aagcctttcg gtgtcccctc catccccccc aaaaaaaaga 60

aagagaaaaa gaaaaaaggc cctagcacta ctagaactca agcagaaatt cacattctcc 120

cagcactcac gtaggataag aaatagaatt ctaccccaag tccaaaaaag agtctggccc 180

aatatcaaaa tagactttac ctccaaagca gtttgatatt tattaaacac tacctttttc 240
tcttcattaa catctcttgc ctcctggtaa tttgcccttt acttttcctc tgtttctcca 300
gctttgaaat cacacgccat tttttttaag acaatgtaac aatgtgcaag atattttttg 360
ttgttgtttt ttgcactttt gacataccct atccccaaac acaccccaaa ccaggacact 420
gtggaattaa tacagtttgg aaattgggta tagtttagtg actcagtgtt agttaagatg 480
ctggcaacag atgttggcaa aggctatgga atgagtccca agaatgacaa accttacatt 540
actccaaagg tttgaaatcc aggatttctc tggttaatat ggttaatagt tccactgctg 600
gttttaattg tcttagagtg gaaggaggtg tcatattttc aggccctagt aatctcttcc 660
ttcagaattc ttctgaataa aaagcttagc aacaaaatca ctagagttag agtgctatgc 720
cttcttgatt taatactaaa acatggaaca aatgaaacca ctctggcctt tttccaacct 780
ctacagatcc ctcattctta tacttctagt ccgtcttttc ttcctcataa caccccaatt 840
ccctttttgc tctttctttt cctcctcaaa tcatatctct gcattgtctc gctatgtggc 900
cacccttgat ctccagtgac ctccaaaggg caaaacgccc ataacaagac aacaactgca 960
gtaacaatcc agtccaaaag tatttgccaa gagtttattc cgcggttagc accaaactct 1020
ccatctattt tgccactgca aacagtgaac ccatagttcc ccaaaacaat catagttcct 1080
catgcttcac tcttggcagg gctgaggaca ggaaagaagt gaaaataaaa tcttccatta 1140
ctaccctcag tctagccaga agtatacccc accacacaag gagatggcag ccaagtcttc 1200
actccctcag agtttctcct caggctcctc actcacctaa ctcttccatt gtcctacatc 1260
caagatgctc ttgtctcaca ggccgtgagg aacagctttg gcatgtgctg aagaagagga 1320
ccgcaaagga actggatgag acagcataag gcagcctcta taaacctttt tggttcctgt 1380
atttgcttag cacttcctct cttttgattt ggtgagcttt tttttttttt tcataggtta 1440
cacagtttta aaaatgtgac tgacatagaa aggtagatag taagatttgc attatggcag 1500
tgcattggcc agggtgataa agattcaaaa ttaattttgc aaaatttgag gacctactct 1560
gtgccaggat tcatcttgga ttcatgggaa tggcagactt atacttgcgt tttttccaag 1620
tgtaggtacc ccactctgag catctttctt ttttcttttt tttttttctg tgaagctggg 1680
agacacagag gcatagtctc cttcttaaaa tttgcatcag agttgaccag catccatttc 1740
attttgttgt cttatttatt cagaaattat ggttatctgc tttcctgcat ggaacgtaaa 1800
tccttaaaag tagggcagaa gtggattcat gcttggattc tcagggcctg gtttactcag 1860
tggtcccaaa agcaaacatc tatagggtgg tcacaaagaa ggaagccagg tttgacacca 1920
gatgttaaac tgtgtgcatc tcaattaagc ataatccctg cctagataca ctgccttata 1980
gcaatttctt tggaaagtaa c 2001

<210> 217
<211> 2001
<212> DNA
<213> Homo sapiens

<400> 217

ggagtgggct gcatgtgccg cgcggggagc ccgcccgact tcggggaggc agcggggcgc 60
cgtggccccg cggctcagct ccctgccggg tcgggtgtcc cagccctgcg acttccgggg 120
cccggcggct cctcggggag ctcgtccaca ggcagcctcc ggggcgagag agcgcgcggc 180
gcgggcgccg ggacggtccg cggcacgcgg gtggagccgc cggcgctcgg gctcccgcag 240
gggcggcggg cggggcgggg tgaggcgggc cggccgccct cccctctcct ccctcccgca 300
gcccgccctc ccttcctccg gtcccgcagc caattaggcg gccccctcgg gcgggaggcg 360
tggggcgctg cataaagcgg cggggagctg ccaccccggg agcgggctgc gcagtgtccg 420
ggccgagccg gtgcgccgca gactagggcg cctcggccca gggagcgcgg aggagccatg 480
gccaccgcta acggggccgt ggaaaacggg cagccggaca ggaagccgcc ggccctgccg 540
cgccccatcc gcaacctgga ggtcaagttc accaaggtga ggcgggcgcc cctcccaccc 600
gacggccgcg ggcccctgcg ctgggcagcc agactcgggg cggcgggggc gagggagcag 660
cgggccgggg gtccgccggg cgccgccgag acccgagcct ccctgcccgg gcgcggctct 720
ccagaaaccg cacctttcgc gggacgtctc gggcgggatc gcaggcggcg gggctcggcg 780
ctgtgagcct cgaagccggg aaaccgaggc cgtccagcgc ccgcgcgggg ccgggccgcc 840
cgcatccctg cgaggccccg acgtgtccct gcgctgcccg ctttgatcgc gagtggaggt 900
gcgcgccgcg ttgactcgga gcgcgctggt tttggggagc tccctcccca aaaacccctg 960
cgatgatttc cagggtggac gagtctggct ttgagagggt cggagggtcc gagagcgcca 1020
agagagagtc cgggaggcag atggcggagc tgcagttgga agactgggga agcgggtcct 1080
ccgagcaaag cgcagcgggc gccttccgtg cttagtttat acccggctca gcagcagaac 1140
gaggaggagt aggaaggtgc tcgcttactg acgaaacctc cccggagccc agaggtcccg 1200
gtgtttacac acaaggcgcg cgctccggaa ggtcgcagac ctgggccttt gggaagcggt 1260
gggtcgcgag gccgcagaag cgcacggttc tctgcctcct cgctctgtgg gctgggaggc 1320
tacagcgttg gcagaacggc gagattacct gcccggggca gaaaggaata aaagaagatg 1380
gtgaggcctc cgaggcctct gagctggggc tgaggagggc cgagagaccc gggtccggga 1440
gcgcgacccg atggaggaca cccccaggct gtctggcgtg cgcgtctgtc gtgggccggt 1500
gctggggcac tttagaggca caggccaggc cgagagccat tgccttaact ttccccacct 1560
ttggcttctc ttgatttttg cttttaccca tagcccccaa ctccagctga caccgcactt 1620
agagaatttc ctcatccgtt ttactttaag aagtagcaat tatctttctc aaagaatttt 1680
aatcaagcgg agaaagttgc agggcactat gtgtggcctt cagatttggg tctcttggca 1740
cttaattccc aatgtcggct gagcctctga tcacaagaca tttaaattaa ctgttttcag 1800
aaggctctgt aatcaacaaa gacacgtcgc tgtaaactaa acgctggaaa ttattcttaa 1860
tgtagtttta tttgtaatat cagcttcaga gtgaaaattc caacacgaat tctgggaaga 1920
gtgaaaatgg tacccgaatt ttgtaagacc ctaaccccag gtgcttggct gctgctactg 1980
atggttaagt ggggaggttg t 2001

<210>  218
<211>  2001
<212>  DNA
<213>  Homo sapiens

<400>  218
aggcaggaga atcacttgaa cccgggaggc gtgggttgca gtgagccgag atcgcgccgt      60

ggcactccag cctgggcggc cagagtgaaa ctccgtttca aaaaaataaa ttaataaatt     120

aaattaaagg tatgtgaatg caatctctcg ctgacaaaat atctttatgt actgtagatc     180

ttggcaacct tagcatacga ttttgttttc tttccttatt atgttaaaaa ctttcccatt     240

ttcacttaaa ggaagcaagc actttacagc ttctttttgg catatttcaa tcgccagcat     300

tgctaccctt gtactttggg gccattatta agtaccataa gggttaattg aacacaagca     360

ctgagatacc accaggacag tcactctgat aactgcgacc gctcctaagt gactaaggta     420

cagagagcgc agccagccaa cagcatgggg actcaggaca aagggaggat taccatccca     480

gggcagggct gactcaagag cttatcacac tactcagaag ggctggcgac ttaaaattta     540

tgtattgcct atttctggaa ttttccaatt aatattttgg aacctcaggt aactgaaagc     600

acagaaagga aaaccctgga taagggaaga ctactgtagt tggagcagag aattgcaata     660

cctaaaatat ttgccatcta gtctttcaca gaacaagttt gccagctcta gttttagagg     720

agcaaaatga aagatagcaa atttctcagc tcaccagttc ctaaagttgc gagtgcatcc     780

agatgattca gtgcagctca gtatgttcca ctctgggcca caagggaaag cgaagctgga     840

gcttccttgt ctccacctcc gggagaaatt tgtccagaga ccggcagcac tgcttccgca     900

ggcgaacaac gccgcttgct cttcaaagtc ctttctgagg ccagatcaga aggtcccact     960

ccttttaagc cccttattgc ccaatcattt ttccagtcac gtgtctgaag cagtttatcc    1020

tgattggatt ttcagttgtt gtccagcttg attgccaaaa gttttcctcc aattgctact    1080

caaaggaaag aatgagggtg gggcttcagg gctgctttcc aggttgattg agctgctgat    1140

gggcatcctc cctcctctct ctcttgttca gagtctacca ctttctctgc agtgtgcctg    1200

ggccctcagc tattggggat gagaagggat gcggagtggg aggcagtcca catgtctgtt    1260

ccaacaaagc tcagaggtga gcgagttaaa aagcagatgt ctttccagta ggagccccag    1320

gggacagggt gggtctcaca gcccttgaag accgagctcc cgtaggagtt tccaggtgtc    1380

caaacagggt cctcactgtg agtcttcaac agctttctcc aagcagcagc ccactccctt    1440

cttaatccat ttccaggggt caaaggtcta tcctggttcc ctcccatgcc ctctcacttc    1500

cttccctctg tctctccctc ctccctcctc cttcactacc ccctccctgg aaccttctca    1560

gctgaatttc aggcccaccc tttcactctc tttacaggct ttgcaacaca gattagaaag    1620

cgggggaggg ggagcctcaa gagtcccttg acaggtaaga aatatttata tttccacccc    1680

cattccaacg caatgcaatc aagtctcaaa attaaaacga aaatcatttg caaattaaaa    1740

tgttctaaag taccatctcc tgataaaact ttaaaaatac ctttctgggc cggatgtggt    1800

gactcacatc tgtaatccca gcacttttga aggccgaggc agcagatcac atgagcccat    1860

tagtttgaga ccagcctggc caacatgatg aaaacctgtc tcttattctt tataaagtaa    1920

aatacaaaaa tttcctgggc gtggccgtgc gtgcctgtaa tcccaggtac tcgggaggct 1980

gaggcagaat aatcacttga a 2001

<210> 219
<211> 2001
<212> DNA
<213> Homo sapiens

<400> 219
gaggctaagc cggcagatca tttgatgtca ggagttcgag aacagcctga ccaaaattgt 60

gaaatcccat ctctgttaaa aatacaaaaa aattagccag acatagtggc acacatttgt 120

aatctagcta ctcaggaggc tgaagcagga gaattgcttg aacccgggag gtggtggttg 180

cagtgattcg agatccgtgc cactgcactg caacaaagca aaagcaagac tctgtctcaa 240

aaaaaaaagg acaaggacaa ggtcagaggc aaaacaggag gaacagagga aggaagggac 300

aaagagggac cccaatgcag actggacgag tgcagagggc agggatggaa agtcacgtcc 360

tgagctgttt gctgttgcag aatctcagca gctgggccat aaagaaggga cttgacgctg 420

tcccagtctc tacacttggc acccttgacc tctctcatcc cacctttccc taggccgagg 480

tcacacctgc tttcattttg tttttttttg tttttttttt ttaagacggg gtctcgctag 540

attgccctga cgggagtgca gtggctattc aagggcacca tgatagtgca ctgcggcctg 600

gaattcctgg gctcaagcga tcctcccacc ccagcctctc gagttgctgg gactacaggc 660

gtgtgcccct gcacccagct gcgcccgttt ttgtcctcct gtctggcttc tcttcatgat 720

ccccacccct ttattcggcc tcaggggaac cccgtggtct attccgtgag accgagcaga 780

gttttgggga gccccgactc ttggatctcc ctgctggctc acctttctct gttcggtctc 840

caaaccagac cagcaatggc cccaggaaga aggtggcgct gggagaagtt ccctaagcta 900

tgtatgccta cctgtcccct tcctgacttt tcggttcccc aaacccgagg ctggacctac 960

ctttaatcag atccacgatg cgcagctcga agggaatgtc gttcttcttg gcaaagatgt 1020

aaacagcgcg gcagggctgg gacagcaggt ccaggtacag ctccaggccc atagtgggga 1080

ccgaccgacc tgaccggaaa ccagagggag tcagcaaact ccagtgccag gactcgggtc 1140

tcggacctca gaccacgccc ctgcggctac gcaccccgac cgctgtgccc cattgggcgg 1200

cacggacgcc tgggacactc tttagtccaa ttgcgggcgg cgggcggcct gtctcagggc 1260

tgggcccgcc tggctcctgt gggatcaggg tgggtggcca agagaccgtt gtctctcagg 1320

gggacgcaca gagaggcccc agtcccatcc ctttcgtgcc tggctcgctc atttcactta 1380

gcataatgtc ttcaaggtgc atctgtgttg aagcgtgtgt cagaatttcc caccgtccgc 1440

attttagagg aggagacaga cactgaatga ttatagaccc agaaagtctg gctccaaagt 1500

ccatcctctt ggctcaacac tgcagagtcc ttggtgccag caagtggcaa agcctcaggg 1560

cttacgttca gtgttttgtg gtcactcgct tcactgcagt cgcactccct ggaatgtcac 1620

agccttggtc ttcctgcccc tgccagcaca gtgccatcta tcgcattccc tgggtttatt 1680

tcttcctttc tccaggcctg tgtttctcca tctttaaaat gacagggaag ctagagccct 1740

ccagagtcct tgcaagtcca cccccctgga ttcatctgct gagccgtaag cccaggcctg 1800

ccccttgctg ccatggagcc tgtcaaatag tatcagggtc acatgtcatt ttctaaaacg 1860

aatcccactg aaagtgtgtc agccaacgca taatgttttc tagagaataa ggaacatttc 1920

tgcaaataaa gtggaagtaa caactgtttt attttattta gatagagtct cactctcttg 1980

cccagggtag aatgcagtgg c 2001

<210> 220
<211> 1147
<212> DNA
<213> Homo sapiens

<400> 220
gggggaggag ccaagatggc cgaataggaa cagctccggt ctacagctcc cagcgtgagc 60

gacgcagaag acgggtgatt tctgcatttc catctgaggt accgggttca tctcactagg 120

gagtgccaga cagtgggcgc aggccactgt gtgcgcgcac cgtgcgcgag ccgaagcagg 180

gcgaggcatt gcctcacctg ggaagcgcaa ggggtcaggg agttcccttt ccgagtcaaa 240

gaaaggggtg acggacgcac ctggaaaatc gggtcactcc cacccgaata ttgcgctttt 300

cagaccggct taagaaacgg cgcaccacga gactatatcc cacacctggc tcagagggtc 360

ctacgcccac ggaatctcgc tgattgctag cacagcagtc tgagatcaaa ctgcaaggcg 420

gcaacgaggc tgggggaggg gcgcccgcca ttgcccaggc ttgcttaggt aaacaaagca 480

gccgggaagc tcgaactggg tggagcccac cacagctcaa ggaggcctac ctgcctctgt 540

aggctccacc tctgggggca gggcacagac aaacaaaaag acagcagtaa cctctgcaga 600

cttaagtgtc cctgtctgac agctttgaag agagcagtgg ttctcccagc acgcagctgg 660

agatctgaga acgggcagac tgcctcctca agtgggtccc tgacccctga cccccgagca 720

gcctaactgg gaggcacccc ccagcagggc acactgacac ctcacacggc agggtattcc 780

aacagacctg cagctgaggg tcctgtctgt tagaaggaaa actaacaacc agaaaggaca 840

tctacacgaa aacccatctg tacatcacca tcatcaaaga ccaaaagtag ataaaaccac 900

aaagatgggg aaaaaacaga acagaaaaac tggaaactct aaaacgcaga ggccctctcc 960

tcctccaaag gaacgcagtt cctcaccagc aacagaacaa agctggatgg agaatgattt 1020

tgacgagctg agagaagaag gcttcagacg atcaaattac tctgagctac aggaggacat 1080

tcaaaccaaa ggcaaagaag ttgaaaactt tgaaaaaaat ttagaagaat gtataactag 1140

aataacc 1147

<210> 221
<211> 20
<212> DNA
<213> Artificial Sequence

<220>
<223> Synthetic Primer

<400> 221
tttttgagt taggtgtggg 20

<210>  222
<211>  23
<212>  DNA
<213>  Artificial Sequence

<220>
<223>  Synthetic Primer

<400>  222
tctcactaaa aaataccaaa caa                                              23


<210>  223
<211>  13
<212>  DNA
<213>  Artificial Sequence

<220>
<223>  Synhetic Primer

<400>  223
gggtgggagt gat                                                         13


<210>  224
<211>  18
<212>  DNA
<213>  Artificial Sequence

<220>
<223>  Synthetic Primer

<400>  224
cgagcgcggc tacagctt                                                    18


<210>  225
<211>  20
<212>  DNA
<213>  Artificial Sequence

<220>
<223>  Synthetic Primer

<400>  225
ccttaatgtc acgcacgatt                                                  20


<210>  226
<211>  18
<212>  DNA
<213>  Artificial Sequence

<220>
<223>  Synthetic Primer

<400>  226
accaccacgg ccgagcgg                                                    18


<210>  227
<211>  22
<212>  DNA
<213>  Artificial Sequence

<220>
<223>  Synthetic Primer

<400>  227
tgtcgtcgga aattggcagt at                                                22

<210>  228
<211>  22
<212>  DNA
<213>  Artificial Sequence

<220>
<223>  Synthetic Primer

<400>  228
caaagaccag ctgcaaggaa ct                                                22

<210>  229
<211>  17
<212>  DNA
<213>  Artificial Sequence

<220>
<223>  Synthetic Primer

<400>  229
tctttcctgt gatcttc                                                      17

## Description

### FIELD OF THE INVENTION

The present invention relates to the methylation of genomic DNA sequences, including *Long Interspersed Nucleotide Element-1 (LINE-1)* sequences and genome-wide gene methylation profiles. More specifically, the invention relates to: i) the use of low levels of methylation of *LINE-1* sequences as a prognostic and predictive marker in human cutaneous melanoma; ii) the use of multiple gene methylation signatures as prognostic and predictive markers in human cutaneous melanoma; and iii) the use of combined *LINE-1* methylation and multiple gene methylation signatures as a further improved prognostic and predictive marker in human cutaneous melanoma.

### BACKGROUND OF THE INVENTION

Cutaneous melanoma (CM) is characterized by a constantly growing incidence and high mortality rates in industrialized countries, representing the leading cause of skin cancer-related deaths worldwide (MacKie et al., 2009). Only surgery, in the initial phases of disease, may have a curative intent for CM patients; otherwise, advanced stage CM is characterized by a substantial unresponsiveness to conventional therapies (Bhatia et al., 2009). At present, the unique predictor of the 5-year survival of CM patients is the clinico-pathological staging of the disease, which defines overall survival (OS) rates ranging from 95% to 7% for stage I to IV patients, respectively (Balch et al., 2001; Balch et al., 2009). However, CM patients within the same clinico-pathological category often behave extremely differently, and the current lack of established prognostic molecular markers greatly impairs physicians ability to identify CM patients who will evolve through a highly aggressive rather than through a more favorable course of disease (Jennings and Murphy, 2009). Thus, the definition of prognostic molecular markers that enable predicting survival of patients within the same clinico-pathological stage of disease is a mandatory requisite to boost the current clinical management of CM patients, providing physicians and patients with more efficient clinical, follow-up and treatment procedures.

In mammals, DNA methylation, occurring at 5C-position of cytosine in the context of CpG dinucleotides, represents a major epigenetic mechanism in controlling gene expression, chromosome X inactivation, imprinting and repression of endogenous parasitic sequences (Esteller, 2008). Alterations in the genomic DNA methylation patterns represent a hallmark of cancer, and include both global genomic DNA hypomethylation and gene-specific hypermethylation. Each of these apparently contrasting events are thought to play a major role in tumor development and progression.

Global genomic DNA hypomethylation (i.e., overall reduction of the 5-methylcytosine content) is a frequent molecular event in cancer and has been observed in neoplastic cells of different histotype (Gama-Sosa et al., 1983). Genomic hypomethylation might contribute to cancer development and progression through different mechanisms including generation of chromosomal instability, reactivation of transposable elements, and loss of imprinting (Esteller, 2008). Substantial decreases in the 5-methylcytosine content in the genome mainly reflect the hypomethylation of repetitive genomic sequences. Among these, the *Long Interspersed Nucleotide Element-1 (LINE-1)* family accounts for 17% of the human genomic DNA, is capable of transposition, and its methylation level may be utilized as a surrogate for the overall level of genomic DNA methylation (Yang et al., 2004). In accordance with the proposed role of genomic DNA hypomethylation in cancer biology, preliminary studies have reported *LINE-1* hypomethylation to be associated with a reduced overall (OS) and cancer-specific survival in patients with colorectal and ovarian cancer (Ogino et al., 2008; Pattamadilok et al., 2008). However, these studies did not investigate the role of *LINE-1* methylation as a prognostic factor in patients in specific stages of disease, but rather looked at the patients' population as a whole. Besides, no literature data is available on the prognostic role *of LINE-1* in CM.

In the application WO 2008/134596, the methylation status of *LINE-1* in breast, colorectal, esophageal and prostate cancer as well as in melanoma was investigated. No significant difference in *LINE-1* hypomethylation level was observed between primary melanoma and adjacent normal skin (Fig. 29), nor between different stages of disease other than between stage 1 and stage 4 (Fig. 31). A significantly higher *LINE-1* hypomethylation of metastatic CM vs adjacent normal skin or primary CM (Fig. 30), and of stage 4 CM vs primary CM or adjacent normal skin (Fig. 31) was observed. The application does not disclose any correlation between *LINE-1* methylation level and survival variables (OS, cancer-specific, or disease-free survival) in cancer patients. In addition, it does not provide neither suggest evidence of the existence of molecular markers able to predict different survivals of patients, in general, and, most importantly, of individuals within identical clinico-pathological stages of disease.

By contrast, the authors of the present invention examined the prognostic role of *LINE-1* methylation status in survival of CM patients, in particular at identical stages of the disease. The authors found that within identical clinico-pathological stage of disease (e.g., stage IIIC), CM patients which neoplastic cells have a higher *LINE-1* hypomethylation survive significantly longer, and a higher *LINE-1* hypomethylation level represents a positive, independent, prognostic factor for CM patients.

Besides the overall hypomethylation of genomic DNA discussed above, the aberrant gene-specific hypermethylation observed in human malignancies might dramatically impact their biology by affecting different key cellular pathways involved in cancer development and progression (Esteller, 2008). Along this line, initial evidences in CM have demonstrated that this malignancy is characterized by a profound epigenetic deregulation, characterized by the transcriptional silencing of over 50 genes through the aberrant hypermethylation of CpG islands located within their promoter regions (Sigalotti et al., 2010). The inactivation of these genes affects diverse cellular pathways including cellular proliferation/differentiation, apoptosis, DNA repair, extracellular matrix interaction, metastatization and immune recognition, and is likely to play a relevant role in CM development and progression (Sigalotti et al., 2010). Accordingly, initial studies, conducted on very small cohorts of patients, have examined the prognostic potential of the methylation status of single or limited panels of genes in CM, showing an association between the methylation of the Methylated IN Tumors locus 31 (MINT31) in tumor tissues and disease outcome in stage III patients, and a negative correlation between presence of circulating methylated DNA for Ras Association (RalGDS/AF-6) domain Family member 1A (RASSF1A) and Estrogen Receptor alpha (ER-α) and survival of CM patients receiving biochemotherapy (Mori et al., 2006; Mori et al., 2005; Tanemura et al., 2009). Despite these initial evidences, no study has investigated the genome-wide gene methylation profile in CM patients and its prognostic/predictive potential. Thus, the authors have utilized a whole-genome approach to identify genes or gene signatures which methylation status has a prognostic/predictive role in CM, paying particular emphasis in identifying multi-gene methylation signatures able to discriminate survival of CM patients at identical stages of disease. With this approach the authors were able to define distinct methylation signatures that were able to sort stage III CM patients into categories with highly significantly different survival rates. Even more important, the gene methylation signatures the authors defined enable to identify patients with significantly different survival also in subsatges of disease (e.g., stage IIIC), which currently represent the upper limit of our ability to segregate patients into groups characterized by different clinical outcomes using the tools that are at present available. In this context, the concomitant evaluation of both the extent of *LINE-1* methylation and of the gene-methylation signature(s), proved to have a further increased power to discriminate survival of patients as compared to each marker alone.

In consideration of such arguments, our invention has a significant industrial transferability to the clinical setting, where it will provide a key contribution in guiding the daily clinical management of CM patients.

### SUMMARY OF THE INVENTION

*LINE-1* sequences account for a substantial portion of the genome and are usually heavily methylated in normal somatic tissues. Reduced levels of *LINE-1* methylation (i.e. *LINE-1* hypomethylation) significantly contribute to the well-known global genomic DNA hypomethylation occurring in cancer tissues. Besides *LINE-1* hypomethylation, neoplastic tissues are characterized by a genome-wide alteration of gene methylation patterns that appears to profoundly contribute to the neoplastic phenotype. The inventors developed a method to predict survival in CM patients at identical stage of disease through the evaluation of: i) *LINE-1* methylation status; ii) multiple gene methylation signatures; iii) combined *LINE-1* methylation and multiple gene methylation signatures.

Assessment of neoplastic cells from CM patients within the same clinico-pathological stage of disease demonstrated a significant association between higher *LINE-1* hypomethylation and longer OS. *LINE-1* hypomethylation represents an independent factor predicting a more favorable prognosis of CM patients (Example 1). *LINE-1* hypomethylation can be used as a surrogate marker for the expression of different methylation regulated molecules, such as tumor-associated antigens, that are relevant for cancer therapy (Example 2).

Assessing with high-throughput approaches the genome-wide gene methylation status in neoplastic cells of CM patients at the same stage of disease, the authors have defined multigene methylation signatures that identify groups with significantly different OS within stage III and IIIC patient cohorts (Table 9, Example 3). In particular, the differential methylation pattern of: **i) signature A:** MGC29671, GJB5, KCNK16, IGLL1, ANXA8, OR12D3, TUB, CST11, TMEM10, CRHR1, TRIM40, GAS2L2, FOXN1, FXYD4, KRTHB2, FLJ33860, S100A9, FOXI1, GUCA2B, CYP2B6, IL8RA, CYP8B1, WFDC8, and SLPI genes identifies 2 classes of stage III patients with significantly different survivals; **ii) signature B:** FLJ25421, LAMC2, Clorf36, MGC29671, GJB5, KCNK16, ANGPTL3, IGLL1, DEFB4, STK38, SEMG2, ANXA8, OR12D3, PREPL, IL17F, DCLRE1A, FLJ20581, PTCD2, CX3CR1, TUB, UBE2E1, CST11, IFT140, TMEM10, SLC22A8, MLN, DIRC1, Clorf64, CRHR1, TRIM40, TNFSF13B, GAS2L2, FOXN1, CYP2B6, ACSL5, SERPINB12, APOBEC4, TNNI1, C1orf42, FXYD4, PPAT, PSD4, SCN4A, KRTHB2, IL8RB, UNC45B, C1orf201, SFTPG, LOC124751, FLJ33860, ANKRD1, FMO2, ARSG, GJB4, S100A9, LRRN5, MSMB, HLA-DOB, R3HDML, SPINK5, DLG5, PALLD, WFDC12, WDR20, FLJ32771, PKHD1, FOXI1, DEFB123, KRTHB3, GUCA2B, CYP2B6, BSND, NALP10, NR5A2, KRT25D, IL8RA, APOD, CLEC4F, THRB, KRTHB5, CYP8B1, WFDC8, SLC13A1, C3orf27, MGC35206, C3orf63, DARC, SLPI, SLC2A7, CPA3, MIS12, KRTHB4, MRVI1, MIA2, CYP2A7, ANGPT4, SLC44A5, and CST9L identifies 3 classes of stage III patients with significantly different survivals; **iii) signature C:** SLC18A2, GJB5, SLC6A18, IGLL1, ALOX12B, WNT10B, TUB, S100A9, CRHR1, TRIM40, SLC6A11, FLJ33860, FGF4, S100A9, MGC35206, PCDHAC2, and GRM4 identifies 2 classes of stage IIIC patients with significantly different survivals; **iv) signature D:** FLJ25421, ANXA13, SLC18A2, CTAG2, GJB5, KCNK16, SLC6A18, STK38, PVALB, OR12D3, ALOX12B, HA02, DCLRE1A, WNT10B, TUB, UBE2E1, IFT140, SLC4A7, S100A9, DIRC1, CRHR1, TRIM40, SLC6A11, TDRD5, ACSL5, APOBEC4, FAAH, CD1D, C1orf201, FLJ33860, ANKRD1, FGF4, FLJ39501, S100A9, LRRN5, GLT1D1, IL28B, AVPR1B, GJB3, MGC35206, PCDHAC2, GABRG3, MIA2, CTAG1B, UNQ9433, GRM4, and CEACAM6 identifies 3 classes of stage IIIC patients with significantly different survivals. Furthermore, the concomitant evaluation of whole genome expression and methylation profiles allowed to identify 2 multi-gene methylation signatures of 51 and 73 genes that were constituted by the sole genes displaying a significant (p<0.05) correlation between expression and methylation. These latter signatures identify groups with significantly different OS within IIIC patient cohorts (Table 10, example 3). Specifically, the differential methylation pattern of: **i) signature E:** DENND2D, CTAG2, PGCP, KCNG1, FAM107B, FANCE, FAM38B, FOXF2, BGN, MAGEA10, SPATS1, PRRX1, MLSTD1, PDZD4, HCP5, OCIAD2, CSAG1, SLC04A1, C9orf90, CSAG1, AMPH, MT1G, C20orf100, CST7, KIAA0367, RNASE1, HCP5, NDN, RAC2, AXL, BATF, EMILIN1, ARHGEF7, EVL, RAC2, KIAA0367, TFF3, CTSK, C21orf70, PRKCZ, MLSTD1, COL5A2, LY96, MAGEA1, MAGEC2, DENND2D, PPIE, SORBS2, PROCR, TMEPAI, SSNA1, and ZNF324 identifies 2 classes of stage IIIC patients with significantly different survivals; and **ii) signature F:** FLJ25421, DENND2D, CTAG2, PGCP, KCNG1, MGMT, FAM107B, FANCE, GAGE2, C11orf1, ABCB4, BGN, MAGEA10, SPATS1, CTNNAL1, PRRX1, EMP1, RNASE1, PDZD4, MAGEA6, CHMP4A, ADORA2B, HCP5, PLA1A, KIAA0141, OLFM1, ERO1L, CSAG1, SLCO4A1, CXorf12, C9orf90, CSAG1, AMPH, MT1G, ARHGDIB, C20orf100, CST7, KIAA0367, RNASE1, HCP5, NDN, RAC2, AXL, GRAMD3, ARSG, PLD1, HNMT, ZNF558, ATP6V1E2, EMILIN1, ARHGEF7, KRT25C, ZNF532, EVL, RAC2, SLAMF9, NBL1, KIAA0367, MAGEA10, TFF3, C21orf70, APOD, ABCA8, ACOX2, COL5A2, LY96, MAGEA1, MAGEC2, TIMP3, PPIE, RPUSD3, PROCR, and TMEPAI identifies 3 classes of stage IIIC patients with significantly different survivals. When, among the genes having significant (p<0.05) association between expression and methylation status, only those with a coefficient of correlation Rho<-0.4 were taken into account, an additional signature of 35 genes, **signature G:** PRDX2, DENND2D, CTAG2, PCOLCE, BGN, OCIAD2, RNASE1, MAGEA6, HCP5, OCIAD2, CSAG1, C9orf90, CSAG1, CTSK, ADM, RNASE1, HCP5, PAGE5, NNMT, EMILIN1, MAGEA3, RAB11FIP5, ARHGDIB, EMILIN1, LPXN, CTSK, ALDH1A3, SUHW1, ABCA8, COL5A2, LY96, MAGEA1, MAGEC2, GSTT1, DENND2D was defined, which identifies 2 classes of stage IIIC patients with significantly different survivals. The identified multi-gene methylation signatures represent independent factors predicting prognosis of CM patients.

In addition to the prognostic value of the multi-gene methylation signatures above described, the methylation status of specific genes taken from these signatures may also have a prognostic impact *per se*, as exemplified in Example 4.

The extent of hypomethylation of *LINE-1* sequences and/or the methylation pattern of the (multi-)gene methylation signatures can be used, alone, or in conjunction with other biomarkers, to predict prognosis and response to therapy of CM patients, thus enabling the administration of patient-tailored clinical, follow-up and therapeutic procedures.

Accordingly, the invention provides a method of prognosing cutaneous melanoma or of predicting and monitoring the efficacy of a cutaneous melanoma therapy comprising measuring *LINE-1* methylation and multi-gene methylation signatures in neoplastic cells. The term, "neoplastic cells" refers to any source of neoplastic cells from CM patients, including, but not limited to, primary cultures of autologous neoplastic cells, neoplastic cells purified from fresh autologous tumor tissues or from cryopreserved cell suspensions by immunosorting (e.g. using antibodies specific for cancer cells) or other means, cryopreserved tumor tissues, formalin fixed paraffin embedded (FFPE) tumor tissues, or other types of fixed tumor tissue, micro-/macro-dissected tumor tissues, circulating neoplastic cells present in body fluids (Table 13, Example 6). Neoplastic cells may be obtained from a subject by any means known in the art.

As used herein, a "subject" is defined as a human. In a preferred embodiment the subject is a human with cancer. "Patient" refers to a human with cancer.

Methods for extracting genomic DNA are well-known in the art. Depending on the source of neoplastic cells, a preliminary step to remove embedding substances by organic solvents may be required. Classically, neoplastic cells from the subject are lysed using detergents and proteins are removed by different proteases. DNA is then purified from cell lysates either through extraction with organic solvents (e.g. phenol) or by affinity binding on silica-gel matrixes. DNA is then either precipitated with alcohols and dissolved in aqueous solutions, or washed with different solutions and eluted in aqueous solutions. Similar procedures are used to obtain genomic DNA from biologic fluids or circulating neoplastic cells.

The extent of methylation of *LINE-1* is then measured on the DNA extracted from neoplastic cells. Different methods can be used to determine the extent of methylation of *LINE-1,* and are well-known in the art. These include, but are not limited to, Methylight, bisulfite sequencing, bisulfite pyrosequencing, Combined Bisulfite Restriction Analysis (CoBRA). Level of methylation *of LINE-1* is typically defined as the ratio between the level of methylated *LINE-1* and the level of methylated or unmethylated *LINE-1,* but any other mathematical formula relating to the level *of LINE-1* methylation also applies. Furthermore, level of methylation of *LINE-1* may refer to the extent of methylation at a single or multiple CpG sites as well as to aggregating measurements of methylation of multiple CpG sites, including, but not limited to, mean, median, mode. Similarly, the methylation status of all or any of the genes included in the 7 methylation signatures is measured on the DNA extracted from neoplastic cells. Different methods can be used to determine the extent of methylation of the multigene methylation signatures, and are well-known in the art. These include, but are not limited to, microarray-based methylation analysis, Methylight, bisulfite sequencing, bisulfite pyrosequencing, CoBRA. Level of methylation of each sequence/gene included in the signature is typically defined as the ratio between the level of methylated target sequence and the level of methylated or unmethylated target sequence, but any other mathematical formula relating to the level of gene/sequence methylation also applies. Furthermore, level of methylation of any gene/sequence included in the signature may refer to the extent of methylation at a single or multiple CpG sites as well as to aggregating measurements of methylation of multiple CpG sites, including, but not limited to, mean, median, mode.

MethyLight is the quantitative implementation of Methylation Specific PCR (MSP) using real-time PCR technology. MSP is a PCR-based technique that amplifies the target sequence depending on its methylation status. The approach requires an initial chemical modification of genomic DNA by incubation with sodium bisulfite, which converts unmethylated cytosines (C) to uracil (U), leaving methylated ones unaltered. This methylation-specific difference in the primary sequence generated by treatment is exploited to design oligonucleotide primers specific for either methylated or unmethylated target DNA, which are then employed in PCR-amplification reactions to discriminate between methylated and unmethylated sequences. Performing MSP reactions through real-time quantitative PCR approaches allows an efficient and sensitive quantification of the extent of methylation of the target sequence (MethyLight). Furthermore, when performing MethyLight using fluorogenic hybridization probe approaches (e.g., TaqMan® technology), the quantitative discrimination between methylated and unmethylated sequences may rely on selectivity for different methylation status of primers, probe or both.

Bisulfite sequencing allows to determine the methylation status of each CpG dinucleotide in the target sequence by conducting standard sequencing analysis. The technique relies on the differences in the sequence between methylated and unmethylated DNA that are generated by bisulfite treatment. The sequence of interest is PCR-amplified typically with primers amplifying all states, irrespective of methylation status. Usually, PCR products are cloned into plasmid vectors and single colonies are subjected to DNA sequencing through different methodologies available in the art. The extent of methylation at each CpG site is then typically established by evaluating the percentage of colonies with methylated CpG (i.e., C base in sense strand sequence).

Bisulfite pyrosequencing. Pyrosequencing is a high-throughput nonelectrophoretic nucleotide extension sequencing technology for various applications including DNA methylation analysis. Pyrosequencing-based methylation analysis investigates quantitatively the degree of methylation at CpG positions after treatment of genomic DNA with sodium bisulfite. The target region is amplified by PCR using a pair of primers complementary to the bisulfite-treated DNA sequence, amplifying all states, irrespective of methylation status. Usually, one of these two amplification primers carries a biotin label at the 5'-end that is exploited to subsequently purify the PCR product by immobilization on streptavidin-coated sepharose beads. Immobilized PCR product is then rendered single-stranded by alkali and hybridized to a pyrosequencing primer complementary to a region in close proximity to the sequence for which methylation data is going to be analyzed. The pyrosequencing reaction is performed by the sequential addition of single nucleotides in a predefined order. Each incorporation event is accompanied by release ofpyrophosphate (PPi) in a quantity equimolar to the amount of nucleotide incorporated. ATP sulfurylase quantitatively converts PPi to ATP in the presence of adenosine 5' phosphosulfate. ATP then drives the luciferase-mediated conversion of luciferin to oxyluciferin that generates visible light in amounts that are proportional to the amount of ATP. Light emission is detected by an instrument and reported as a peak which area represents the quantity of the respective nucleotide. The level of methylation at each CpG position in a sequence is usually determined from the ratio of C over the sum of C and T.

CoBRA is a technique that enables quantitative evaluation of DNA methylation by exploiting the sequence differences that exist between methylated and unmethylated DNA upon treatment with sodium bisulfite. In particular, it relies on the generation, or suppression, of a recognition site for restriction enzymes depending on the methylation status of the sequence under analysis. The target region is amplified by PCR using a pair of primers complementary to the bisulfite-treated DNA sequence, amplifying all states, irrespective of methylation status. After amplification, PCR products are digested with a specific restriction enzyme that cuts only DNA molecules that were methylated (or unmethyated) before bisulfite treatment. Digestion products are then separated by conventional, microfluidic or capillary electrophoresis, and the proportion of digested versus undigested products is quantitated and used to determine the extent of methylation at that particular site.

High-throughput DNA microarray technologies can be efficiently used to define genome-wide gene methylation profiles. Different methodologies either rely on the enrichment of genomic DNA for methylated sequences, through methylated DNA immunoprecipitation (MeDIP) approaches, or on the modification of the genomic DNA by sodium bisulfite. MeDIP can be conducted utilizing either anti-5-methylcytosine antibodies or proteins which selectively bind to methylated DNA (e.g., methyl-CpG-binding domain (MBD) proteins). Capturing of the genomic DNA with these reagents bound to an insoluble matrix allows to enrich the sample for methylated sequences. Classically, for each test sample, genomic DNA immunoprecipitated (IP) and DNA non-IP, utilized as control, are labelled with different fluorescently-coupled nucleotides (e.g., Cy5-dCTP for IP DNA and Cy3-dCTP for non-IP DNA), and co-hybridized to a microarray containing the probes for the sequence set of interest (e.g., CpG island arrays representing over 27,000 CpG sites distributed genome-wide). Fluorescence intensity data are then extracted from the scanned microarrays, pre-processed and analyzed using different software packages to provide a methylation score for each analyzed CpG site. On the other hand, the treatment of genomic DNA with sodium bisulfite translates the methylation data into primary DNA sequence modifications that are interrogated using oligonuclotide probes. Typically, bisulfite-modified genomic DNA is evaluated at each locus through hybridization of a pair of oligonuclotide probes constituted by one probe specific for the methylated and one specific for the unmethylated target sequence. Probes can be elongated by a polymerase only in case of match, so that probes for methylated or unmethylated sequences are quantitatively elongated only in the presence of methylated or unmethylated sequences in the test genomic DNA, respectively. A classic application employs single-base probe extension using fluorescently-labelled nucleotides to reveal the methylation status at each locus. The level of methylation for the interrogated locus can be determined by calculating the ratio of the fluorescent signals from the methylated vs unmethylated probes. In a typical application, this technology enables to interrogate the genome-wide methylation status of over 27,000 CpG sites covering over 14,000 genes. Besides microarray technology, the identity of methylated sequences can also be determined by applying next generation sequencing approaches to the MeDIP-enriched or the bisulfite-modified genomic DNA. The identification of methylation profiles (multi-gene methylation signatures) specifically associated with different prognosis (e.g., OS, time to progression (TTP), CM-specific survival) of patients may be obtained through different approaches including, but not limited to, that illustrated in Example 3.

The invention further provides a method for determining prognosis (including, but not limited to, OS, disease- and progression-free survival, CM-specific survival) of subjects with CM. In one such method, a neoplastic cell sample is obtained from a subject and the level of *LINE-1* methylation is determined. If the level of *LINE-1* methylation in the sample is lower than a reference value, the subject survives longer. On the contrary, if the level of *LINE-1* methylation in the sample is higher than a reference value, the subject survives less. The reference value of *LINE-1* methylation is determined in a test population of subjects with cancer as the value that more specifically and sensitively discriminates patients with different survivals. This reference value can be defined through any approach available in the art, including, but not limited to, receiver-operator characteristic (ROC) curve analysis, mean, median, mode value of *LINE-1* methylation of neoplastic cells from the population of cancer patients under evaluation. In an additional method for determining prognosis, a neoplastic cell sample is obtained from a subject affected by CM, and the methylation profile of any, multiple or all of the genes included in the multi-gene methylation signatures (Table 1, 2) is evaluated. The methylation profile obtained from the neoplastic cells of the patient is then fed into a methylation-based classifier algorithm that will assign patients at the same stage of disease into either a high- or a low-risk group. Patients assigned to the high-risk group based on their (multi-)gene methylation profile will survive less, while patients assigned to the low-risk group will survive longer. Furthermore, combining both *LINE-1* methylation and gene methylation signature classifications of patients results in a stronger prognostic marker, which, for instance, predicts that patients whose neoplastic cells have *LINE-1* methylation lower than a reference value and a low-risk methylation signature have a longer survival (Example 5).

In another method of determining prognosis, a biological fluid sample is obtained from a subject and is evaluated for *LINE-1* methylation and/or for (multi-)gene methylation signatures. A "biological fluid" refers to any fluid present in the subject in which acellular DNA or cancer cells may be present, including, without limitation, blood, serum, plasma, bone marrow, cerebrospinal fluid, peritoneal/pleural fluid, lymph fluid, ascitic, serous fluid, sputum, lacrimal fluid, stool, and urine. Body fluid samples can be obtained from a subject using any of the methods known in the art.

Accordingly to the above reported role of *LINE-1* methylation level and of (multi)gene methylation signatures as prognostic markers, the invention includes the measurement and use of *LINE-1* methylation and/or of (multi-)gene methylation signatures to define differentiated therapeutic treatments and differentiated follow-up procedures and schedules for CM patients, according to their different prognosis as defined by *LINE-1* methylation and/or (multi-)gene methylation signatures.

The invention further provides *LINE-1* methylation level as a surrogate marker of presence and levels of expression in neoplastic cells of different therapeutic targets regulated by DNA methylation, including, but not limited to, Cancer Testis Antigens of the MAGE, GAGE, NY-ESO, SSX families, High Molecular Weight-Melanoma Associated Antigen, class I and II Human Leukocyte Antigens, and Intercellular Adhesion Molecule-1. In such a method, a neoplastic cell sample is obtained from a subject/patient and the level of *LINE-1* methylation is determined. If the level of *LINE-1* methylation in the sample is lower than a reference value, neoplastic cells are likely to express a higher number of distinct methylation-regulated therapeutic targets and/or increased levels of their expression. As a consequence, this population of patients is most likely to respond to any therapeutic treatment (including, but not limited to, chemotherapy, immunotherapy, biologic therapy, radiotherapy and targeted therapy utilized alone or in combination) involving methylation-regulated molecules, and thus it represents the preferred cohort of individuals to be treated with these therapies utilizing a patient-tailored approach. Accordingly, the invention includes the use of the evaluation of the extent of *LINE-1* methylation to identify patients to be treated with therapeutic approaches targeting one or multiple DNA methylation-regulated molecules, including, but not limited to, Cancer Testis Antigens of the MAGE, GAGE, NY-ESO, SSX families, High Molecular Weight-Melanoma Associated Antigen, class I and II Human Leukocyte Antigens, and Intercellular Adhesion Molecule-1.

The invention also provides a method for predicting response to therapy. In such a method, a neoplastic cell sample is obtained from a subject/patient and the level of *LINE-1* methylation and/or the (multi-)gene methylation signatures are determined. The level of *LINE-1* methylation and/or the (multi-)gene methylation signature is associated with the fact that the patient is more likely to respond better to different therapeutic approaches, including, but not limited to, immunotherapy, chemotherapy, radiotherapy, biologic therapy, targeted therapy utilized alone or in combination. Accordingly, the invention includes the use of the level of *LINE-1* methylation and or of (multi-)gene methylation signatures for selecting patients to be treated with specific therapeutic approaches.

Patients whose neoplastic cells have a level of *LINE-1* methylation lower than a reference value have a more favorable prognosis, surviving longer. Similarly, patients whose neoplastic cells have more hypomethylated multi-gene methylation signatures (see Example 3) are characterized by a longer survival. Accordingly, one aspect of the invention includes the administration of compounds able to reduce the extent of methylation of genomic DNA (DNA hypomethylating agents, DHA) in cancer patients whose neoplastic cells have levels of *LINE-1* methylation higher than a reference value and/or hypermethylated (multi-)gene methylation signatures. The hypomethylation of genomic DNA, of *LINE-1* DNA, and of the genes included in the methylation signatures in neoplastic cells of these treated patients is expected to drive cells to a less aggressive phenotype and to provide patients with a longer survival. Treatment of patients may be done with DHA alone or in combination with any other therapeutic option available. In one, not limiting, embodiment, pharmacologic DNA hypomethylation could be used as a feasible chemoprevention approach in the initial phases of disease and/or in patients at high-risk of disease recurrence.

It is therefore an object of the present invention an in vitro method of prognosing melanoma and/or of predicting and/or monitoring the response and/or the efficacy of a melanoma therapy or to identify a subject to be treated with a DNA hypomethylation agent comprising determining the methylation status of at least one cytosine residue in a methylation relevant region of at least one gene selected from any one of ABCA8, ABCB4, ACOX2, ACSL5, ADM, ADORA2B, ALDH1A3, ALOX12B, AMPH, ANGPT4, ANGPTL3, ANKRD1, ANXA13, ANXA8, APOBEC4, APOD, ARHGDIB, ARHGEF7, ARSG, ATP6V1E2, AVPR1B, AXL, BATF, BGN, BSND, C11orf1, C1orf201, C1orf36, C1orf42, C1orf64, C20orf100, C21orf70, C3orf27, C3orf63, C9orf90, CD1D, CEACAM6, CHMP4A, CLEC4F, COL5A2, CPA3, CRHR1, CSAG1, CST11, CST7, CST9L, CTAG1B, CTAG2, CTNNAL1, CTSK, CX3CR1, CXorf12, CYP2A7, CYP2B6, CYP8B1, DARC, DCLRE1A, DEFB123, DEFB4, DENND2D, DIRC1, DLG5, EMILIN1, EMP1, ERO1L, EVL, FAAH, FAM107B, FAM38B, FANCE, FGF4, FLJ20581, FLJ25421, FLJ32771, FLJ33860, FLJ39501, FMO2, FOXF2, FOXI1, FOXN1, FXYD4, GABRG3, GAGE2, GAS2L2, GJB3, GJB4, GJB5, GLT1D1, GRAMD3, GRM4, GSTT1, GUCA2B, HA02, HCP5, HLA-DOB, HNMT, IFT140, IGLL1, IL17F, IL28B, IL8RA, IL8RB, KCNG1, KCNK16, KIAA0141, KIAA0367, KRT25C, KRT25D, KRTHB2, KRTHB3, KRTHB4, KRTHB5, LAMC2, LOC124751, LPXN, LRRN5, LY96, MAGEA1, MAGEA10, MAGEA3, MAGEA6, MAGEC2, MGC29671, MGC35206, MGMT, MIA2, MIS12, MLN, MLSTD1, MRVI1, MSMB, MT1G, NALP10, NBL1, NDN, NNMT, NR5A2, OCIAD2, OLFM1, OR12D3, PAGE5, PALLD, PCDHAC2, PCOLCE, PDZD4, PGCP, PKHD1, PLA1A, PLD1, PPAT, PPIE, PRDX2, PREPL, PRKCZ, PROCR, PRRX1, PSD4, PTCD2, PVALB, R3HDML, RAB11FIP5, RAC2, RNASE1, RPUSD3, S100A9, SCN4A, SEMG2, SERPINB12, SFTPG, SLAMF9, SLC13A1, SLC18A2, SLC22A8, SLC2A7, SLC44A5, SLC4A7, SLC6A11, SLC6A18, SLC04A1, SLPI, SORBS2, SPATS1, SPINK5, SSNA1, STK38, SUHW1, TDRD5, TFF3, THRB, TIMP3, TMEM10, TMEPAI, TNFSF13B, TNNI1, TRIM40, TUB, UBE2E1, UNC45B, UNQ9433, WDR20, WFDC12, WFDC8, WNT10B, ZNF324, ZNF532, ZNF558 and the *LINE-1* repetitive sequence or a variant thereof in a biological sample obtained from the subject.

Preferably, the method comprises determining the methylation status of at least one cytosine residue in each of the methylation relevant region of the genes selected from: ANXA8, CRHR1, CST11, CYP2B6, CYP8B1, FLJ33860, FOXI1, FOXN1, FXYD4, GAS2L2, GJB5, GUCA2B, IGLL1, IL8RA, KCNK16, KRTHB2, MGC29671, OR12D3, S100A9, SLPI, TMEM10, TRIM40, TUB, and WFDC8, as indicated in Table 1 signature A.

Preferably, the method comprises determining the methylation status of at least one cytosine residue in each of the methylation relevant region of the genes selected from: ACSL5, ANGPT4, ANGPTL3, ANKRD1, ANXA8, APOBEC4, APOD, ARSG, BSND, Clorf201, Clorf36, C1orf42, C1orf64, C3orf27, C3orf63, CLEC4F, CPA3, CRHR1, CST11, CST9L, CX3CR1, CYP2A7, CYP2B6, CYP8B1, DARC, DCLRE1A, DEFB123, DEFB4, DIRC1, DLG5, FLJ20581, FLJ25421, FLJ32771, FLJ33860, FMO2, FOXI1, FOXN1, FXYD4, GAS2L2, GJB4, GJB5, GUCA2B, HLA-DOB, IFT140, IGLL1, IL17F, IL8RA, IL8RB, KCNK16, KRT25D, KRTHB2, KRTHB3, KRTHB4, KRTHB5, LAMC2, LOC124751, LRRN5, MGC29671, MGC35206, MIA2, MIS12, MLN, MRVI1, MSMB, NALP10, NR5A2, OR12D3, PALLD, PKHD1, PPAT, PREPL, PSD4, PTCD2, R3HDML, S100A9, SCN4A, SEMG2, SERPINB12, SFTPG, SLC13A1, SLC22A8, SLC2A7, SLC44A5, SLPI, SPINK5, STK38, THRB, TMEM10, TNFSF13B, TNNI1, TRIM40, TUB, UBE2E1, UNC45B, WDR20, WFDC12 andWFDC8 as indicated in Table 1 signature B.

Preferably, the method comprises determining the methylation status of at least one cytosine residue in each of the methylation relevant region of the genes selected from: ALOX12B, CRHR1, FGF4, FLJ33860, GJB5, GRM4, IGLL1, MGC35206, PCDHAC2, S100A9, SLC18A2, SLC6A11, SLC6A18, TRIM40, TUB and WNT10B as indicated in Table 1 signature C.

Preferably, the method comprises determining the methylation status of at least one cytosine residue in each of the methylation relevant region of the genes selected from: ACSL5, ALOX12B, ANKRD1, ANXA13, APOBEC4, AVPR1B, C1orf201, CD1D, CEACAM6, CRHR1, CTAG1B, CTAG2, DCLRE1A, DIRC1, FAAH, FGF4, FLJ25421, FLJ33860, FLJ39501, GABRG3, GJB3, GJB5, GLT1D1, GRM4, HA02, IFT140, IL28B, KCNK16, LRRN5, MGC35206, MIA2, OR12D3, PCDHAC2, PVALB, S100A9, SLC18A2, SLC4A7, SLC6A11, SLC6A18, STK38, TDRD5, TRIM40, TUB, UBE2E1, UNQ9433 and WNT10B as indicated in Table 1 signature D.

Preferably, the method comprises determining the methylation status of at least one cytosine residue in each of the methylation relevant region of the genes selected from: AMPH, ARHGEF7, AXL, BATF, BGN, C20orf100, C21orf70, C9orf90, COL5A2, CSAG1, CST7, CTAG2, CTSK, DENND2D, EMILIN1, EVL, FAM107B, FAM38B, FANCE, FOXF2, HCP5, KCNG1, KIAA0367, LY96, MAGEA1, MAGEA10, MAGEC2, MLSTD1, MT1G, NDN, OCIAD2, PDZD4, PGCP, PPIE, PRKCZ, PROCR, PRRX1, RAC2, RNASE1, SLC04A1, SORBS2, SPATS1, SSNA1, TFF3, TMEPAI and ZNF324 as indicated in Table 1 signature E.

Preferably, the method comprises determining the methylation status of at least one cytosine residue in each of the methylation relevant region of the genes selected from: ABCA8, ABCB4, ACOX2, ADORA2B, AMPH, APOD, ARHGDIB, ARHGEF7, ARSG, ATP6VIE2, AXL, BGN, C11orf1, C20orf100, C21orf70, C9orf90, CHMP4A, COL5A2, CSAG1, CST7, CTAG2, CTNNAL1, CXorf12, DENND2D, EMILIN1, EMP1, ERO1L, EVL, FAM107B, FANCE, FLJ25421, GAGE2, GRAMD3, HCP5, HNMT, KCNG1, KIAA0141, KIAA0367, KRT25C, LY96, MAGEA1, MAGEA10, MAGEA6, MAGEC2, MGMT, MT1G, NBL1, NDN, OLFM1, PDZD4, PGCP, PLA1A, PLD1, PPIE, PROCR, PRRX1, RAC2, RNASE1, RPUSD3, SLAMF9, SLC04A1, SPATS1, TFF3, TIMP3, TMEPAI, ZNF532 and ZNF558 as indicated in Table 1 signature F.

Preferably, the method comprises determining the methylation status of at least one cytosine residue in each of the methylation relevant region of the genes selected from: ABCA8, ADM, ALDH1A3, ARHGDIB, BGN, C9orf90, COL5A2, CSAG1, CTAG2, CTSK, DENND2D, EMILIN1, GSTT1, HCP5, LPXN, LY96, MAGEA1, MAGEA3, MAGEA6, MAGEC2, NNMT, OCIAD2, PAGE5, PCOLCE, PRDX2, RAB11FIP5, RNASE1 and SUHW1 as indicated in Table 1 signature G.

Preferably, the method further comprises determining the methylation status of at least one cytosine residue in the methylation relevant region of the *LINE-1* repetitive sequence.

In a preferred embodiment, the methylation relevant region of said genes comprises a nucleotide sequence selected from SEQ ID No 1 to SEQ ID No. 220 as shown in Table 2 , an allelic variant or a complementary sequence thereof.

Still preferably the methylation relevant region of said genes consists in a nucleotide selected from nt 941 to nt 1062 of SEQ ID No. 1, nt 940 to nt 1061 of SEQ ID No. 2, nt 940 to nt 1061 of SEQ ID No. 3, nt 941 to nt 1062 of SEQ ID No. 4, nt 940 to nt 1061 of SEQ ID No. 5, nt 940 to nt 1061 of SEQ ID No. 6, nt 940 to nt 1061 of SEQ ID No. 7 , nt 941 to nt 1062 of SEQ ID No. 8, nt 941 to nt 1062 of SEQ ID No. 9, nt 941 to nt 1062 of SEQ ID No. 10, nt 940 to nt 1061 of SEQ ID No. 11, nt 940 to nt 1061 of SEQ ID No. 12, nt 941 to nt 1062 of SEQ ID No. 13, nt 940 to nt 1061 of SEQ ID No. 14, nt 941 to nt 1062 of SEQ ID No. 15, nt 940 to nt 1061 of SEQ ID No. 16, nt 940 to nt 1061 of SEQ ID No. 17, nt 941 to nt 1062 of SEQ ID No. 18, nt 940 to nt 1061 of SEQ ID No. 19, nt 940 to nt 1061 of SEQ ID No. 20, nt 940 to nt 1061 of SEQ ID No. 21, nt 940 to nt 1061 of SEQ ID No. 22, nt 940 to nt 1061 of SEQ ID No. 23, nt 940 to nt 1061 of SEQ ID No. 24, nt 940 to nt 1061 of SEQ ID No. 25, nt 941 to nt 1062 of SEQ ID No. 26, nt 941 to nt 1062 of SEQ ID No. 27, nt 941 to nt 1062 of SEQ ID No. 28, nt 940 to nt 1061 of SEQ ID No. 29, nt 940 to nt 1061 of SEQ ID No. 30, nt 940 to nt 1061 of SEQ ID No. 31, nt 940 to nt 1061 of SEQ ID No. 32, nt 940 to nt 1061 of SEQ ID No. 33, nt 940 to nt 1061 of SEQ ID No. 34, nt 940 to nt 1061 of SEQ ID No. 35, nt 940 to nt 1061 of SEQ ID No. 36, nt 940 to nt 1061 of SEQ ID No. 37, nt 940 to nt 1061 of SEQ ID No. 38, nt 940 to nt 1061 of SEQ ID No. 39, nt 941 to nt 1062 of SEQ ID No. 40, nt 940 to nt 1061 of SEQ ID No. 41, nt 940 to nt 1061 of SEQ ID No. 42, nt 940 to nt 1061 of SEQ ID No. 43, nt 940 to nt 1061 of SEQ ID No. 44, nt 941 to nt 1062 of SEQ ID No. 45, nt 940 to nt 1061 of SEQ ID No. 46, nt 941 to nt 1062 of SEQ ID No. 47, nt 940 to nt 1061 of SEQ ID No. 48, nt 941 to nt 1062 of SEQ ID No. 49, nt 940 to nt 1061 of SEQ ID No. 50, nt 940 to nt 1061 of SEQ ID No. 51, nt 940 to nt 1061 of SEQ ID No. 52, nt 941 to nt 1062 of SEQ ID No. 53, nt 941 to nt 1062 of SEQ ID No. 54, nt 940 to nt 1061 of SEQ ID No. 55, nt 940 to nt 1061 of SEQ ID No. 56, nt 940 to nt 1061 of SEQ ID No. 57, nt 940 to nt 1061 of SEQ ID No. 58, nt 940 to nt 1061 of SEQ ID No. 59, nt 941 to nt 1062 of SEQ ID No. 60, nt 941 to nt 1062 of SEQ ID No. 61, nt 940 to nt 1061 of SEQ ID No. 62, nt 940 to nt 1061 of SEQ ID No. 63, nt 941 to nt 1062 of SEQ ID No. 64, nt 940 to nt 1061 of SEQ ID No. 65, nt 940 to nt 1061 of SEQ ID No. 66, nt 941 to nt 1062 of SEQ ID No. 67, nt 941 to nt 1062 of SEQ ID No. 68, nt 940 to nt 1061 of SEQ ID No. 69, nt 940 to nt 1061 of SEQ ID No. 70, nt 941 to nt 1062 of SEQ ID No. 71, nt 940 to nt 1061 of SEQ ID No. 72, nt 941 to nt 1062 of SEQ ID No. 73, nt 941 to nt 1062 of SEQ ID No. 74, nt 940 to nt 1061 of SEQ ID No. 75, nt 940 to nt 1061 of SEQ ID No. 76, nt 940 to nt 1061 of SEQ ID No. 77, nt 940 to nt 1061 of SEQ ID No. 78, nt 941 to nt 1062 of SEQ ID No. 79, nt 941 to nt 1062 of SEQ ID No. 80, nt 940 to nt 1061 of SEQ ID No. 81, nt 940 to nt 1061 of SEQ ID No. 82, nt 940 to nt 1061 of SEQ ID No. 83, nt 940 to nt 1061 of SEQ ID No. 84, nt 941 to nt 1062 of SEQ ID No. 85, nt 941 to nt 1062 of SEQ ID No. 86, nt 941 to nt 1062 of SEQ ID No. 87, nt 941 to nt 1062 of SEQ ID No. 88, nt 941 to nt 1062 of SEQ ID No. 89, nt 940 to nt 1061 of SEQ ID No. 90, nt 940 to nt 1061 of SEQ ID No. 91, nt 940 to nt 1061 of SEQ ID No. 92, nt 941 to nt 1062 of SEQ ID No. 93, nt 940 to nt 1061 of SEQ ID No. 94, nt 941 to nt 1062 of SEQ ID No. 95, nt 941 to nt 1062 of SEQ ID No. 96, nt 941 to nt 1062 of SEQ ID No. 97, nt 941 to nt 1062 of SEQ ID No. 98, nt 941 to nt 1062 of SEQ ID No. 99, nt 940 to nt 1061 of SEQ ID No. 100, nt 940 to nt 1061 of SEQ ID No. 101, nt 941 to nt 1062 of SEQ ID No. 102, nt 941 to nt 1062 of SEQ ID No. 103, nt 940 to nt 1061 of SEQ ID No. 104, nt 941 to nt 1062 of SEQ ID No. 105, nt 940 to nt 1061 of SEQ ID No. 106, nt 940 to nt 1061 of SEQ ID No. 107, nt 940 to nt 1061 of SEQ ID No. 108, nt 940 to nt 1061 of SEQ ID No. 109, nt 940 to nt 1061 of SEQ ID No. 110, nt 940 to nt 1061 of SEQ ID No. 111, nt 940 to nt 1061 of SEQ ID No. 112, nt 941 to nt 1062 of SEQ ID No. 113, nt 941 to nt 1062 of SEQ ID No. 114, nt 941 to nt 1062 of SEQ ID No. 115, nt 940 to nt 1061 of SEQ ID No. 116, nt 941 to nt 1062 of SEQ ID No. 117, nt 941 to nt 1062 of SEQ ID No. 118, nt 940 to nt 1061 of SEQ ID No. 119, nt 941 to nt 1062 of SEQ ID No. 120, nt 940 to nt 1061 of SEQ ID No. 121, nt 941 to nt 1062 of SEQ ID No. 122, nt 941 to nt 1062 of SEQ ID No. 123, nt 940 to nt 1061 of SEQ ID No. 124, nt 940 to nt 1061 of SEQ ID No. 125, nt 940 to nt 1061 of SEQ ID No. 126, nt 941 to nt 1062 of SEQ ID No. 127, nt 941 to nt 1062 of SEQ ID No. 128, nt 941 to nt 1062 of SEQ ID No. 129, nt 941 to nt 1062 of SEQ ID No. 130, nt 941 to nt 1062 of SEQ ID No. 131, nt 940 to nt 1061 of SEQ ID No. 132, nt 940 to nt 1061 of SEQ ID No. 133, nt 941 to nt 1062 of SEQ ID No. 134, nt 940 to nt 1061 of SEQ ID No. 135, nt 941 to nt 1062 of SEQ ID No. 136, nt 941 to nt 1062 of SEQ ID No. 137, nt 940 to nt 1061 of SEQ ID No. 138, nt 941 to nt 1062 of SEQ ID No. 139, nt 940 to nt 1061 of SEQ ID No. 140, nt 940 to nt 1061 of SEQ ID No. 141, nt 941 to nt 1062 of SEQ ID No. 142, nt 941 to nt 1062 of SEQ ID No. 143, nt 941 to nt 1062 of SEQ ID No. 144, nt 941 to nt 1062 of SEQ ID No. 145, nt 941 to nt 1062 of SEQ ID No. 146, nt 941 to nt 1062 of SEQ ID No. 147, nt 941 to nt 1062 of SEQ ID No. 148, nt 941 to nt 1062 of SEQ ID No. 149, nt 941 to nt 1062 of SEQ ID No. 150, nt 940 to nt 1061 of SEQ ID No. 151, nt 940 to nt 1061 of SEQ ID No. 152, nt 940 to nt 1061 of SEQ ID No. 153, nt 941 to nt 1062 of SEQ ID No. 154, nt 941 to nt 1062 of SEQ ID No. 155, nt 940 to nt 1061 of SEQ ID No. 156, nt 940 to nt 1061 of SEQ ID No. 157, nt 940 to nt 1061 of SEQ ID No. 158, nt 941 to nt 1062 of SEQ ID No. 159, nt 940 to nt 1061 of SEQ ID No. 160, nt 941 to nt 1062 of SEQ ID No. 161, nt 940 to nt 1061 of SEQ ID No. 162, nt 940 to nt 1061 of SEQ ID No. 163, nt 940 to nt 1061 of SEQ ID No. 164, nt 941 to nt 1062 of SEQ ID No. 165, nt 941 to nt 1062 of SEQ ID No. 166, nt 941 to nt 1062 of SEQ ID No. 167, nt 941 to nt 1062 of SEQ ID No. 168, nt 941 to nt 1062 of SEQ ID No. 169, nt 940 to nt 1061 of SEQ ID No. 170, nt 940 to nt 1061 of SEQ ID No. 171, nt 941 to nt 1062 of SEQ ID No. 172, nt 940 to nt 1061 of SEQ ID No. 173, nt 941 to nt 1062 of SEQ ID No. 174, nt 941 to nt 1062 of SEQ ID No. 175, nt 941 to nt 1062 of SEQ ID No. 176, nt 941 to nt 1062 of SEQ ID No. 177, nt 940 to nt 1061 of SEQ ID No. 178, nt 941 to nt 1062 of SEQ ID No. 179, nt 941 to nt 1062 of SEQ ID No. 180, nt 941 to nt 1062 of SEQ ID No. 181, nt 941 to nt 1062 of SEQ ID No. 182, nt 940 to nt 1061 of SEQ ID No. 183, nt 940 to nt 1061 of SEQ ID No. 184, nt 940 to nt 1061 of SEQ ID No. 185, nt 940 to nt 1061 of SEQ ID No. 186, nt 940 to nt 1061 of SEQ ID No. 187, nt 940 to nt 1061 of SEQ ID No. 188, nt 940 to nt 1061 of SEQ ID No. 189, nt 940 to nt 1061 of SEQ ID No. 190, nt 941 to nt 1062 of SEQ ID No. 191, nt 940 to nt 1061 of SEQ ID No. 192, nt 940 to nt 1061 of SEQ ID No. 193, nt 941 to nt 1062 of SEQ ID No. 194, nt 940 to nt 1061 of SEQ ID No. 195, nt 940 to nt 1061 of SEQ ID No. 196, nt 941 to nt 1062 of SEQ ID No. 197, nt 941 to nt 1062 of SEQ ID No. 198, nt 941 to nt 1062 of SEQ ID No. 199, nt 940 to nt 1061 of SEQ ID No. 200, nt 940 to nt 1061 of SEQ ID No. 201, nt 940 to nt 1061 of SEQ ID No. 202, nt 940 to nt 1061 of SEQ ID No. 203, nt 940 to nt 1061 of SEQ ID No. 204, nt 940 to nt 1061 of SEQ ID No. 205, nt 940 to nt 1061 of SEQ ID No. 206, nt 941 to nt 1062 of SEQ ID No. 207, nt 940 to nt 1061 of SEQ ID No. 208, nt 940 to nt 1061 of SEQ ID No. 209, nt 940 to nt 1061 of SEQ ID No. 210, nt 941 to nt 1062 of SEQ ID No. 211, nt 941 to nt 1062 of SEQ ID No. 212, nt 941 to nt 1062 of SEQ ID No. 213, nt 940 to nt 1061 of SEQ ID No. 214, nt 940 to nt 1061 of SEQ ID No. 215, nt 941 to nt 1062 of SEQ ID No. 216, nt 941 to nt 1062 of SEQ ID No. 217, nt 940 to nt 1061 of SEQ ID No. 218, nt 940 to nt 1061 of SEQ ID No. 219, nt 111 to nt 358 of SEQ ID No. 220, an allelic variant or a complementary sequence thereof.

In a preferred embodiment the at least one cytosine residue is selected from nt 1001 of SEQ ID No. 1, nt 1000 of SEQ ID No. 2, nt 1000 of SEQ ID No. 3, nt 1001 of SEQ ID No. 4, nt 1000 of SEQ ID No. 5, nt 1000 of SEQ ID No. 6, nt 1000 of SEQ ID No. 7, nt 1001 of SEQ ID No. 8, nt 1001 of SEQ ID No. 9, nt 1001 of SEQ ID No. 10, nt 1000 of SEQ ID No. 11, nt 1000 of SEQ ID No. 12, nt 1001 of SEQ ID No. 13, nt 1000 of SEQ ID No. 14, nt 1001 of SEQ ID No. 15, nt 1000 of SEQ ID No. 16, nt 1000 of SEQ ID No. 17, nt 1001 of SEQ ID No. 18, nt 1000 of SEQ ID No. 19, nt 1000 of SEQ ID No. 20, nt 1000 of SEQ ID No. 21, nt 1000 of SEQ ID No. 22, nt 1000 of SEQ ID No. 23, nt 1000 of SEQ ID No. 24, nt 1000 of SEQ ID No. 25, nt 1001 of SEQ ID No. 26, nt 1001 of SEQ ID No. 27, nt 1001 of SEQ ID No. 28, nt 1000 of SEQ ID No. 29, nt 1000 of SEQ ID No. 30, nt 1000 of SEQ ID No. 31, nt 1000 of SEQ ID No. 32, nt 1000 of SEQ ID No. 33, nt 1000 of SEQ ID No. 34, nt 1000 of SEQ ID No. 35, nt 1000 of SEQ ID No. 36, nt 1000 of SEQ ID No. 37, nt 1000 of SEQ ID No. 38, nt 1000 of SEQ ID No. 39, nt 1001 of SEQ ID No. 40, nt 1000 of SEQ ID No. 41, nt 1000 of SEQ ID No. 42, nt 1000 of SEQ ID No. 43, nt 1000 of SEQ ID No. 44, nt 1001 of SEQ ID No. 45, nt 1000 of SEQ ID No. 46, nt 1001 of SEQ ID No. 47, nt 1000 of SEQ ID No. 48, nt 1001 of SEQ ID No. 49, nt 1000 of SEQ ID No. 50, nt 1000 of SEQ ID No. 51, nt 1000 of SEQ ID No. 52, nt 1001 of SEQ ID No. 53, nt 1001 of SEQ ID No. 54, nt 1000 of SEQ ID No. 55, nt 1000 of SEQ ID No. 56, nt 1000 of SEQ ID No. 57, nt 1000 of SEQ ID No. 58, nt 1000 of SEQ ID No. 59, nt 1001 of SEQ ID No. 60, nt 1001 of SEQ ID No. 61, nt 1000 of SEQ ID No. 62, nt 1000 of SEQ ID No. 63, nt 1001 of SEQ ID No. 64, nt 1000 of SEQ ID No. 65, nt 1000 of SEQ ID No. 66, nt 1001 of SEQ ID No. 67, nt 1001 of SEQ ID No. 68, nt 1000 of SEQ ID No. 69, nt 1000 of SEQ ID No. 70, nt 1001 of SEQ ID No. 71, nt 1000 of SEQ ID No. 72, nt 1001 of SEQ ID No. 73, nt 1001 of SEQ ID No. 74, nt 1000 of SEQ ID No. 75, nt 1000 of SEQ ID No. 76, nt 1000 of SEQ ID No. 77, nt 1000 of SEQ ID No. 78, nt 1001 of SEQ ID No. 79, nt 1001 of SEQ ID No. 80, nt 1000 of SEQ ID No. 81, nt 1000 of SEQ ID No. 82, nt 1000 of SEQ ID No. 83, nt 1000 of SEQ ID No. 84, nt 1001 of SEQ ID No. 85, nt 1001 of SEQ ID No. 86, nt 1001 of SEQ ID No. 87, nt 1001 of SEQ ID No. 88, nt 1001 of SEQ ID No. 89, nt 1000 of SEQ ID No. 90, nt 1000 of SEQ ID No. 91, nt 1000 of SEQ ID No. 92, nt 1001 of SEQ ID No. 93, nt 1000 of SEQ ID No. 94, nt 1001 of SEQ ID No. 95, nt 1001 of SEQ ID No. 96, nt 1001 of SEQ ID No. 97, nt 1001 of SEQ ID No. 98, nt 1001 of SEQ ID No. 99, nt 1000 of SEQ ID No. 100, nt 1000 of SEQ ID No. 101, nt 1001 of SEQ ID No. 102, nt 1001 of SEQ ID No. 103, nt 1000 of SEQ ID No. 104, nt 1001 of SEQ ID No. 105, nt 1000 of SEQ ID No. 106, nt 1000 of SEQ ID No. 107, nt 1000 of SEQ ID No. 108, nt 1000 of SEQ ID No. 109, nt 1000 of SEQ ID No. 110, nt 1000 of SEQ ID No. 111, nt 1000 of SEQ ID No. 112, nt 1001 of SEQ ID No. 113, nt 1001 of SEQ ID No. 114, nt 1001 of SEQ ID No. 115, nt 1000 of SEQ ID No. 116, nt 1001 of SEQ ID No. 117, nt 1001 of SEQ ID No. 118, nt 1000 of SEQ ID No. 119, nt 1001 of SEQ ID No. 120, nt 1000 of SEQ ID No. 121, nt 1001 of SEQ ID No. 122, nt 1001 of SEQ ID No. 123, nt 1000 of SEQ ID No. 124, nt 1000 of SEQ ID No. 125, nt 1000 of SEQ ID No. 126, nt 1001 of SEQ ID No. 127, nt 1001 of SEQ ID No. 128, nt 1001 of SEQ ID No. 129, nt 1001 of SEQ ID No. 130, nt 1001 of SEQ ID No. 131, nt 1000 of SEQ ID No. 132, nt 1000 of SEQ ID No. 133, nt 1001 of SEQ ID No. 134, nt 1000 of SEQ ID No. 135, nt 1001 of SEQ ID No. 136, nt 1001 of SEQ ID No. 137, nt 1000 of SEQ ID No. 138, nt 1001 of SEQ ID No. 139, nt 1000 of SEQ ID No. 140, nt 1000 of SEQ ID No. 141, nt 1001 ofSEQ ID No. 142, nt 1001 of SEQ ID No. 143, nt 1001 of SEQ ID No. 144, nt 1001 of SEQ ID No. 145, nt 1001 of SEQ ID No. 146, nt 1001 of SEQ ID No. 147, nt 1001 of SEQ ID No. 148, nt 1001 of SEQ ID No. 149, nt 1001 of SEQ ID No. 150, nt 1000 of SEQ ID No. 151, nt 1000 of SEQ ID No. 152, nt 1000 of SEQ ID No. 153, nt 1001 of SEQ ID No. 154, nt 1001 of SEQ ID No. 155, nt 1000 of SEQ ID No. 156, nt 1000 of SEQ ID No. 157, nt 1000 of SEQ ID No. 158, nt 1001 of SEQ ID No. 159, nt 1000 of SEQ ID No. 160, nt 1001 of SEQ ID No. 161, nt 1000 of SEQ ID No. 162, nt 1000 of SEQ ID No. 163, nt 1000 of SEQ ID No. 164, nt 1001 of SEQ ID No. 165, nt 1001 of SEQ ID No. 166, nt 1001 of SEQ ID No. 167, nt 1001 of SEQ ID No. 168, nt 1001 of SEQ ID No. 169, nt 1000 of SEQ ID No. 170, nt 1000 of SEQ ID No. 171, nt 1001 of SEQ ID No. 172, nt 1000 of SEQ ID No. 173, nt 1001 of SEQ ID No. 174, nt 1001 of SEQ ID No. 175, nt 1001 of SEQ ID No. 176, nt 1001 of SEQ ID No. 177, nt 1000 of SEQ ID No. 178, nt 1001 of SEQ ID No. 179, nt 1001 of SEQ ID No. 180, nt 1001 of SEQ ID No. 181, nt 1001 of SEQ ID No. 182, nt 1000 of SEQ ID No. 183, nt 1000 of SEQ ID No. 184, nt 1000 of SEQ ID No. 185, nt 1000 of SEQ ID No. 186, nt 1000 of SEQ ID No. 187, nt 1000 of SEQ ID No. 188, nt 1000 of SEQ ID No. 189, nt 1000 of SEQ ID No. 190, nt 1001 of SEQ ID No. 191, nt 1000 of SEQ ID No. 192, nt 1000 of SEQ ID No. 193, nt 1001 of SEQ ID No. 194, nt 1000 of SEQ ID No. 195, nt 1000 of SEQ ID No. 196, nt 1001 of SEQ ID No. 197, nt 1001 of SEQ ID No. 198, nt 1001 of SEQ ID No. 199, nt 1000 of SEQ ID No. 200, nt 1000 of SEQ ID No. 201, nt 1000 of SEQ ID No. 202, nt 1000 of SEQ ID No. 203, nt 1000 of SEQ ID No. 204, nt 1000 of SEQ ID No. 205, nt 1000 of SEQ ID No. 206, nt 1001 of SEQ ID No. 207, nt 1000 of SEQ ID No. 208, nt 1000 of SEQ ID No. 209, nt 1000 of SEQ ID No. 210, nt 1001 of SEQ ID No. 211, nt 1001 of SEQ ID No. 212, nt 1001 of SEQ ID No. 213, nt 1000 of SEQ ID No. 214, nt 1000 of SEQ ID No. 215, nt 1001 of SEQ ID No. 216, nt 1001 of SEQ ID No. 217, nt 1000 of SEQ ID No. 218, nt 1000 of SEQ ID No. 219, nt 252, 256 or 270 of SEQ ID No. 220 or at the corresponding position on the complementary sequence of SEQ ID No. 1 to 220. The position of the at least cytosine residue on the complementary sequence to SEQ ID No. 1 to 220 is deductible from the sequences indicated in table 2.

Preferably, the melanoma is cutaneous melanoma, preferably at stage III or IIIC.

Preferably, the method comprises the steps of:
(a) isolating DNA from a sample obtained from a subject;
(b) subjecting said DNA to methylation-sensitive sequence modification through incubation with chemicals (e.g., sodium bisulfite), or contacting said DNA with a polypeptide capable of binding methylated DNA;
(c) optionally amplifying said DNA modified with sodium bisulfite or bound by said polypeptide; and
(d) determining the methylation status of said DNA.

It is a further object of the invention a kit for performing the method as described above, comprising a chemical able to differentially modify DNA sequence depending on methylation status, or a polypeptide capable of binding methylated DNA, and probes, primers and/or aptamers specific for at least one gene as defined above or a variant thereof.

Preferably the kit is a kit for prognosing melanoma and/or of predicting and/or monitoring the response and/or the efficacy of a melanoma therapy or to identify a subject to be treated with a DNA hypomethylation agent.

It is a further object of the invention a microarray able to determine the methylation status of at least one cytosine residue in a methylation relevant region of at least one gene as defined above.

The kit may be in the form of a microarray or microchip.

In the present invention, the "Methylation status" refers to the presence, absence and/or quantity of methylation at a particular nucleotide, or nucleotides within a portion of DNA. The methylation status of a particular DNA sequence (e.g., a methylation relevant region as described herein) can indicate the methylation state of every base in the sequence or can indicate the methylation state of a subset of the base pairs (e.g., of cytosines or the methylation state of one or more specific restriction enzyme recognition sequences) within the sequence, or can indicate information regarding regional methylation density within the sequence without providing precise information of where in the sequence the methylation occurs. The methylation status can optionally be represented or indicated by a "methylation value." A methylation value can be generated, for example, by quantifying the amount of methylated DNA by quantitative MSP. In this particular example, distinct primer pairs can be utilized to selectively amplify methylated or unmethylated target sequence. The amount of methylated and unmethylated DNA could be measured, for instance, by extrapolation of the values (e.g., the n. of PCR cycles at which the quantity of amplification product reaches a particular threshold level) obtained for the test sample against standard curves generated with different known amounts of methylated and unmethylated target sequences. Accordingly, a value, i.e., a methylation value, for example from the above described example, represents the methylation status and can thus be used as a quantitative indicator of methylation status. This is of particular use when it is desirable to compare the methylation status of a sequence in a sample to a threshold value.

The "methylation relevant region" is defined as a region containing CpG dinucleotides potential targets for DNA methylation which may extend from over 5000 bases 5' to over 5000 bases 3' respective to the putative transcription start site of the gene, and may include gene promoter(s), enhancer(s), exonic and intronic sequences.

As used in the present invention, the term "biologic sample" refers to any sample taken from a subject or derived from it, including, without limitation, any source of neoplastic cells from CM patients, such as primary cultures of autologous neoplastic cells, neoplastic cells purified from fresh autologous tumor tissues or from cryopreserved cell suspensions by immunosorting (e.g. using antibodies specific for cancer cells) or other means, cryopreserved tumor tissues, FFPE tumor tissues, or other types of fixed tumor tissue, micro-/macro-dissected tumor tissues, circulating neoplastic cells present in body fluids. The term further applies to biological fluids, including, without limitation, any fluid present in the subject in which acellular DNA or cancer cells may be present, such as blood, serum, plasma, bone marrow, cerebrospinal fluid, peritoneal/pleural fluid, lymph fluid, ascitic, serous fluid, sputum, lacrimal fluid, stool, and urine. Biological samples can be obtained from a subject using any of the methods known in the art.

**Table 1: Signatures A, B, C, D, E, F and G associated with their SEQ ID No. to be analyzed for methylation status. * GeneBank accession number, † Illumina unique gene identifyer, § Illumina unique probe identifier that defines the exact CpG dinucleotide that is evaluated.**

| **Signature A** | | | | |
|---|---|---|---|---|
| **Seq ID no.** | **Gene Symbol** | **Accession N. *** | **Gene ID**^{†} | **lImnID§** |
| 1 | MGC29671 | NM_182538.3 | 201305 | cg01138020 |
| 2 | GJB5 | NM_005268.2 | 2709 | cg01333788 |
| 3 | KCNK16 | NM_032115.2 | 83795 | cg01669948 |
| 4 | IGLL1 | NM_020070.2 | 3543 | cg02415431 |
| 5 | ANXA8 | NM_001630.1 | 244 | cg03399459 |
| 6 | OR12D3 | NM_030959.2 | 81797 | cg03608577 |
| 7 | TUB | NM_177972.1 | 7275 | cg05492113 |
| 8 | CST11 | NM_080830.2 | 140880 | cg06489008 |
| 9 | TMEM10 | NM_033207.2 | 93377 | cg06825166 |
| 10 | CRHR1 | NM_004382.2 | 1394 | cg08929103 |
| 11 | TRIM40 | NM_138700.2 | 135644 | cg09196959 |
| 12 | GAS2L2 | NM_139285.1 | 246176 | cg10016608 |
| 13 | FOXN1 | NM_003593.2 | 8456 | cg10213812 |
| 14 | FXYD4 | NM_173160.2 | 53828 | cg12619162 |
| 15 | KRTHB2 | NM_033033.3 | 3888 | cg13696887 |
| 16 | FU33860 | NM_173644.1 | 284756 | cg14236389 |
| 17 | S100A9 | NM_002965.2 | 6280 | cg16139316 |
| 18 | FOXI1 | NM_012188.3 | 2299 | cg19233472 |
| 19 | GUCA2B | NM_007102.1 | 2981 | cg19728577 |
| 20 | CYP2B6 | NM_000767.4 | 1555 | cg19756068 |
| 21 | IL8RA | NM_000634.2 | 3577 | cg21004129 |
| 22 | CYP8B1 | NM_004391.1 | 1582 | cg21979032 |
| 23 | WFDC8 | NM_181510.1 | 90199 | cg22021786 |
| 24 | SLPI | NM_003064.2 | 6590 | cg23889010 |

| **Signature B** | | | | |
|---|---|---|---|---|
| **Seq ID no.** | **Gene Symbol** | **Accession N. *** | **Gene ID**^{†} | **lImnID§** |
| 25 | FU25421 | NM_152512.3 | 150350 | cg00044729 |
| 26 | LAMC2 | NM_005562.1 | 3918 | cg00208830 |
| 27 | C1orf36 | NM_183059.1 | 343035 | cg00658007 |
| 1 | MGC29671 | NM_182538.3 | 201305 | cg01138020 |
| 2 | GJB5 | NM_005268.2 | 2709 | cg01333788 |
| 3 | KCNK16 | NM_032115.2 | 83795 | cg01669948 |
| 28 | ANGPTL3 | NM_014495.2 | 27329 | cg02218214 |
| 4 | IGLL1 | NM_020070.2 | 3543 | cg02415431 |
| 29 | DEFB4 | NM_004942.2 | 1673 | cg02658251 |
| 30 | STK38 | NM_007271.2 | 11329 | cg02964385 |
| 31 | SEMG2 | NM_003008.2 | 6407 | cg03312792 |
| 5 | ANXA8 | NM_001630.1 | 244 | cg03399459 |
| 6 | OR12D3 | NM_030959.2 | 81797 | cg03608577 |
| 32 | PREPL | NM_006036.2 | 9581 | cg03894103 |
| 33 | IL17F | NM_052872.3 | 112744 | cg04063348 |
| 34 | DCLRE1A | NM_014881.2 | 9937 | cg04281204 |
| 35 | FU20581 | NM_017888.2 | 54988 | cg04428453 |
| 36 | PTCD2 | NM_024754.2 | 79810 | cg04527989 |
| 37 | CX3CR1 | NM_001337.3 | 1524 | cg04569233 |
| 7 | TUB | NM_177972.1 | 7275 | cg05492113 |
| 38 | UBE2E1 | NM_182666.1 | 7324 | cg06282059 |
| 8 | CST11 | NM_080830.2 | 140880 | cg06489008 |
| 39 | IFT140 | NM_014714.2 | 9742 | cg06730286 |
| 9 | TMEM10 | NM_033207.2 | 93377 | cg06825166 |
| 40 | SLC22A8 | NM_004254.2 | 9376 | cg06917325 |
| 41 | MLN | NM_002418.1 | 4295 | cg08332212 |
| 42 | DIRC1 | NM_052952.1 | 116093 | cg08654334 |
| 43 | C1orf64 | NM_178840.2 | 149563 | cg08887581 |
| 10 | CRHR1 | NM_004382.2 | 1394 | cg08929103 |
| 11 | TRIM40 | NM_138700.2 | 135644 | cg09196959 |
| 44 | TNFSF13B | NM_006573.3 | 10673 | cg09646392 |
| 12 | GAS2L2 | NM_139285.1 | 246176 | cg10016608 |
| 13 | FOXN1 | NM_003593.2 | 8456 | cg10213812 |
| 45 | CYP2B6 | NM_000767.4 | 1555 | cg10322876 |
| 46 | ACSL5 | NM_016234.3 | 51703 | cg11152574 |
| 47 | SERPINB12 | NM_080474.1 | 89777 | cg11435943 |
| 48 | APOBEC4 | NM_203454.1 | 403314 | cg11505048 |
| 49 | TNN11 | NM_003281.3 | 7135 | cg12114524 |
| 50 | C1orf42 | NM_019060.1 | 54544 | cg12588301 |
| 14 | FXYD4 | NM_173160.2 | 53828 | cg12619162 |
| 51 | PPAT | NM_002703.3 | 5471 | cg13086586 |
| 52 | PSD4 | NM_012455.2 | 23550 | cg13273136 |
| 53 | SCN4A | NM_000334.3 | 6329 | cg13694749 |
| 15 | KRTHB2 | NM_033033.3 | 3888 | cg13696887 |
| 54 | IL8RB | NM_001557.2 | 3579 | cg13739417 |
| 55 | UNC45B | NM_173167.2 | 146862 | cg13859324 |
| 56 | C1orf201 | NM_178122.2 | 90529 | cg13980834 |
| 57 | SFTPG | NM_205854.1 | 389376 | cg14034870 |
| 58 | LOC124751 | NM_213597.1 | 124751 | cg14069619 |
| 16 | FU33860 | NM_173644.1 | 284756 | cg14236389 |
| 59 | ANKRD1 | NM_014391.2 | 27063 | cg14558138 |
| 60 | FMO2 | NM_001460.2 | 2327 | cg14721213 |
| 61 | ARSG | NM_014960.2 | 22901 | cg15308737 |
| 62 | GJB4 | NM_153212.1 | 127534 | cg15554401 |
| 17 | S100A9 | NM_002965.2 | 6280 | cg16139316 |
| 63 | LRRN5 | NM_006338.2 | 10446 | cg16456919 |
| 64 | MSMB | NM_002443.2 | 4477 | cg17030820 |
| 65 | HLA-DOB | NM_002120.2 | 3112 | cg17103109 |
| 66 | R3HDML | NM_178491.2 | 140902 | cg17692403 |
| 67 | SPINK5 | NM_006846.2 | 11005 | cg17788013 |
| 68 | DLG5 | NM_004747.2 | 9231 | cg17791617 |
| 69 | PALLD | NM_016081.2 | 23022 | cg17925436 |
| 70 | WFDC12 | NM_080869.1 | 128488 | cg18241647 |
| 71 | WDR20 | NM_181291.1 | 91833 | cg18412984 |
| 72 | FU32771 | NM_145017.1 | 220004 | cg18432384 |
| 73 | PKHD1 | NM_138694.2 | 5314 | cg18885346 |
| 18 | FOXI1 | NM_012188.3 | 2299 | cg19233472 |
| 74 | DEFB123 | NM_153324.2 | 245936 | cg19241311 |
| 75 | KRTHB3 | NM_002282.2 | 3889 | cg19258973 |
| 19 | GUCA2B | NM_007102.1 | 2981 | cg19728577 |
| 20 | CYP2B6 | NM_000767.4 | 1555 | cg19756068 |
| 76 | BSND | NM_057176.2 | 7809 | cg19971655 |
| 77 | NALP10 | NM_176821.2 | 338322 | cg20311730 |
| 78 | NR5A2 | NM_003822.3 | 2494 | cg20406878 |
| 79 | KRT25D | NM_181535.2 | 162605 | cg20484002 |
| 21 | IL8RA | NM_000634.2 | 3577 | cg21004129 |
| 80 | APOD | NM_001647.2 | 347 | cg21084260 |
| 81 | CLEC4F | NM_173535.2 | 165530 | cg21148892 |
| 82 | THRB | NM_000461.2 | 7068 | cg21303011 |
| 83 | KRTHB5 | NM_002283.2 | 3891 | cg21518208 |
| 22 | CYP8B1 | NM_004391.1 | 1582 | cg21979032 |
| 23 | WFDC8 | NM_181510.1 | 90199 | cg22021786 |
| 84 | SLC13A1 | NM_022444.3 | 6561 | cg22024657 |
| 85 | C3orf27 | NM_007354.1 | 23434 | cg22068211 |
| 86 | MGC35206 | NM_178552.2 | 339669 | cg22088368 |
| 87 | C3orf63 | NM_015224.1 | 23272 | cg22332306 |
| 88 | DARC | NM_002036.2 | 2532 | cg23507131 |
| 24 | SLPI | NM_003064.2 | 6590 | cg23889010 |
| 89 | SLC2A7 | NM_207420.1 | 155184 | cg24027679 |
| 90 | CPA3 | NM_001870.1 | 1359 | cg24290574 |
| 91 | MIS12 | NM_024039.1 | 79003 | cg24445405 |
| 92 | KRTHB4 | NM_033045.2 | 3890 | cg24461814 |
| 93 | MRVI1 | NM_130385.1 | 10335 | cg24541550 |
| 94 | MIA2 | NM_054024.3 | 117153 | cg24603941 |
| 95 | CYP2A7 | NM_030589.2 | 1549 | cg25427638 |
| 96 | ANGPT4 | NM_015985.2 | 51378 | cg26540515 |
| 97 | SLC44A5 | NM_152697.2 | 204962 | cg27336379 |
| 98 | CST9L | NM_080610.1 | 128821 | cg27655855 |

| **Signature C** | | | | |
|---|---|---|---|---|
| **Seq ID no.** | **Gene Symbol** | **Accession N. *** | **Gene ID**^{†} | **lImnID§** |
| 99 | SLC18A2 | NM_003054.2 | 6571 | cg00498305 |
| 2 | GJB5 | NM_005268.2 | 2709 | cg01333788 |
| 100 | SLC6A18 | NM_182632.1 | 348932 | cg02064402 |
| 4 | IGLL1 | NM_020070.2 | 3543 | cg02415431 |
| 101 | ALOX12B | NM_001139.1 | 242 | cg03742272 |
| 102 | WNT10B | NM_003394.2 | 7480 | cg05164634 |
| 7 | TUB | NM_177972.1 | 7275 | cg05492113 |
| 103 | S100A9 | NM_002965.2 | 6280 | cg07039113 |
| 10 | CRHR1 | NM_004382.2 | 1394 | cg08929103 |
| 11 | TRIM40 | NM_138700.2 | 135644 | cg09196959 |
| 104 | SLC6A11 | NM_014229.1 | 6538 | cg09395732 |
| 16 | FU33860 | NM_173644.1 | 284756 | cg14236389 |
| 105 | FGF4 | NM_002007.1 | 2249 | cg14578030 |
| 17 | S100A9 | NM_002965.2 | 6280 | cg16139316 |
| 86 | MGC35206 | NM_178552.2 | 339669 | cg22088368 |
| 106 | PCDHAC2 | NM_031883.2 | 56134 | cg24076884 |
| 107 | GRM4 | NM_000841.1 | 2914 | cg26424956 |

| **Signature D** | | | | |
|---|---|---|---|---|
| **Seq ID no.** | **Gene Symbol** | **Accession N. *** | **Gene ID^{†}** | **lImnID§** |
| 25 | FU25421 | NM_152512.3 | 150350 | cg00044729 |
| 108 | ANXA13 | NM_004306.2 | 312 | cg00283535 |
| 99 | SLC18A2 | NM_003054.2 | 6571 | cg00498305 |
| 109 | CTAG2 | NM_020994.2 | 30848 | cg01325515 |
| 2 | GJB5 | NM_005268.2 | 2709 | cg01333788 |
| 3 | KCNK16 | NM_032115.2 | 83795 | cg01669948 |
| 100 | SLC6A18 | NM_182632.1 | 348932 | cg02064402 |
| 30 | STK38 | NM_007271.2 | 11329 | cg02964385 |
| 110 | PVALB | NM_002854.2 | 5816 | cg02978737 |
| 6 | OR12D3 | NM_030959.2 | 81797 | cg03608577 |
| 101 | ALOX12B | NM_001139.1 | 242 | cg03742272 |
| 111 | HA02 | NM_016527.2 | 51179 | cg03762535 |
| 34 | DCLRE1A | NM_014881.2 | 9937 | cg04281204 |
| 102 | WNT10B | NM_003394.2 | 7480 | cg05164634 |
| 7 | TUB | NM_177972.1 | 7275 | cg05492113 |
| 38 | UBE2E1 | NM_182666.1 | 7324 | cg06282059 |
| 39 | IFT140 | NM_014714.2 | 9742 | cg06730286 |
| 112 | SLC4A7 | NM_003615.2 | 9497 | cg06798189 |
| 103 | S100A9 | NM_002965.2 | 6280 | cg07039113 |
| 42 | DIRC1 | NM_052952.1 | 116093 | cg08654334 |
| 10 | CRHR1 | NM_004382.2 | 1394 | cg08929103 |
| 11 | TRIM40 | NM_138700.2 | 135644 | cg09196959 |
| 104 | SLC6A11 | NM_014229.1 | 6538 | cg09395732 |
| 113 | TDRD5 | NM_173533.2 | 163589 | cg09656934 |
| 46 | ACSL5 | NM_016234.3 | 51703 | cg11152574 |
| 48 | APOBEC4 | NM_203454.1 | 403314 | cg11505048 |
| 114 | FAAH | NM_001441.1 | 2166 | cg12671744 |
| 115 | CD1D | NM_001766.2 | 912 | cg13765621 |
| 56 | C1orf201 | NM_178122.2 | 90529 | cg13980834 |
| 16 | FU33860 | NM_173644.1 | 284756 | cg14236389 |
| 59 | ANKRD1 | NM_014391.2 | 27063 | cg14558138 |
| 105 | FGF4 | NM_002007.1 | 2249 | cg14578030 |
| 116 | FU39501 | NM_173483.1 | 126410 | cg14851685 |
| 17 | S100A9 | NM_002965.2 | 6280 | cg16139316 |
| 63 | LRRN5 | NM_006338.2 | 10446 | cg16456919 |
| 117 | GLT1D1 | NM_144669.1 | 144423 | cg16717225 |
| 118 | IL28B | NM_172139.2 | 282617 | cg17982671 |
| 119 | AVPR1B | NM_000707.2 | 553 | cg18992688 |
| 120 | GJB3 | NM_024009.2 | 2707 | cg21151355 |
| 86 | MGC35206 | NM_178552.2 | 339669 | cg22088368 |
| 106 | PCDHAC2 | NM_031883.2 | 56134 | cg24076884 |
| 121 | GABRG3 | NM_033223.1 | 2567 | cg24244000 |
| 94 | MIA2 | NM_054024.3 | 117153 | cg24603941 |
| 122 | CTAG1B | NM_001327.1 | 1485 | cg25531166 |
| 123 | UNQ9433 | NM_207413.1 | 389658 | cg26021627 |
| 107 | GRM4 | NM_000841.1 | 2914 | cg26424956 |
| 124 | CEACAM6 | NM_002483.3 | 4680 | cg26813458 |

| **Signature E** | | | | |
|---|---|---|---|---|
| **Seq ID no.** | **Gene Symbol** | **Accession N. *** | **Gene ID**^{†} | **lImnID§** |
| 125 | DENND2D | NM_024901.3 | 79961 | cg00619207 |
| 109 | CTAG2 | NM_020994.2 | 30848 | cg01325515 |
| 126 | PGCP | NM_016134.2 | 10404 | cg01892689 |
| 127 | KCNG1 | NM_002237.2 | 3755 | cg02305261 |
| 128 | FAM107B | NM_031453.2 | 83641 | cg02876062 |
| 129 | FANCE | NM_021922.2 | 2178 | cg03030757 |
| 130 | FAM38B | NM_022068.1 | 63895 | cg03426225 |
| 131 | FOXF2 | NM_001452.1 | 2295 | cg03848675 |
| 132 | BGN | NM_001711.3 | 633 | cg04929865 |
| 133 | MAGEA10 | NM_021048.3 | 4109 | cg04950711 |
| 134 | SPATS1 | NM_145026.2 | 221409 | cg05010967 |
| 135 | PRRX1 | NM_022716.2 | 5396 | cg05593300 |
| 136 | MLSTD1 | NM_018099.3 | 55711 | cg05697976 |
| 137 | PDZD4 | NM_032512.2 | 57595 | cg06051662 |
| 138 | HCP5 | NM_006674.2 | 10866 | cg07971188 |
| 139 | OCIAD2 | NM_152398.2 | 132299 | cg08942875 |
| 140 | CSAG1 | NM_153478.1 | 158511 | cg08977028 |
| 141 | SLCO4A1 | NM_016354.3 | 28231 | cg09210315 |
| 142 | C9orf90 | NM_197956.1 | 203245 | cg09296212 |
| 143 | CSAG1 | NM_153478.1 | 158511 | cg09917461 |
| 144 | AMPH | NM_001635.2 | 273 | cg09966445 |
| 145 | MT1G | NM_005950.1 | 4495 | cg09985279 |
| 146 | C20orf100 | NM_032883.1 | 84969 | cg11319389 |
| 147 | CST7 | NM_003650.2 | 8530 | cg11804789 |
| 148 | KIAA0367 | NM_015225.1 | 23273 | cg11880010 |
| 149 | RNASE1 | NM_198232.1 | 6035 | cg13718960 |
| 150 | HCP5 | NM_006674.2 | 10866 | cg13784557 |
| 151 | NDN | NM_002487.2 | 4692 | cg13828758 |
| 152 | RAC2 | NM_002872.3 | 5880 | cg14072120 |
| 153 | AXL | NM_021913.2 | 558 | cg14892768 |
| 154 | BATF | NM_006399.2 | 10538 | cg15645309 |
| 155 | EMILIN1 | NM_007046.1 | 11117 | cg15895197 |
| 156 | ARHGEF7 | NM_003899.2 | 8874 | cg16427670 |
| 157 | EVL | NM_016337.2 | 51466 | cg17813891 |
| 158 | RAC2 | NM_002872.3 | 5880 | cg18265887 |
| 159 | KIAA0367 | NM_015225.1 | 23273 | cg19282250 |
| 160 | TFF3 | NM_003226.2 | 7033 | cg20488657 |
| 161 | CTSK | NM_000396.2 | 1513 | cg20802392 |
| 162 | C21orf70 | NM_058190.2 | 85395 | cg20884362 |
| 163 | PRKCZ | NM_002744.4 | 5590 | cg21137823 |
| 164 | MLSTD1 | NM_018099.3 | 55711 | cg21522988 |
| 165 | COL5A2 | NM_000393.2 | 1290 | cg22774472 |
| 166 | LY96 | NM_015364.2 | 23643 | cg23732024 |
| 167 | MAGEA1 | NM_004988.3 | 4100 | cg23776892 |
| 168 | MAGEC2 | NM_016249.2 | 51438 | cg23813564 |
| 169 | DENND2D | NM_024901.3 | 79961 | cg24641737 |
| 170 | PPIE | NM_006112.2 | 10450 | cg24920358 |
| 171 | SORBS2 | NM_021069.3 | 8470 | cg26583078 |
| 172 | PROCR | NM_006404.3 | 10544 | cg26806924 |
| 173 | TMEPAI | NM_020182.3 | 56937 | cg26912636 |
| 174 | SSNA1 | NM_003731.1 | 8636 | cg27291231 |
| 175 | ZNF324 | NM_014347.1 | 25799 | cg27401095 |

| **Signature F** | | | | |
|---|---|---|---|---|
| **Seq ID no.** | **Gene Symbol** | **Accession N. *** | **Gene ID**^{†} | **lImnID§** |
| 25 | FU25421 | NM_152512.3 | 150350 | cg00044729 |
| 125 | DENND2D | NM_024901.3 | 79961 | cg00619207 |
| 109 | CTAG2 | NM_020994.2 | 30848 | cg01325515 |
| 126 | PGCP | NM_016134.2 | 10404 | cg01892689 |
| 127 | KCNG1 | NM_002237.2 | 3755 | cg02305261 |
| 175 | MGMT | NT_008818.15 | 4255 | cg02330106 |
| 128 | FAM107B | NM_031453.2 | 83641 | cg02876062 |
| 129 | FANCE | NM_021922.2 | 2178 | cg03030757 |
| 176 | GAGE2 | NM_001472.1 | 2574 | cg04431776 |
| 177 | C11orf1 | NM_022761.1 | 64776 | cg04545708 |
| 178 | ABCB4 | NM_018849.1 | 5244 | cg04737046 |
| 132 | BGN | NM_001711.3 | 633 | cg04929865 |
| 133 | MAGEA10 | NM_021048.3 | 4109 | cg04950711 |
| 134 | SPATS1 | NM_145026.2 | 221409 | cg05010967 |
| 179 | CTNNAL1 | NM_003798.1 | 8727 | cg05485060 |
| 135 | PRRX1 | NM_022716.2 | 5396 | cg05593300 |
| 180 | EMP1 | NM_001423.1 | 2012 | cg05885720 |
| 181 | RNASE1 | NM_198232.1 | 6035 | cg05958352 |
| 137 | PDZD4 | NM_032512.2 | 57595 | cg06051662 |
| 182 | MAGEA6 | NM_005363.2 | 4105 | cg06899808 |
| 183 | CHMP4A | NM_014169.2 | 29082 | cg07296772 |
| 184 | ADORA2B | NM_000676.2 | 136 | cg07677850 |
| 138 | HCP5 | NM_006674.2 | 10866 | cg07971188 |
| 185 | PLA1A | NM_015900.1 | 51365 | cg08141463 |
| 186 | KIAA0141 | NM_014773.2 | 9812 | cg08158289 |
| 187 | OLFM1 | NM_014279.2 | 10439 | cg08268099 |
| 188 | ERO1L | NM_014584.1 | 30001 | cg08554114 |
| 140 | CSAG1 | NM_153478.1 | 158511 | cg08977028 |
| 141 | SLCO4A1 | NM_016354.3 | 28231 | cg09210315 |
| 189 | CXorf12 | NM_003492.1 | 8269 | cg09275137 |
| 142 | C9orf90 | NM_197956.1 | 203245 | cg09296212 |
| 143 | CSAG1 | NM_153478.1 | 158511 | cg09917461 |
| 144 | AMPH | NM_001635.2 | 273 | cg09966445 |
| 145 | MT1G | NM_005950.1 | 4495 | cg09985279 |
| 190 | ARHGDIB | NM_001175.4 | 397 | cg10925082 |
| 146 | C20orf100 | NM_032883.1 | 84969 | cg11319389 |
| 147 | CST7 | NM_003650.2 | 8530 | cg11804789 |
| 148 | KIAA0367 | NM_015225.1 | 23273 | cg11880010 |
| 149 | RNASE1 | NM_198232.1 | 6035 | cg13718960 |
| 150 | HCP5 | NM_006674.2 | 10866 | cg13784557 |
| 151 | NDN | NM_002487.2 | 4692 | cg13828758 |
| 152 | RAC2 | NM_002872.3 | 5880 | cg14072120 |
| 153 | AXL | NM_021913.2 | 558 | cg14892768 |
| 191 | GRAMD3 | NM_023927.1 | 65983 | cg15165084 |
| 61 | ARSG | NM_014960.2 | 22901 | cg15308737 |
| 192 | PLD1 | NM_002662.2 | 5337 | cg15329866 |
| 193 | HNMT | NM_006895.2 | 3176 | cg15441973 |
| 194 | ZNF558 | NM_144693.1 | 148156 | cg15751406 |
| 195 | ATP6V1E2 | NM_080653.3 | 90423 | cg15759873 |
| 155 | EMILIN1 | NM_007046.1 | 11117 | cg15895197 |
| 156 | ARHGEF7 | NM_003899.2 | 8874 | cg16427670 |
| 196 | KRT25C | NM_181537.2 | 342574 | cg16488522 |
| 197 | ZNF532 | NM_018181.4 | 55205 | cg17675150 |
| 157 | EVL | NM_016337.2 | 51466 | cg17813891 |
| 158 | RAC2 | NM_002872.3 | 5880 | cg18265887 |
| 198 | SLAMF9 | NM_033438.1 | 89886 | cg18913171 |
| 199 | NBL1 | NM_005380.3 | 4681 | cg19136075 |
| 159 | KIAA0367 | NM_015225.1 | 23273 | cg19282250 |
| 200 | MAGEA10 | NM_021048.3 | 4109 | cg19964192 |
| 160 | TFF3 | NM_003226.2 | 7033 | cg20488657 |
| 162 | C21orf70 | NM_058190.2 | 85395 | cg20884362 |
| 80 | APOD | NM_001647.2 | 347 | cg21084260 |
| 201 | ABCA8 | NM_007168.2 | 10351 | cg21660392 |
| 202 | ACOX2 | NM_003500.2 | 8309 | cg22012981 |
| 165 | COL5A2 | NM_000393.2 | 1290 | cg22774472 |
| 166 | LY96 | NM_015364.2 | 23643 | cg23732024 |
| 167 | MAGEA1 | NM_004988.3 | 4100 | cg23776892 |
| 168 | MAGEC2 | NM_016249.2 | 51438 | cg23813564 |
| 203 | TIMP3 | NM_000362.4 | 7078 | cg24080529 |
| 170 | PPIE | NM_006112.2 | 10450 | cg24920358 |
| 204 | RPUSD3 | NM_173659.2 | 285367 | cg26417554 |
| 172 | PROCR | NM_006404.3 | 10544 | cg26806924 |
| 173 | TMEPAI | NM_020182.3 | 56937 | cg26912636 |

| **Signature G** | | | | |
|---|---|---|---|---|
| **Seq ID no.** | **Gene Symbol** | **Accession N. *** | **Gene ID**^{†} | **lImnID§** |
| 205 | PRDX2 | NM_005809.4 | 7001 | cg00155609 |
| 125 | DENND2D | NM_024901.3 | 79961 | cg00619207 |
| 109 | CTAG2 | NM_020994.2 | 30848 | cg01325515 |
| 206 | PCOLCE | NM_002593.2 | 5118 | cg02797569 |
| 132 | BGN | NM_001711.3 | 633 | cg04929865 |
| 207 | OCIAD2 | NM_152398.2 | 132299 | cg04961553 |
| 181 | RNASE1 | NM_198232.1 | 6035 | cg05958352 |
| 182 | MAGEA6 | NM_005363.2 | 4105 | cg06899808 |
| 138 | HCP5 | NM_006674.2 | 10866 | cg07971188 |
| 139 | OCIAD2 | NM_152398.2 | 132299 | cg08942875 |
| 140 | CSAG1 | NM_153478.1 | 158511 | cg08977028 |
| 142 | C9orf90 | NM_197956.1 | 203245 | cg09296212 |
| 143 | CSAG1 | NM_153478.1 | 158511 | cg09917461 |
| 208 | CTSK | NM_000396.2 | 1513 | cg11946165 |
| 209 | ADM | NM_001124.1 | 133 | cg12320306 |
| 149 | RNASE1 | NM_198232.1 | 6035 | cg13718960 |
| 150 | HCP5 | NM_006674.2 | 10866 | cg13784557 |
| 210 | PAGE5 | NM_130467.3 | 90737 | cg13991029 |
| 211 | NNMT | NM_006169.2 | 4837 | cg14209518 |
| 155 | EMILIN1 | NM_007046.1 | 11117 | cg15895197 |
| 212 | MAGEA3 | NM_005362.3 | 4102 | cg16390856 |
| 213 | RAB11FIP5 | NM_015470.1 | 26056 | cg18943195 |
| 214 | ARHGDIB | NM_001175.4 | 397 | cg19826026 |
| 215 | EMILIN1 | NM_007046.1 | 11117 | cg20516209 |
| 216 | LPXN | NM_004811.1 | 9404 | cg20654468 |
| 161 | CTSK | NM_000396.2 | 1513 | cg20802392 |
| 217 | ALDH1A3 | NM_000693.1 | 220 | cg21359747 |
| 218 | SUHW1 | NM_080740.3 | 129025 | cg21604856 |
| 201 | ABCA8 | NM_007168.2 | 10351 | cg21660392 |
| 165 | COL5A2 | NM_000393.2 | 1290 | cg22774472 |
| 166 | LY96 | NM_015364.2 | 23643 | cg23732024 |
| 167 | MAGEA1 | NM_004988.3 | 4100 | cg23776892 |
| 168 | MAGEC2 | NM_016249.2 | 51438 | cg23813564 |
| 219 | GSTT1 | NM_000853.1 | 2952 | cg24330042 |
| 169 | DENND2D | NM_024901.3 | 79961 | cg24641737 |

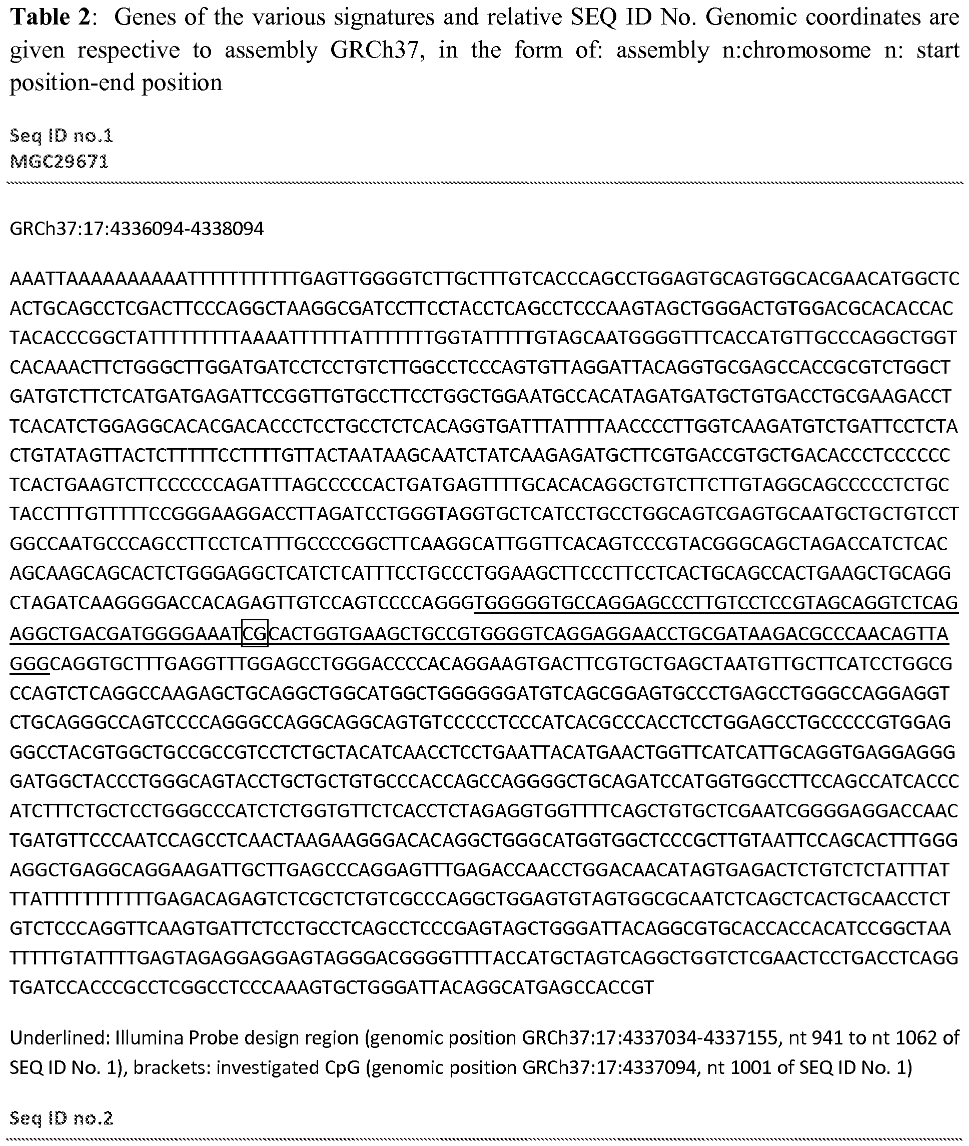

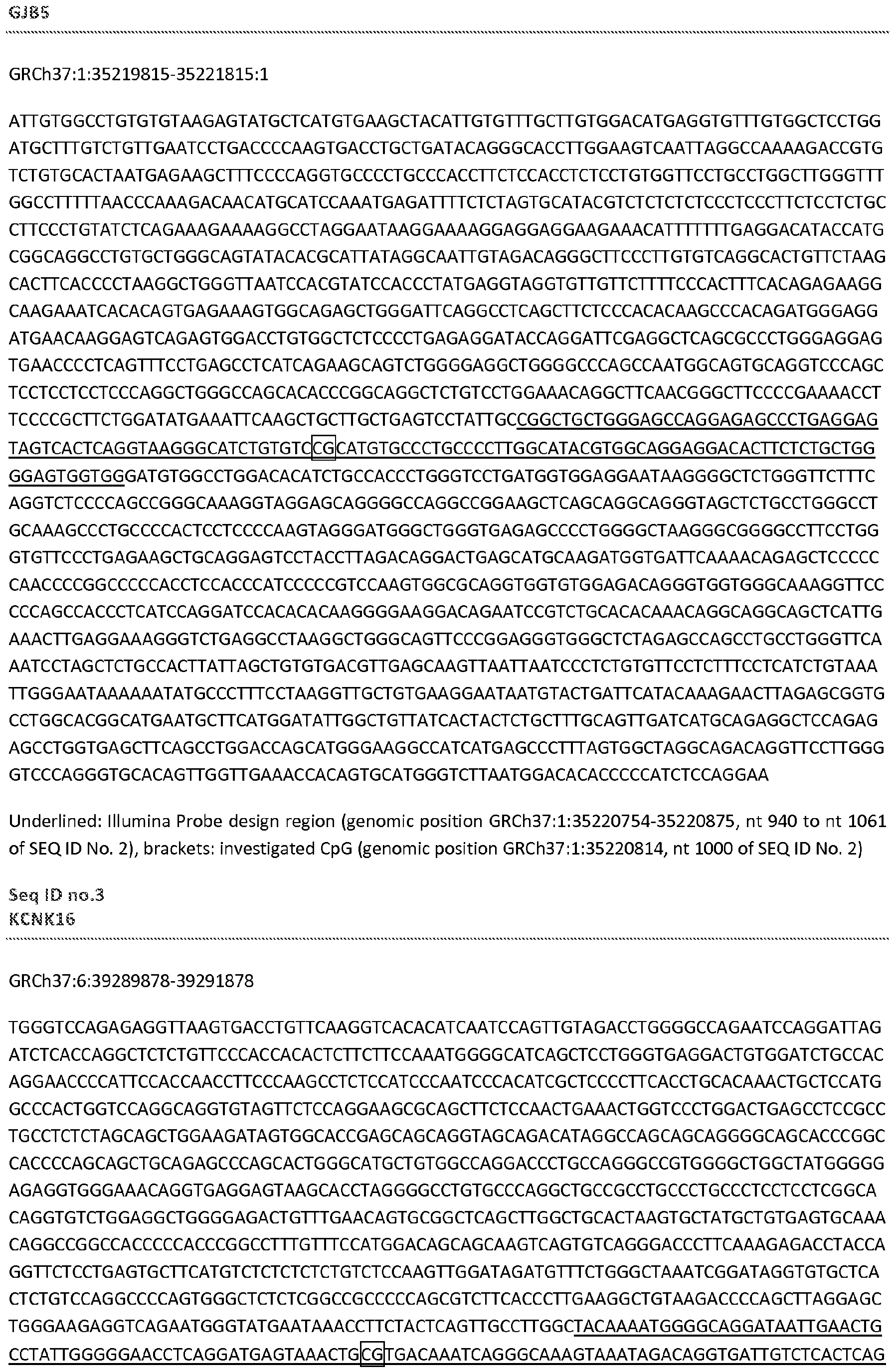

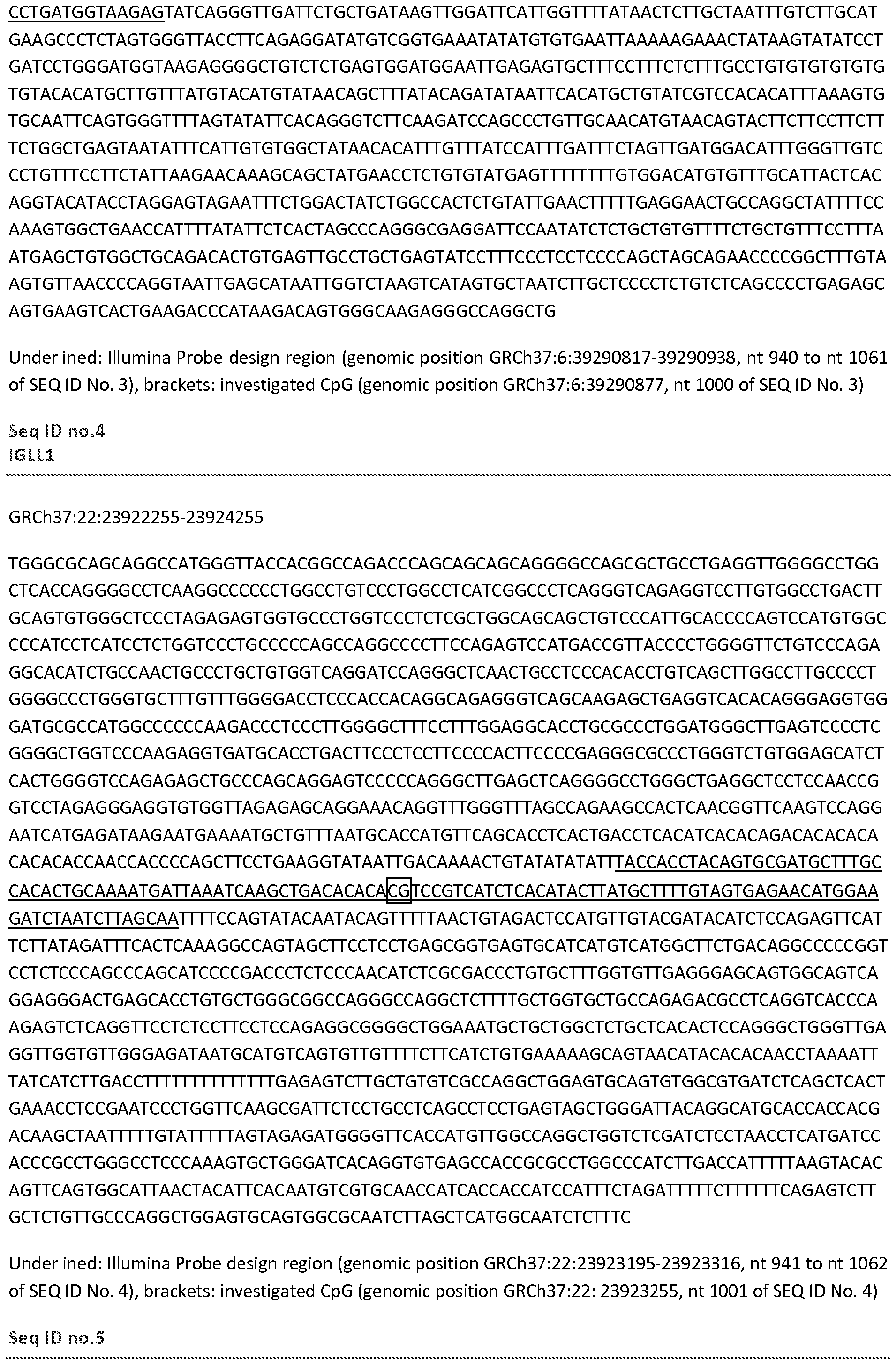

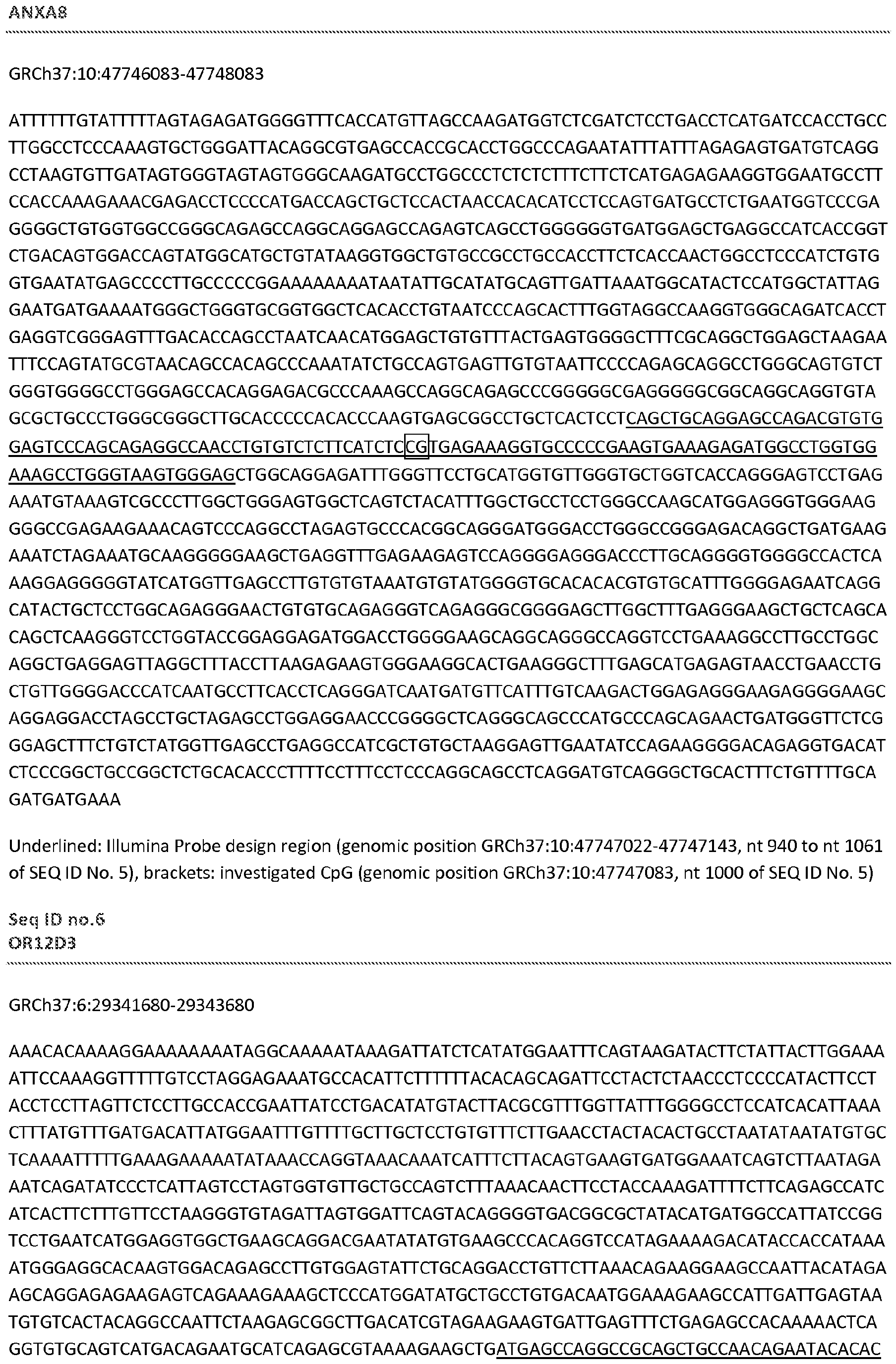

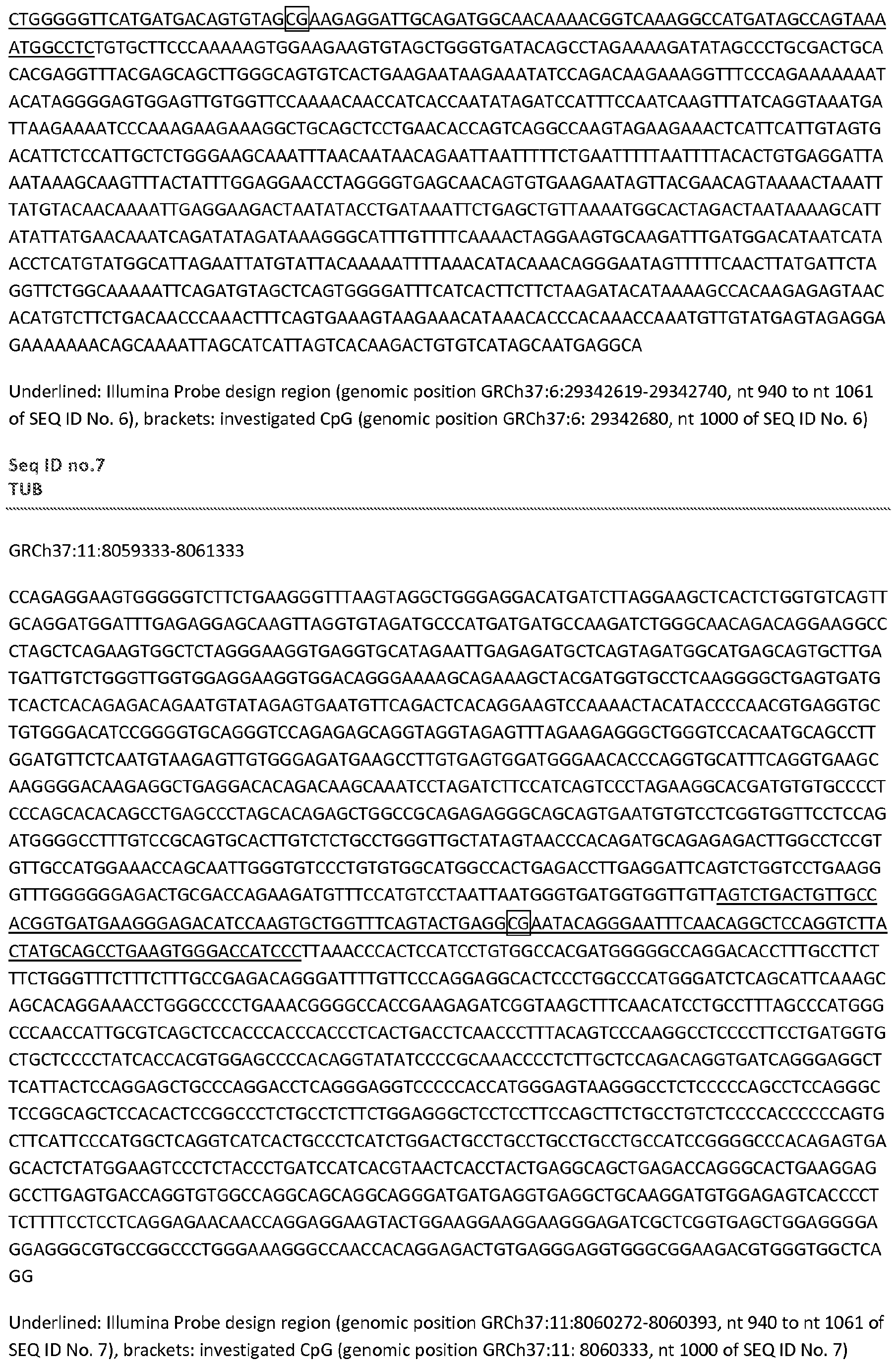

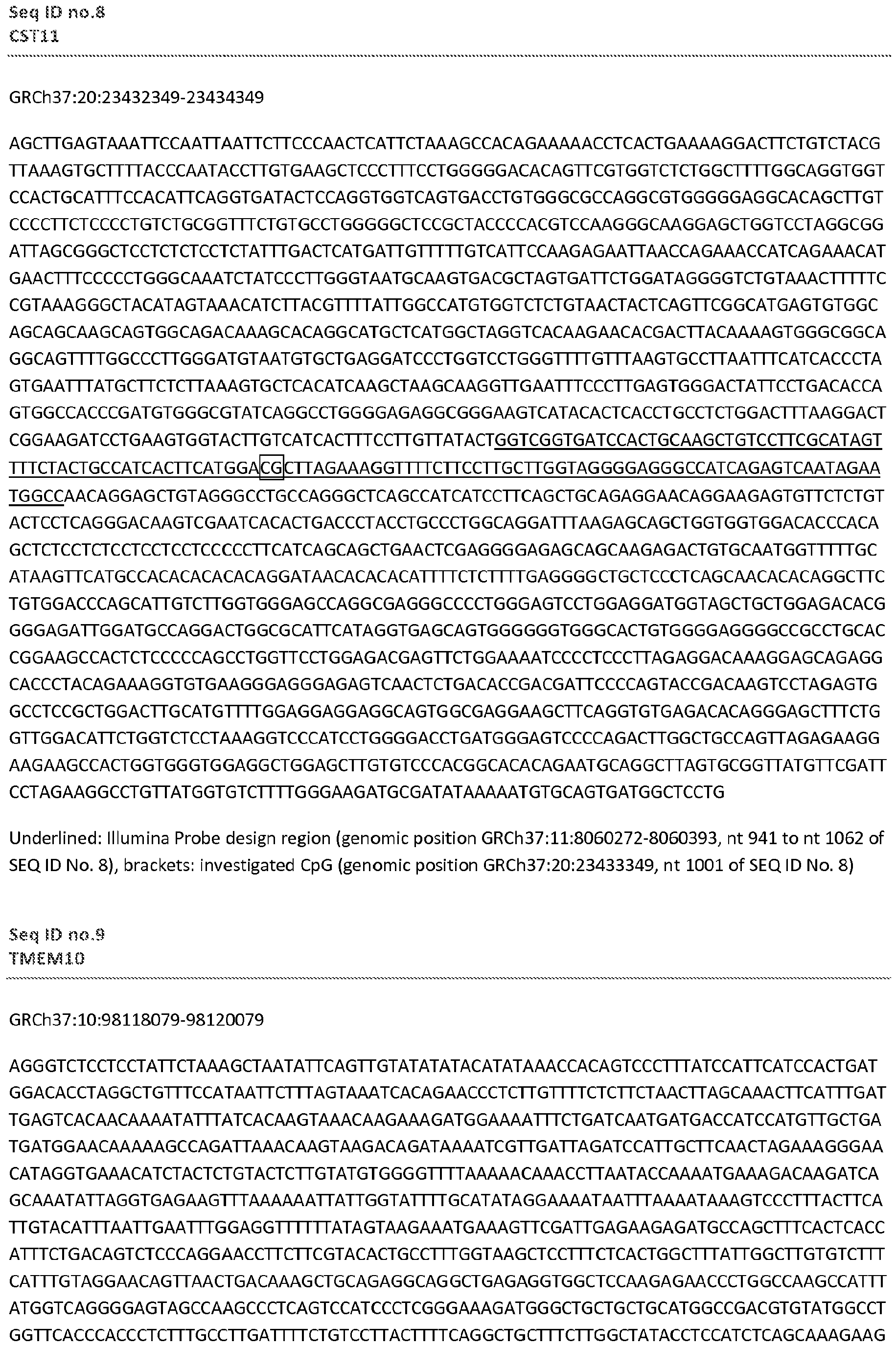

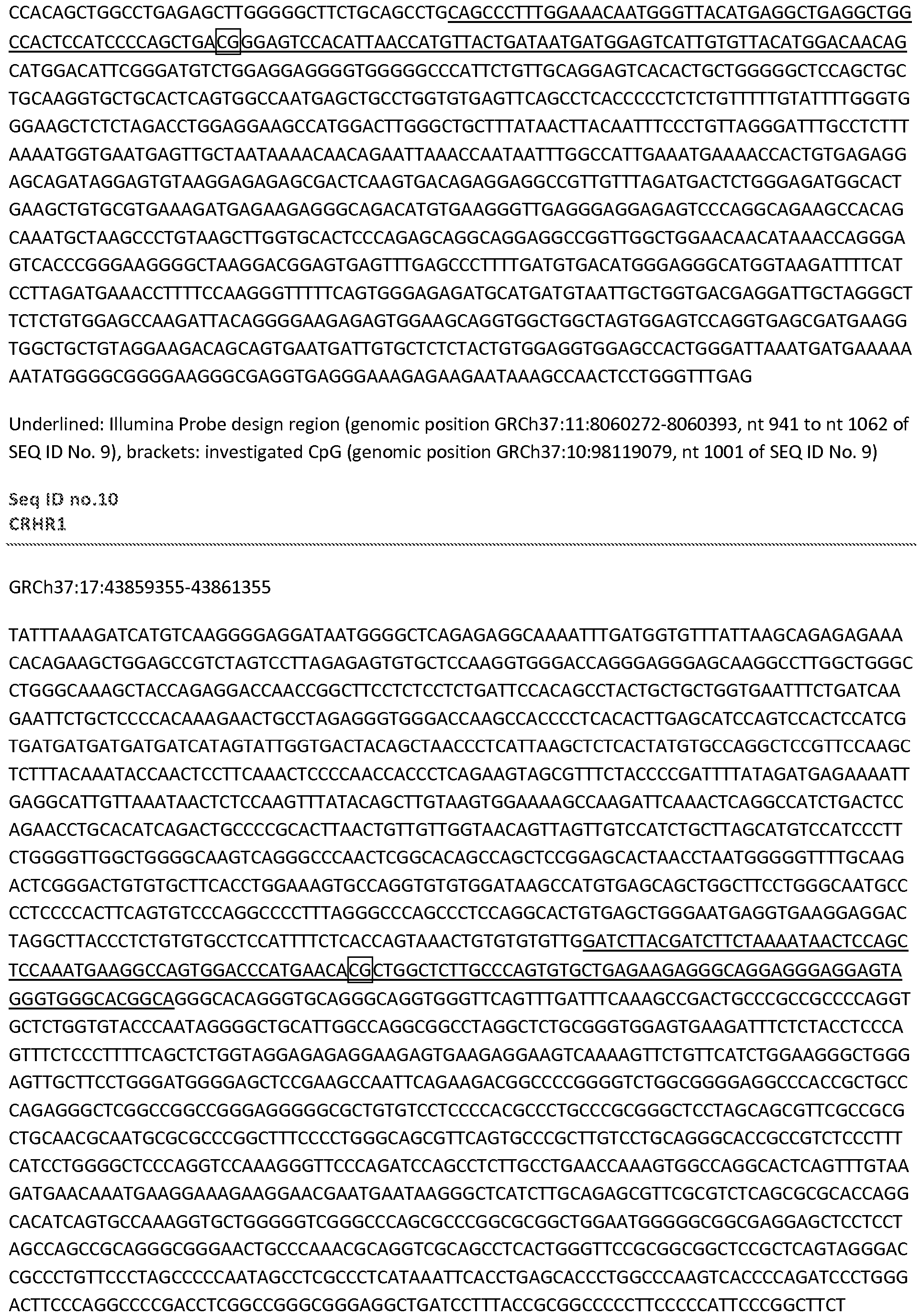

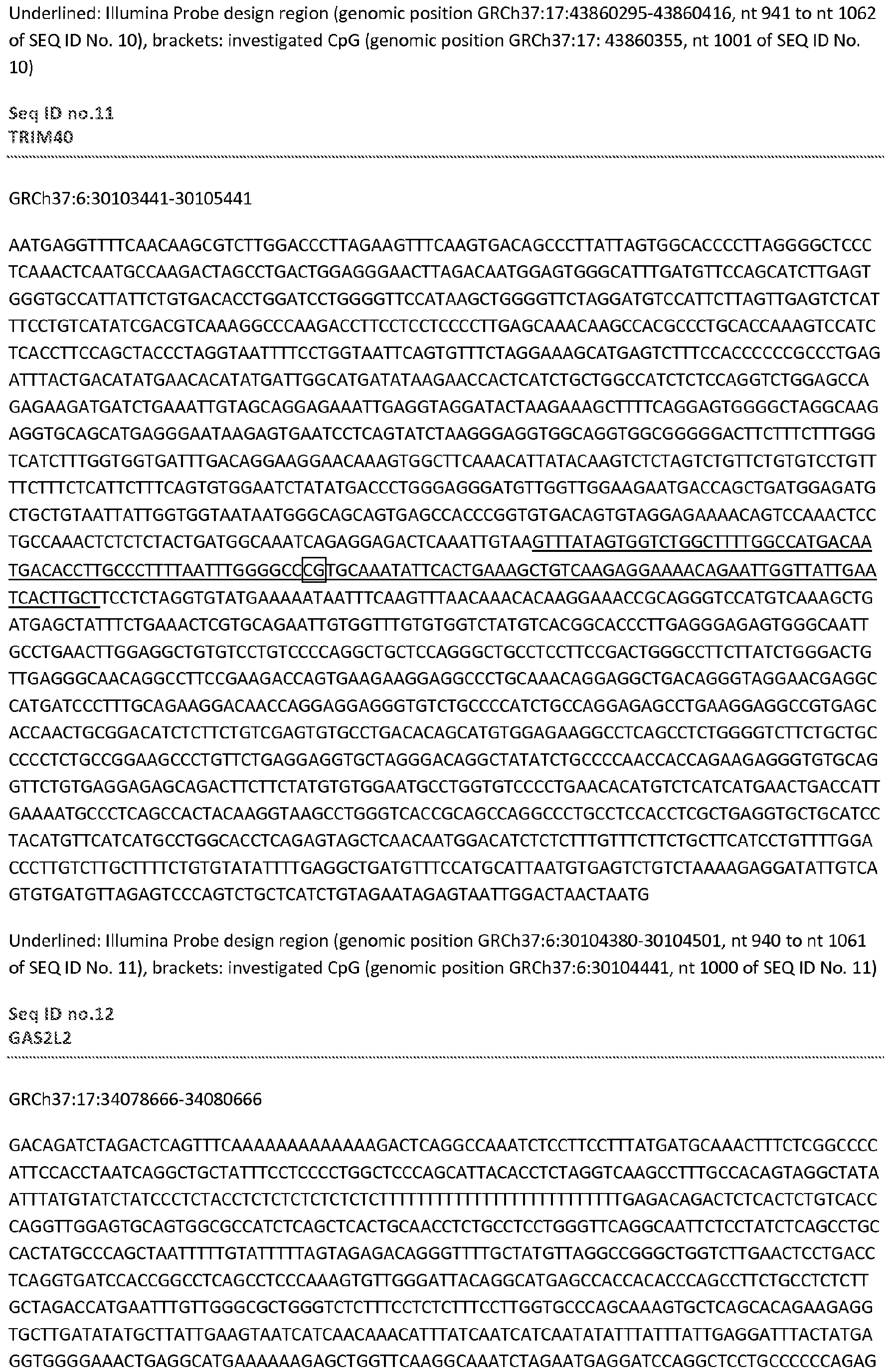

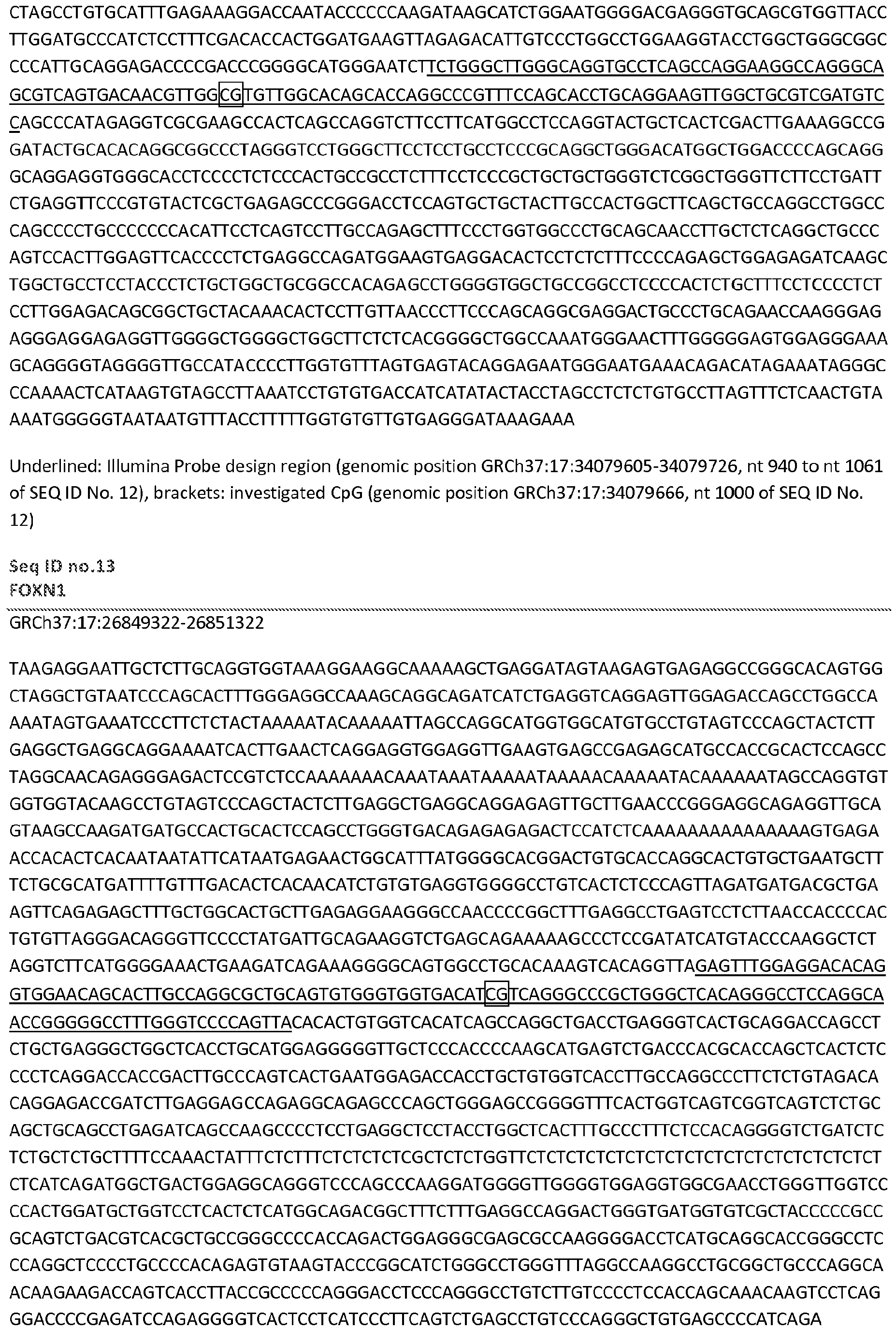

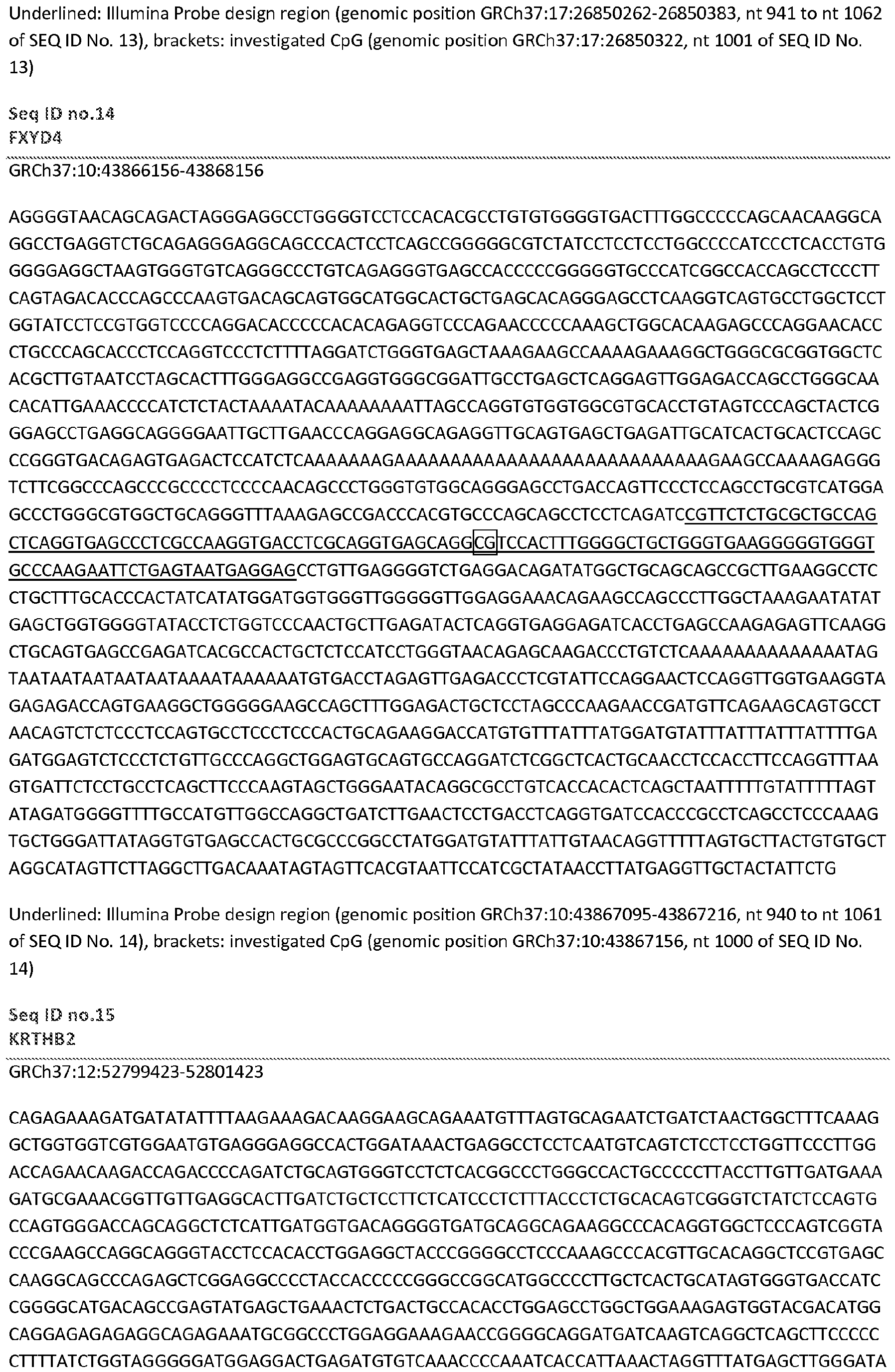

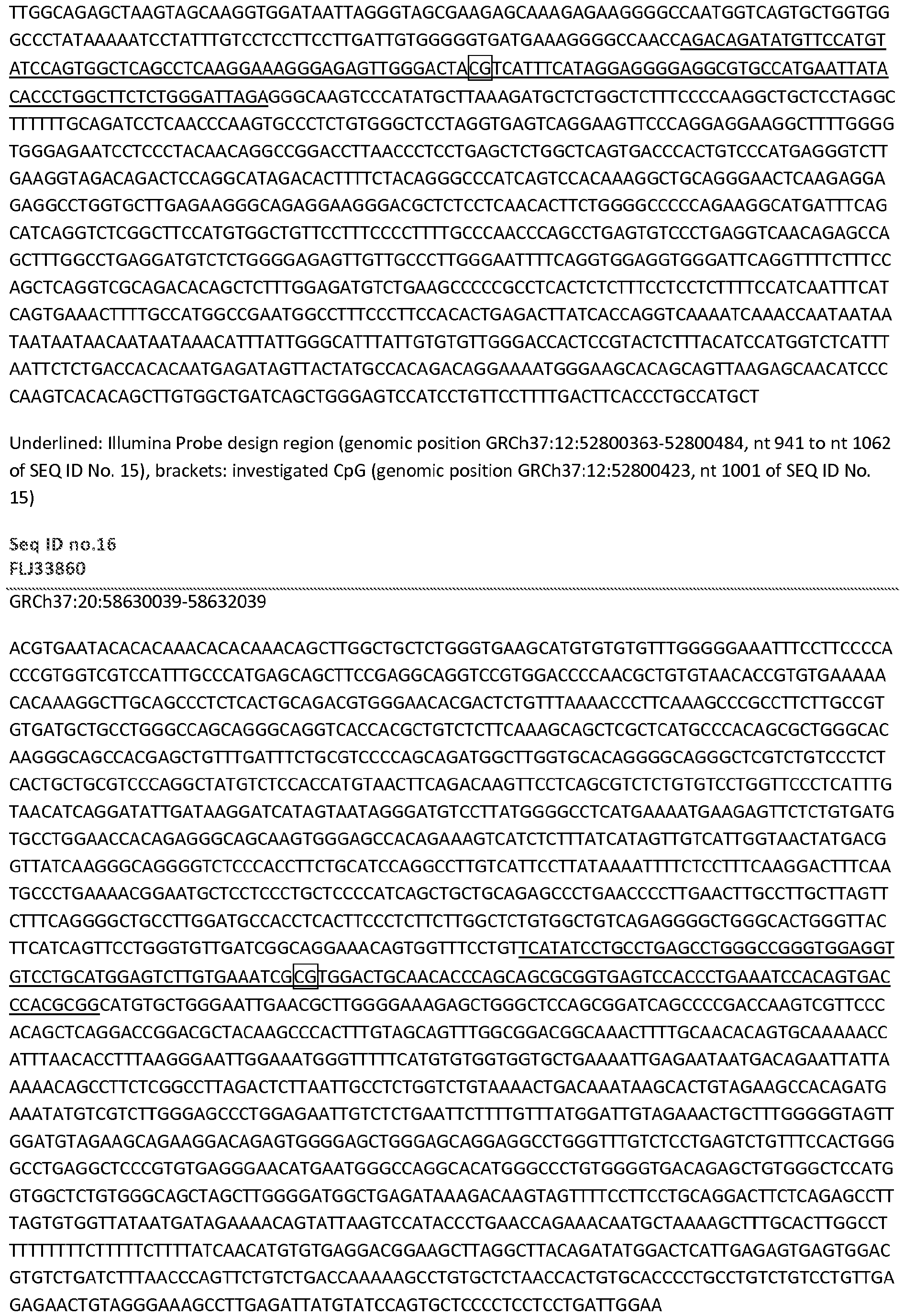

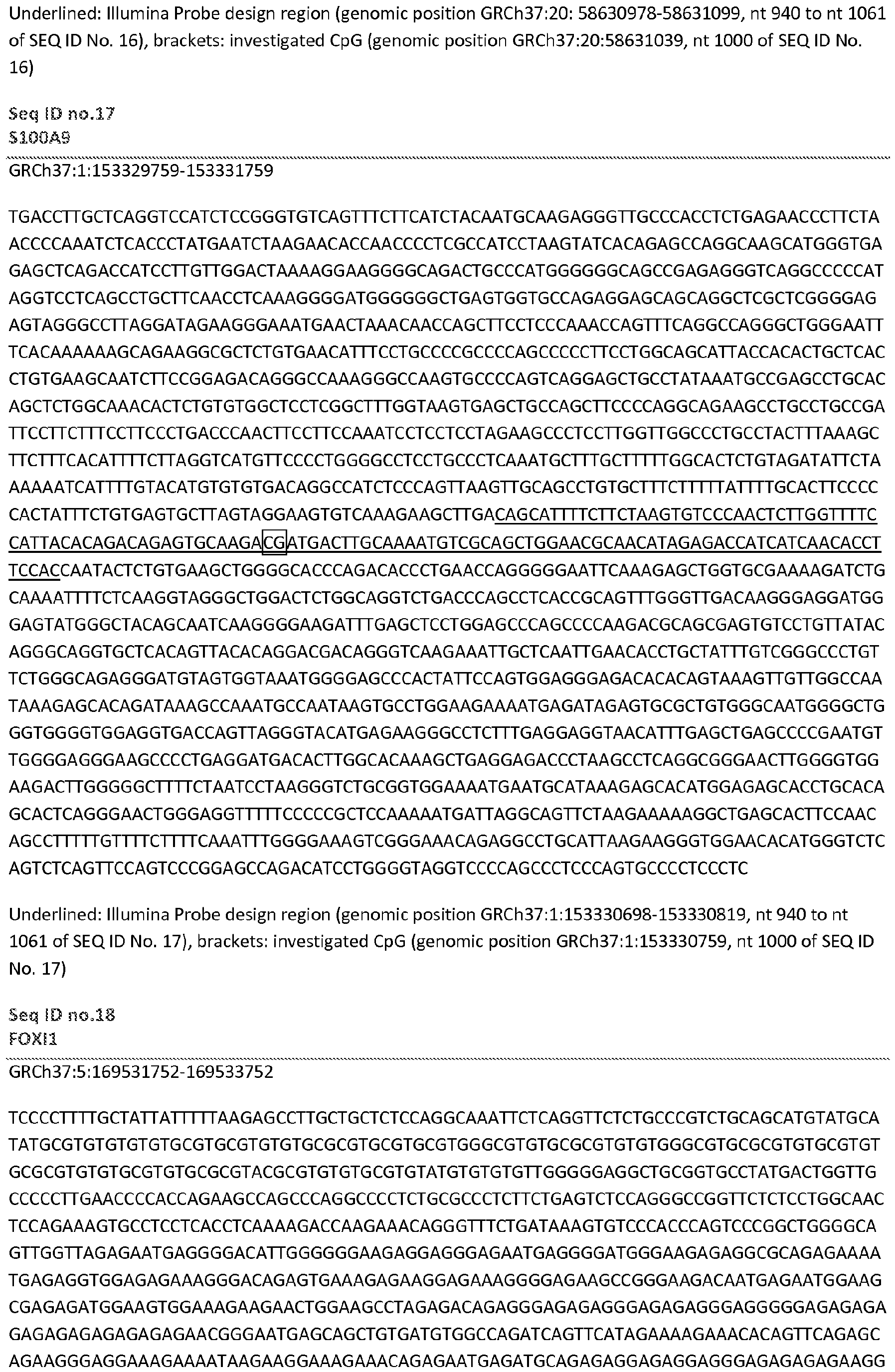

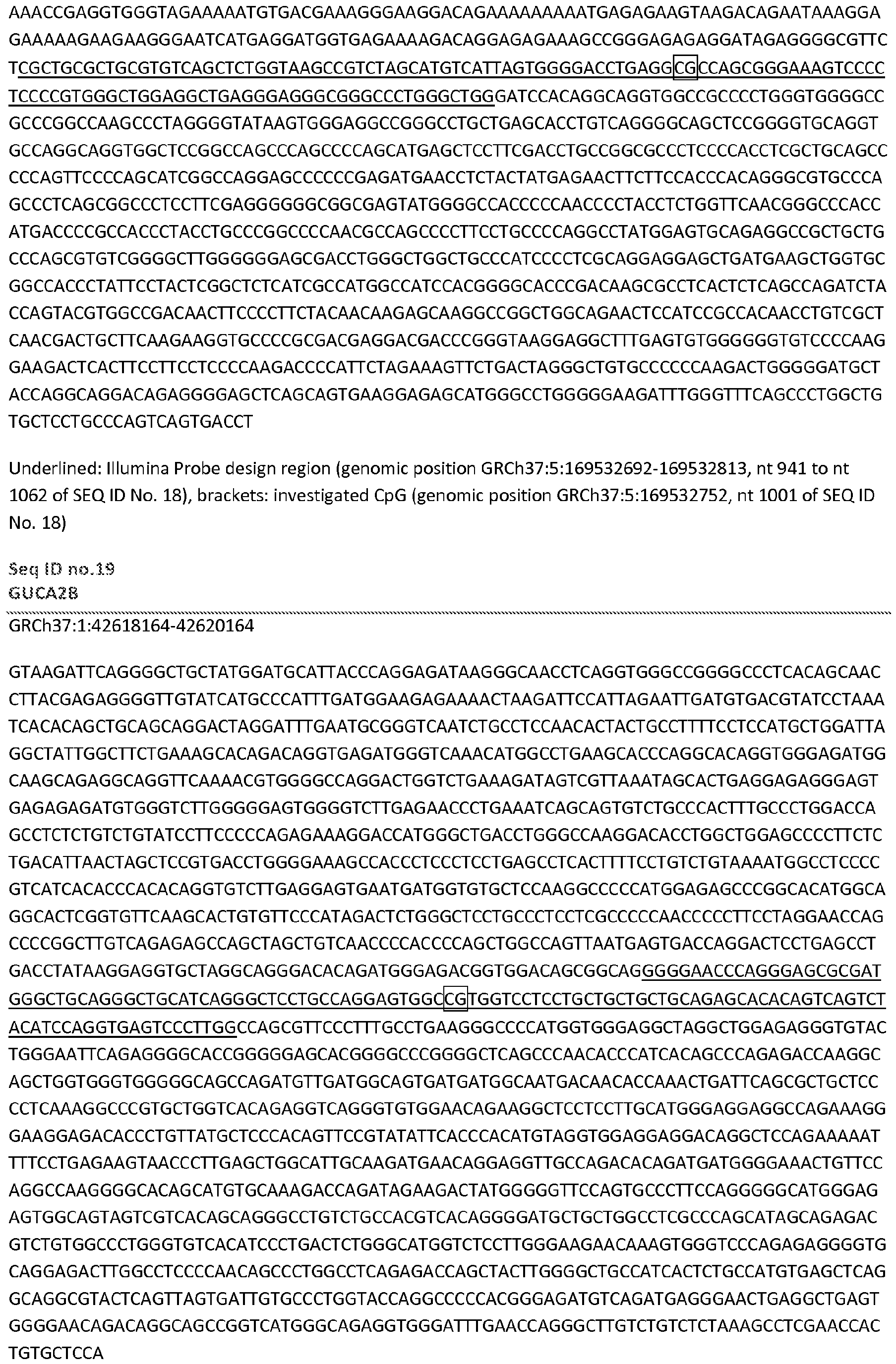

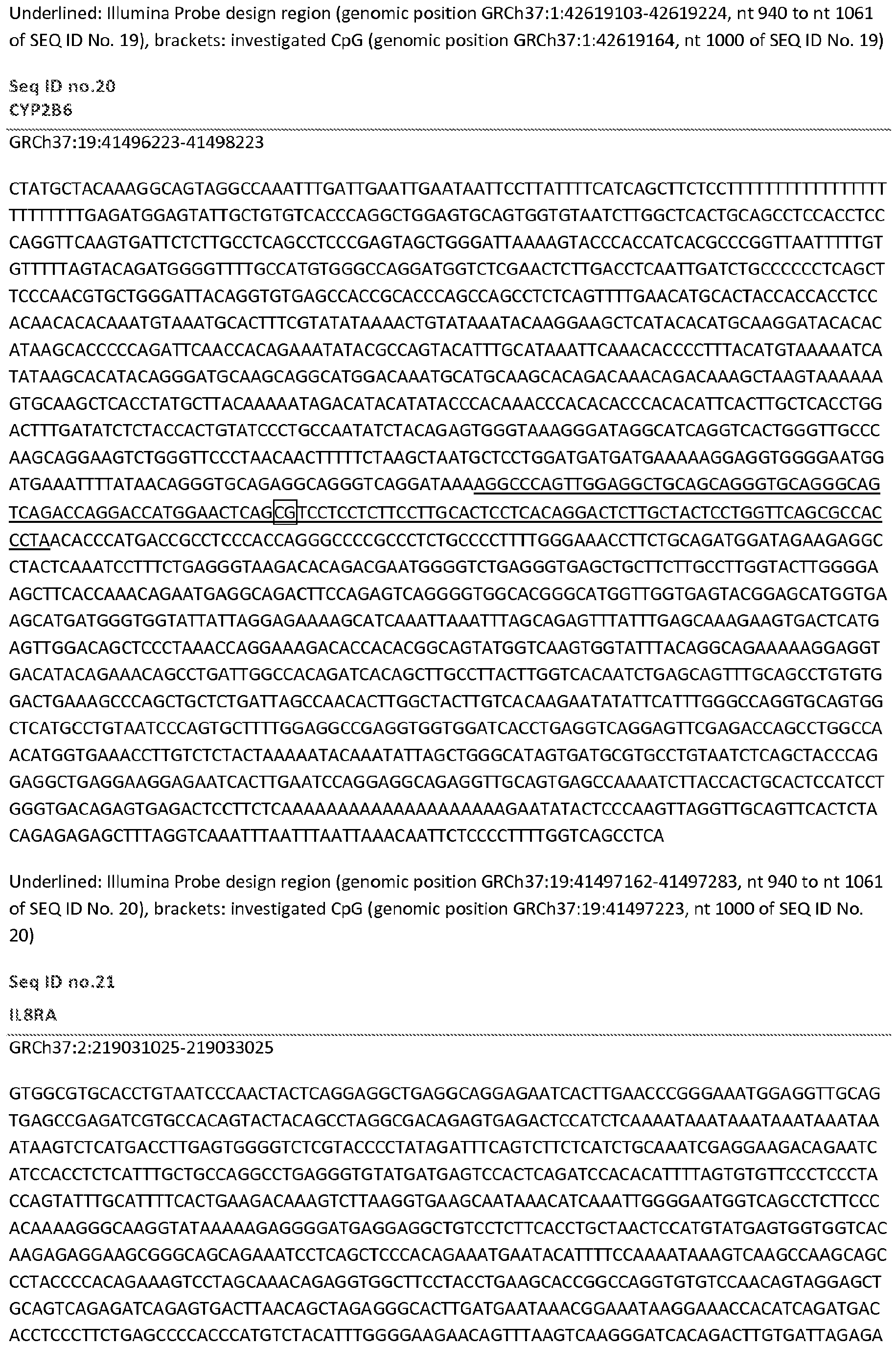

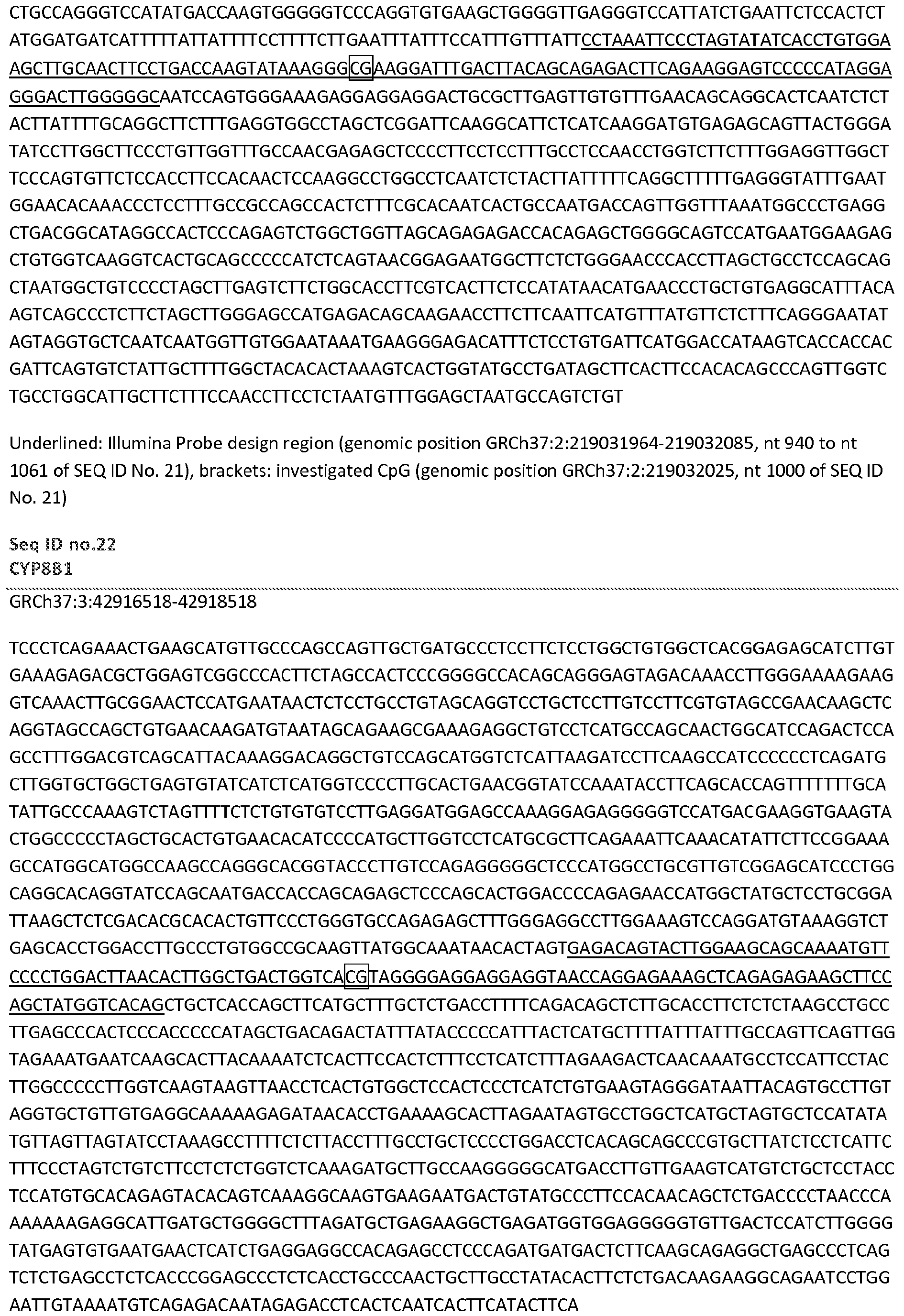

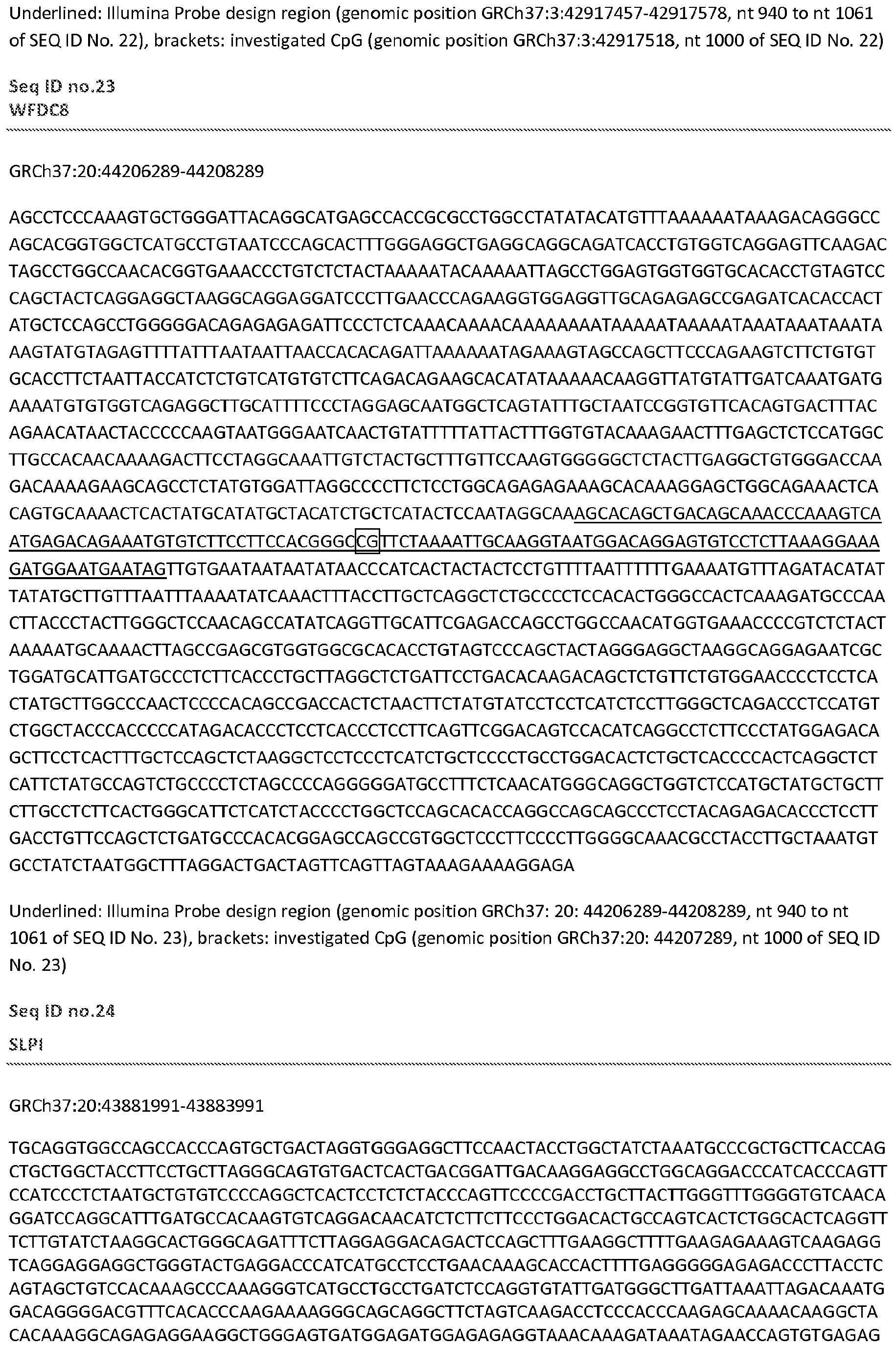

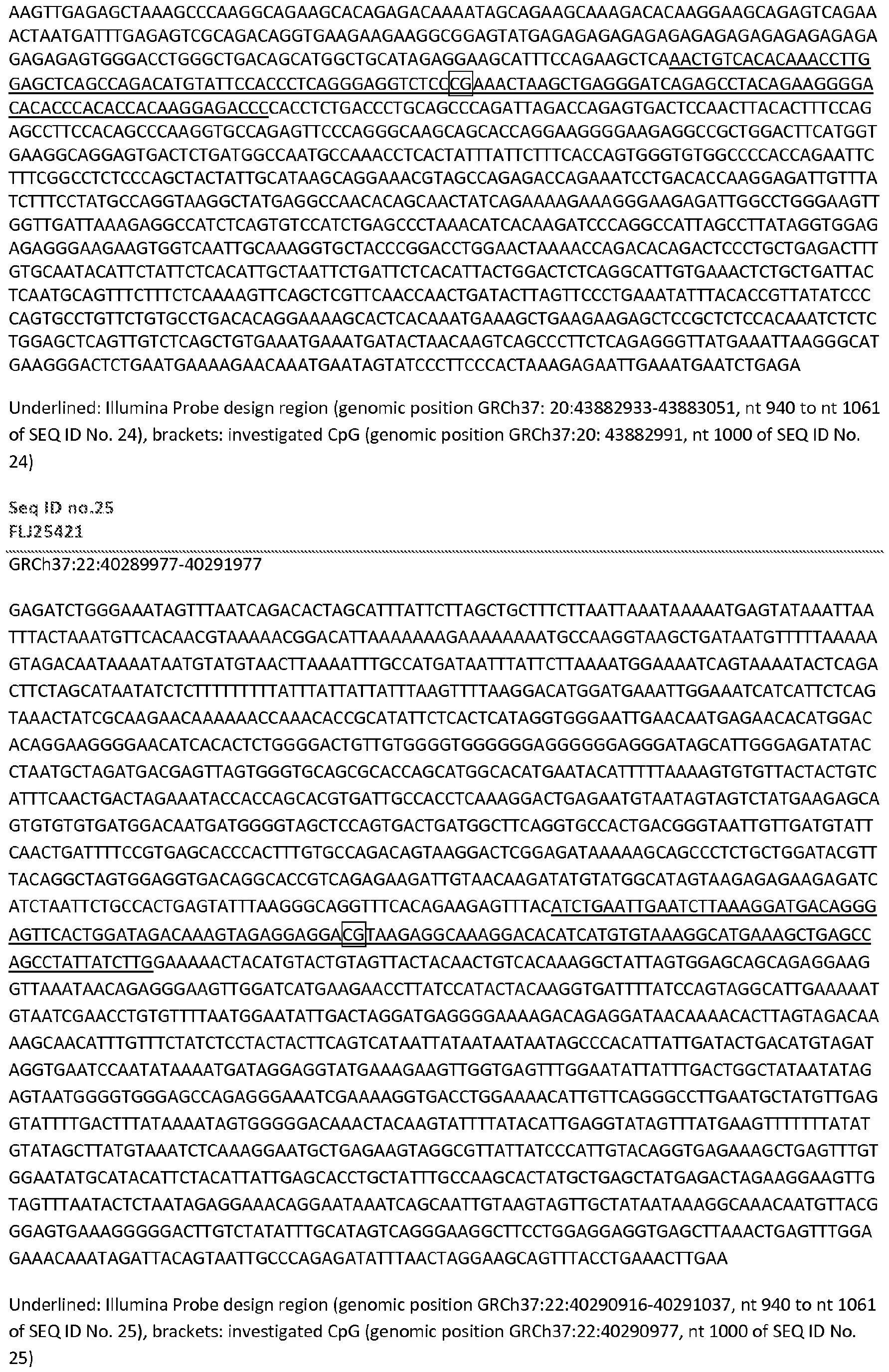

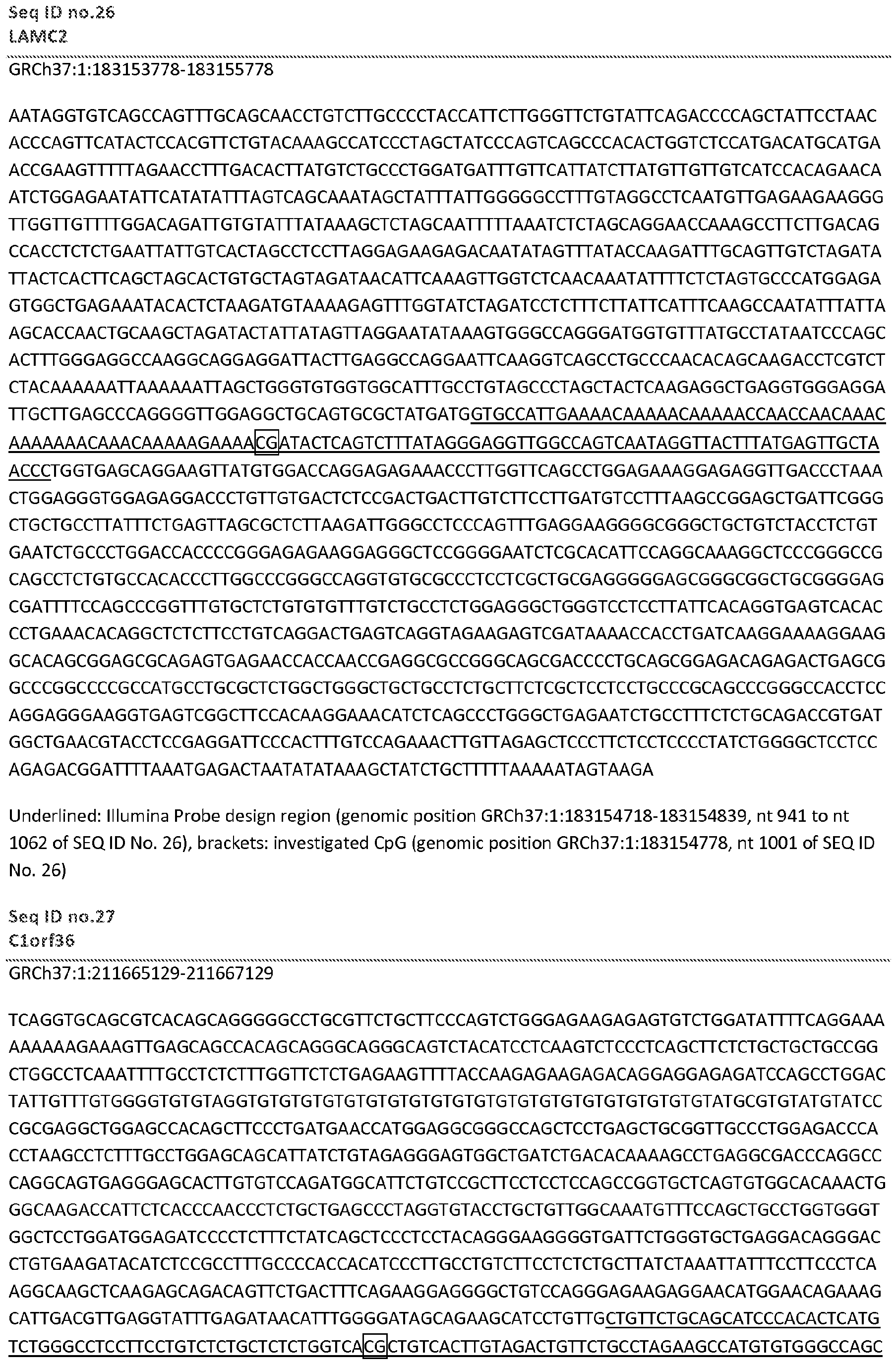

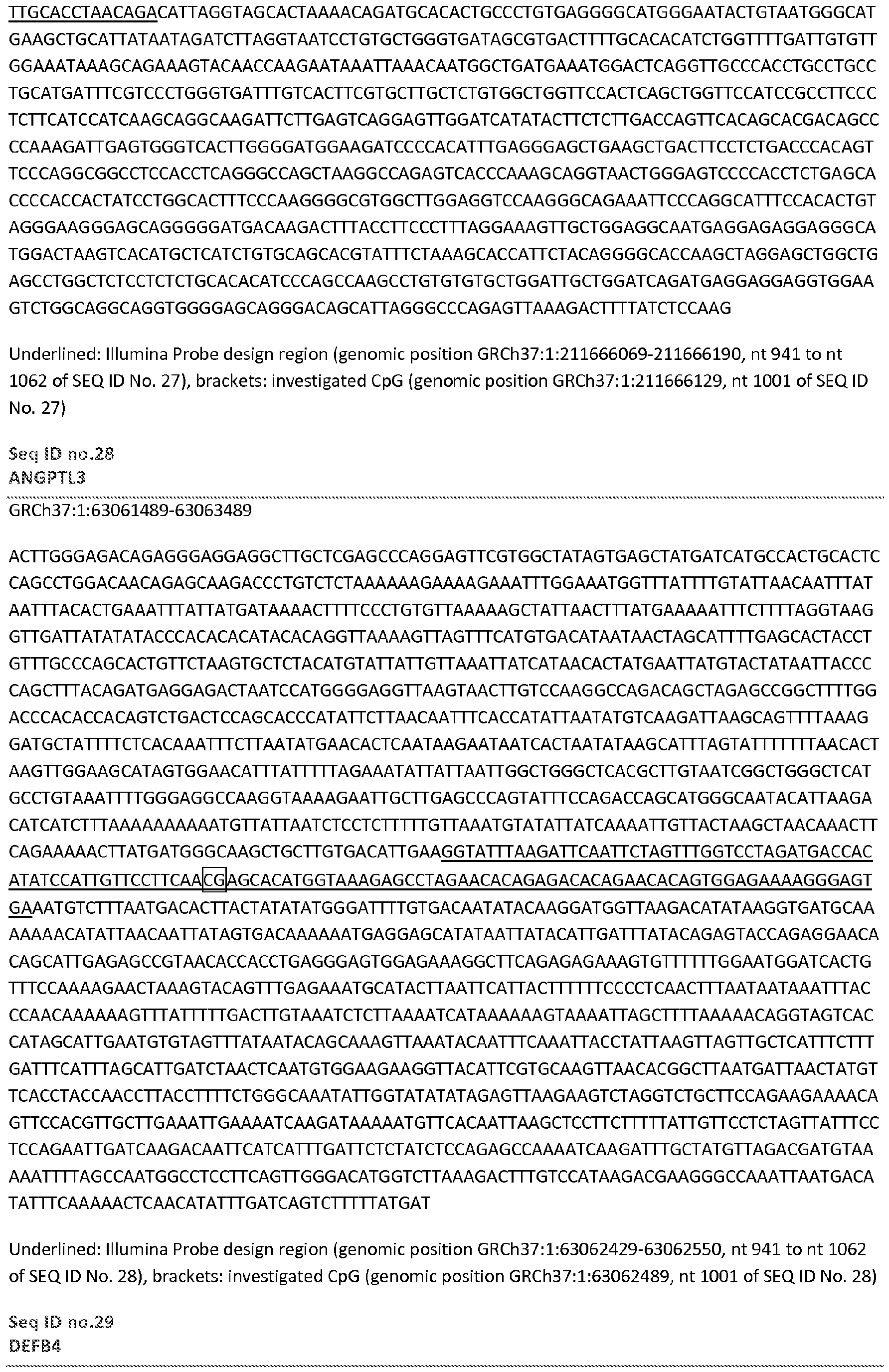

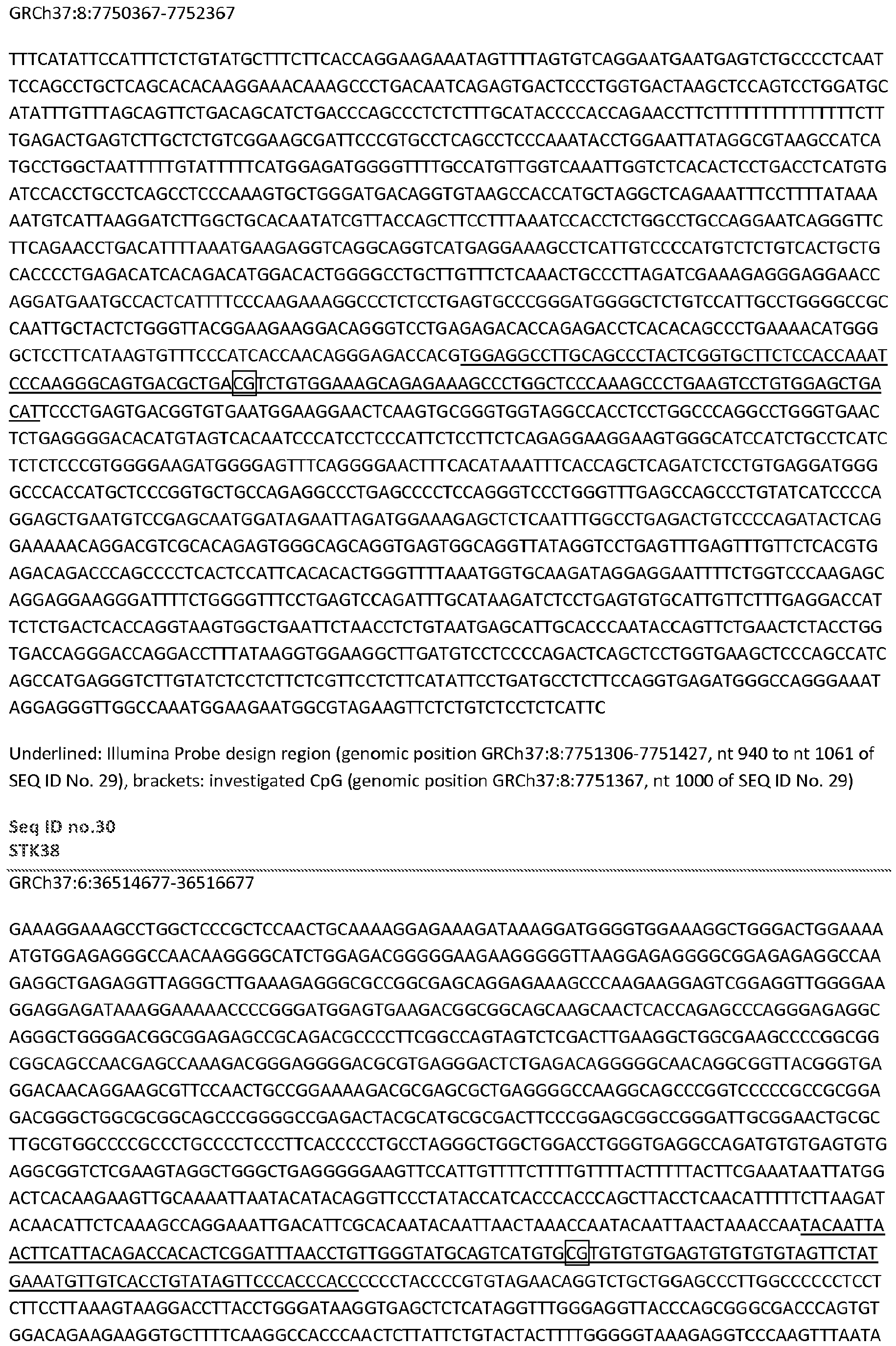

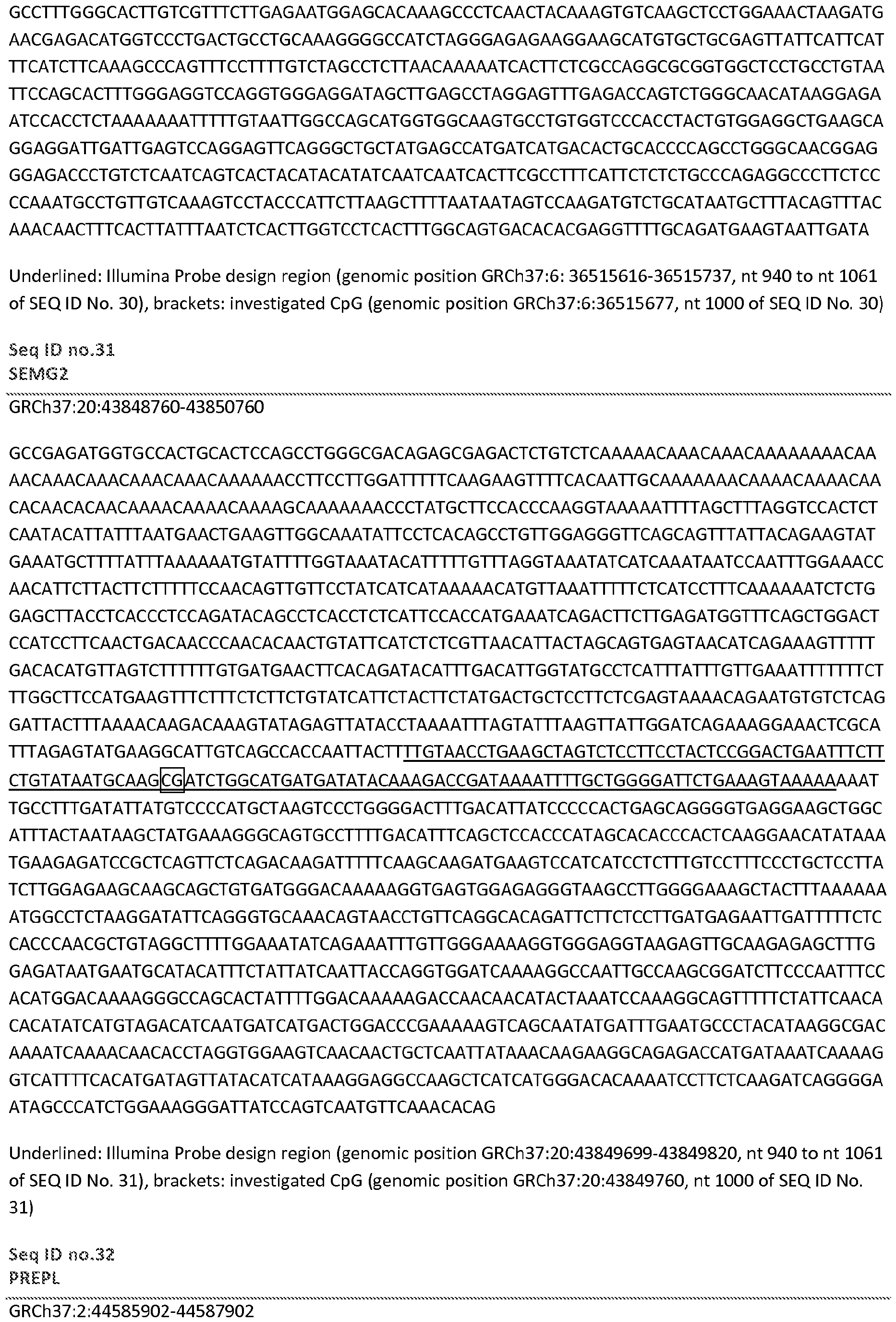

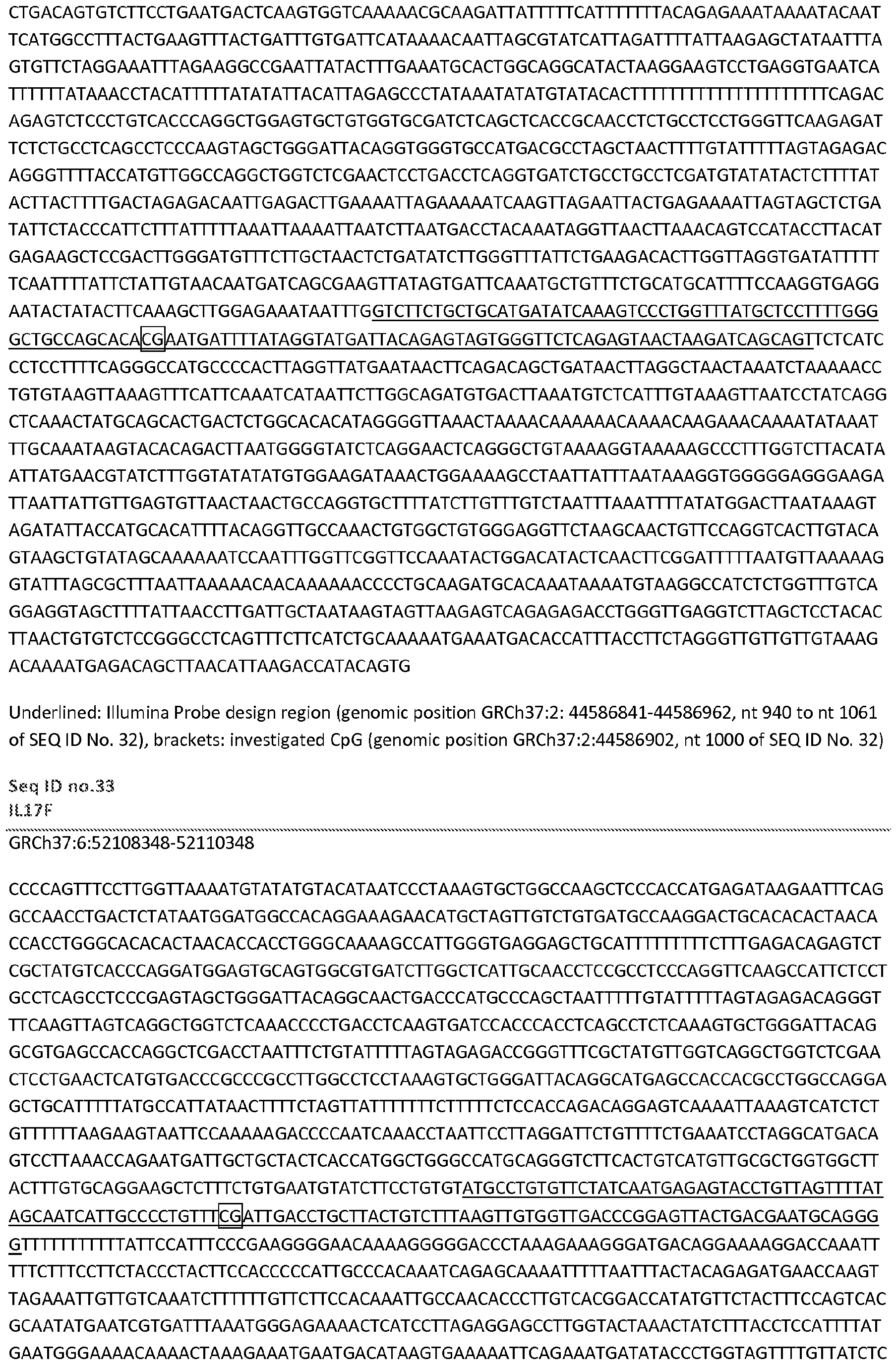

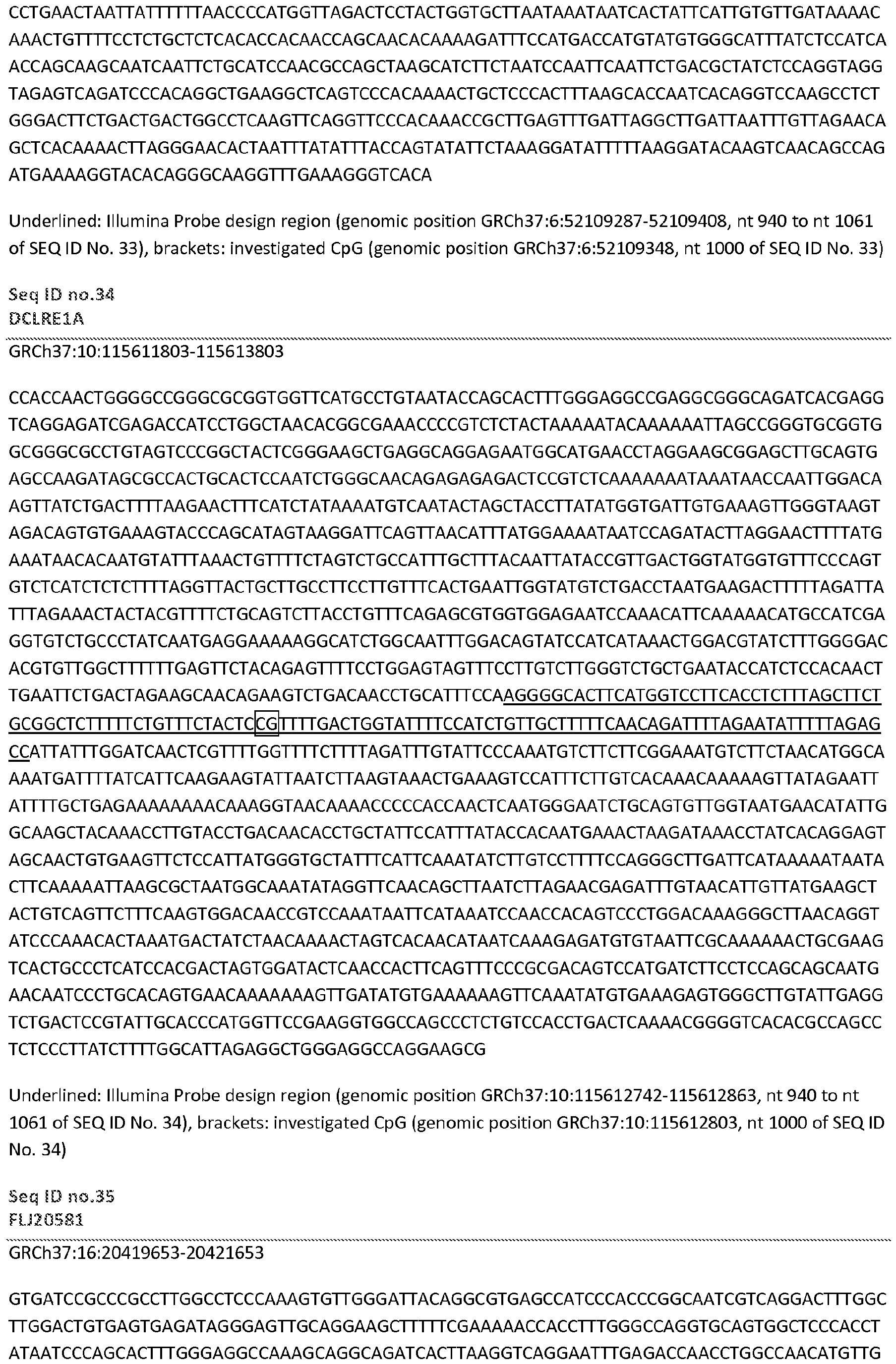

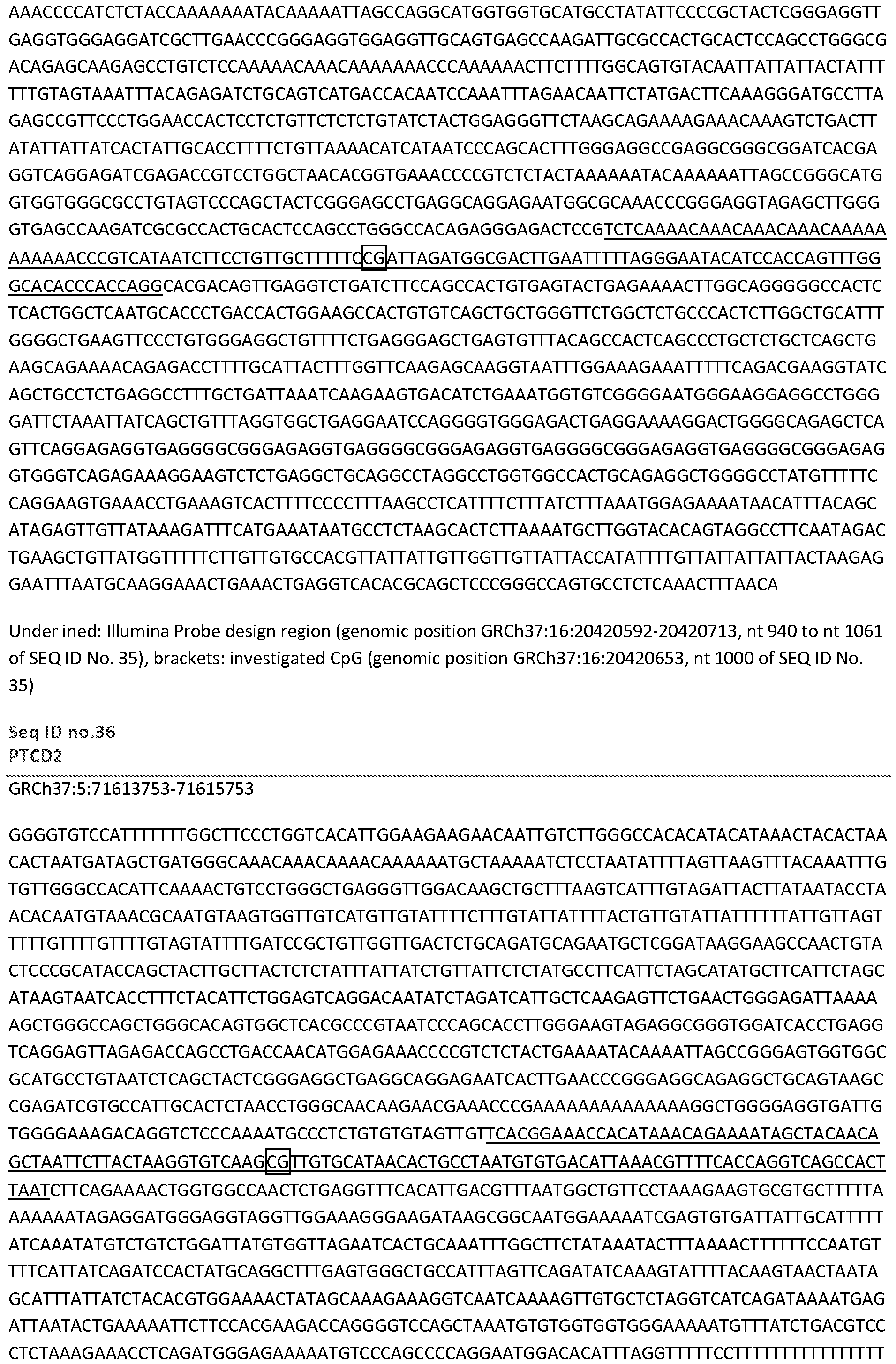

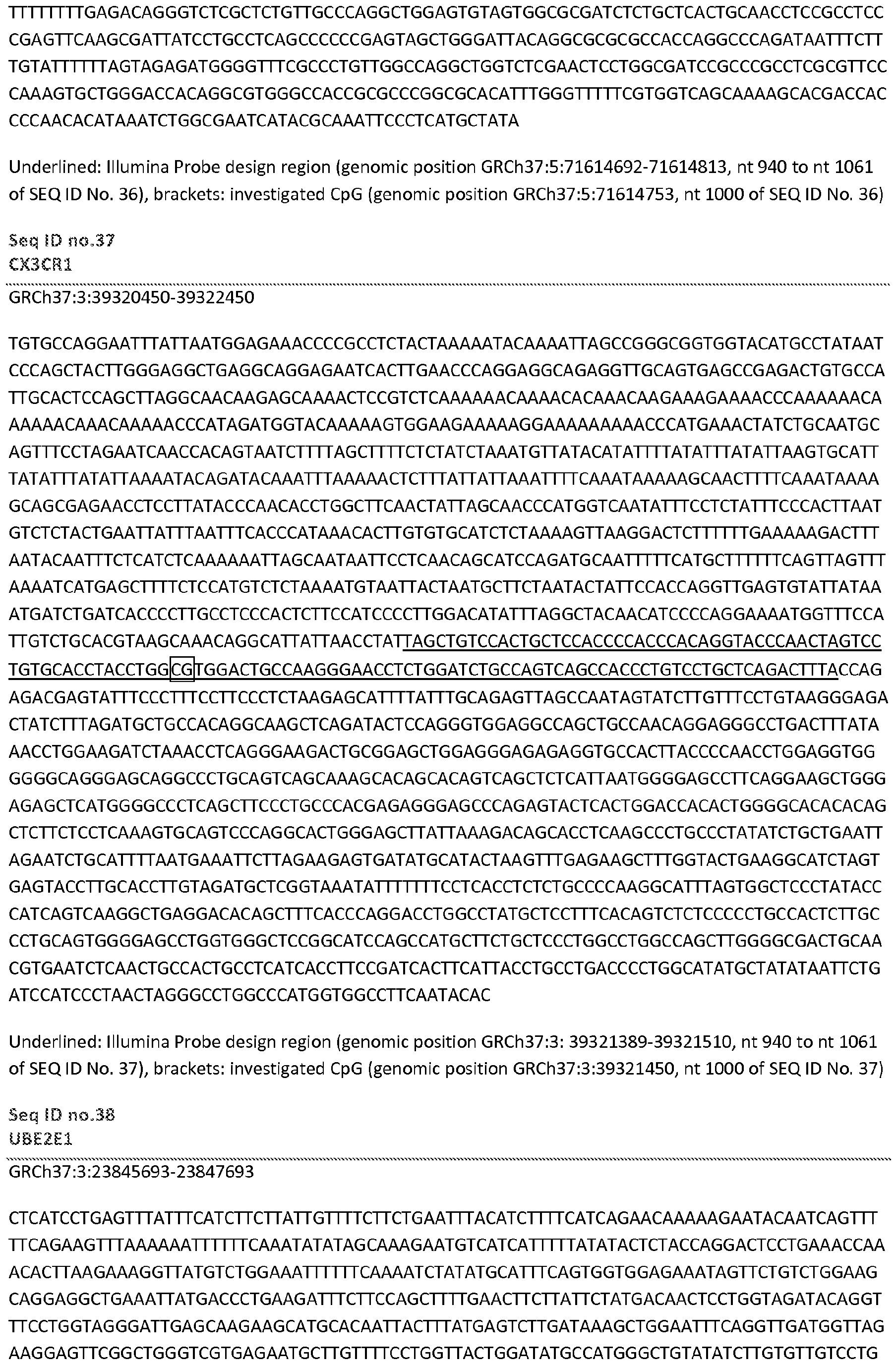

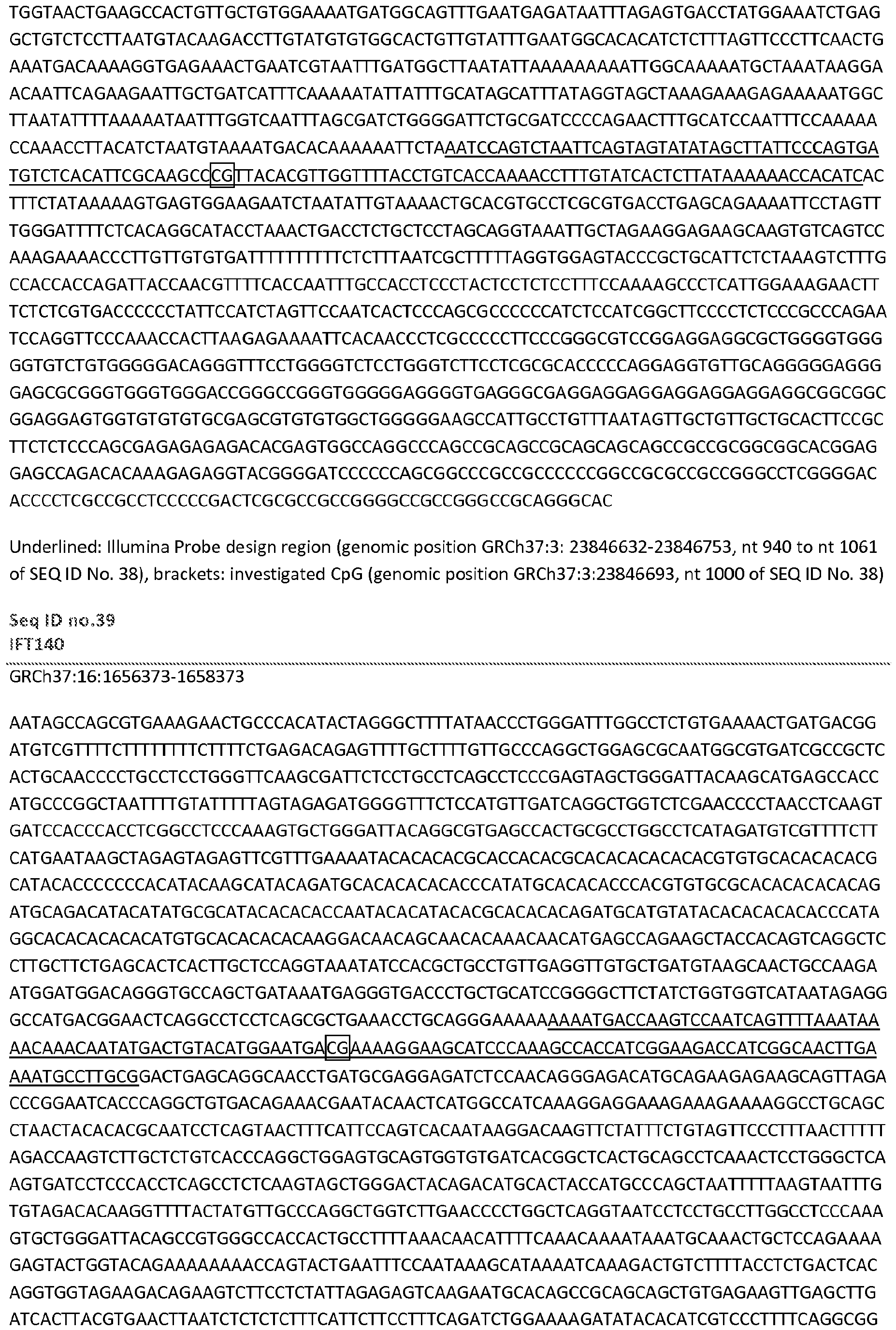

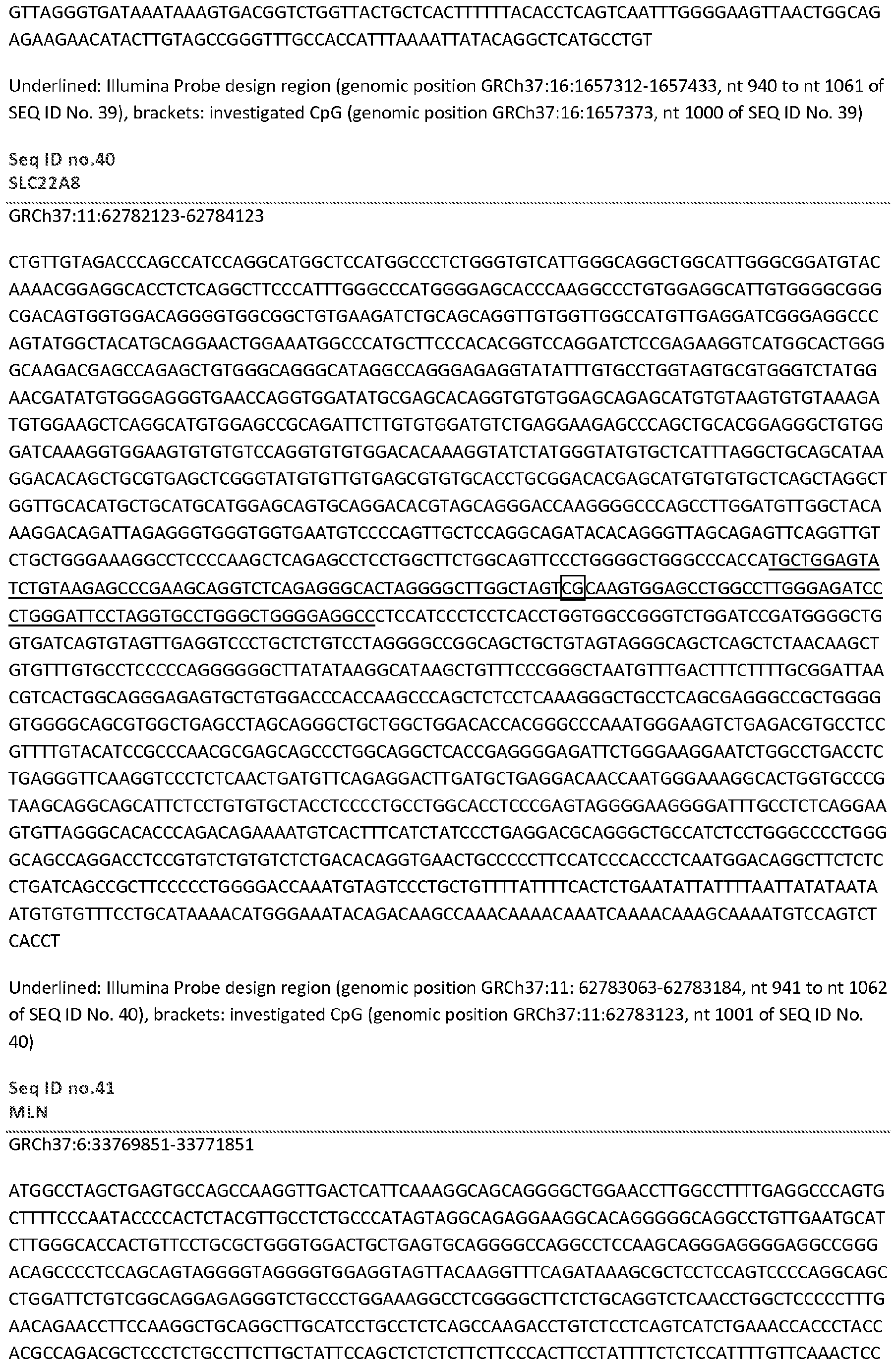

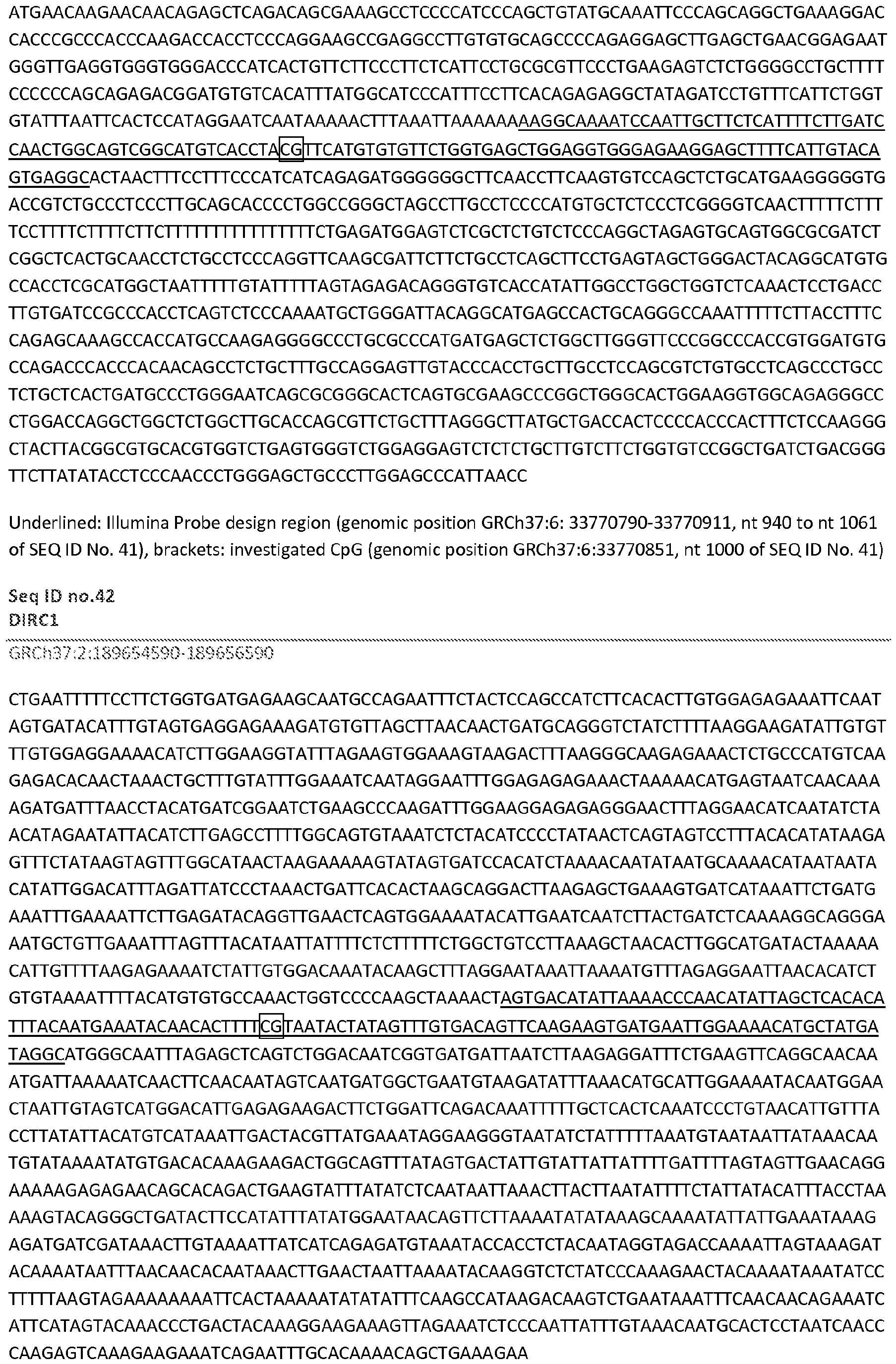

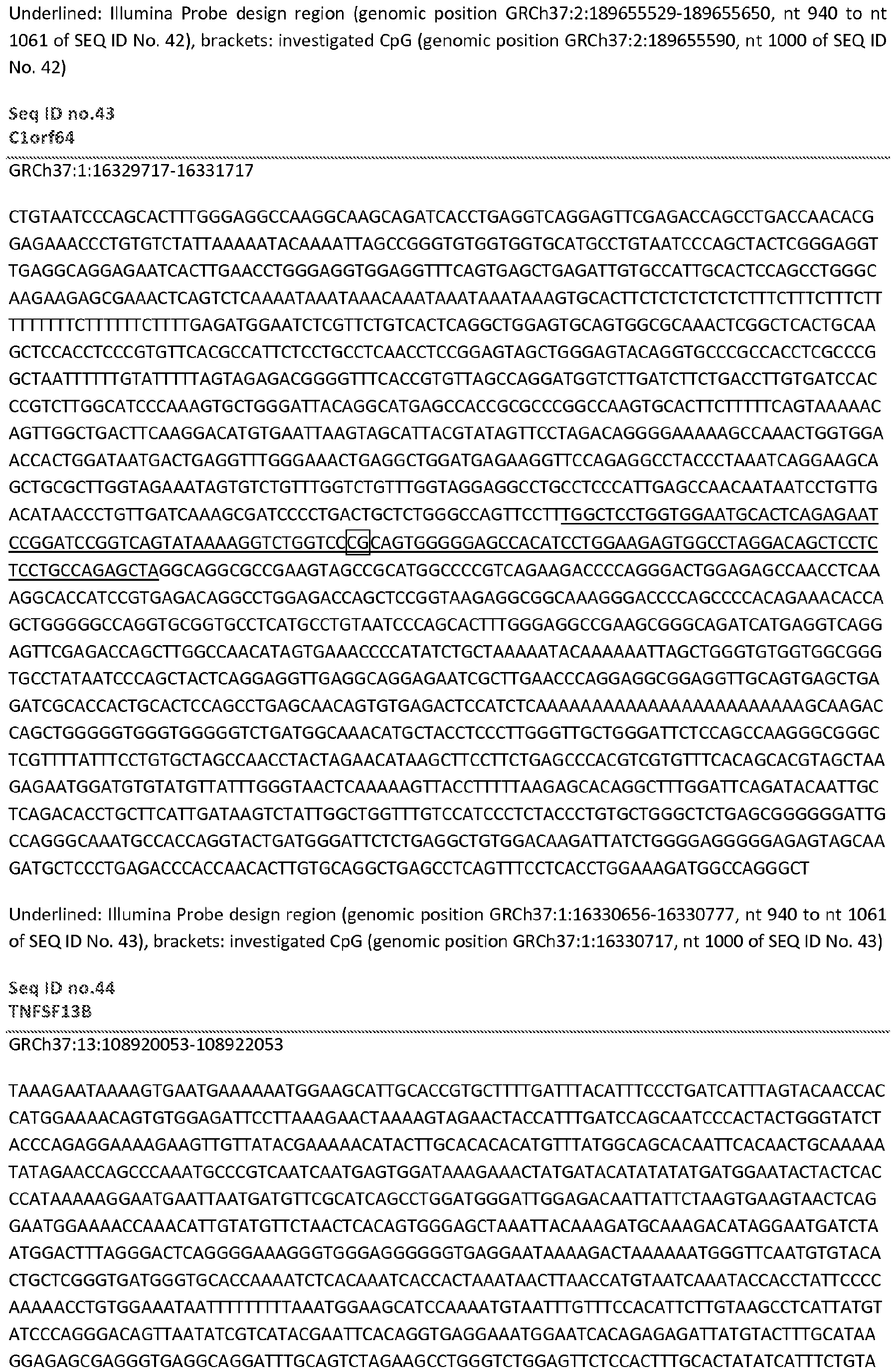

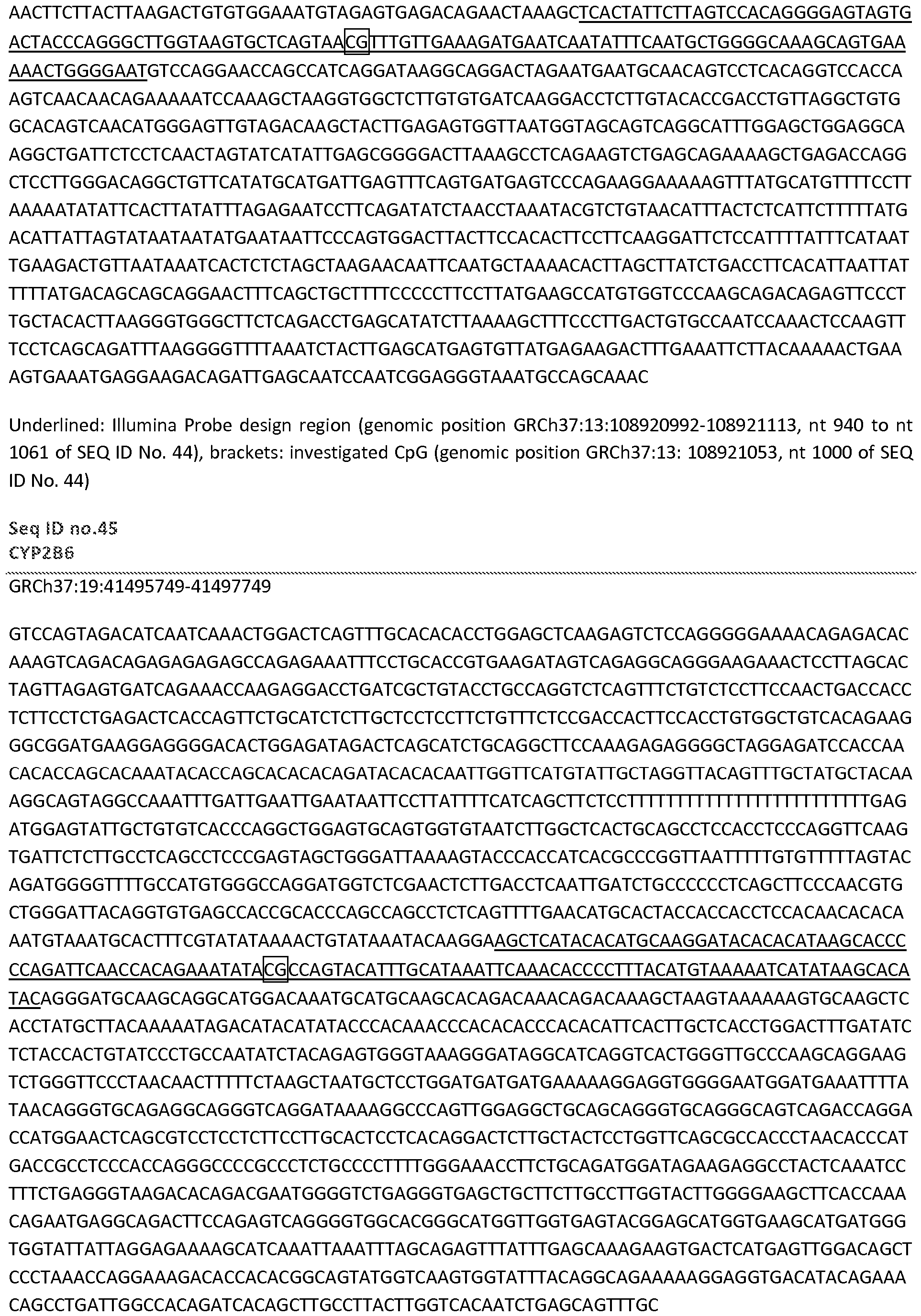

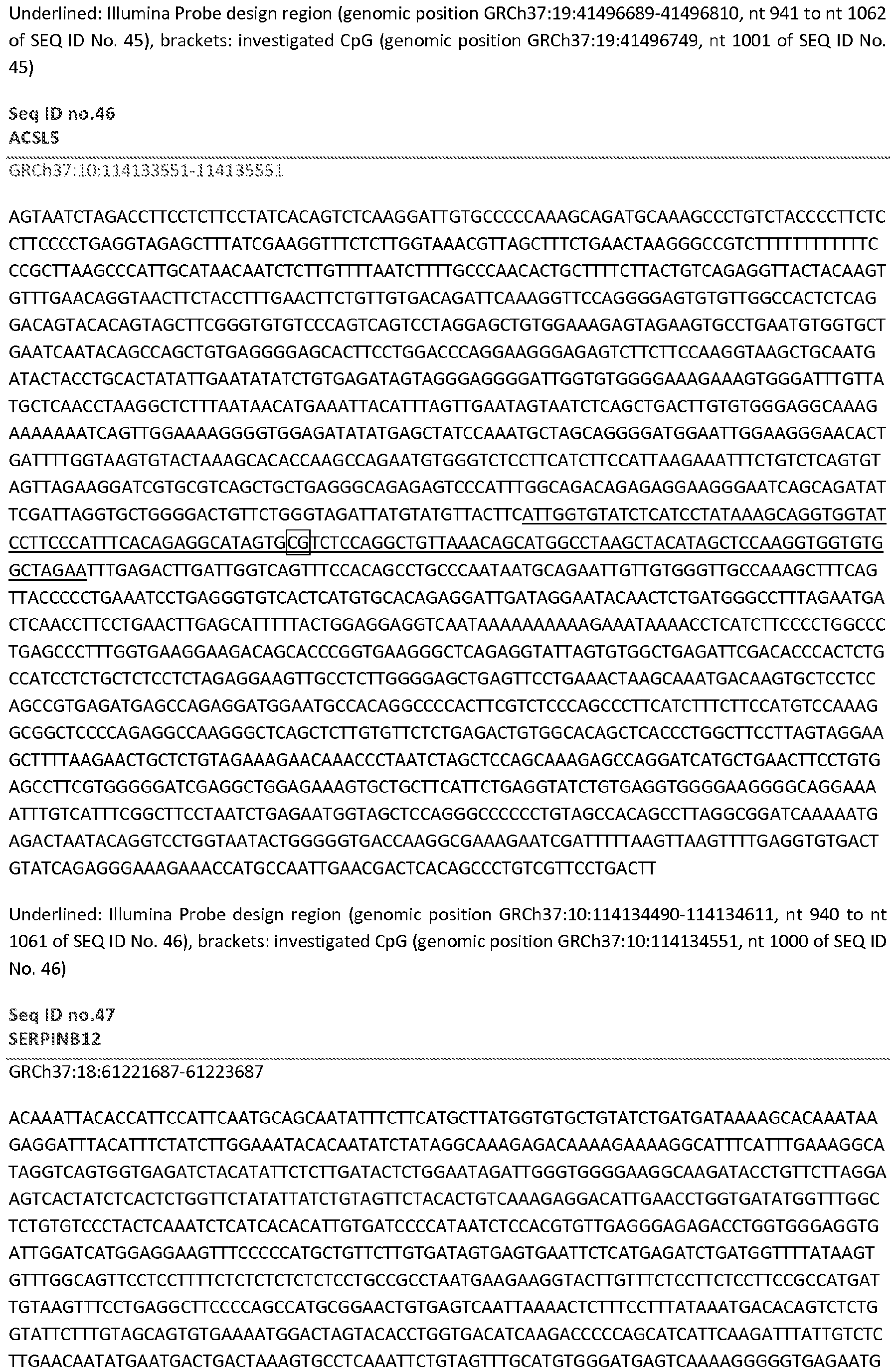

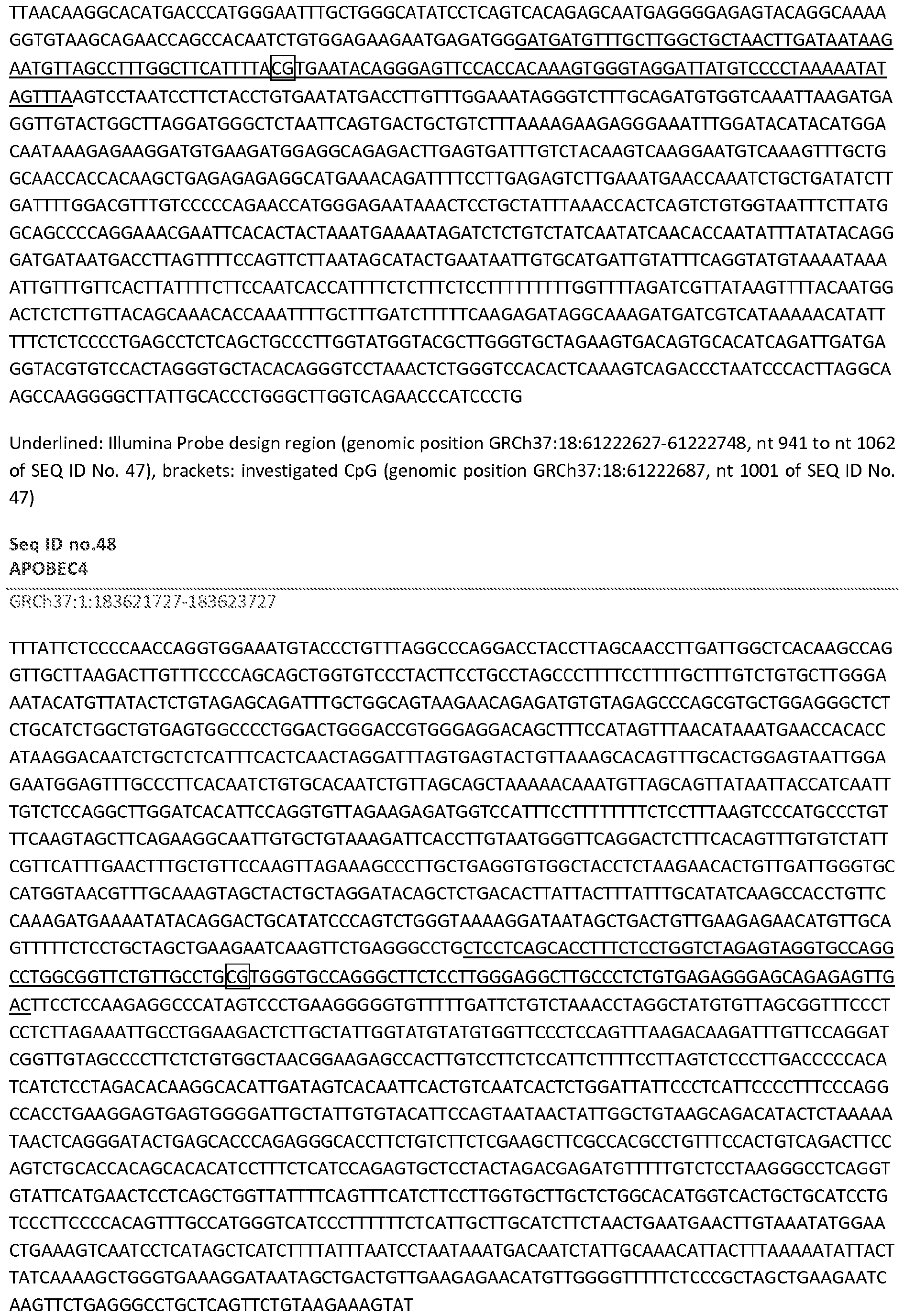

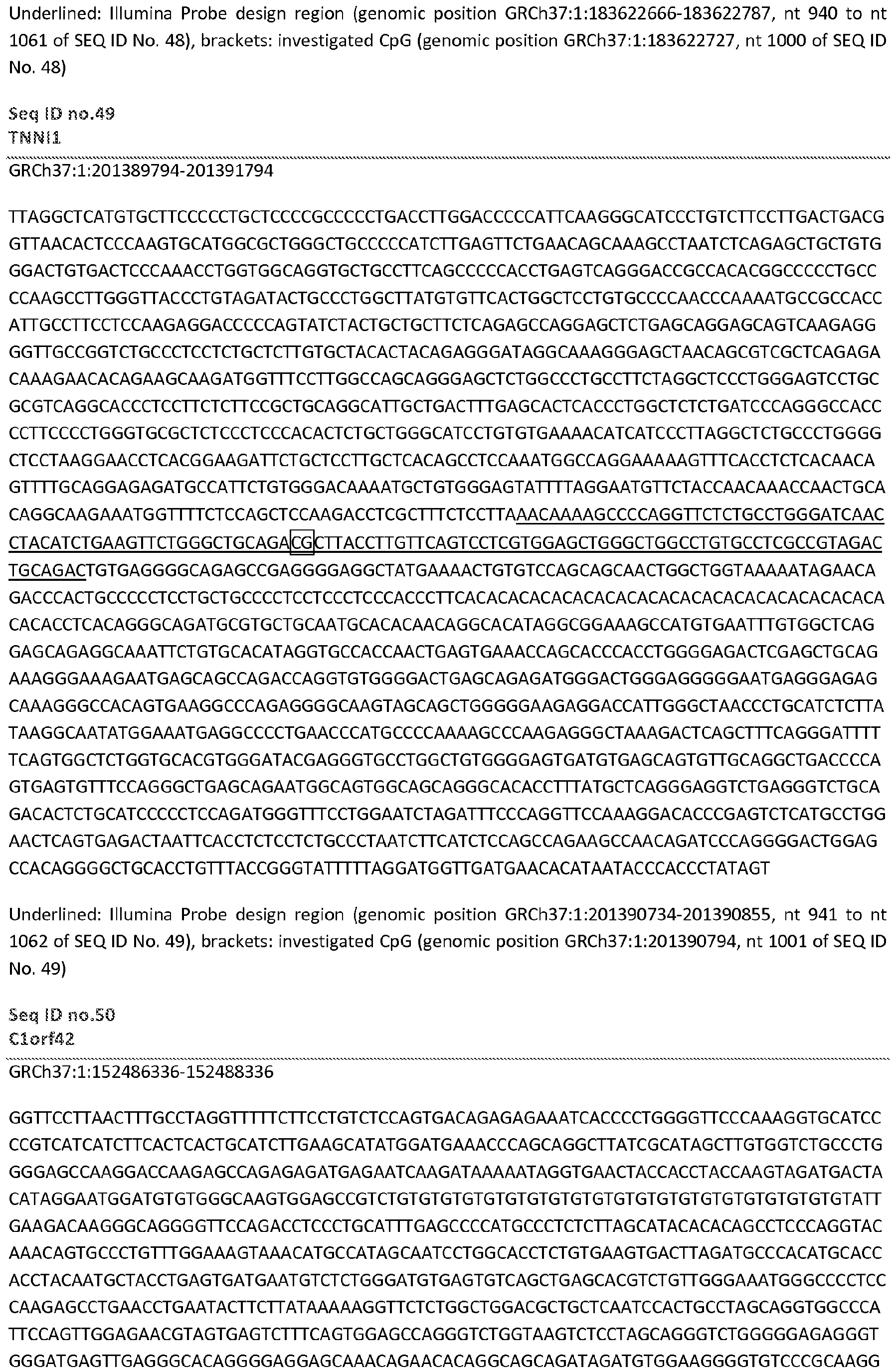

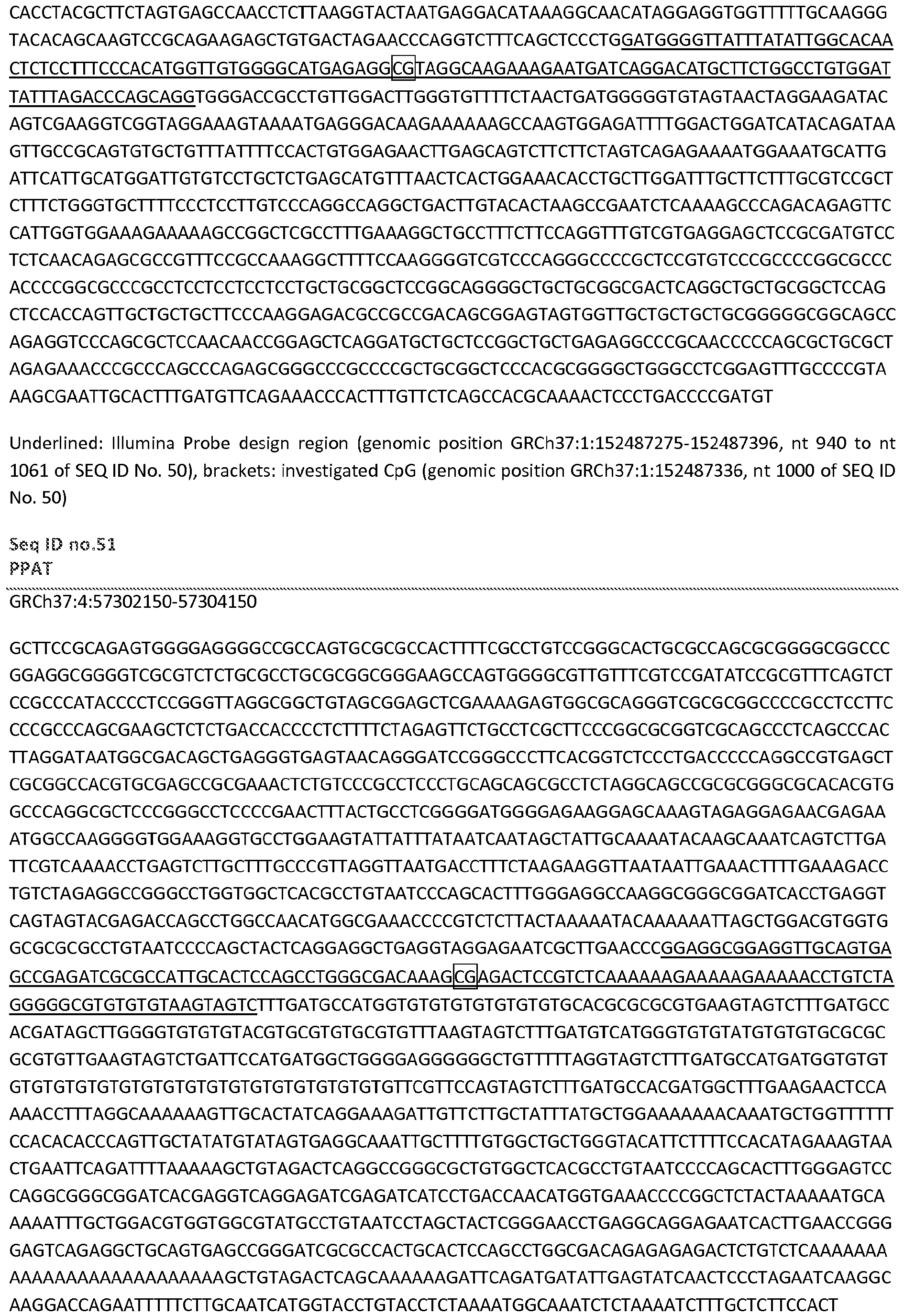

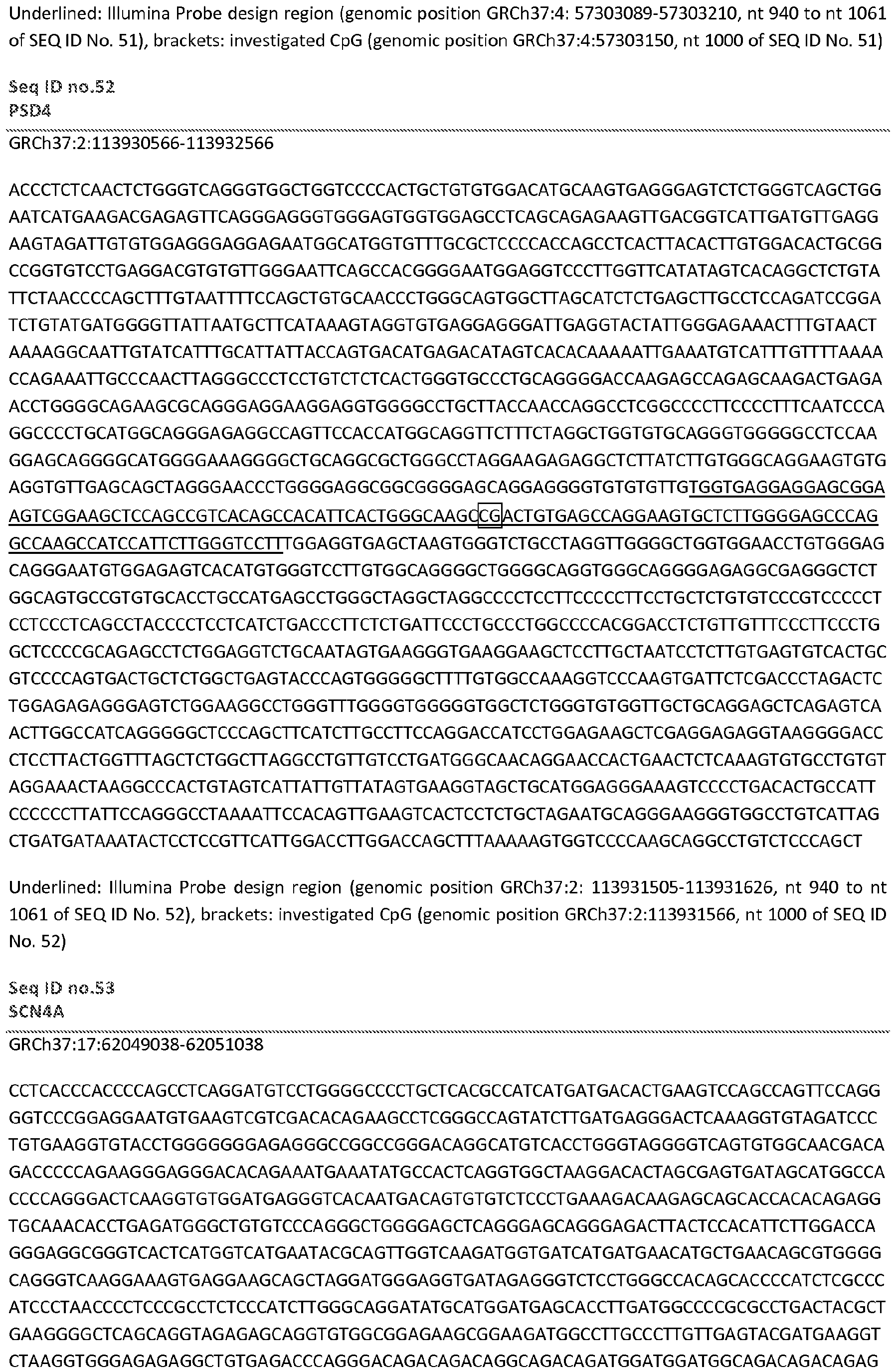

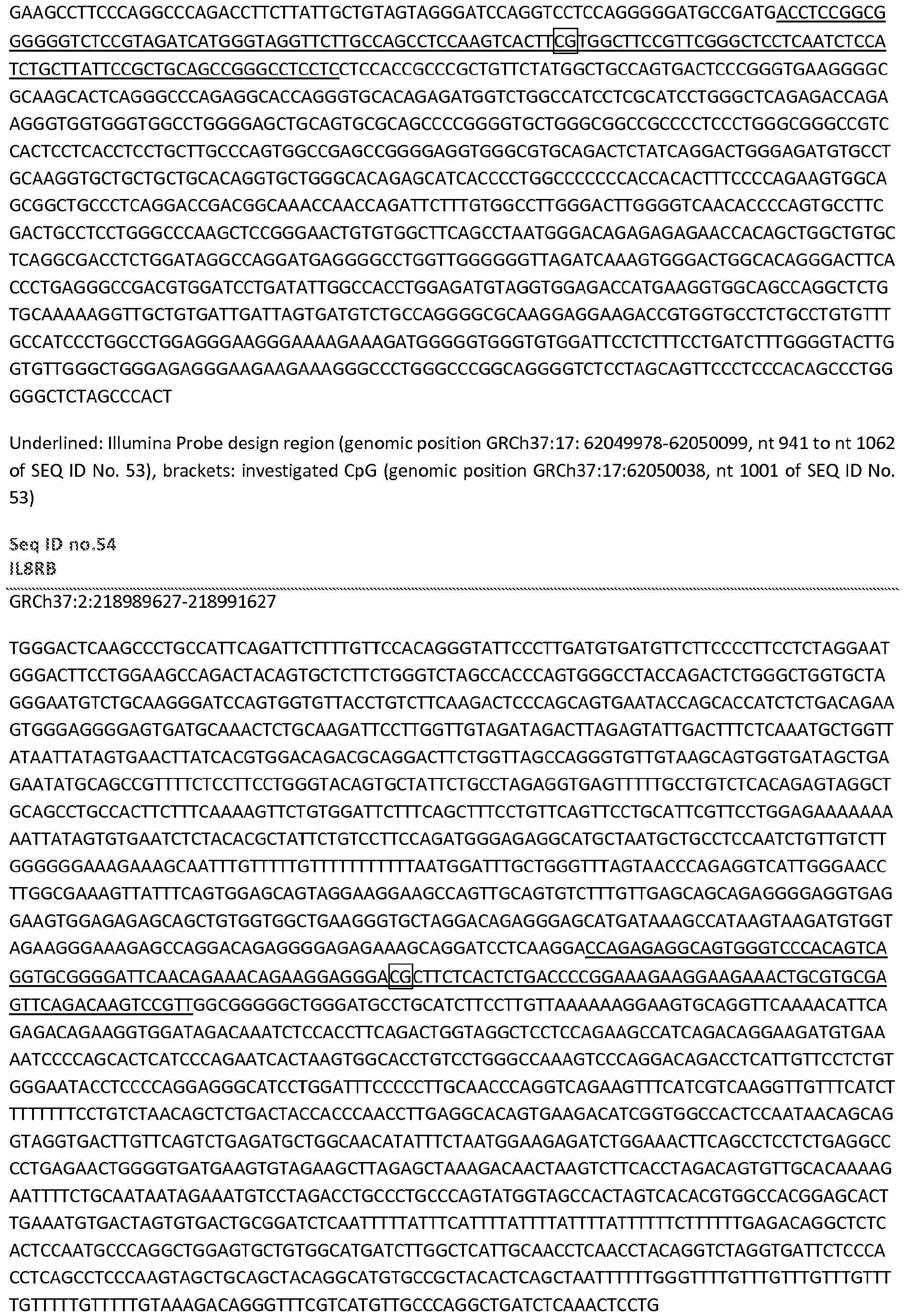

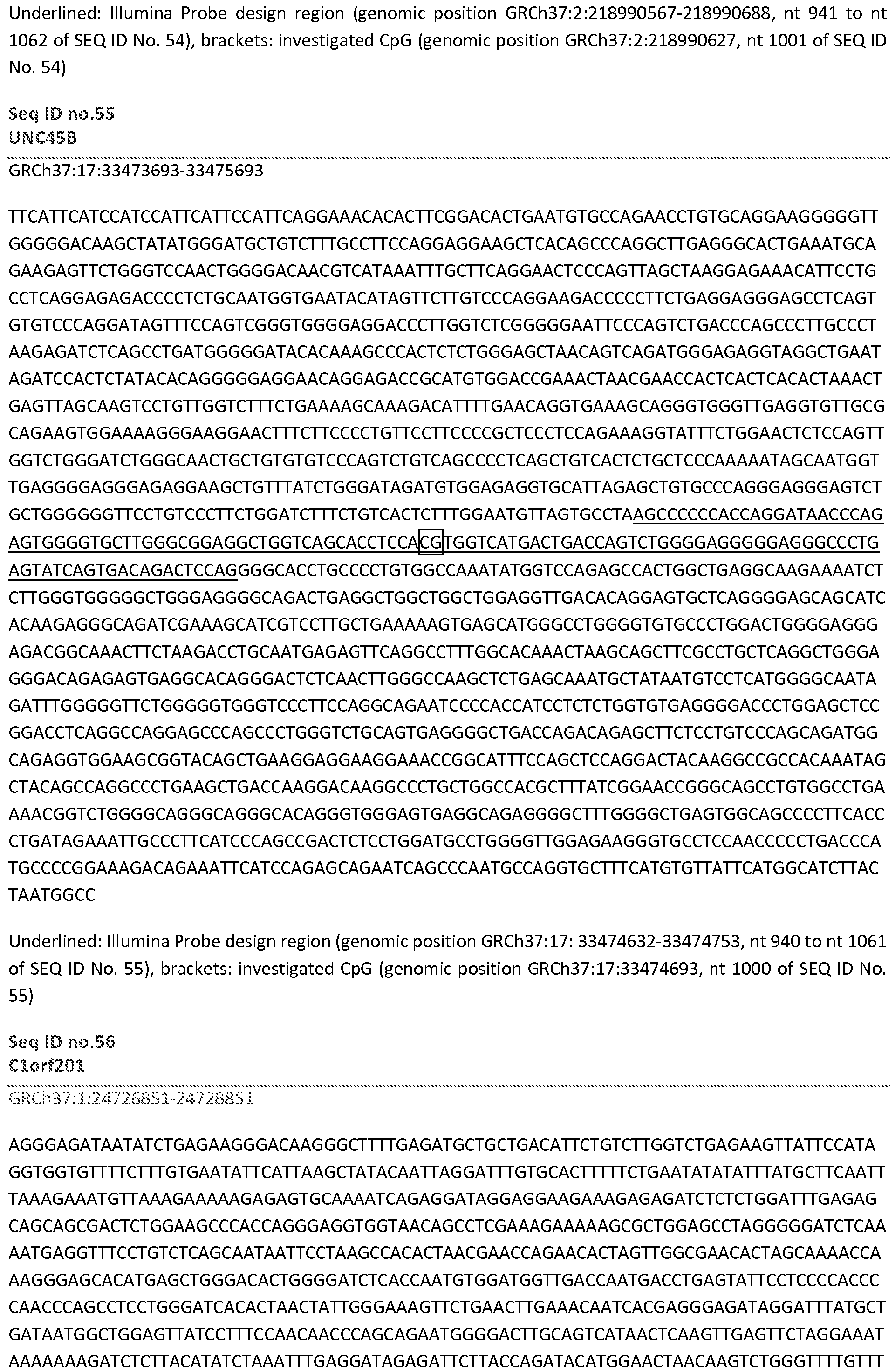

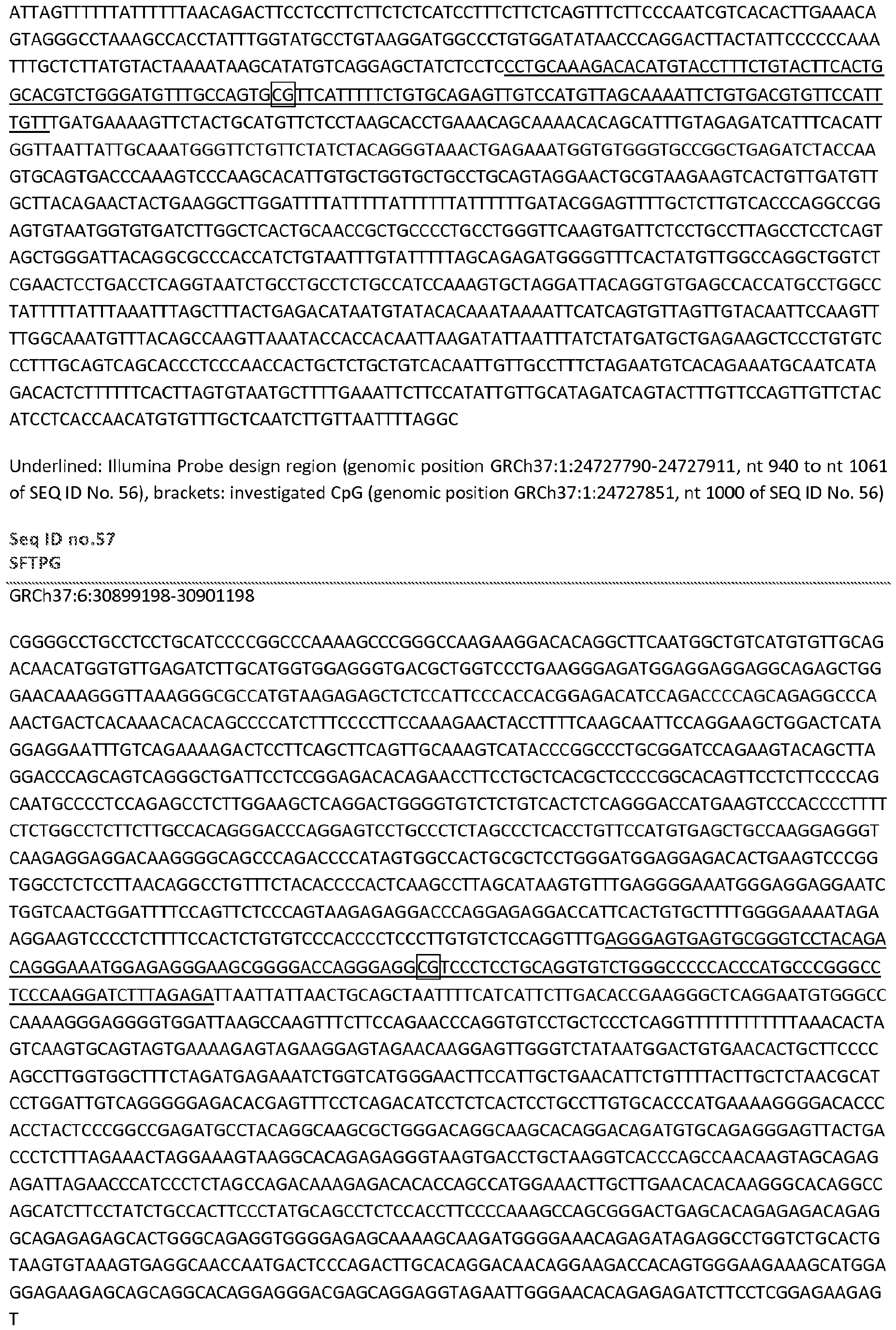

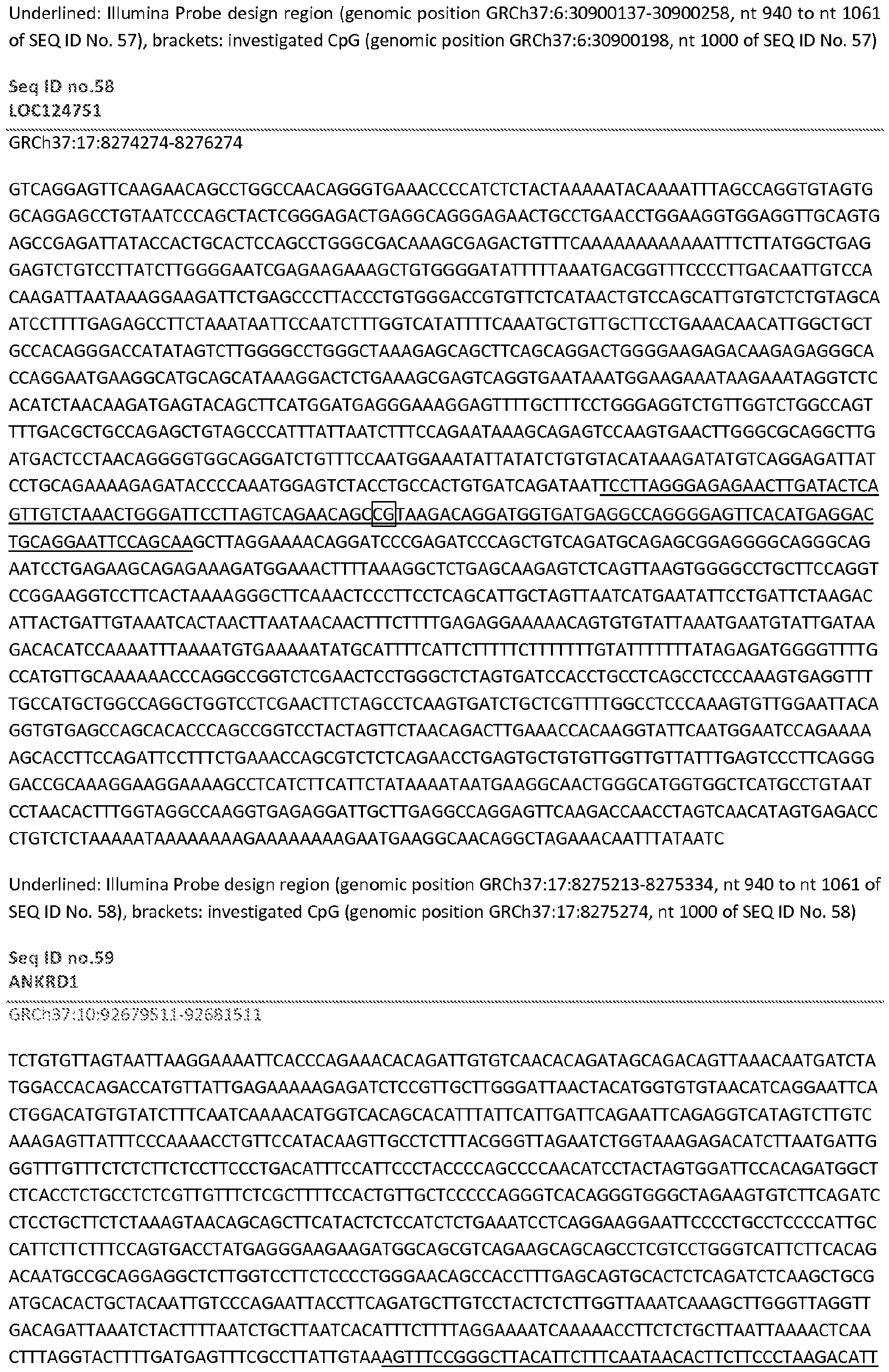

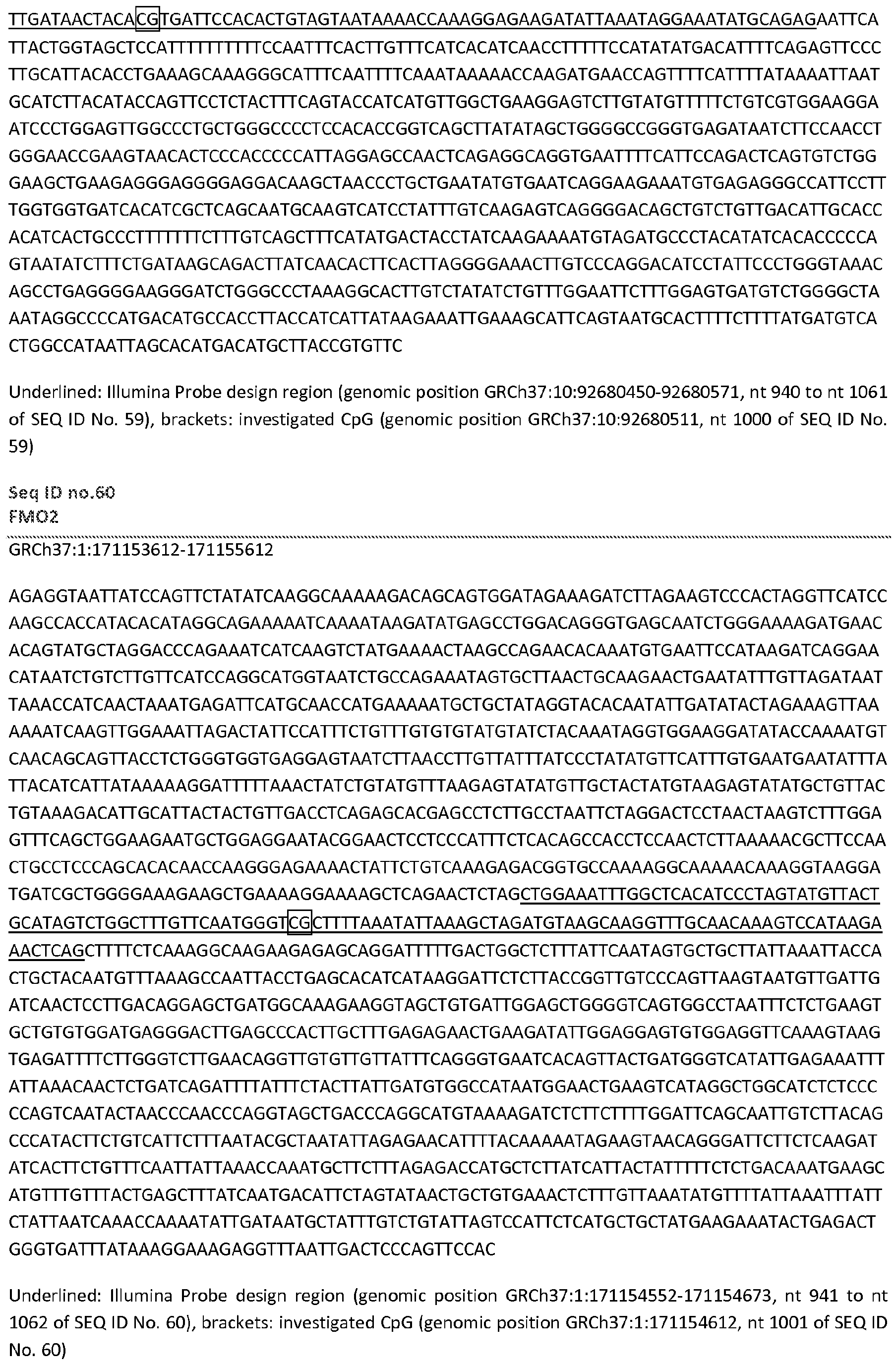

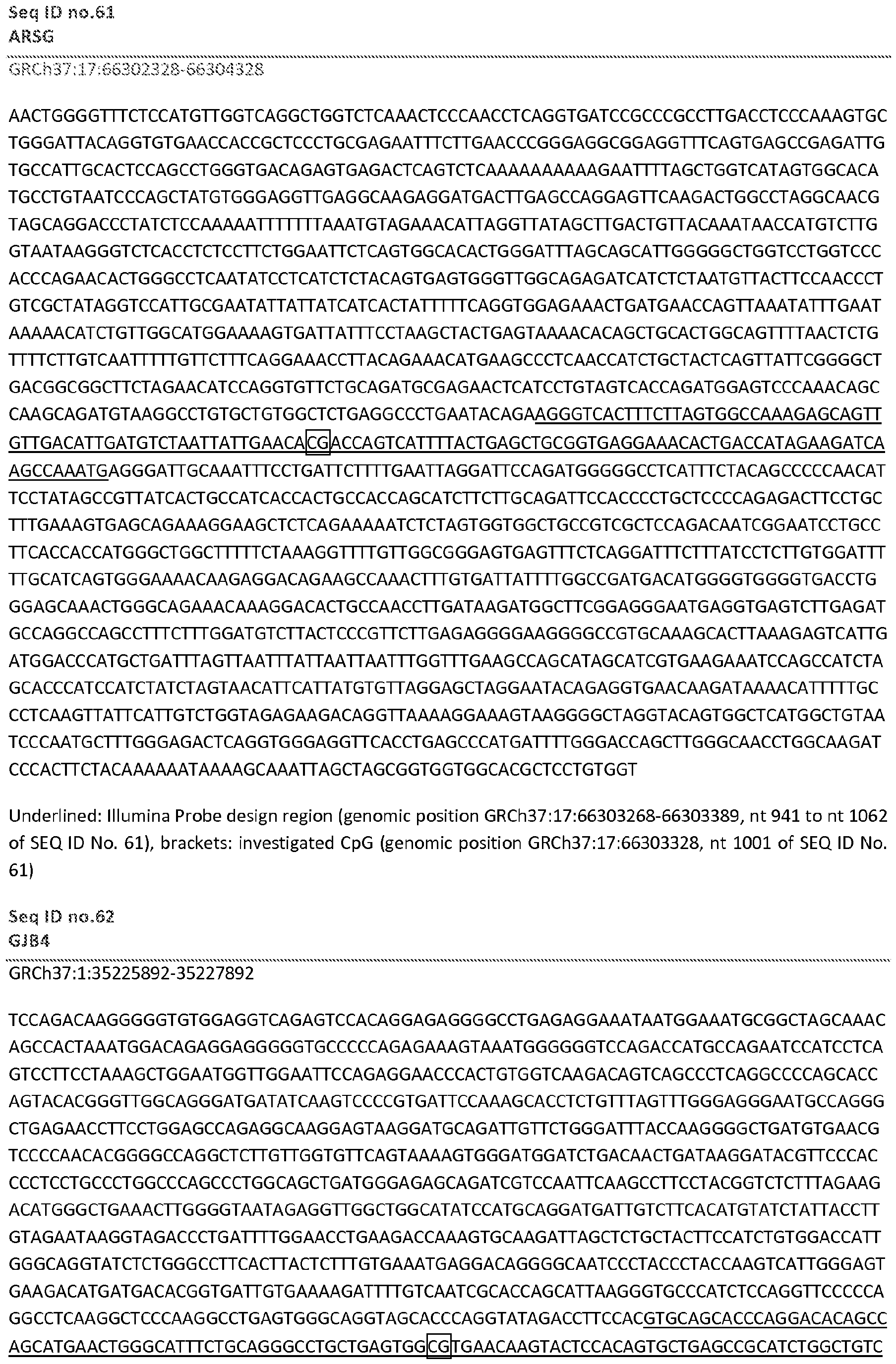

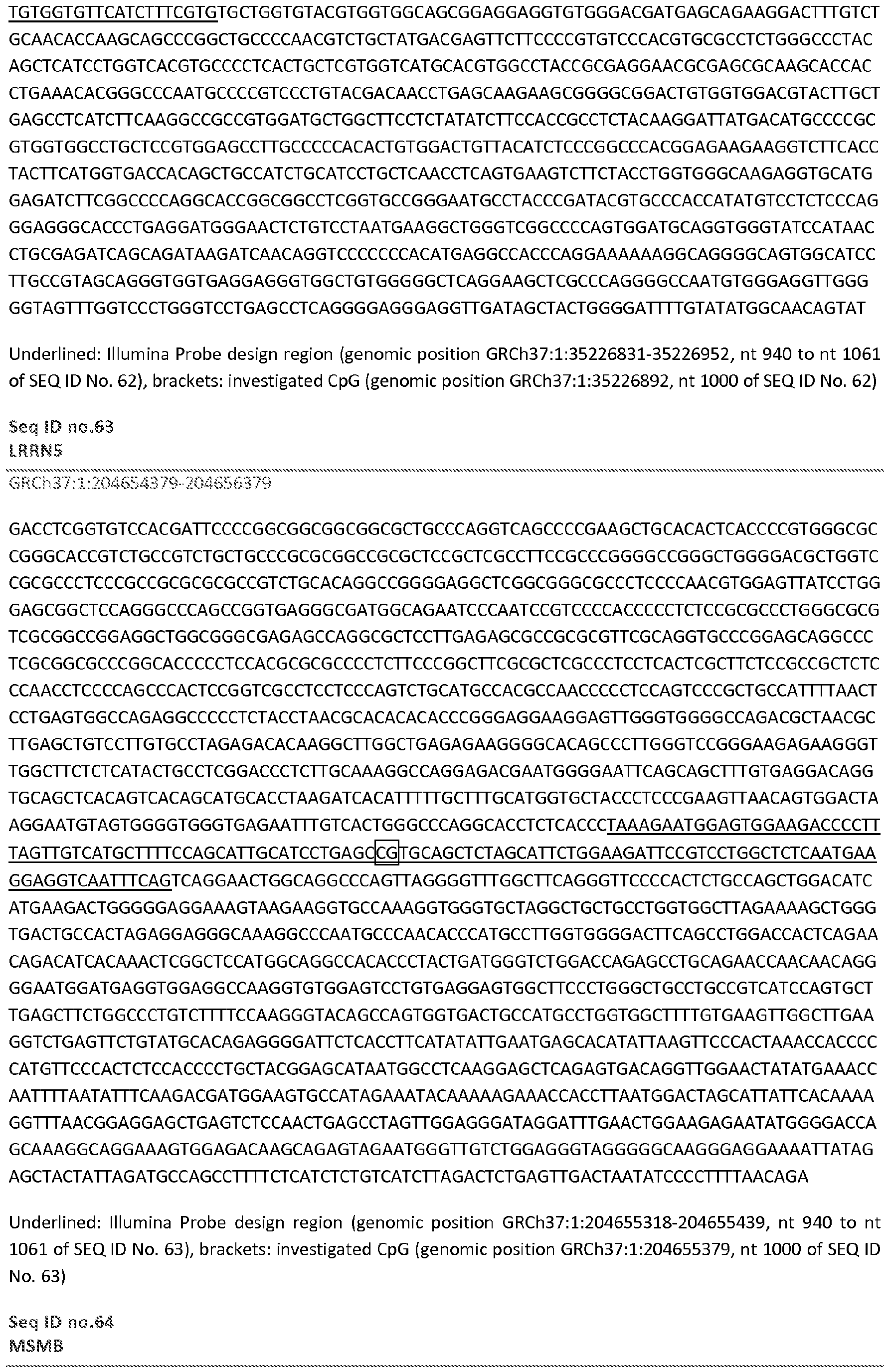

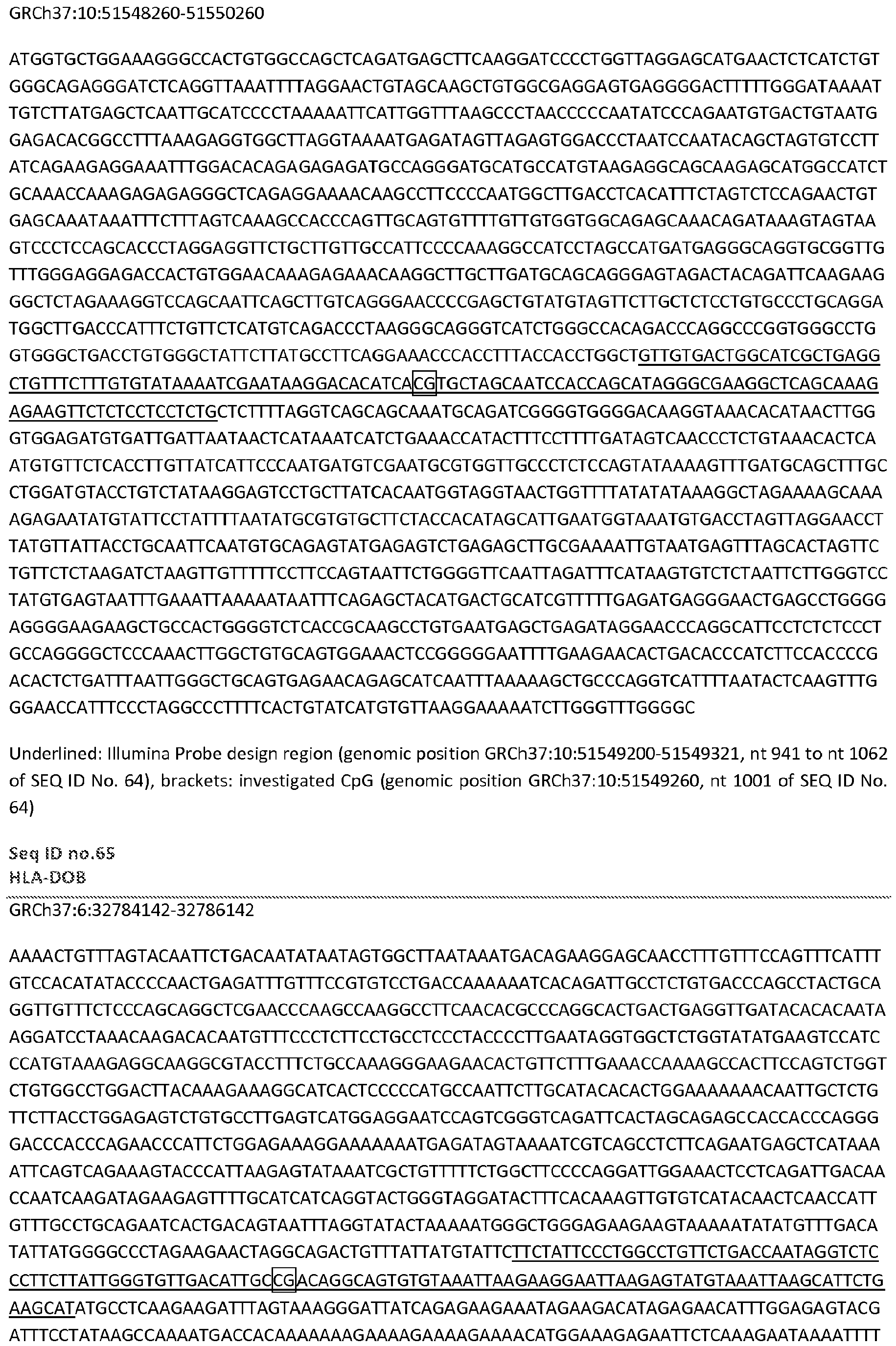

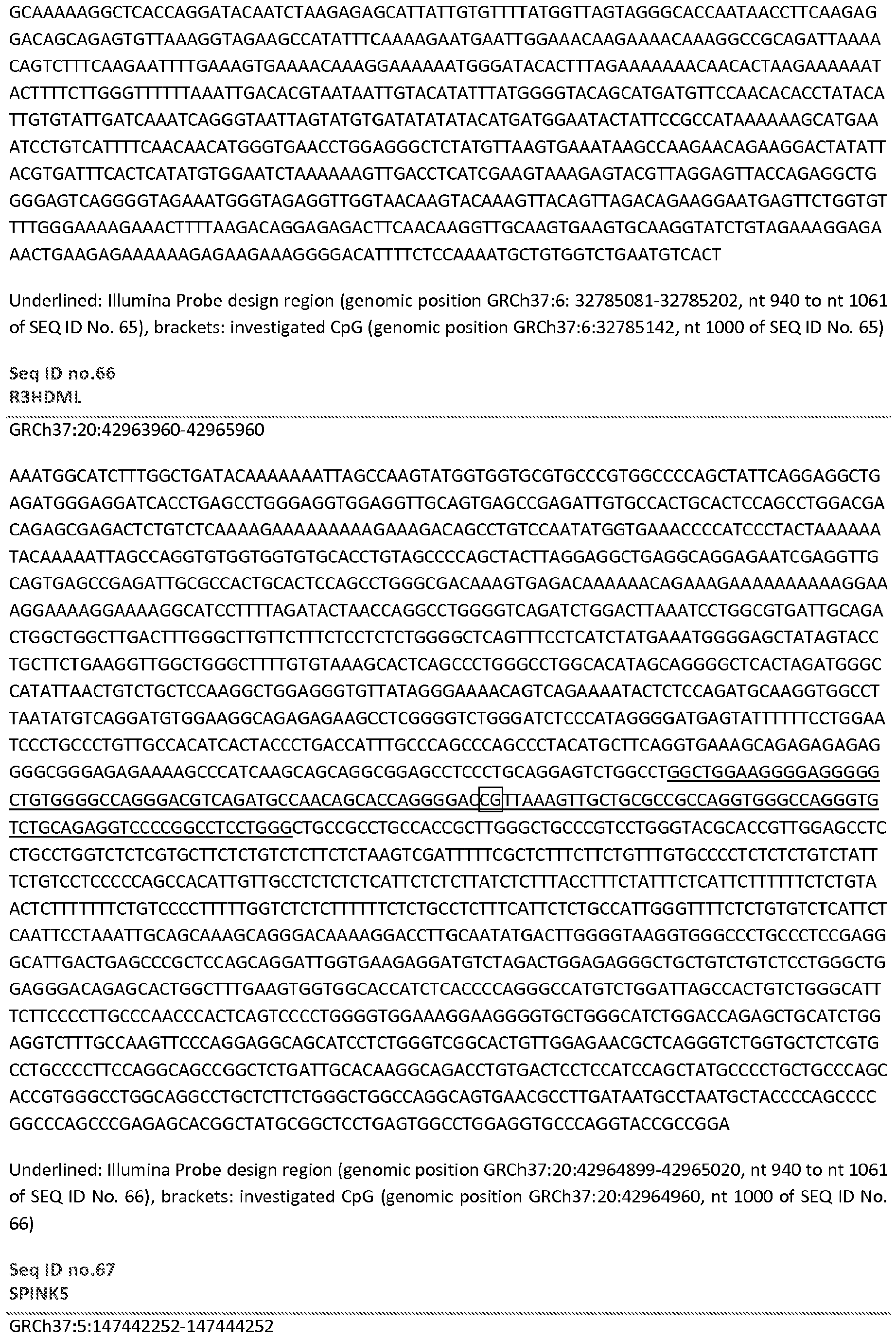

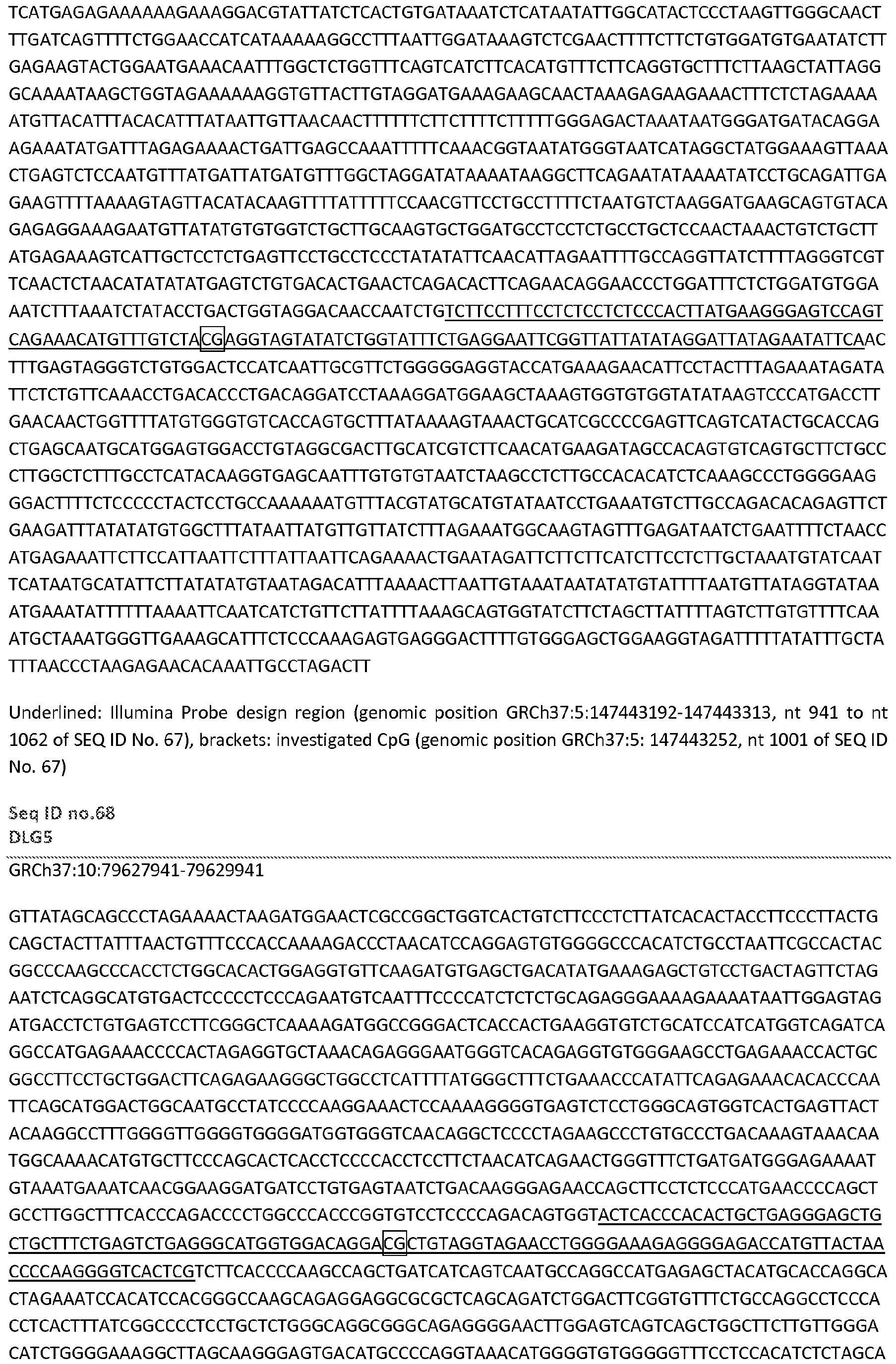

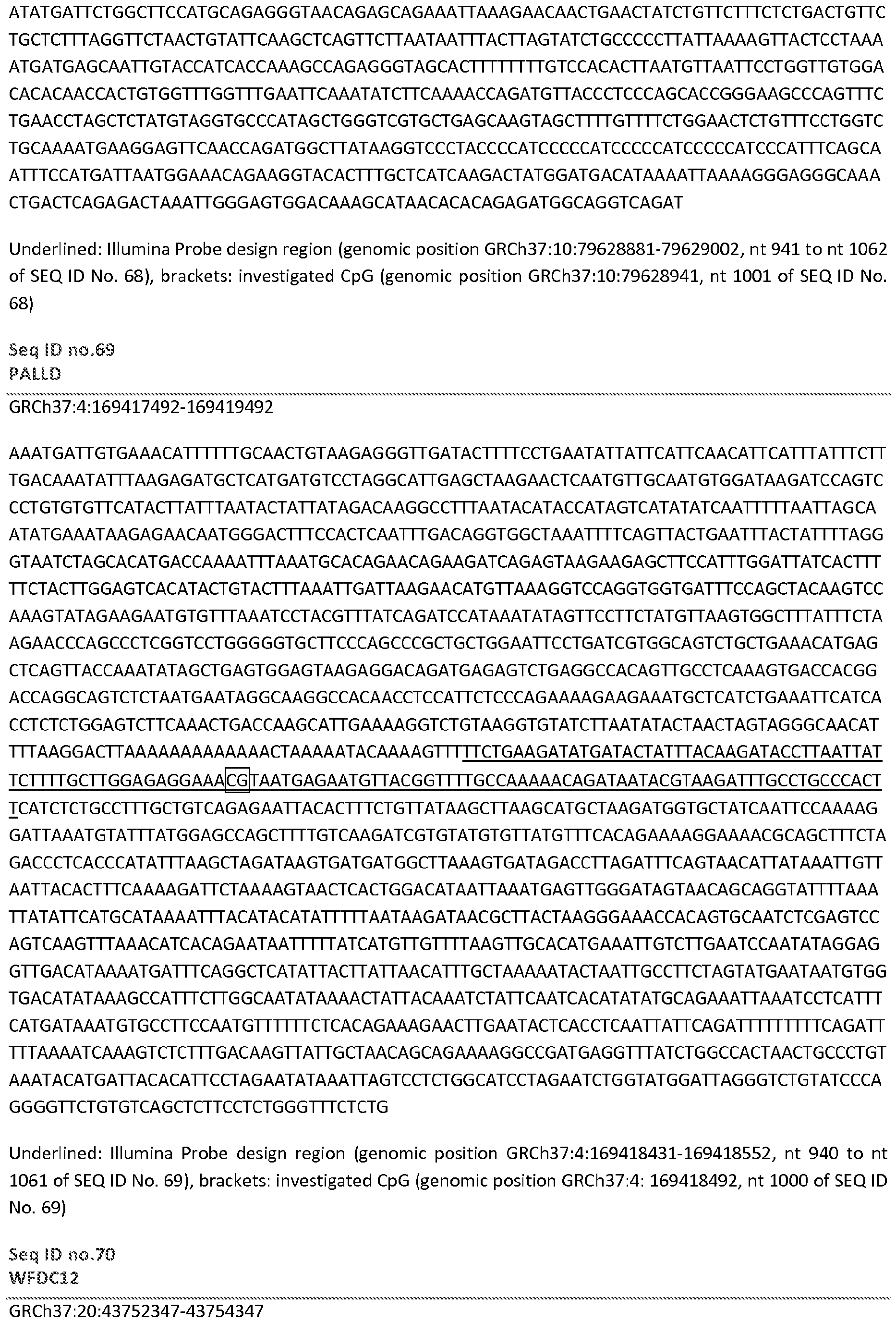

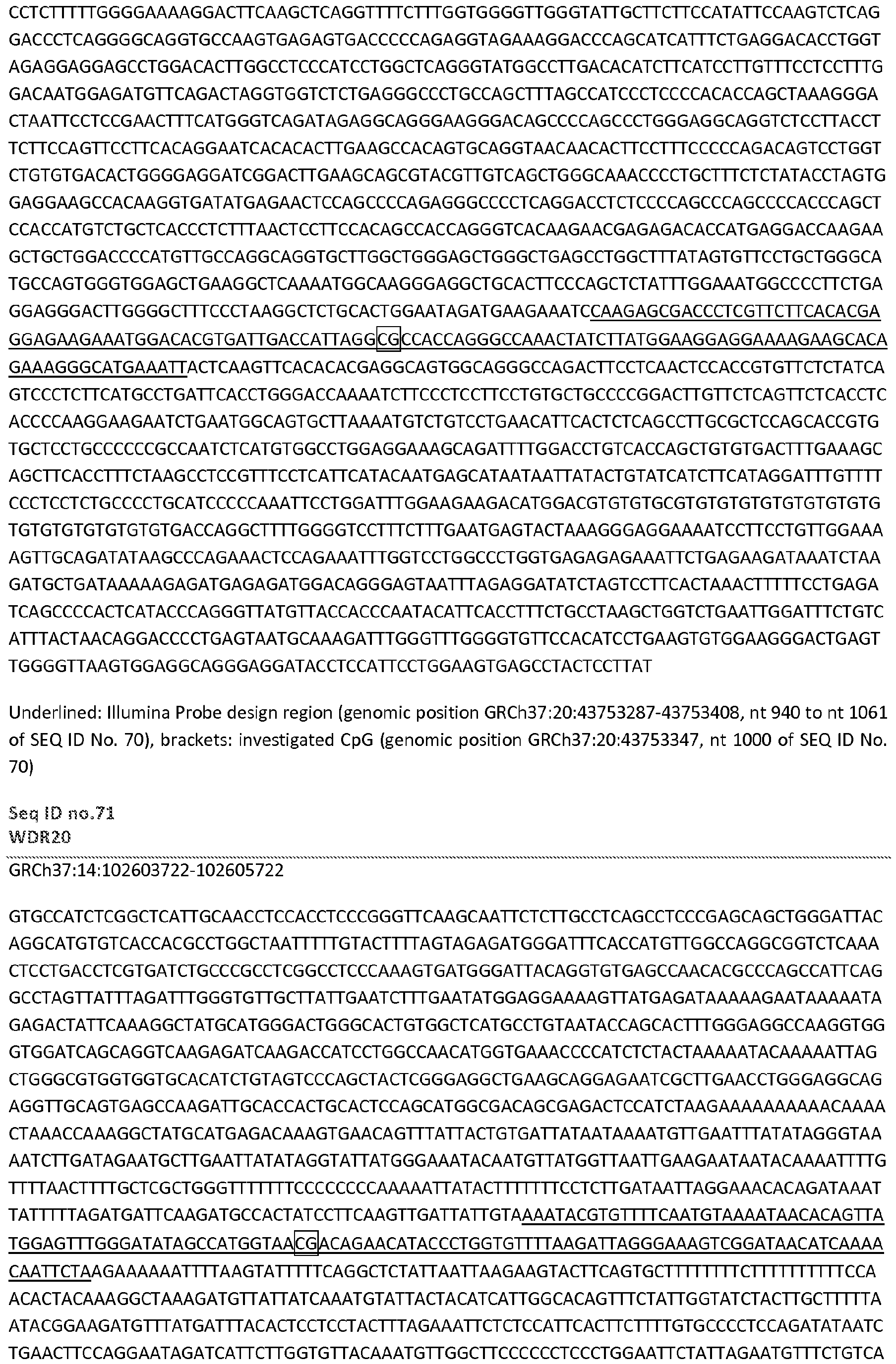

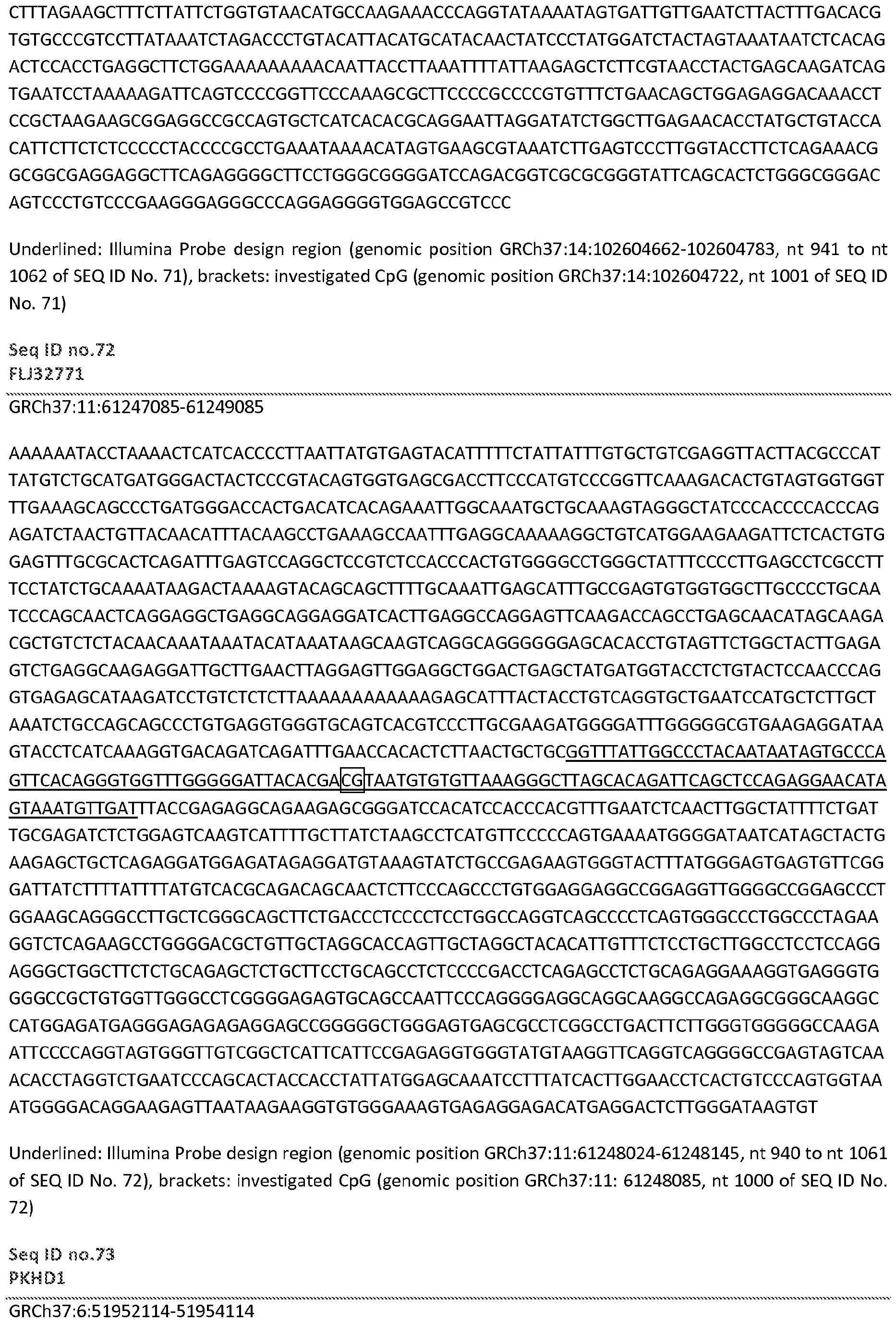

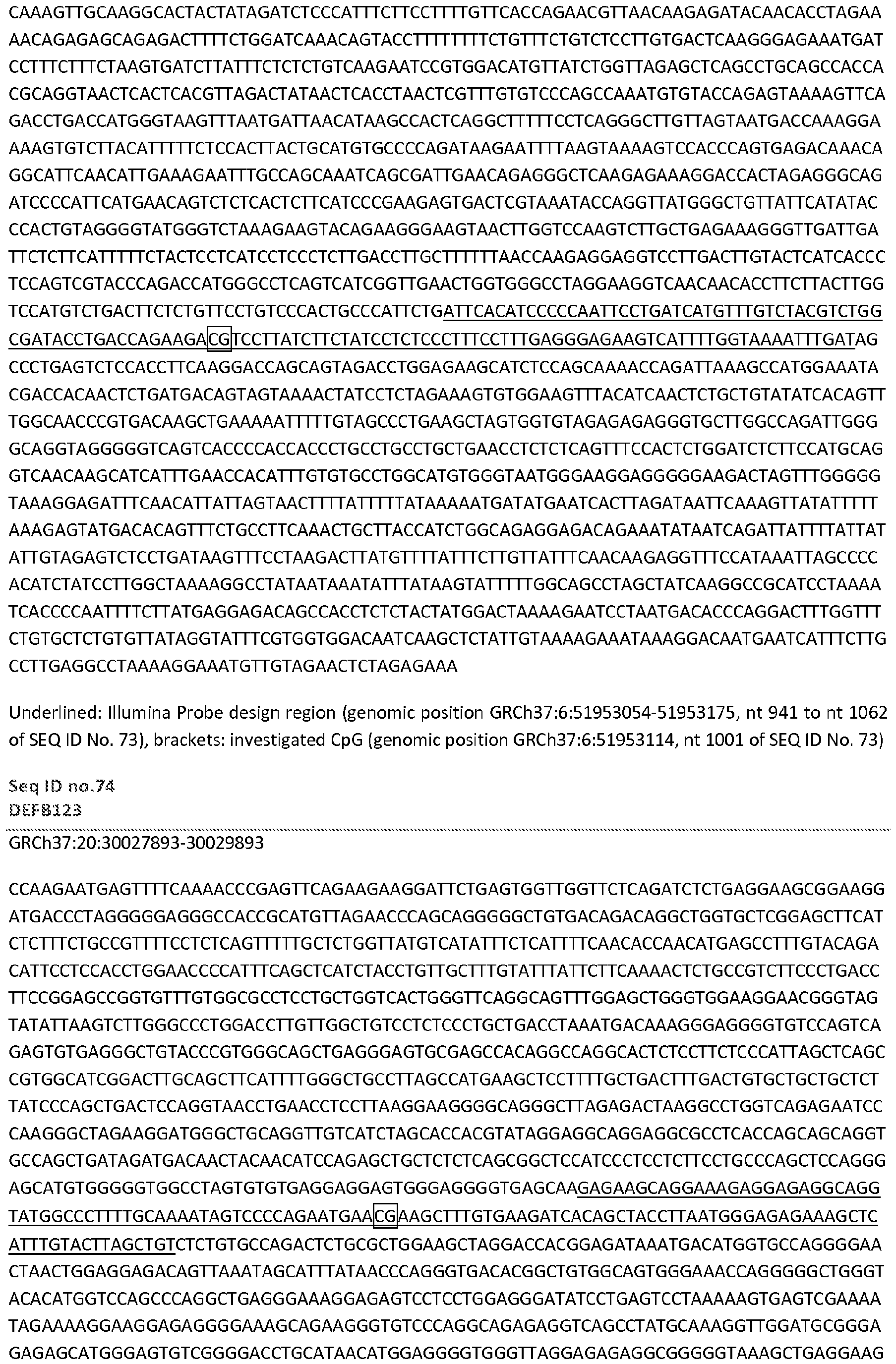

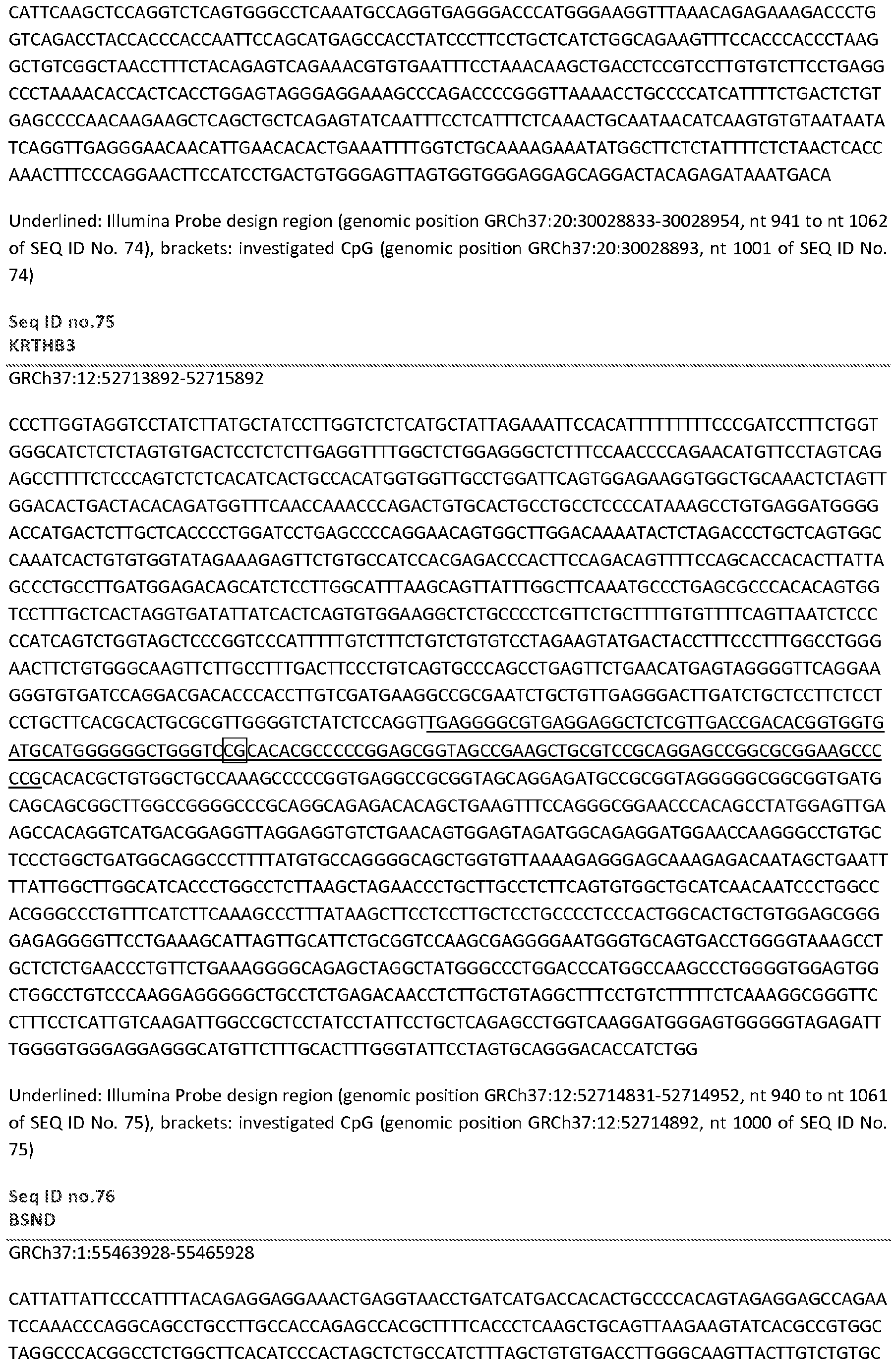

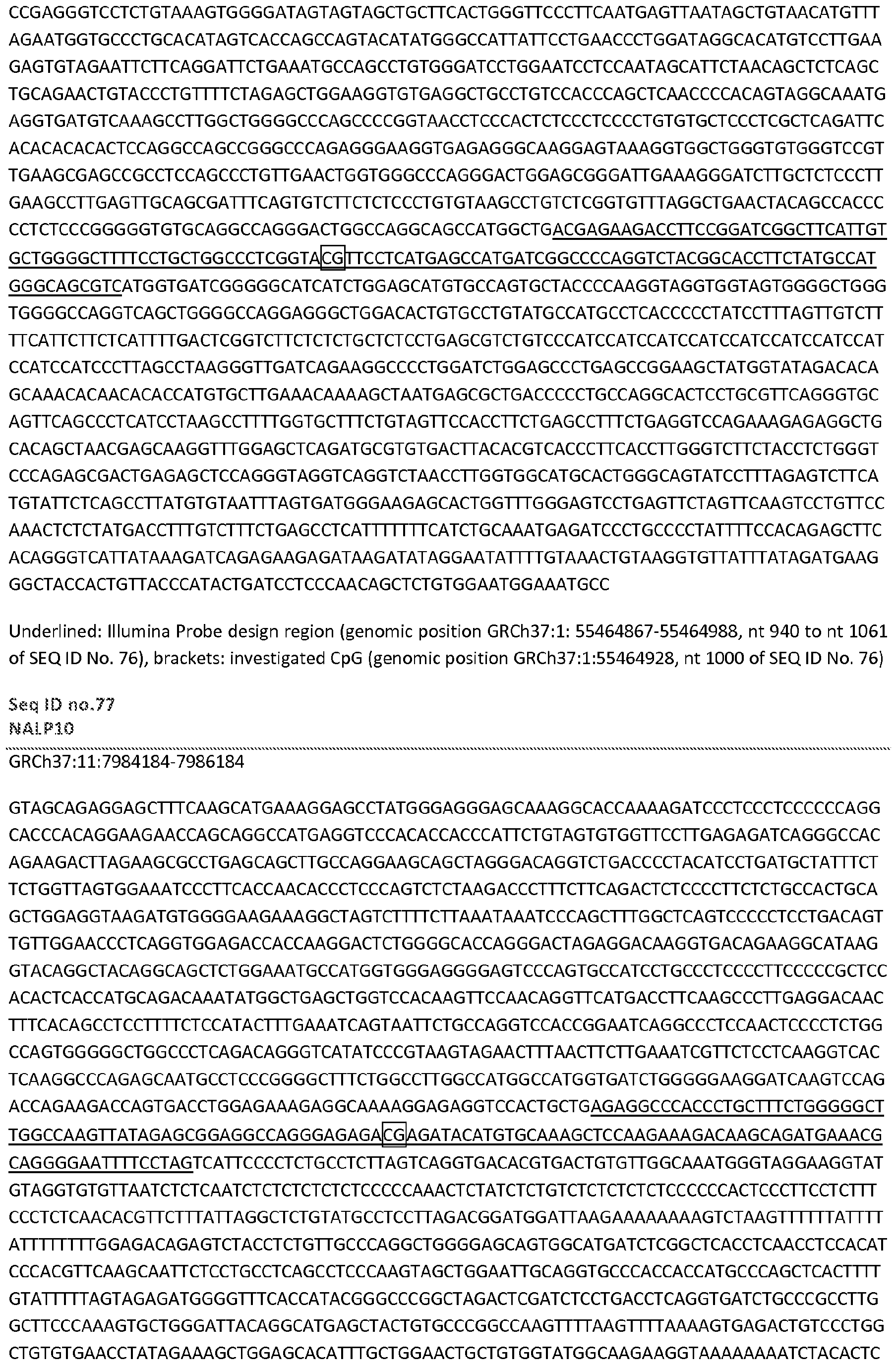

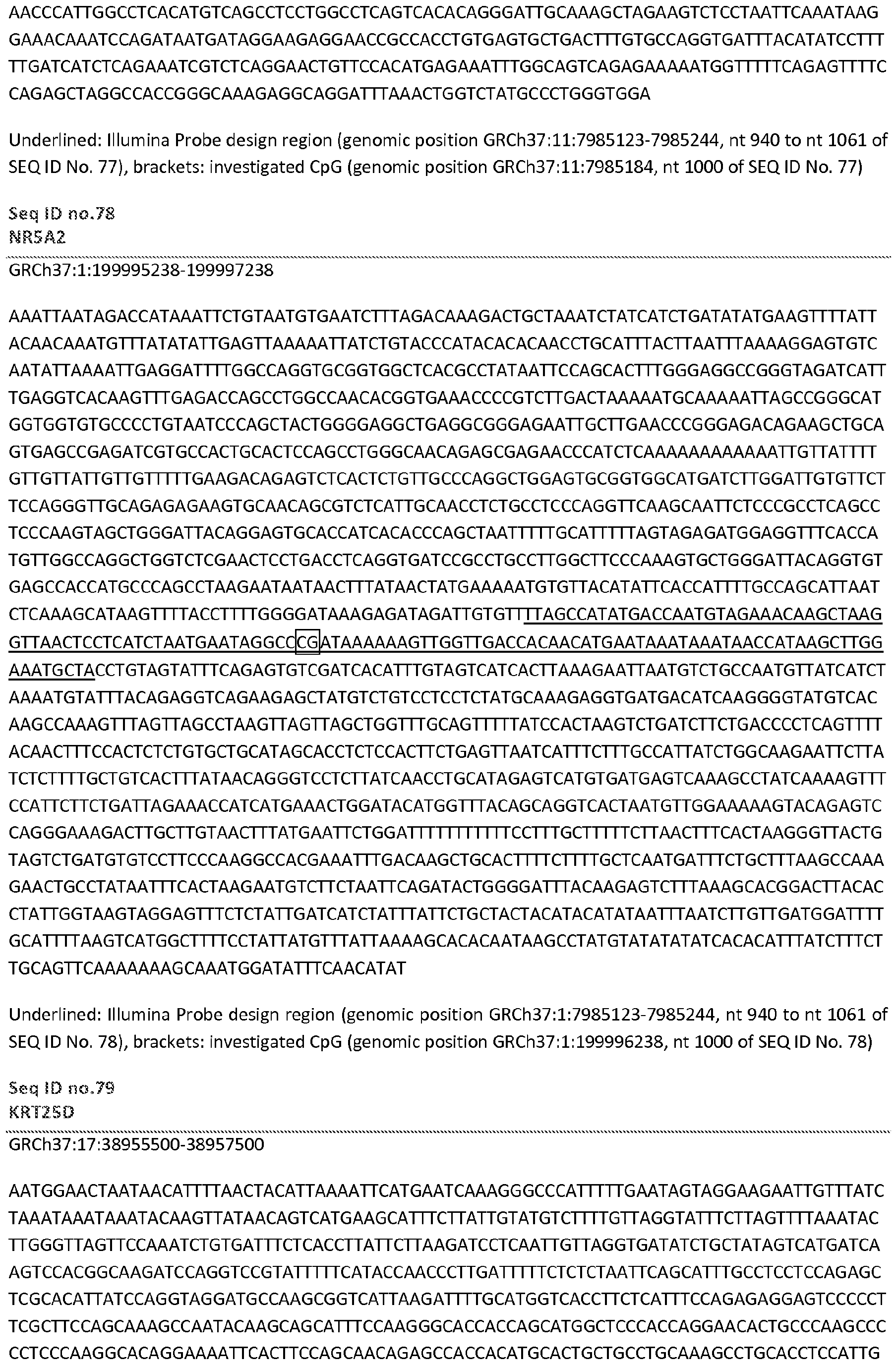

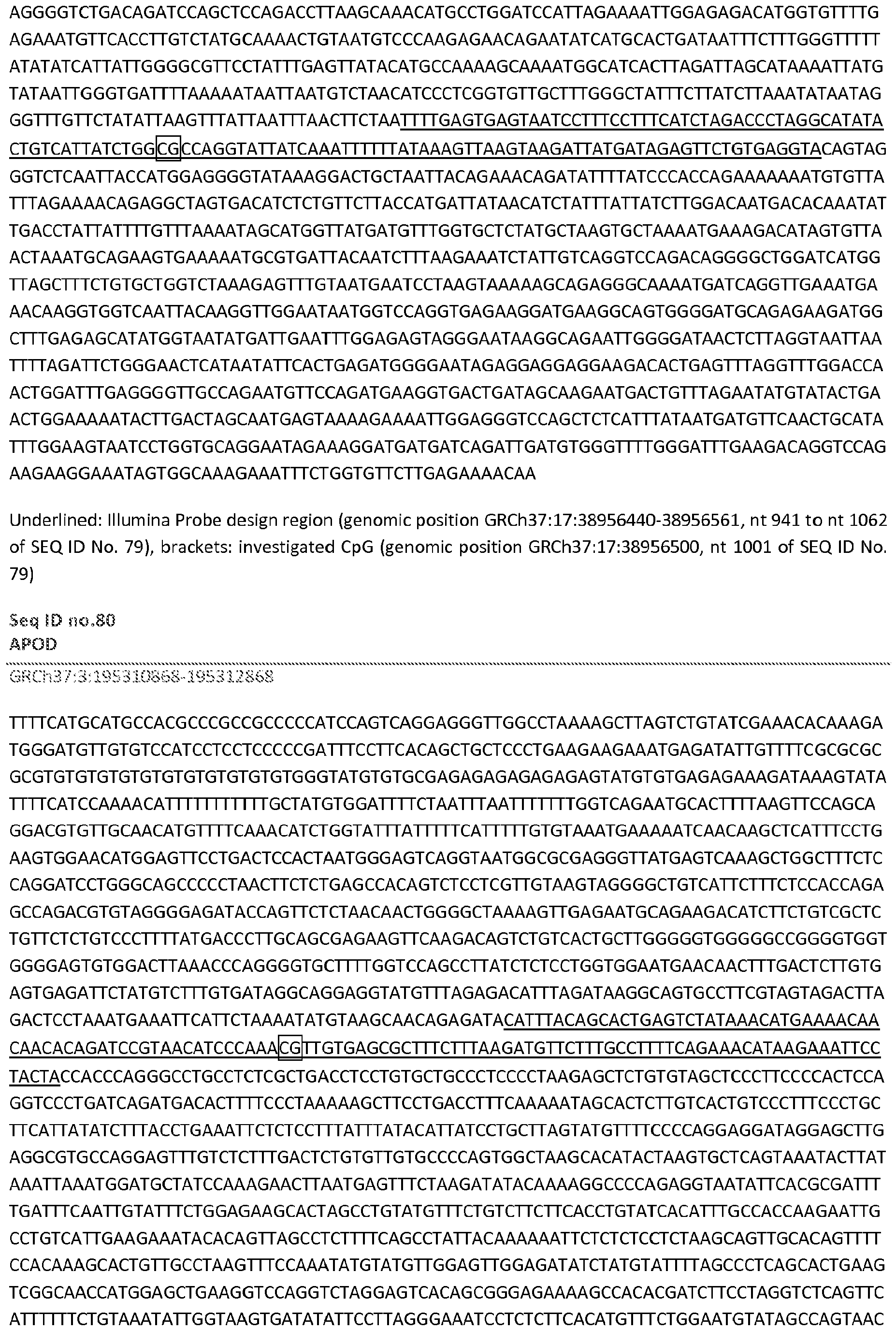

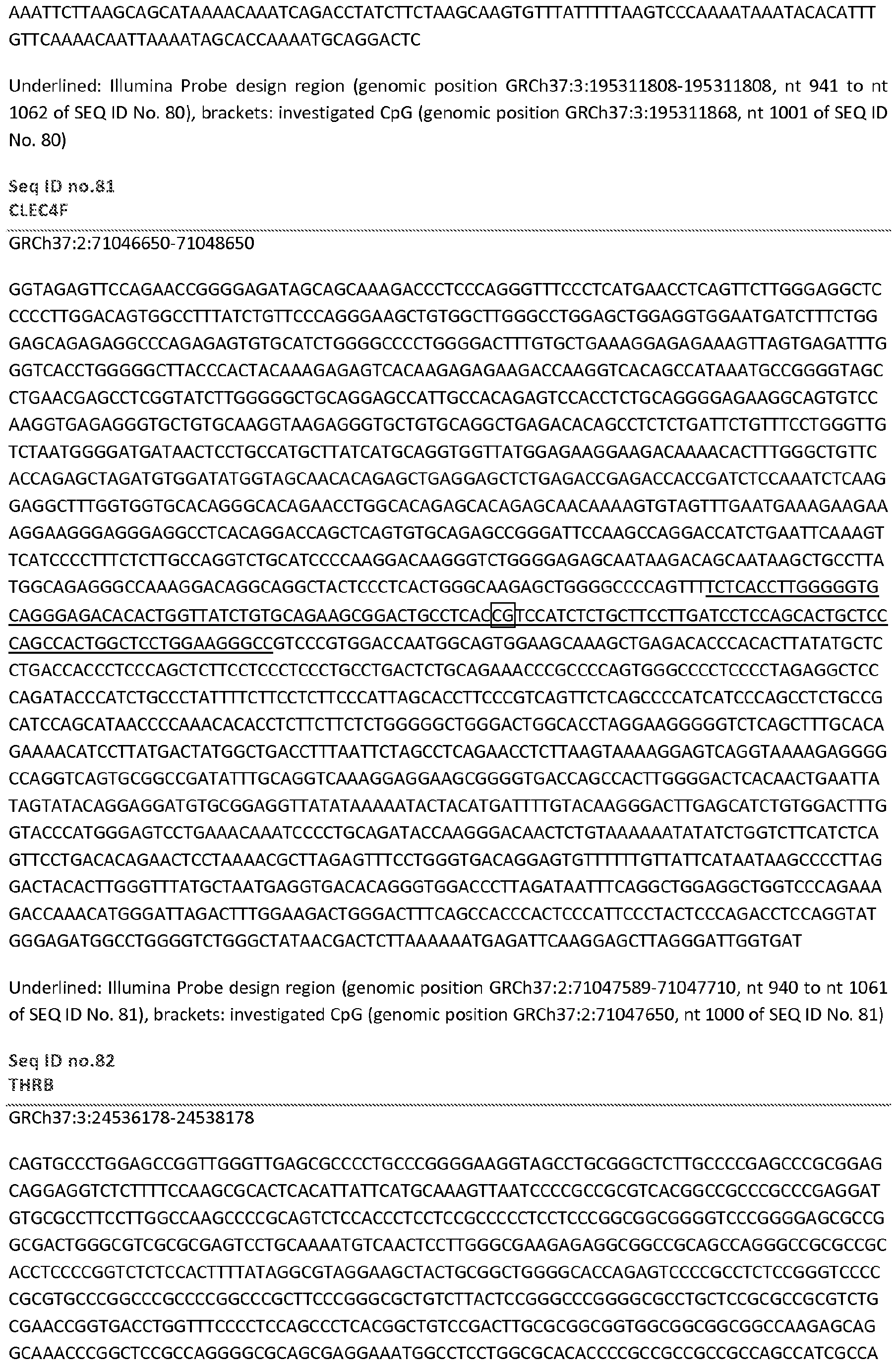

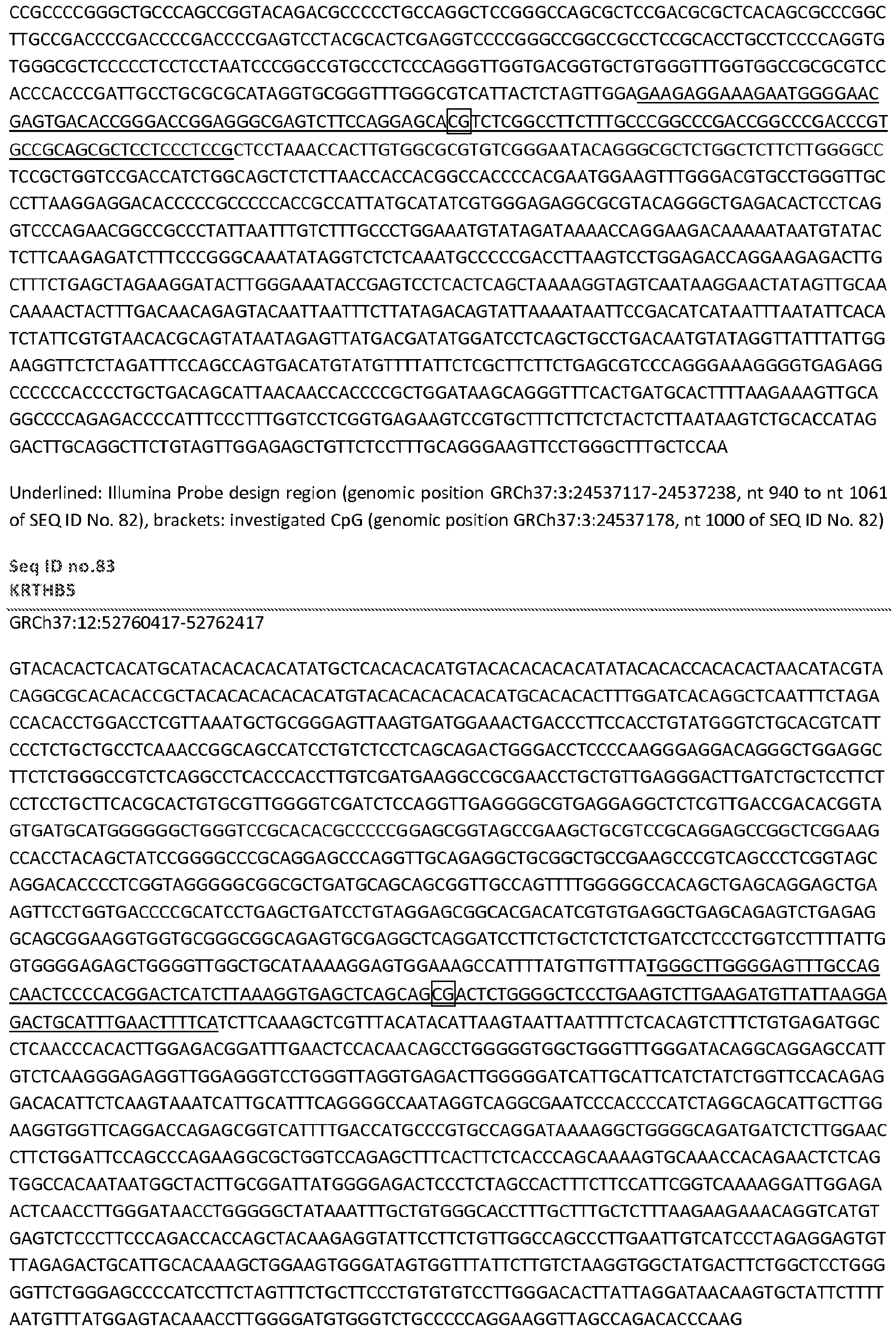

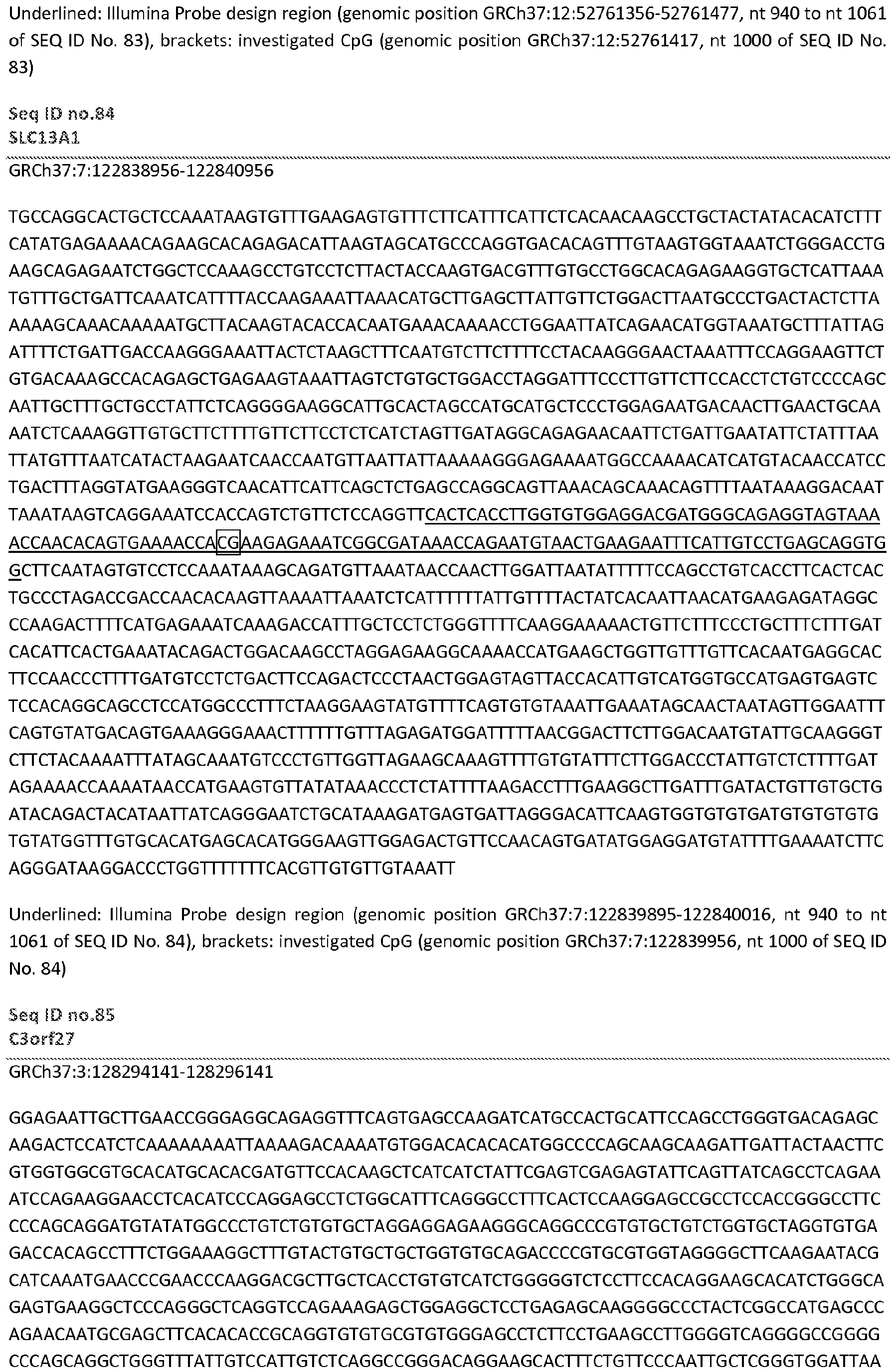

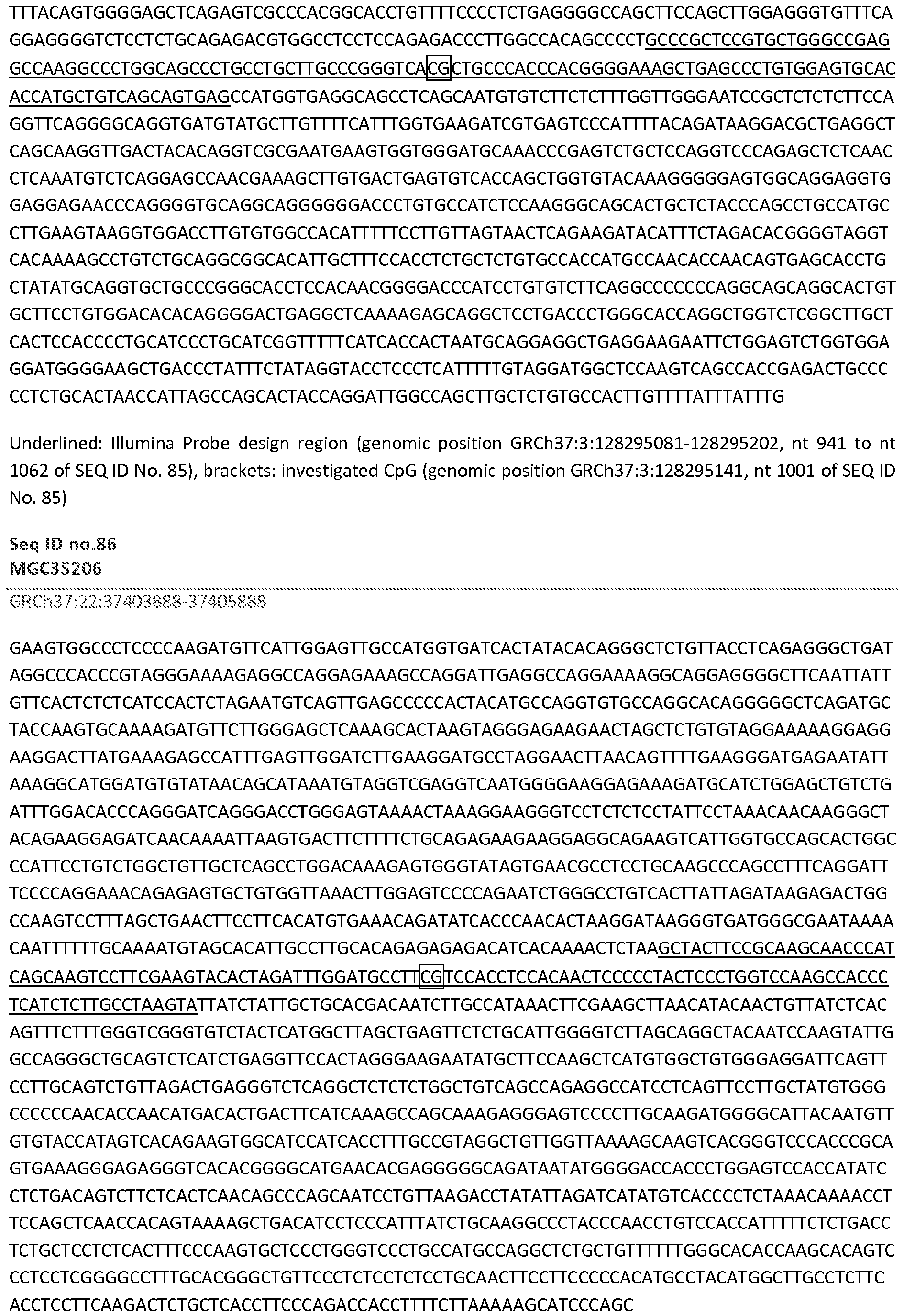

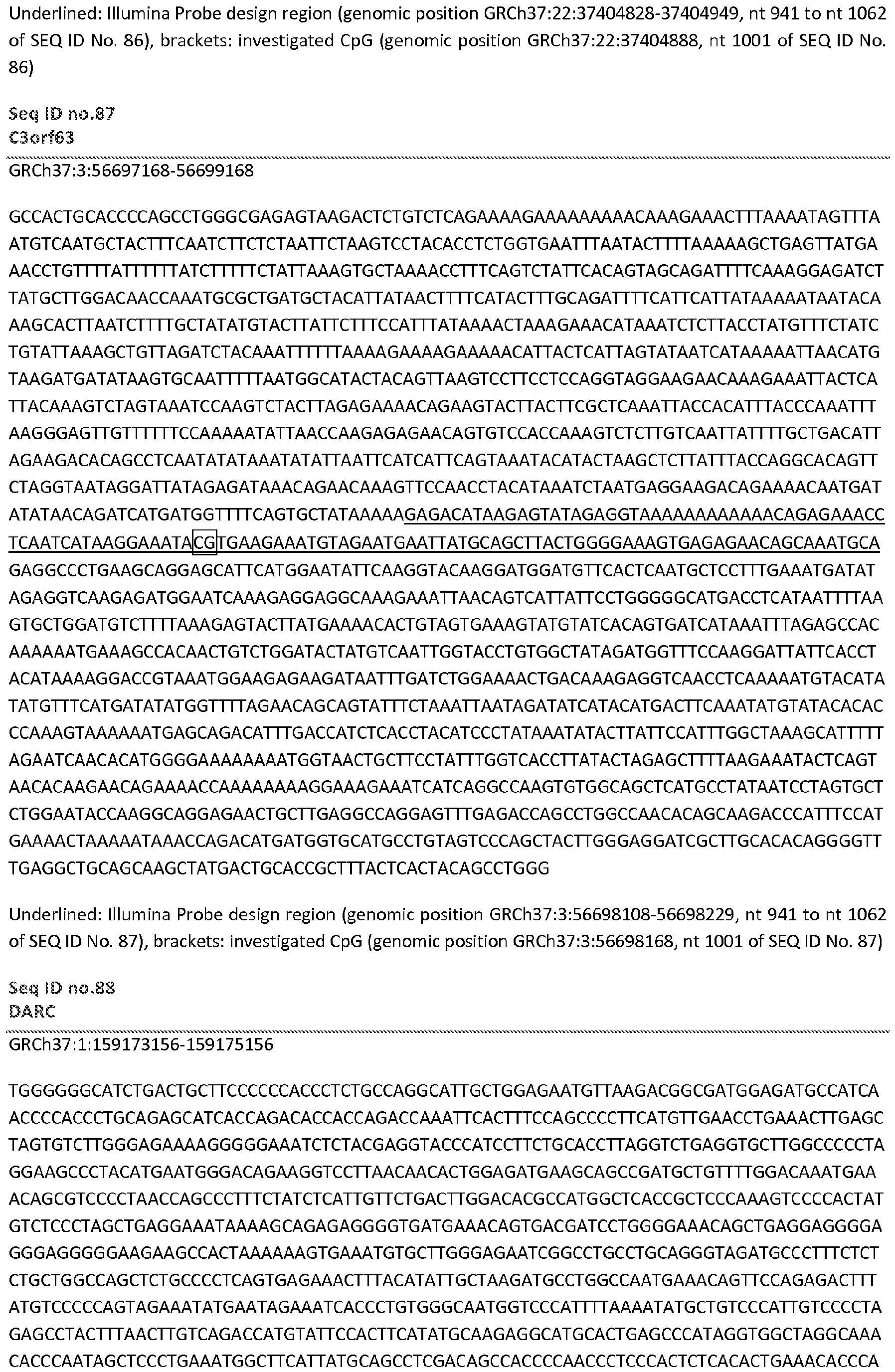

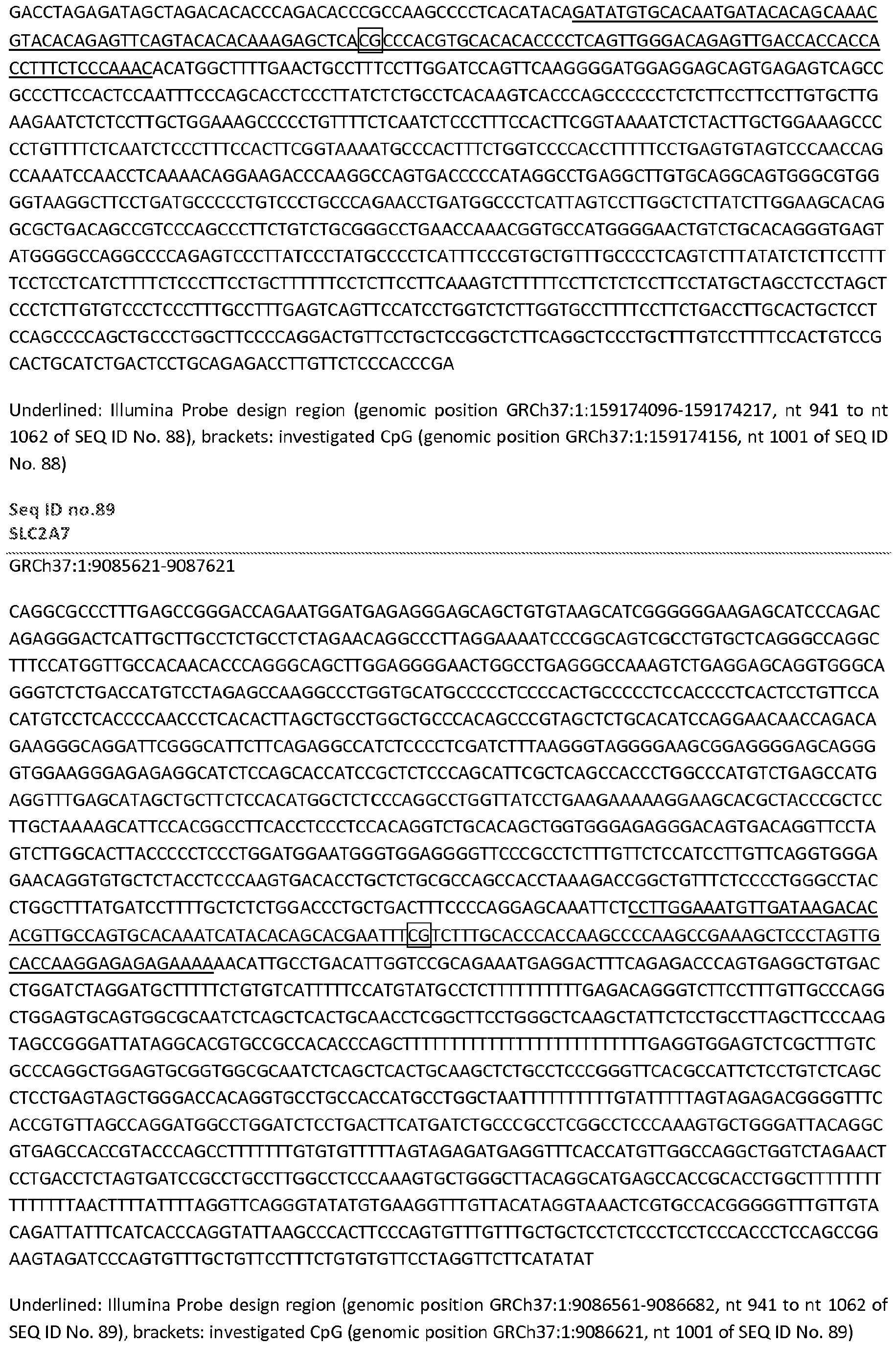

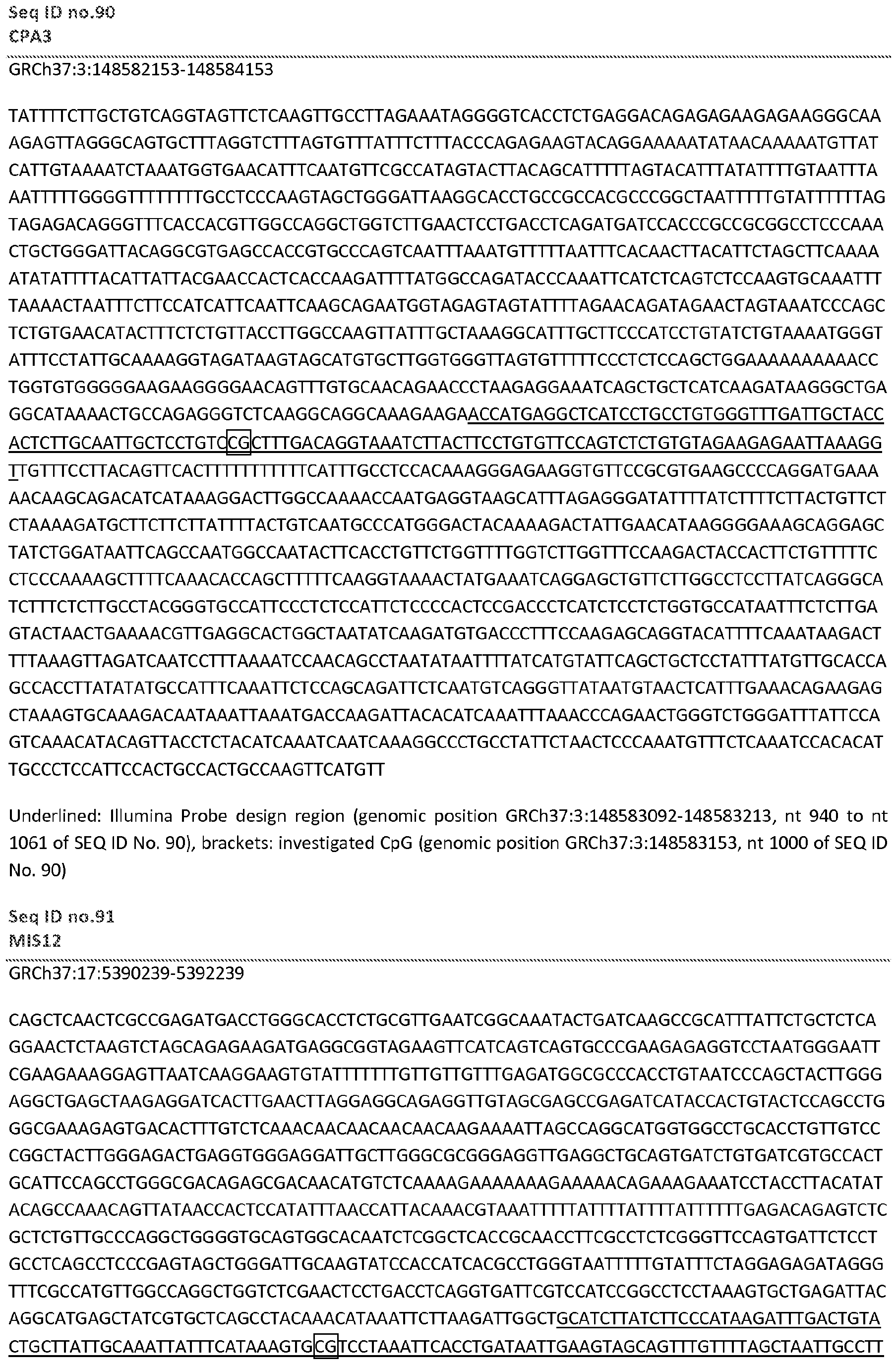

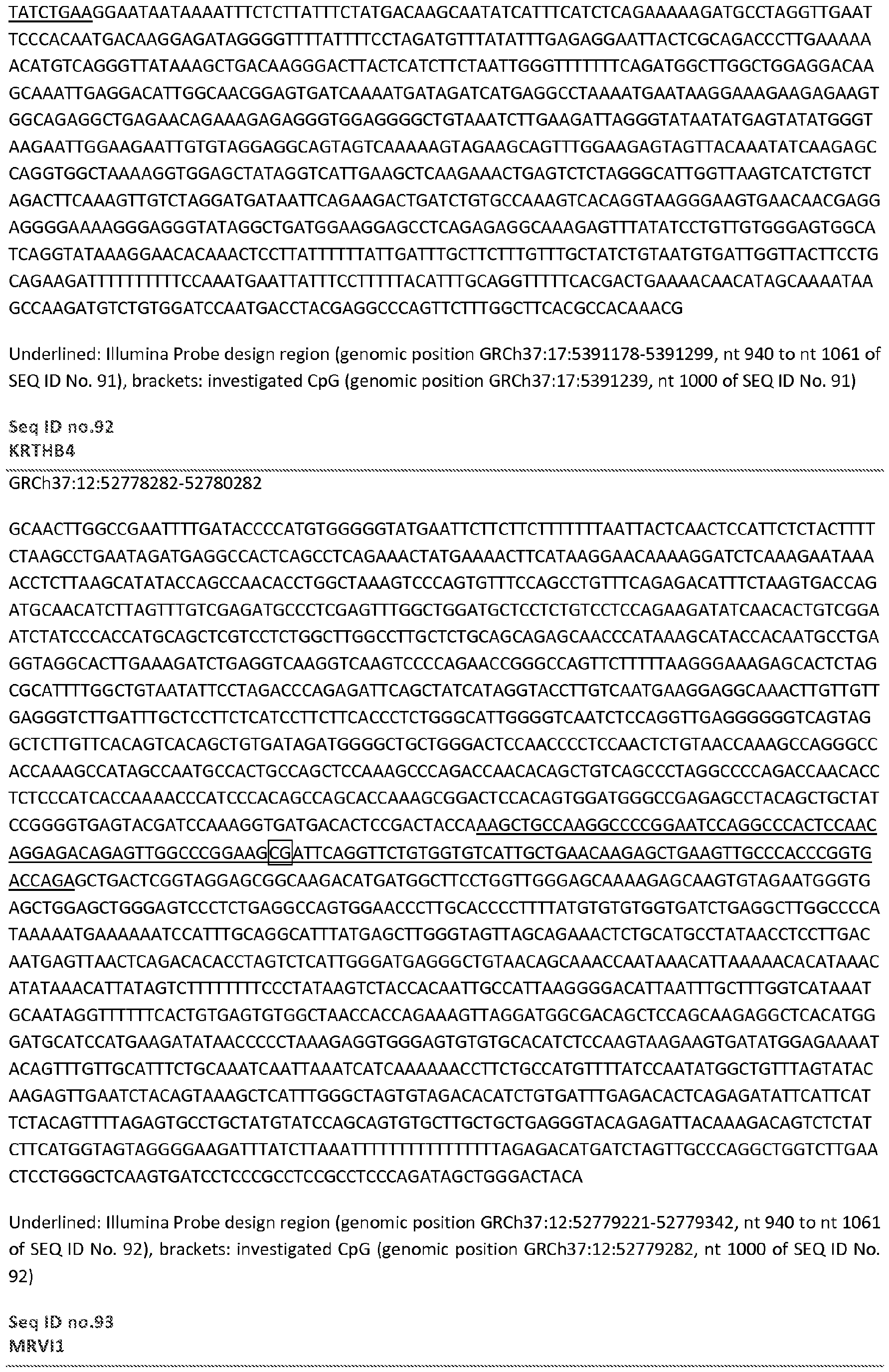

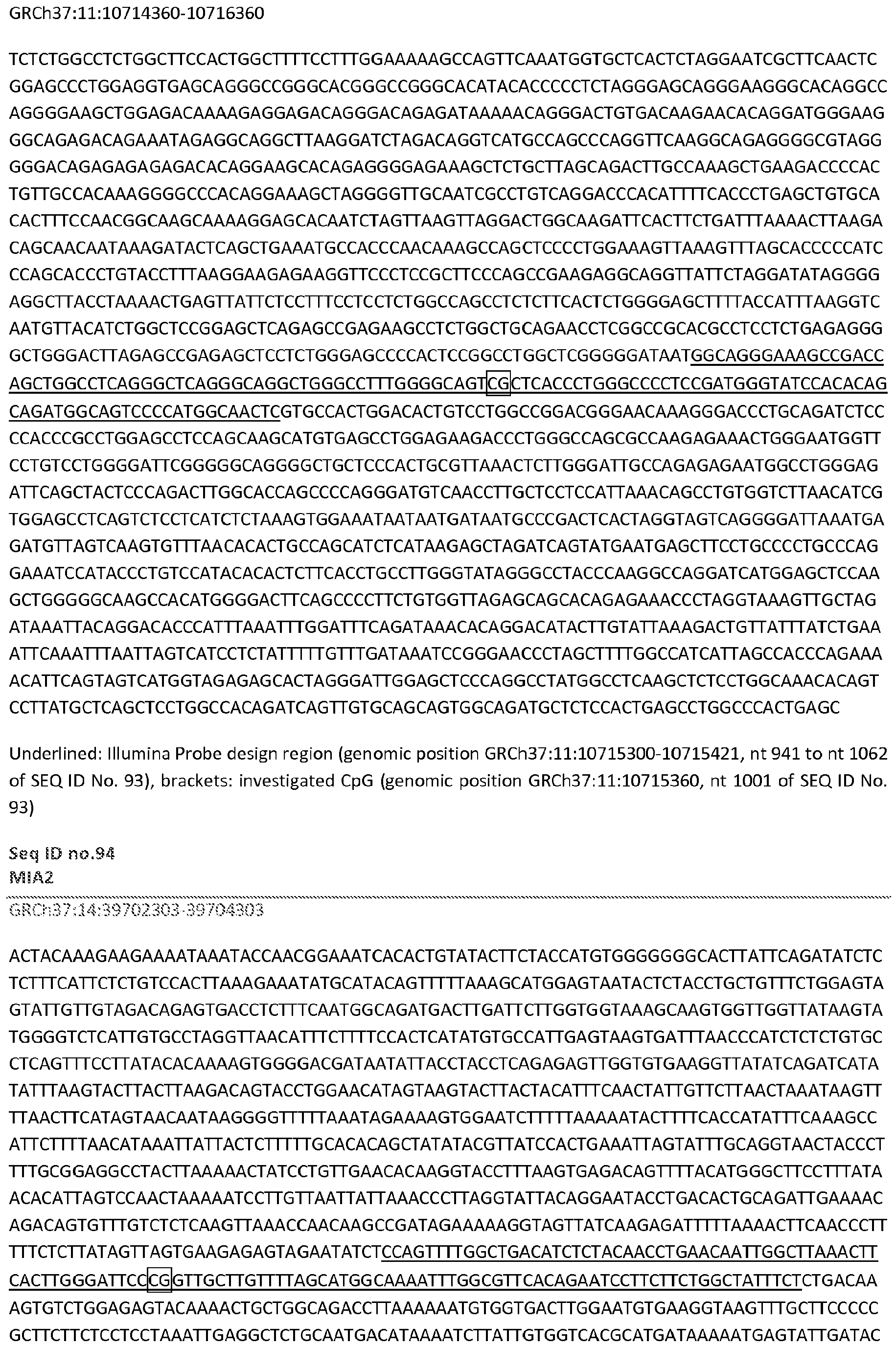

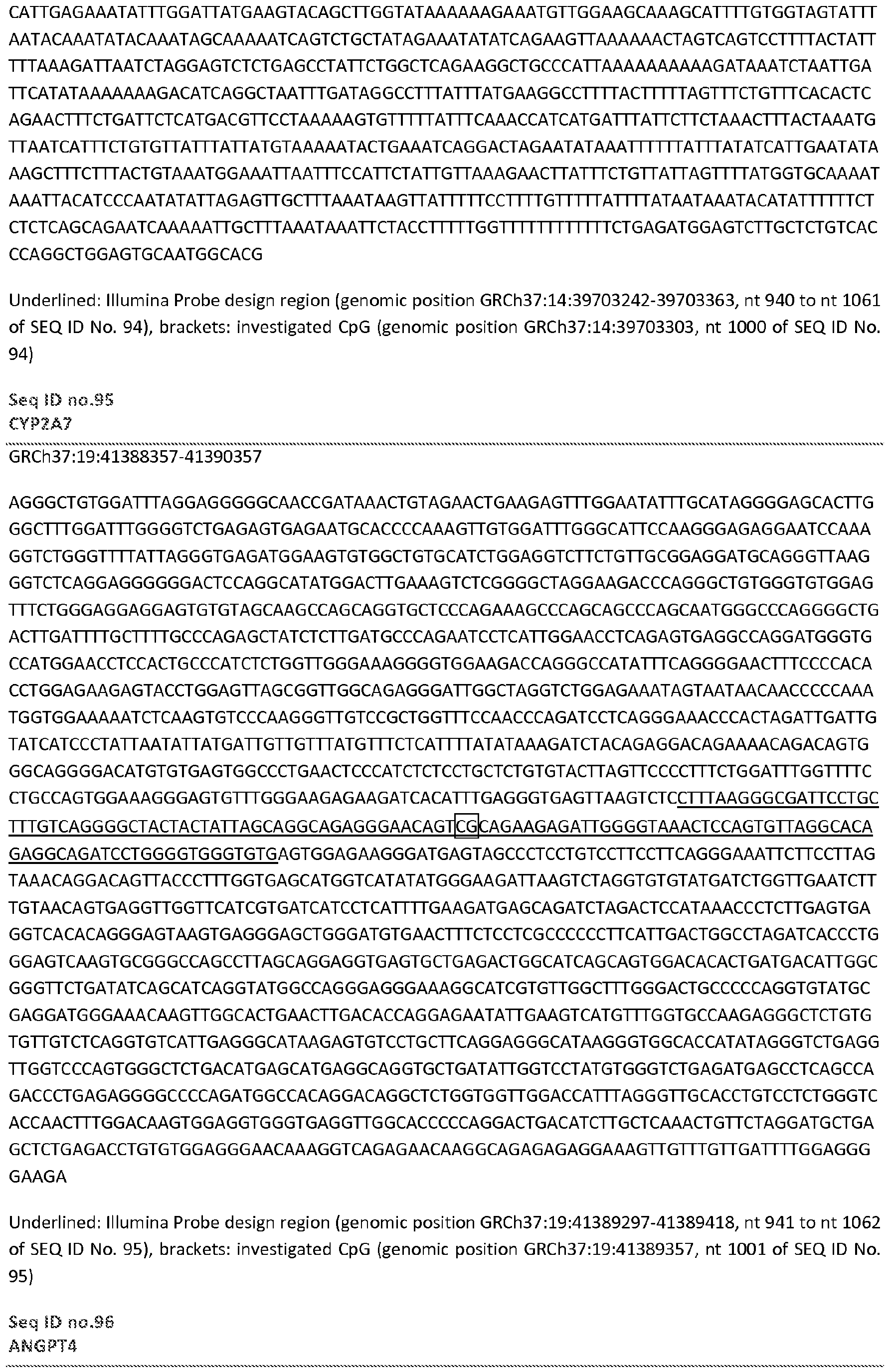

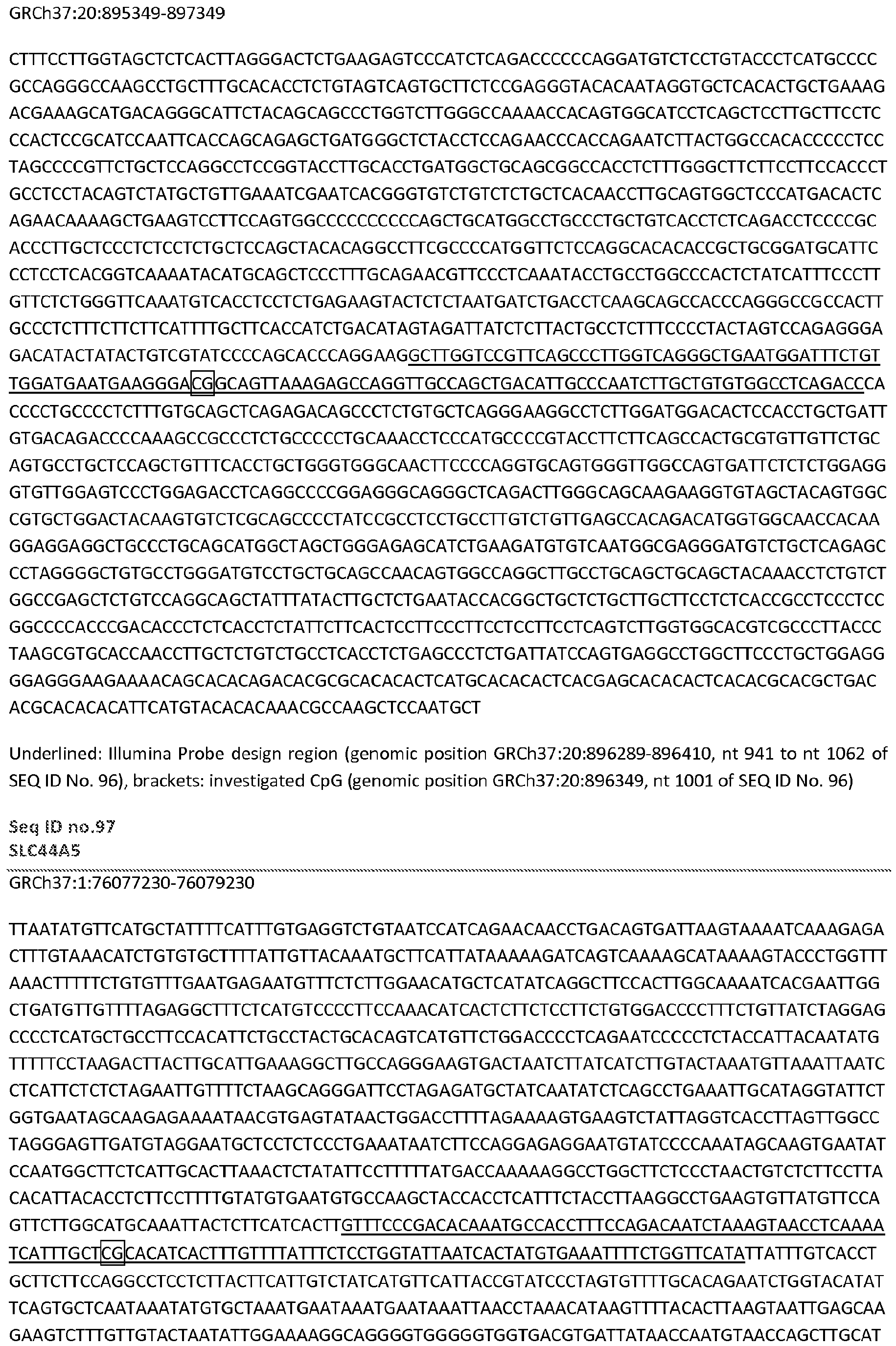

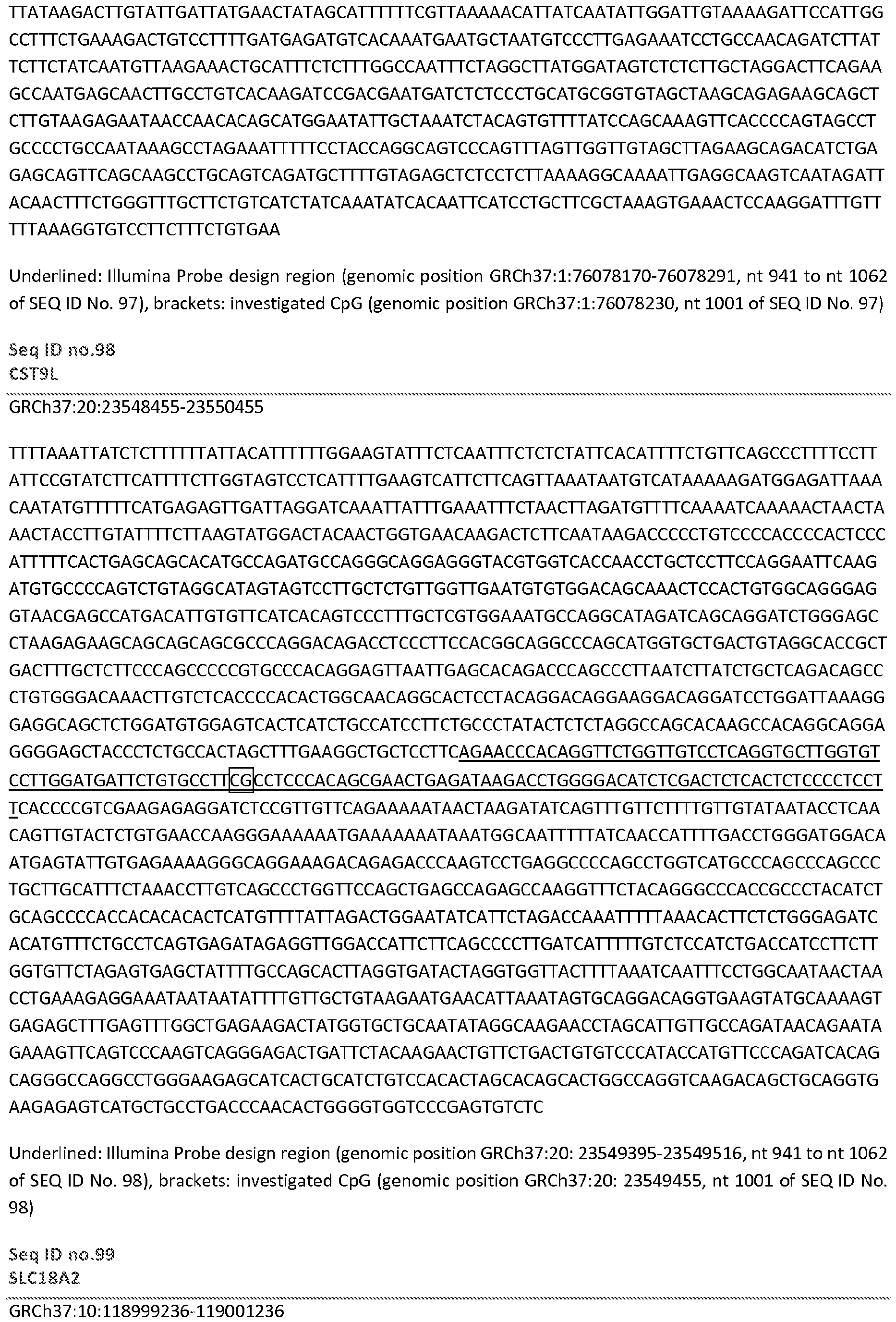

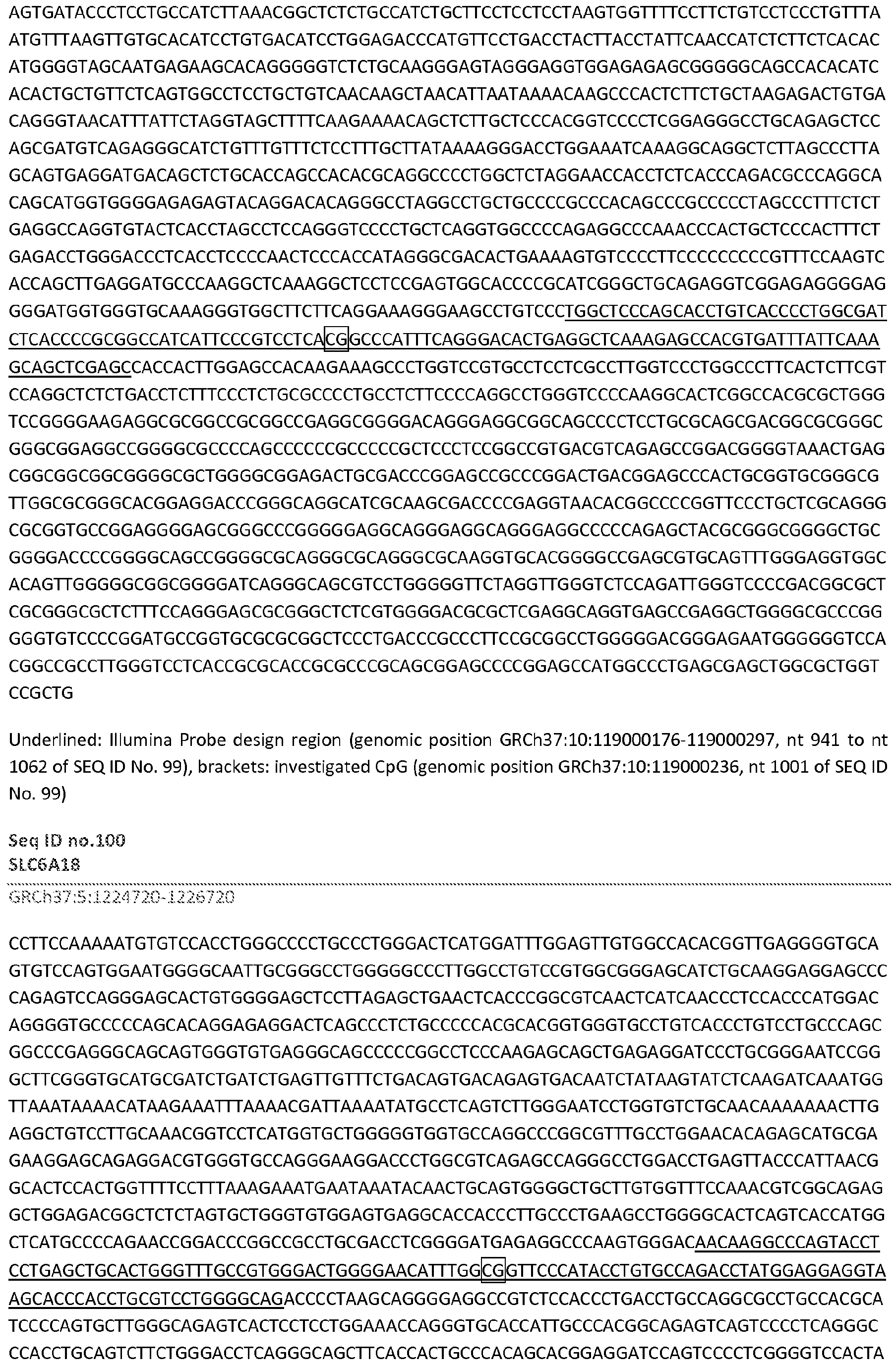

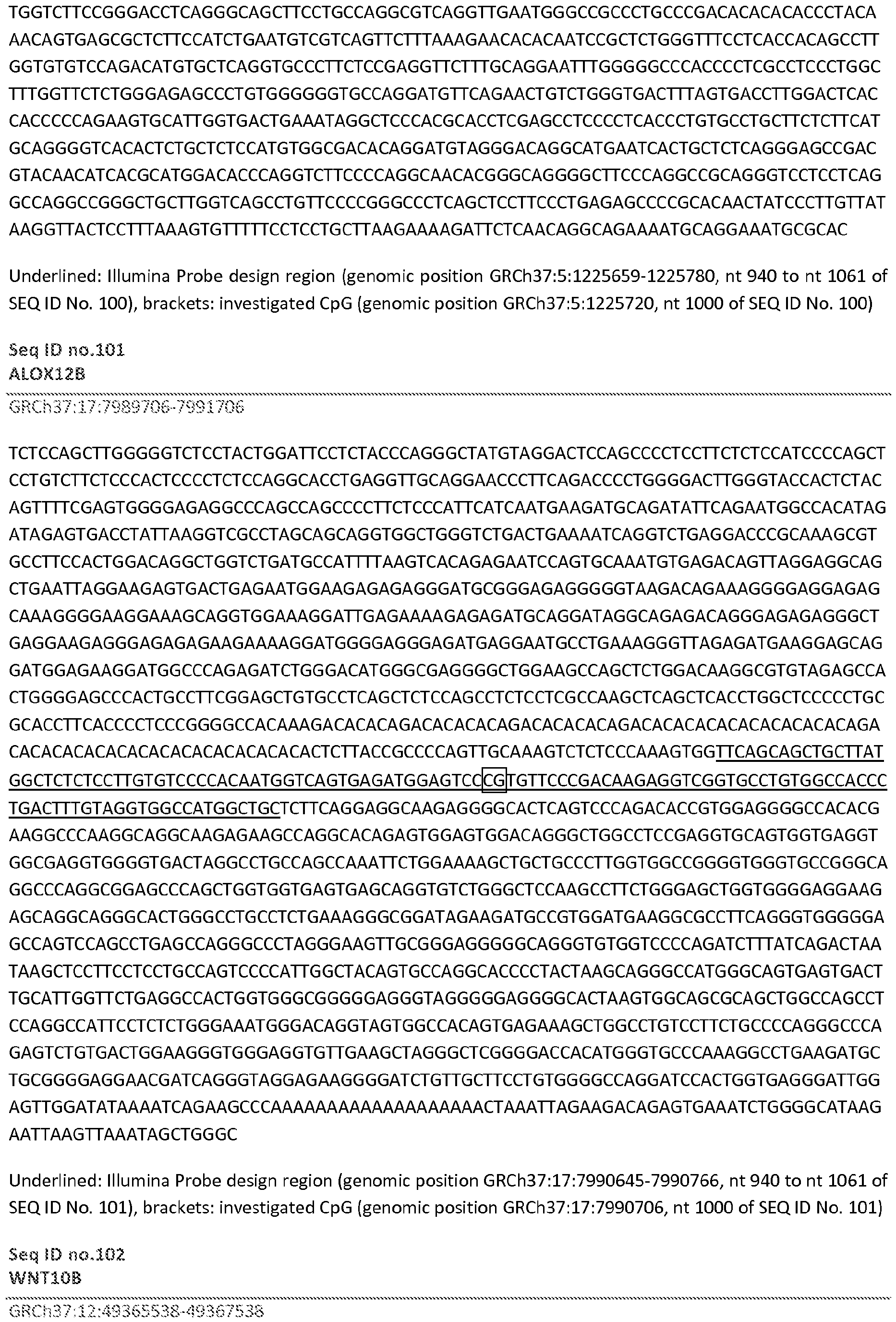

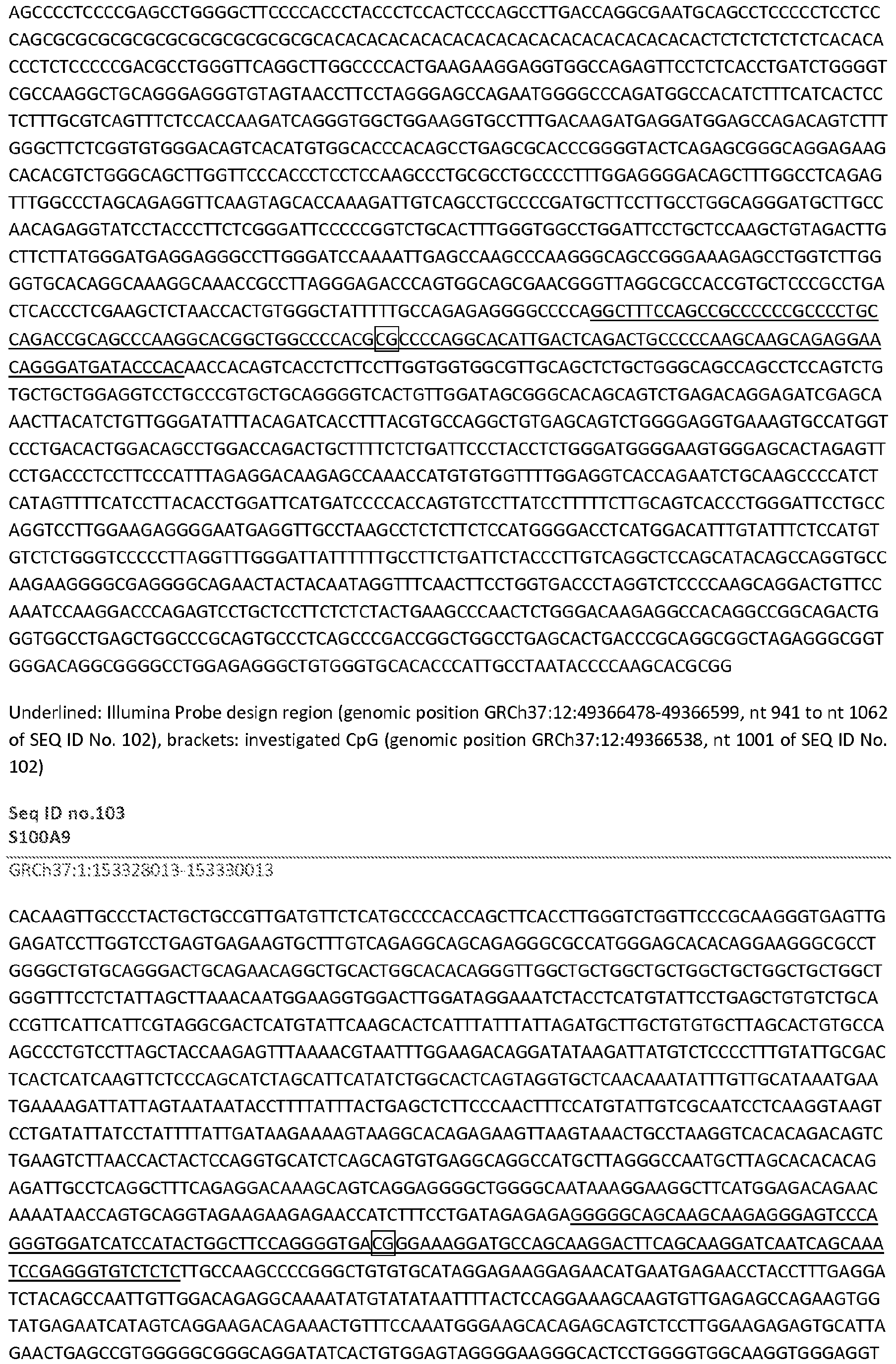

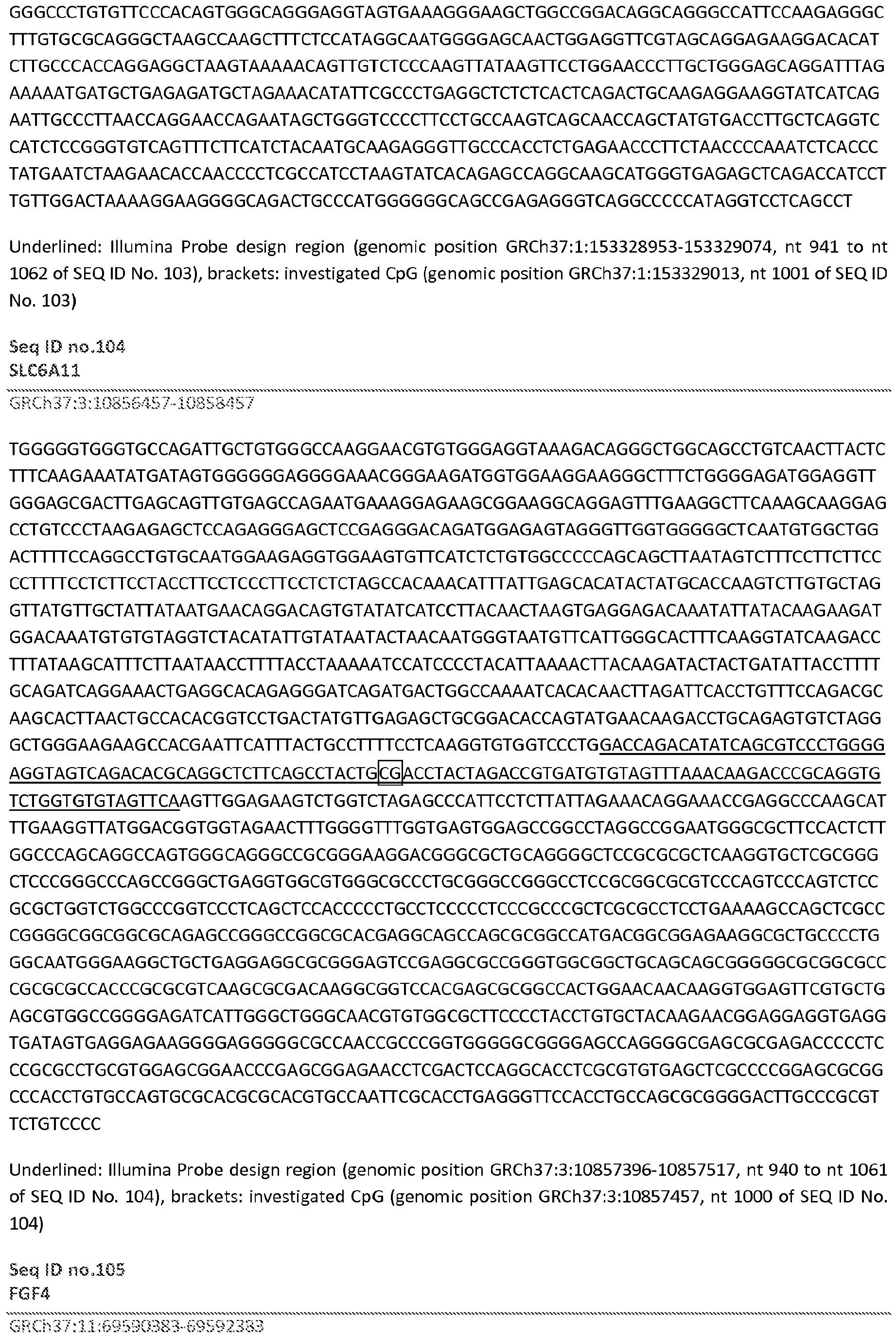

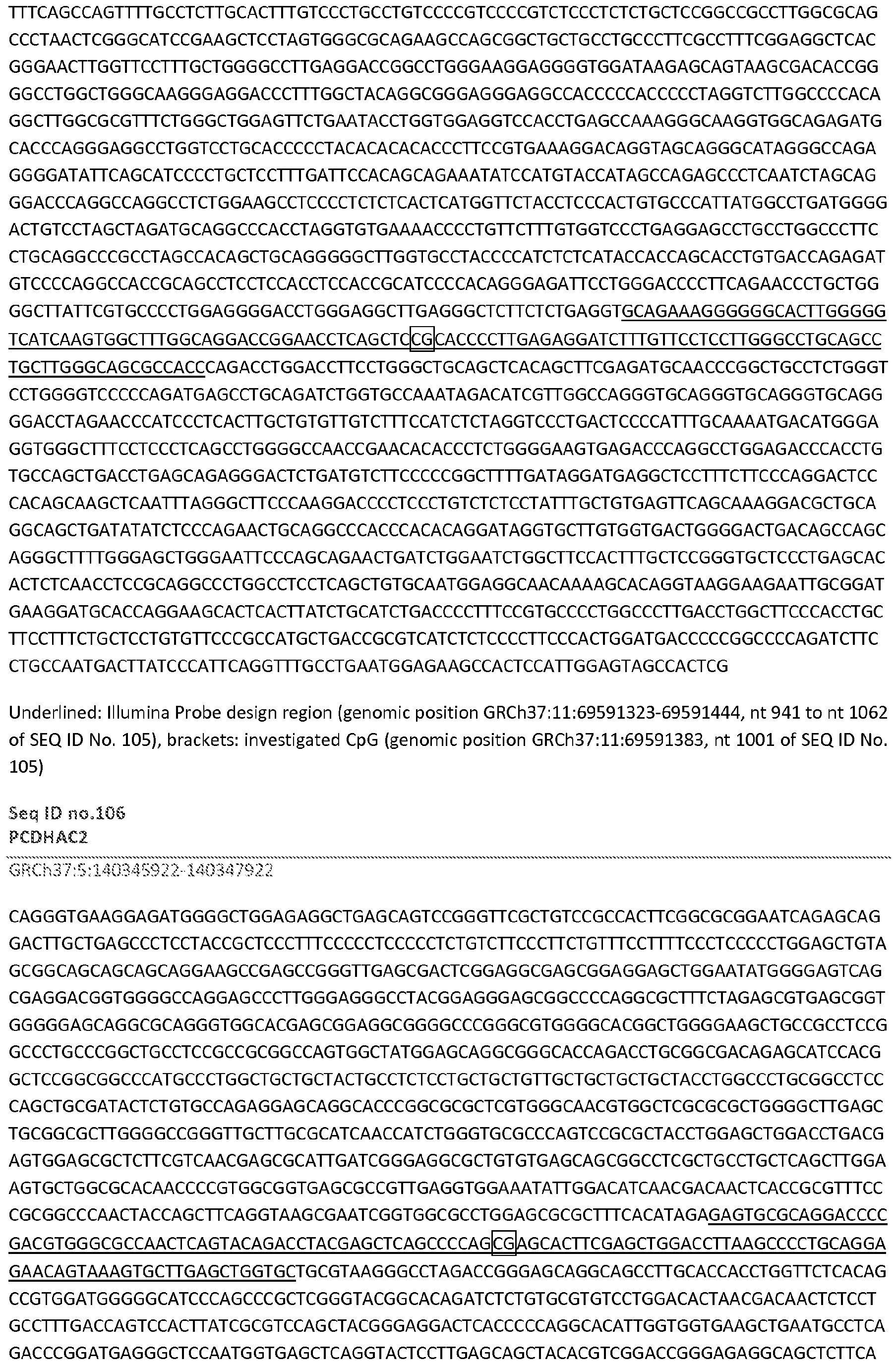

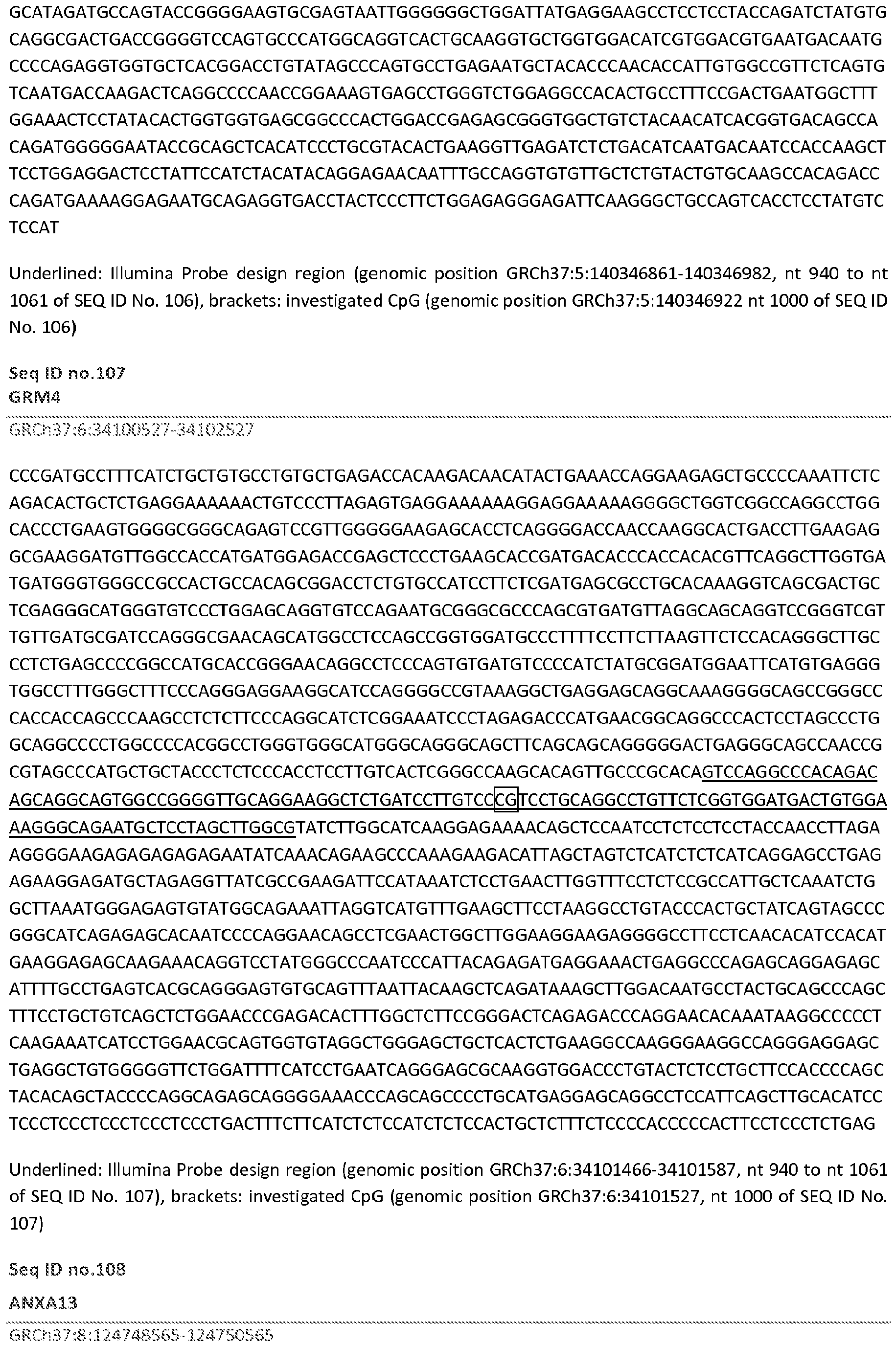

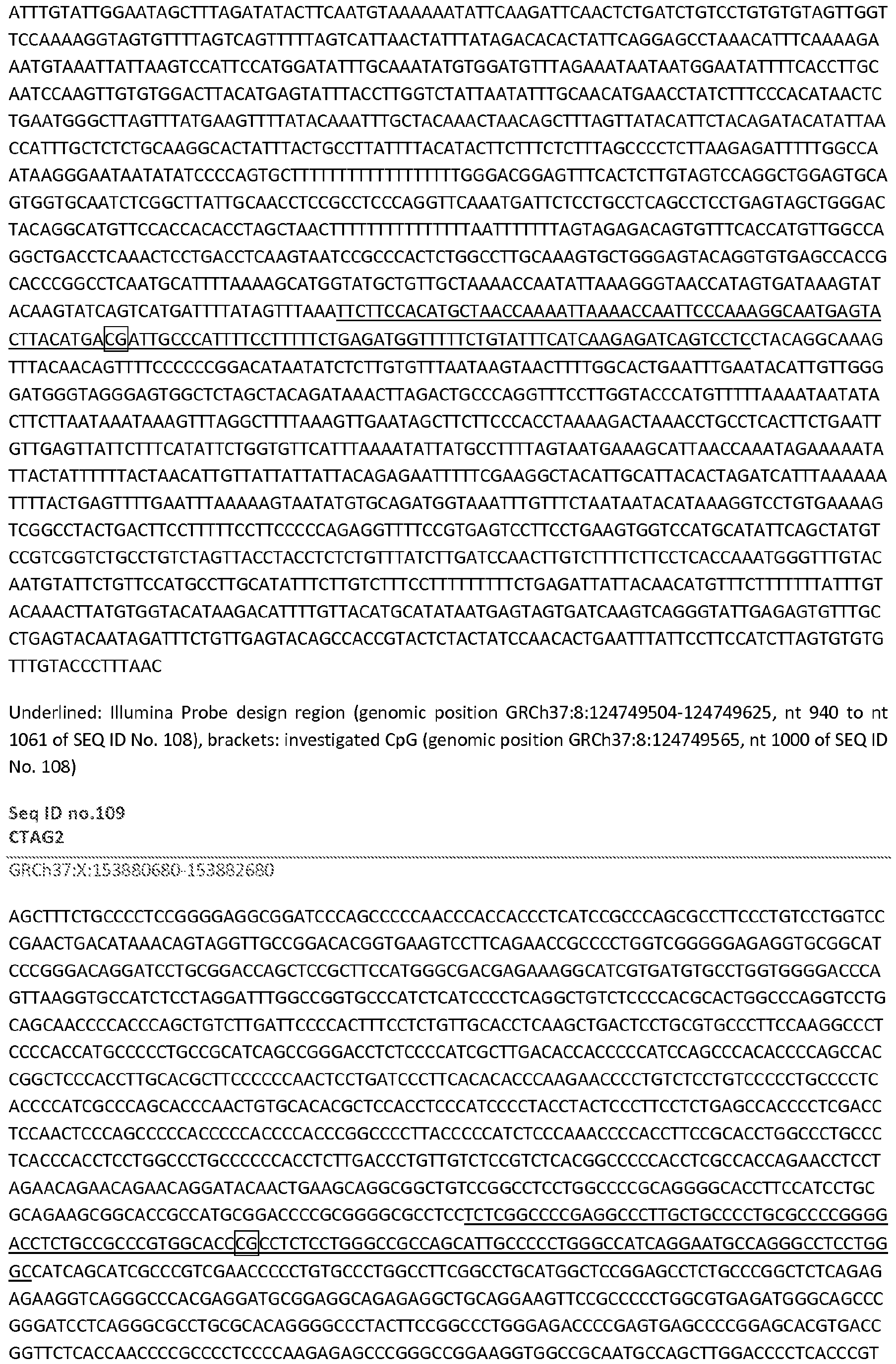

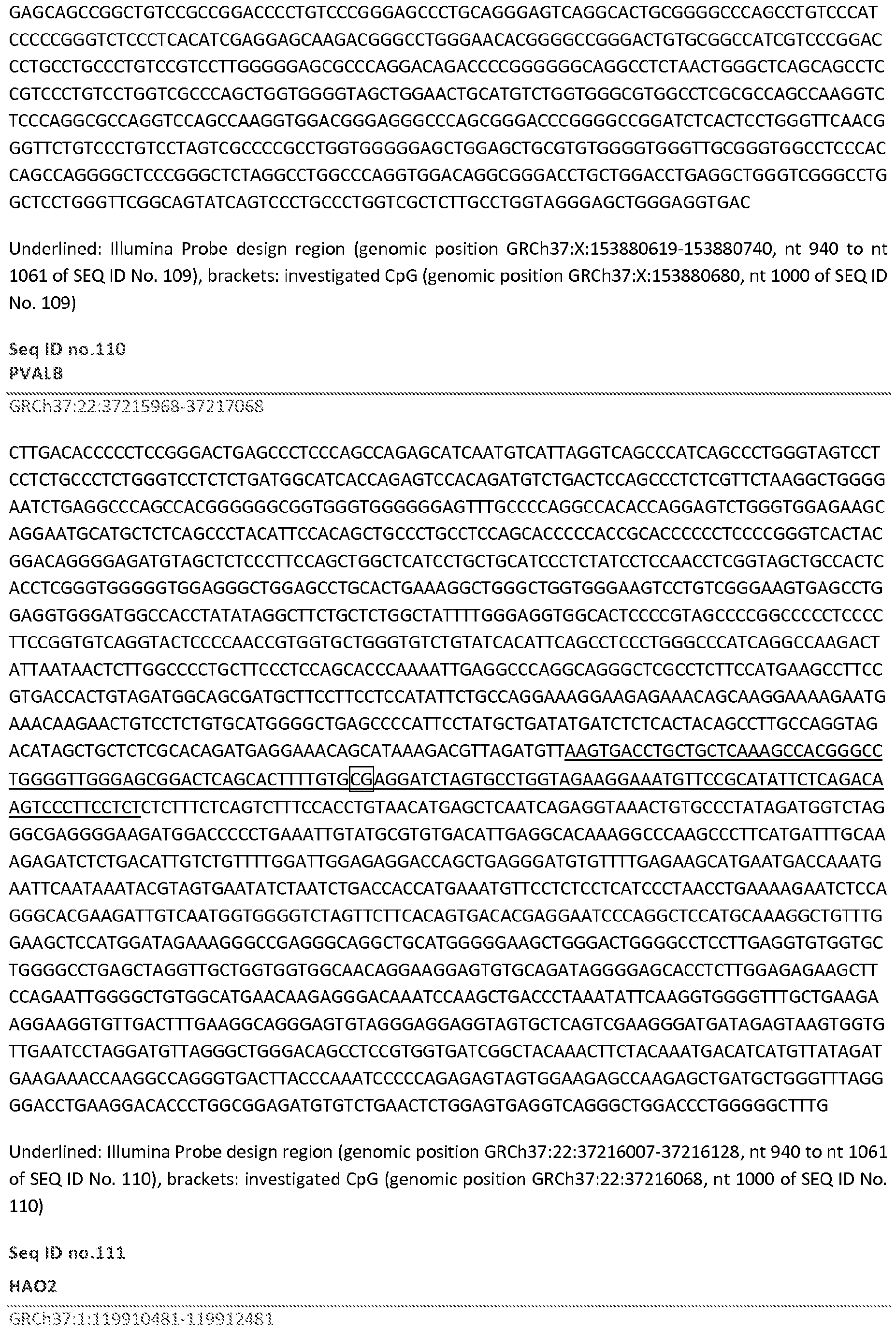

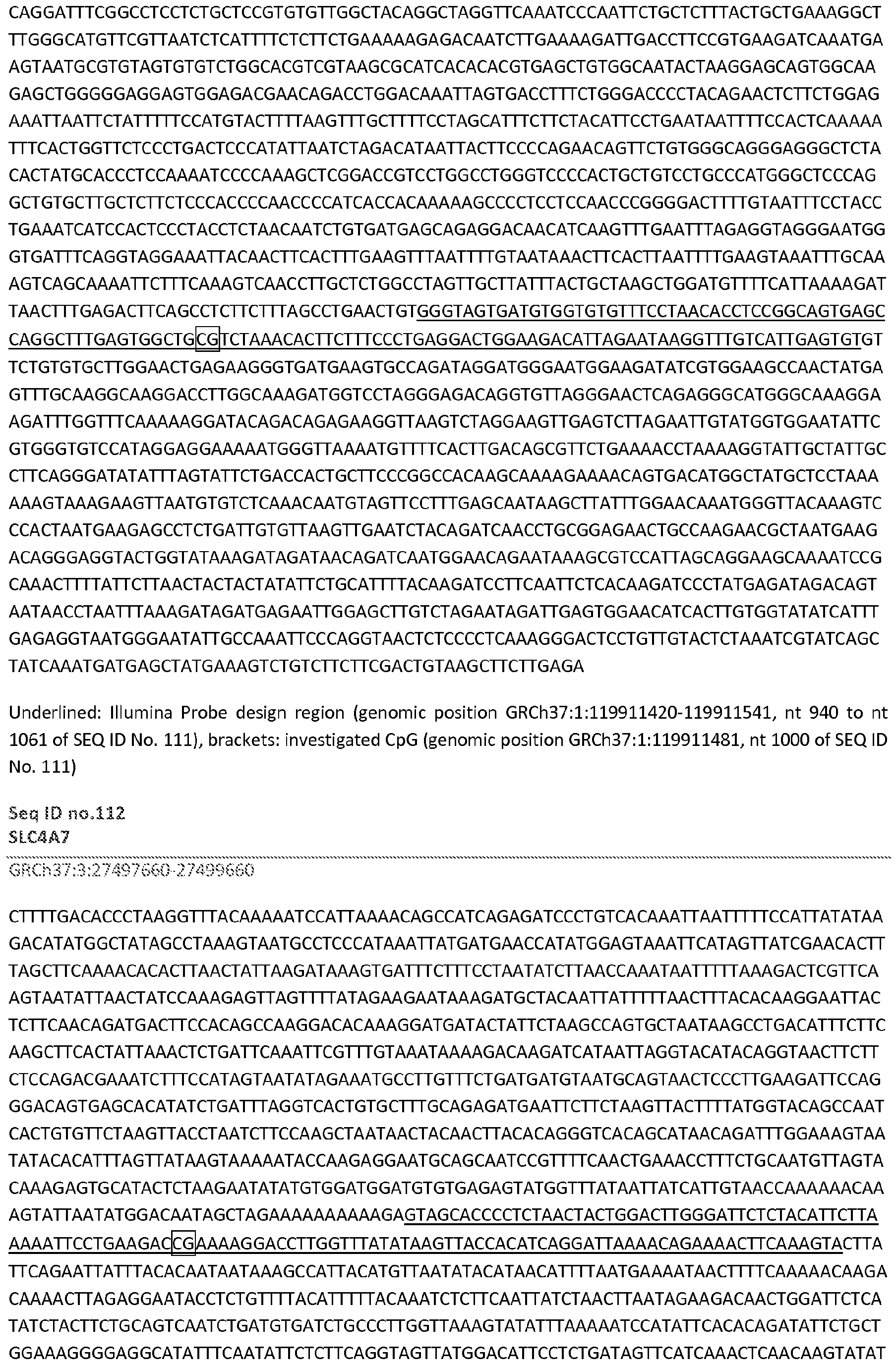

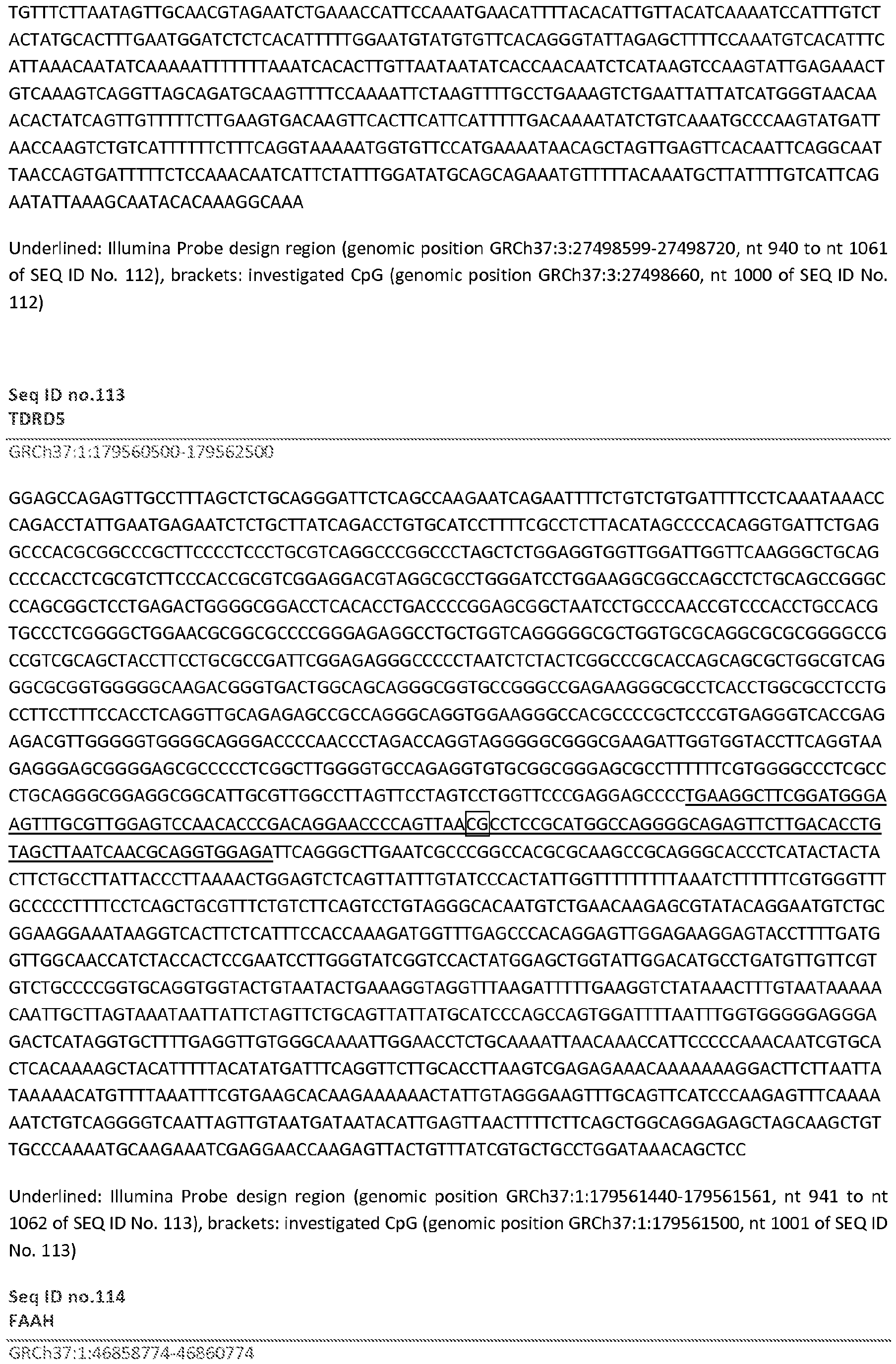

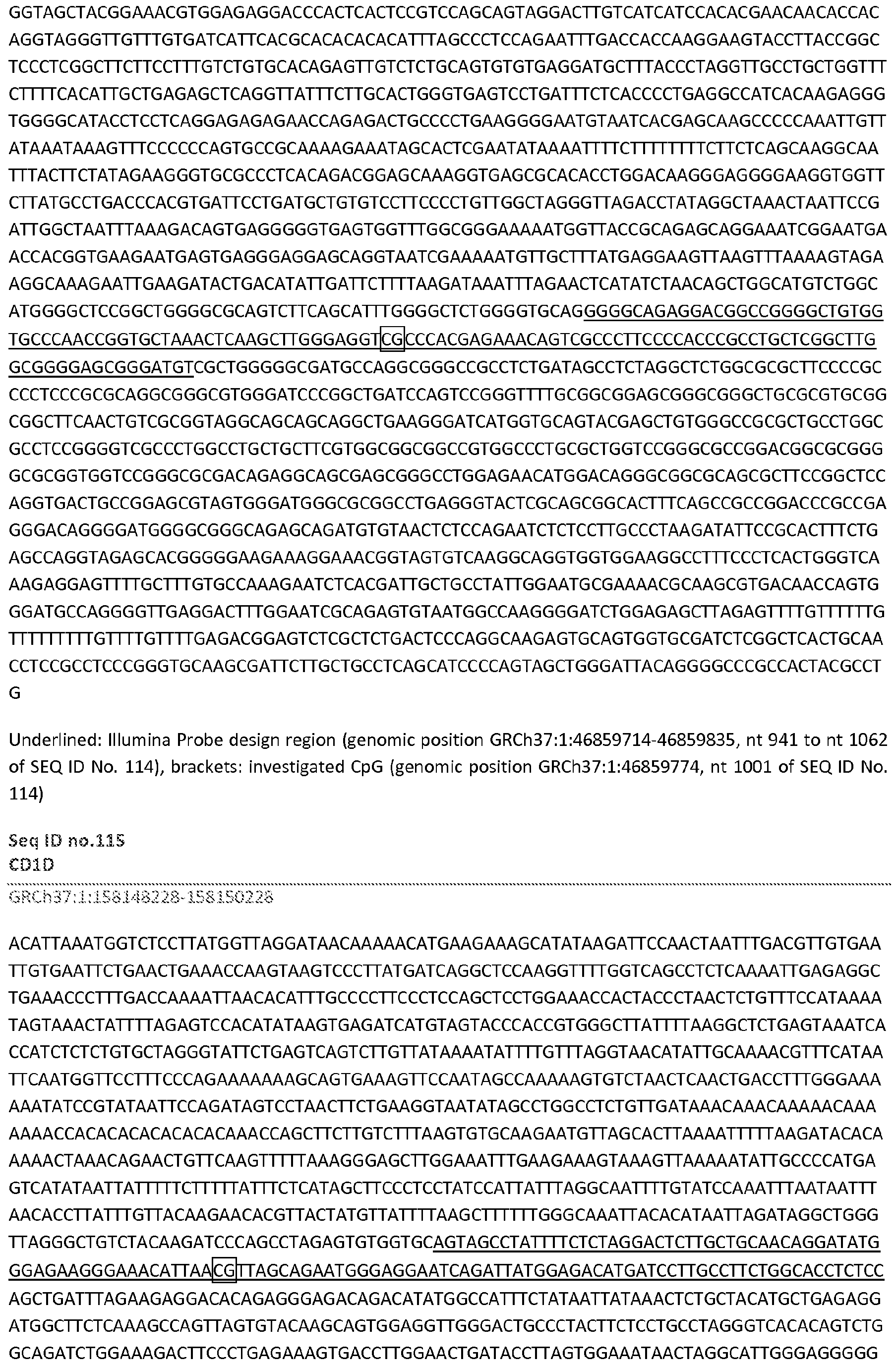

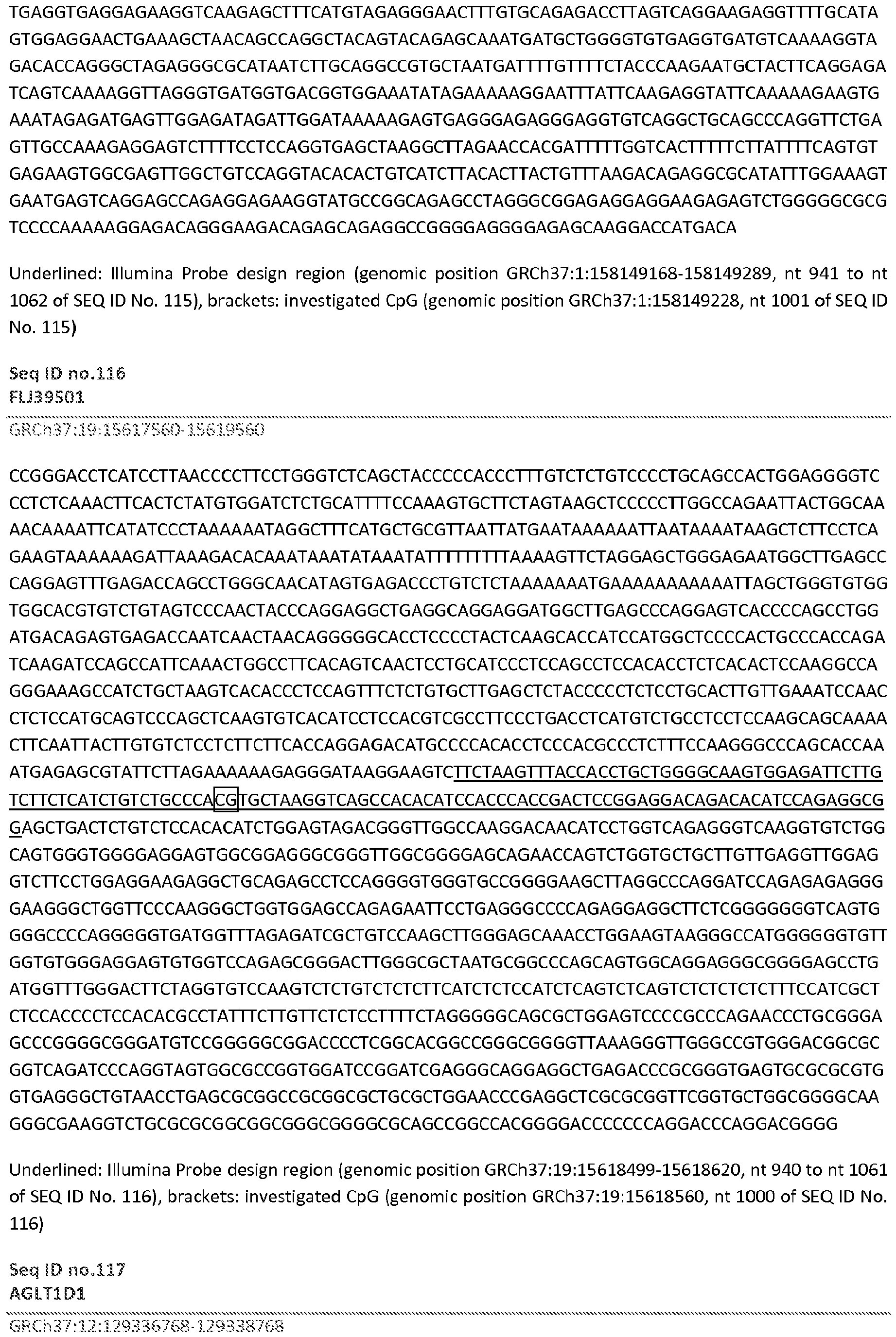

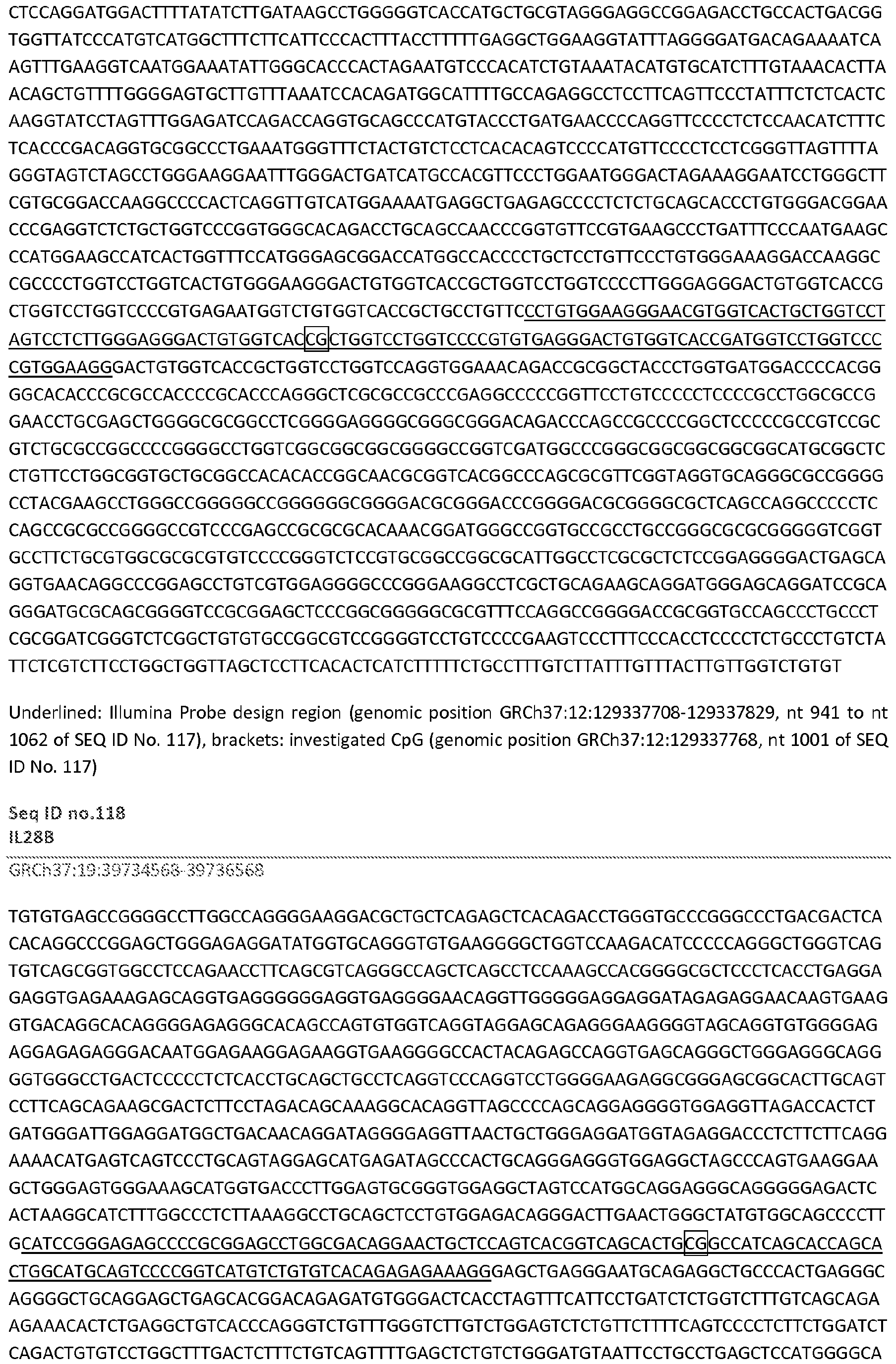

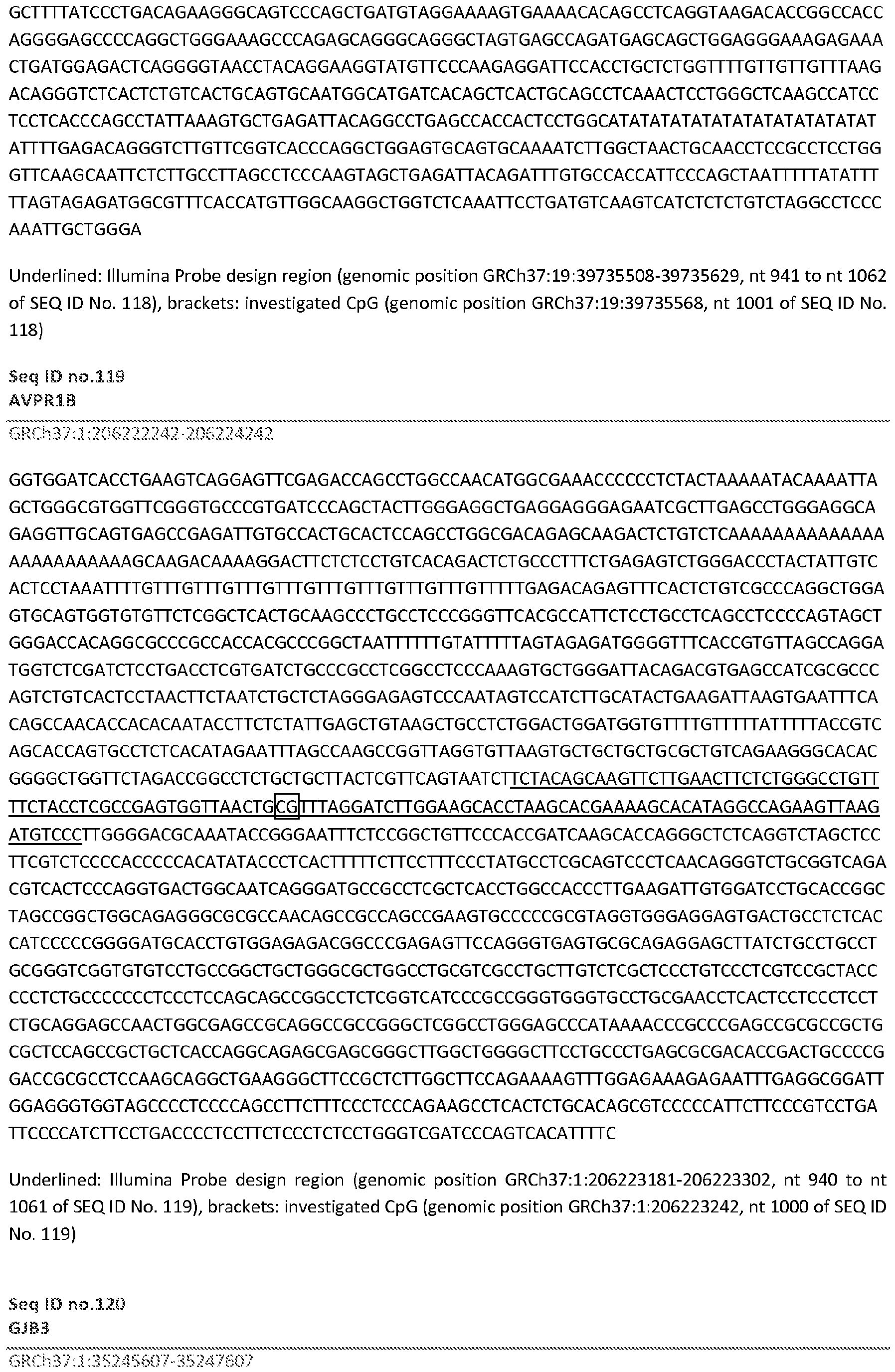

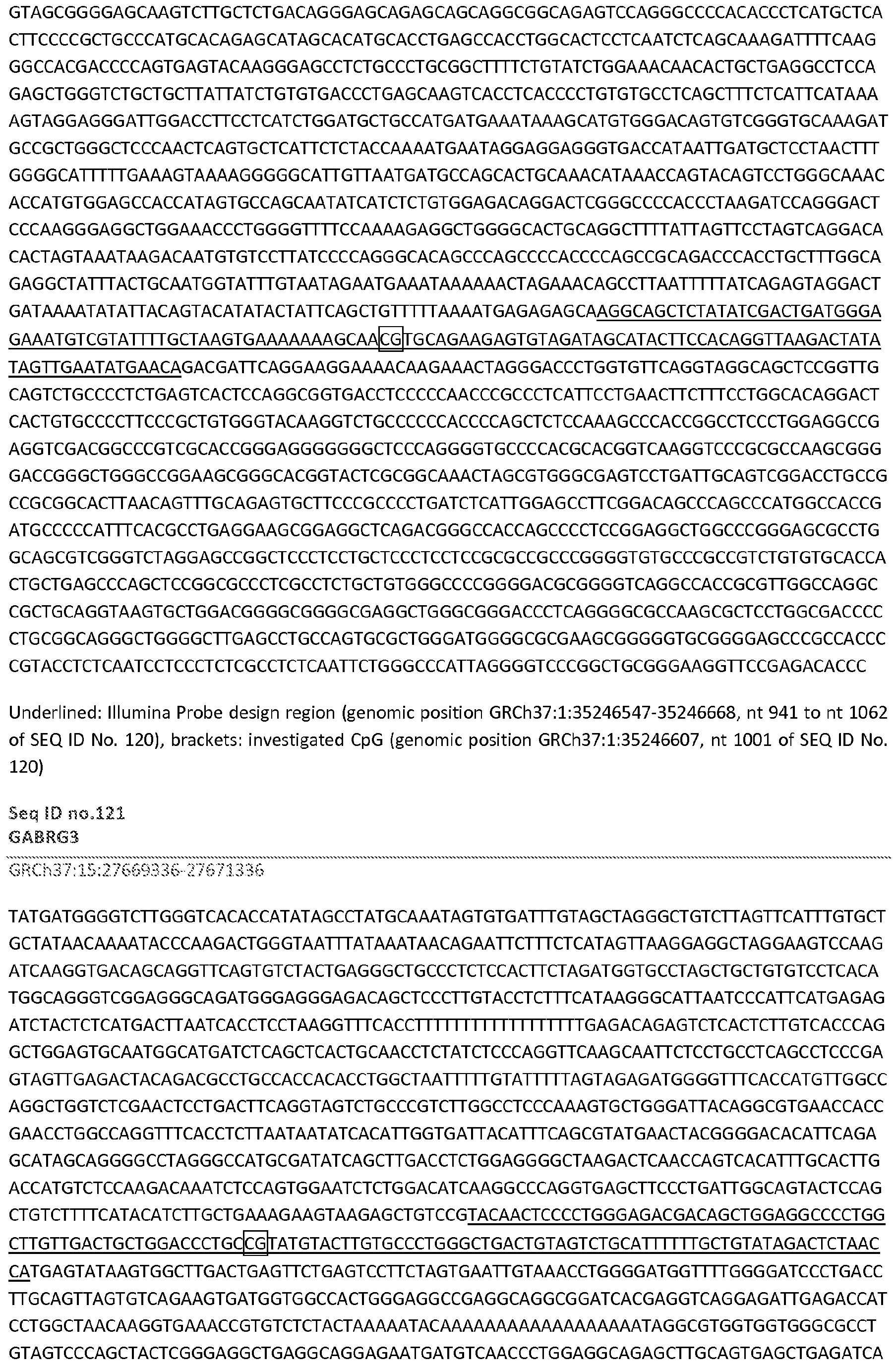

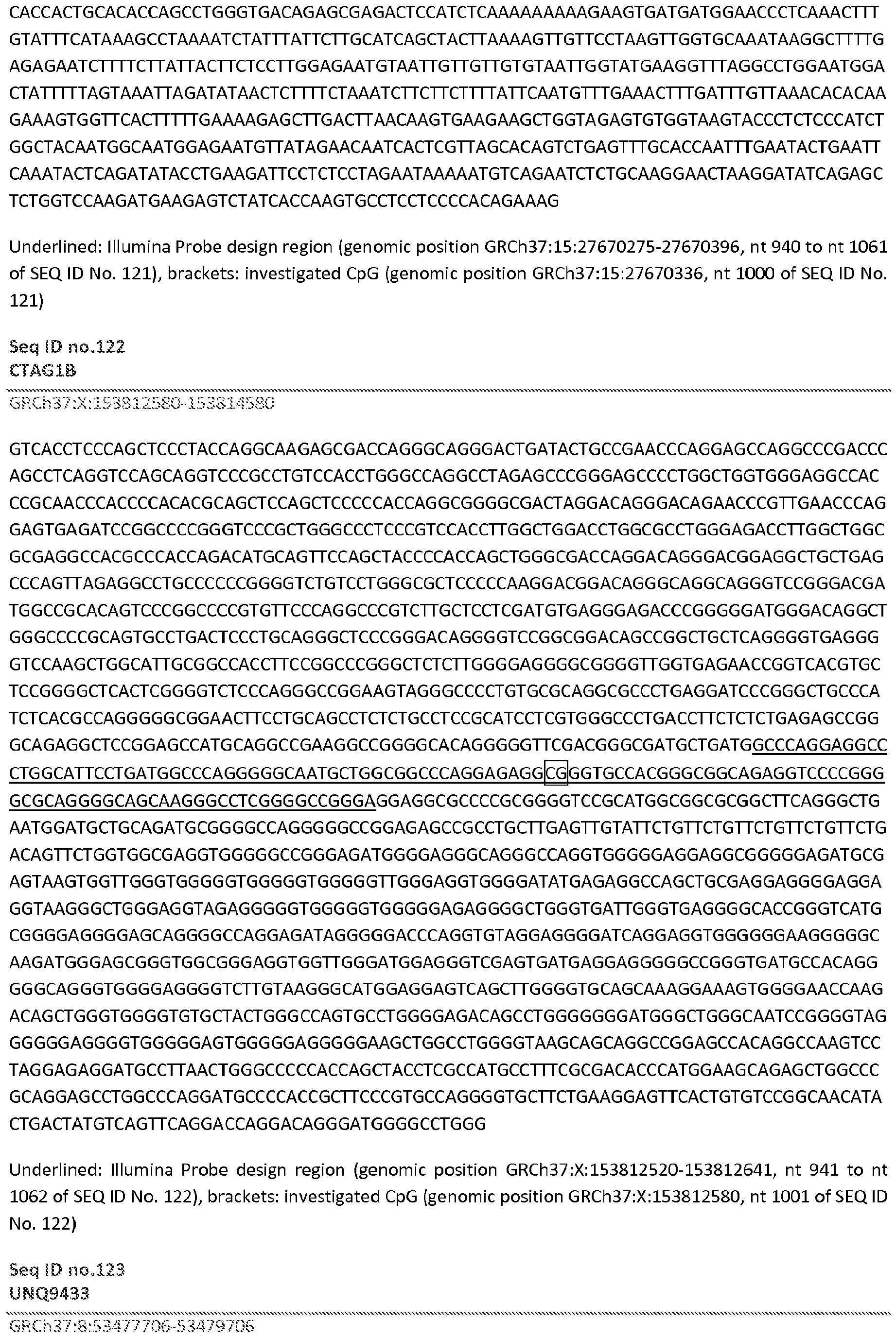

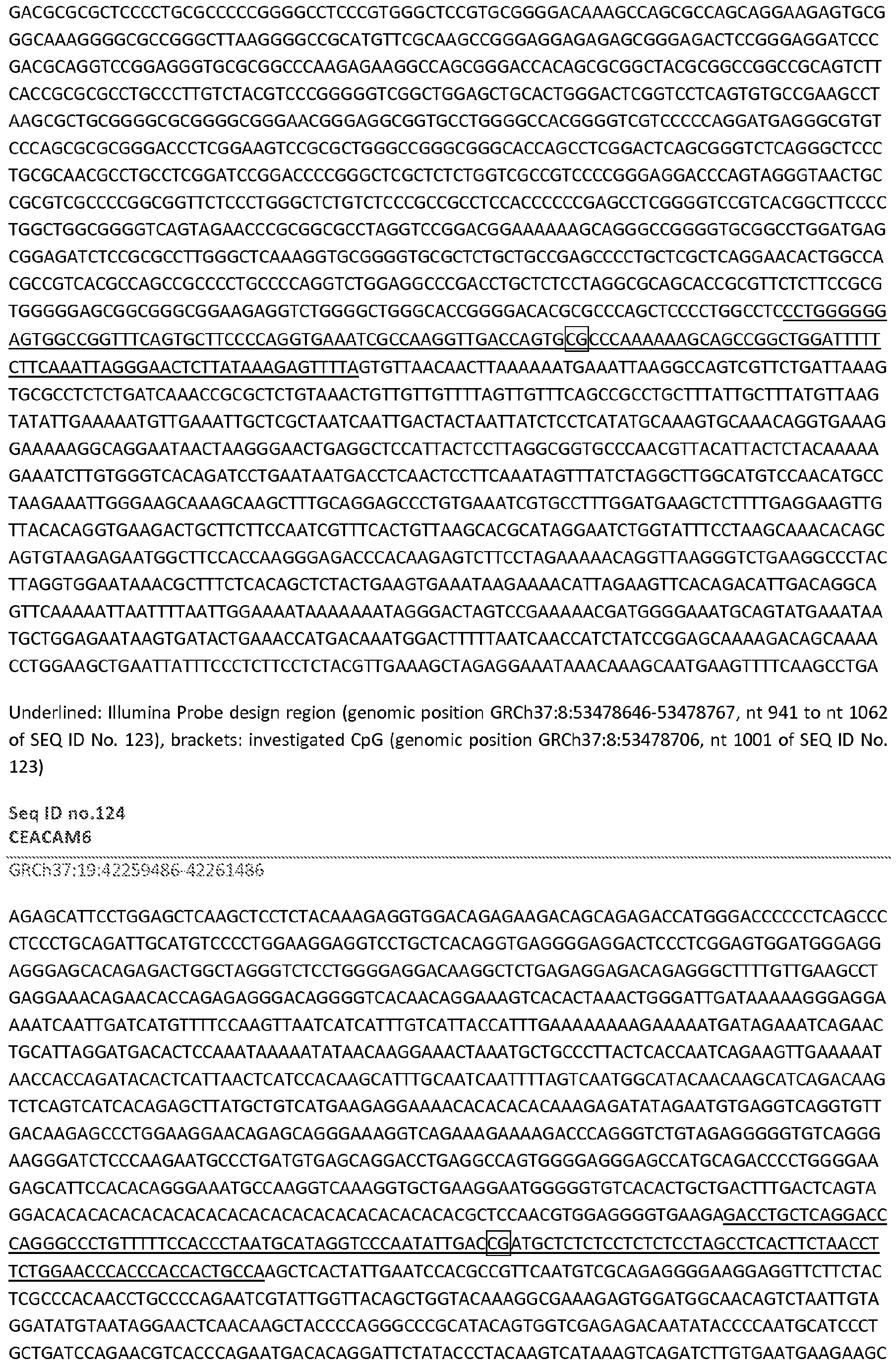

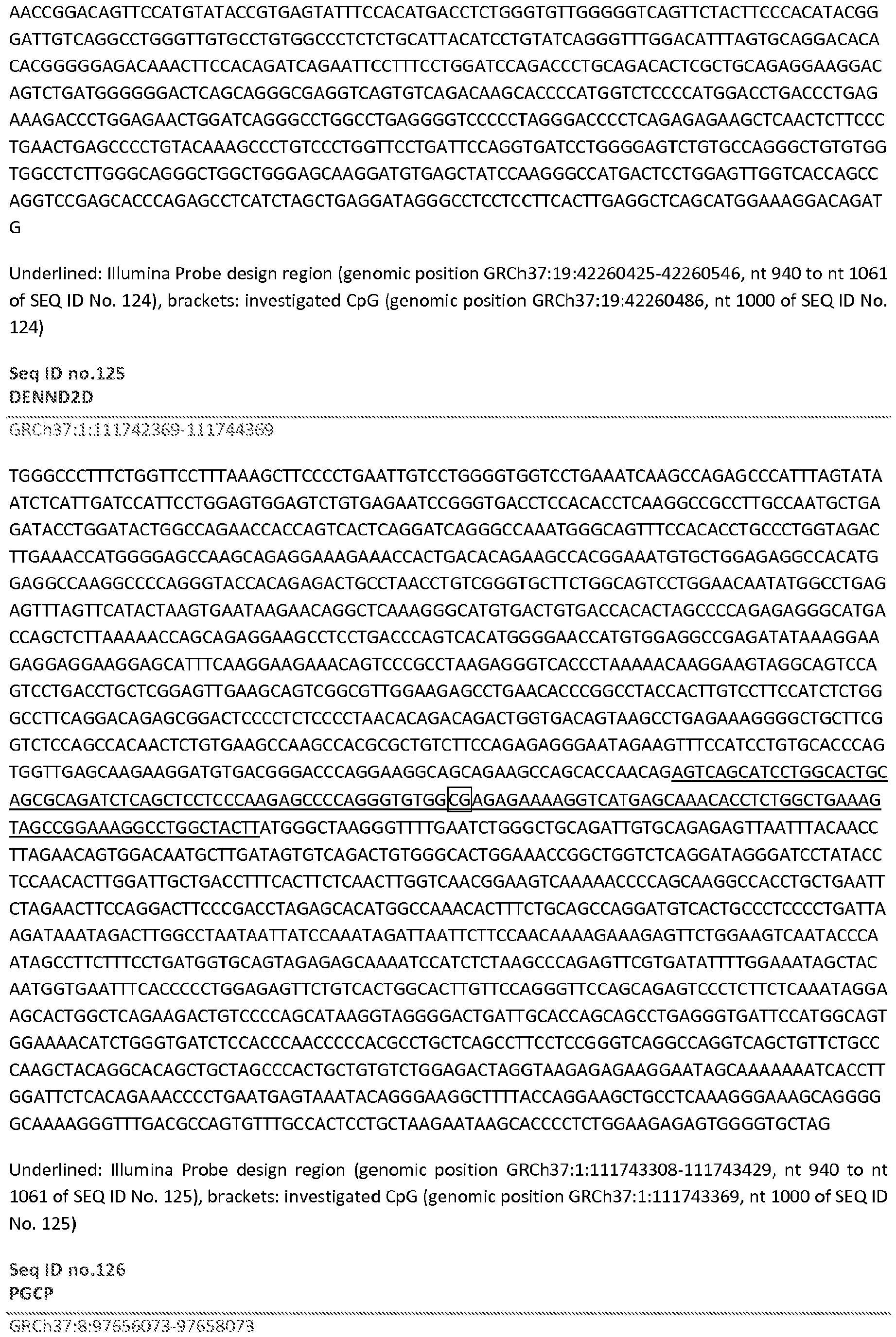

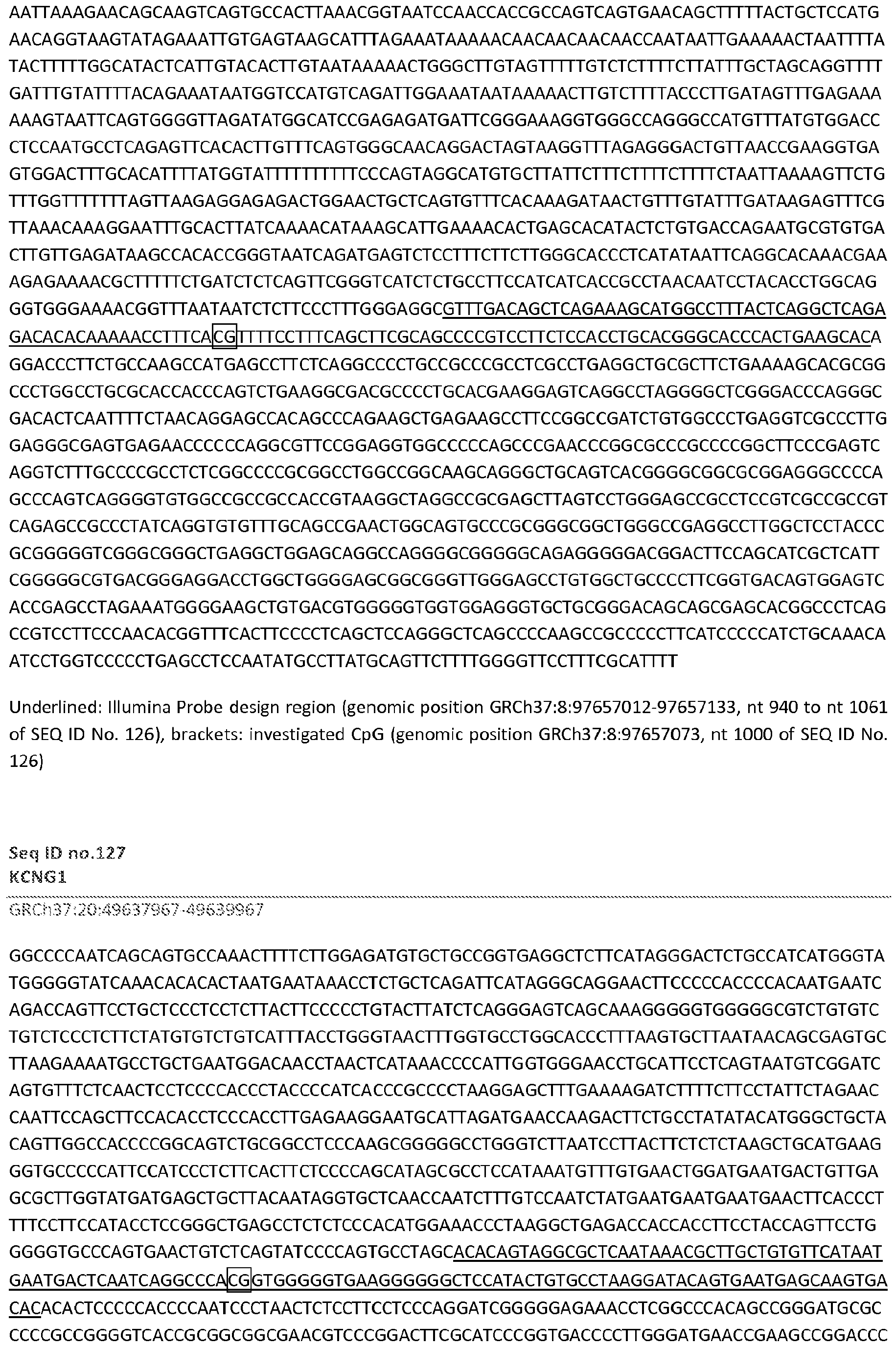

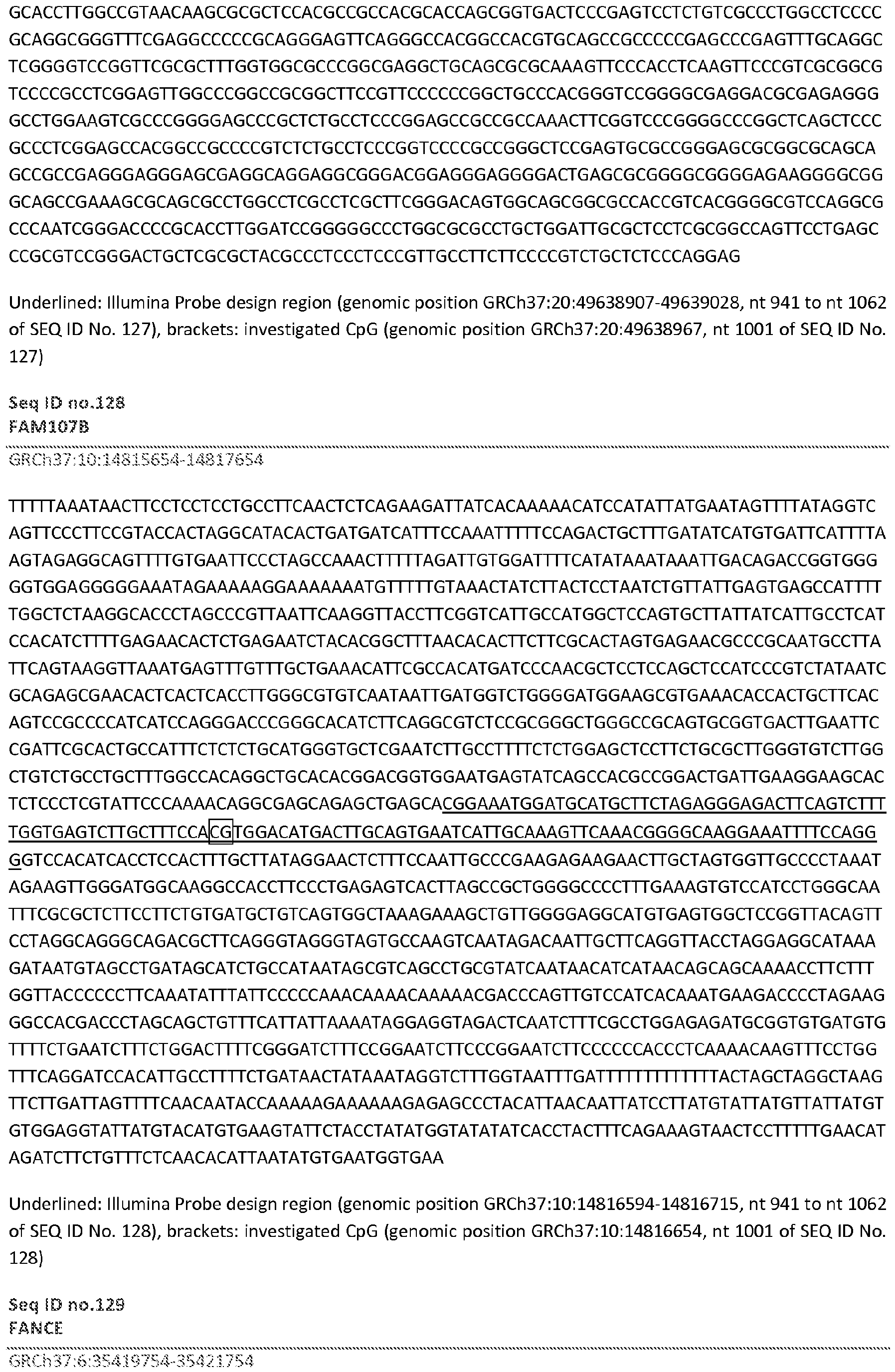

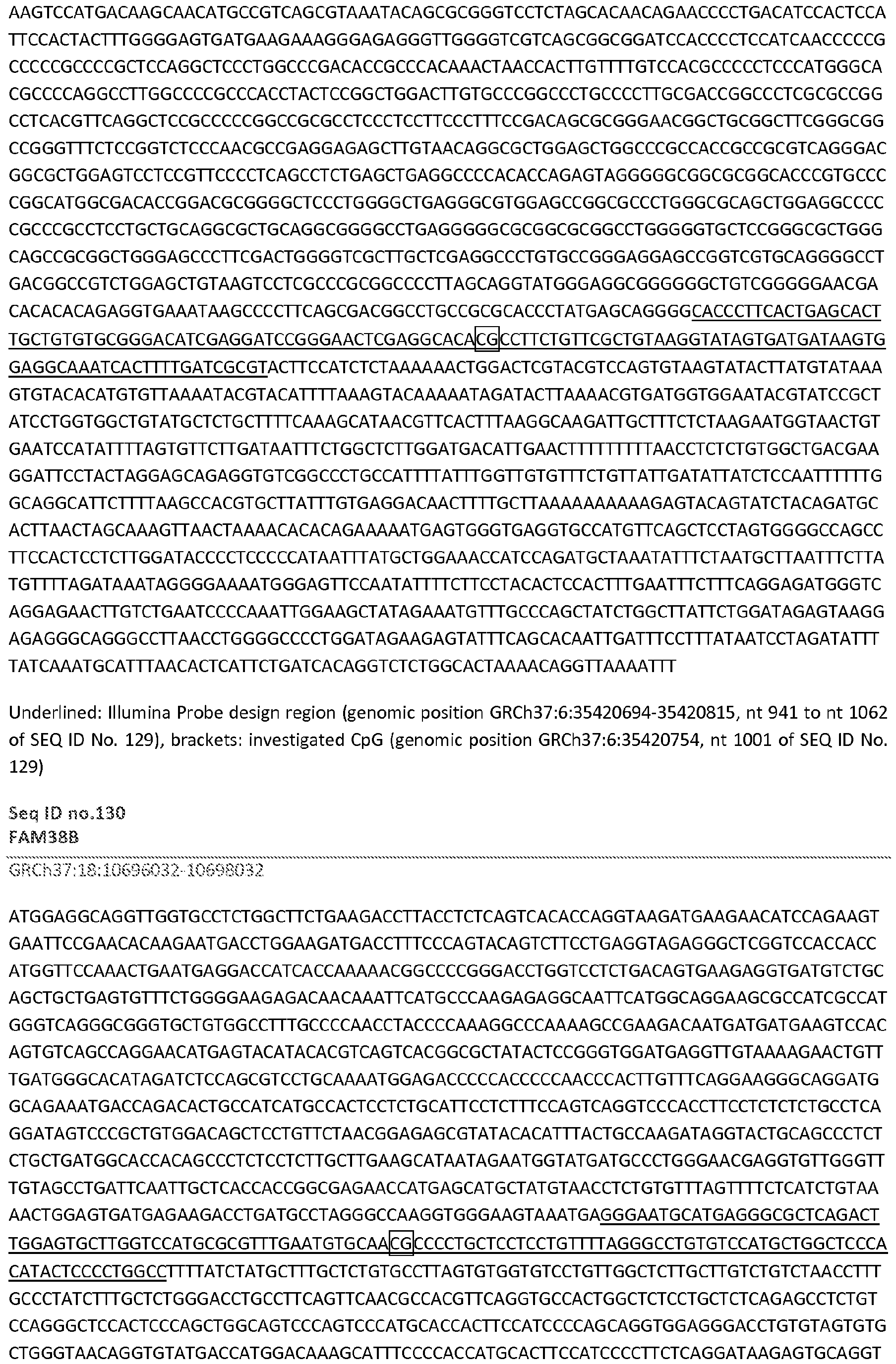

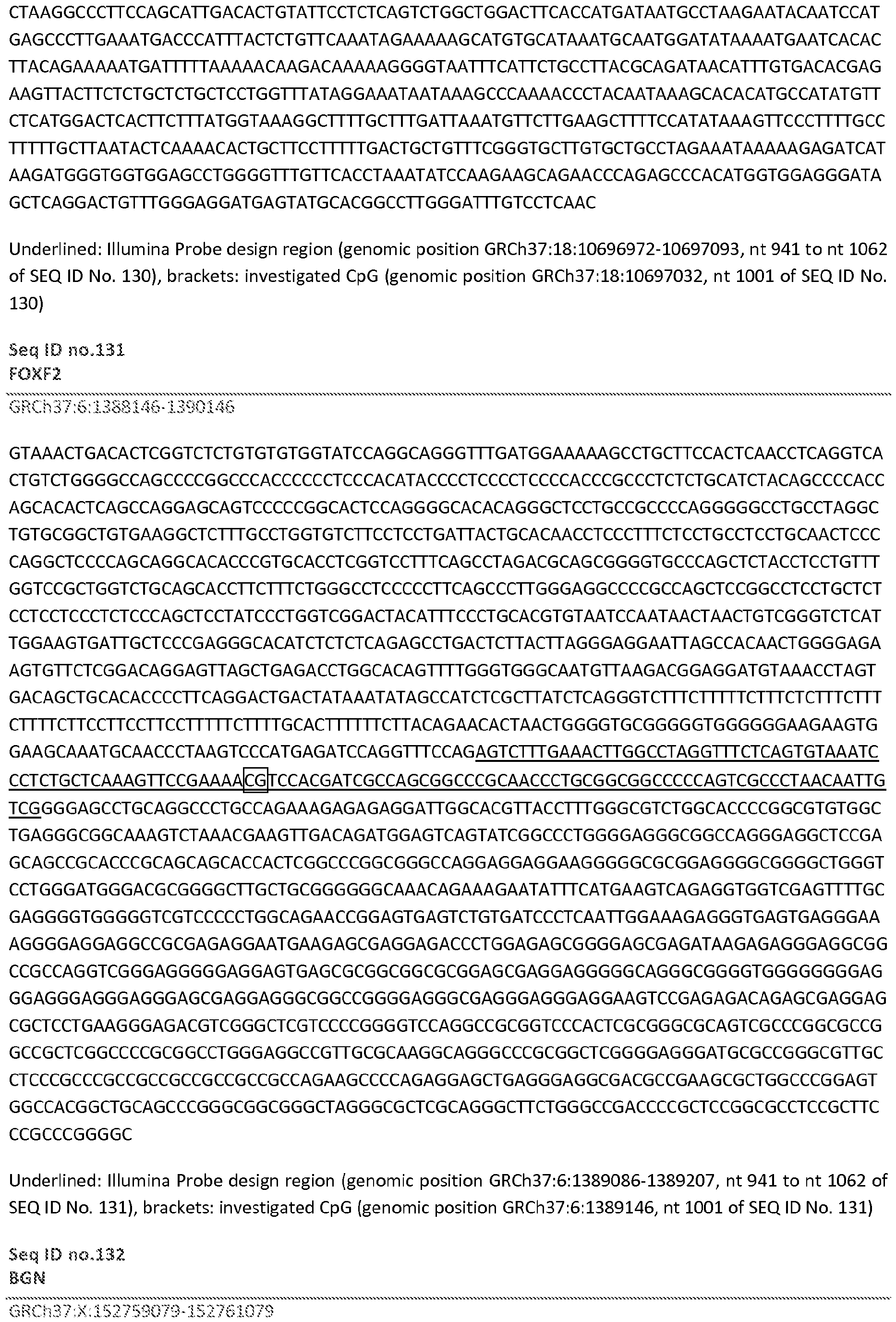

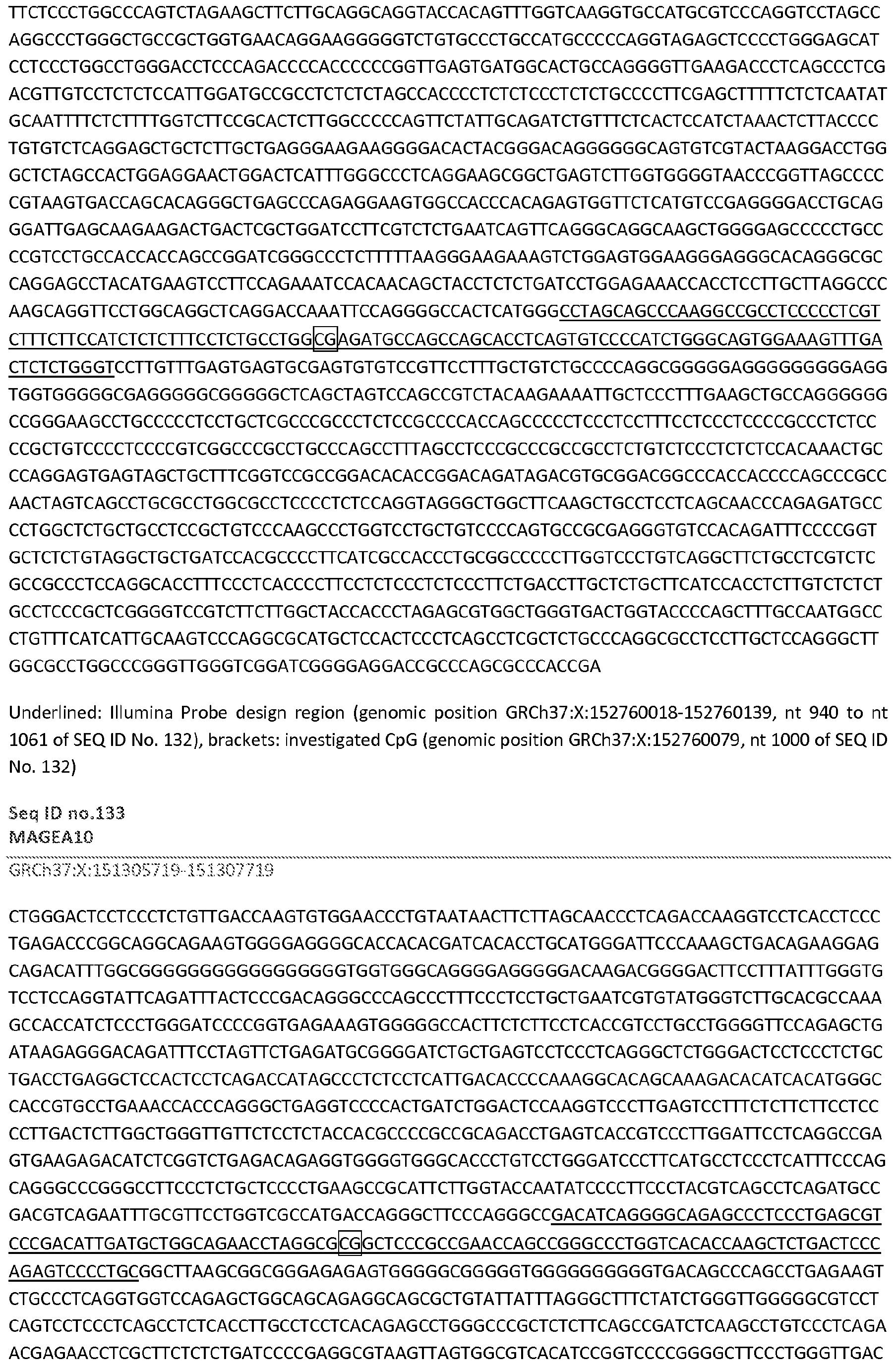

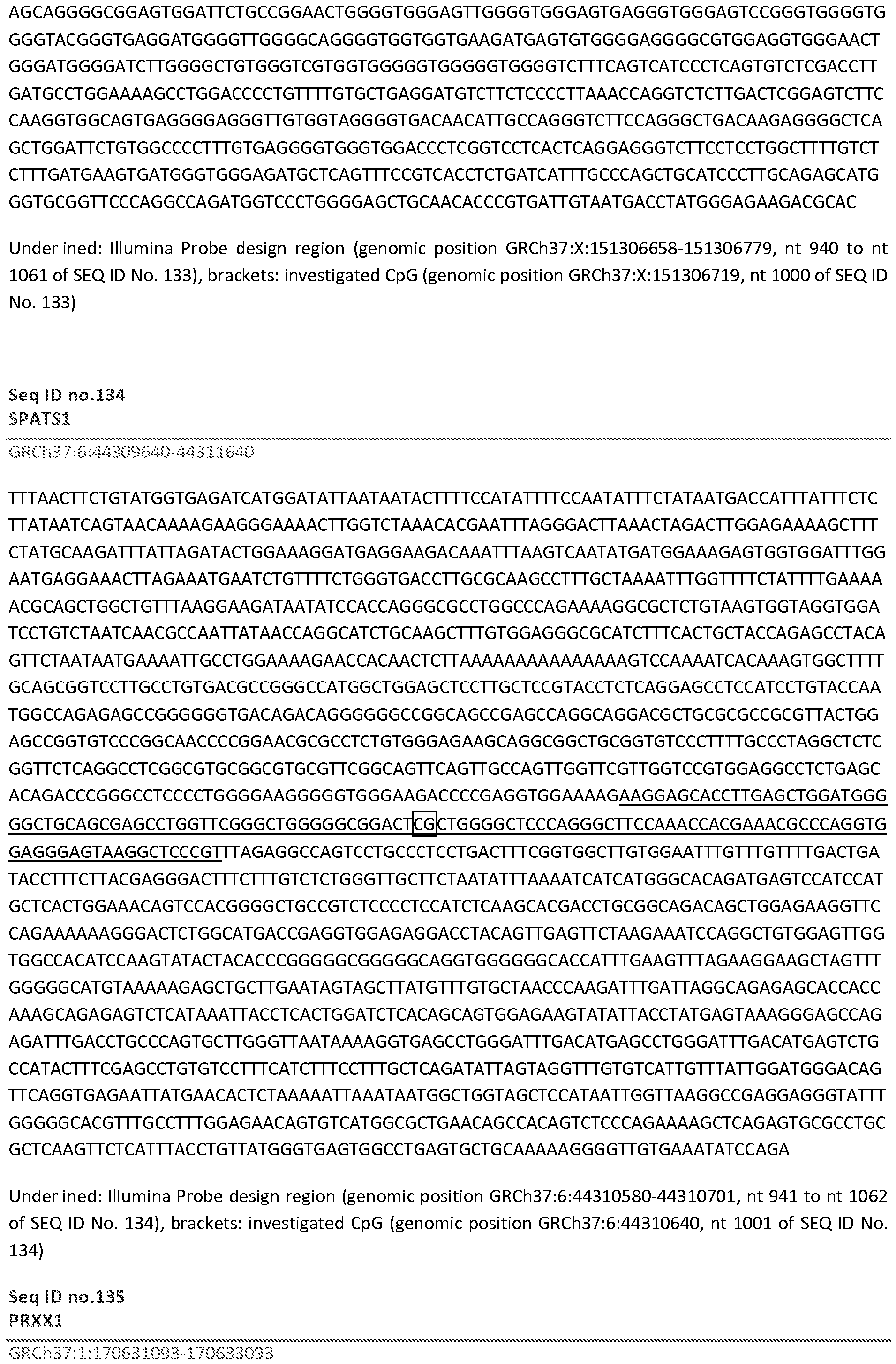

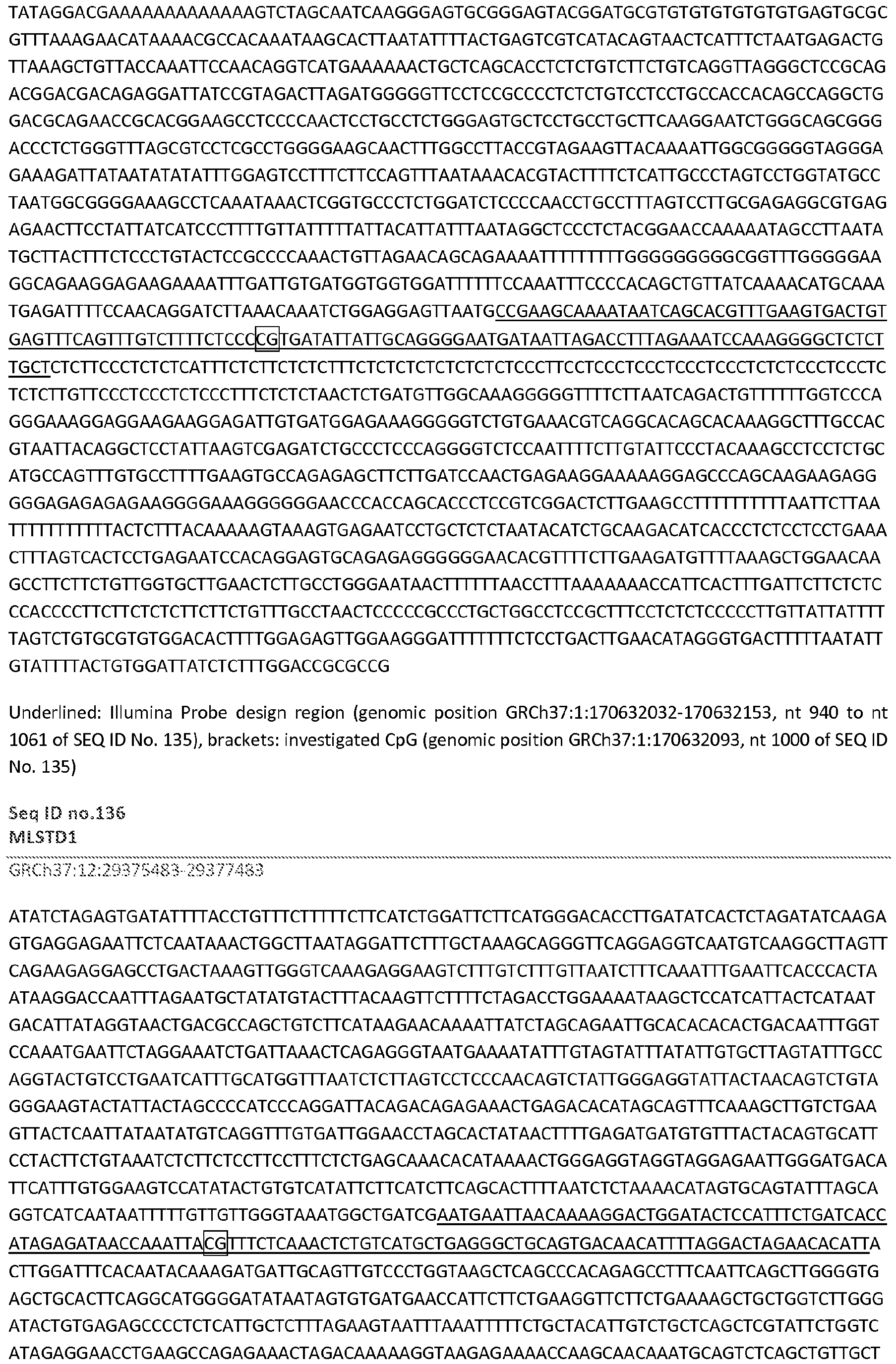

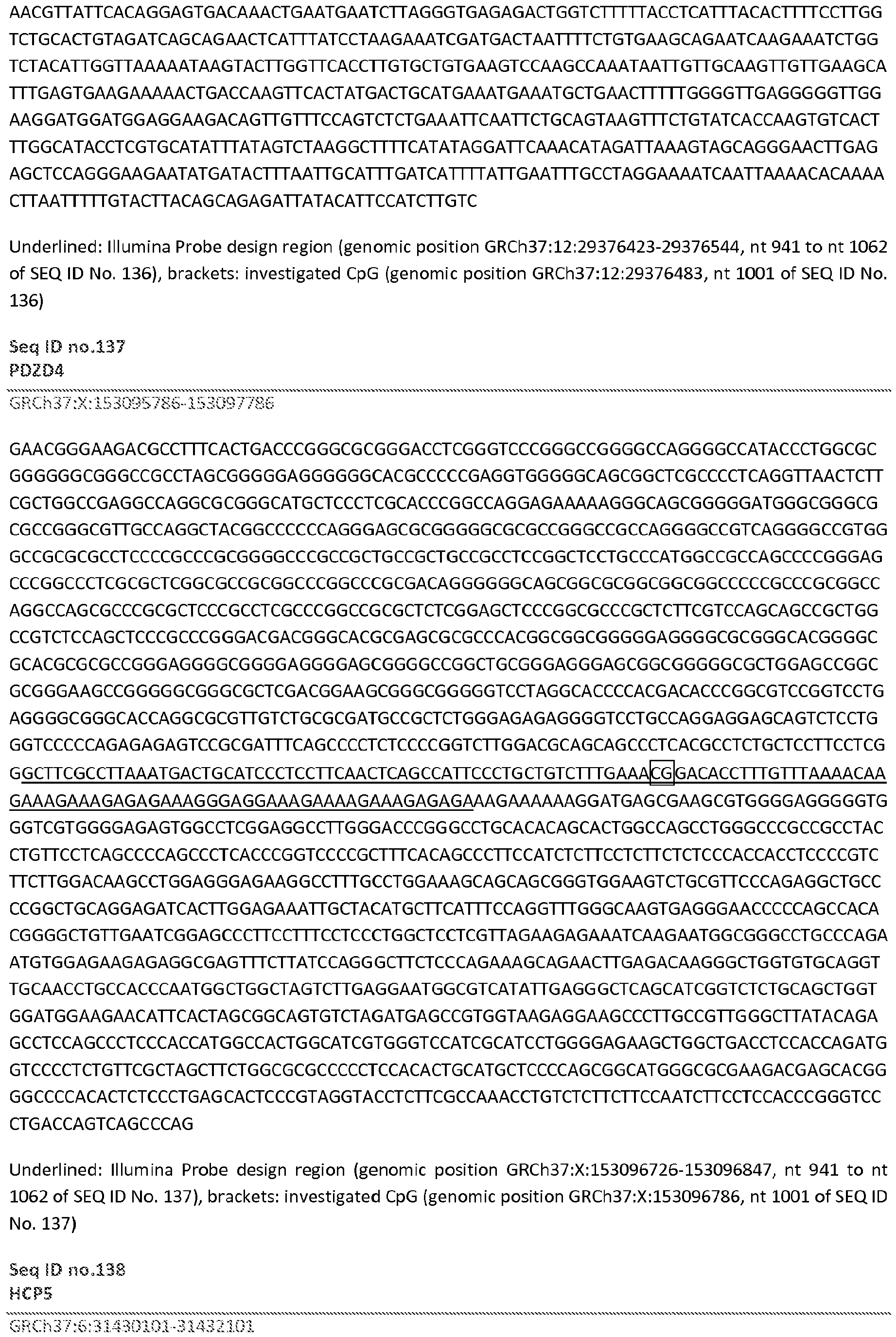

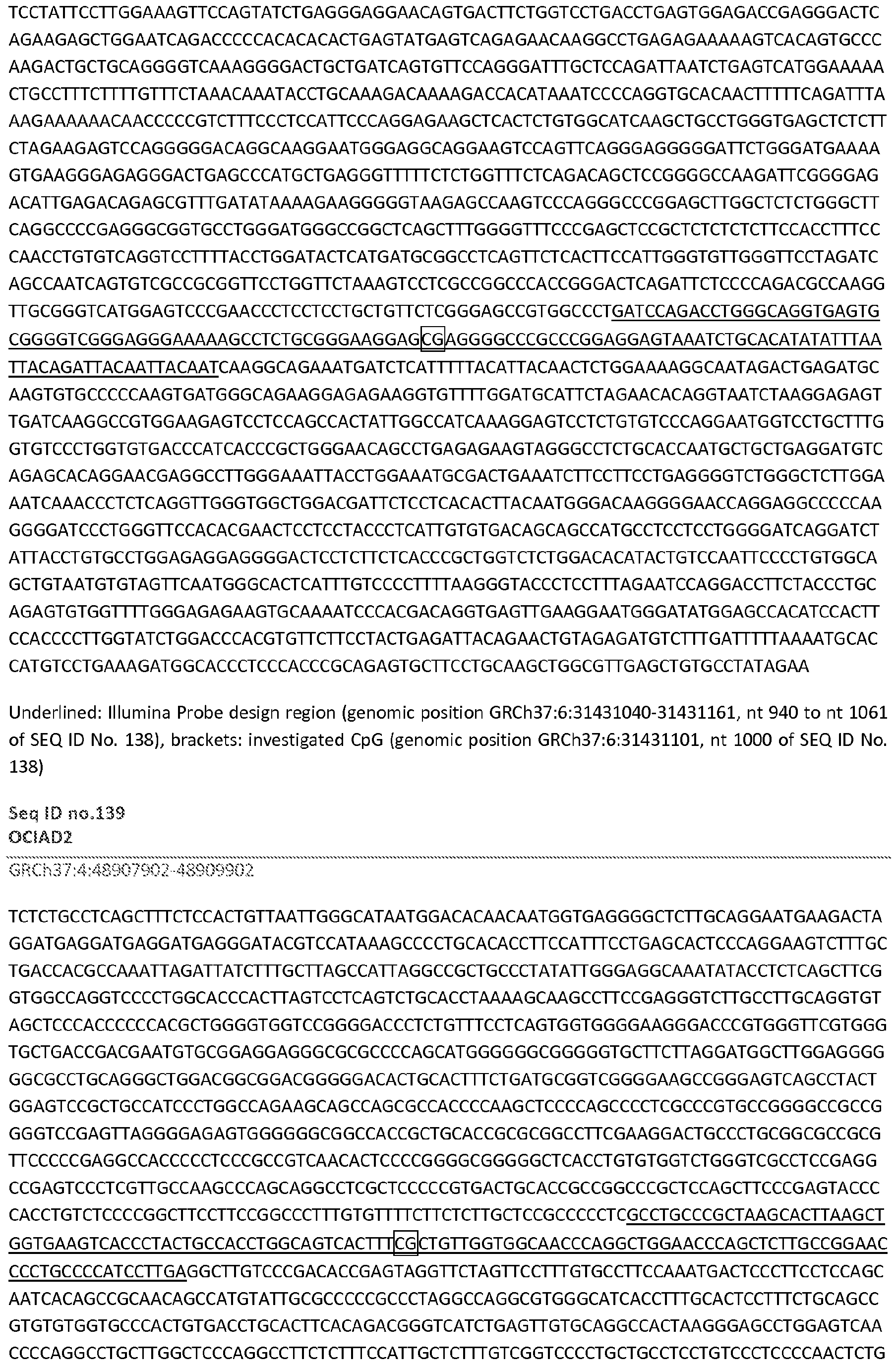

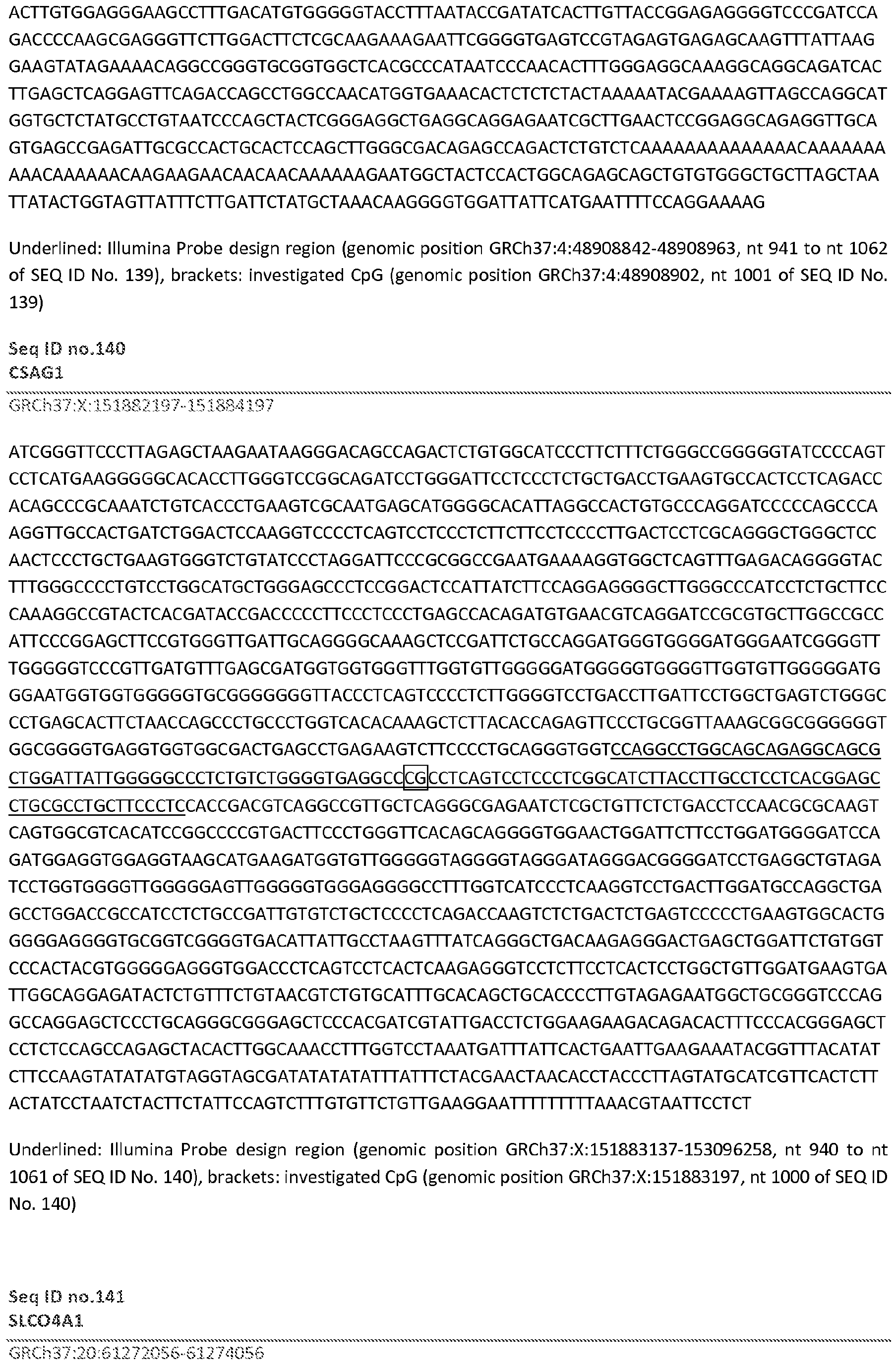

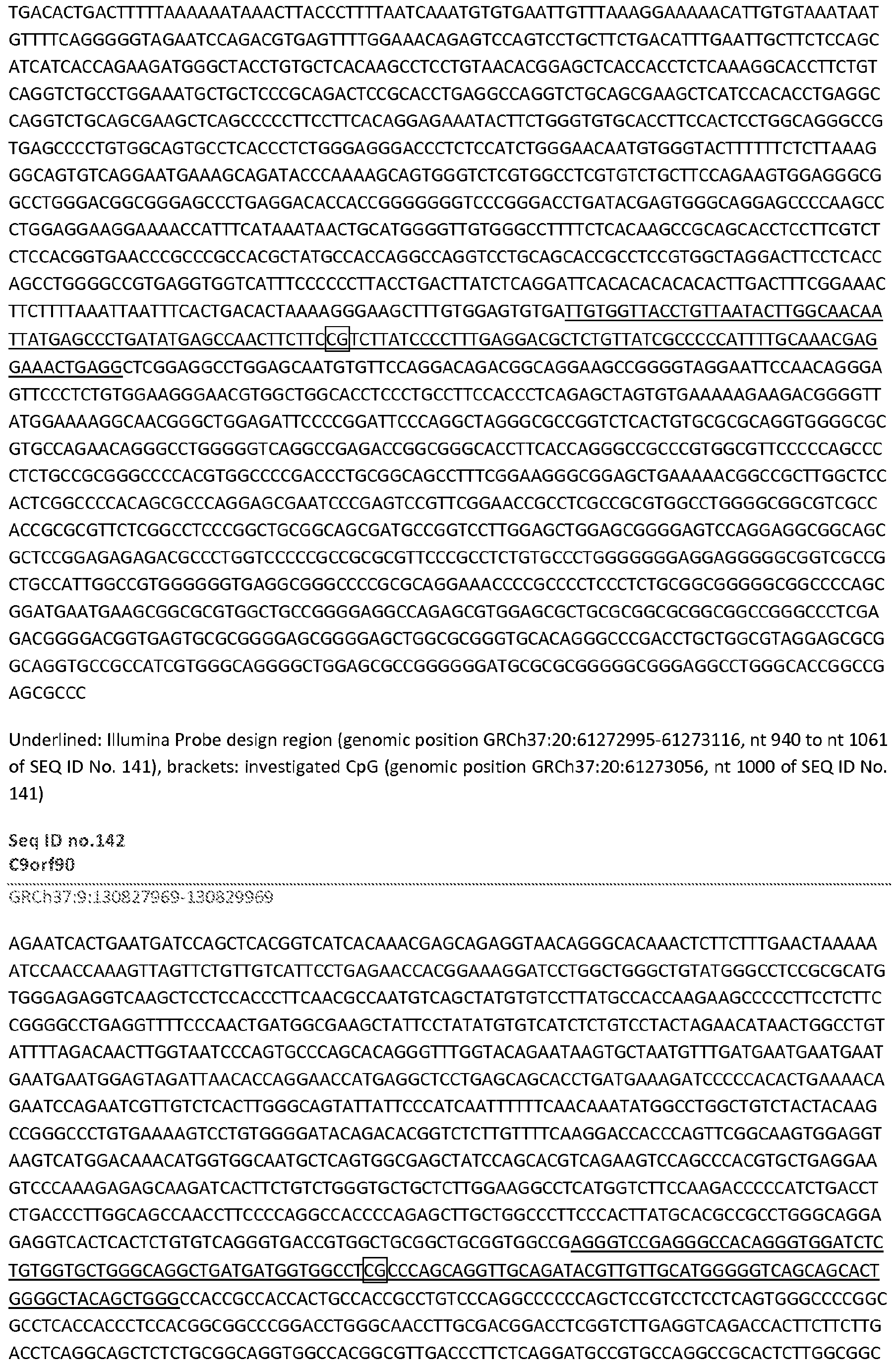

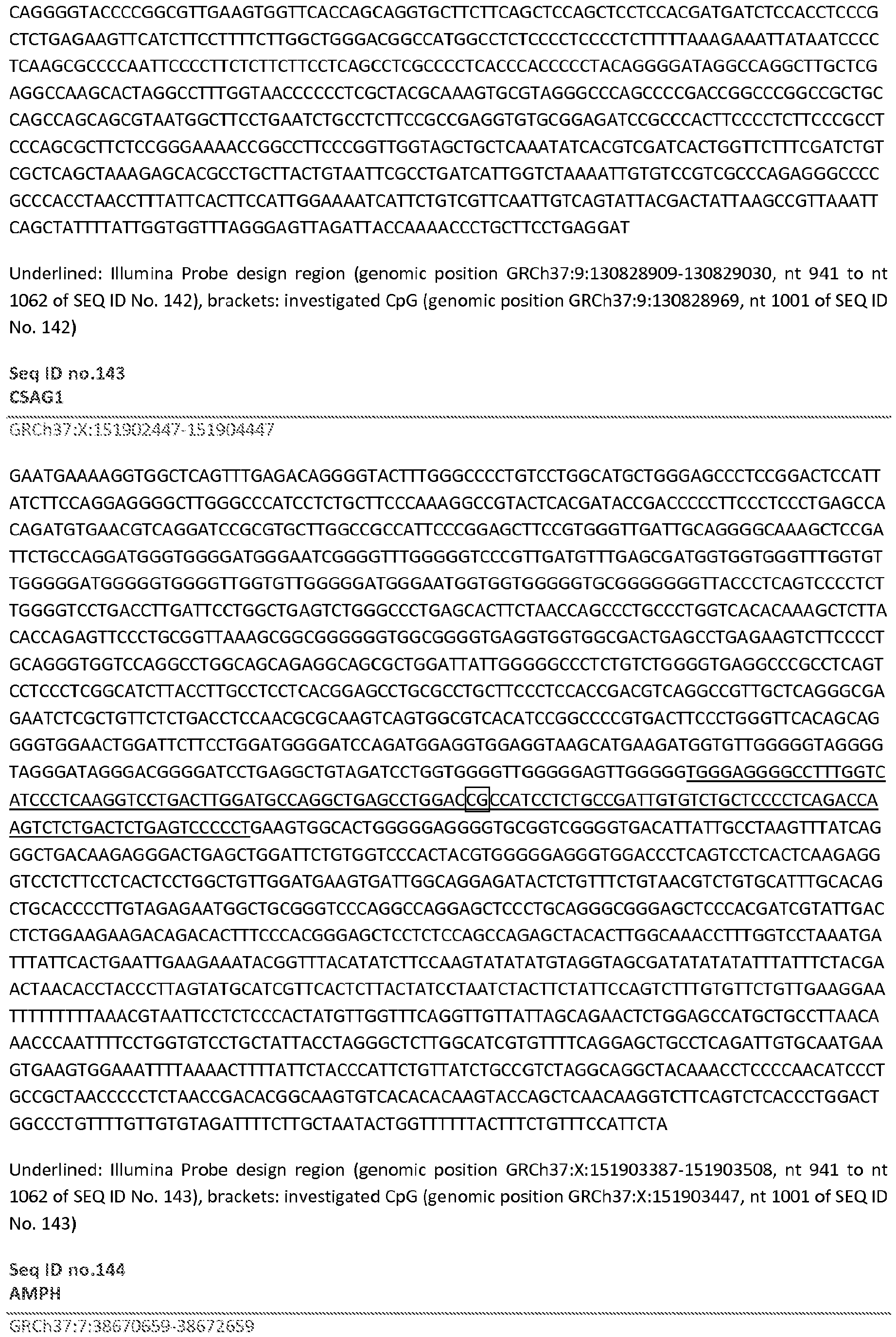

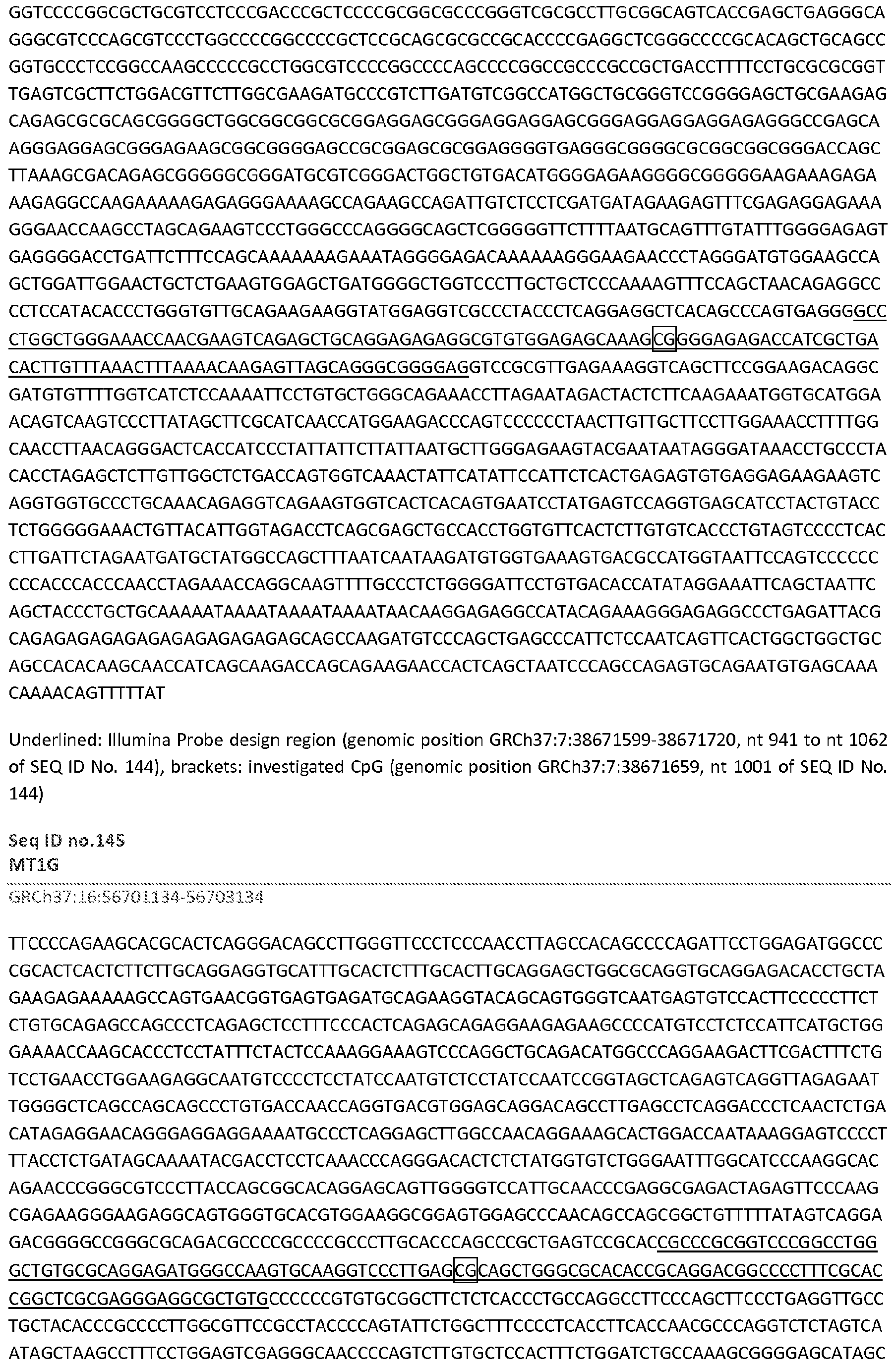

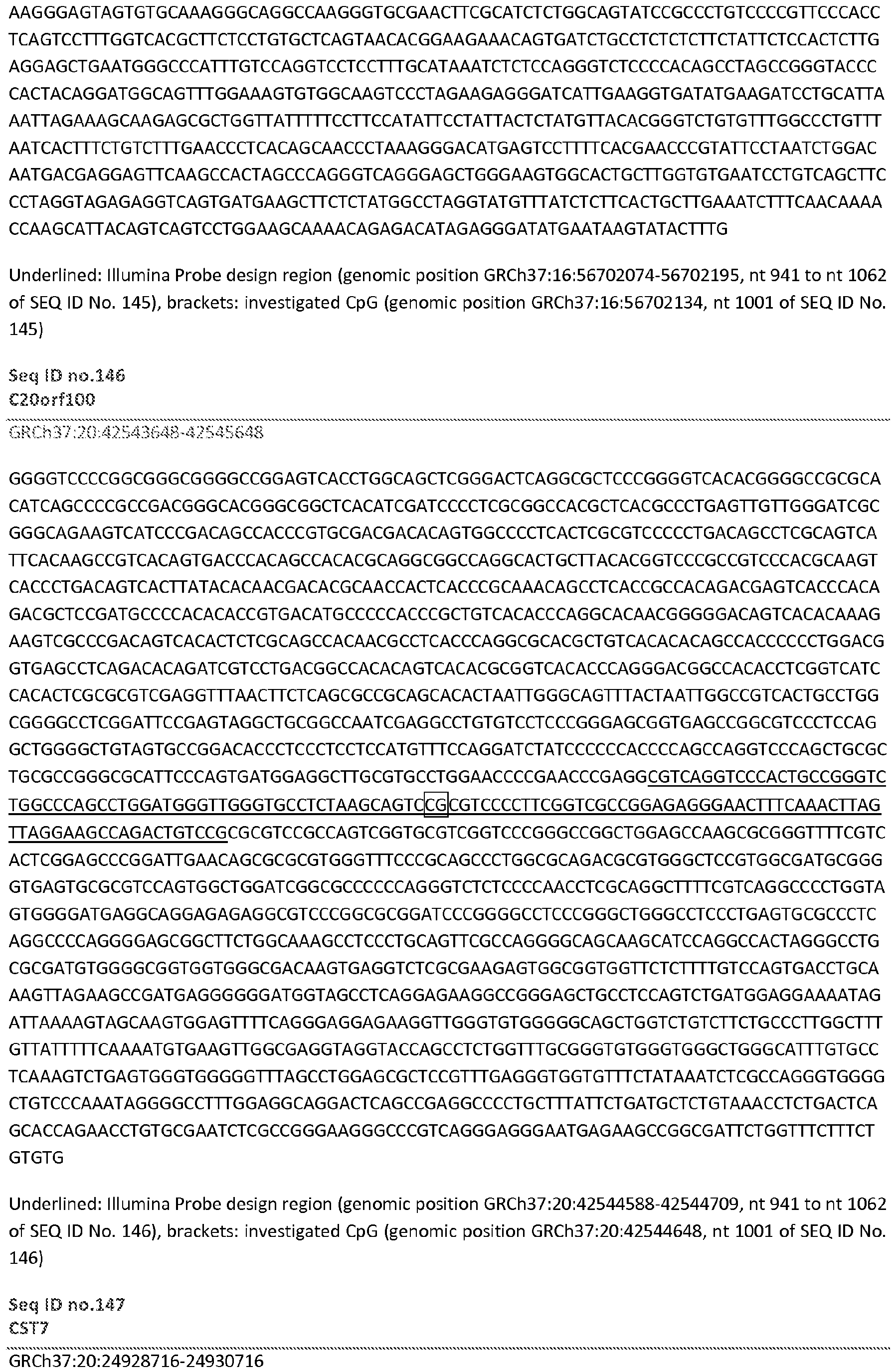

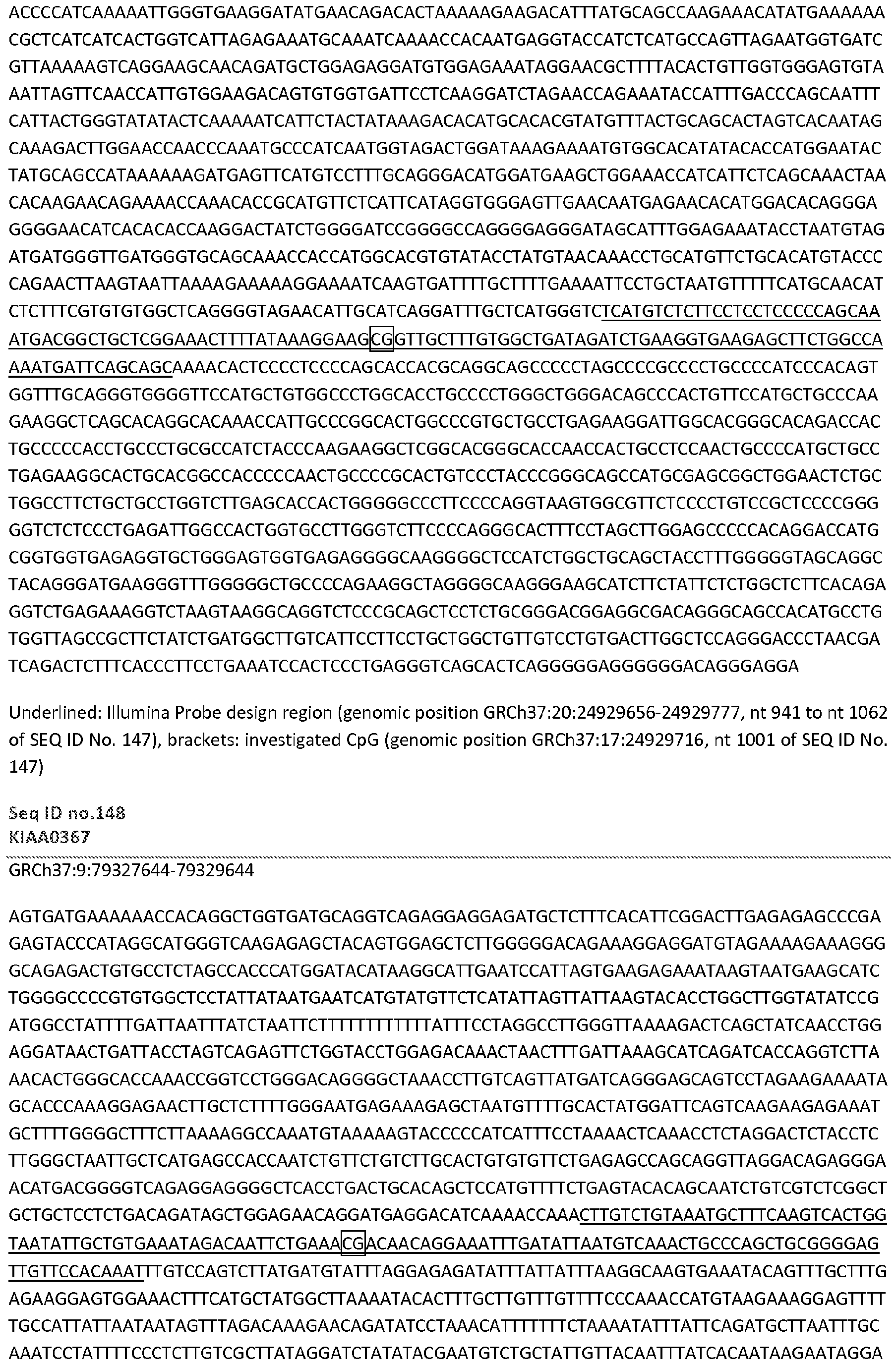

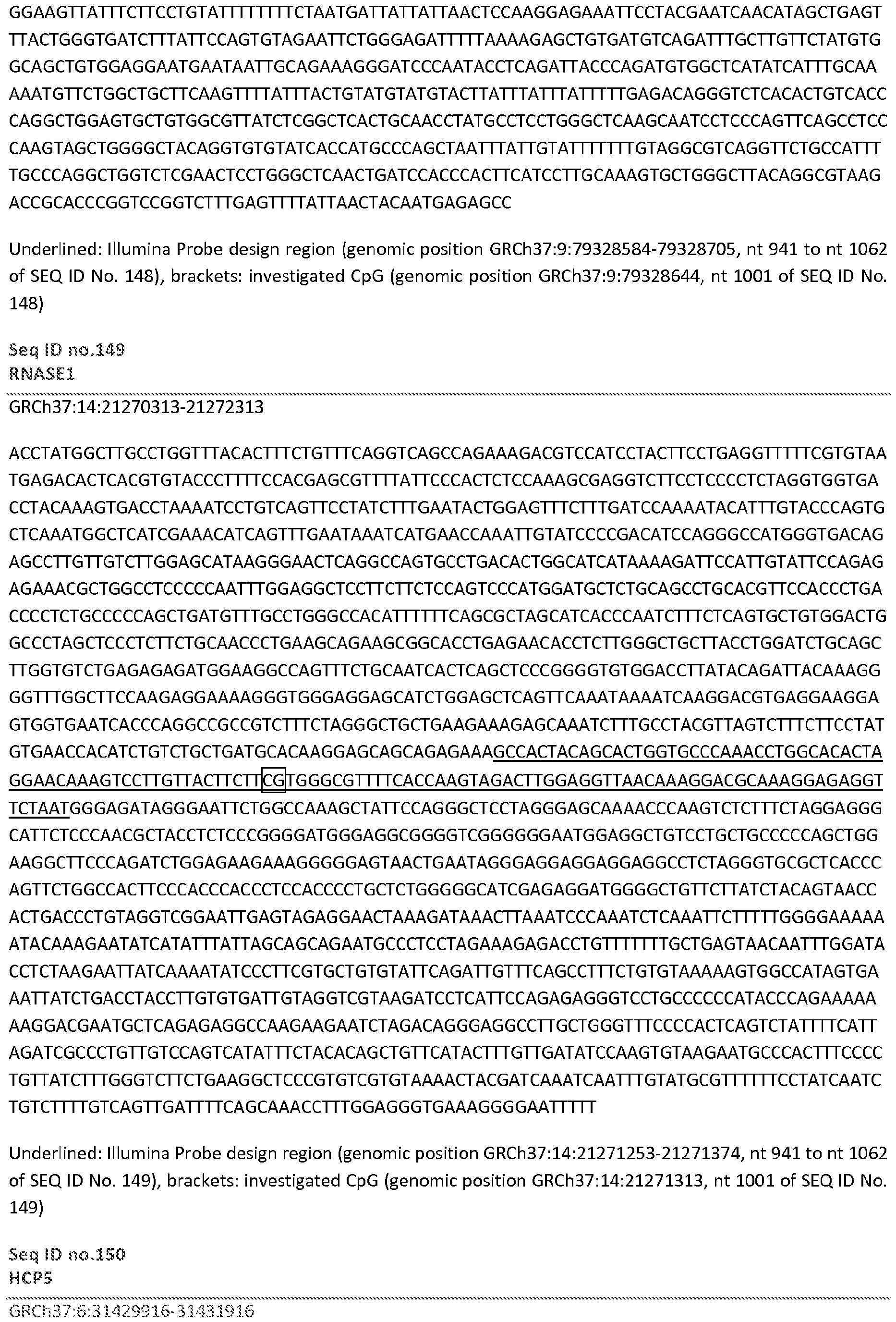

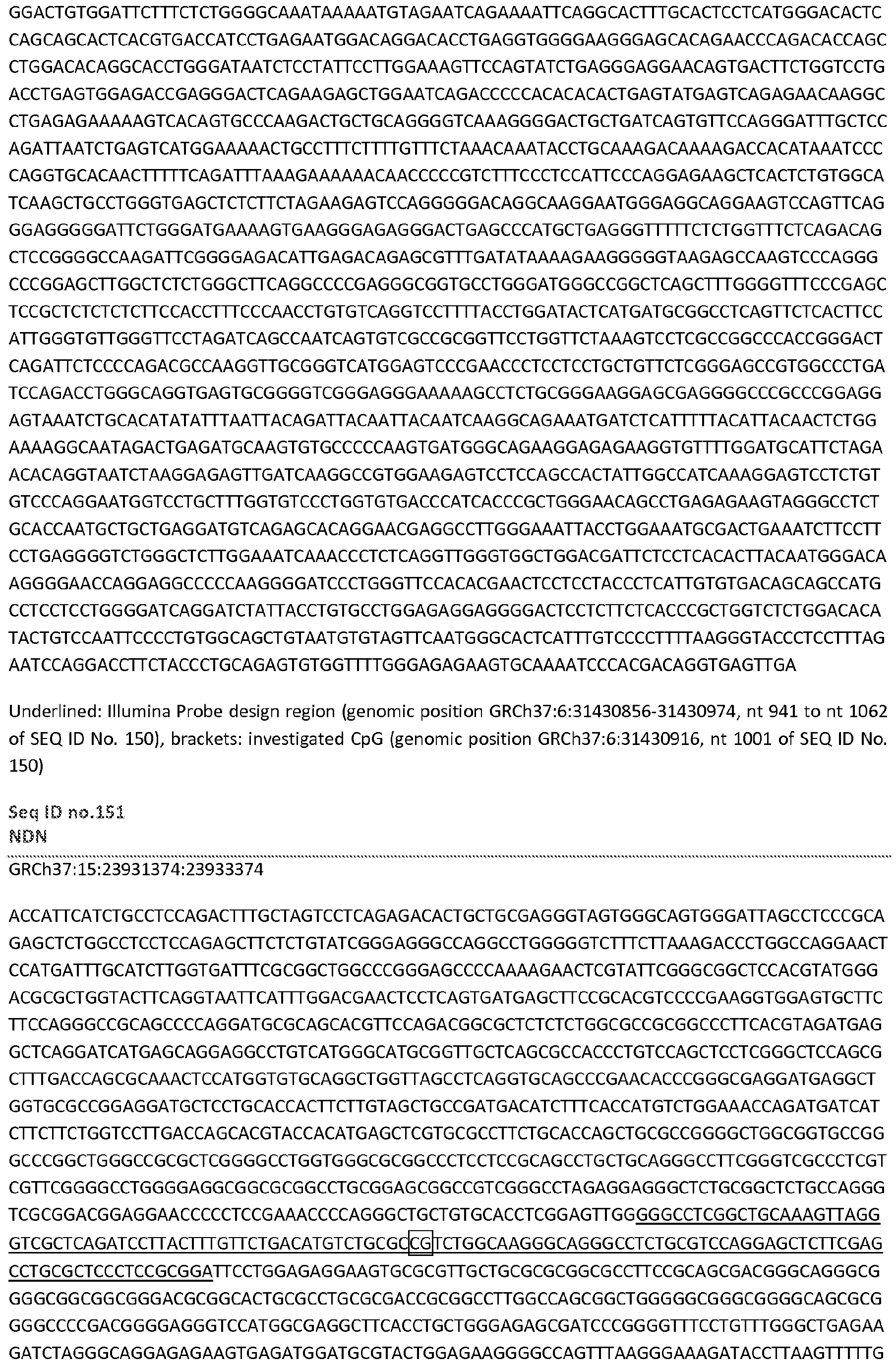

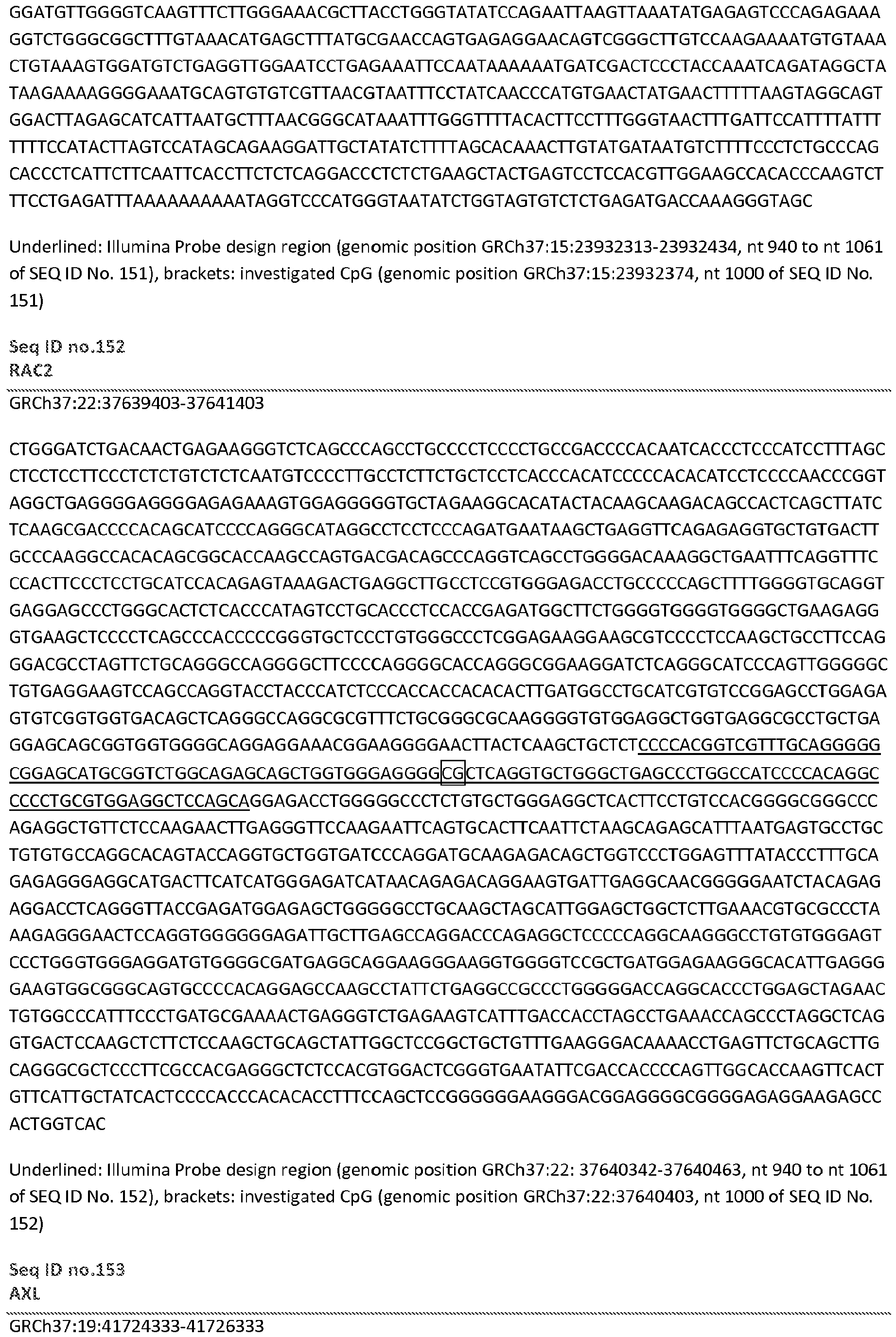

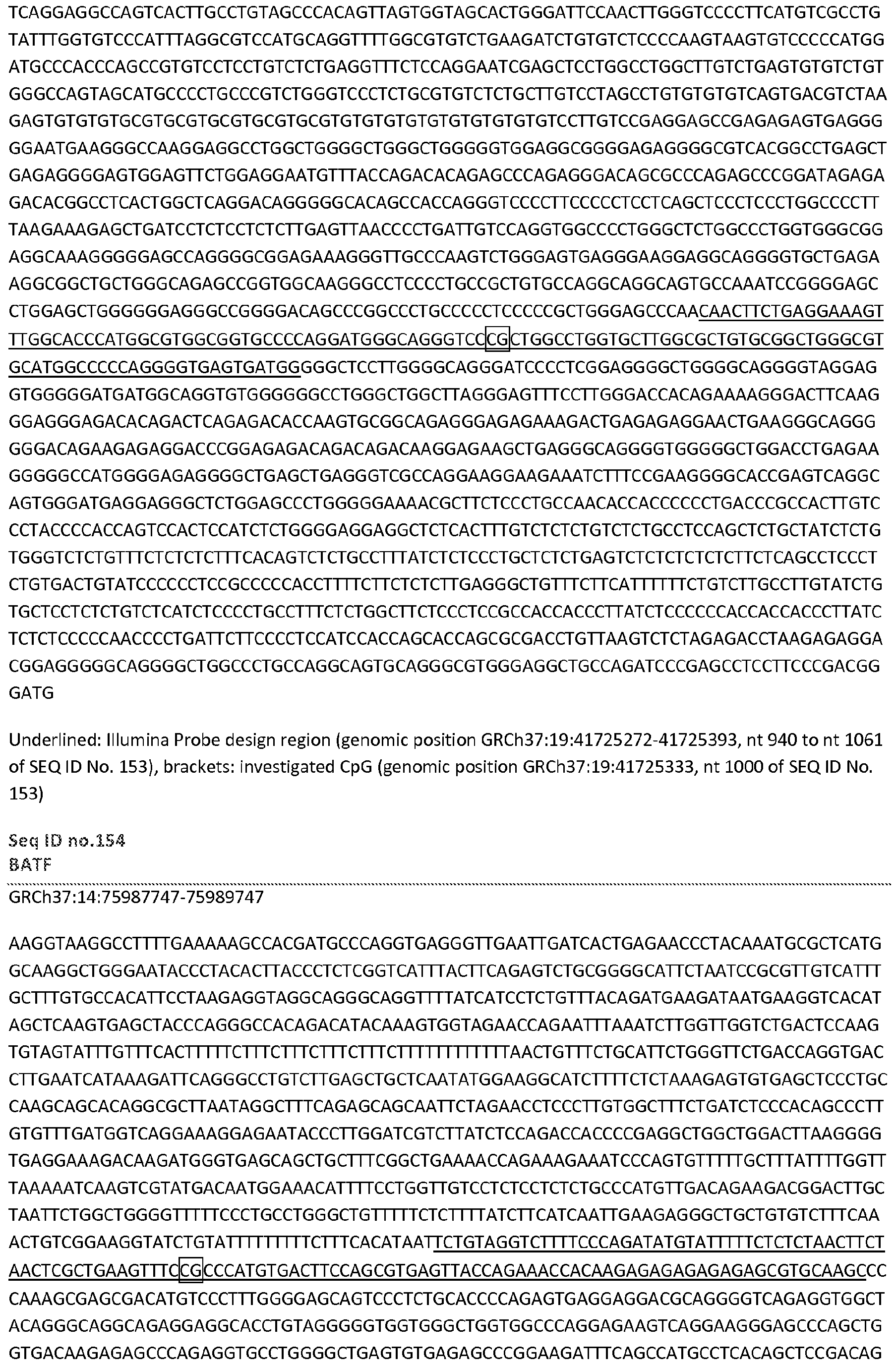

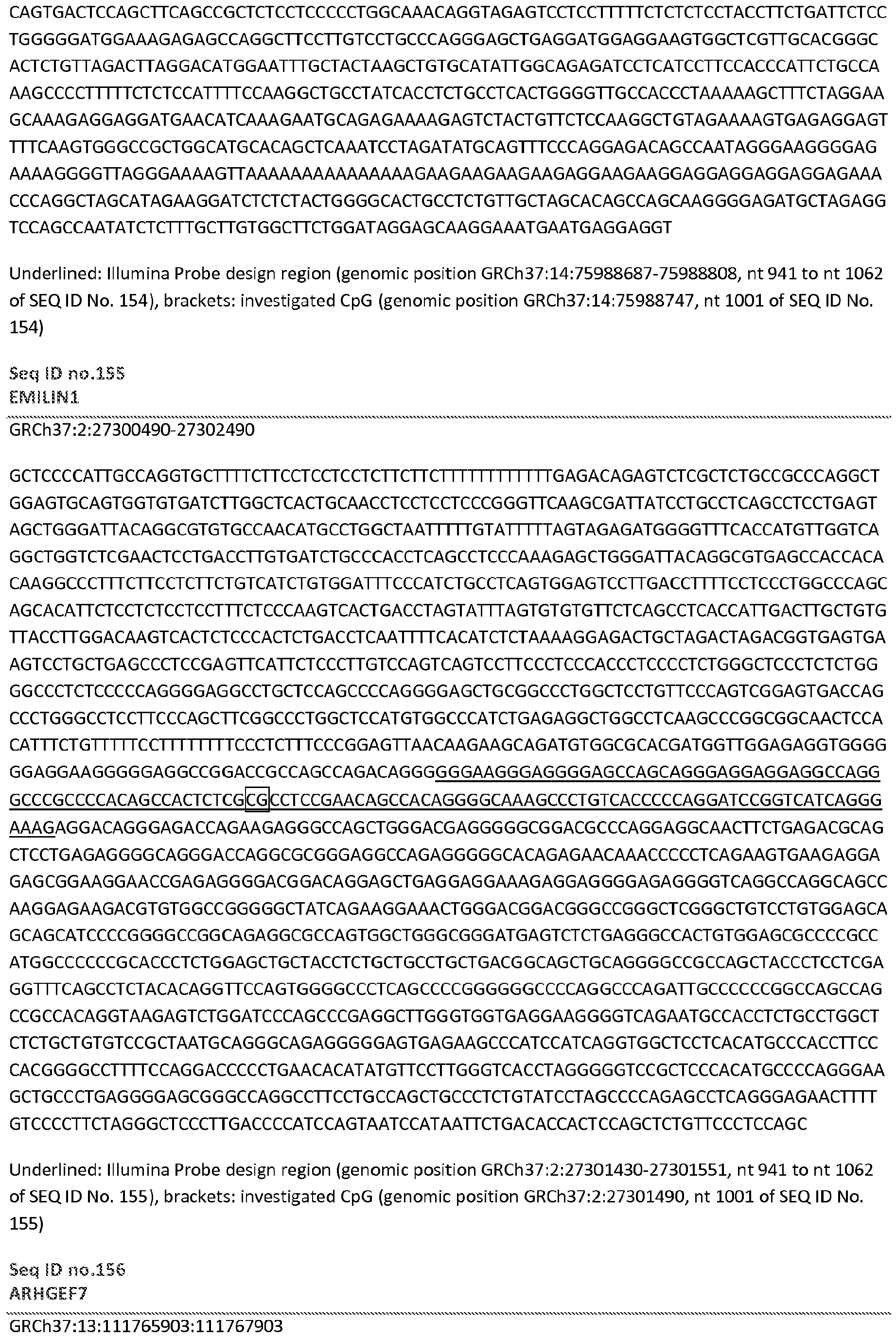

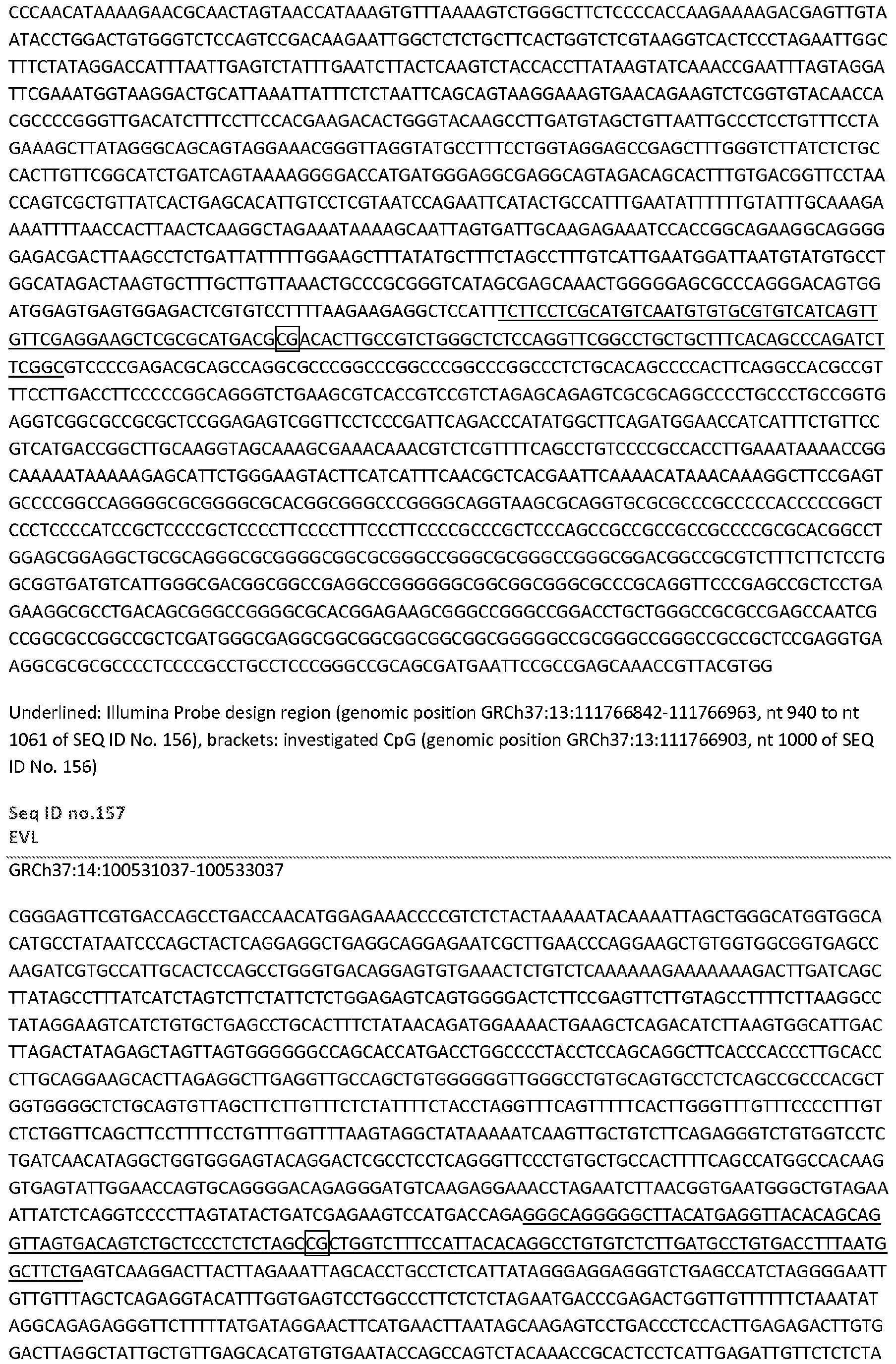

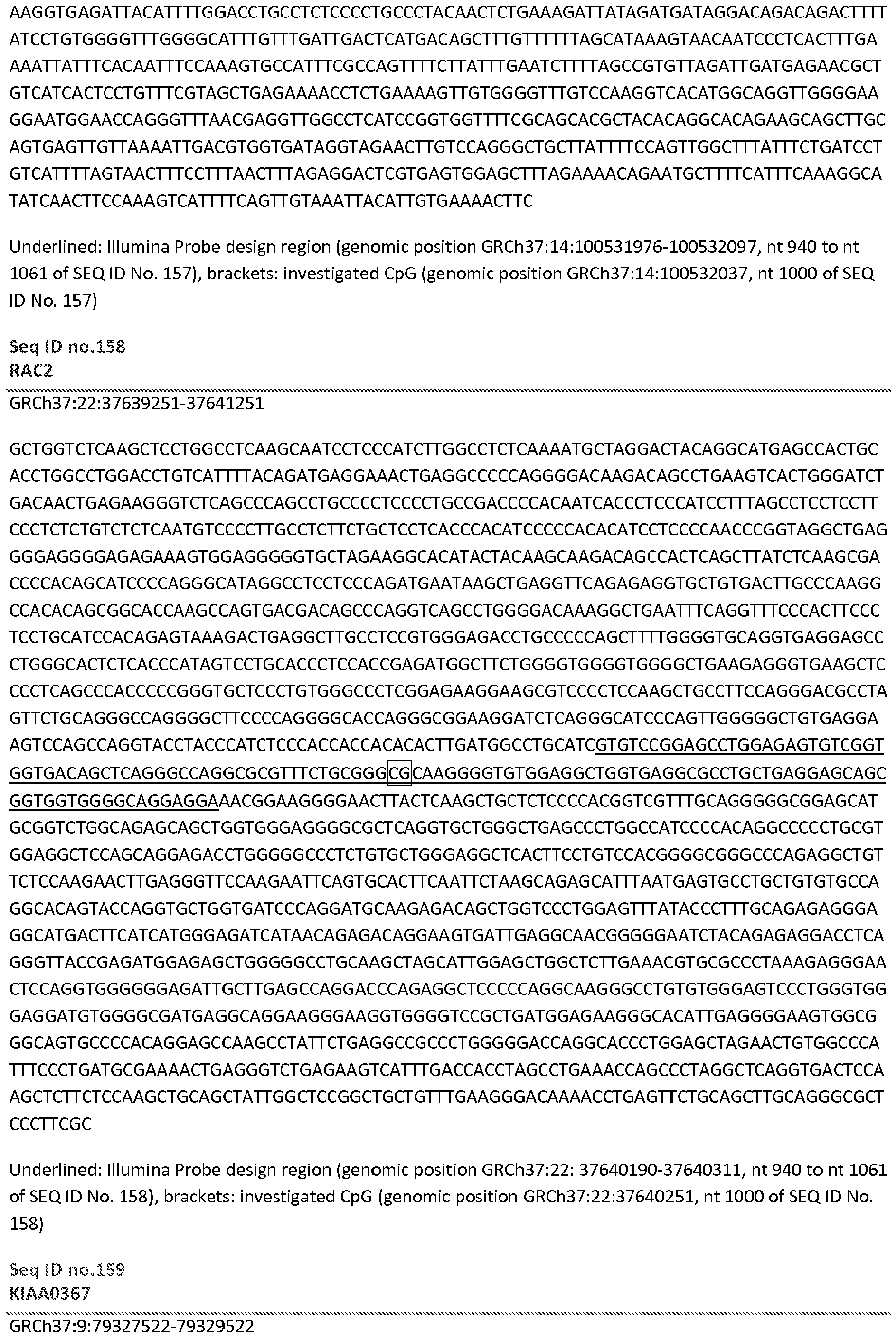

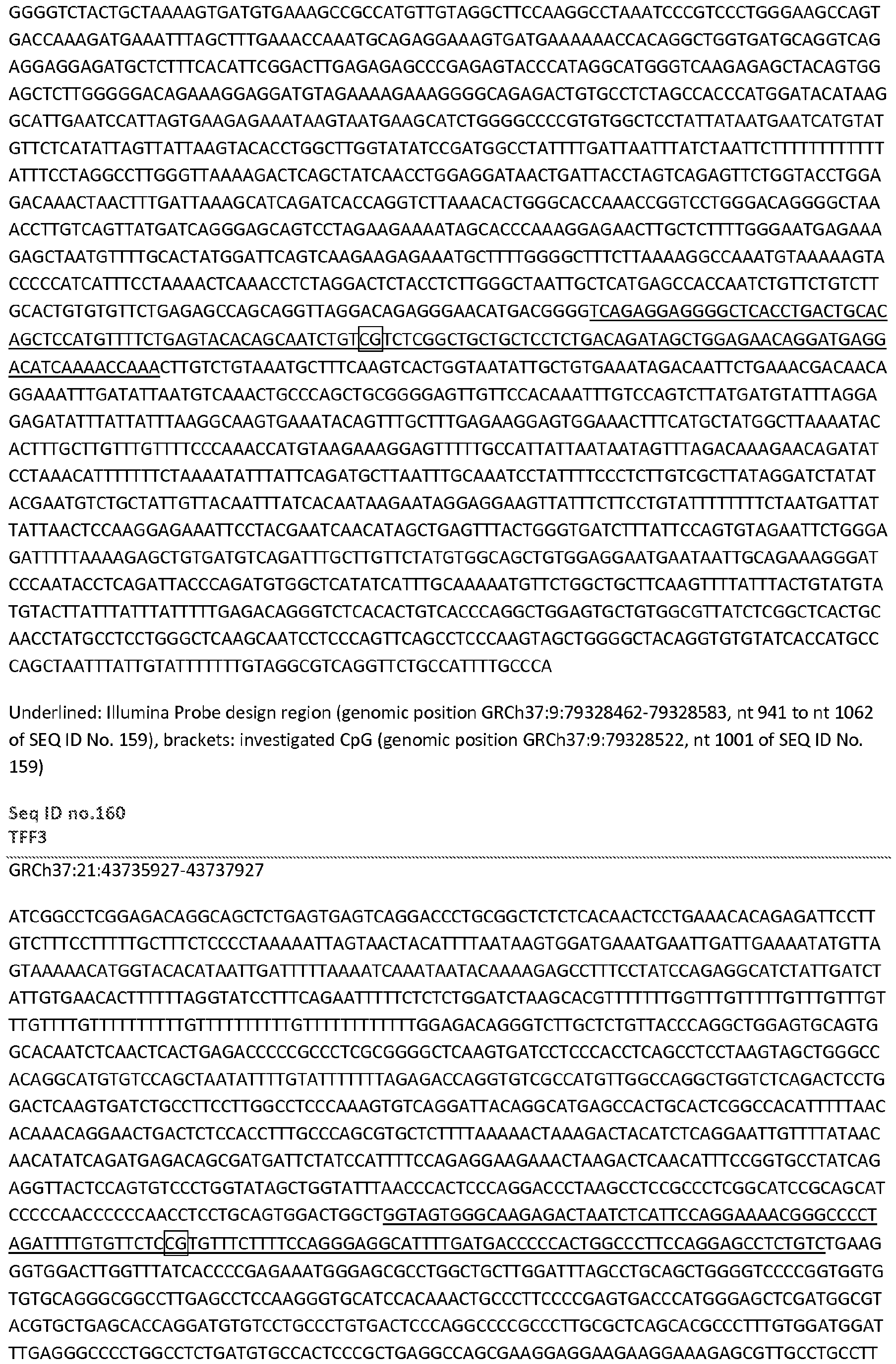

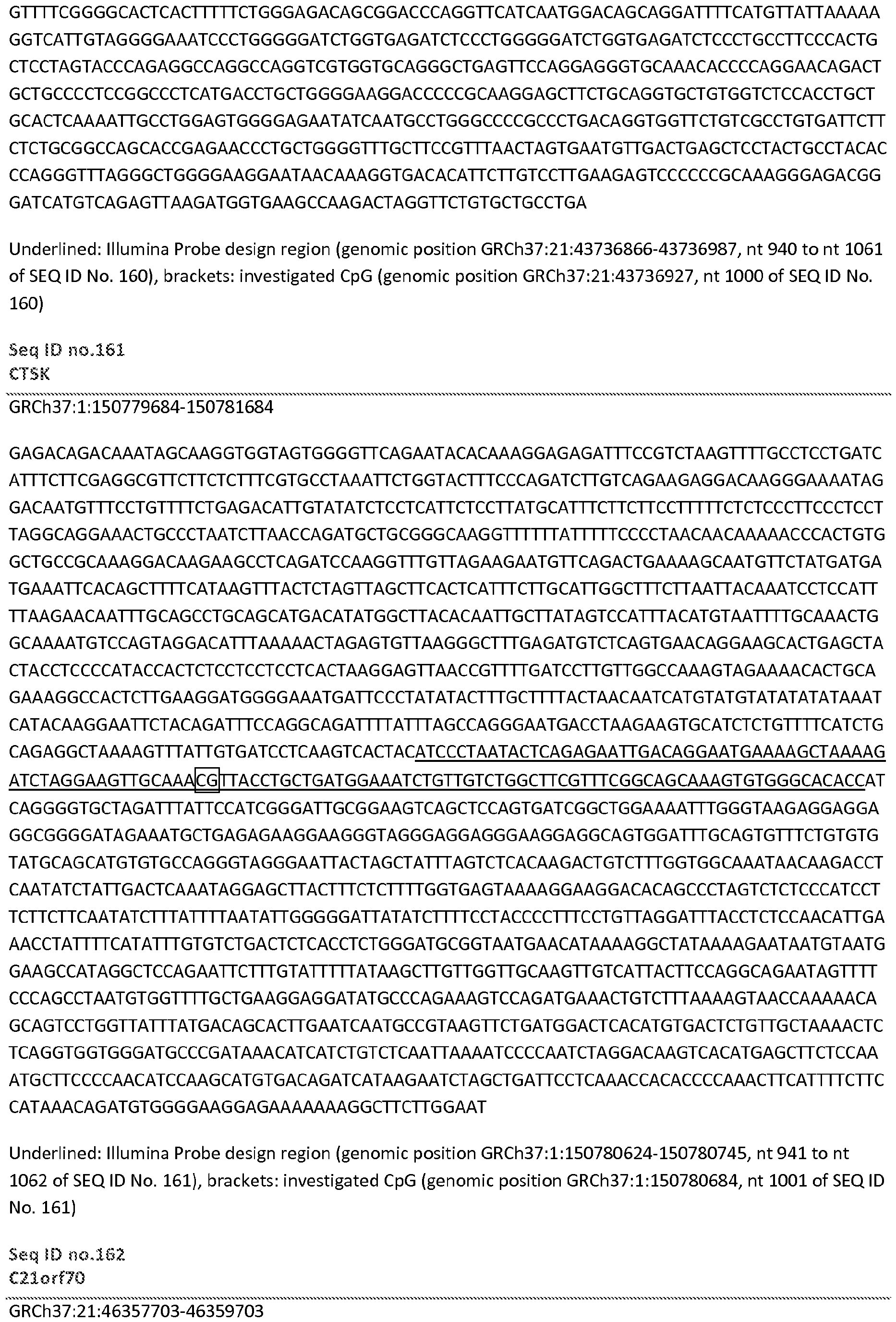

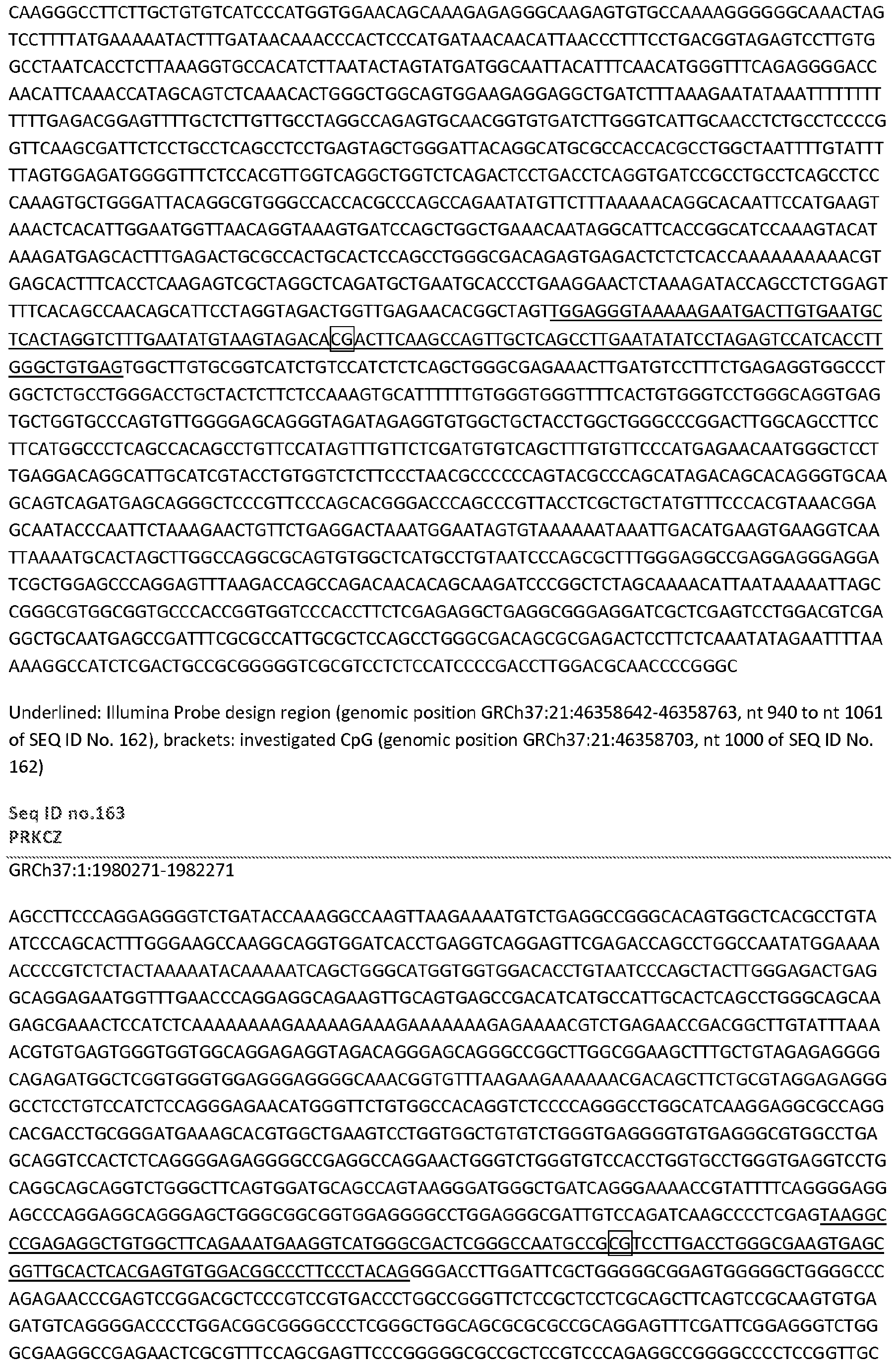

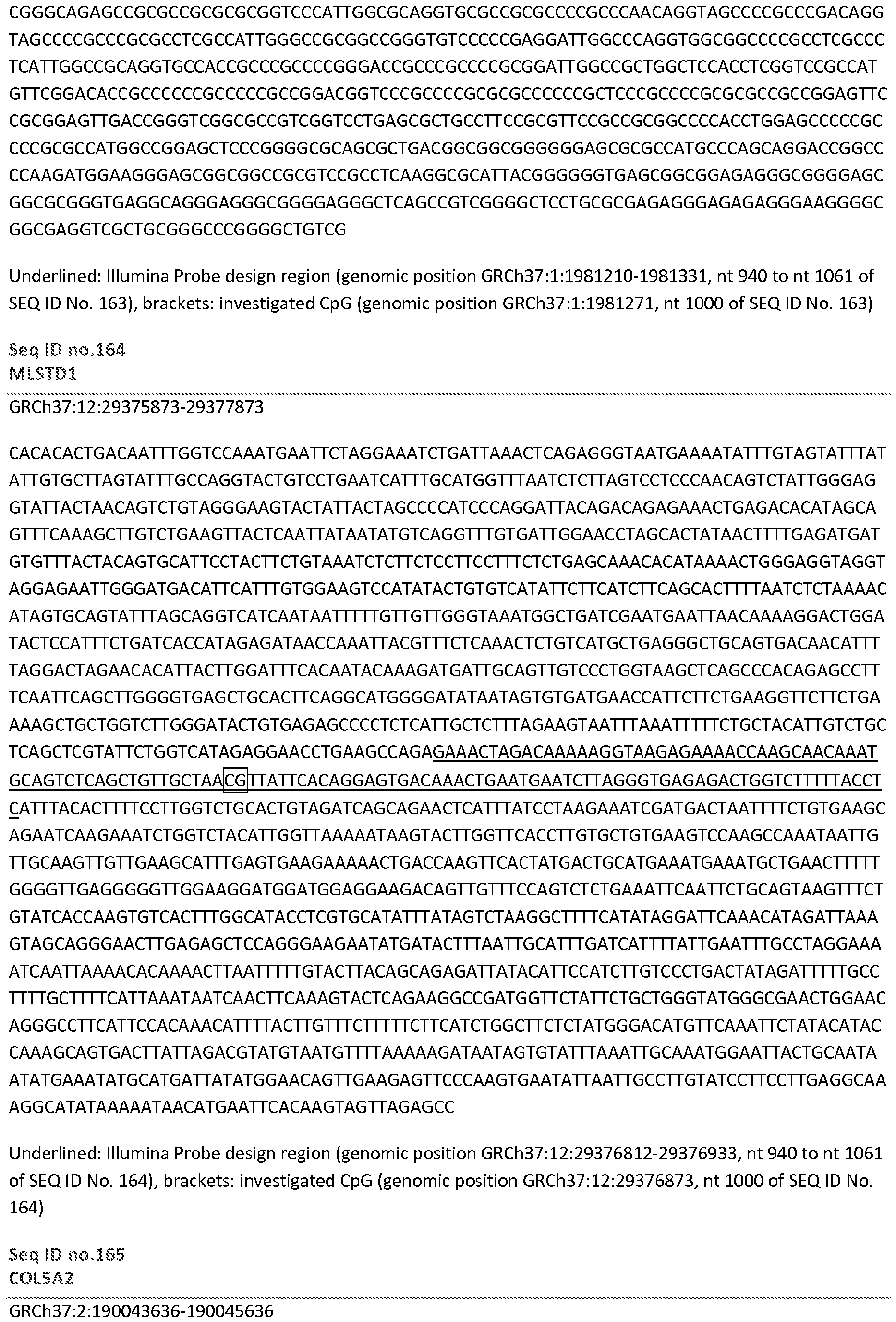

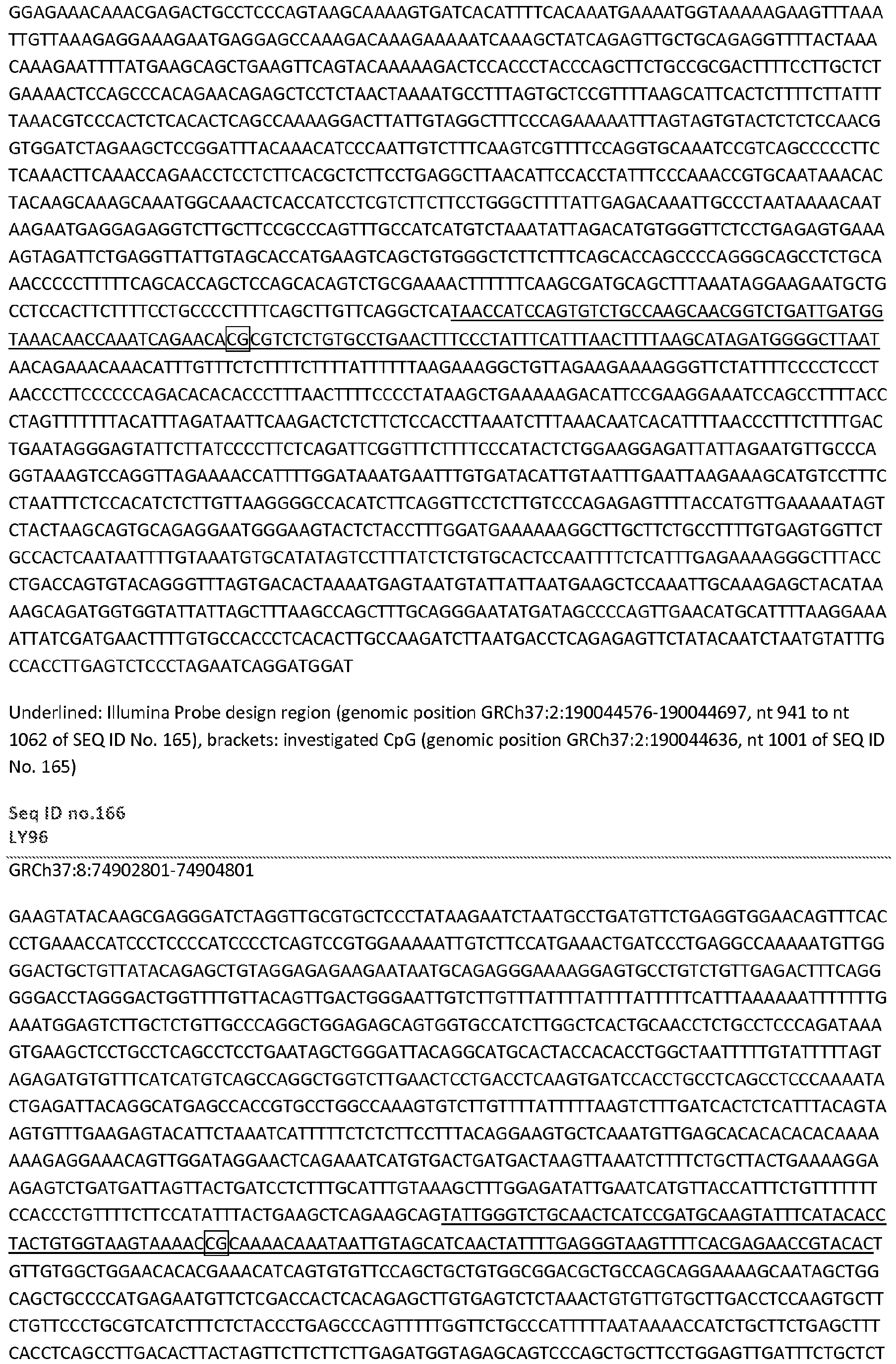

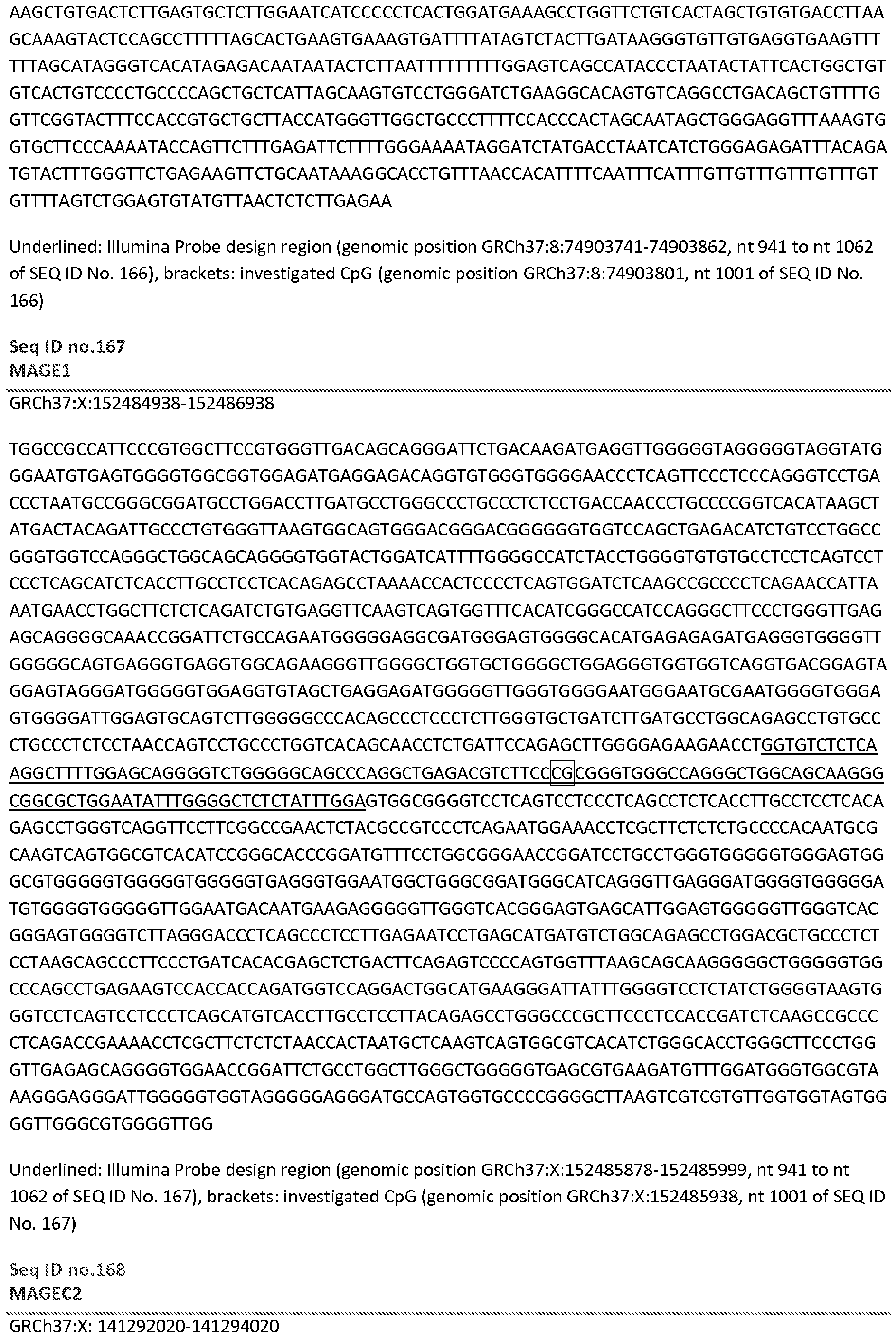

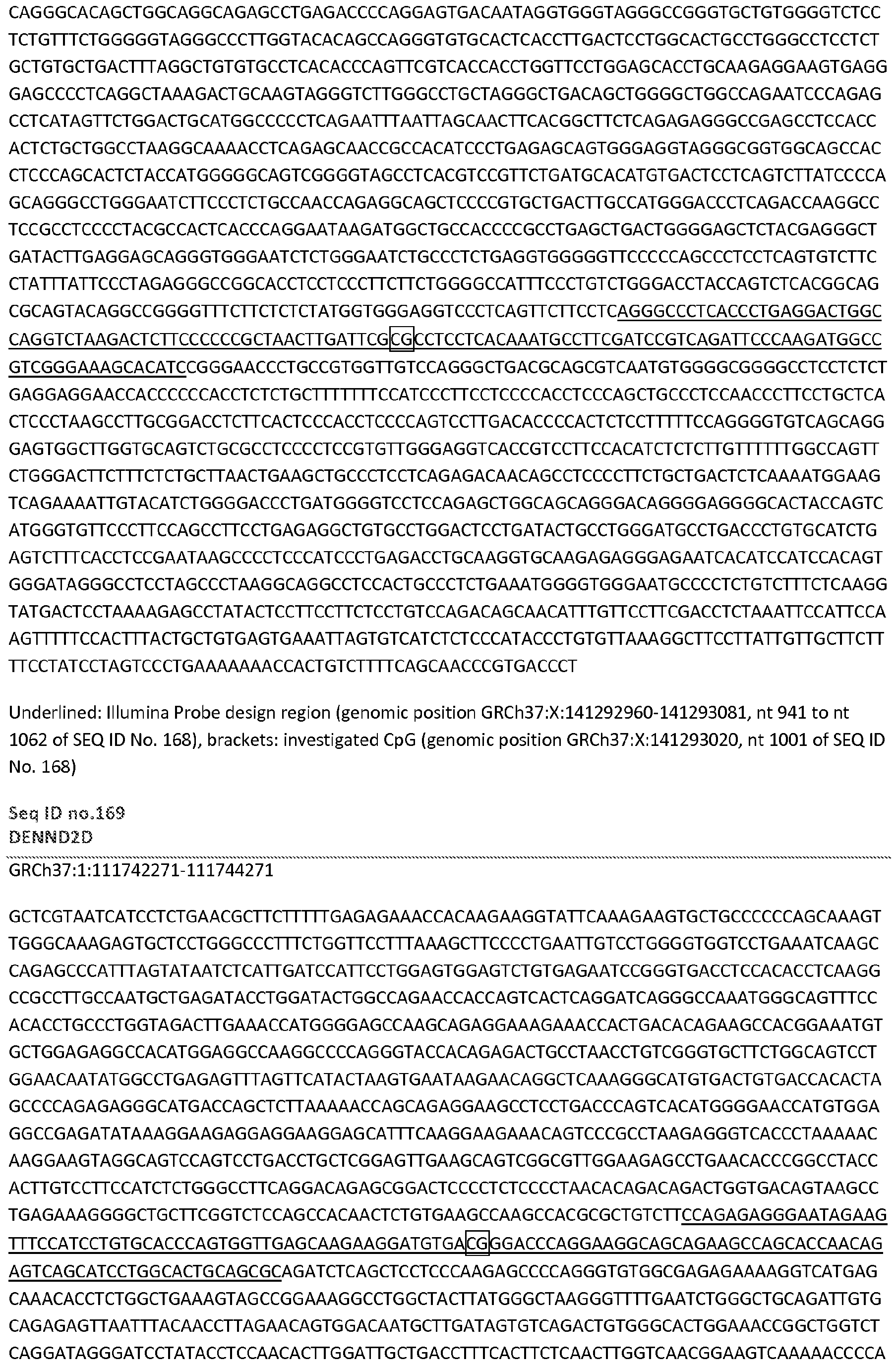

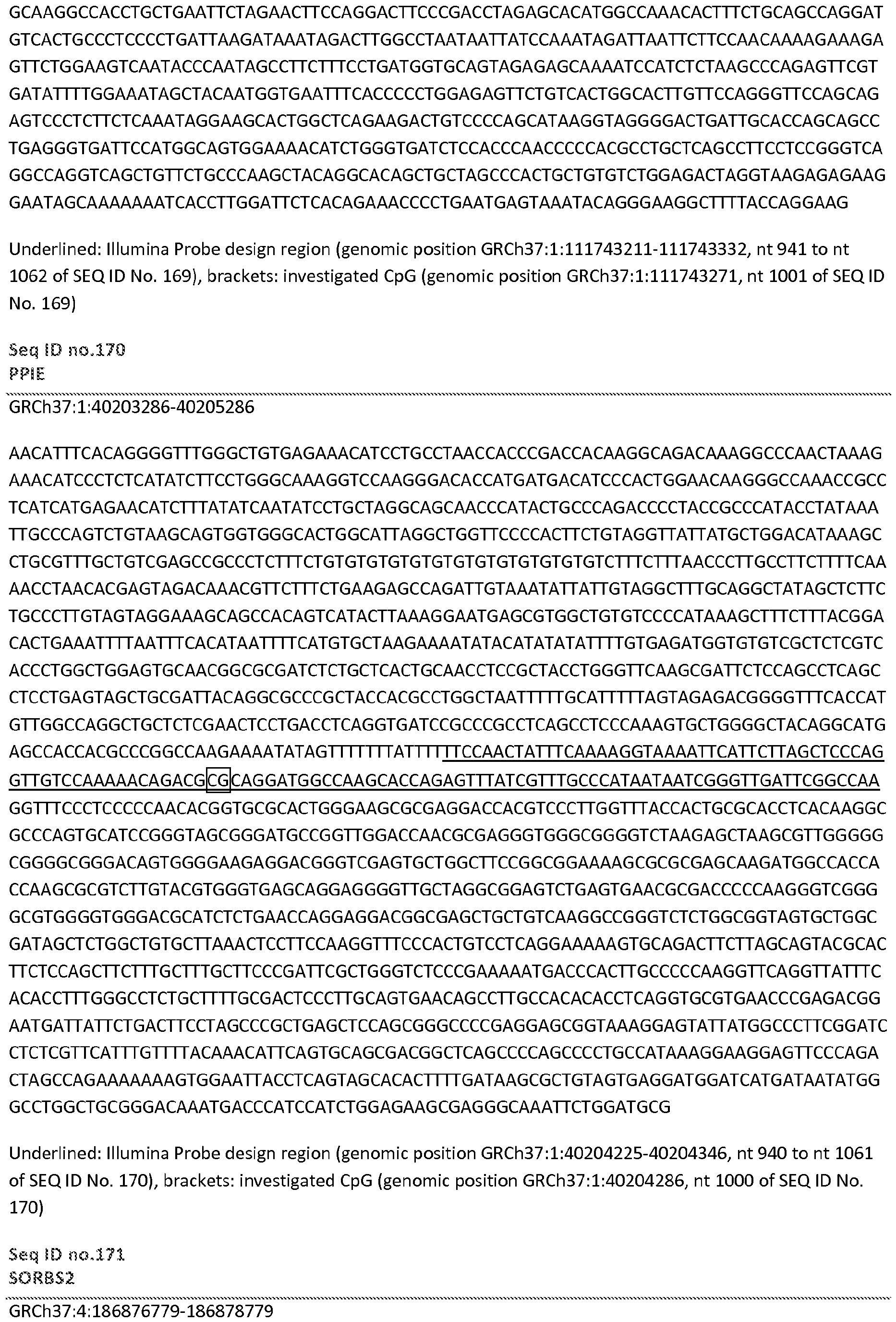

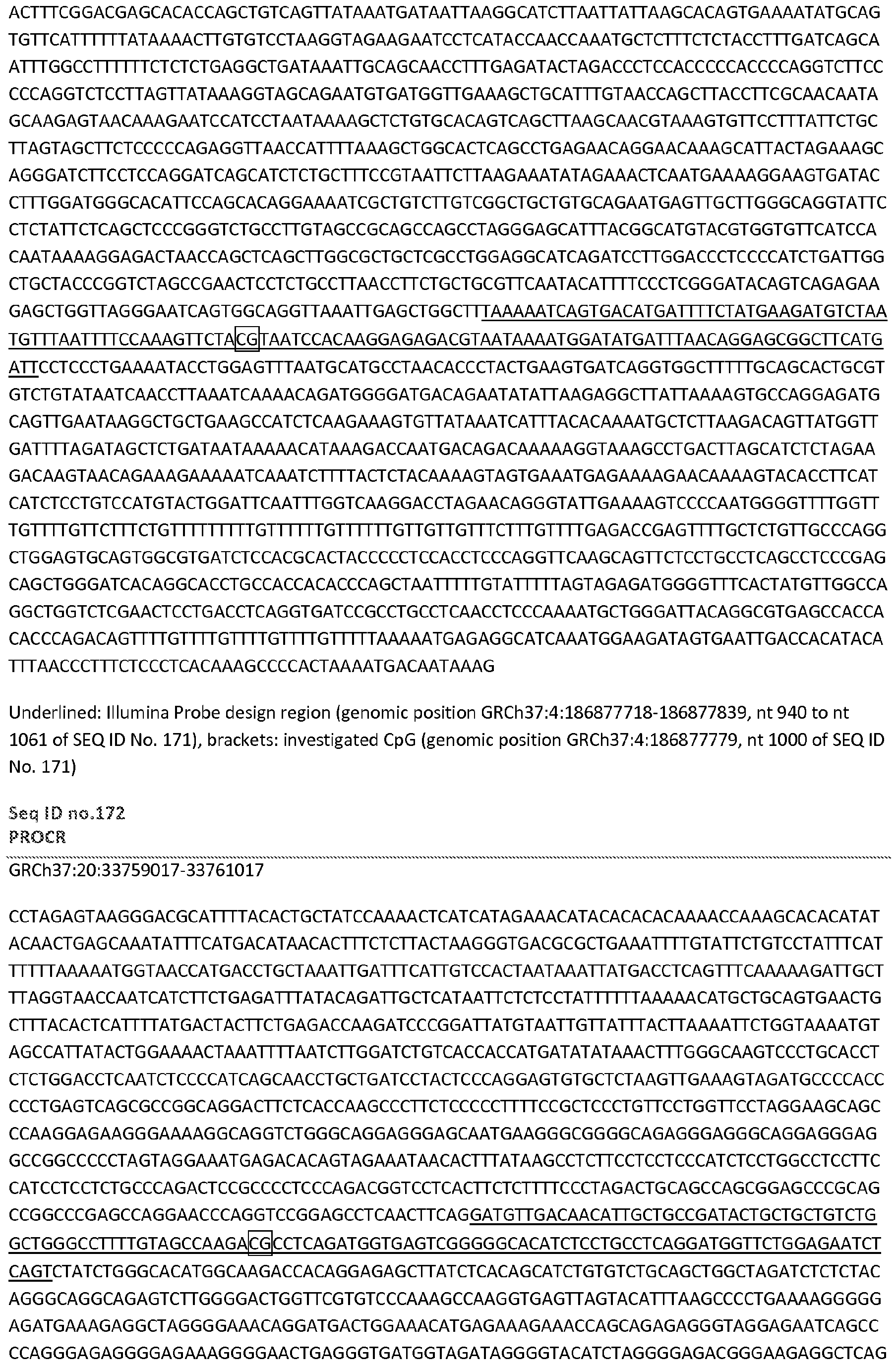

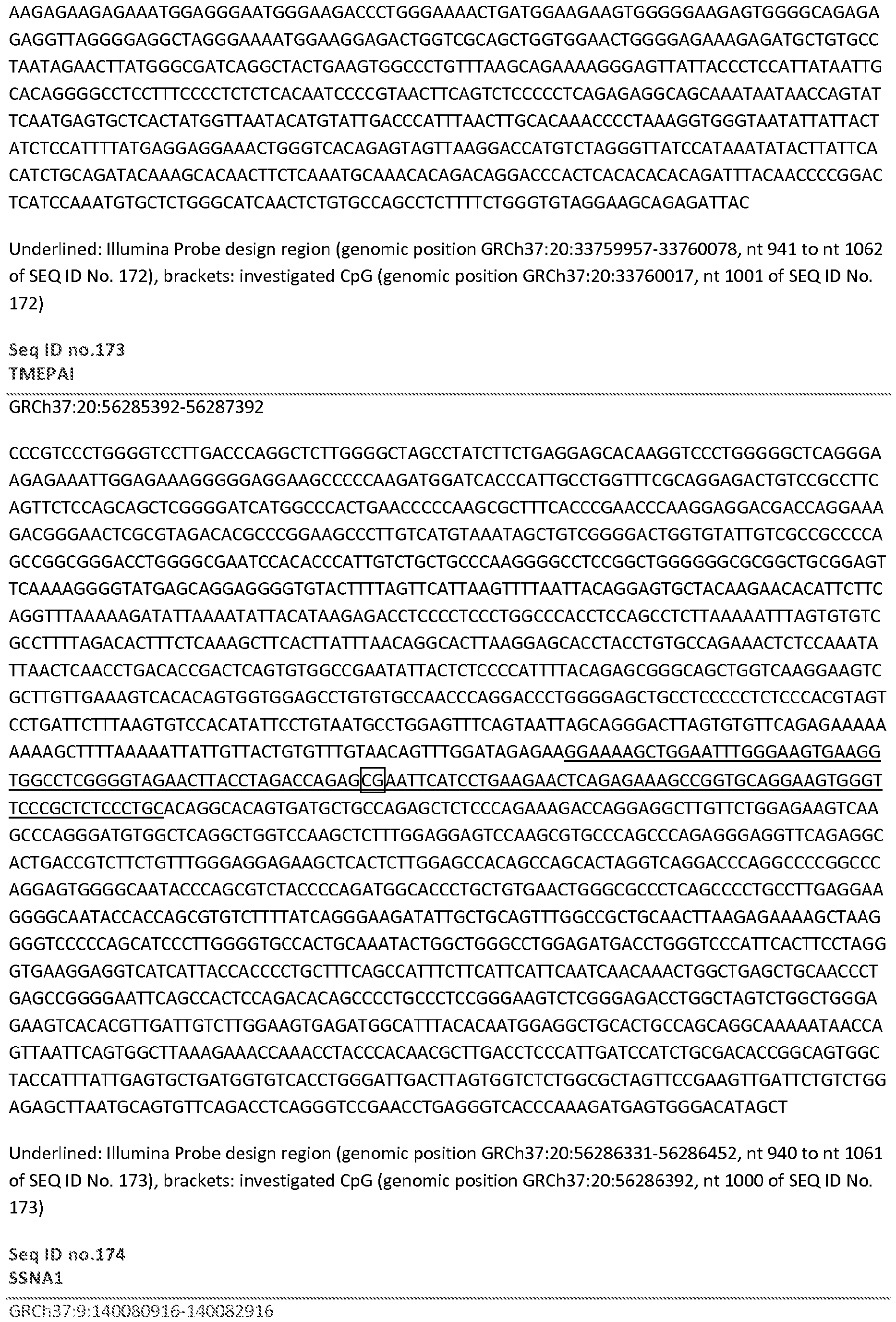

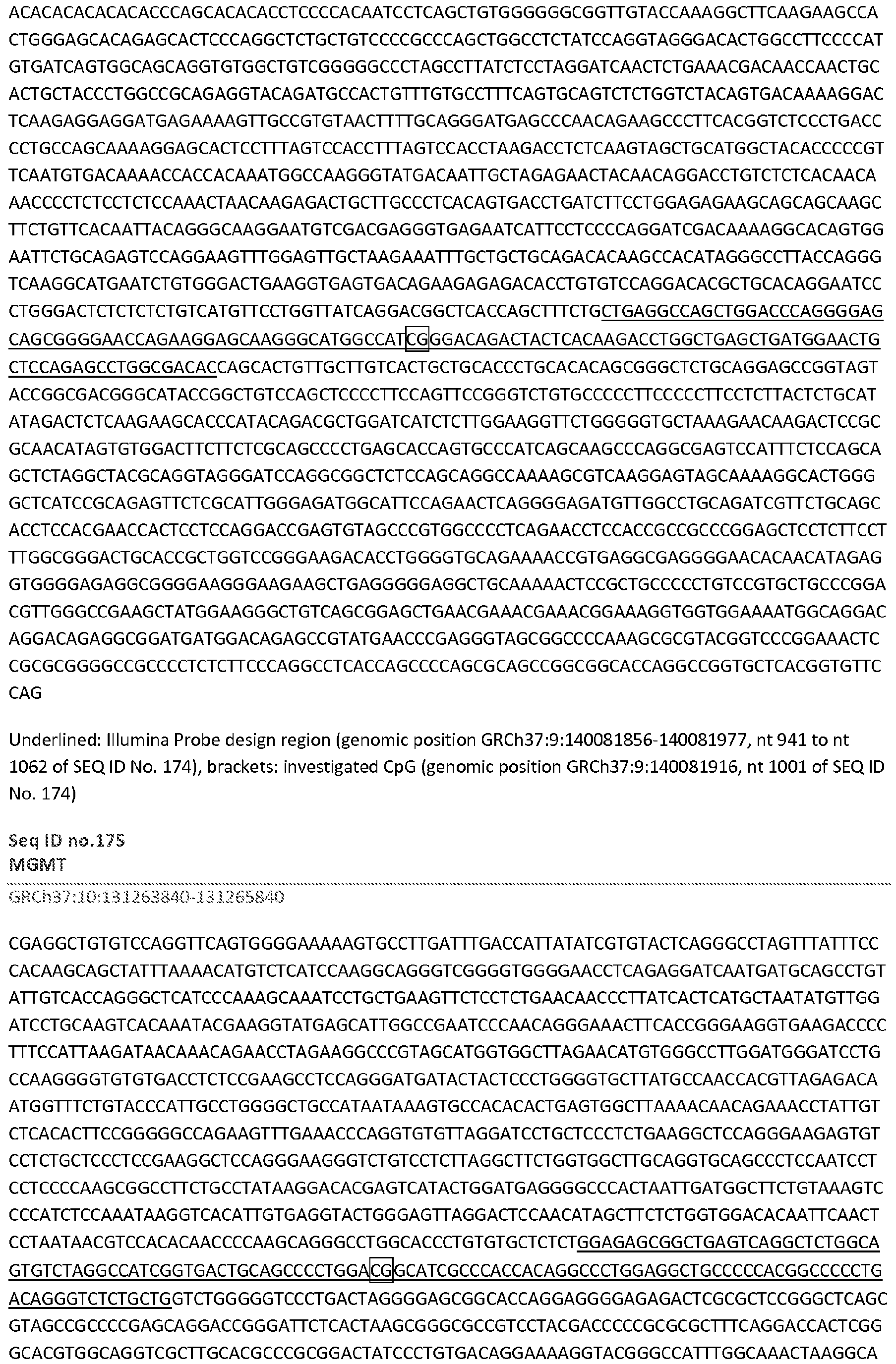

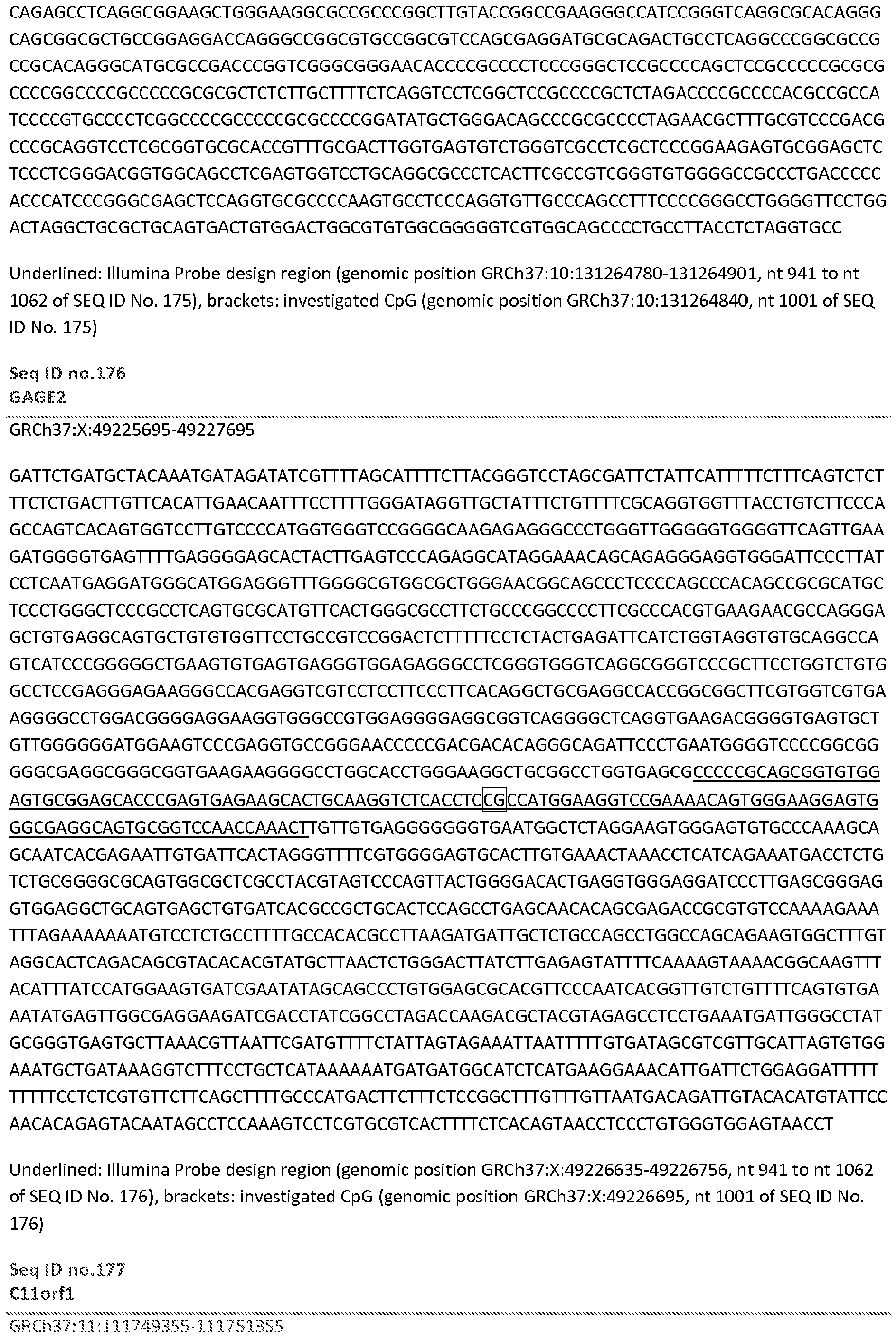

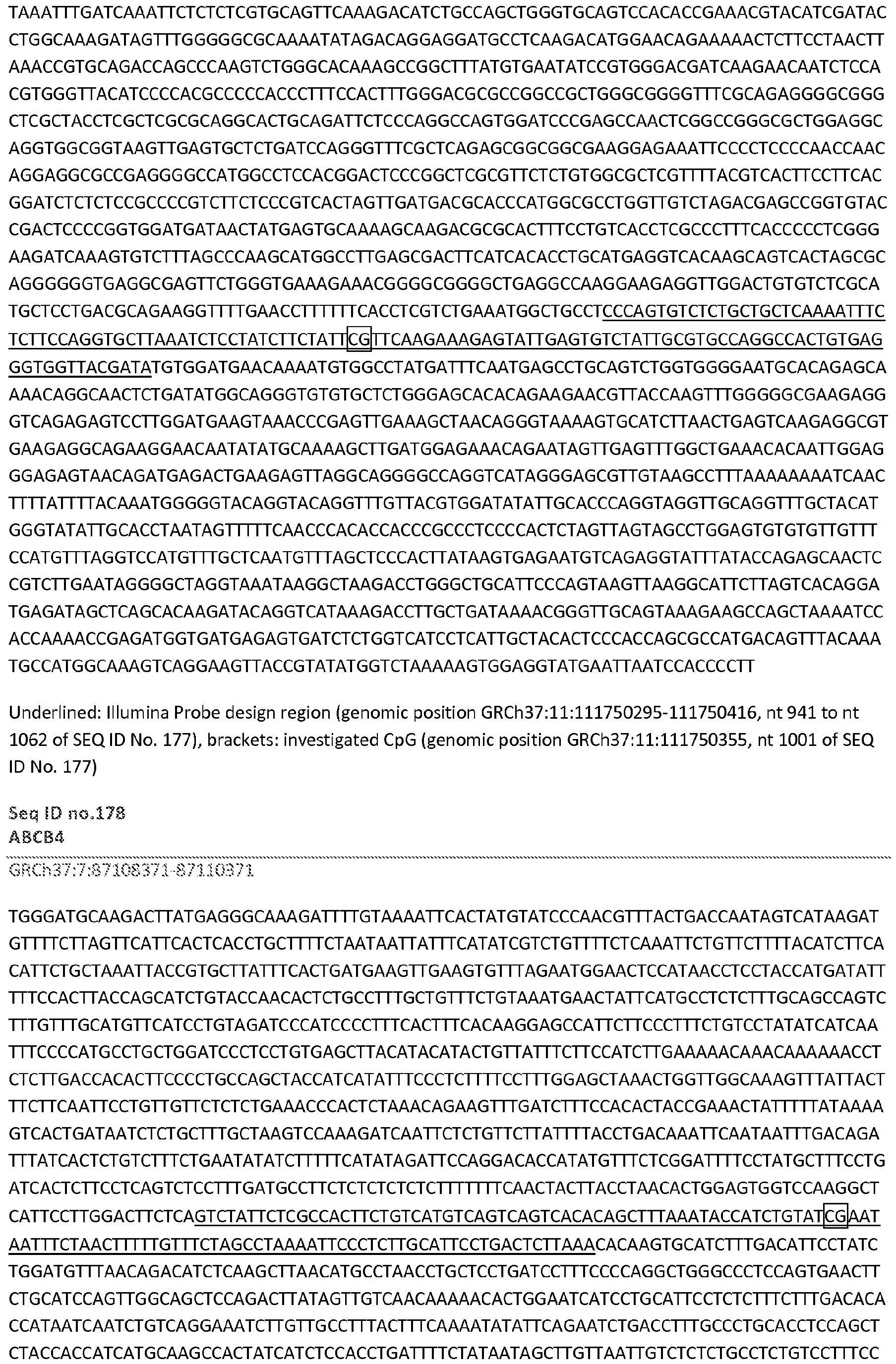

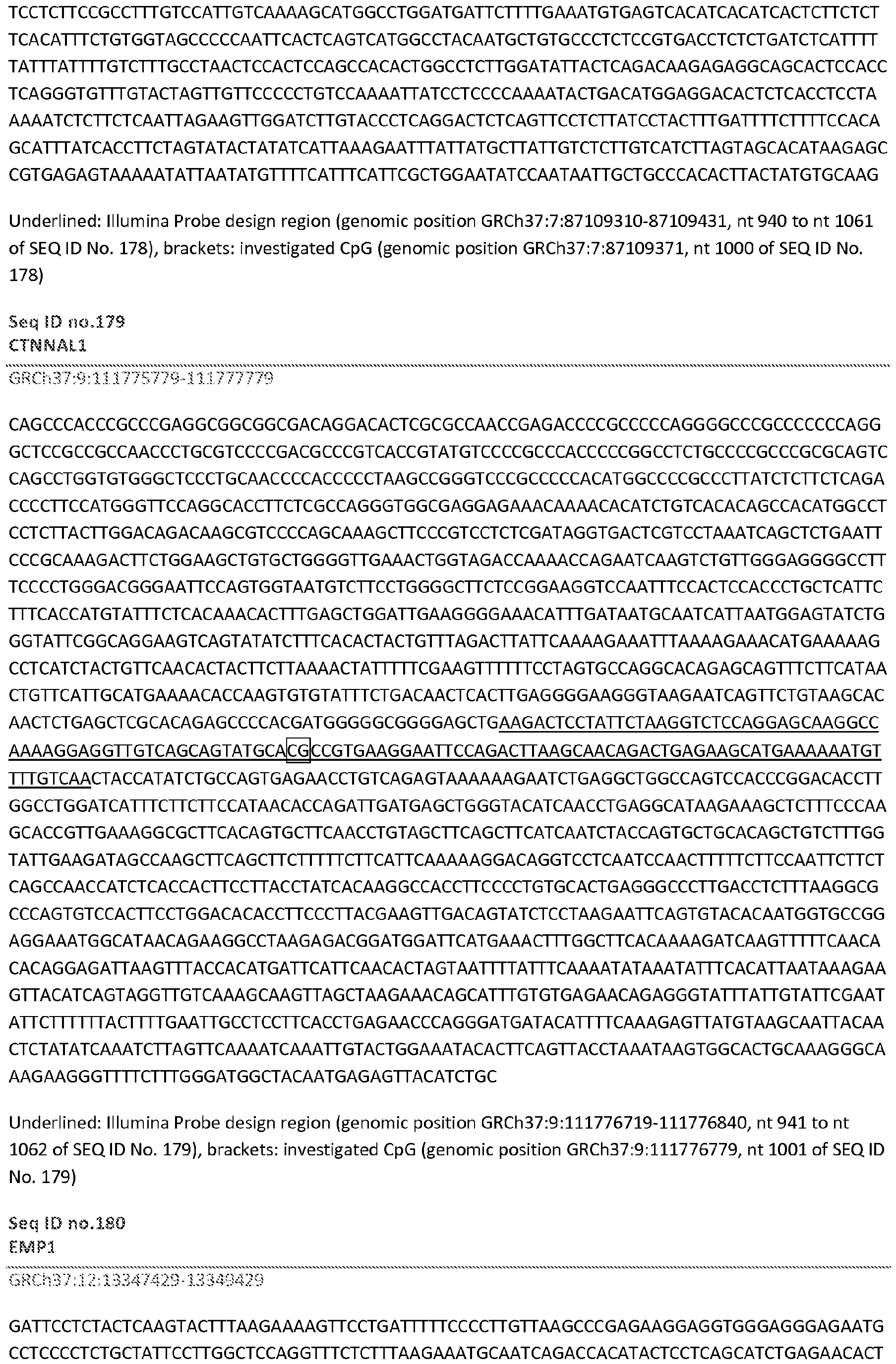

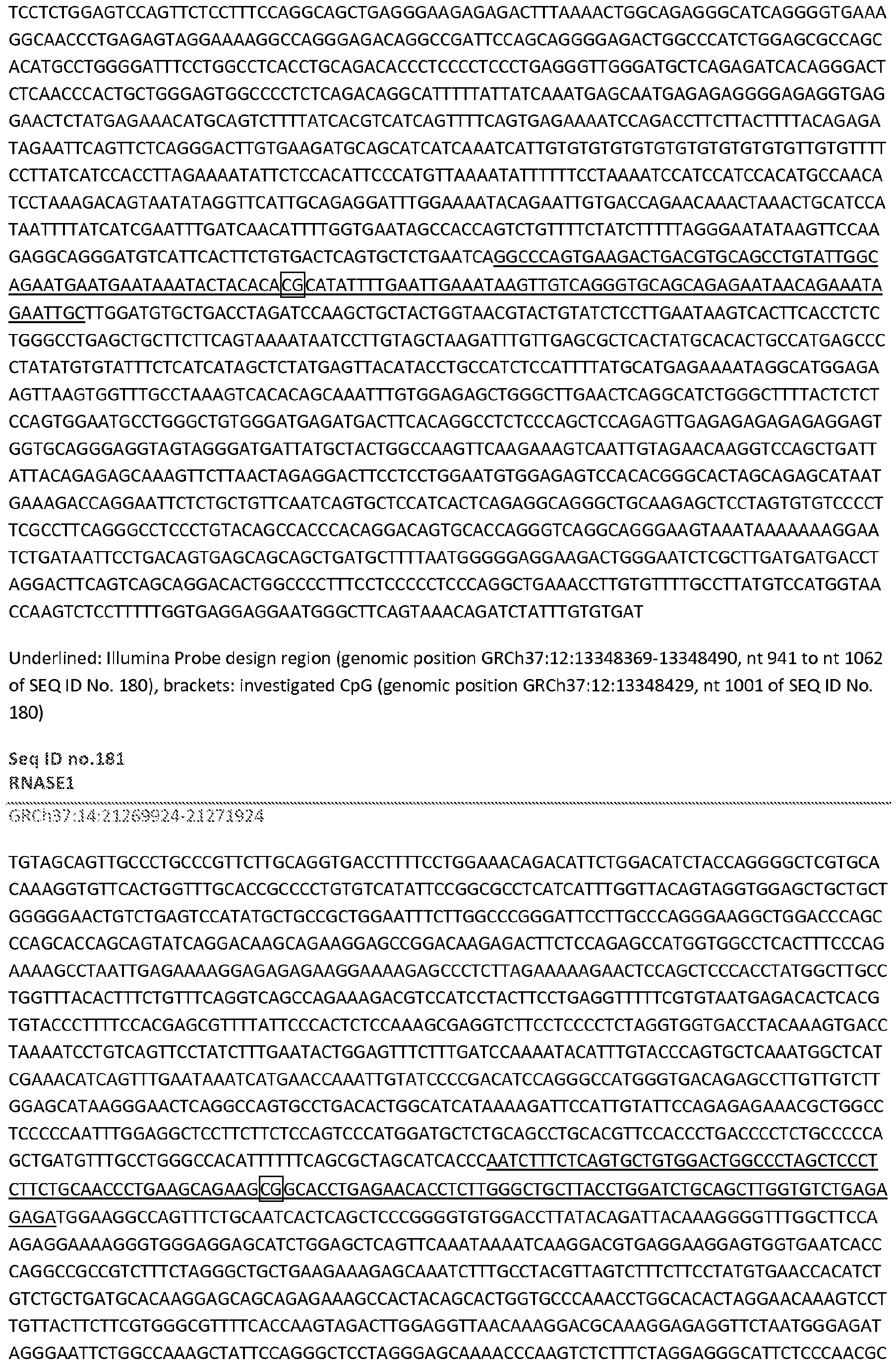

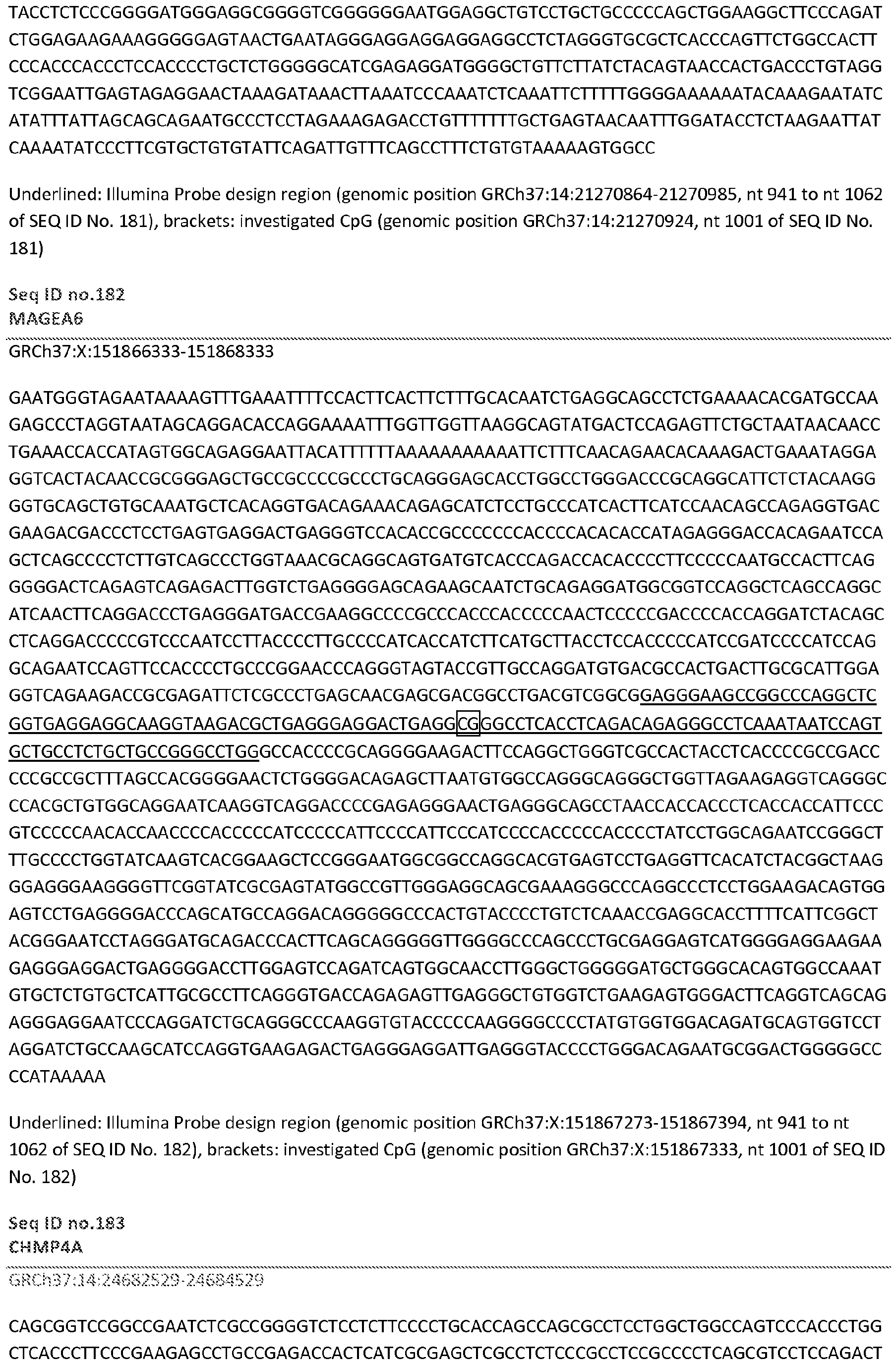

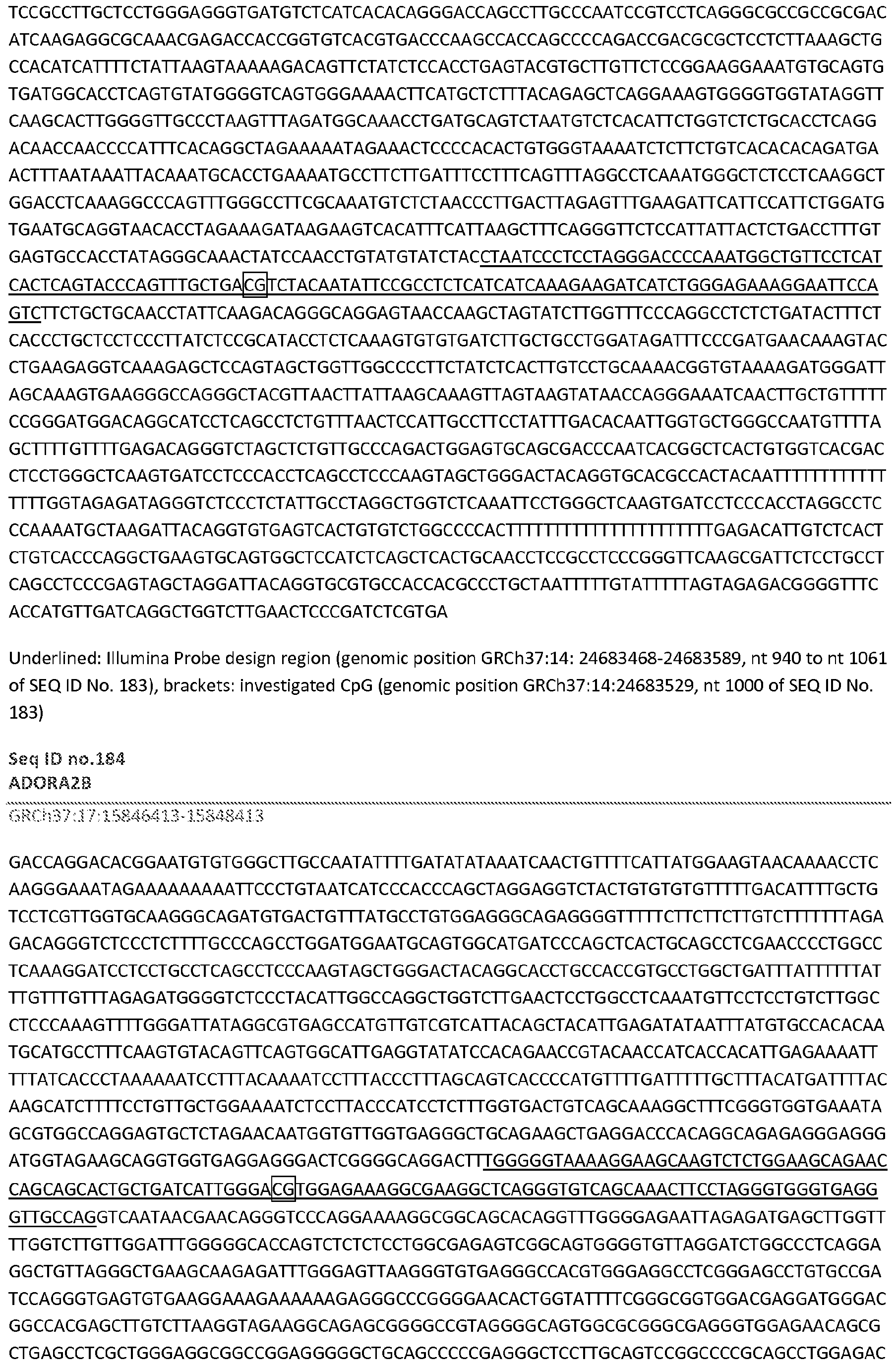

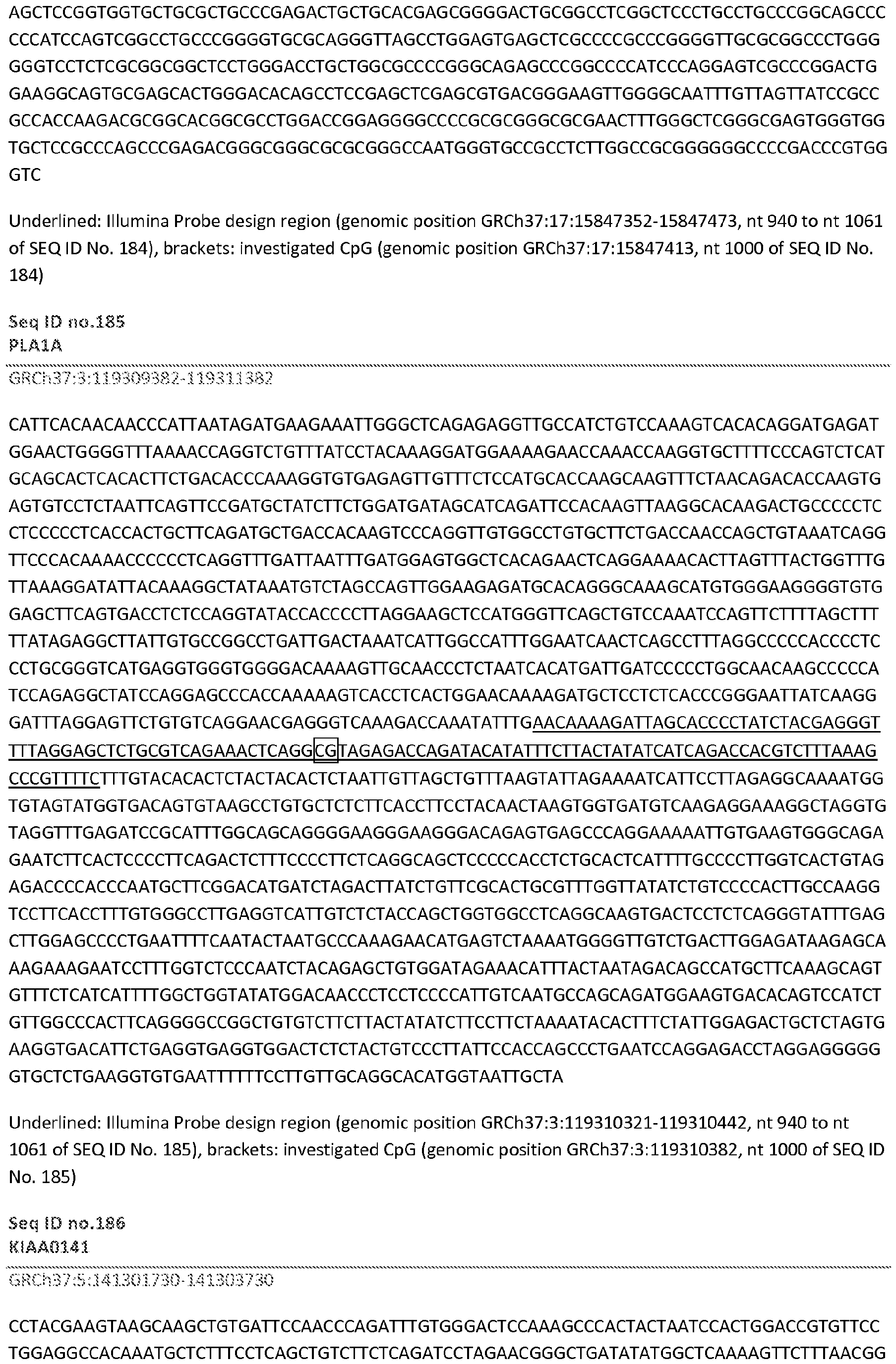

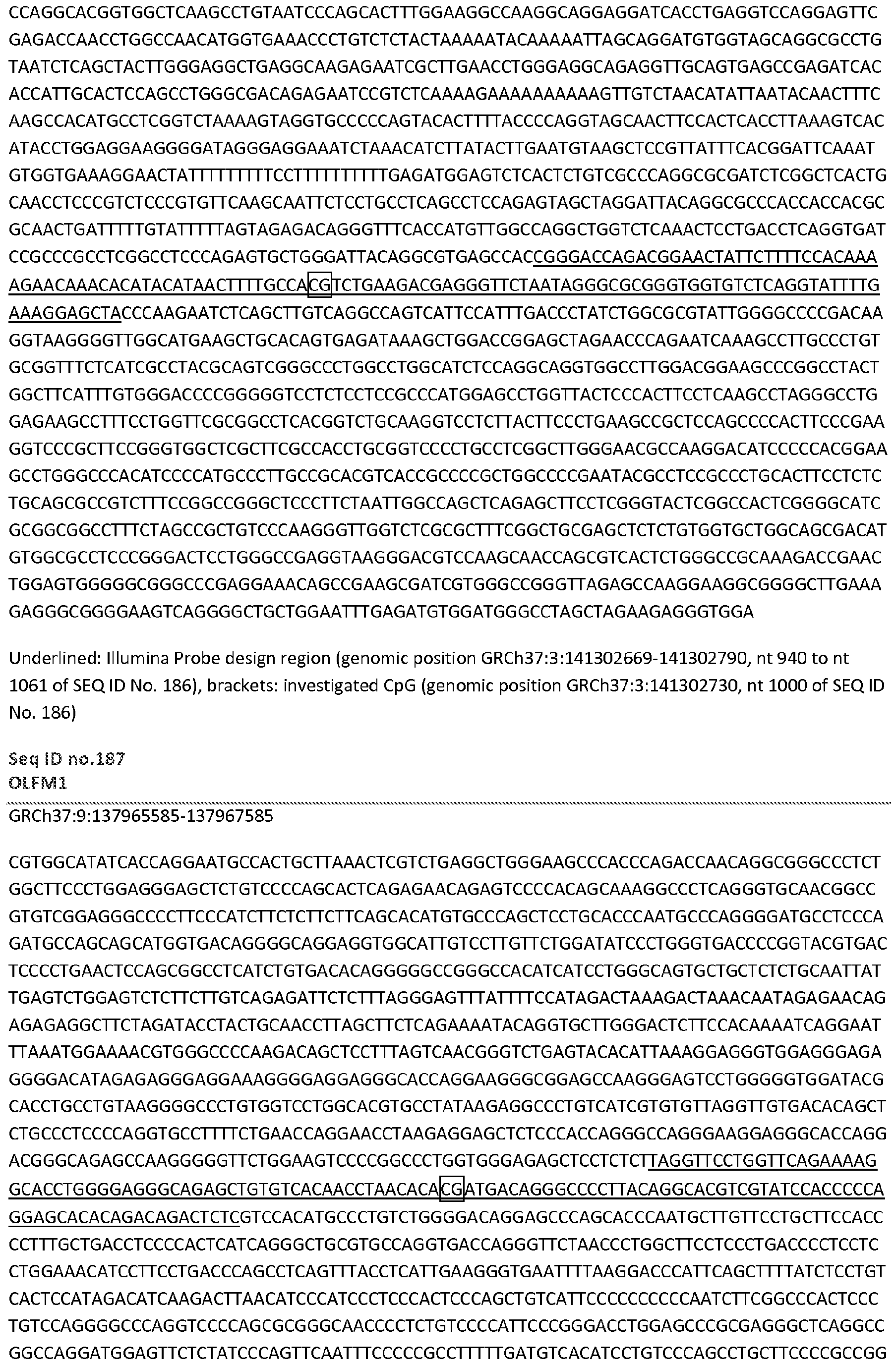

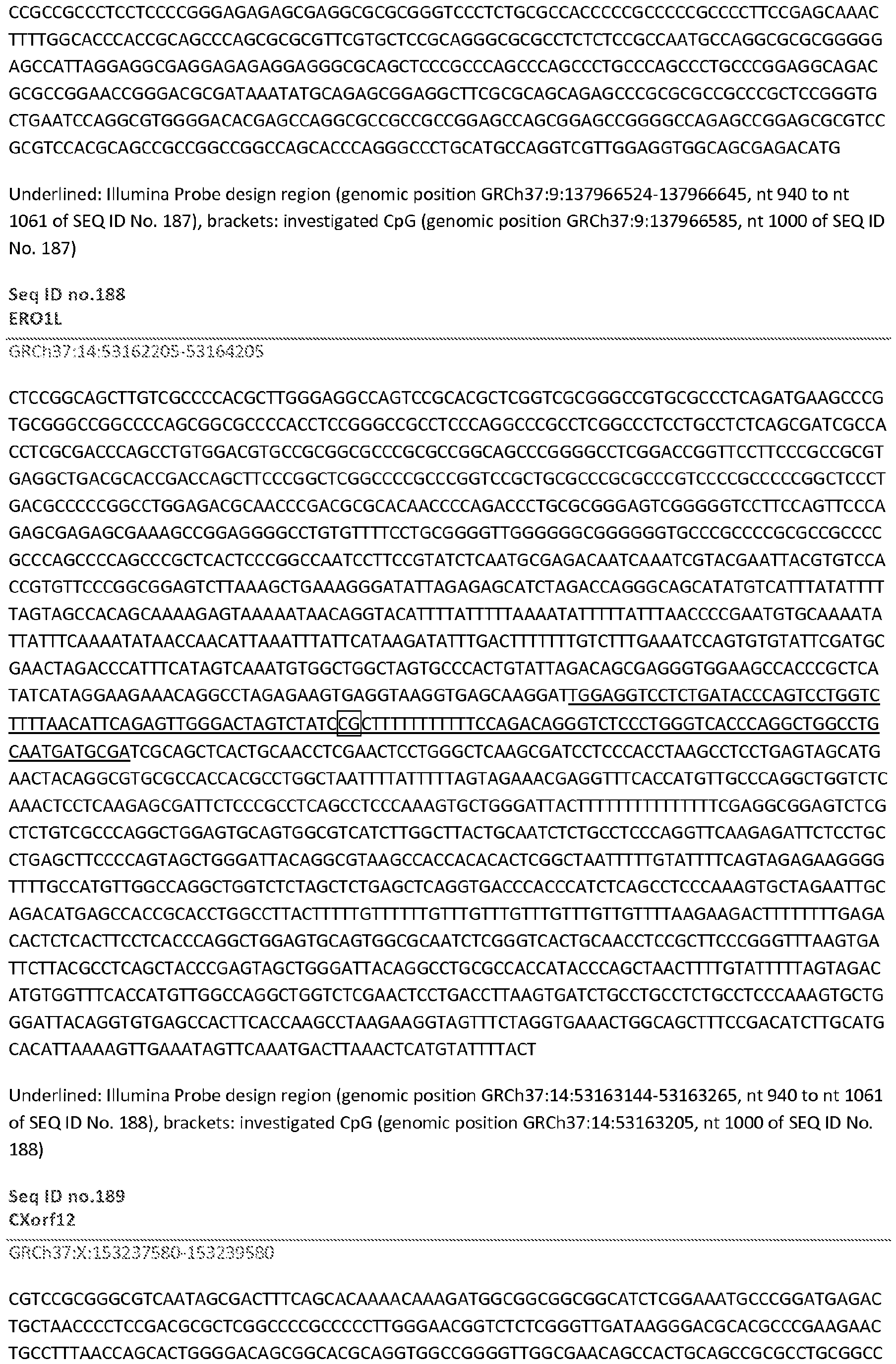

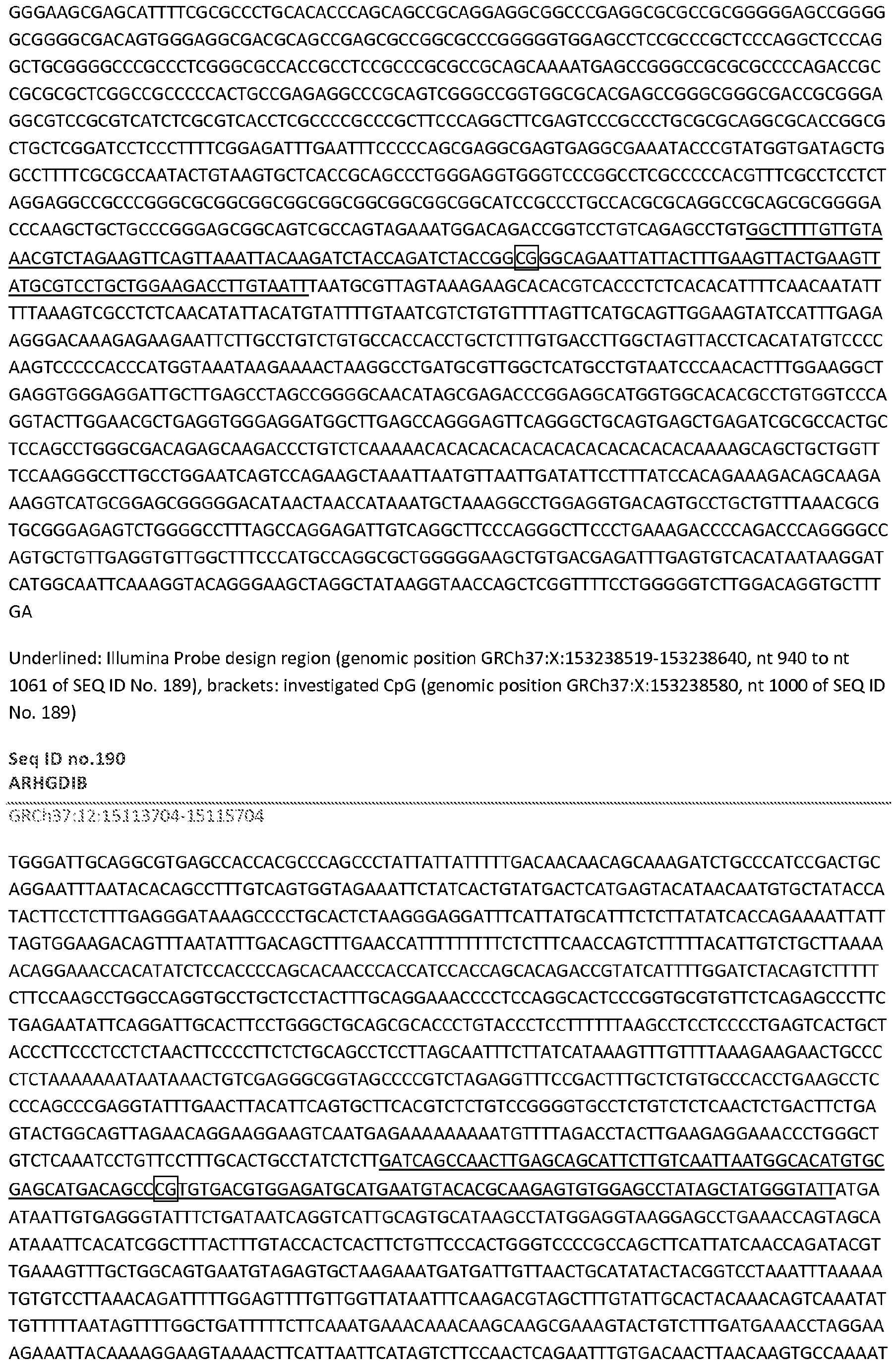

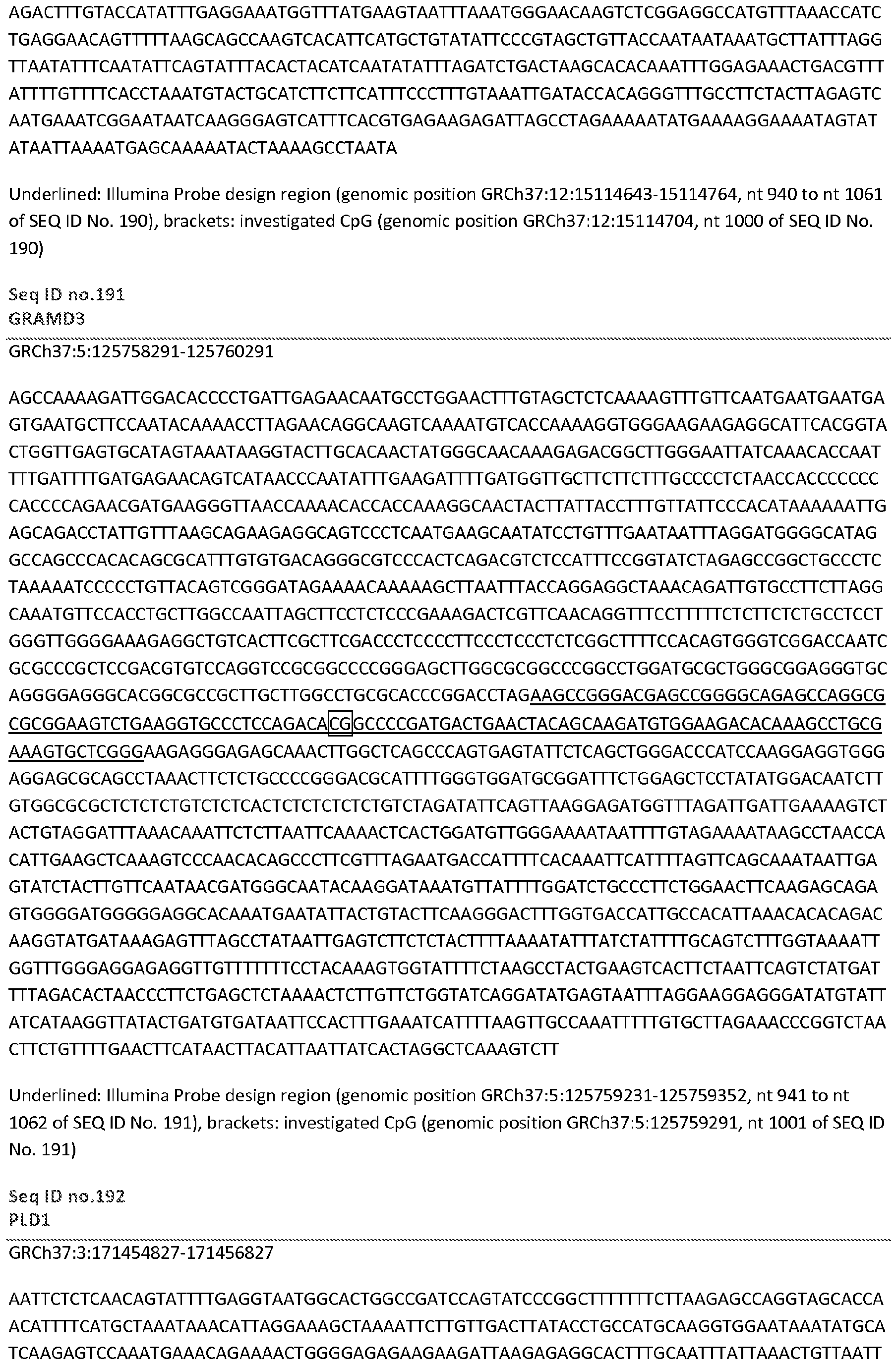

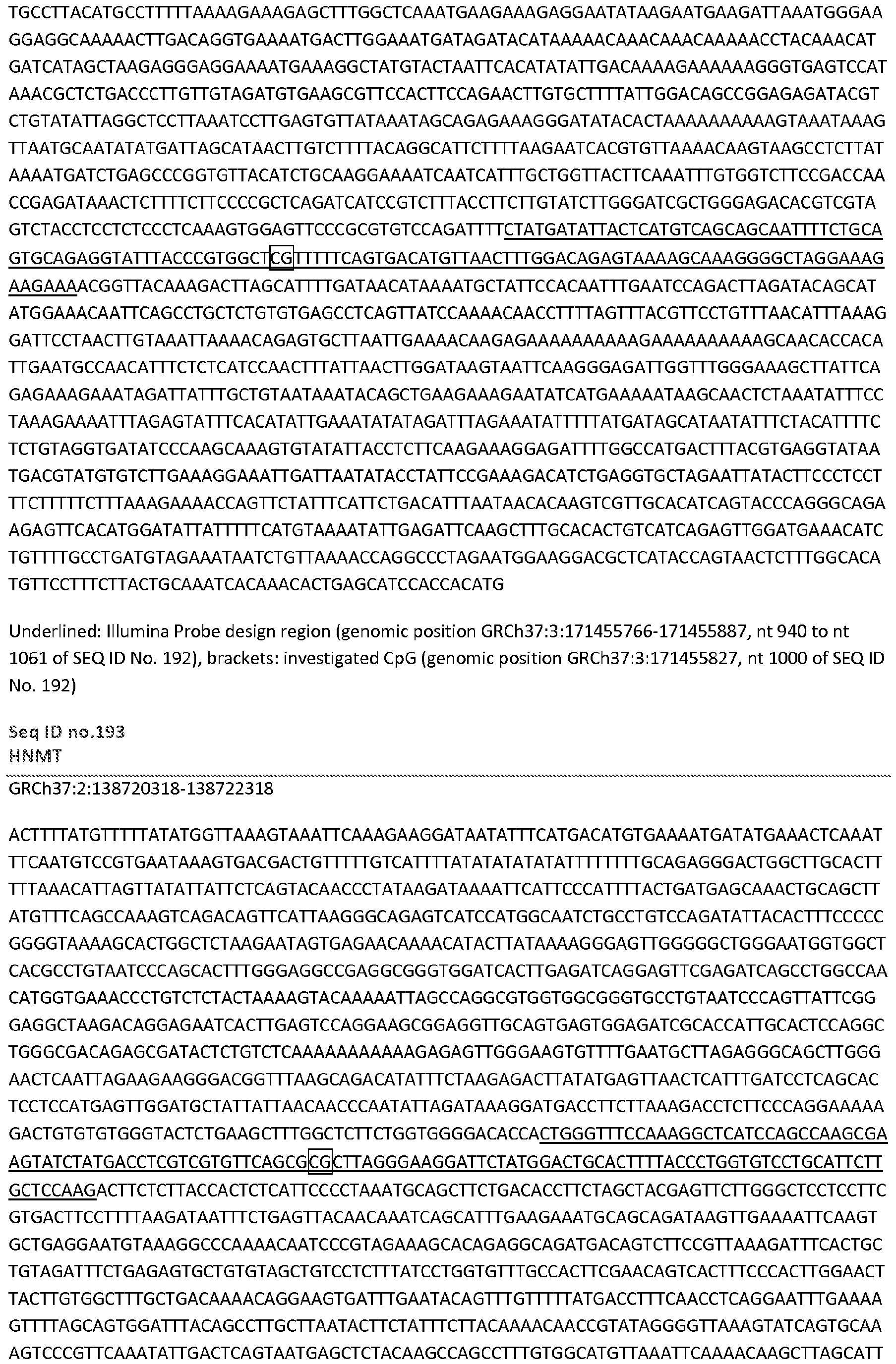

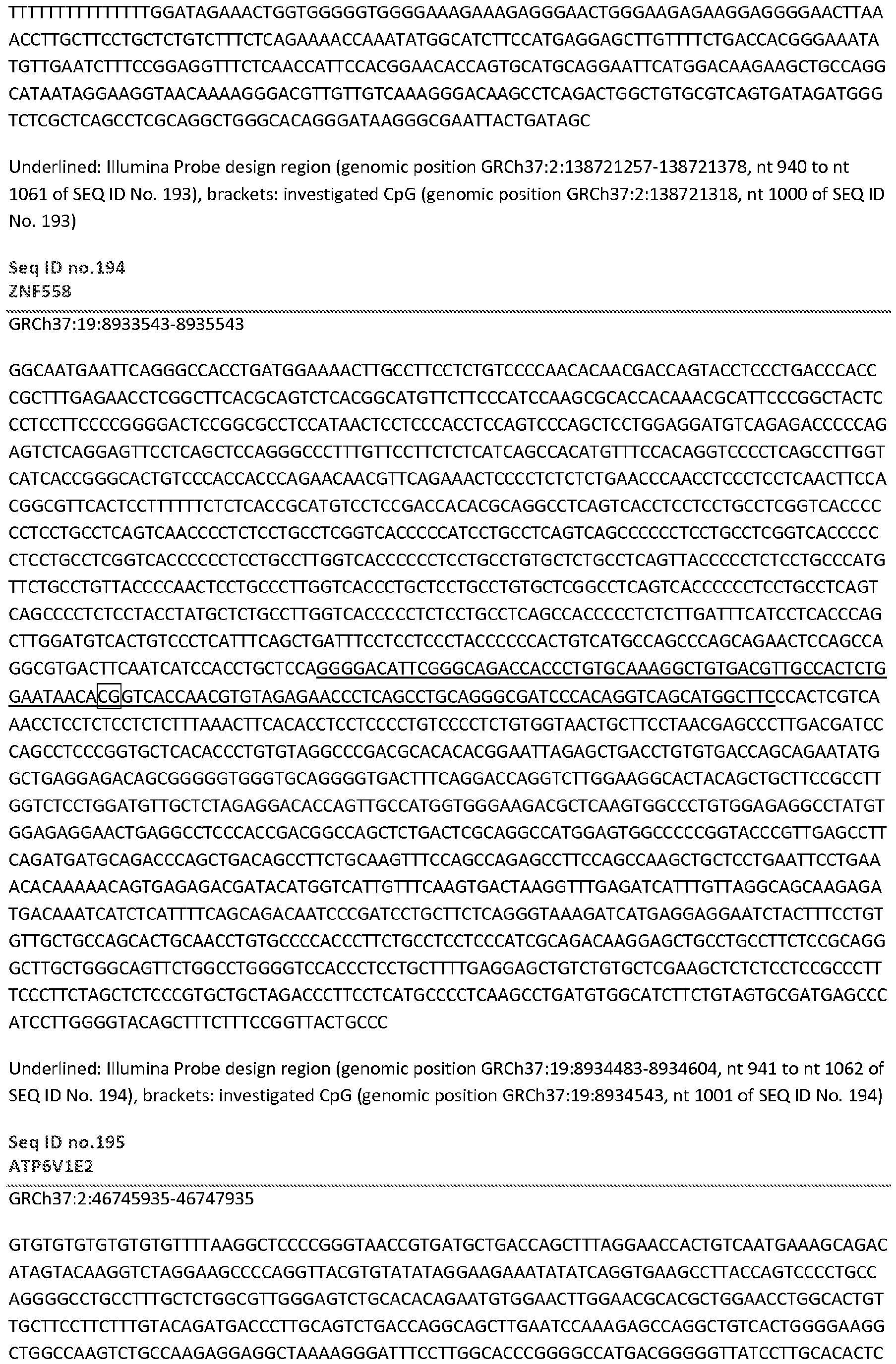

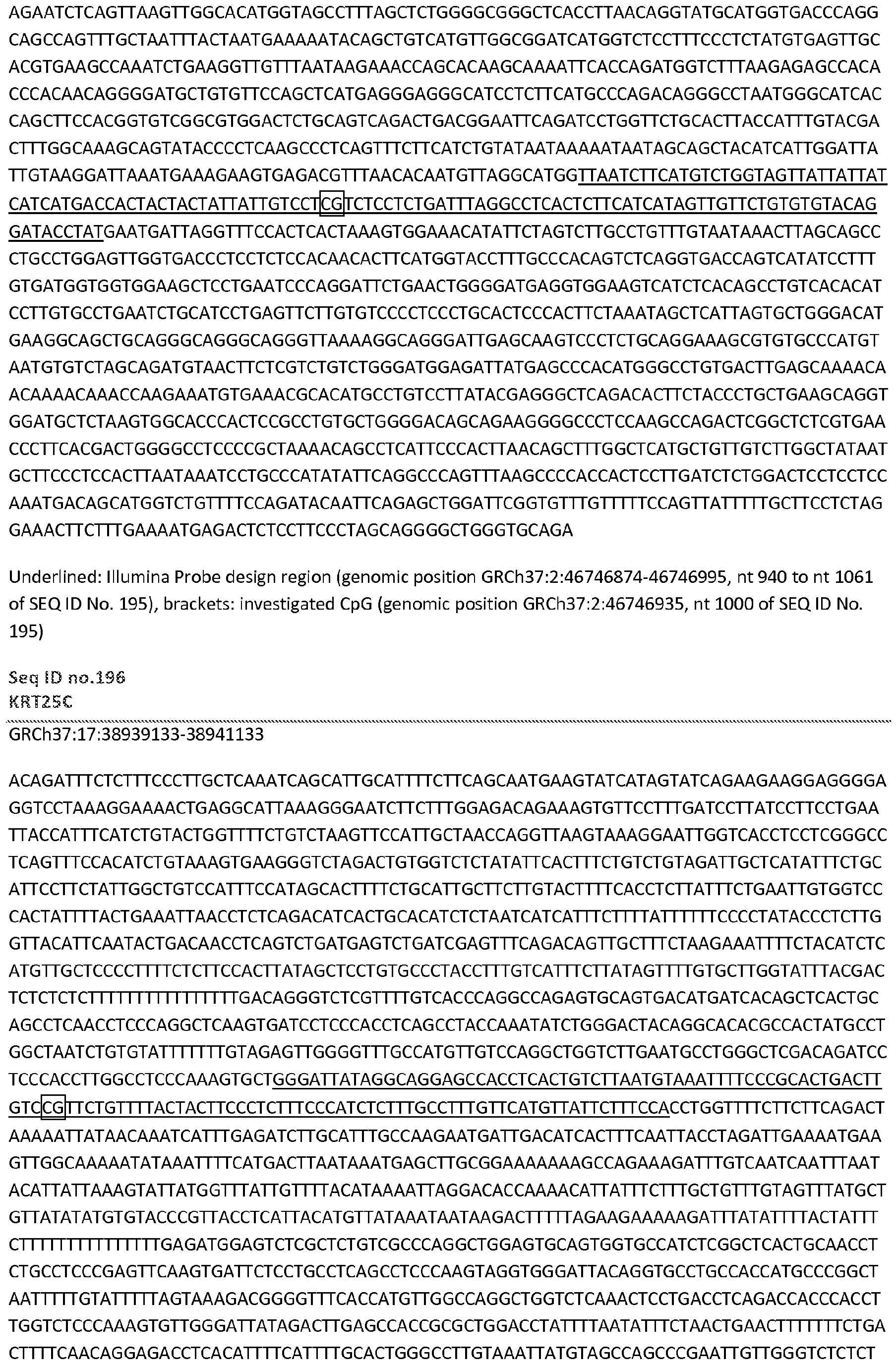

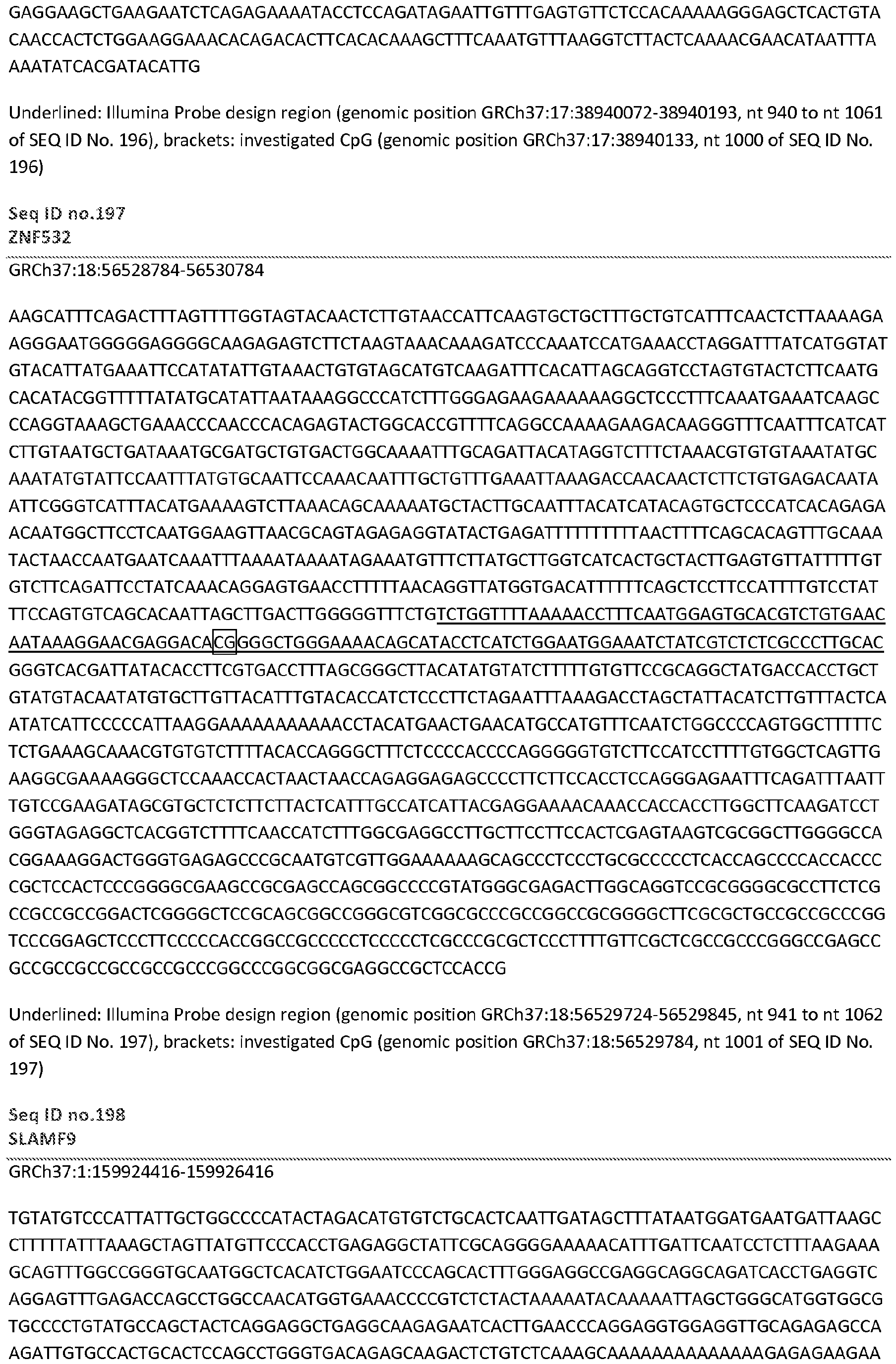

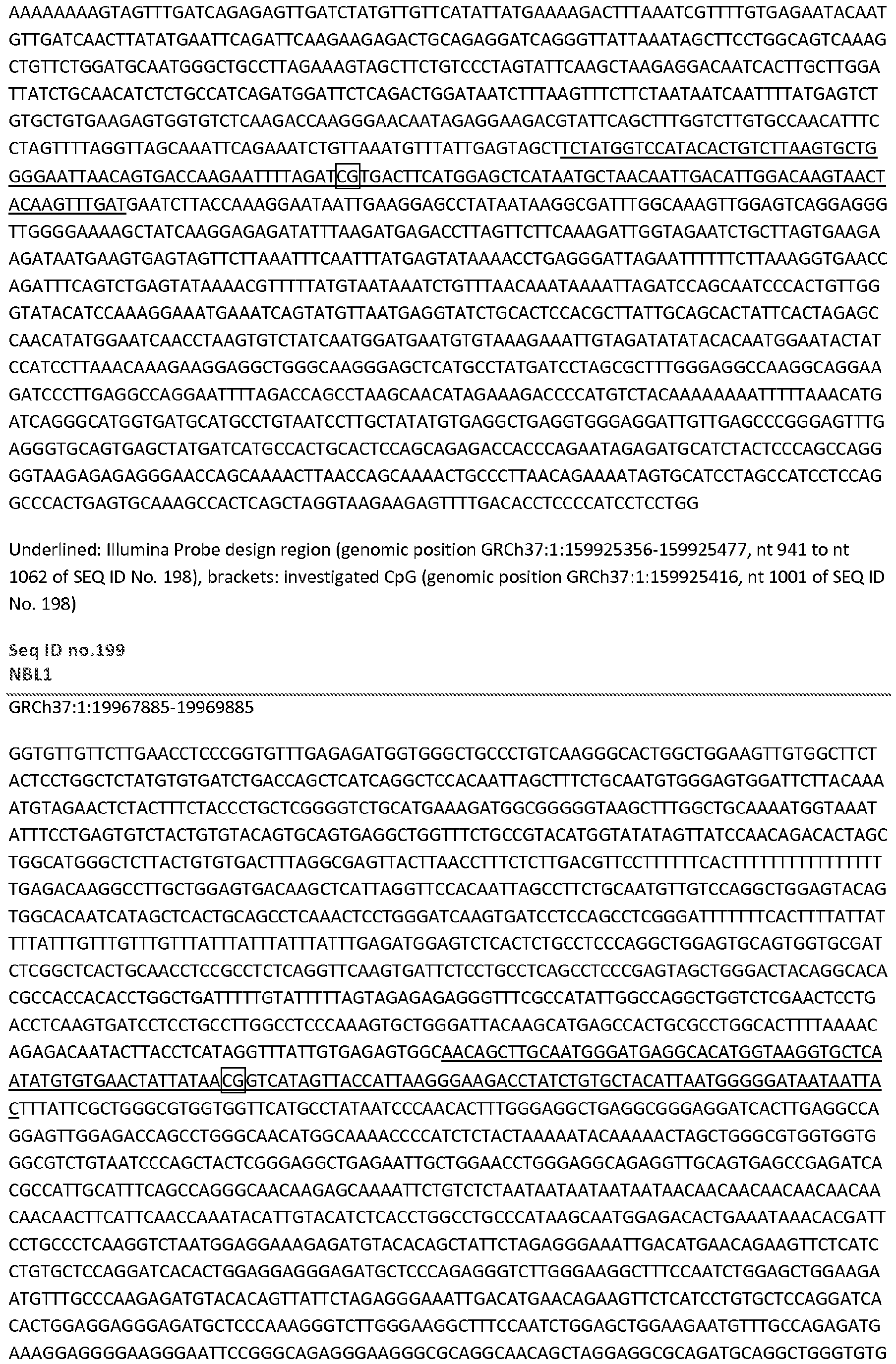

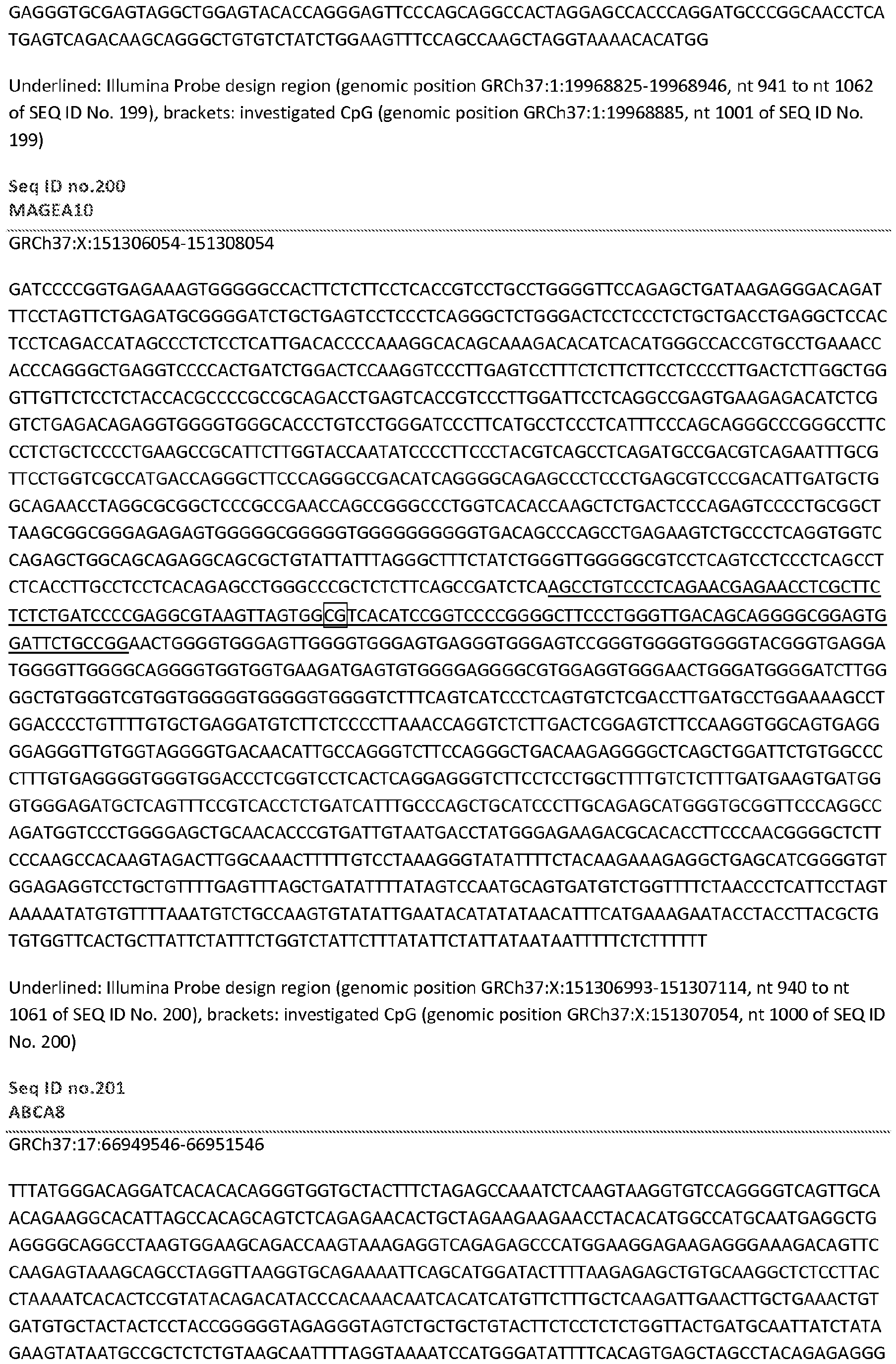

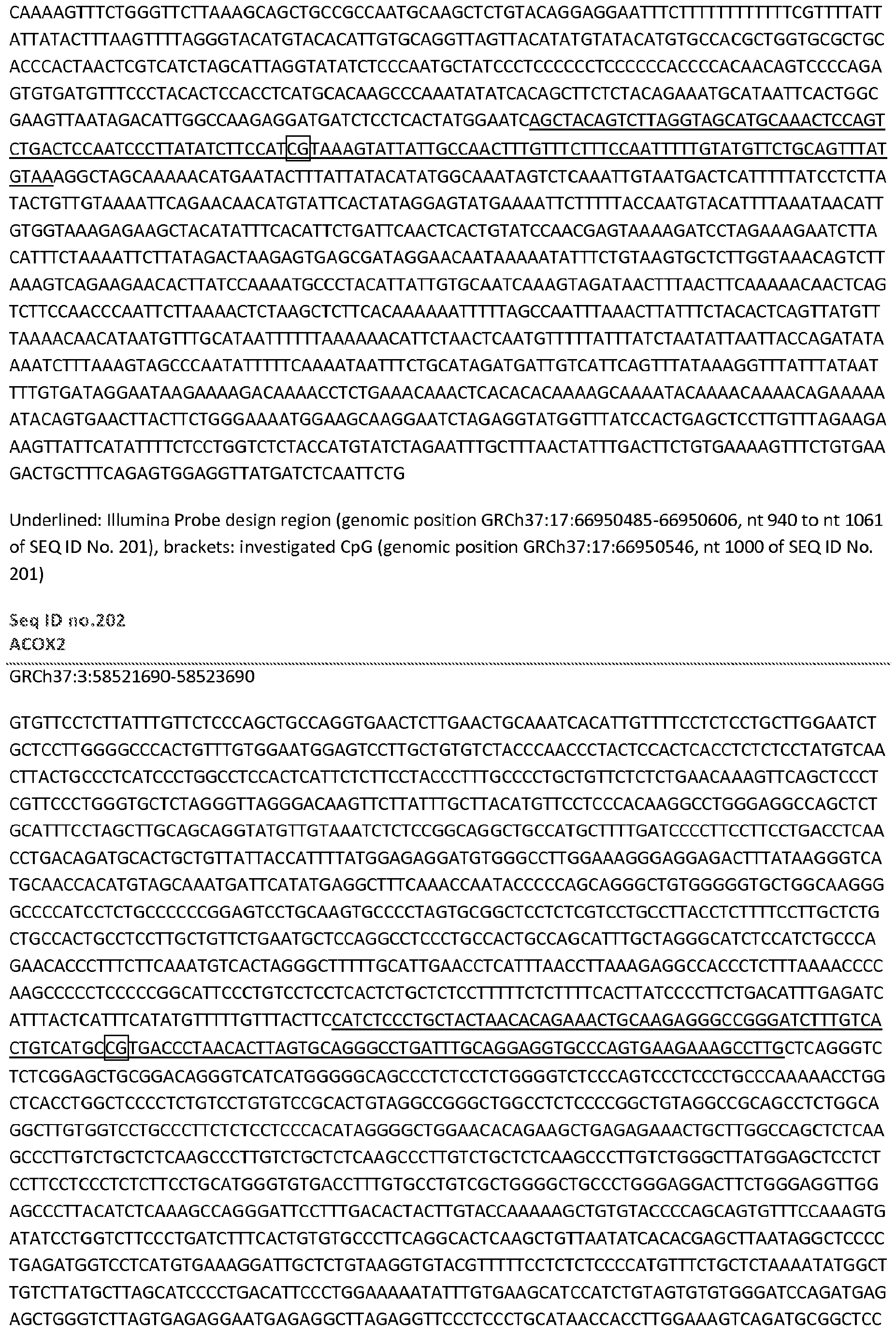

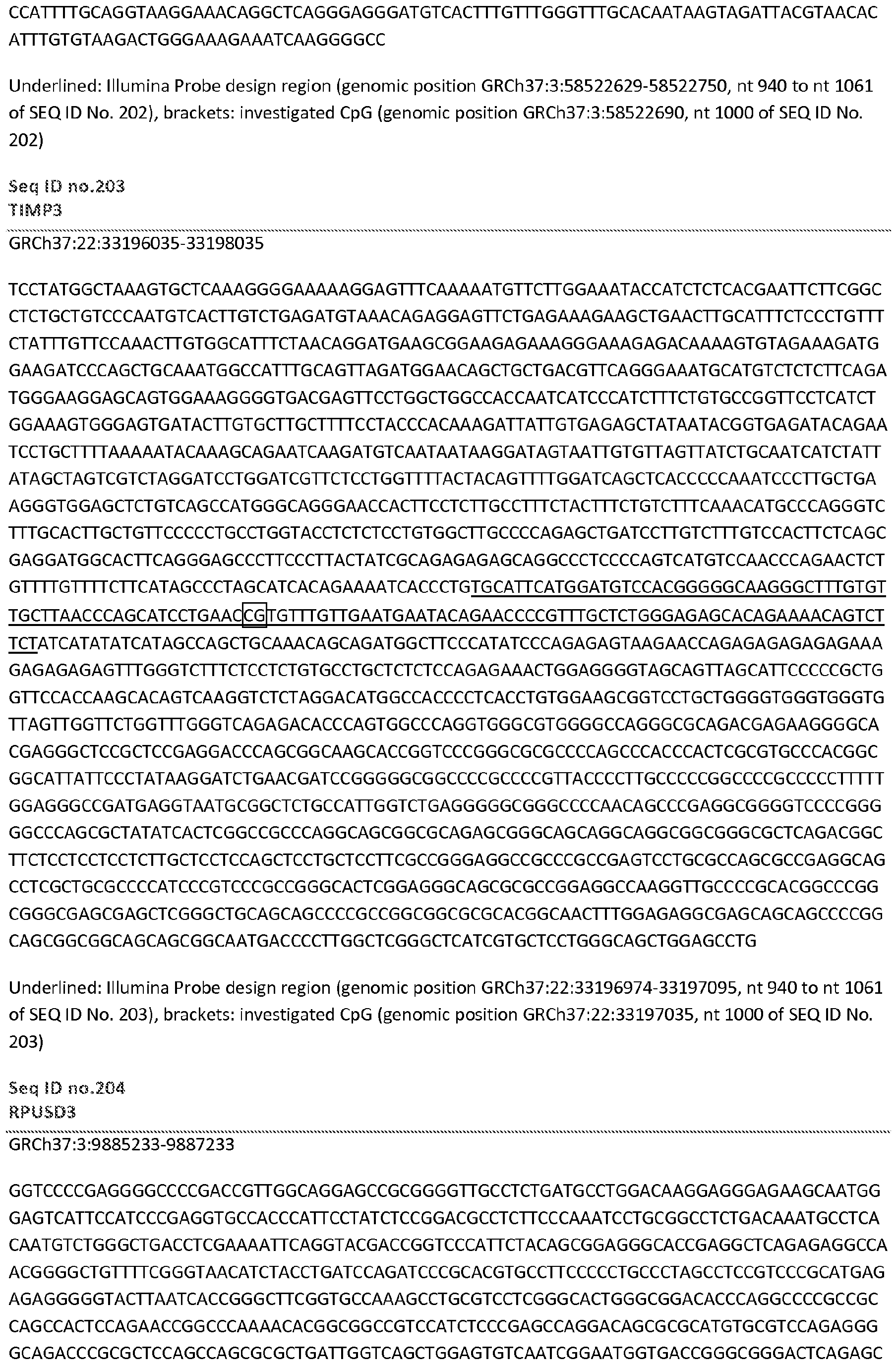

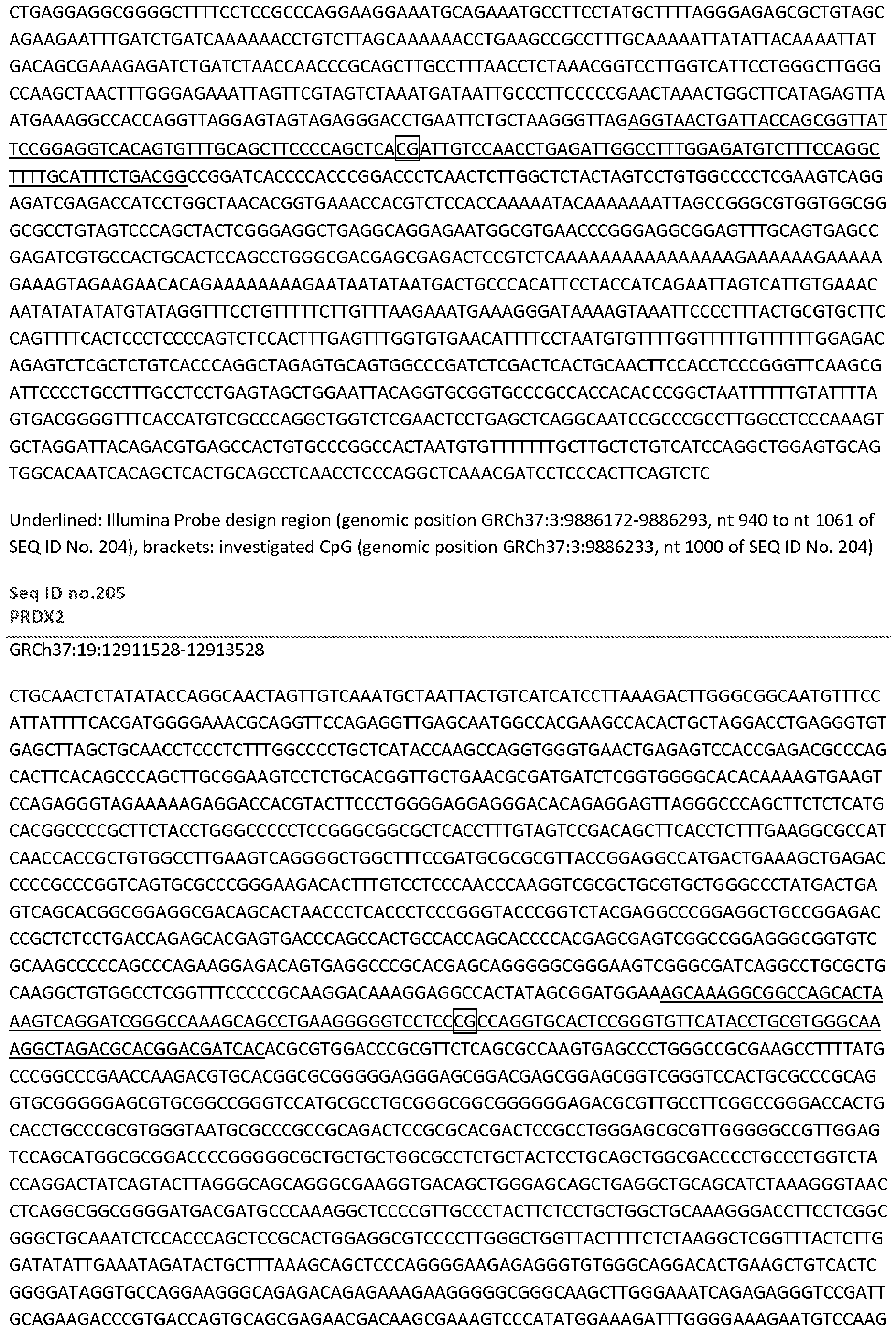

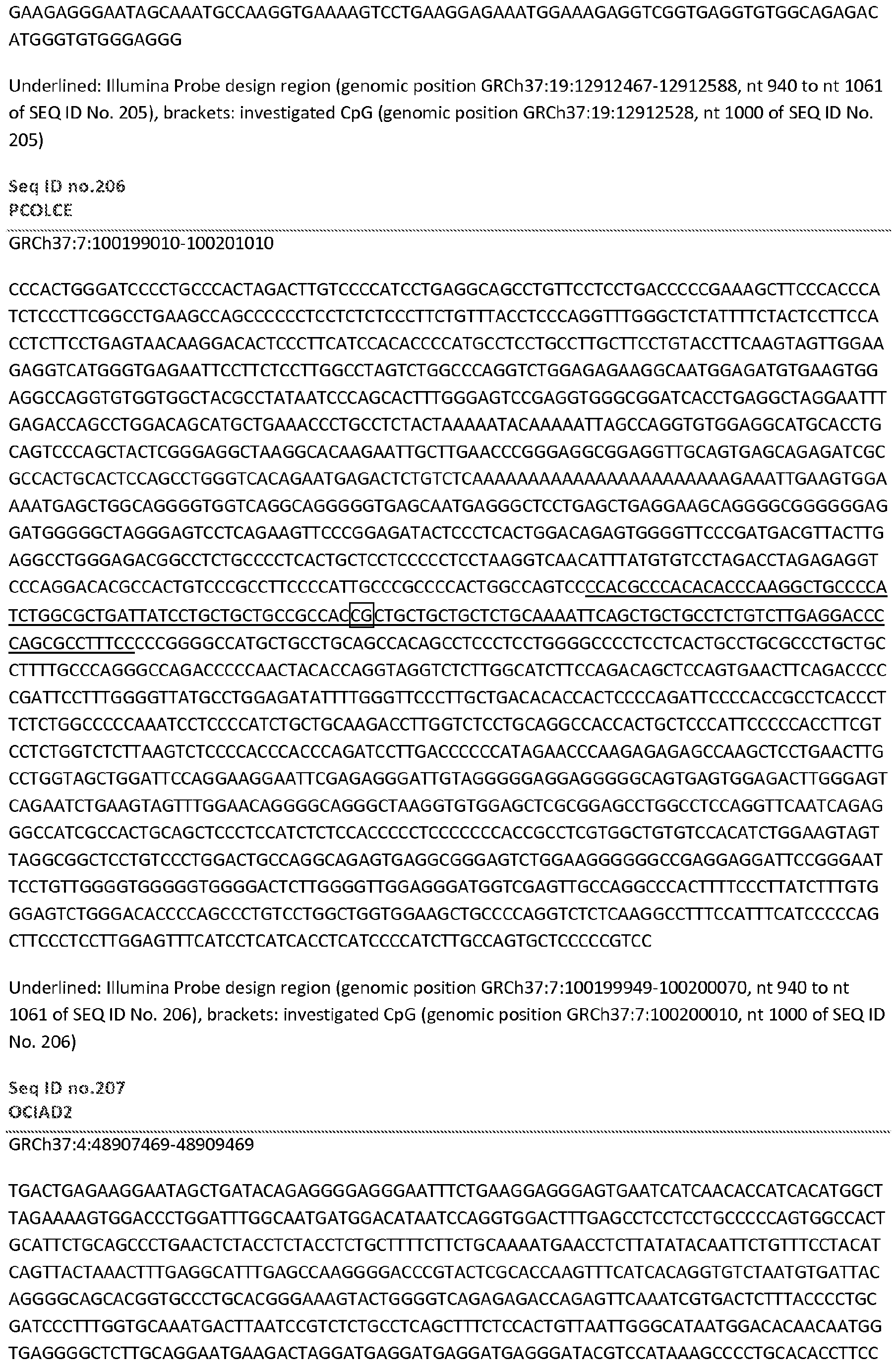

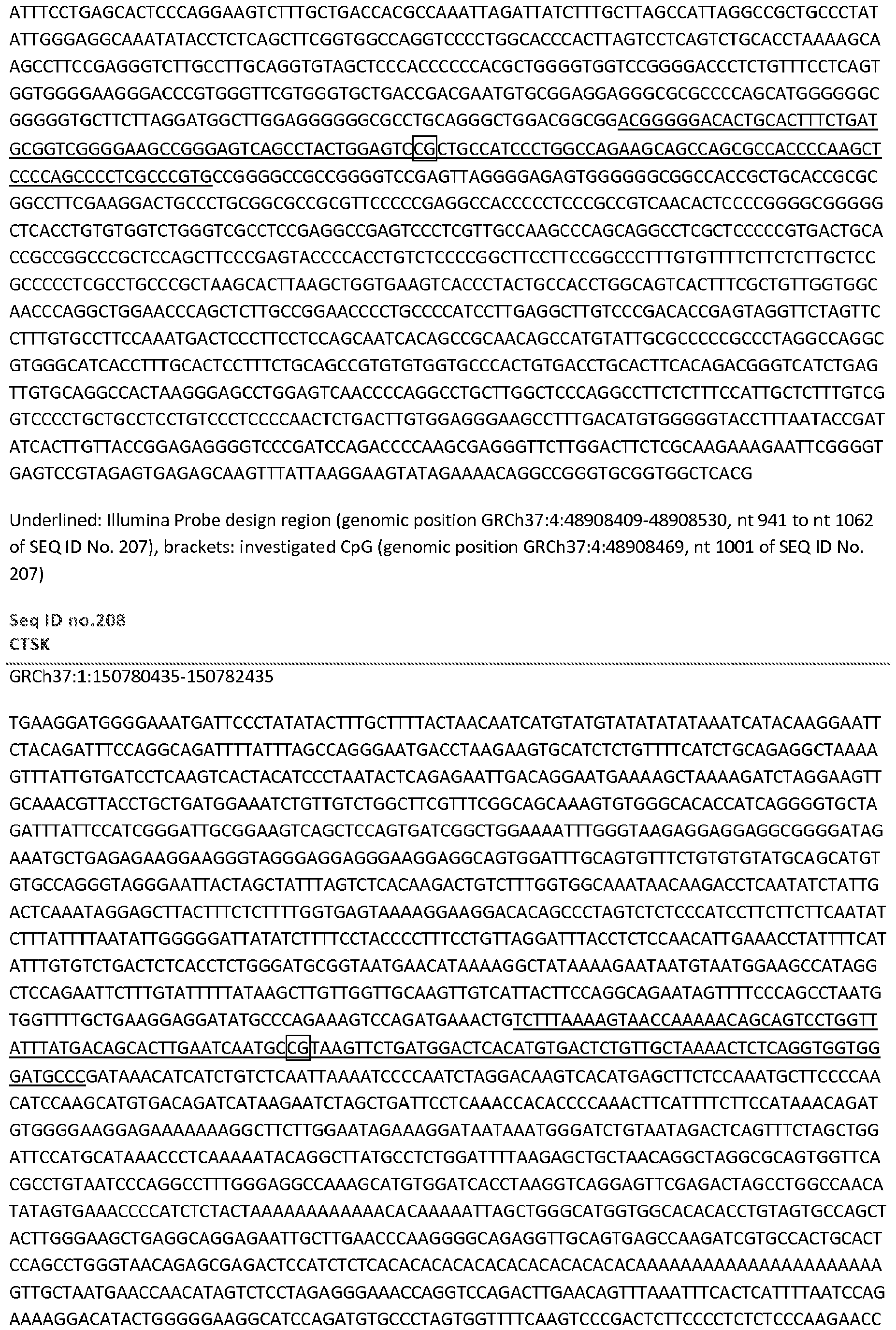

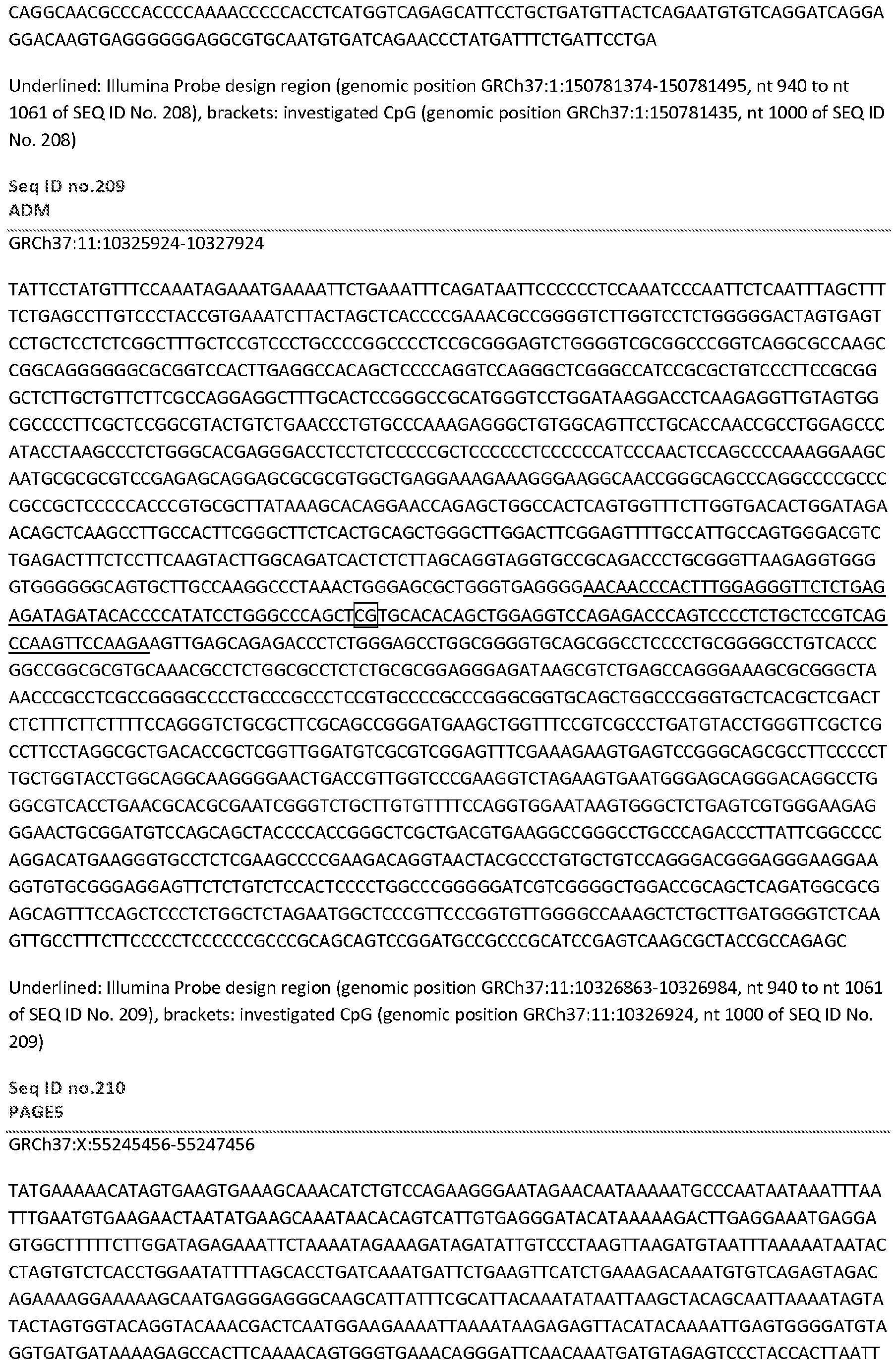

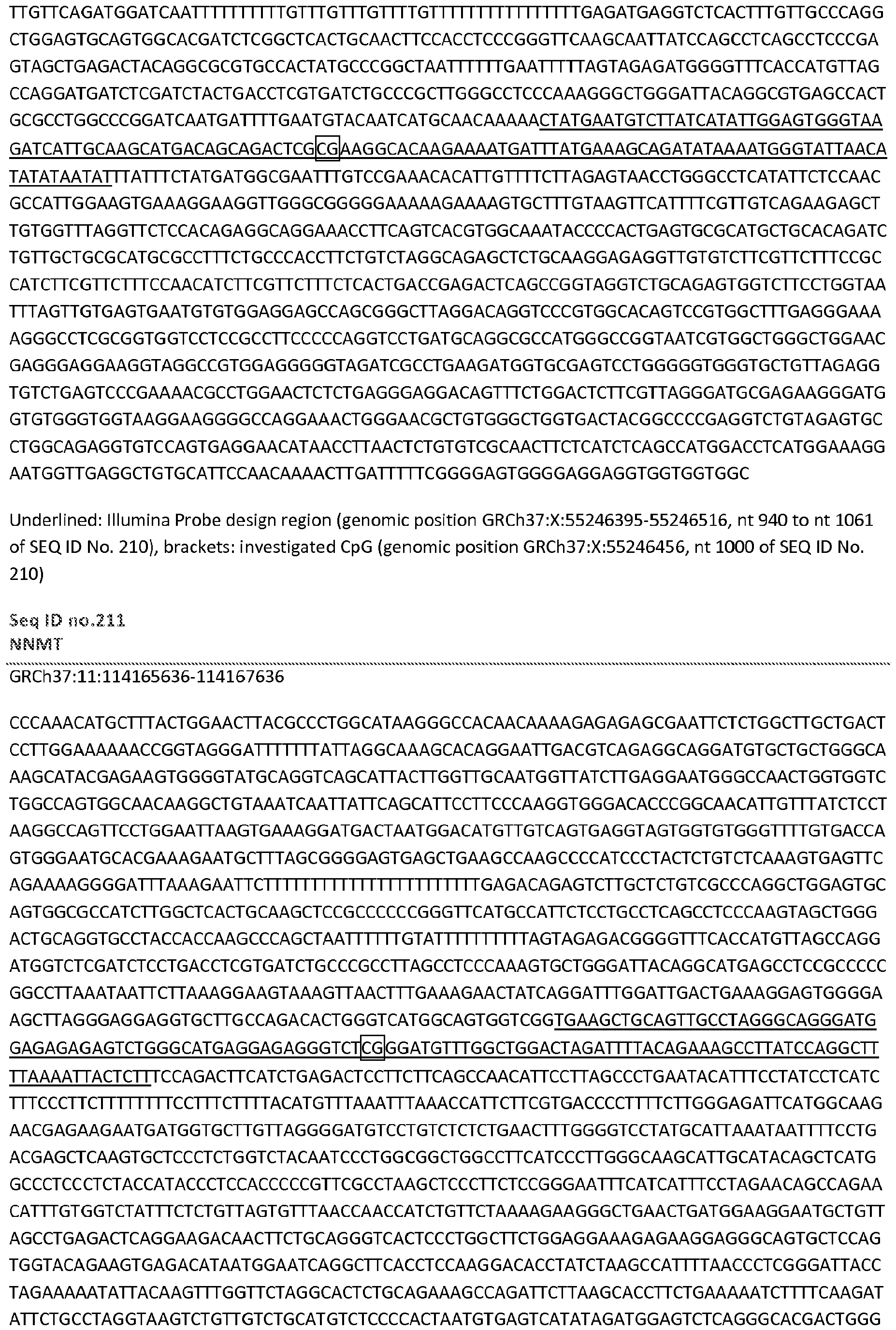

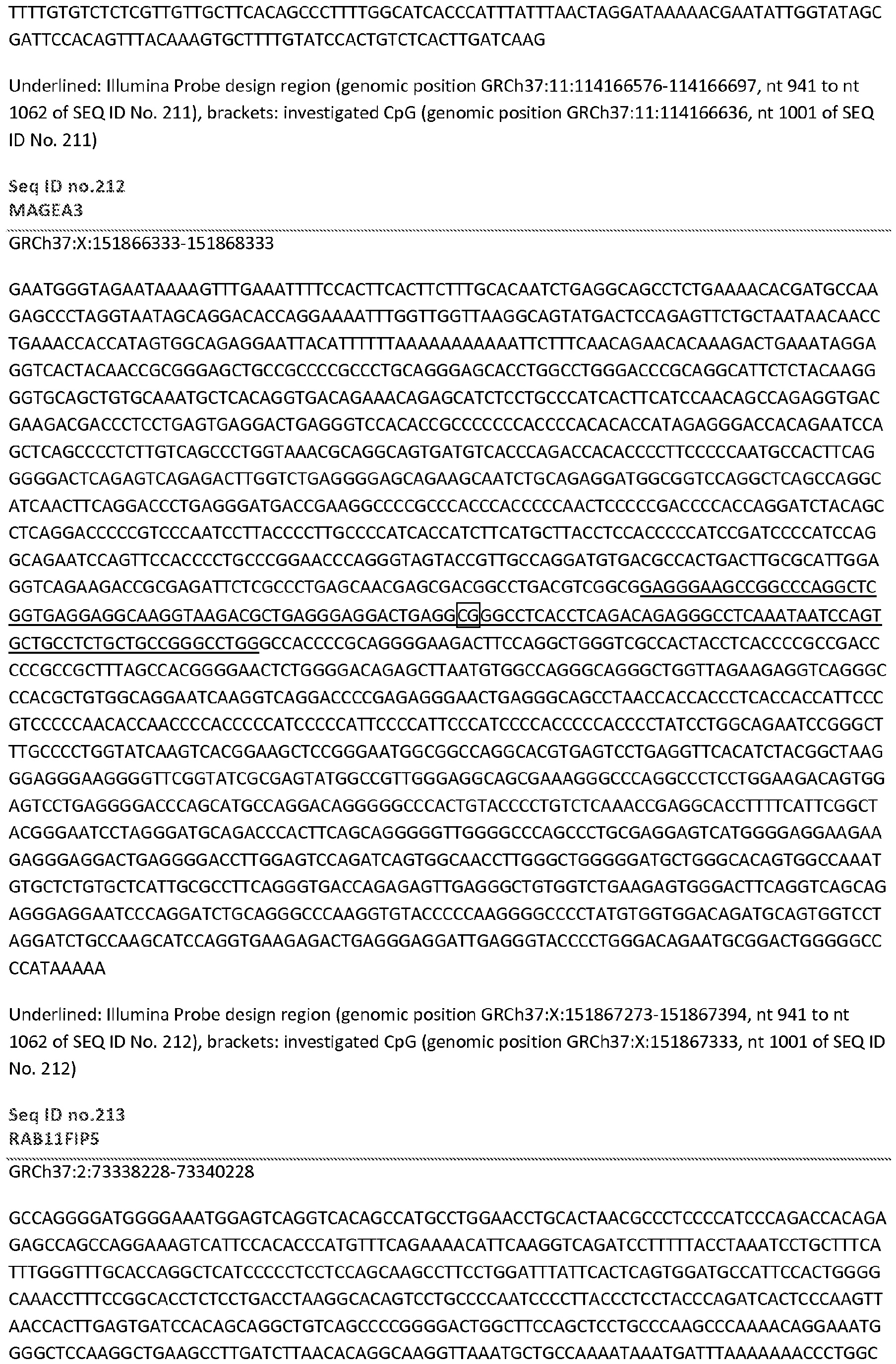

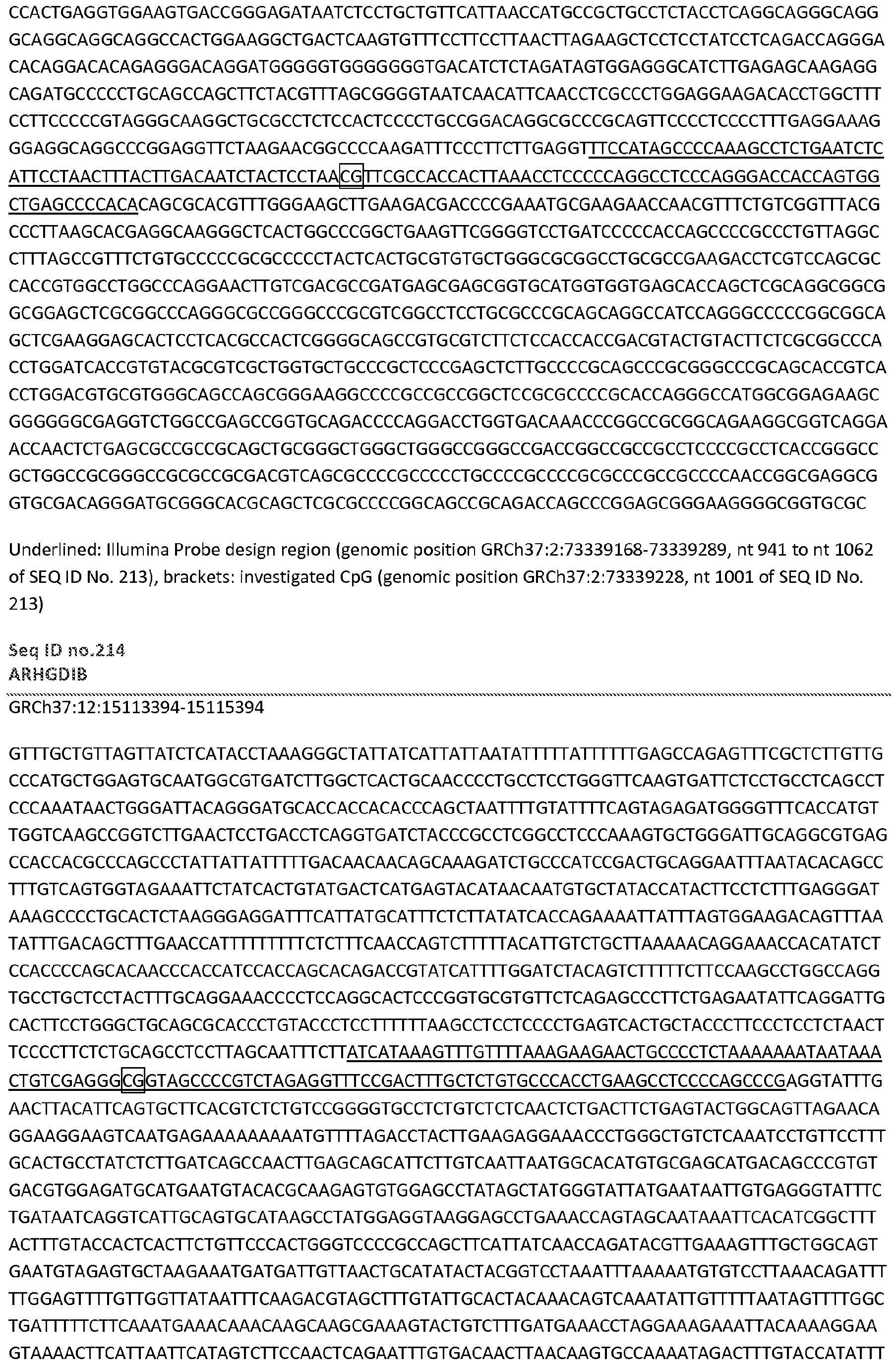

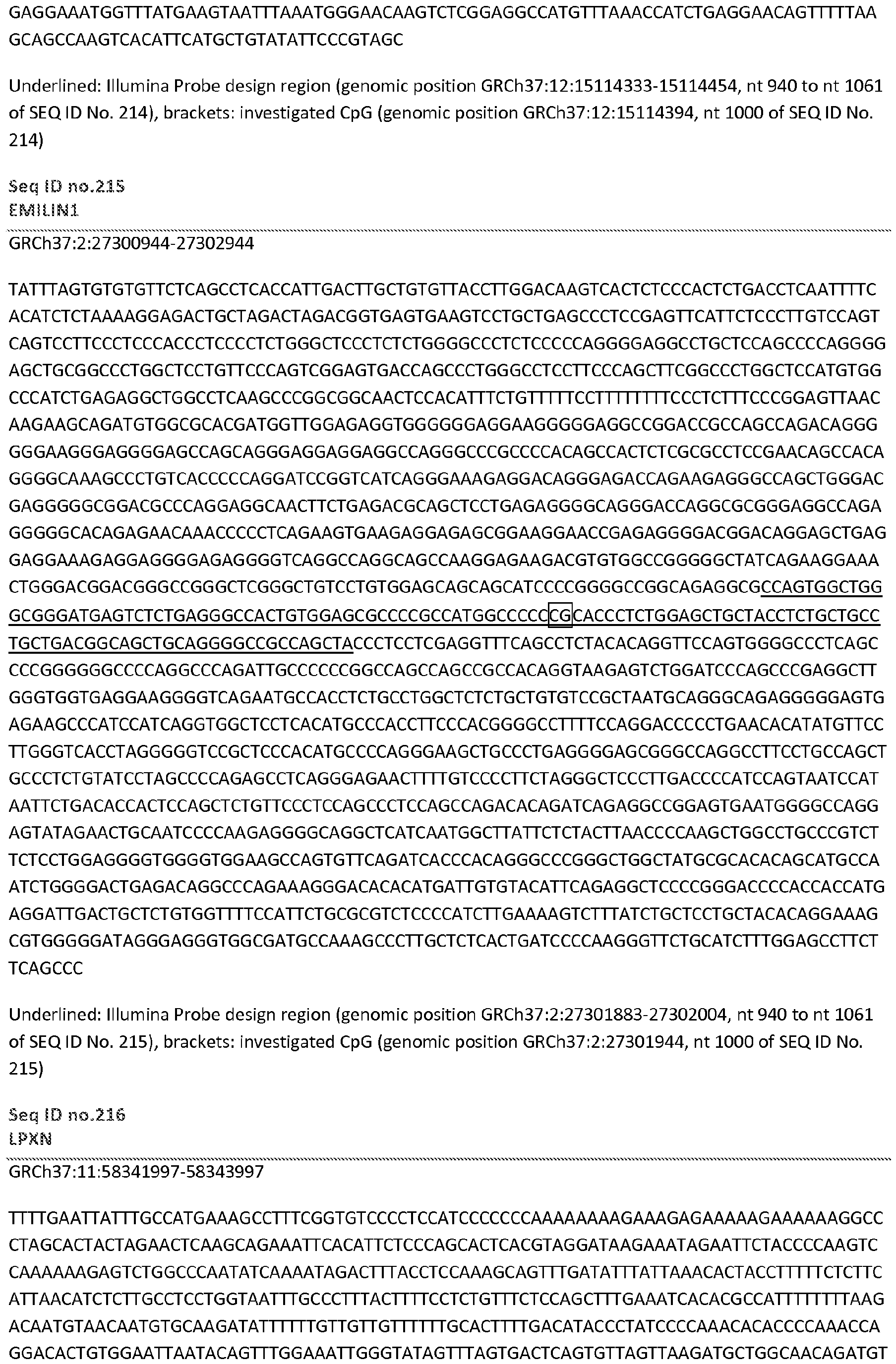

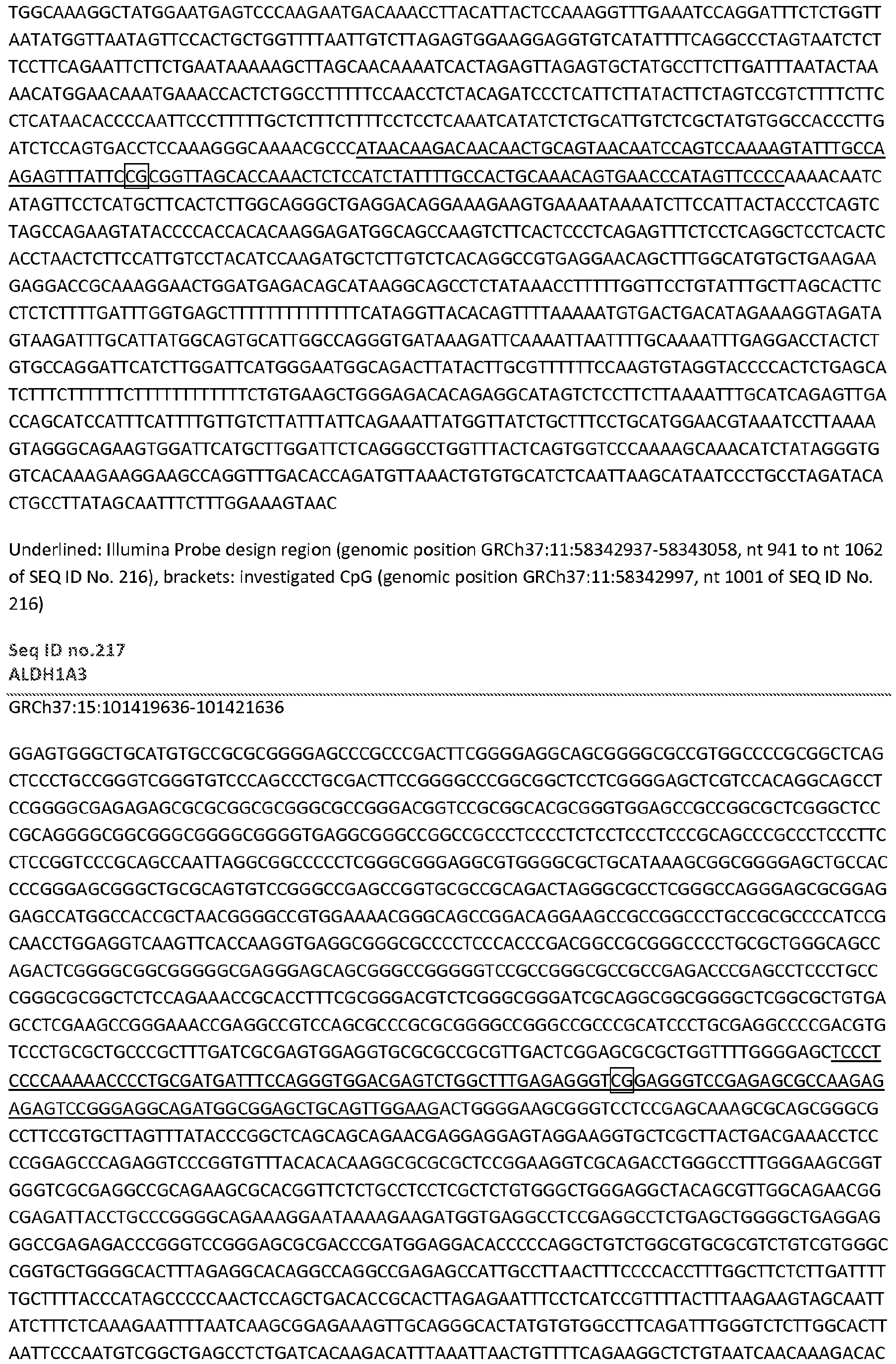

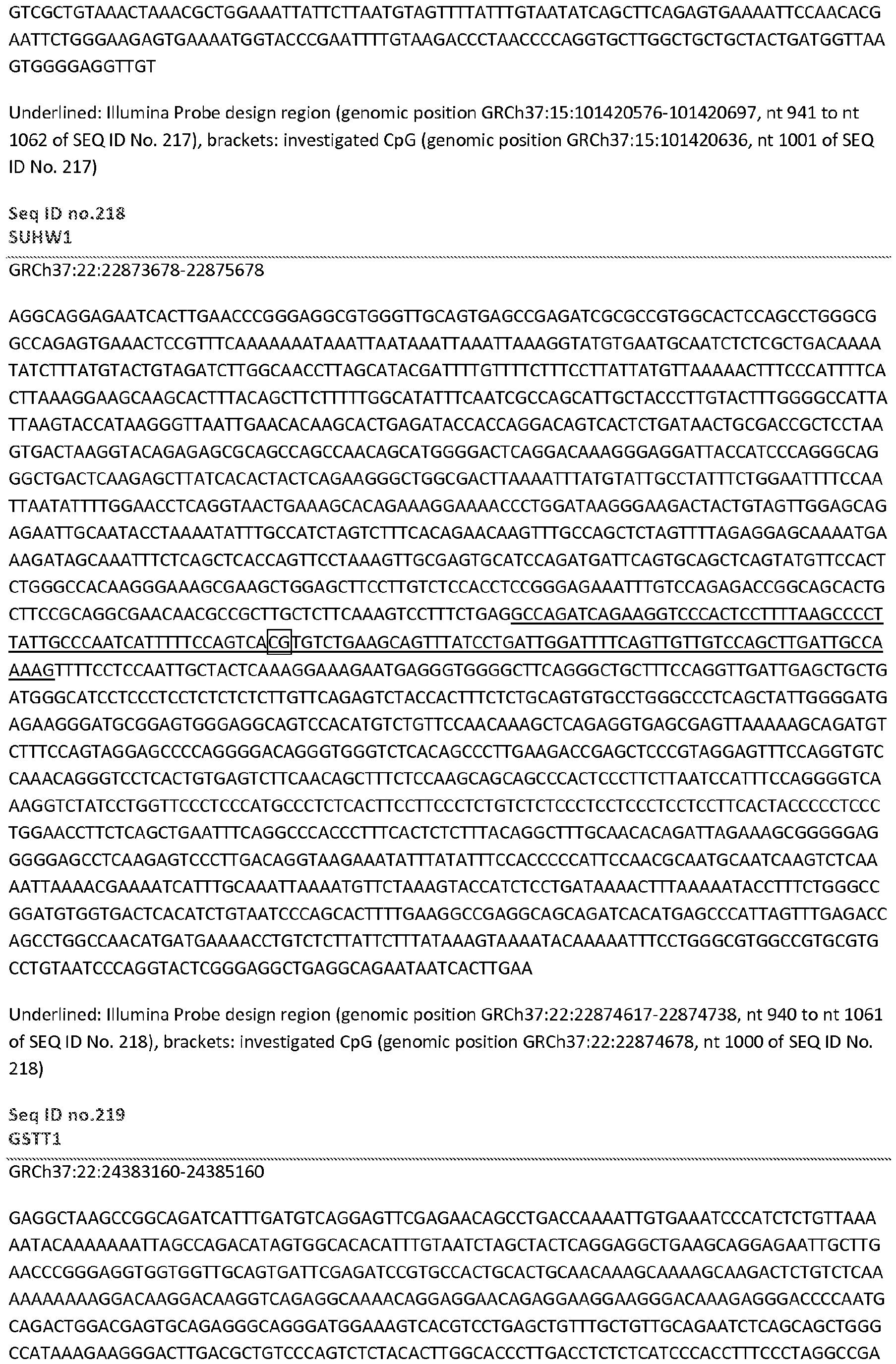

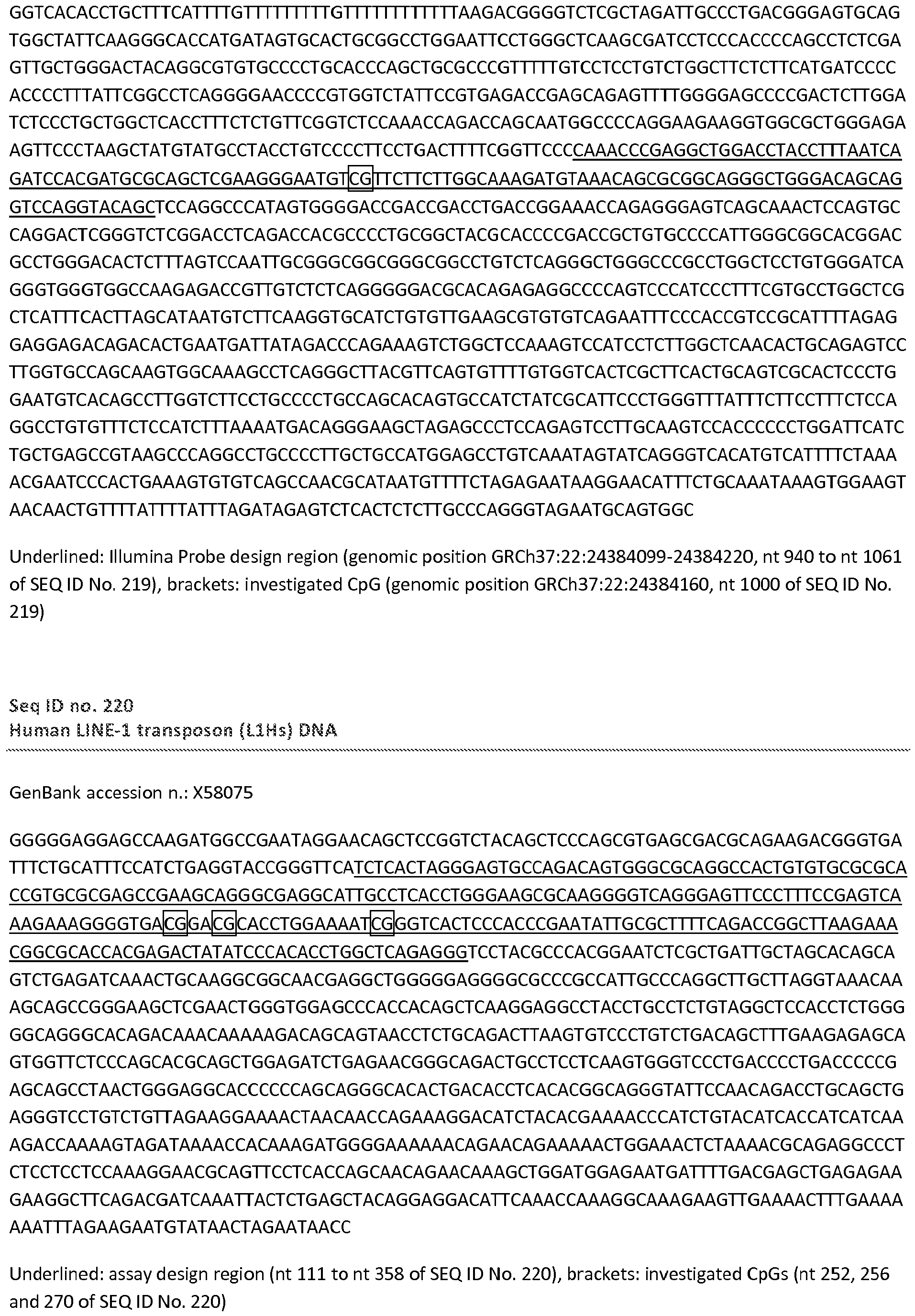

The following examples are intended to illustrate, but not to limit, the scope of the invention. The examples are illustrated by reference to the non limiting following figures.

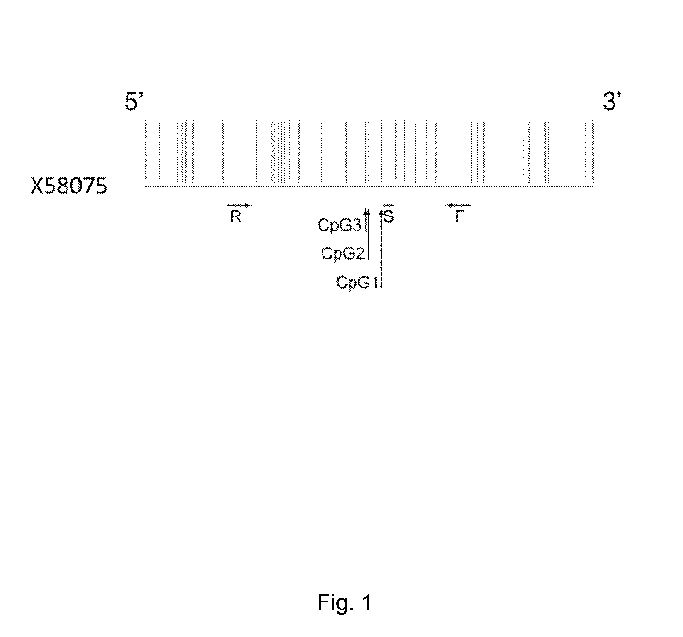

**Figure 1****.** *LINE-1* bisulfite pyrosequencing assay map. The region of the *LINE-1* sequence (GenBank accession n. X58075) utilized for the design of the assay is reported. Vertical bars indicate individual CpG sites. Horizontal lines indicate forward (F), reverse (R) and sequencing (S) primers. Vertical arrows indicate the CpG sites (CpG1, CpG2, CpG3) analyzed by pyrosequencing (adapted from Aparicio et al, Epigenetics, 4:176-84, 2009).

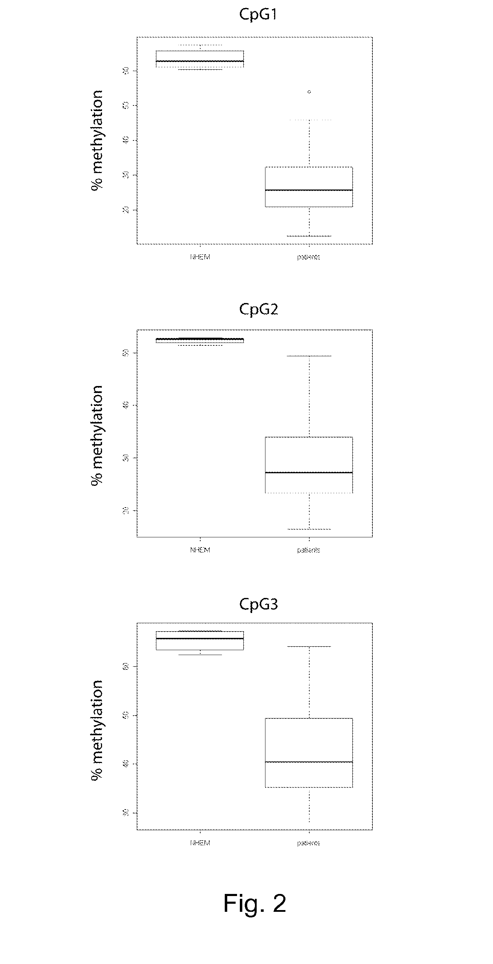

**Figure 2****.** *LINE-1* methylation in stage IIIC CM patients *LINE-1* methylation at 3 CpG sites (CpG1, CpG2, CpG3) was evaluated by bisulfite pyrosequencing analysis in 4 independent primary cultures of Normal Human Epidermal Melanocites (NHEM) and in short-term cultures of CM cells generated from neoplastic lesions of 42 stage IIIC melanoma patients. All cells were analyzed at 6^{th} *in vitro* passage. Separate box plots have been generated for each of the CpG sites under analysis. Black horizontal bars represent the median values of methylation for each group.

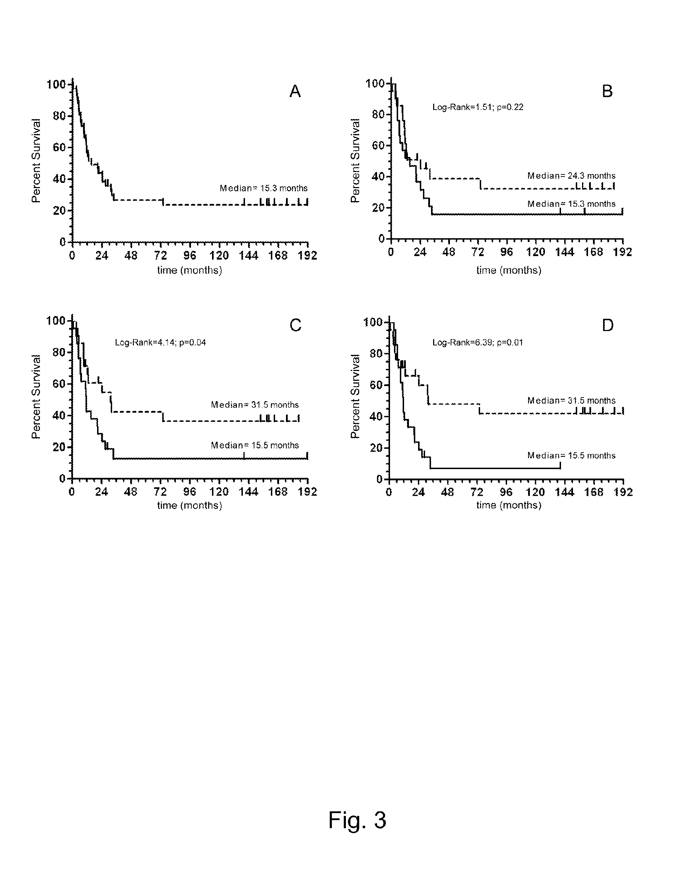

**Figure 3**. Kaplan-Meier analysis of CM patients survival according to *LINE-1* methylation *LINE-1* methylation at 3 CpG sites (CpG1, CpG2, CpG3) was evaluated by bisulfite pyrosequencing analysis in short-term cultures of CM cells generated from neoplastic lesions of 42 stage IIIC melanoma patients. Cells were analyzed at 6^{th} *in vitro* passage. Kaplan-Meyer function for OS was calculated for CM patients either unstratified (A) or stratified according to median methylation of CpG1 (B), CpG2 (C) or CpG3 (D) site of *LINE-1* elements. Dashed and solid lines refer to patients with *LINE-1* methylation below or above the median, respectively. Vertical bars represent censored patients. Cumulative survival by *LINE-1* methylation level was evaluated using the Log-Rank test, reported *P* values were two sided.

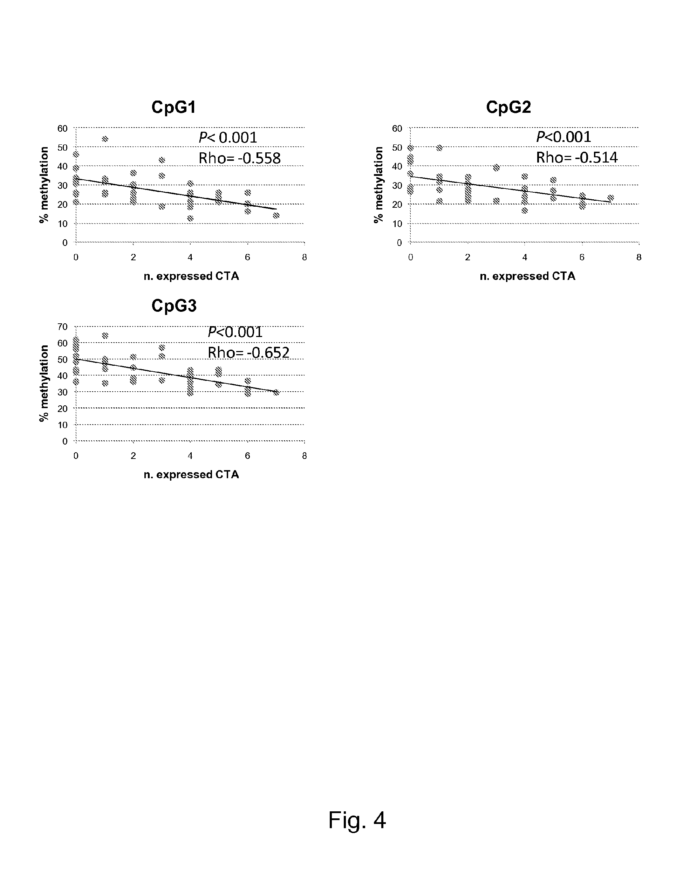

**Figure 4****.** *LINE-1* methylation correlates with the number of CTA concomitantly expressed by CM cells.
   *LINE-1* methylation at 3 CpG sites (CpG1, CpG2, CpG3) was evaluated by bisulfite pyrosequencing analysis in primary cultures of CM cells at the 6^{th} *in vitro* passage. Total RNA was extracted from the same cell cultures and subjected to RT-PCR analyses for the expression of the CTA MAGE-A1, -A2, -A3, -A4, -A10, SSX-2 and NY-ESO-1. For each cell culture, the number of different CTA expressed was evaluated. The correlation between *LINE-1* methylation at each CpG site and the number of expressed CTA was evaluated through Spearman's rank correlation test. P values < 0.05 were considered to be statistically significant.

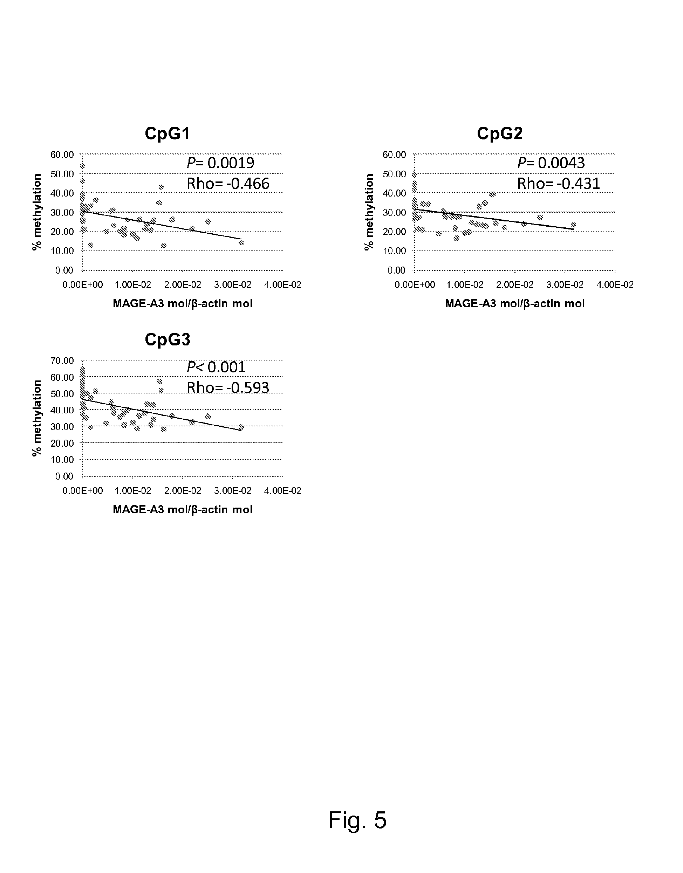

**Figure 5****.** *LINE-1* methylation correlates with the quantitative expression of MAGE-A3 by CM cells.
   *LINE-1* methylation at 3 CpG sites (CpG1, CpG2, CpG3) was evaluated by bisulfite pyrosequencing analysis in primary cultures of CM cells at the 6^{th} *in vitro* passage. Total RNA was extracted from the same cell cultures and subjected to quantitative RT-PCR analysis for the expression of the CTA MAGE-A3 and of the house-keeping gene β-actin. MAGE-A3 expression in each sample was normalized to that of β-actin, and reported as MAGE-A3 molecules/β-actin molecules. The correlation between *LINE-1* methylation at each CpG site and the quantitative levels of expression of MAGE-A3 was evaluated through Spearman's rank correlation test. P values < 0.05 were considered to be statistically significant.

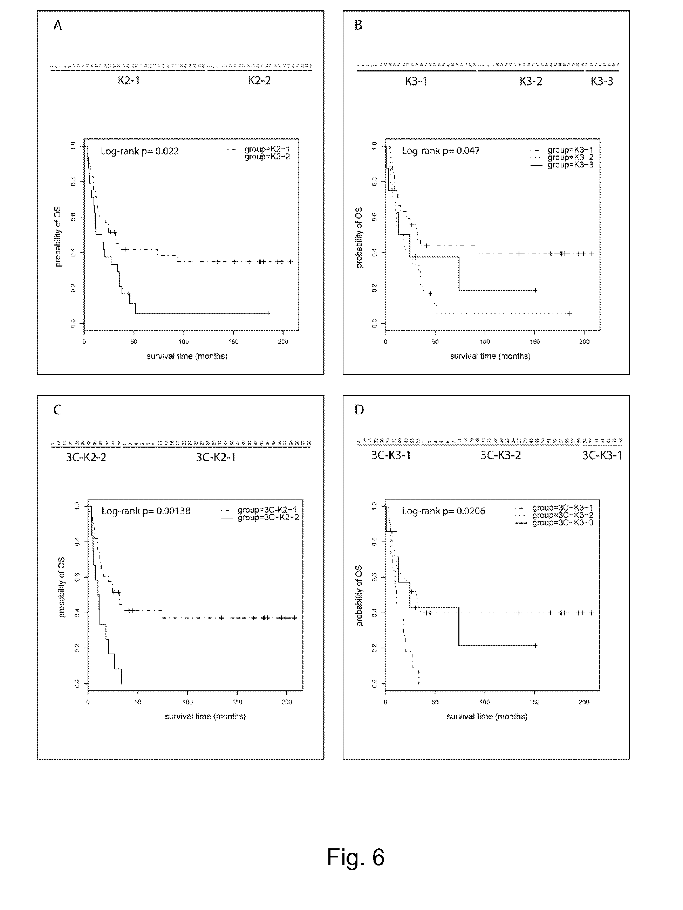

**Figure 6**. Kaplan-Meier analysis of CM patients survival according to genome-wide methylation profiles
   Genome-wide DNA methylation profile was evaluated by Illumina HumanMethylation27 Bead-Chip whole-genome assay in short-term cultures of CM cells generated from neoplastic lesions of stage III CM patients. Cells were analyzed at 6^{th} *in vitro* passage. Patients were divided in either 2 or 3 classes based on the whole genome methylation profile of their tumor cells through the k-means algorithm. Kaplan-Meyer function for OS was calculated for stage III and stage IIIC CM patients stratified according to k-means-defined methylation classes. Vertical bars in the Kaplan-Meyer curves represent censored patients. Cumulative survival by k-means-defined methylation group was evaluated using the Log-Rank test, reported P values were two sided. Numbers above the horizontal bars in the upper portion of panels A-D represent the patients' numeric identifiers, horizontal bars and text below identify the k-means-defined methylation classes. Panel A, B, stage III patients stratified according to two (K2-1, K2-2) or three (K3-1, K3-2, K3-3) k-means-defined methylation classes, respectively; Panel C, D stage IIIC patients stratified according to two (3C-K2-1, 3C-K2-2) or three (3C-K3-1, 3C-K3-2, 3C-K3-3) k-means-defined methylation classes, respectively.

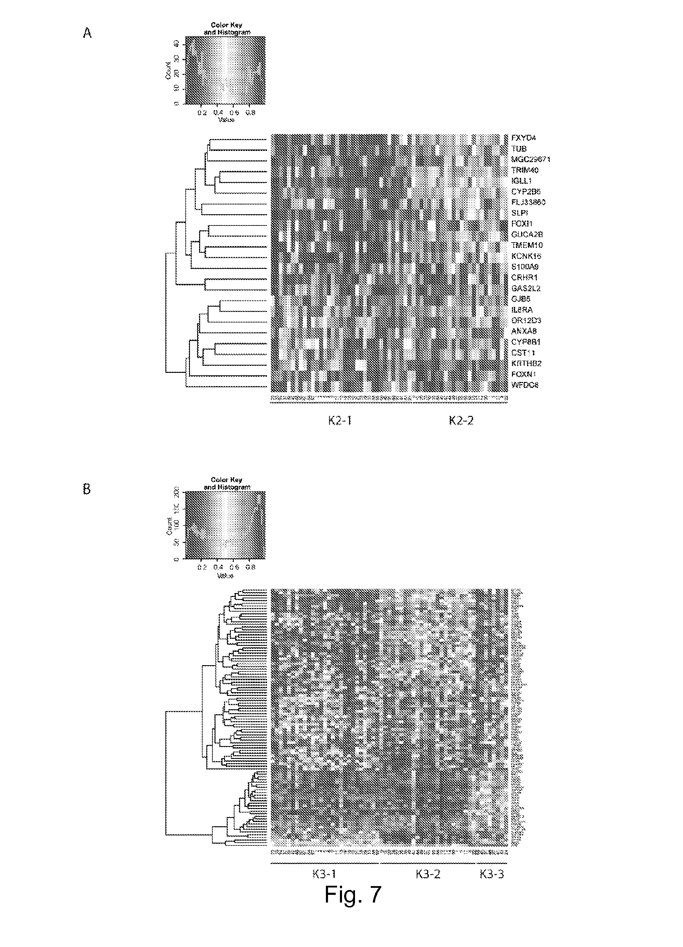

**Figure 7**. Prognostic methylation signatures identified by the "nearest shrunken centroid" algorithm in stage III CM patients.
   Pre-processed genome-wide methylation data from stage III CM patients under study was analyzed through nearest shrunken centroids algorithm to identify genes best characterizing the k-means defined prognostic methylation groups. The methylation values of the genes composing the methylation signatures in the patients' population have been reported as a heatmap. Genes defining the signature have been reported as Gene Symbol on the right of the heatmap, while patient identifier numbers and the k-means-defined classes have been reported on foot of the heatmap. Each color patch represents the methylation level of one gene in each patient, with a continuum of methylation levels from dark blue (completely unmethylated, β=0) to dark red (completely methylated, β=1). Panel A, 24 genes signature classifying patients into 2 k-means-defined classes (K2-1 and K2-2) with an overall error rate of 0.05; Panel B, 98 genes signature classifying patients into 3 k-means-defined classes (K3-1, K3-2, and K3-3) with an overall error rate of 0.034.

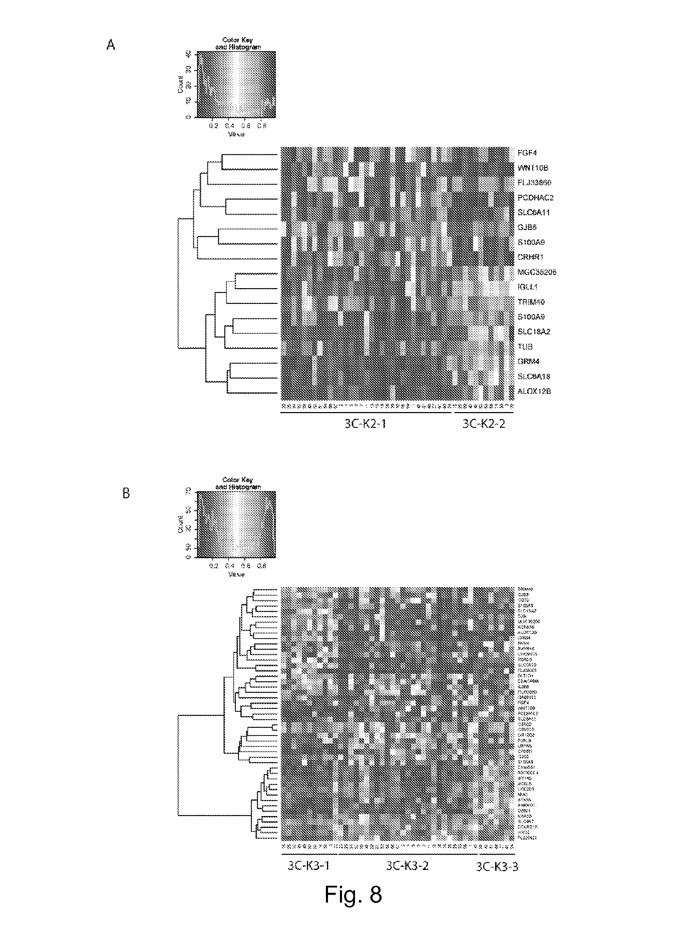

**Figure 8**. Prognostic methylation signatures identified by the "nearest shrunken centroid" algorithm in stage IIIC CM patients.
   Pre-processed genome-wide methylation data from stage IIIC CM patients under study was analyzed through nearest shrunken centroids algorithm to identify genes best characterizing the k-means defined prognostic methylation groups. The methylation values of the genes composing the methylation signatures in the patients' population have been reported as a heatmap. Genes defining the signature have been reported as Gene Symbol on the right of the heatmap, while patient identifier numbers and the k-means-defined classes have been reported on foot of the heatmap. Each color patch represents the methylation level of one gene in each patient, with a continuum of methylation levels from dark blue (completely unmethylated, β=0) to dark red (completely methylated, β=1). Panel A, 17 genes signature classifying patients into 2 k-means-defined classes (3C-K2-1 and 3C-K2-2) with an overall error rate of 0; Panel B, 47 genes signature classifying patients into 3 k-means-defmed classes (3C-K3-1, 3C-K3-2, and 3C-K3-3) with an overall error rate of 0.022.

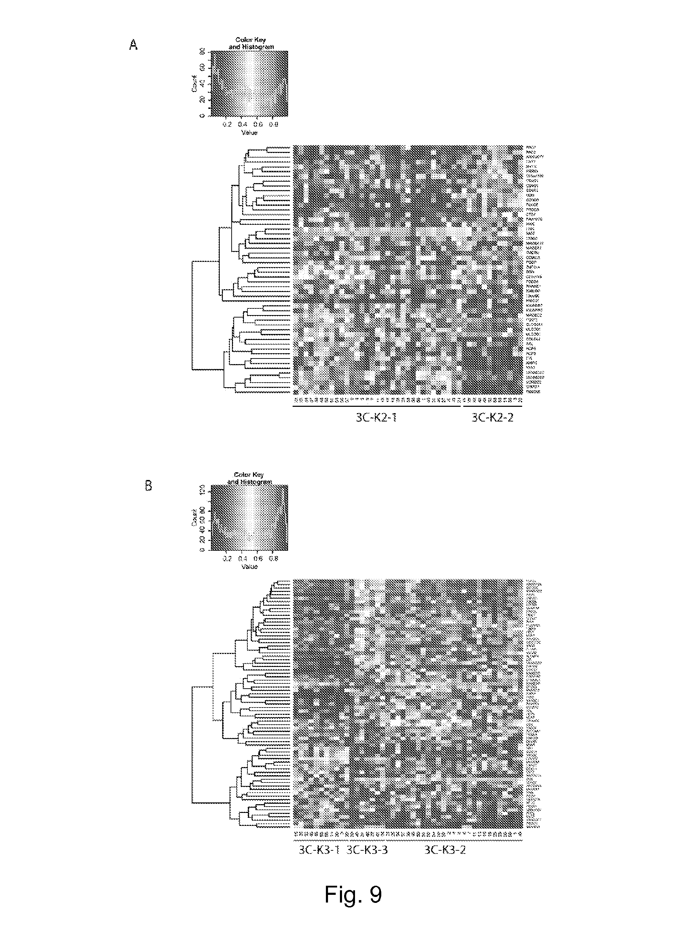

**Figure 9**. Prognostic methylation signatures identified by the "nearest shrunken centroid" algorithm in stage IIIC CM patients using correlation between gene methylation and gene expression (p≤0.05).
   Pre-processed genome-wide methylation data from stage IIIC CM patients under study was analyzed through nearest shrunken centroids algorithm to identify genes best characterizing the k-means defined prognostic methylation groups. The analysis has been restricted to 820 methylation probes showing significant association between gene methylation and gene expression values in the same patient population, as evaluated by Spearman test. The methylation values of the genes composing the methylation signatures in the patients' population have been reported as a heatmap. Genes defining the signature have been reported as Gene Symbol on the right of the heatmap, while patient identifier numbers and the k-means-defined classes have been reported on foot of the heatmap. Each color patch represents the methylation level of one gene in each patient, with a continuum of methylation levels from dark blue (completely unmethylated, β=0) to dark red (completely methylated, β=1). Duplicate Gene Symbol entries refer to methylation data read for the same gene from different probes. Panel A, 51 genes signature classifying patients into 2 k-means-defined classes (3C-K2-1 and 3C-K2-2) with an overall error rate of 0.022; Panel B, 73 genes signature classifying patients into 3 k-means-defined classes (3C-K3-1, 3C-K3-2, and 3C-K3-3) with an overall error rate of 0.

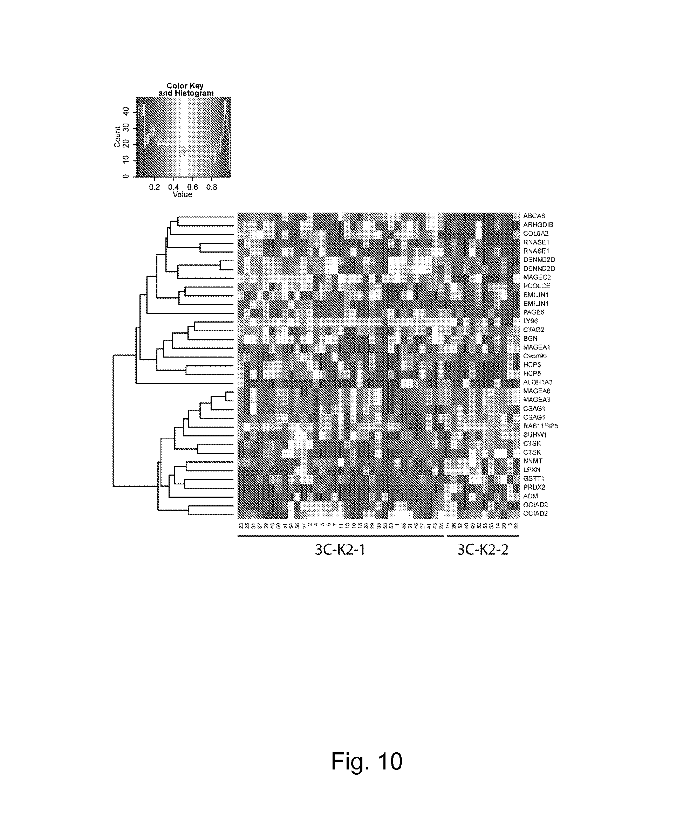

**Figure 10**. Prognostic methylation signatures identified by the "nearest shrunken centroid" algorithm in stage IIIC CM patients using correlation between gene methylation and gene expression (p≤0.05, Rho≤-0.4).
   Pre-processed genome-wide methylation data from stage IIIC CM patients under study was analyzed through nearest shrunken centroids algorithm to identify genes best characterizing the k-means defined prognostic methylation groups. The analysis has been restricted to 274 methylation probes showing significant (p≤0.05) association between gene methylation and gene expression values in the same patient population, and with a coefficient of correlation Rho≤-0.4, as evaluated by Spearman test. The methylation values of the genes composing the methylation signatures in the patients' population have been reported as a heatmap. Genes defining the signature have been reported as Gene Symbol on the right of the heatmap, while patient identifier numbers and the k-means-defined classes have been reported on foot of the heatmap. Each color patch represents the methylation level of one gene in each patient, with a continuum of methylation levels from dark blue (completely unmethylated, β=0) to dark red (completely methylated, β=1). Duplicate Gene Symbol entries refer to methylation data read for the same gene from different probes.

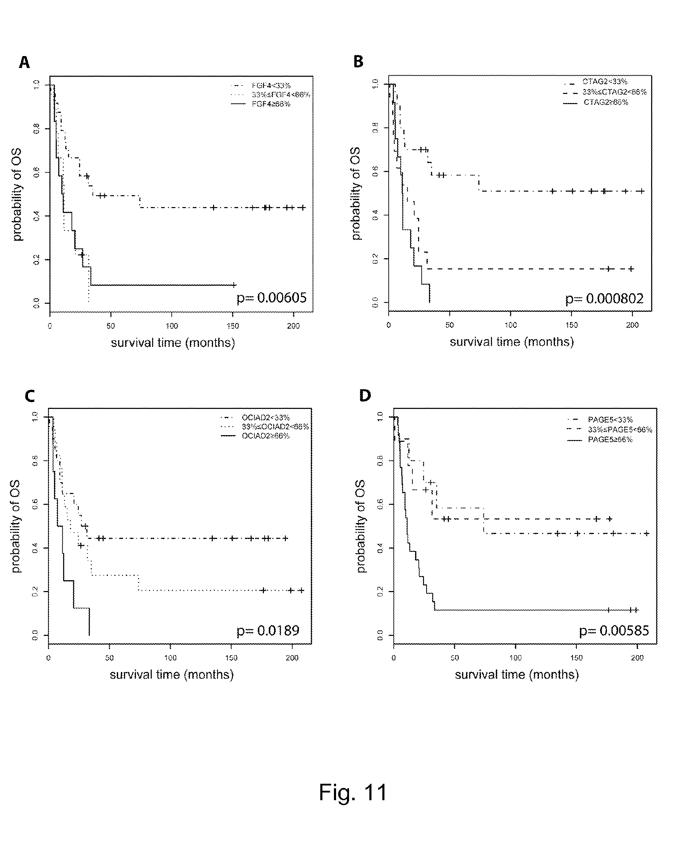

**Figure 11**. Kaplan-Meier analysis of stage IIIC CM patients survival according to *FGF4*, *CTAG2, OCIAD2* or *PAGE5* methylation status.
   Genome-wide DNA methylation profile was evaluated by Illumina HumanMethylation27 Bead-Chip whole-genome assay in short-term cultures of CM cells generated from neoplastic lesions of stage IIIC CM patients. Cells were analyzed at 6^{th} *in vitro* passage. The methylation level of the genes *FGF4* (panel A), *CTAG2* (panel B), *OCIAD2* (panel C) and *PAGE5* (panel D), selected from the whole-genome defined prognostic methylation signatures E and G, have been utilized to subdivide patients according to the methylation level of each gene being <33%, between 33% and 66%, or > 66%. Kaplan-Meyer function for OS was calculated for stage IIIC patients stratified according to the methylation level of each gene. Vertical bars in the Kaplan-Meyer curves represent censored patients. Cumulative survival by methylation group was evaluated using the Log-Rank test, reported P values were two sided.

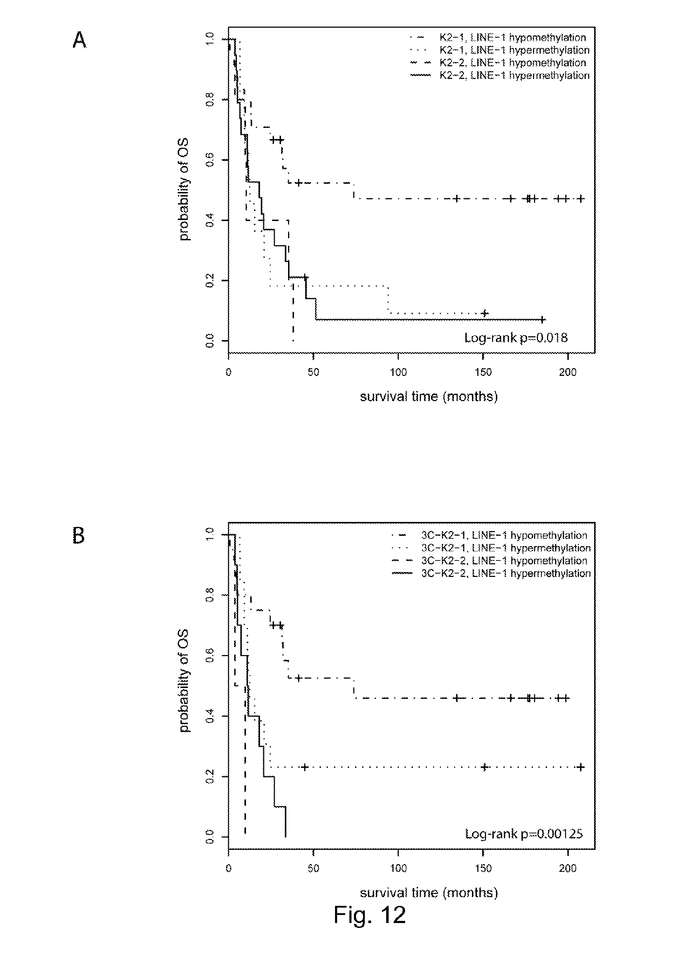

**Figure 12**. Kaplan-Meier analysis of CM patients survival according to genome-wide methylation profiles and *LINE-1* methylation status
   Genome-wide DNA methylation profile was evaluated by Illumina HumanMethylation27 Bead-Chip whole-genome assay in short-term cultures of CM cells generated from neoplastic lesions of stage III CM patients. Cells were analyzed at 6^{th} *in vitro* passage. Patients were scored as K2-1 or k2-2 (stage III patients) or as S3-K2-1 or S3-K2-2 (substage IIIC patients) based on the whole genome methylation profile of their tumor cells through the k-means algorithm. Concomitantly, short-term cultures of CM cells were evaluated for the extent of *LINE-1* methylation by pyrosequencing, and scored as hypomethylated or hypermethylated depending on their value being < or ≥ median value, respectively. Kaplan-Meyer function for OS was calculated for stage III (panel A) and stage IIIC (panel B) CM patients stratified according to both genome-wide methylation profile-defined classes and *LINE-1* methylation. Vertical bars in the Kaplan-Meyer curves represent censored patients. Cumulative survival by methylation group was evaluated using the Log-Rank test, reported P values were two sided.

### DETAILED DESCRIPTION OF THE INVENTION

### Example 1 - Methylation levels of the "long interspersed nucleotide element-1" repetitive sequences predict survival of melanoma patients

### Introduction

The prognosis of cutaneous melanoma (CM) differs for patients with identical clinico-pathological stage, and no molecular markers discriminating the prognosis of stage III individuals have been established. Genome-wide alterations in DNA methylation are a common event in cancer. This example defines the prognostic value of genomic DNA methylation levels in stage III CM patients.

Overall level of genomic DNA methylation was measured using bisulfite pyrosequencing at three CpG sites (CpG1, CpG2, CpG3) of the *Long Interspersed Nucleotide Element-1* (*LINE-1*) sequences in short-term CM cultures from 42 stage IIIC patients. The impact of *LINE-1* methylation on overall survival (OS) was assessed using Cox regression and Kaplan-Meier analysis.

Here the authors provide the first evidence that the methylation level of *LINE-1* repetitive elements has a significant prognostic value in stage IIIC CM patients. Specifically, patients whose tumors exhibit more hypomethylated *LINE-1* sequences had a significantly longer overall survival. The reported data have significant prospective practical clinical implications. Indeed, it can be expected that the evaluation of *LINE-1* methylation status will: i) provide CM patients with improved clinico-pathological sub-staging information; ii) allow physicians to personalize follow-up procedures for CM patients ; iii) guide the future selection and/or stratification of patients for trials of adjuvant treatment; iv) select patients who are most likely to benefit from therapy, including DNA hypomethylating drugs. Overall, the evaluation of *LINE-1* methylation in the routine clinico-pathological ascertainment of CM patients is anticipated to greatly help in personalizing their comprehensive clinical management.

### Materials and methods

### Patients and cell cultures

Short-term cell cultures were established from metastatic lesions removed surgically from consecutive CM patients referred to the National Cancer Institute of Aviano (Italy) for stage III surgery from 1991 to 2007, as previously described (Altomonte et al., 1993). Autologous tumor cell cultures were successfully established from 30% of patients. The micrometastatic nature of lymph-node tumor tissues from AJCC stage IIIA patients precluded their use for cell culture generation, while short-term CM cultures were available only from 12 stage IIIB patients, and were excluded from the statistical analyses. Thus, the planned studies were conducted on a total of 42 available short-term cultures, identified as having been generated from CM patients classified as AJCC stage IIIC. Short-term CM cell cultures were grown in RPMI 1640 Medium (Biochrome KG, Berlin, Germany) supplemented with 20% heat-inactivated fetal calf serum (Biochrome KG) and 2mM L-glutamine (Biochrome KG). Four independent cultures of NHEM were purchased from Invitrogen (Milan, Italy), Gentaur (Brussels, Belgium), Provitro (Berlin, Germany), and ScienCell (Carlsbad, CA, USA), and were maintained in M254 Medium supplemented with Human Melanocyte Growth Supplement (Invitrogen). To minimize alterations potentially arising with extended *in vitro* culturing, all cell cultures were utilized for molecular assays at the 6^{th} *ex vivo* passage.

### LINE-1 bisulfite pyrosequencing analysis

Genomic DNA was extracted from short-term cultures of CM cells by proteinase K treatment followed by standard phenol/chloroform extraction and ethanol precipitation (Ausubel et al., 1998). Bisulfite conversion was carried out on 500 ng genomic DNA using EZ DNA Methylation-Gold^{™} Kit (Zymo Research, Orange, CA, USA), according to the manufacturer's protocol. Methylation analysis of the *LINE-1* elements was performed as previously described (Yang et al., 2004), with minor modifications. *LINE-1* elements were amplified using 50 pmol each of forward primer 5'-TTTTTTGAGTTAGGTGTGGG-3' (SEQ ID No. 221) and reverse biotinylated primer 5'-TCTCACTAAAAAATACCAAACAA-3' (SEQ ID No. 222) in a 50 µL reaction volume containing 2.5 ng of bisulfite-treated DNA, 1x PCR buffer, 1.5 mM MgCl₂ and 1.25 U of Platinum Taq DNA polymerase (Invitrogen). PCR thermal amplification profile consisted of an initial denaturation step of 5 min at 95°C, followed by 50 cycles of 30 s at 95°C, 30 s at 58°C, and 1 min at 72°C. The PCR product was purified using Streptavidin Sepharose High Performance beads (Amersham Biosciences, Uppsala, Sweden) and denatured using 0.2 mol/L of NaOH solution. Next, 0.3 µmol/L of the sequencing primer (5'-GGGTGGGAGTGAT-3') (SEQ ID No. 223) was annealed to the purified single-stranded PCR product and the Pyrosequencing reaction was performed using the PSQ HS 96 Pyrosequencing System (Pyrosequencing, Inc., Westborough, MA). The level of methylation for each of the 3 analyzed CpG sites (CpG1, CpG2, CpG3) was expressed as the percentage of methylated cytosines over the sum of methylated and unmethylated cytosines (Figure 1).

### Statistical analysis

The primary objective was to determine differences in survival among various *LINE-1* DNA methylation level groups. *LINE-1* DNA methylation levels were dichotomized using median value into the following categories: CpG1 (<25.68, ≥25.68), CpG2 (<27.26, ≥27.26), and CpG3 (<40.46, ≥40.46). The characteristics including age, gender, primary tumor localization, Breslow thickness, Clark level, and ulceration of the primary tumor, number of lymph nodes involved, and pre-operative serum LDH values were examined. Survival time was calculated in months from the date of stage IIIC diagnosis until the date of death. According with the specific goals of the analysis, the authors did not classify the deaths considering their cause. Patients were censored at the last follow-up date or the last date the patient was last known to be alive. Median survival duration was determined by the Kaplan-Meier method (Kaplan and Meier, 1958). Cumulative survival by DNA methylation level was evaluated using the log-rank test. P values were two sided and values <0.05 were considered to be statistically significant. Cox proportional hazard method (Cox, 1972) was used to examine the effect of DNA methylation level on survival and results were presented as Hazard Ratios (HR) with corresponding 95% Confidence Intervals (CI). *LINE-1* methylation was also entered in the model as a continuous variable with the unit set at 10% of methylation. Patient characteristics and clinical and pathologic features were examined by univariate Cox model. The statistical analyses were carried out using the SAS Software version 9.13 (SAS Institute Inc., Cary, North Carolina, USA).

### Results

### Patients

The study was conducted on CM patients who underwent radical lymph node dissection for stage III disease at the Centro di Riferimento Oncologico National Cancer Institute between 1991 and 2007. Patients diagnosed with a stage IIIC disease, and for whom a short-term cell culture had been successfully generated from the surgically removed autologous neoplastic tissue, were included in the study. Table 3 summarizes the 42 patients under study and their clinico-pathologic characteristics at presentation.

**Table 3. Characteristics of the 42 AJCC stage IIIC melanoma patients**

| **Variable** | | **n. patients** | **%** |
|---|---|---|---|
| Age, years | | | |
| | Median | 54 | |
| | Range | 29-83 | |
| Gender | | | |
| | Male | 27 | 64 |
| | Female | 15 | 36 |
| Localization of primary tumor | | | |
| | extremities | 14 | 33 |
| | trunk | 23 | 55 |
| | head & neck | 3 | 7 |
| | NA* | 2 | 5 |
| | | | |
| Breslow thickness of primary tumor | | | |
| | ≤2.0 mm | 13 | 31 |
| | >2.0 mm | 22 | 52 |
| | NA | 7 | 17 |
| | | | |
| Clark level of primary tumor | | | |
| | 1- 3 | 12 | 29 |
| | 4-5 | 24 | 57 |
| | NA | 6 | 14 |
| | | | |
| Ulceration of primary tumor | | | |
| | No | 10 | 24 |
| | Yes | 30 | 71 |
| | NA | 2 | 5 |
| | | | |
| N. lymph nodes involved | | | |
| | 1 | 9 | 21 |
| | >1 | 33 | 79 |
| | | | |
| LDH | | | |
| | Low_{†} | 28 | 67 |
| | High | 11 | 26 |
| | NA | 3 | 7 |

| | | | |
|---|---|---|---|
| *NA, not available ^{†} low LDH is established as LDH values ≤ 0.8 times the upper limit of normal, high LDH is established as LDH values > 0.8 times the upper limit of normal | | | |

### Extent of LINE-1 methylation in CM patients

To define if CM undergoes changes in the overall content of 5-methylcytosine, bisulfite pyrosequencing analyses were utilized to compare the extent of methylation of *LINE-1* repetitive elements in the 42 short-term CM cell cultures under study to that of primary cultures of NHEM. *LINE-1* sequences were heavily methylated at all investigated CpG sites in NHEM (CpG1: median 62.82%, range 60.43%-67.53%; CpG2: median 52.57%, range 51.37%-52.87%; CpG3: median 65.77%, range 62.40%-67.33%; Figure 2).

On the other hand, much lower and largely heterogeneous levels of methylation of the *LINE-1* elements were observed in CM cells from stage IIIC patients (CpG1: median 25.68%, range 12.45%-54.05%; CpG2: median 27.26%, range 16.50%-49.43%; CpG3: median 40.46%, range 28.10%-64.15%; Figure 2), demonstrating that highly variable alterations in the overall level of genomic DNA occur in CM.

### Prognostic value of LINE-1 methylation in CMpatients

The highly heterogeneous levels *of LINE-1* methylation observed in CM cells from stage IIIC patients led us to investigate whether they correlated with a different clinical outcome of patients under study.

Kaplan-Meier analysis indicated that median OS for stage IIIC CM patients under analysis was 15.3 months (95% CI, 11.0-31.5; Figure 3A). To evaluate the association between *LINE-1* methylation status and OS, patients were divided according to the median value of methylation of each analyzed CpG site (CpG1=25.68%; CpG2=27.26%; CpG3=40.46%). Patients were defined as having hypomethylated or hypermethylated *LINE-1* sequences, depending on the methylation level being below or above the median value for each group, respectively. Kaplan-Meier analysis showed a trend towards an increased OS rate for patients with hypomethylated CpG1, however, the difference did not reach statistical significance (P=0.22, log-rank=1.51; Figure 3B). On the other hand, a significant survival advantage was observed in patients with CpG2<27.26% as compared to patients with CpG2≥27.26% (P=0.04, log-rank=4.14) (Figure 3C). Similarly, the survival rate of patients with CpG3<40.46% was significantly higher than that of patients with CpG3≥40.46% (P=0.01, log-rank=6.39) (Figure 3D). In line with these data, median OS of patients with hypomethylated CpG1, CpG2 and CpG3 sites was 24.3, 31.5, and 31.9 months, respectively, as compared to 15.3, 11.5, and 11.5 months of patients with hypermethylated *LINE-1* CpGs (Figure 3, Table 4).

**Table 4. OS of stage IIIC CM patients according to LINE-1 methylation**

| **LINE1 CpG site** | **Extent methylation*** | **Median OS (95%CI)^{†}** | **5 year OS (%)** |
|---|---|---|---|
| **CpG1** | <25.68 | 24.3 (11.1-inf) | 39 |
| | ≥25.68 | 15.3 (6.8-26.9) | 16 |
| **CpG2** | <27.26 | 31.5 (12.5-inf) | 43 |
| | ≥27.26 | 11.5 (6.8-20.9) | 13 |
| **CpG3** | <40.46 | 31.9 (13.1-inf) | 48 |
| | ≥40.46 | 11.5 (9.2-20.6) | 7 |

| | | | |
|---|---|---|---|
| * Patients were divided according to the % of methylation of the specified CpG site being < or ≥ the median % methylation measured in the examined patients' population; ^{†} Survival functions were calculated by the Kaplan-Meier method. Data are reported as Median OS in months, together with the corresponding 95% Confidence Intervals (CI). | | | |

Accordingly, the 5 year OS was 39%, 43%, and 48% for patients with hypomethylated CpG1, CpG2, and CpG3 sites, respectively, as compared to 16%, 13%, and 7% of patients with hypermethylated *LINE-1* CpGs (Table 4).

Cox univariate analysis was carried out to identify patient characteristics and clinico-pathologic factors that predicted survival. Among all factors examined, including age, gender, localization of primary tumor, Breslow thickness, Clark level and ulceration of primary tumor, number of lymph nodes involved, and level of pre-operative LDH, only CpG2 methylation (HR=2.12 for CpG2≥27.26% vs. CpG2<27.26; 95% CI: 1.01-4.44; P=0.04) and CpG3 methylation (HR=2.63 for CpG2≥40.46% vs. CpG2<40.46; 95% CI: 1.21-5.69; P=0.01) were associated with statistically significant differences in OS (Table 5). When *LINE-1* methylation was analyzed as a continuous variable, a statistically significant inverse association emerged between OS and an increase of 10% of methylation of CpG1 (HR=1.51; 95%CI:1.06-2.16; P=0.02), CpG2 (HR=1.60; 95%CI:1.02-2.52; P=0.04) and CpG3 (HR=1.49; 95%CI:1.06-2.09; P=0.02) (Table 5).

**Table 5. Cox analysis of the influence of LINE-1 methylation on OS of stage IIIC CM patients**

| **LINE1 CpG site** | **Extent methylation*** | **HR**^{†} | **95% CI; *P* value** | **HR_{cont.}**^{‡} | **95% CI; *P* value** |
|---|---|---|---|---|---|
| **CpG1** | <25.68 | 1^{§} | | 1.51 | 1.06-2.16; 0.02 |
| | ≥25.68 | 1.57 | 0.76-3.24; 0.22 | | |
| **CpG2** | <27.26 | 1 | | 1.60 | 1.02-2.52; 0.04 |
| | ≥27.26 | 2.12 | 1.01-4.44; 0.04 | | |
| **CpG3** | <40.46 | 1 | | 1.49 | 1.06-2.09; 0.02 |
| | ≥40.46 | 2.63 | 1.21-5.69;. 0.01 | | |

| | | | | | |
|---|---|---|---|---|---|
| * Patients were divided according to the % of methylation of the specified CpG site being <or ≥ the median % methylation measured in the examined patients' population; ^{†} Cox proportional hazard method was used to examine the effect of *LINE-1* methylation on OS. Results were presented as Hazard Ratios (HR) with corresponding 95% Confidence Intervals (CI); ^{‡} *LINE-1* methylation was also evaluated as continuous variable. The HR value is that of the *LINE-1* methylation relative to an increase of 10 %; ^{§} set as reference. | | | | | |

### Discussion

In this study the authors demonstrate that the global level of *LINE-1* methylation of short-term tumor cell cultures grown from patients with nodal disease is a significant predictor of OS in stage IIIC CM patients. This finding is of remarkable clinical relevance, since, to the best of our knowledge, it provides the first evidence of a molecular marker capable of differentiating the prognosis of CM patients in this high-risk substage. These results are of particular emphasis given the conduct of this study in subjects within a single clinically well-defined clinico-pathological staging sub-group, which has become the focus of several ongoing clinical trials in the US and Europe (i.e., ECOG intergroup trial E4697, EORTC trial 18071, GSK trial 111482 "DERMA").

Genomic DNA hypomethylation has been proposed to have an important impact on tumor biology through the generation of chromosomal instability, reactivation of transposable elements, and loss of imprinting (Esteller, 2008). Thus, a negative correlation between genomic hypomethylation and survival of CM patients could have been expected. Instead, the authors found that hypomethylation of *LINE-1* elements at CpG2 or CpG3 sites was associated with a significantly better OS, as demonstrated by Kaplan-Meier analysis and log-rank test. The positive prognostic value of *LINE-1* hypomethylation the authors have identified in CM is in sharp contrast with data most recently obtained in colon and ovarian cancer patients, in which *LINE-1* hypomethylation in neoplastic tissues was associated with a poorer prognosis (Ogino et al., 2008; Pattamadilok et al., 2008). Since CM behaves differently from other solid tumors, the underlying biological effect(s) of *LINE-1* hypomethylation on patients' outcome could depend on the tumor histotype. Nevertheless, it should be emphasized that our findings are generated from patients in the same clinico-pathological stage of disease, while the studies on ovarian and colon cancer were conducted on the heterogeneous patients population as a whole, and did not investigate the prognostic potential of *LINE-1* methylation in specific clinically defined stages of disease.

The mechanism(s) through which *LINE-1* hypomethylation affects survival of CM patients remains to be fully explored; however, some speculation can be made, based on recent data in the literature. Tellez *et al* (Tellez et al., 2009) have demonstrated that higher levels of *LINE-1* methylation correlate with an increased number of aberrantly hypermethylated tumor suppressor genes (TSG) in cultured melanoma cell lines. Thus, epigenetic inactivation of TSG might account for more aggressive disease the authors have observed in patients with elevated *LINE-1* methylation in their neoplastic cells. This notion is in accordance with initial studies reporting a negative association between survival and the presence of hypermethylated *ER-*α, *RASSF1A, RAR-*β*2, or MINT31* DNA in neoplastic tissues or sera of stage III/IV CM patients (Mori et al., 2006; Mori et al., 2005; Tanemura et al., 2009). On the other hand, hypomethylation, and consequent transcriptional activation, of *LINE-1* elements might *per se* reduce the tumorigenic potential of neoplastic cells by triggering apoptosis and a senescence-like state through the activity of the second open reading frame of *LINE-1* (Wallace et al., 2008). Furthermore, genomic DNA hypomethylation has been associated with the *de novo* expression of tumor associated antigens belonging to the Cancer Testis Antigen (CTA) class by neoplastic cells of different histotype (De Smet et al., 1996; Sigalotti et al., 2002; Woloszynska-Read et al., 2008), and the authors have recently identified a significant correlation between a hypomethylated status of *LINE-1* elements and increased presence, levels and total number of CTA concomitantly expressed in short-term cultures of CM cells (see example 2). Thus, it is intriguing to speculate that a better immune recognition of *LINE-1* hypomethylated CM cells might contribute to the improved survival of these patients.

Irrespective of the underlying biological mechanism(s) triggered by *LINE-1* hypomethylation, the prognostic value of *LINE-1* methylation here identified for stage IIIC CM patients bears several important practical clinical implications. Among these, the goal to provide CM patients with improved clinico-pathological sub-stage and/or follow-up-procedures would be enhanced using *LINE-1* methylation status, and these findings might be used to select and/or stratify patients for adjuvant treatment based on the methylation level of *LINE-1* in their tumors. In addition, the significant positive prognosis of *LINE-1* hypomethylated patients should prompt the incorporation of this in new studies aimed at understanding whether pharmacologic DNA hypomethylation (Sigalotti et al., 2007) could be regarded as a feasible chemoprevention approach in the initial phases of disease and/or in patients at high-risk of disease recurrence.

The authors' present findings will be further investigated in prospective multicenter studies in which the prognostic significance and the predictive value for different treatments of CM will be validated. Providing further support to our data will undoubtedly add the evaluation of *LINE-1* methylation into the routine clinico-pathological ascertainment of CM patients, helping to personalize their comprehensive clinical management

### Conclusions

Hypomethylation (i.e., methylation below median) at CpG2 and CpG3 sites significantly associated with improved prognosis of CM, CpG3 showing the strongest association. Patients with hypomethylated CpG3 had increased OS (P=0.01, log-rank=6.39) by Kaplan-Meyer analysis. Median OS of patients with hypomethylated or hypermethylated CpG3 were 31.9 and 11.5 months, respectively. The 5 year OS for patients with hypomethylated CpG3 was 48% compared to 7% for patients with hypermethylated sequences. Among the variables examined by Cox regression analysis, *LINE-1* methylation at CpG2 and CpG3 was the only predictor of OS (Hazard Ratio=2.63, for hypermethylated CpG3; 95% Confidence Interval: 1.21-5.69; P=0.01).

*LINE-1* methylation is identified as a molecular marker of prognosis for CM patients in stage IIIC. Evaluation of *LINE-1* promises to represent a key tool for driving the most appropriate clinical management of stage III CM patients.

### Example 2 - Methylation levels of the "long interspersed nucleotide element-1" repetitive sequences correlate with presence and levels of expression of cancer testis antigens in cutaneous melanoma

Cancer Testis Antigens (CTA) represent an important class of immunogenic tumor antigens that are currently utilized as targets in several cancer immunotherapy approaches. The expression of CTA has been shown to depend on the methylation status of their promoters. In view of the therapeutic relevance of CTA expression and of its regulation by promoter methylation, the authors asked the question whether *LINE-1* methylation might correlate with CTA expression in CM cells, thus representing a surrogate marker for their presence and levels of expression. To this end, *LINE-1* methylation was measured in CM cells by pyrosequencing assays and CTA expression was evaluated by qualitative or quantitative RT-PCR analyses. The correlation between *LINE-1* methylation and CTA expression was evaluated through Spearman's rank correlation test.

### Materials and methods

### LINE-1 methylation

The extent of methylation of *LINE-1* elements was measured in primary cultures of CM cells at the 6^{th} *in vitro* passage by bisulfite pyrosequencing as reported in example 1.

### Qualitative and Quantitative RT-PCR analyses

Total RNA was extracted from CM cells at the *6^{th} in vitro* passage using the TRIzol reagent (Invitrogen, Milan, Italy) following manufacturer's recommendations. RT-PCR reactions were performed as previously described (Coral et al., 1999). The oligonucleotide primer sequences and gene-specific PCR amplification programs utilized have been defined for MAGE-A1, -A2, -A3, -A4, -A10 (De Plaen et al., 1994; Rimoldi et al., 1999), NY-ESO-1 (Jager et al., 1998), SSX-2 (Gure et al., 1997). The integrity of each RNA and oligo-dT-synthesized cDNA sample was confirmed by the amplification of the β-actin housekeeping gene (Coral et al., 1999). Ten µl of each RT-PCR sample were run on a 2% agarose gel and visualized by ethidium bromide staining.

Real-time quantitative RT-PCR analyses were performed as previously described (Calabro et al., 2005). Briefly, total RNA was digested with RNase free DNase (Roche Diagnostics, Milan, Italy) to remove contaminating genomic DNA. Synthesis of cDNA was performed on 1 µg total RNA using MMLV reverse transcriptase (Invitrogen) and random hexamer primers (Promega, Milan, Italy), following manufacturers' instructions. TaqMan quantitative RT-PCR reactions were performed on 10 ng of retrotranscribed total RNA in a final volume of 25 µl TaqMan Universal Master Mix (Applyed Biosystems, Milan, Italy) at 95°C for 10 min, followed by 45 cycles at 95°C for 15 sec and at 60°C for 1 min. TaqMan primers/probe sets were as follows: β-actin forward CGAGCGCGGCTACAGCTT (SEQ ID No. 224), β-actin reverse CCTTAATGTCACGCACGATT (SEQ ID No. 225), β-actin probe FAM-ACCACCACGGCCGAGCGG-BHQ1 (SEQ ID No. 226); MAGE-A3 forward TGTCGTCGGAAATTGGCAGTAT (SEQ ID No. 227), MAGE-A3 reverse CAAAGACCAGCTGCAAGGAACT (SEQ ID No. 228), MAGE-A3 probe FAM-TCTTTCCTGTGATCTTC-MGB (SEQ ID No. 229). Measurement of gene expression was performed utilizing the ABI PRISM 7000 Sequence Detection System (Applyed Biosystems) and the copy number of MAGE-A3 and of the reference gene β-actin was established in each sample by extrapolation of the standard curve. The number of MAGE-A3 cDNA molecules in each sample was then normalized to the number of cDNA molecules of β-actin.

### Statistical analyses

The correlation between *LINE-1* methylation at each CpG site and the number of expressed CTA or the quantitative levels of expression of MAGE-A3 was evaluated through Spearman's rank correlation test. P values < 0.05 were considered to be statistically significant.

### Results

Results demonstrate a significant inverse association of *LINE-1* methylation with both number of CTA concomitantly expressed (Figure 4) and level of expression of the CTA MAGE-A3 (Figure 5).

### Example 3 - methylation signature(s) predict survival of melanoma patients

### Introduction

Cutaneous melanoma (CM) is a very aggressive neoplasm of growing incidence and mortality rates in industrialized countries, and the leading cause of skin cancer-related deaths worldwide (MacKie et al., 2009). Surgery, in early phases of disease has curative potential for patients; for advanced CM conventional therapies have failed to prolong survival (Bhatia et al., 2009). At present, the best predictor of 5-year survival is the clinico-pathological stage of disease, which defines overall survival (OS) rates ranging from 95% to 7% for stage I to IV patients, respectively (Balch et al., 2001; Balch et al., 2009). However, within the same clinico-pathological stage category, patients often behave radically differently, and the current lack of prognostic molecular markers impairs our ability to identify CM patients with highly aggressive as opposed to more indolent courses of disease (Jennings and Murphy, 2009).

Methylation of genomic DNA in mammals occurs at 5C-position of the cytosine in the context of CpG dinucleotides, and results in gene silencing through different mechanisms (Sigalotti et al., 2010). Recent studies have demonstrated that alterations in genomic DNA methylation patterns represent a hallmark of cancer cells, being proposed to actively contribute to cancer development and progression mainly through inactivation of tumor suppressor genes by aberrant promoter hypermethylation (Esteller, 2008). In particular, epigenetic alterations are emerging as important players in CM tumorigenic process, as demonstrated by the continuously growing list of genes that are transcriptionally inactivated by aberrant DNA hypermethylation in CM, and which potentially affect key cellular pathways such as cellular proliferation/differentiation, apoptosis, DNA repair, extracellular matrix interaction, signaling, metastatization and immune recognition (Sigalotti et al., 2010). The increasing role of aberrant methylation in CM biology strongly suggests for the opportunity to test methylation markers as potential indicators of disease prognosis. Along this line, preliminary investigations are suggesting a possible prognostic role of the methylation status of selected genes in CM patients. Indeed, Lahtz *et al* have most recently evaluated a total of 230 CM patients at stage 0 to IV of disease, demonstrating *PTEN* methylation to be an independent negative prognostic factor, though it did not outperform the strongest traditional markers of tumor thickness and ulceration (Lahtz et al., 2010). Similarly, methylation of the tumor suppressor gene *TSLC1* was found to be significantly increased in advancing tumor stage and to associate with a reduced disease-related survival of CM patients (You et al., 2010), and the methylation of the methylated in tumors (MINT) locus MINT31 has been recently shown to be significantly associated with advancing clinical stage in 107 stage I to IV CM patients, and to predict improved disease-free and overall survival in the 25 analyzed stage III patients (Tanemura et al., 2009).

In line with the above reported information, initial studies have also investigated the methylation status of selected tumor suppressor genes in sera of CM patients. Among the genes more frequently methylated in CM, a negative correlation between presence of circulating methylated DNA for RASSF1A and ER-α and survival of CM patients receiving biochemotherapy was found, suggesting for the potential usefulness of soluble methylation markers as prognostic factors in this malignancy (Mori et al., 2006; Mori et al., 2005).

Despite these promising initial results, the studies so far available have several limitations, including: i) investigation of only single/few genes; ii) reduced number of patients evaluated; iii) rarely investigated the prognostic significance of methylation markers in patients at the same stage of disease.

Furthermore, to the best of our knowledge, no studies have investigated the influence of the genome-wide gene methylation profiles on CM prognosis. Accordingly, the authors investigated whether whole-genome methylation profiles may account for the differing survival patterns of CM patients of identical clinico-pathological stage of disease. The study was conducted on a series of 59 consecutive stage III CM patients for whom the autologous short-term cell cultures were available. The latter were analyzed early during *in vitro* passage, and utilized instead of tumor tissues to overcome possible alterations in the evaluation of genomic methylation profiles due to the unavoidable presence of contaminating normal cells.

Results demonstrated that whole-genome methylation profiling is a powerful tool to identify CM patients with a significantly different prognosis, and that methylation signatures of 17 to 98 genes can be utilized to assign CM patients to distinct prognostic groups. These findings demonstrate that evaluation of whole-genome-defined methylation signatures may greatly help in guiding the daily clinical management of CM patients, and provide a strong rationale for the development of a large prospective validation study.

### Materials and methods

### Patients and cell cultures

Short-term cell cultures were established from metastatic lesions removed surgically from consecutive CM patients referred to the National Cancer Institute of Aviano (Italy) for stage III surgery from 1991 to 2007, as previously described (Altomonte et al., 1993). Autologous tumor cell cultures were successfully established from 30% of patients. The micrometastatic nature of lymph-node tumor tissues from AJCC stage IIIA patients precluded their use for cell culture generation. Thus, the planned studies were conducted on a total of 59 available short-term cultures, identified as having been generated from CM patients classified as AJCC stage IIIB or IIIC. Short-term CM cell cultures were grown in RPMI 1640 Medium (Biochrome KG, Berlin, Germany) supplemented with 20% heat-inactivated fetal calf serum (Biochrome KG) and 2mM L-glutamine (Biochrome KG). To minimize alterations potentially arising with extended in vitro culturing, all cell cultures were utilized for molecular assays at the 6^{th} ex vivo passage.

### Whole genome methylation profiling

Genomic DNA was extracted from short-term cultures of CM cells by proteinase K treatment followed by standard phenol/chloroform extraction and ethanol precipitation (Fratta et al., 2010).The 59 samples under analysis were evaluated for genome-wide promoter methylation using the Illumina Infinium HumanMethylation27 Bead array (Illumina Inc, San Diego, CA), which allows the interrogation of 27,578 CpG dinucleotides, covering 14,495 genes. The assay is based on the conversion of unmethylated C-nucleotides into U(T) nucleotides by the bisulfite treatment, leaving unaltered methylated ones. Accordingly, the EZ DNA methylation kit (Zymo Research, Orange,CA, USA) was used for bisulfite conversion of 500 ng of genomic DNA, and the remaining assay steps were performed as per Illumina protocol. Briefly, the bisulfite-modified genomic DNA was whole-genome amplified, enzymatically fragmented, precipitated, resuspended, and hybridized overnight to locus-specific oligonucleotide primers on the BeadArray. Each interrogated locus is represented by specific oligomers linked to two bead types: one representing the sequence for the methylated DNA (M) and the other for unmethylated DNA (U). After hybridization, the C or T nucleotides were detected on M- or U-beads, respectively, by single-base primer extension. The arrays were then imaged using a BeadArray^{™} Reader. Image processing and intensity data extraction were performed according to Illumina's instructions. The methylation status of a specifc CpG site is calculated from the intensity of the M- and U-beads, as the ratio of fluorescent signals: β=Max(M,0)/[Max(M,0)+Max(U,0)]. DNA methylation β values are continuous variables between 0 (no methylation) and 1 (completely methylated) representing the ratio of combined locus intensity.

### Gene expression profiling

Total cellular RNA was purified from short-term cultures of CM cells by TRIzol reagent (Invitrogen, Milan, Italy) following manufacturer's recommendations. Gene expression profiling was conducted on Illumina human WG-6 version 3 arrays using standard Illumina protocols.

### Statistical analysis

All statistical analyses were performed using the R, version 2.11.1, statistical environment (http://www.R-project.org) and bioconductor 2.6 packages (http://www.bioconductor.org). The authors used the methylumi package for importing and pre-processing the methylation data, the package lumi (Du et al., 2008) for importing and pre-processing the gene expression data, the survival package for Kaplan-Meier estimates and the pamr package for the shrunken centroid supervised analysis. In brief, the methylation data exported from the Illumina Beadstudio software were imported in R, checked for quality and normalized using the methylumi bioconductor package. K-means partitioning clustering was used to divide the transformed data into respectively 2 and 3 groups. The survival times of each of the 2/3 groups were evaluated using a Kaplan-Meier analysis. The characteristics including age, gender, primary tumor localization, Breslow thickness, Clark level, and ulceration of the primary tumor, number of lymph nodes involved, and pre-operative serum LDH values were also examined. Survival time was calculated in months from the date of stage III or IIIC diagnosis until the date of death. According with the specific goals of the analysis, the authors did not classify the deaths considering their cause. Patients were censored at the last follow-up date or the last date the patient was last known to be alive. Median survival duration was determined by the Kaplan-Meier method. Cumulative survival was evaluated using the log-rank test. P values were two sided and values <0.05 were considered to be statistically significant. Methylation patterns (signatures) related to the discovered groups were determined using the nearest shrunken centroid classification algorithm (PAM). As the algorithm requires a threshold to balance between the number of sample correctly classified and the subset of features representing the methylation patterns, the authors determined this threshold by cross-validation as recommended by the inventors of the algorithm (Tibshirani et al., 2002).

### Results

### Patients

The study was conducted on CM patients who underwent radical lymph node dissection for stage III disease at the Centro di Riferimento Oncologico National Cancer Institute between 1991 and 2007. Patients diagnosed with a stage III disease, and for whom a short-term cell culture had been successfully generated from the surgically removed autologous neoplastic tissue, were included in the study. Table 6 summarizes the 59 patients under study and their clinico-pathologic characteristics at presentation.

**Table 6. Characteristics of the 59 AJCC stage III melanoma patients**

| **Variable** | | **n. patients** | **%** |
|---|---|---|---|
| Sub-stage | | | |
| | IIIB | 13 | 22 |
| | IIIC | 45 | 76 |
| | NA* | 1 | 2 |
| Age, years | | | |
| | Median | 52 | |
| | Range | 27-84 | |
| Gender | | | |
| | Male | 35 | 59 |
| | Female | 24 | 41 |
| Localization of primary tumor | | | |
| | extremities | 21 | 36 |
| | trunk | 31 | 52 |
| | head & neck | 4 | 7 |
| | NA | 3 | 5 |
| Breslow thickness of primary tumor | | | |
| | ≤2.0 mm | 20 | 34 |
| | >2.0 mm | 29 | 49 |
| | NA | 10 | 17 |
| Clark level of primary tumor | | | |
| | 1- 3 | 19 | 32 |
| | 4-5 | 31 | 53 |
| | NA | 9 | 15 |
| Ulceration of primary tumor | | | |
| | No | 20 | 34 |
| | Yes | 36 | 61 |
| | NA | 3 | 5 |
| N. lymph nodes involved | | | |
| | 1 | 19 | 32 |
| | >1 | 39 | 66 |
| | NA | 1 | 2 |
| LDH | | | |
| | Low_{†} | 37 | 63 |
| | High | 19 | 32 |
| | NA | 3 | 5 |

| | | | |
|---|---|---|---|
| *NA, not available ^{†} low LDH is established as LDH values ≤ 0.8 times the upper limit of normal, high LDH is established as LDH values > 0.8 times the upper limit of normal | | | |

### Unsupervised analysis of whole-genome methylation profiles identifies CM patients subgroups with different prognosis

In light of the likely influence of altered genome-wide methylation profiles on CM biology, the authors sought to investigate whether distinct methylation patterns might associate with a different clinical outcome of CM patients.

Genome-wide gene methylation profiles were evaluated in the 59 short-term CM cell cultures under study using the Illumina HumanMethylation27 Bead-Chip whole-genome assay, which interrogates the methylation status of 27,578 CpG sites, corresponding to 14,495 genes. Patients were then grouped according to their whole genome methylation profile by applying a k-means clustering, which is a non hierarchical unsupervised method that allows partitioning of data into a predetermined number (k) of groups (Figure 6). K-means clustering algorithm was applied by requiring a separation of all stage III CM patients under analysis into 2 or 3 subgroups (Figure 6). Accordingly, the classification split the population in either 2 subgroups (group K2-1 and K2-2), consisting of 35 and 24 patients, respectively, or in 3 subgroups (group K3-1, K3-2, K3-3), counting 27, 24 and 8 patients, respectively (figure 6).

The prognostic relevance of the genome-wide methylation profile-based classification was assessed by evaluating the obtained subgroups of patients for overall survival (OS) through Kaplan-Meier analysis. Results demonstrated a significant survival advantage for patients classified as K2-1 as compared to those belonging to group K2-2 (P=0.022, log-rank=5.2) (Figure 6A). The statistically significant difference in OS among groups was maintained also when splitting the population in 3 classes (P=0.047, log-rank=6.1) (Figure 6B). In line with these data, an increased median OS was observed for patients classified as K2-1 (31.5 months) as compared to patients in the K2-2 group (14.8 months) (Table 7). Similarly, patients in the K3-1 class had a superior median OS (31.9 months) as compared to K3-3 (18.8 months) and K3-2 (14.8 months) (Table 7). Accordingly, the 5 year OS of patients classified as K2-1 and K2-2 was 41.8% and 5.6%, respectively, while that of patients classified as K3-1, K3-3 and K3-2 was 43.7%, 37.5%, and 5.6%, respectively (Table 7).

**Table 7. OS of stage III CM patients according to genome-wide methylation profiles**

| **stage** | **group*** | **Median OS (95%CI)^{†}** | **5 year OS (%)** |
|---|---|---|---|
| **III all** | K2-1 | 31.5 (13.5-NA‡) | 41.8 |
| | K2-2 | 14.8 (10.4-35.4) | 5.6 |
| **III all** | K3-1 | 31.9(15.3-NA) | 43.7 |
| | K3-2 | 14.8(10.4-35.4) | 5.6 |
| | K3-3 | 18.8(11.5-NA) | 37.5 |
| **IIIc** | 3C-K2-1 | 31.5(13.12-NA) | 41.2 |
| | 3C-K2-2 | 10.4(5.29-NA) | 0 |
| **IIIc** | 3C-K3-1 | 11.0(7.3-NA) | 0 |
| | 3C-K3-2 | 31.5(12.5-NA) | 39.9 |
| | 3C-K3-3 | 24.5(11.5-NA) | 42.9 |

| | | | |
|---|---|---|---|
| * Patients were divided by the k-means clustering algorithm in either 2 or 3 groups, according to their genome-wide methylation profile; ^{†} Survival functions were calculated by the Kaplan-Meier method. Data are reported as Median OS in months, together with the corresponding 95% Confidence Intervals (CI). ‡ NA, not available. | | | |

In light of the above reported prognostic significance of genome-wide methylation profiles in stage III CM patients, the authors asked the question whether methylation profiling could allow to identify different survival classes also in sub-stage IIIC patients, which are characterized by highly homogeneous clinico-pathological characteristics. Thus, the same analytical procedures employed for the whole population of stage III patients were applied to the subgroup of 45 stage IIIC patients. K-means classification split the population in either 2 subgroups (group 3C-K2-1 and 3C-K2-2), counting of 33 and 12 patients, respectively, or in 3 subgroups (group 3C-K3-1, 3C-K3-2, 3C-K3-3), including 11, 27 and 7 patients, respectively (figure 6). Kaplan-Meier analysis demonstrated a significant survival advantage for patients classified as 3C-K2-1 as compared to group 3C-K2-2 (P=0.001, log-rank=10.2) (Figure 6C). Similarly, a significant difference in OS was also observed among groups 3C-K3-3, 3C-K3-2, and 3C-K3-1 (P=0.021, log-rank=7.8) (Figure 6D). An increased median OS was observed for patients classified as 3C-K2-1 (31.5 months) as compared to patients in the 3C-K2-2 group (10.4 months) (Table 7). Similarly, patients in the 3C-K3-2 class had a superior median OS (31.5 months) as compared to 3C-K3-3 (24.5 months) and 3C-K3-1 (11 months) (Table 7). Accordingly, the 5 year OS of patients classified as 3C-K2-1 and 3C-K2-2 was 41.2% and 0%, respectively, while that of patients classified as 3C-K3-3, 3C-K3-2 and 3C-K3-1 was 42.9%, 39.9%, and 0%, respectively (Table 7).

Laying on the above reported information, Cox univariate analysis was carried out to identify patient characteristics and clinico-pathologic factors that predicted survival. Among all examined factors, including age, gender, localization of primary tumor, Breslow thickness, Clark level and ulceration of primary tumor, number of lymph nodes involved, and level of pre-operative LDH, only the classes defined by k-means algorithm on the genome-wide methylation profiles were associated with statistically significant differences in OS, both when evaluating the entire population of stage III CM patients (HR=1.99 for group K2-2 vs. K2-1; 95% CI: 1.09-3.64; P=0.025), and the sole substage IIIC patients (HR=3.2 for group 3C-K2-2 vs. 3C-K2-1; 95% CI: 1.51-6.79; 0.0023) (Table 8).

**Table 8. Cox analysis of the influence of genome-wide methylation profile on OS of stage III CM patients**

| **stage** | **group*** | **HR^{†}** | **95% CI; *P* value** |
|---|---|---|---|
| **III** | K2-1 | 1^{§} | |
| | K2-2 | 1.99 | 1.09-3.64; 0.025 |
| **IIIC** | 3C-K2-1 | 1 | |
| | 3C-K2-2 | 3.20 | 1.51-6.79; 0.0023 |

| | | | |
|---|---|---|---|
| * Patients were divided by the k-means clustering algorithm in 2 groups, according to their genome-wide methylation profile; ^{†} Cox proportional hazard method was used to examine the effect of methylation-based k-means clustering on OS. Results were presented as Hazard Ratios (HR) with corresponding 95% Confidence Intervals (CI); ^{§} set as reference. | | | |

### Supervised analysis of whole-genome methylation profiles by shrunken centroids identifies the methylation signature of CMpatients subgroups with different prognosis

To define the methylation signature, that is the minimal number of methylation markers succinctly characterizing each of the CM patient prognostic group defined by the unsupervised clustering analysis, the authors have applied the "nearest shrunken centroid" algorithm (Tibshirani et al., 2002) (Figure 7, 8).

Using this approach the authors have identified different methylation signatures able to correctly classify patients into the previously defined prognostic classes, with acceptable error rates (Figure 7, 8). Indeed, methylation signature A, consisting of 24 genes, had an overall error rate of 0.05 and classified stage III patients into classes K2-1 and K2-2 with a total of 3 errors, while methylation signature B, including 98 genes, had an overall error rate of 0.034 and classified stage III patients into classes K3-1, K3-2, and K3-3 with a total of 2 errors (Table 9, Figure 7). The same approach conducted on sub-stage IIIC patients led to the identification of a methylation signature of 17 genes (signature C) able to sort stage IIIC patients into groups 3C-K2-1 and 3C-K2-2 without any misinterpretation, and a methylation signature of 47 genes (signature D) able to sort stage IIIC patients into groups 3C-K3-1, 3C-K3-2, and 3C-K3-3 with a total of 1 error (overall error rate of 0.022) (Table 9, Figure 8).

**Table 9: Methylation signatures associated with different prognosis in stage III CM patients**

| **Stage II** | | | |
|---|---|---|---|
| **Signature A** | | | |
| **Gene Symbol** | **Accession N. *** | **Gene ID^{†}** | **lImnID§** |
| MGC29671 | NM_182538.3 | 201305 | cg01138020 |
| GJB5 | NM_005168.2 | 2709 | cg01333788 |
| KCNK16 | NM_032115.2 | 83795 | cg01669948 |
| IGLL1 | NM_020070.2 | 3543 | cg02415431 |
| ANXA8 | NM_001630.1 | 244 | cg03399459 |
| OR12D3 | NM_030959.2 | 81797 | cg03608577 |
| TUB | NM_177972.1 | 7275 | cg05492113 |
| CST11 | NM_080830.2 | 140880 | cg06489008 |
| TMEM10 | NM_033207.2 | 93377 | cg06825166 |
| CRHR1 | NM_004382.2 | 1394 | cg08929103 |
| TRIM40 | NM_138700.2 | 135644 | cg09196959 |
| GAS2L2 | NM_139285.1 | 246176 | cg10016608 |
| FOXN1 | NM_003593.2 | 8456 | cg10113812 |
| FXYD4 | NM_173160.2 | 53328 | cg12619162 |
| KRTHB2 | NM_033033.3 | 3888 | cg13696887 |
| FU33860 | NM_173644.1 | 284756 | cg14236389 |
| S100A9 | NM_001965.2 | 6280 | cg16139316 |
| FOXI1 | NM_012188.3 | 2299 | cg19233472 |
| GUCA2B | NM_007102.1 | 2981 | cg19728577 |
| CYP2B6 | NM_000767.4 | 1555 | cg19756068 |
| IL8RA | NM_000634.2 | 3577 | cg21004129 |
| CYP8B1 | NM_004391.1 | 1582 | cg21979032 |
| WFDC8 | NM_181510.1 | 90199 | cg22021786 |
| SLPI | NM_003064.2 | 6590 | cg23889010 |

| **Stage III** | | | |
|---|---|---|---|
| **Signature B** | | | |
| **Gene Symbol** | **Accession N.** | **Gene ID** | **lImnID** |
| FU25421 | NM_152512.3 | 150350 | cg00044729 |
| LAMC2 | NM_005562.1 | 3918 | cg00208830 |
| C1orf36 | NM_183059.1 | 343035 | cg00658007 |
| MGC29671 | NM_182538.3 | 201305 | cg01138020 |
| GJB5 | NM_005263.2 | 2709 | cg01333788 |
| KCNK16 | NM_032115.2 | 83795 | cg01669948 |
| ANGPTL3 | NM_014495.2 | 27329 | cg02218214 |
| IGLL1 | NM_020070.2 | 3543 | cg02415431 |
| DEFB4 | NM_004942.2 | 1673 | cg02658251 |
| STK38 | NM_007171.2 | 11329 | cg02964385 |
| SEMG2 | NM_003008.2 | 6407 | cg03312792 |
| ANXA8 | NM_001630.1 | 244 | cg03399459 |
| OR12D3 | NM_030959.2 | 81797 | cg03608577 |
| PREPL | NM_006036.2 | 9581 | cg03894103 |
| IL17F | NM_052872.3 | 112744 | cg04063348 |
| DCLRE1A | NM_014881.2 | 9937 | cg04281204 |
| FL120581 | NM_017888.2 | 54988 | cg04428453 |
| PTCD2 | NM_024754.2 | 79810 | cg04527989 |
| CX3CR1 | NM_001337.3 | 1524 | cg04569233 |
| TUB | NM_177972.1 | 7275 | cg05492113 |
| UBE2E1 | NM_182666.1 | 7324 | cg06282059 |
| CST11 | NM_080830.2 | 140880 | cg06489008 |
| IFT140 | NM_014714.2 | 9742 | cg06730286 |
| TMEM10 | NM_033207.2 | 93377 | cg06825166 |
| SLC22A8 | NM_004254.2 | 9376 | cg06917325 |
| MLN | NM_002418.1 | 4295 | cg08332212 |
| DIRC1 | NM_052952.1 | 116093 | cg08654334 |
| C1orf64 | NM_178840.2 | 149563 | cg08887581 |
| CRHR1 | NM_004382.2 | 1394 | cg08929103 |
| TRIM40 | NM_138700.2 | 135644 | cg09196959 |
| TNFSF13B | NM_006573.3 | 10673 | cg09646392 |
| GAS2L2 | NM_139285.1 | 246176 | cg10016608 |
| FOXN1 | NM_003593.2 | 8456 | cg10213812 |
| CYP2B6 | NM_000767.4 | 1555 | cg10322876 |
| ACSL5 | NM_016234.3 | 51703 | cg11151574 |
| SERPINB12 | NM_080474.1 | 89777 | cg11435943 |
| APOBEC4 | NM_203454.1 | 403314 | cg11505048 |
| TNNI1 | NM_003281.3 | 7135 | cg12114524 |
| C1orf42 | NM_019060.1 | 54544 | cg12588301 |
| FXYD4 | NM_173160.2 | 53828 | cg12619162 |
| PPAT | NM_002703.3 | 5471 | cg13086586 |
| PSD4 | NM_012455.2 | 23550 | cg13273136 |
| SCN4A | NM_000334.3 | 6329 | cg13694749 |
| KRTHB2 | NM_033033.3 | 3888 | cg13696887 |
| IL8RB | NM_001557.2 | 3579 | cg13739417 |
| UNC45B | NM_173167.2 | 146862 | cg13859324 |
| C1orf201 | NM_178122.2 | 90529 | cg13980834 |
| SFTPG | NM_205854.1 | 389376 | cg14034870 |
| LOC124751 | NM_213597.1 | 124751 | cg14069619 |
| FLI33860 | NM_173644.1 | 284756 | cg14236389 |
| ANKRD1 | NM_014391.2 | 27063 | cg14558138 |
| FMO2 | NM_001460.2 | 2327 | cg14721213 |
| ARSG | NM_014960.2 | 22901 | cg15308737 |
| GJB4 | NM_153212.1 | 127534 | cg15554401 |
| S100A9 | NM_002965.2 | 6280 | cg16139316 |
| LRRN5 | NM_006338.2 | 10446 | cg16456919 |
| MSMB | NM_002443.2 | 4477 | cg17030820 |
| HLA-DOB | NM_002120.2 | 3112 | cg17103109 |
| R3HDML | NM_178491.2 | 140902 | cg17692403 |
| SPINK5 | NM_006846.2 | 11005 | cg17788013 |
| DLG5 | NM_004747.2 | 9231 | cg17791617 |
| PALLD | NM_016081.2 | 23022 | cg17925436 |
| WFDC12 | NM_080869.1 | 128488 | cg18141647 |
| WDR20 | NM_181291.1 | 91833 | cg18411984 |
| FLI32771 | NM_145017.1 | 220004 | cg18432384 |
| PKHD1 | NM_133694.2 | 5314 | cg18885346 |
| FOXI1 | NM_012188.3 | 2299 | cg19233472 |
| DEFB123 | NM_153324.2 | 245936 | cg19241311 |
| KRTHB3 | NM_002282.2 | 3389 | cg19258973 |
| GUCA2B | NM_007102.1 | 2981 | cg19728577 |
| CYP2B6 | NM_000767.4 | 1555 | cg19756068 |
| BSND | NM_057176.2 | 7309 | cg19971655 |
| NALP10 | NM_176821.2 | 338322 | cg20311730 |
| NR5A2 | NM_003822.3 | 2494 | cg20406878 |
| KRT25D | NM_181535.2 | 162605 | cg20484002 |
| IL8RA | NM_000634.2 | 3577 | cg21004129 |
| APOD | NM_001647.2 | 347 | cg21084260 |
| CLEC4F | NM_173535.2 | 165530 | cg21148892 |
| THRB | NM_000461.2 | 7068 | cg21303011 |
| KRTHB5 | NM_002283.2 | 3891 | cg21518208 |
| CYP8B1 | NM_004391.1 | 1582 | cg21979032 |
| WFDC8 | NM_181510.1 | 90199 | cg22021786 |
| SLC13A1 | NM_022444.3 | 6561 | cg22024657 |
| C3orf27 | NM_007354.1 | 23434 | cg22068211 |
| MGC35206 | NM_178552.2 | 339669 | cg22088368 |
| C3orf63 | NM_015214.1 | 23272 | cg22332306 |
| DARC | NM_002036.2 | 2532 | cg23507131 |
| SLPI | NM_003064.2 | 6590 | cg13889010 |
| SLC2A7 | NM_207420.1 | 155134 | cg24027679 |
| CPA3 | NM_001870.1 | 1359 | cg24290574 |
| MIS12 | NM_024039.1 | 79003 | cg24445405 |
| KRTHB4 | NM_033045.2 | 3890 | cg24461814 |
| MRVI1 | NM_130385.1 | 10335 | cg24541550 |
| MIA2 | NM_054024.3 | 117153 | cg24603941 |
| CYP2A7 | NM_030589.2 | 1549 | cg25427638 |
| ANGPT4 | NM_015985.2 | 51378 | cg26540515 |
| SLC44A5 | NM_152697.2 | 204962 | cg27336379 |
| CST9L | NM_080610.1 | 128821 | cg27655855 |

| **Stage IIIC** | | | |
|---|---|---|---|
| **Signature C** | | | |
| **Gene Symbol** | **Accession N.** | **Gene ID** | **lImnID** |
| SLC18A2 | NM_003054.2 | 6571 | cg00498305 |
| GJB5 | NM_005268.2 | 2709 | cg01333788 |
| SLC6A18 | NM_182632.1 | 348932 | cg02064402 |
| IGLL1 | NM_020070.2 | 3543 | cg02415431 |
| ALOX12B | NM_001139.1 | 242 | cg03742272 |
| WNT10B | NM_003394.2 | 7480 | cg05164634 |
| TUB | NM_177972.1 | 7275 | cg05492113 |
| S100A9 | NM_002965.2 | 6280 | cg07039113 |
| CRHR1 | NM_004382.2 | 1394 | cg08929103 |
| TRIM40 | NM_138700.2 | 135644 | cg09196959 |
| SLC6A11 | NM_014229.1 | 6538 | cg09395732 |
| FU33860 | NM_173644.1 | 284756 | cg14236389 |
| FGF4 | NM_002007.1 | 2249 | cg14578030 |
| S100A9 | NM_002965.2 | 6280 | cg16139316 |
| MGC35206 | NM_178552.2 | 339669 | cg22088368 |
| PCDHAC2 | NM_031883.2 | 56134 | cg24076884 |
| GRM4 | NM_000841.1 | 2914 | cg26424956 |

| **Signature D** | | | |
|---|---|---|---|
| **Gene Symbol** | **Accession N.** | **Gene ID** | **lImnID** |
| FU25421 | NM_152512.3 | 150350 | cg00044729 |
| ANXA13 | NM_004306.2 | 312 | cg00283535 |
| SLC18A2 | NM_003054.2 | 6571 | cg00498305 |
| CTAG2 | NM_020994.2 | 30848 | cg01325515 |
| GJB5 | NM_005268.2 | 2709 | cg01333788 |
| KCNK16 | NM_032115.2 | 83795 | cg01669948 |
| SLC6A18 | NM_182632.1 | 348932 | cg02064402 |
| STK38 | NM_007271.2 | 11329 | cg02964385 |
| PVALB | NM_002854.2 | 5816 | cg02978737 |
| OR12D3 | NM_030959.2 | 81797 | cg03608577 |
| ALOX12B | NM_001139.1 | 242 | cg03742272 |
| HA02 | NM_016527.2 | 51179 | cg03762535 |
| DCLREIA | NM_014881.2 | 9937 | cg04281204 |
| WNT10B | NM_003394.2 | 7480 | cg05164634 |
| TUB | NM_177972.1 | 7275 | cg05492113 |
| UBE2E1 | NM_182666.1 | 7324 | cg06282059 |
| IFT140 | NM_014714.2 | 9742 | cg06730286 |
| SLC4A7 | NM_003615.2 | 9497 | cg06798189 |
| S100A9 | NM_002965.2 | 6280 | cg07039113 |
| DIRC1 | NM_052952.1 | 116093 | cg08654334 |
| CRHR1 | NM_004382.2 | 1394 | cg08929103 |
| TRIM40 | NM_138700.2 | 135644 | cg09196959 |
| SLC6A11 | NM_014229.1 | 6538 | cg09395732 |
| TDRD5 | NM_173533.2 | 163589 | cg09656934 |
| ACSL5 | NM_016134.3 | 51703 | cg11152574 |
| APOBEC4 | NM_203454.1 | 403314 | cg11505048 |
| FAAH | NM_001441.1 | 2166 | cg12671744 |
| CD1D | NM_001766.2 | 912 | cg13765621 |
| C1orf201 | NM_178122.1 | 90529 | cg13980834 |
| FLI33860 | NM_173644.1 | 284756 | cg14236389 |
| ANKRD1 | NM_014391.2 | 27063 | cg14558138 |
| FGF4 | NM_002007.1 | 2249 | cg14578030 |
| FU39501 | NM_173483.1 | 126410 | cg14851685 |
| S100A9 | NM_002965.2 | 6290 | cg16139316 |
| LRRN5 | NM_006338.2 | 10446 | cg16456919 |
| GLT1D1 | NM_144669.1 | 144423 | cg16717225 |
| IL28B | NM_172139.2 | 282617 | cg17982671 |
| AVPR1B | NM_000707.2 | 553 | cg18992688 |
| GJB3 | NM_024009.2 | 2707 | cg21151355 |
| MGC35206 | NM_178552.2 | 339669 | cg22088368 |
| PCDHAC2 | NM_031883.2 | 56134 | cg24076884 |
| GABRG3 | NM_033223.1 | 2567 | cg24244000 |
| MIA2 | NM_054024.3 | 117153 | cg24603941 |
| CTAG1B | NM_001327.1 | 1485 | cg25531166 |
| UNQ9433 | NM_207413.1 | 389658 | cg26021627 |
| GRM4 | NM_000841.1 | 2914 | cg26424956 |
| CEACAM6 | NM_002483.3 | 4680 | cg26813458 |

| | | | |
|---|---|---|---|
| * GeneBank accession number ^{†} Illumina unique gene identifyer § Illumina unique probe identifyer, defines the exact CpG dinucleotide that is evaluated | | | |

This initial analysis was further refined in the attempt to identify methylation signatures that were likely to be directly associated to a biologic effect through the transcriptional regulation of the respective genes. To this end, a Spearman's correlation analysis was conducted comparing genome-wide methylation data and gene expression profiles obtained from neoplastic cells of the stage III CM patients under study. This analysis yielded a set of 820 methylation probes which methylation status was significantly correlated (p<0.05) with the expression of the respective gene (data not shown). By restricting the application of the "nearest shrunken centroid" algorithm to this sub-set of methylation signals, the authors were able to identify methylation signatures capable to correctly classify stage IIIC patients into the previously defined prognostic classes (Table 10, Figure 9). Of these, a 51 gene methylation signature (signature E) was able to assign stage IIIC patients to groups 3C-K2-1 or 3C-K2-2, committing only one error (overall error rate 0.022), while a 73 gene methylation signature (signature F) was able to sort stage IIIC patients into groups 3C-K3-1, 3C-K3-2, and 3C-K3-3 without any misinterpretation (Table 10, Figure 9). When, among the genes having a significant (p<0.05) association between expression and methylation status, only those with a coefficient of correlation Rho<-0.4 were taken into account, an additional signature of 35 genes (signature G) was defined, which was able to assign stage IIIC patients to groups 3C-K2-1 or 3C-K2-2, committing only one error (overall error rate 0.022) (Table 10, Figure 10).

**Table 10: Methylation signatures correlated with gene expression and associated with different prognosis in stage IIIC CM patients**

| **Stage IIIC** | | | |
|---|---|---|---|
| **Signature E** | | | |
| **Gene** | **Symbol Accession N.** | *** Gene ID†** | **lImnID§** |
| DENND2D | NM_024901.3 | 79961 | cg00619207 |
| CTAG2 | NM_020994.2 | 30848 | cg01325515 |
| PGCP | NM_016134.2 | 10404 | cg01892689 |
| KCNG1 | NM_002237.2 | 3755 | cg02305261 |
| FAM107B | NM_031453.2 | 83641 | cg02876062 |
| FANCE | NM_011922.2 | 2178 | cg03030757 |
| FAM38B | NM_022068.1 | 63895 | cg03426225 |
| FOXF2 | NM_001452.1 | 2295 | cg03848675 |
| BGN | NM_001711.3 | 633 | cg04929365 |
| MAGEA10 | NM_021048.3 | 4109 | cg04950711 |
| SPATS1 | NM_145026.2 | 221409 | cg05010967 |
| PRRX1 | NM_022716.2 | 5336 | cg05593300 |
| MlSTD1 | NM_018099.3 | 55711 | cg05697976 |
| PDZD4 | NM_032512.2 | 57595 | cg06051662 |
| HCP5 | NM_006674.2 | 10866 | cg07971188 |
| OCIAD2 | NM_152398.2 | 132299 | cg08942875 |
| CSAG1 | NM_153478.1 | 158511 | cg08977028 |
| SLC04A1 | NM_016354.3 | 28231 | cg09210315 |
| C9orf90 | NM_197956.1 | 203245 | cg09296212 |
| CSAG1 | NM_153478.1 | 158511 | cg09917461 |
| AMPH | NM_001635.2 | 273 | cg09966445 |
| MT1G | NM_005950.1 | 4495 | cg09995279 |
| C20orf100 | NM_031883.1 | 84969 | cg11319389 |
| CST7 | NM_003650.2 | 8530 | cg11804789 |
| KIAA0367 | NM_015125.1 | 23273 | cg11880010 |
| RNASE1 | NM_198232.1 | 6035 | cg13718960 |
| HCP5 | NM_006674.2 | 10866 | cg13784557 |
| NDN | NM_002487.2 | 4692 | cg13828758 |
| RAC2 | NM_002872.3 | 5880 | cg14072120 |
| AXL | NM_021913.2 | 558 | cg14892768 |
| BATF | NM_006399.2 | 10538 | cg15645309 |
| EMILIN1 | NM_007046.1 | 11117 | cg15895197 |
| ARHGEF7 | NM_003899.2 | 3874 | cg16427670 |
| EVL | NM_016337.2 | 51466 | cg17813891 |
| RAC2 | NM_002872.3 | 5888 | cg18265887 |
| KIAA0367 | NM_015225.1 | 23273 | cg19282250 |
| TFF3 | NM_003226.2 | 7033 | cg20483657 |
| CTSK | NM_000396.2 | 1513 | cg20802392 |
| C21orf70 | NM_058190.2 | 85395 | cg20884362 |
| PRKCZ | NM_002744.4 | 5590 | cg21137823 |
| MLSTD1 | NM_018099.3 | 55711 | cg21522988 |
| COL5A2 | NM_000393.2 | 1290 | cg22774472 |
| LY96 | NM_015364.2 | 23643 | cg23732024 |
| MAGEA1 | NM_004988.3 | 4100 | cg23776892 |
| MAGEC2 | NM_016249.2 | 51438 | cg23313564 |
| DENND2D | NM_024901.3 | 79961 | cg24641737 |
| PPIE | NM_006112.2 | 10450 | cg24920358 |
| SORBS2 | NM_021069.3 | 8470 | cg26533078 |
| PROCR | NM_006404.3 | 10544 | cg26806924 |
| TMEPAI | NM_020182.3 | 56337 | cg26912636 |
| SSNA1 | NM_003731.1 | 8636 | cg27291231 |
| ZNF324 | NM_014347.1 | 25799 | cg27401095 |

| **Signature F** | | | |
|---|---|---|---|
| **Gene Symbol** | **Accession N.** | **Gene ID** | **lImnID** |
| FU25421 | NM_151512.3 | 150350 | cg00044729 |
| DENND2D | NM_024901.3 | 79961 | cg00619207 |
| CTAG2 | NM_020994.2 | 30848 | cg01325515 |
| PGCP | NM_016134.2 | 10404 | cg01892689 |
| KCNG1 | NM_002237.2 | 3755 | cg02305261 |
| MGMT | NT_008818.15 | 4255 | cg02330106 |
| FAM107B | NM_031453.2 | 83641 | cg02876062 |
| FANCE | NM_021922.2 | 2178 | cg03030757 |
| GAGE2 | NM_001472.1 | 2574 | cg04431776 |
| C11orf1 | NM_022761.1 | 64776 | cg04545708 |
| ABCB4 | NM_018849.1 | 5244 | cg04737046 |
| BGN | NM_001711.3 | 633 | cg04929865 |
| MAGEA10 | NM_021048.3 | 4109 | cg04950711 |
| SPATS1 | NM_145026.2 | 221409 | cg05010967 |
| CTNNAL1 | NM_003798.1 | 8727 | cg05495060 |
| PRRX1 | NM_022716.2 | 5396 | cg05593300 |
| EMP1 | NM_001423.1 | 2012 | cg05885720 |
| RNASE1 | NM_198232.1 | 6035 | cg05958352 |
| PDZD4 | NM_032512.2 | 57595 | cg06051662 |
| MAGEA6 | NM_005363.2 | 4105 | cg06899808 |
| CHMP4A | NM_014169.2 | 29082 | cg07296772 |
| ADORA2B | NM_000676.2 | 136 | cg07677850 |
| HCP5 | NM_006674.2 | 10866 | cg07971188 |
| PLA1A | NM_015900.1 | 51365 | cg08141463 |
| KIAA0141 | NM_014713.2 | 9812 | cg08158289 |
| OLFM1 | NM_014179.2 | 10439 | cg08268099 |
| ERO1L | NM_014584.1 | 30001 | cg08554114 |
| CSAG1 | NM_153478.1 | 158511 | cg08977028 |
| SLC04A1 | NM_016354.3 | 28231 | cg09210315 |
| CXorf12 | NM_003492.1 | 8269 | cg09275137 |
| C9orf90 | NM_197956.1 | 203245 | cg09296212 |
| CSAG1 | NM_153478.1 | 158511 | cg09917461 |
| AMPH | NM_001635.2 | 273 | cg09966445 |
| MT1G | NM_005950.1 | 4495 | cg09985279 |
| ARHGDIB | NM_001175.4 | 397 | cg10925082 |
| C20orf100 | NM_032883.1 | 84969 | cg11319389 |
| CST7 | NM_003650.2 | 8530 | cg11804789 |
| KIAA0367 | NM_015225.1 | 23273 | cg11880010 |
| RNASE1 | NM_198232.1 | 6035 | cg13718960 |
| HCP5 | NM_006674.2 | 10866 | cg13784557 |
| NDN | NM_002487.2 | 4692 | cg13828758 |
| RAC2 | NM_002872.3 | 5880 | cg14072120 |
| AXL | NM_021913.2 | 558 | cg14892768 |
| GRAMD3 | NM_023927.1 | 65983 | cg15165084 |
| ARSG | NM_014960.2 | 22901 | cg15308737 |
| PLD1 | NM_002662.2 | 5337 | cg15329866 |
| HNMT | NM_006895.2 | 3176 | cg15441973 |
| ZNF558 | NM_144693.1 | 148156 | cg15751406 |
| ATP6V1E2 | NM_080653.3 | 90423 | cg15759873 |
| EMILIN1 | NM_007046.1 | 11117 | cg15895197 |
| ARHGEF7 | NM_003899.2 | 8874 | cg16427670 |
| KRT25C | NM_181537.2 | 342574 | cg16488522 |
| ZNF532 | NM_018181.4 | 55205 | cg17675150 |
| EVL | NM_016337.2 | 51466 | cg17813891 |
| RAC2 | NM_002872.3 | 5880 | cg18265887 |
| SLAMF9 | NM_033438.1 | 89886 | cg18913171 |
| NBL1 | NM_005380.3 | 4681 | cg19136075 |
| KIAA0367 | NM_015225.1 | 23273 | cg19282250 |
| MAGEA10 | NM_021048.3 | 4109 | cg19964192 |
| TFF3 | NM_003226.2 | 7033 | cg20488657 |
| C21orf70 | NM_058190.2 | 85395 | cg20884362 |
| APOD | NM_001647.2 | 347 | cg21084260 |
| ABCA8 | NM_007168.2 | 10351 | cg21660392 |
| ACOX2 | NM_003500.2 | 8309 | cg22012981 |
| COL5A2 | NM_000393.2 | 1290 | cg22774472 |
| LY96 | NM_015364.2 | 23643 | cg23732024 |
| MAGEA1 | NM_004988.3 | 4100 | cg23776892 |
| MAGEC2 | NM_016249.2 | 51438 | cg23813564 |
| TIMP3 | NM_000362.4 | 7078 | cg24080529 |
| PPIE | NM_006112.2 | 10450 | cg24920358 |
| RPUSD3 | NM_173659.2 | 285367 | cg26417554 |
| PROCR | NM_006404.3 | 10544 | cg26806924 |
| TMEPAI | NM_020182.3 | 56937 | cg26912636 |

| **STAGE IIIC** | | | |
|---|---|---|---|
| **Signature G** | | | |
| **Gene Symbol** | **Accession N. *** | **Gene ID^{†}** | **lImnID§** |
| PRDX2 | NM_005809.4 | 7001 | cg00155609 |
| DENND2D | NM_024901.3 | 79961 | cg00619207 |
| CTAG2 | NM_020994.2 | 30848 | cg01325515 |
| PCOLCE | NM_002593.2 | 5118 | cg02797569 |
| BGN | NM_001711.3 | 633 | cg04929865 |
| OCIAD2 | NM_152398.2 | 132299 | cg04961553 |
| RNASE1 | NM_198232.1 | 6035 | cg05958352 |
| MAGEA6 | NM_005363.2 | 4105 | cg06899808 |
| HCP5 | NM_006674.2 | 10866 | cg07971188 |
| OCIAD2 | NM_152398.2 | 132299 | cg08942875 |
| CSAG1 | NM_153478.1 | 158511 | cg08977028 |
| C9orf90 | NM_197956.1 | 203245 | cg09296212 |
| CSAG1 | NM_153478.1 | 158511 | cg09917461 |
| CTSK | NM_000396.2 | 1513 | cg11946165 |
| ADM | NM_001124.1 | 133 | cg12320306 |
| RNASE1 | NM_198232.1 | 6035 | cg13718960 |
| HCP5 | NM_006674.2 | 10866 | cg13784557 |
| PAGE5 | NM_130467.3 | 90737 | cg13991029 |
| NNMT | NM_006169.2 | 4837 | cg14209518 |
| EMILIN1 | NM_007046.1 | 11117 | cg15895197 |
| MAGEA3 | NM_005362.3 | 4102 | cg16390856 |
| RAB11FIP5 | NM_015470.1 | 26056 | cg18943195 |
| ARHGDIB | NM_001175.4 | 397 | cg19826026 |
| EMILIN1 | NM_007046.1 | 11117 | cg20516209 |
| LPXN | NM_004811.1 | 9404 | cg20654468 |
| CTSK | NM_000396.2 | 1513 | cg20802392 |
| ALDH1A3 | NM_000693.1 | 220 | cg21359747 |
| SUHW1 | NM_080740.3 | 129025 | cg21604856 |
| ABCA8 | NM_007168.2 | 10351 | cg21660392 |
| COL5A2 | NM_000393.2 | 1290 | cg22774472 |
| LY96 | NM_015364.2 | 23643 | cg23732024 |
| MAGEA1 | NM_004988.3 | 4100 | cg23776892 |
| MAGEC2 | NM_016249.2 | 51438 | cg23813564 |
| GSTT1 | NM_000853.1 | 2952 | cg24330042 |
| DENND2D | NM_024901.3 | 79961 | cg24641737 |

| | | | |
|---|---|---|---|
| * GeneBank accession number ^{†} Illumina unique gene identifyer § Illumina unique probe identifyer, defines the exact CpG dinucleotide that is evaluated | | | |

### Conclusions

Whole genome methylation profiles identified classes with significantly different OS in stage III CM patients. As an example, stage IIIC patients with a "favorable" methylation profile had increased OS (P=0.001, log-rank=10.2) by Kaplan-Meyer analysis. Median OS of stage IIIC patients with a "favorable" vs "unfavorable" methylation profile were 31.5 and 10.4 months, respectively. The 5 year OS for stage IIIC patients with a "favorable" methylation profile was 41.2% as compared to 0% for patients with an "unfavorable" methylation profile. Among the variables examined by Cox regression analysis, classification defined by methylation profile was the only predictor of OS (Hazard Ratio=3.2, for "unfavorable" methylation profile; 95% Confidence Interval: 1.51-6.79; P=0.002). Different methylation signatures, consisting of 17 to 98 genes, able to correctly assign (overall error rate ≤0.05) stage III or IIIC patients to distinct methylation-defined prognostic groups, were identified.

Discrete whole-genome methylation signatures have been identified as molecular markers of prognosis for CM patients in stage III. Their use in the daily practice will drive the most appropriate clinical management of stage III CM patients.

### Example 4 - Prognostic significance of the methylation status of single genes taken from whole-genome defined prognostic methylation signatures.

In light of the prognostic relevance of whole-genome methylation profiles that the authors have identified (see example 3 above), the authors have addressed the question whether specific genes taken from the prognostic methylation signatures may retain a prognostic information. To this end, the authors selected genes *FGF4*, *CTAG2*, *OCIAD2* and *PAGE5* from signature E and G as playing a major contribution in the classification tasks. The 45 stage IIIC patients under study were scored accordingly to the methylation of each gene in their tumor cells being <33%, between 33% and 66%, or > 66%. The impact on patients' survival of *FGF4*, *CTAG2*, *OCIAD2* and *PAGE5* methylation levels was evaluated by Kaplan-Meyer analysis and Log-rank test, showing a significantly improved survival for patients with unmethylated vs methylated genes (Table 11, Figure 11). Similar results were found for other tested genes, including: *ALOX12B, BATF, BGN, CRHR1, CSAG1, CTSK, DENND2D, FLJ33860, GJB5, GRM4, IGLL1, LY96, MAGEA1, MAGEA10, MGC35206, MLSTD1, S100A9, SLC18A2, SLC6A11, SLC6A18, SORBS2, TRIM40, TUB* (data not shown).

**Table 11. OS of stage IIIC CM patients according to FGF4, CTAG2, OCIAD2 and PAGE5 methylation status**

| **gene** | **extent methylation*** | **Median OS (95%CI)^{†}** | **5 year OS (%)** |
|---|---|---|---|
| *FGF4* | <33% | 35.2 (24.5-NA‡) | 49 |
| | ≥33%, <66% | 11.5 (6.8-NA) | 0 |
| | ≥66% | 9.8 (5.3 -NA) | 8 |
| *CTAG2* | <33% | NA (31.9-NA) | 58 |
| | ≥33%, <66% | 15.3 (4.5-NA) | 15 |
| | ≥66% | 11.3 (7.3 -NA) | 0 |
| *OCIAD2* | <33% | 27.0 (11.5-NA) | 44 |
| | ≥33%, <66% | 18.1 (11.1-NA) | 28 |
| | ≥66% | 9.4 (5.0 -NA) | 0 |
| *PAGE5* | <33% | 73.9 (24.5-NA) | 58 |
| | ≥33%, <66% | NA (15.3-NA) | 53 |
| | ≥66% | 11.1(7.3-24.5) | 12 |

| | | | |
|---|---|---|---|
| * Patients were divided according to the methylation *of FGF4, CTAG2, OCIAD2* or *PAGE5* in their tumor cells being <33%, between 33% and 66%, or > 66%. ^{†} Survival functions were calculated by the Kaplan-Meier method. Data are reported as Median OS in months, together with the corresponding 95% Confidence Intervals (CI). ‡ NA, not available. | | | |

### Example 5 - Prognostic significance of combined evaluation of LINE-1 methylation levels and multigene methylation signatures in stage III cutaneous melanoma patients.

In light of the prognostic relevance of *LINE-1* methylation status and of whole-genome methylation profiles that the authors have identified (see examples 1, 3 above), the authors have addressed the question whether combining these two markers would result in an additional prognostic information. To this end, 59 stage III melanoma patients were scored according to their whole genome methylation profile as K2-1 or k2-2 (stage III patients) or S3-K2-1 or S3-K2-2 (substage IIIC patients), and as having hypomethylated or hypermethylated *LINE-1* sequences, as detailed in examples 1 and 3. The impact on patients' survival of the combined evaluation of the 2 markers was evaluated by Kaplan-Meyer analysis and Log-rank test (Figure 12, Table 12). Results demonstrated that the combined use of the 2 markers allowed to further discriminate survival groups within the population under study (P<0.05).

**Table 12. OS of stage III CM patients according to genome-wide methylation profiles and LINE-1 methylation status**

| **stage** | **group*** | **Median OS (95%CI)^{†}** | **5 year OS (%)** |
|---|---|---|---|
| **III all** | K2-1, *LINE-1* hypomethylated | 73.9 (31.5-NA‡) | 52.4 |
| | K2-1, *LINE-1* hypermethylated | 12.5 (9.4-NA) | 18.2 |
| | K2-2, *LINE-1* hypomethylated | 10.4 (9.8-NA) | 0 |
| | K2-2, *LINE-1* hypermethylated | 18.1 (11.01-45.6) | 7 |
| **IIIC** | 3C-K2-1, LINE-1 hypomethylated | 73.8 (31.5-NA) | 52.5 |
| | 3C-K2-2, *LINE-1* hypermethylated | 12.5 (9.3-NA) | 23.1 |
| | 3C-K2-2, *LINE-1* hypomethylated | 6.8 (3.7-NA) | 0 |
| | 3C-K2-2, *LINE-1* hypermethylated | 11.3 (5.3-NA) | 0 |

| | | | |
|---|---|---|---|
| * Patients were scored by k-means clustering algorithm as K2-1 or K2-2 (stage III patients) or as S3-K2-1 or S3-K2-2 (sub-stage IIIC patients), according to their genome-wide methylation profile, and as having hypomethylated (< median value) or hypermethylated (≥ median value) *LINE-1* sequences. Combined evaluation of this 2 markers resulted in the definition of 4 classes. ^{†} Survival functions were calculated by the Kaplan-Meier method. Data are reported as Median OS in months, together with the corresponding 95% Confidence Intervals (CI). ‡ NA, not available | | | |

### Example 6 - Pyrosequencing analysis of LINE-1 methylation in neoplastic material from different sources

DNA was extracted from neoplastic cells and evaluated for LINE-1 methylation at 3 CpG sites (CpG1, 2, 3) by pyrosequencing.

Neoplastic cells were purified using anti-HMW-MAA antibody-coated magnetic beads from cryopreserved cell suspensions obtained by mechanical and enzymatic dissection of neoplastic lesions surgically removed from CM patients.

Surgically-removed neoplastic tissues from CM patients were flesh-frozen in liquid nitrogen and maintained at -80°C until use.

Surgically-removed neoplastic tissues from CM patients were formalin-fixed and paraffin-embedded. Ten micrometer sections (total 2 to 6) were cut from paraffin embedded lesions and used for evaluation of LINE-1 methylation.

Finally, neoplastic tissue of different extensions (area 9-100 mm2) was scraped from 15 micrometer sections of formalin-fixed paraffin-embedded CM tissues, and used for evaluation of LINE-1 methylation.

The results are shown in Table 13.

**Table 13. Pyrosequencing analysis of LINE-1 methylation in neoplastic material from different sources Pyrosequencing analysis of LINE-1 methylation in neoplastic material from different sources***

| **neoplastic cells immunosorted from cryopreserved cell suspensions^{†}** | | | | |
|---|---|---|---|---|
| **patient** | | **CpG1** | **CpG2** | **CpG3** |
| 403 | | 28.4 | 23.2 | 34.0 |
| 390 | | 44.3 | 41.1 | 60.1 |
| 554 | | 21.9 | 26.2 | 42.0 |
| 578 | | 32.2 | 39.7 | 59.5 |
| 475 | | 40.3 | 33.9 | 47.3 |
| 514 | | 38.6 | 28.7 | 43.9 |

| **cryopreserved CM neoplastic tissues‡** | | | | |
|---|---|---|---|---|
| **patient** | | **CpG1** | **CpG2** | **CpG3** |
| 588 | | 68.7 | 51.4 | 67.2 |
| 603 | | 29.0 | 25.7 | 39.3 |
| 629 | | 48.0 | 37.9 | 58.0 |
| 790 | | 50.0 | 32.9 | 47.3 |
| 816 | | 42.3 | 34.9 | 50.5 |
| 11766 | | 50.6 | 32.0 | 49.9 |
| 2605 | | 37.4 | 26.3 | 40.4 |

| f**ormalin-fixed paraffin embedded neoplastic lesions§** | | | | |
|---|---|---|---|---|
| **patient** | **n. of sections** | **CpG1** | **CpG2** | **CpG3** |
| 11482 | 2 | 40.5 | 25.7 | 38.6 |
| | 4 | 38.6 | 24.8 | 37.6 |
| | 6 | 37.8 | 24.9 | 38.7 |
| 11766 | 2 | 48.4 | 29.2 | 47.3 |
| | 4 | 45.5 | 30.4 | 44.2 |
| | 6 | 45.3 | 30.7 | 44.8 |
| 6432 | 2 | 46.9 | 32.2 | 50.1 |
| | 4 | 48.7 | 36.0 | 53.6 |
| | 6 | 50.1 | 37.6 | 53.2 |
| 2605 | 2 | 42.8 | 29.2 | 43.3 |
| | 4 | 39.5 | 27.3 | 41.6 |
| | 6 | 42.3 | 29.0 | 40.8 |

| **macro-dissected formalin-fixed paraffin embedded neoplastic lesions^{††}** | | | | |
|---|---|---|---|---|
| **patient** | **area (mm²)** | **CpG1** | **CpG2** | **CpG3** |
| 17193 | 9 | 22.8 | 30.2 | 44.2 |
| | 25 | 25.9 | 32.4 | 50.4 |
| | 100 | 28.8 | 28.1 | 47.6 |
| 6432 | 9 | 29.6 | 36.3 | 54.6 |
| | 25 | 23.6 | 35.2 | 55.0 |
| | 100 | 25.3 | 36.0 | 53.6 |

| | | | | |
|---|---|---|---|---|
| * DNA was extracted from neoplastic cells and evaluated for LINE-1 methylation at 3 CpG sites (CpG1, 2, 3) by pyrosequencing; † neoplastic cells were purified using anti-HMW-MAA antibody-coated magnetic beads from cryopreserved cell suspensions obtained by mechanical and enzymatic dissection of neoplastic lesions surgically removed from CM patients; ‡ surgically-removed neoplastic tissues from CM patients were flesh-frozen in liquid nitrogen and maintained at -80°C until use; § surgically-removed neoplastic tissues from CM patients were formalin-fixed and paraffin-embedded. Ten micrometer sections (total 2 to 6) were cut from paraffin embedded lesions and used for evaluation of LINE-1 methylation; ^{††}Neoplastic tissue of different extensions (area 9-100 mm2) was scraped from 15 micrometer sections of formalin-fixed paraffin-embedded CM tissues, and used for evaluation of LINE-1 methylation. | | | | |

### REFERENCES

Altomonte, M., et al. 1993. Cancer Res. 53:3343-3348.
Ausubel, F.M., et al. 1998. Current Protocols in Molecular Biology John Wiley & Sons, NY.
Balch, C.M., et al. 2001. J Clin Oncol. 19:3635-3648.
Balch, C.M., et al. 2009. J Clin Oncol. 27:6199-6206.
Bhatia, S., et al. 2009. Oncology (Williston Park). 23:488-496.
Calabro, L., et al. 2005. J Cell Physiol. 202:474-477.
Coral, S., et al. 1999. J Immunother. 22:16-24.
Cox, D.R. 1972. J Roy Statist Soc B. 34:187-220.
De Plaen, E., et al. 1994. Immunogenetics. 40:360-369.
De Smet, C., et al. 1996. Proc Natl.Acad.Sci. U.S.A. 93:7149-7153.
Du, P., et al. 2008. Bioinformatics. 24:1547-1548.
Esteller, M. 2008. N Engl J Med. 358:1148-1159.
Fratta, E., et al. 2010. J Cell Physiol. 223:352-358.
Gama-Sosa, M.A., et al. 1983. Nucleic Acids Res. 11:6883-6894.
Gure, A.O., et al. 1997. Int J Cancer. 72:965-971.
Jager, E., et al. 1998. J Exp Med. 187:265-270.
Jennings, L., and G.M. Murphy. 2009. Br J Dermatol. 161:496-503.
Kaplan, E.L., and P. Meier. 1958. J. of the Am. Statistical Association. 53:457-481.
Lahtz, C., et al. 2010. J Invest Dermatol. 130:620-622.
MacKie, R.M., et al. 2009. Ann Oncol. 20 Suppl 6:vil-7.
Mori, T., et al. 2006. Cancer Res. 66:6692-6698.
Mori, T., et al. 2005. J Clin Oncol. 23:9351-9358.
Ogino, S., et al. 2008. J Natl Cancer Inst. 100:1734-1738.
Pattamadilok, J., et al. 2008. Int J Gynecol Cancer. 18:711-717.
Rimoldi, D., et al. 1999. Int J Cancer. 82:901-907.
Sigalotti, L., et al. 2002. J Immunother.(1997.). 25:16-26.
Sigalotti, L., et al. 2010. J Transl Med. 8:56.
Sigalotti, L., et al. 2007. J Cell Physiol. 212:330-344.
Tanemura, A., et al. 2009. Clin Cancer Res. 15:1801-1807.
Tellez, C.S., et al. 2009. Melanoma Res. 19:146-155.
Tibshirani, R., et al. 2002. Proc Natl Acad Sci USA. 99:6567-6572.
Wallace, N.A., et al. 2008. Gene. 419:75-81.
Woloszynska-Read, A., et al. 2008. Clin. Cancer Res. 14:3283-3290.
Yang, A.S., et al. 2004. Nucleic Acids Res. 32:e38.
You, Y., et al. 2010. Melanoma Res. 20:179-183.

## Claims

1. An in vitro method of prognosing melanoma and/or of predicting and/or monitoring the response and/or the efficacy of a cutaneous melanoma therapy or to identify a subject to be treated with a DNA hypomethylating agent comprising determining the methylation status of at least one cytosine residue in a methylation relevant region of at least one gene selected from any one of ABCA8, ABCB4, ACOX2, ACSL5, ADM, ADORA2B, ALDH1A3, ALOX12B, AMPH, ANGPT4, ANGPTL3, ANKRD1, ANXA13, ANXA8, APOBEC4, APOD, ARHGDIB, ARHGEF7, ARSG, ATP6V1E2, AVPR1B, AXL, BATF, BGN, BSND, C11orf1, C1orf201, C1orf36, C1orf42, C1orf64, C20orf100, C21orf70, C3orf27, C3orf63, C9orf90, CD1D, CEACAM6, CHMP4A, CLEC4F, COL5A2, CPA3, CRHR1, CSAG1, CST11, CST7, CST9L, CTAG1B, CTAG2, CTNNAL1, CTSK, CX3CR1, CXorf12, CYP2A7, CYP2B6, CYP8B1, DARC, DCLRE1A, DEFB123, DEFB4, DENND2D, DIRC1, DLG5, EMILIN1, EMP1, ERO1L, EVL, FAAH, FAM107B, FAM38B, FANCE, FGF4, FLJ20581, FLJ25421, FLJ32771, FLJ33860, FLJ39501, FMO2, FOXF2, FOXI1, FOXN1, FXYD4, GABRG3, GAGE2, GAS2L2, GJB3, GJB4, GJB5, GLT1D1, GRAMD3, GRM4, GSTT1, GUCA2B, HA02, HCP5, HLA-DOB, HNMT, IFT140, IGLL1, IL17F, IL28B, IL8RA, IL8RB, KCNG1, KCNK16, KIAA0141, KIAA0367, KRT25C, KRT25D, KRTHB2, KRTHB3, KRTHB4, KRTHB5, LAMC2, LOC124751, LPXN, LRRN5, LY96, MAGEA1, MAGEA10, MAGEA3, MAGEA6, MAGEC2, MGC29671, MGC35206, MGMT, MIA2, MIS12, MLN, MLSTD1, MRVI1, MSMB, MT1G, NALP10, NBL1, NDN, NNMT, NR5A2, OCIAD2, OLFM1, OR12D3, PAGE5, PALLD, PCDHAC2, PCOLCE, PDZD4, PGCP, PKHD1, PLA1A, PLD1, PPAT, PPIE, PRDX2, PREPL, PRKCZ, PROCR, PRRX1, PSD4, PTCD2, PVALB, R3HDML, RAB11FIP5, RAC2, RNASE1, RPUSD3, S100A9, SCN4A, SEMG2, SERPINB12, SFTPG, SLAMF9, SLC13A1, SLC18A2, SLC22A8, SLC2A7, SLC44A5, SLC4A7, SLC6A11, SLC6A18, SLCO4A1, SLPI, SORBS2, SPATS1, SPINK5, SSNA1, STK38, SUHW1, TDRD5, TFF3, THRB, TIMP3, TMEM10, TMEPAI, TNFSF13B, TNNI1, TRIM40, TUB, UBE2E1, UNC45B, UNQ9433, WDR20, WFDC12, WFDC8, WNT10B, ZNF324, ZNF532, ZNF558 and the *LINE-1* repetitive sequence or a variant thereof in a biological sample obtained from the subject.

2. The method of claim 1, comprising determining the methylation status of at least one cytosine residue in each of the methylation relevant region of the genes selected from:
ANXA8, CRHR1, CST11, CYP2B6, CYP8B1, FLJ33860, FOXI1, FOXN1, FXYD4, GAS2L2, GJB5, GUCA2B, IGLL1, IL8RA, KCNK16, KRTHB2, MGC29671, OR12D3, S100A9, SLPI, TMEM10, TRIM40, TUB, and WFDC8, as indicated in Table 1 signature A.

3. The method of claim 1, comprising determining the methylation status of at least one cytosine residue in each of the methylation relevant region of the genes selected from:
ACSL5, ANGPT4, ANGPTL3, ANKRD1, ANXA8, APOBEC4, APOD, ARSG, BSND, C1orf201, C1orf36, C1orf42, C1orf64, C3orf27, C3orf63, CLEC4F, CPA3, CRHR1, CST11, CST9L, CX3CR1, CYP2A7, CYP2B6, CYP8B1, DARC, DCLRE1A, DEFB123, DEFB4, DIRC1, DLG5, FLJ20581, FLJ25421, FLJ32771, FLJ33860, FMO2, FOXI1, FOXN1, FXYD4, GAS2L2, GJB4, GJB5, GUCA2B, HLA-DOB, IFT140, IGLL1, IL17F, IL8RA, IL8RB, KCNK16, KRT25D, KRTHB2, KRTHB3, KRTHB4, KRTHB5, LAMC2, LOC124751, LRRN5, MGC29671, MGC35206, MIA2, MIS12, MLN, MRVI1, MSMB, NALP10, NR5A2, OR12D3, PALLD, PKHD1, PPAT, PREPL, PSD4, PTCD2, R3HDML, S100A9, SCN4A, SEMG2, SERPINB12, SFTPG, SLC13A1, SLC22A8, SLC2A7, SLC44A5, SLPI, SPINK5, STK38, THRB, TMEM10, TNFSF13B, TNNI1, TRIM40, TUB, UBE2E1, UNC45B, WDR20, WFDC12 andWFDC8 as indicated in Table 1 signature B.

4. The method of claim 1, comprising determining the methylation status of at least one cytosine residue in each of the methylation relevant region of the genes selected from:
ALOX12B, CRHR1, FGF4, FLJ33860, GJB5, GRM4, IGLL1, MGC35206, PCDHAC2, S100A9, SLC18A2, SLC6A11, SLC6A18, TRIM40, TUB and WNT10B as indicated in Table 1 signature C.

5. The method of claim 1, comprising determining the methylation status of at least one cytosine residue in each of the methylation relevant region of the genes selected from:
ACSL5, ALOX12B, ANKRD1, ANXA13, APOBEC4, AVPR1B, C1orf201, CD1D, CEACAM6, CRHR1, CTAG1B, CTAG2, DCLRE1A, DIRC1, FAAH, FGF4, FLJ25421, FLJ33860, FLJ39501, GABRG3, GJB3, GJB5, GLT1D1, GRM4, HA02, IFT140, IL28B, KCNK16, LRRN5, MGC35206, MIA2, OR12D3, PCDHAC2, PVALB, S100A9, SLC18A2, SLC4A7, SLC6A11, SLC6A18, STK38, TDRD5, TRIM40, TUB, UBE2E1, UNQ9433 and WNT10B as indicated in Table 1 signature D.

6. The method of claim 1, comprising determining the methylation status of at least one cytosine residue in each of the methylation relevant region of the genes selected from:
AMPH, ARHGEF7, AXL, BATF, BGN, C20orf100, C21orf70, C9orf90, COL5A2, CSAG1, CST7, CTAG2, CTSK, DENND2D, EMILIN1, EVL, FAM107B, FAM38B, FANCE, FOXF2, HCP5, KCNG1, KIAA0367, LY96, MAGEA1, MAGEA10, MAGEC2, MLSTD1, MT1G, NDN, OCIAD2, PDZD4, PGCP, PPIE, PRKCZ, PROCR, PRRX1, RAC2, RNASE1, SLCO4A1, SORBS2, SPATS1, SSNA1, TFF3, TMEPAI and ZNF324 as indicated in Table 1 signature E.

7. The method of claim 1, comprising determining the methylation status of at least one cytosine residue in each of the methylation relevant region of the genes selected from:
ABCA8, ABCB4, ACOX2, ADORA2B, AMPH, APOD, ARHGDIB, ARHGEF7, ARSG, ATP6V1E2, AXL, BGN, C11orf1, C20orf100, C21orf70, C9orf90, CHMP4A, COL5A2, CSAG1, CST7, CTAG2, CTNNAL1, CXorf12, DENND2D, EMILIN1, EMP1, ERO1L, EVL, FAM107B, FANCE, FLJ25421, GAGE2, GRAMD3, HCP5, HNMT, KCNG1, KIAA0141, KIAA0367, KRT25C, LY96, MAGEA1, MAGEA10, MAGEA6, MAGEC2, MGMT, MT1G, NBL1, NDN, OLFM1, PDZD4, PGCP, PLA1A, PLD1, PPIE, PROCR, PRRX1, RAC2, RNASE1, RPUSD3, SLAMF9, SLCO4A1, SPATS1, TFF3, TIMP3, TMEPAI, ZNF532 and ZNF558 as indicated in Table 1 signature F.

8. The method of claim 1, comprising determining the methylation status of at least one cytosine residue in each of the methylation relevant region of the genes selected from:
ABCA8, ADM, ALDH1A3, ARHGDIB, BGN, C9orf90, COL5A2, CSAG1, CTAG2, CTSK, DENND2D, EMILIN1, GSTT1, HCP5, LPXN, LY96, MAGEA1, MAGEA3, MAGEA6, MAGEC2, NNMT, OCIAD2, PAGE5, PCOLCE, PRDX2, RAB11FIP5, RNASE1 and SUHW1 as indicated in Table 1 signature G.

9. The method of claim 2 to 8 further comprising determining the methylation status of at least one cytosine residue in the methylation relevant region of the *LINE-1* repetitive sequence.

10. The method of any one of previous claim wherein the methylation relevant region of said genes comprises a nucleotide sequence selected from SEQ ID No 1 to SEQ ID No. 220 as shown in Table 2 , an allelic variant or a complementary sequence thereof.

11. The method of claim 10 wherein the methylation relevant region of said genes consists in a nucleotide selected from nt 941 to nt 1062 of SEQ ID No. 1, nt 940 to nt 1061 of SEQ ID No. 2, nt 940 to nt 1061 of SEQ ID No. 3, nt 941 to nt 1062 of SEQ ID No. 4, nt 940 to nt 1061 of SEQ ID No. 5, nt 940 to nt 1061 of SEQ ID No. 6, nt 940 to nt 1061 of SEQ ID No. 7 , nt 941 to nt 1062 of SEQ ID No. 8, nt 941 to nt 1062 of SEQ ID No. 9, nt 941 to nt 1062 of SEQ ID No. 10, nt 940 to nt 1061 of SEQ ID No. 11, nt 940 to nt 1061 of SEQ ID No. 12, nt 941 to nt 1062 of SEQ ID No. 13, nt 940 to nt 1061 of SEQ ID No. 14, nt 941 to nt 1062 of SEQ ID No. 15, nt 940 to nt 1061 of SEQ ID No. 16, nt 940 to nt 1061 of SEQ ID No. 17, nt 941 to nt 1062 of SEQ ID No. 18, nt 940 to nt 1061 of SEQ ID No. 19, nt 940 to nt 1061 of SEQ ID No. 20, nt 940 to nt 1061 of SEQ ID No. 21, nt 940 to nt 1061 of SEQ ID No. 22, nt 940 to nt 1061 of SEQ ID No. 23, nt 940 to nt 1061 of SEQ ID No. 24, nt 940 to nt 1061 of SEQ ID No. 25, nt 941 to nt 1062 of SEQ ID No. 26, nt 941 to nt 1062 of SEQ ID No. 27, nt 941 to nt 1062 of SEQ ID No. 28, nt 940 to nt 1061 of SEQ ID No. 29, nt 940 to nt 1061 of SEQ ID No. 30, nt 940 to nt 1061 of SEQ ID No. 31, nt 940 to nt 1061 of SEQ ID No. 32, nt 940 to nt 1061 of SEQ ID No. 33, nt 940 to nt 1061 of SEQ ID No. 34, nt 940 to nt 1061 of SEQ ID No. 35, nt 940 to nt 1061 of SEQ ID No. 36, nt 940 to nt 1061 of SEQ ID No. 37, nt 940 to nt 1061 of SEQ ID No. 38, nt 940 to nt 1061 of SEQ ID No. 39, nt 941 to nt 1062 of SEQ ID No. 40, nt 940 to nt 1061 of SEQ ID No. 41, nt 940 to nt 1061 of SEQ ID No. 42, nt 940 to nt 1061 of SEQ ID No. 43, nt 940 to nt 1061 of SEQ ID No. 44, nt 941 to nt 1062 of SEQ ID No. 45, nt 940 to nt 1061 of SEQ ID No. 46, nt 941 to nt 1062 of SEQ ID No. 47, nt 940 to nt 1061 of SEQ ID No. 48, nt 941 to nt 1062 of SEQ ID No. 49, nt 940 to nt 1061 of SEQ ID No. 50, nt 940 to nt 1061 of SEQ ID No. 51, nt 940 to nt 1061 of SEQ ID No. 52, nt 941 to nt 1062 of SEQ ID No. 53, nt 941 to nt 1062 of SEQ ID No. 54, nt 940 to nt 1061 of SEQ ID No. 55, nt 940 to nt 1061 of SEQ ID No. 56, nt 940 to nt 1061 of SEQ ID No. 57, nt 940 to nt 1061 of SEQ ID No. 58, nt 940 to nt 1061 of SEQ ID No. 59, nt 941 to nt 1062 of SEQ ID No. 60, nt 941 to nt 1062 of SEQ ID No. 61, nt 940 to nt 1061 of SEQ ID No. 62, nt 940 to nt 1061 of SEQ ID No. 63, nt 941 to nt 1062 of SEQ ID No. 64, nt 940 to nt 1061 of SEQ ID No. 65, nt 940 to nt 1061 of SEQ ID No. 66, nt 941 to nt 1062 of SEQ ID No. 67, nt 941 to nt 1062 of SEQ ID No. 68, nt 940 to nt 1061 of SEQ ID No. 69, nt 940 to nt 1061 of SEQ ID No. 70, nt 941 to nt 1062 of SEQ ID No. 71, nt 940 to nt 1061 of SEQ ID No. 72, nt 941 to nt 1062 of SEQ ID No. 73, nt 941 to nt 1062 of SEQ ID No. 74, nt 940 to nt 1061 of SEQ ID No. 75, nt 940 to nt 1061 of SEQ ID No. 76, nt 940 to nt 1061 of SEQ ID No. 77, nt 940 to nt 1061 of SEQ ID No. 78, nt 941 to nt 1062 of SEQ ID No. 79, nt 941 to nt 1062 of SEQ ID No. 80, nt 940 to nt 1061 of SEQ ID No. 81, nt 940 to nt 1061 of SEQ ID No. 82, nt 940 to nt 1061 of SEQ ID No. 83, nt 940 to nt 1061 of SEQ ID No. 84, nt 941 to nt 1062 of SEQ ID No. 85, nt 941 to nt 1062 of SEQ ID No. 86, nt 941 to nt 1062 of SEQ ID No. 87, nt 941 to nt 1062 of SEQ ID No. 88, nt 941 to nt 1062 of SEQ ID No. 89, nt 940 to nt 1061 of SEQ ID No. 90, nt 940 to nt 1061 of SEQ ID No. 91, nt 940 to nt 1061 of SEQ ID No. 92, nt 941 to nt 1062 of SEQ ID No. 93, nt 940 to nt 1061 of SEQ ID No. 94, nt 941 to nt 1062 of SEQ ID No. 95, nt 941 to nt 1062 of SEQ ID No. 96, nt 941 to nt 1062 of SEQ ID No. 97, nt 941 to nt 1062 of SEQ ID No. 98, nt 941 to nt 1062 of SEQ ID No. 99, nt 940 to nt 1061 of SEQ ID No. 100, nt 940 to nt 1061 of SEQ ID No. 101, nt 941 to nt 1062 of SEQ ID No. 102, nt 941 to nt 1062 of SEQ ID No. 103, nt 940 to nt 1061 of SEQ ID No. 104, nt 941 to nt 1062 of SEQ ID No. 105, nt 940 to nt 1061 of SEQ ID No. 106, nt 940 to nt 1061 of SEQ ID No. 107, nt 940 to nt 1061 of SEQ ID No. 108, nt 940 to nt 1061 of SEQ ID No. 109, nt 940 to nt 1061 of SEQ ID No. 110, nt 940 to nt 1061 of SEQ ID No. 111, nt 940 to nt 1061 of SEQ ID No. 112, nt 941 to nt 1062 of SEQ ID No. 113, nt 941 to nt 1062 of SEQ ID No. 114, nt 941 to nt 1062 of SEQ ID No. 115, nt 940 to nt 1061 of SEQ ID No. 116, nt 941 to nt 1062 of SEQ ID No. 117, nt 941 to nt 1062 of SEQ ID No. 118, nt 940 to nt 1061 of SEQ ID No. 119, nt 941 to nt 1062 of SEQ ID No. 120, nt 940 to nt 1061 of SEQ ID No. 121, nt 941 to nt 1062 of SEQ ID No. 122, nt 941 to nt 1062 of SEQ ID No. 123, nt 940 to nt 1061 of SEQ ID No. 124, nt 940 to nt 1061 of SEQ ID No. 125, nt 940 to nt 1061 of SEQ ID No. 126, nt 941 to nt 1062 of SEQ ID No. 127, nt 941 to nt 1062 of SEQ ID No. 128, nt 941 to nt 1062 of SEQ ID No. 129, nt 941 to nt 1062 of SEQ ID No. 130, nt 941 to nt 1062 of SEQ ID No. 131, nt 940 to nt 1061 of SEQ ID No. 132, nt 940 to nt 1061 of SEQ ID No. 133, nt 941 to nt 1062 of SEQ ID No. 134, nt 940 to nt 1061 of SEQ ID No. 135, nt 941 to nt 1062 of SEQ ID No. 136, nt 941 to nt 1062 of SEQ ID No. 137, nt 940 to nt 1061 of SEQ ID No. 138, nt 941 to nt 1062 of SEQ ID No. 139, nt 940 to nt 1061 of SEQ ID No. 140, nt 940 to nt 1061 of SEQ ID No. 141, nt 941 to nt 1062 of SEQ ID No. 142, nt 941 to nt 1062 of SEQ ID No. 143, nt 941 to nt 1062 of SEQ ID No. 144, nt 941 to nt 1062 of SEQ ID No. 145, nt 941 to nt 1062 of SEQ ID No. 146, nt 941 to nt 1062 of SEQ ID No. 147, nt 941 to nt 1062 of SEQ ID No. 148, nt 941 to nt 1062 of SEQ ID No. 149, nt 941 to nt 1062 of SEQ ID No. 150, nt 940 to nt 1061 of SEQ ID No. 151, nt 940 to nt 1061 of SEQ ID No. 152, nt 940 to nt 1061 of SEQ ID No. 153, nt 941 to nt 1062 of SEQ ID No. 154, nt 941 to nt 1062 of SEQ ID No. 155, nt 940 to nt 1061 of SEQ ID No. 156, nt 940 to nt 1061 of SEQ ID No. 157, nt 940 to nt 1061 of SEQ ID No. 158, nt 941 to nt 1062 of SEQ ID No. 159, nt 940 to nt 1061 of SEQ ID No. 160, nt 941 to nt 1062 of SEQ ID No. 161, nt 940 to nt 1061 of SEQ ID No. 162, nt 940 to nt 1061 of SEQ ID No. 163, nt 940 to nt 1061 of SEQ ID No. 164, nt 941 to nt 1062 of SEQ ID No. 165, nt 941 to nt 1062 of SEQ ID No. 166, nt 941 to nt 1062 of SEQ ID No. 167, nt 941 to nt 1062 of SEQ ID No. 168, nt 941 to nt 1062 of SEQ ID No. 169, nt 940 to nt 1061 of SEQ ID No. 170, nt 940 to nt 1061 of SEQ ID No. 171, nt 941 to nt 1062 of SEQ ID No. 172, nt 940 to nt 1061 of SEQ ID No. 173, nt 941 to nt 1062 of SEQ ID No. 174, nt 941 to nt 1062 of SEQ ID No. 175, nt 941 to nt 1062 of SEQ ID No. 176, nt 941 to nt 1062 of SEQ ID No. 177, nt 940 to nt 1061 of SEQ ID No. 178, nt 941 to nt 1062 of SEQ ID No. 179, nt 941 to nt 1062 of SEQ ID No. 180, nt 941 to nt 1062 of SEQ ID No. 181, nt 941 to nt 1062 of SEQ ID No. 182, nt 940 to nt 1061 of SEQ ID No. 183, nt 940 to nt 1061 of SEQ ID No. 184, nt 940 to nt 1061 of SEQ ID No. 185, nt 940 to nt 1061 of SEQ ID No. 186, nt 940 to nt 1061 of SEQ ID No. 187, nt 940 to nt 1061 of SEQ ID No. 188, nt 940 to nt 1061 of SEQ ID No. 189, nt 940 to nt 1061 of SEQ ID No. 190, nt 941 to nt 1062 of SEQ ID No. 191, nt 940 to nt 1061 of SEQ ID No. 192, nt 940 to nt 1061 of SEQ ID No. 193, nt 941 to nt 1062 of SEQ ID No. 194, nt 940 to nt 1061 of SEQ ID No. 195, nt 940 to nt 1061 of SEQ ID No. 196, nt 941 to nt 1062 of SEQ ID No. 197, nt 941 to nt 1062 of SEQ ID No. 198, nt 941 to nt 1062 of SEQ ID No. 199, nt 940 to nt 1061 of SEQ ID No. 200, nt 940 to nt 1061 of SEQ ID No. 201, nt 940 to nt 1061 of SEQ ID No. 202, nt 940 to nt 1061 of SEQ ID No. 203, nt 940 to nt 1061 of SEQ ID No. 204, nt 940 to nt 1061 of SEQ ID No. 205, nt 940 to nt 1061 of SEQ ID No. 206, nt 941 to nt 1062 of SEQ ID No. 207, nt 940 to nt 1061 of SEQ ID No. 208, nt 940 to nt 1061 of SEQ ID No. 209, nt 940 to nt 1061 of SEQ ID No. 210, nt 941 to nt 1062 of SEQ ID No. 211, nt 941 to nt 1062 of SEQ ID No. 212, nt 941 to nt 1062 of SEQ ID No. 213, nt 940 to nt 1061 of SEQ ID No. 214, nt 940 to nt 1061 of SEQ ID No. 215, nt 941 to nt 1062 of SEQ ID No. 216, nt 941 to nt 1062 of SEQ ID No. 217, nt 940 to nt 1061 of SEQ ID No. 218, nt 940 to nt 1061 of SEQ ID No. 219, nt 111 to nt 358 of SEQ ID No. 220, an allelic variant or a complementary sequence thereof.

12. The method of any one of previous claims wherein the at least one cytosine residue is selected from nt 1001 of SEQ ID No. 1, nt 1000 of SEQ ID No. 2, nt 1000 of SEQ ID No. 3, nt 1001 of SEQ ID No. 4, nt 1000 of SEQ ID No. 5, nt 1000 of SEQ ID No. 6, nt 1000 of SEQ ID No. 7, nt 1001 of SEQ ID No. 8, nt 1001 of SEQ ID No. 9, nt 1001 of SEQ ID No. 10, nt 1000 of SEQ ID No. 11, nt 1000 of SEQ ID No. 12, nt 1001 of SEQ ID No. 13, nt 1000 of SEQ ID No. 14, nt 1001 of SEQ ID No. 15, nt 1000 of SEQ ID No. 16, nt 1000 of SEQ ID No. 17, nt 1001 of SEQ ID No. 18, nt 1000 of SEQ ID No. 19, nt 1000 of SEQ ID No. 20, nt 1000 of SEQ ID No. 21, nt 1000 of SEQ ID No. 22, nt 1000 of SEQ ID No. 23, nt 1000 of SEQ ID No. 24, nt 1000 of SEQ ID No. 25, nt 1001 of SEQ ID No. 26, nt 1001 of SEQ ID No. 27, nt 1001 of SEQ ID No. 28, nt 1000 of SEQ ID No. 29, nt 1000 of SEQ ID No. 30, nt 1000 of SEQ ID No. 31, nt 1000 of SEQ ID No. 32, nt 1000 of SEQ ID No. 33, nt 1000 of SEQ ID No. 34, nt 1000 of SEQ ID No. 35, nt 1000 of SEQ ID No. 36, nt 1000 of SEQ ID No. 37, nt 1000 of SEQ ID No. 38, nt 1000 of SEQ ID No. 39, nt 1001 of SEQ ID No. 40, nt 1000 of SEQ ID No. 41, nt 1000 of SEQ ID No. 42, nt 1000 of SEQ ID No. 43, nt 1000 of SEQ ID No. 44, nt 1001 of SEQ ID No. 45, nt 1000 of SEQ ID No. 46, nt 1001 of SEQ ID No. 47, nt 1000 of SEQ ID No. 48, nt 1001 of SEQ ID No. 49, nt 1000 of SEQ ID No. 50, nt 1000 of SEQ ID No. 51, nt 1000 of SEQ ID No. 52, nt 1001 of SEQ ID No. 53, nt 1001 of SEQ ID No. 54, nt 1000 of SEQ ID No. 55, nt 1000 of SEQ ID No. 56, nt 1000 of SEQ ID No. 57, nt 1000 of SEQ ID No. 58, nt 1000 of SEQ ID No. 59, nt 1001 of SEQ ID No. 60, nt 1001 of SEQ ID No. 61, nt 1000 of SEQ ID No. 62, nt 1000 of SEQ ID No. 63, nt 1001 of SEQ ID No. 64, nt 1000 of SEQ ID No. 65, nt 1000 of SEQ ID No. 66, nt 1001 of SEQ ID No. 67, nt 1001 of SEQ ID No. 68, nt 1000 of SEQ ID No. 69, nt 1000 of SEQ ID No. 70, nt 1001 of SEQ ID No. 71, nt 1000 of SEQ ID No. 72, nt 1001 of SEQ ID No. 73, nt 1001 of SEQ ID No. 74, nt 1000 of SEQ ID No. 75, nt 1000 of SEQ ID No. 76, nt 1000 of SEQ ID No. 77, nt 1000 of SEQ ID No. 78, nt 1001 of SEQ ID No. 79, nt 1001 of SEQ ID No. 80, nt 1000 of SEQ ID No. 81, nt 1000 of SEQ ID No. 82, nt 1000 of SEQ ID No. 83, nt 1000 of SEQ ID No. 84, nt 1001 of SEQ ID No. 85, nt 1001 of SEQ ID No. 86, nt 1001 of SEQ ID No. 87, nt 1001 of SEQ ID No. 88, nt 1001 of SEQ ID No. 89, nt 1000 of SEQ ID No. 90, nt 1000 of SEQ ID No. 91, nt 1000 of SEQ ID No. 92, nt 1001 of SEQ ID No. 93, nt 1000 of SEQ ID No. 94, nt 1001 of SEQ ID No. 95, nt 1001 of SEQ ID No. 96, nt 1001 of SEQ ID No. 97, nt 1001 of SEQ ID No. 98, nt 1001 of SEQ ID No. 99, nt 1000 of SEQ ID No. 100, nt 1000 of SEQ ID No. 101, nt 1001 of SEQ ID No. 102, nt 1001 of SEQ ID No. 103, nt 1000 of SEQ ID No. 104, nt 1001 of SEQ ID No. 105, nt 1000 of SEQ ID No. 106, nt 1000 of SEQ ID No. 107, nt 1000 of SEQ ID No. 108, nt 1000 of SEQ ID No. 109, nt 1000 of SEQ ID No. 110, nt 1000 of SEQ ID No. 111, nt 1000 of SEQ ID No. 112, nt 1001 of SEQ ID No. 113, nt 1001 of SEQ ID No. 114, nt 1001 of SEQ ID No. 115, nt 1000 of SEQ ID No. 116, nt 1001 of SEQ ID No. 117, nt 1001 of SEQ ID No. 118, nt 1000 of SEQ ID No. 119, nt 1001 of SEQ ID No. 120, nt 1000 of SEQ ID No. 121, nt 1001 of SEQ ID No. 122, nt 1001 of SEQ ID No. 123, nt 1000 of SEQ ID No. 124, nt 1000 of SEQ ID No. 125, nt 1000 of SEQ ID No. 126, nt 1001 of SEQ ID No. 127, nt 1001 of SEQ ID No. 128, nt 1001 of SEQ ID No. 129, nt 1001 of SEQ ID No. 130, nt 1001 of SEQ ID No. 131, nt 1000 of SEQ ID No. 132, nt 1000 of SEQ ID No. 133, nt 1001 of SEQ ID No. 134, nt 1000 of SEQ ID No. 135, nt 1001 of SEQ ID No. 136, nt 1001 of SEQ ID No. 137, nt 1000 of SEQ ID No. 138, nt 1001 of SEQ ID No. 139, nt 1000 of SEQ ID No. 140, nt 1000 of SEQ ID No. 141, nt 1001 of SEQ ID No. 142, nt 1001 of SEQ ID No. 143, nt 1001 of SEQ ID No. 144, nt 1001 of SEQ ID No. 145, nt 1001 of SEQ ID No. 146, nt 1001 of SEQ ID No. 147, nt 1001 of SEQ ID No. 148, nt 1001 of SEQ ID No. 149, nt 1001 of SEQ ID No. 150, nt 1000 of SEQ ID No. 151, nt 1000 of SEQ ID No. 152, nt 1000 of SEQ ID No. 153, nt 1001 of SEQ ID No. 154, nt 1001 of SEQ ID No. 155, nt 1000 of SEQ ID No. 156, nt 1000 of SEQ ID No. 157, nt 1000 of SEQ ID No. 158, nt 1001 of SEQ ID No. 159, nt 1000 of SEQ ID No. 160, nt 1001 of SEQ ID No. 161, nt 1000 of SEQ ID No. 162, nt 1000 of SEQ ID No. 163, nt 1000 of SEQ ID No. 164, nt 1001 of SEQ ID No. 165, nt 1001 of SEQ ID No. 166, nt 1001 of SEQ ID No. 167, nt 1001 of SEQ ID No. 168, nt 1001 of SEQ ID No. 169, nt 1000 of SEQ ID No. 170, nt 1000 of SEQ ID No. 171, nt 1001 of SEQ ID No. 172, nt 1000 of SEQ ID No. 173, nt 1001 of SEQ ID No. 174, nt 1001 of SEQ ID No. 175, nt 1001 of SEQ ID No. 176, nt 1001 of SEQ ID No. 177, nt 1000 of SEQ ID No. 178, nt 1001 of SEQ ID No. 179, nt 1001 of SEQ ID No. 180, nt 1001 of SEQ ID No. 181, nt 1001 of SEQ ID No. 182, nt 1000 of SEQ ID No. 183, nt 1000 of SEQ ID No. 184, nt 1000 of SEQ ID No. 185, nt 1000 of SEQ ID No. 186, nt 1000 of SEQ ID No. 187, nt 1000 of SEQ ID No. 188, nt 1000 of SEQ ID No. 189, nt 1000 of SEQ ID No. 190, nt 1001 of SEQ ID No. 191, nt 1000 of SEQ ID No. 192, nt 1000 of SEQ ID No. 193, nt 1001 of SEQ ID No. 194, nt 1000 of SEQ ID No. 195, nt 1000 of SEQ ID No. 196, nt 1001 of SEQ ID No. 197, nt 1001 of SEQ ID No. 198, nt 1001 of SEQ ID No. 199, nt 1000 of SEQ ID No. 200, nt 1000 of SEQ ID No. 201, nt 1000 of SEQ ID No. 202, nt 1000 of SEQ ID No. 203, nt 1000 of SEQ ID No. 204, nt 1000 of SEQ ID No. 205, nt 1000 of SEQ ID No. 206, nt 1001 of SEQ ID No. 207, nt 1000 of SEQ ID No. 208, nt 1000 of SEQ ID No. 209, nt 1000 of SEQ ID No. 210, nt 1001 of SEQ ID No. 211, nt 1001 of SEQ ID No. 212, nt 1001 of SEQ ID No. 213, nt 1000 of SEQ ID No. 214, nt 1000 of SEQ ID No. 215, nt 1001 of SEQ ID No. 216, nt 1001 of SEQ ID No. 217, nt 1000 of SEQ ID No. 218, nt 1000 of SEQ ID No. 219, nt 252, 256 or 270 of SEQ ID No. 220 or at the corresponding position on the complementary sequence of SEQ ID No. 1 to 220.

13. The method of any one of claims 1-12, wherein the melanoma is cutaneous melanoma, preferably it is at stage III or IIIC.

14. The method of any one of claims 1 to 13, comprising the steps of:
(a) isolating DNA from a sample obtained from a subject;
(b) subjecting said DNA to methylation-sensitive sequence modification through incubation with chemicals (e.g., sodium bisulfite), or contacting said DNA with a polypeptide capable of binding methylated DNA;
(c) optionally amplifying said DNA modified with sodium bisulfite or bound by said polypeptide; and
(d) determining the methylation status of said DNA.

15. A kit for performing the method of any one of claims 1 to 14, comprising a chemical able to differentially modify DNA sequence depending on methylation status, or a polypeptide capable of binding methylated DNA, and probes, primers and/or aptamers specific for at least one gene as defined in claim 1 to 13 or a variant thereof.

16. The kit of claim 15 which is a kit for prognosing melanoma and/or of predicting and/or monitoring the response and/or the efficacy of a melanoma therapy or to identify a subject to be treated with a DNA hypomethylation agent.

17. A microarray able to determine the methylation status of at least one cytosine residue in a methylation relevant region of at least one gene as defined in any one of claims 1 to 12.
